(19) Europäisches Patentamt
European Patent Office
Office européen des brevets

(11) EP 2 441 839 A1

(12) EUROPEAN PATENT APPLICATION

(43) Date of publication:
**18.04.2012 Bulletin 2012/16**

(51) Int Cl.:
***C12N 15/82** (2006.01)*

(21) Application number: **11164637.8**

(22) Date of filing: **30.05.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**03.08.2006 US 835303 P**
**02.06.2006 US 810759 P**
**02.06.2006 US 810749 P**
**13.07.2006 US 830551 P**
**14.06.2006 US 813514 P**
**18.07.2006 US 831642 P**
**02.06.2006 US 810572 P**
**30.05.2006 EP 06114735**
**31.05.2006 EP 06114758**
**30.06.2006 EP 06116490**
**28.07.2006 EP 06118060**
**08.06.2006 EP 06115142**
**30.05.2006 EP 06114726**
**12.07.2006 EP 06117060**

(62) Document number(s) of the earlier application(s) in accordance with Art. 76 EPC:
**07729653.1 / 2 035 562**

(71) Applicant: **CropDesign N.V.**
**9052 Zwijnaarde (BE)**

(72) Inventors:
- **Frankard, Valerie**
  **1410 Waterloo (BE)**
- **Mironov, Vladimir**
  **9000 Gent (BE)**
- **Reuzeau, Christophe**
  **24350 La Chapelle Gonaguet (FR)**

(74) Representative: **Krieger, Stephan Gerhard**
**BASF SE**
**Global Intellectual Property - GVX/B**
**Carl-Bosch-Strasse 38**
**67056 Ludwigshafen (DE)**

Remarks:
This application was filed on 03-05-2011 as a divisional application to the application mentioned under INID code 62.

# (54) Plants with reduced expression of REVOLUTA (REV) having enhanced yield-related traits and a method for making the same

(57) The present invention relates generally to the field of molecular biology and concerns a method for improving various plant growth characteristics by modulating expression in a plant of a nucleic acid encoding a GRP (Growth-Related Protein). The present invention also concerns plants having modulated expression of a nucleic acid encoding a GRP, which plants have improved growth characteristics relative to corresponding wild type plants or other control plants. The invention also provides constructs useful in the methods of the invention. The GRP may be a REV delta homeodomain leucine zipper domain polypepetide.

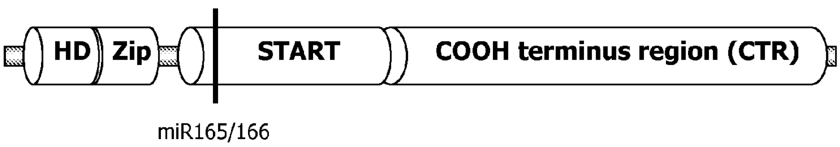


FIGURE 3



Printed by Jouve, 75001 PARIS (FR)

## Description

The present invention relates generally to the field of molecular biology and concerns a method for improving various plant growth characteristics by modulating expression in a plant of a nucleic acid encoding a GRP (Growth Related Protein). The present invention also concerns plants having modulated expression of a nucleic acid encoding a GRP, which plants have improved growth characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigor has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various growth characteristics may be improved in plants by modulating expression in a plant of a nucleic acid encoding a GRP (Growth Related Protein Of Interest) in a plant. The GRP may a REV delta homeodomain leucine zipper domain polypepetide.

## Background

REV delta (Δ) homeodomain leucine zipper domain (REV ΔHDZip/START)

The present invention concerns increasing yield in plants using a particular type of transcription factor. Transcription factor polypeptides are usually defined as proteins that show sequence-specific DNA binding affinity and that are capable of activating and/or repressing transcription. Homeodomain leucine zipper (HDZip) polypeptides constitute a family of transcription factors characterized by the presence of a DNA-binding domain (homeodomain, HD) and an adjacent leucine zipper (Zip) motif. The homeodomain usually consists of approximately 60 conserved amino acid residues that form a helix1-loop-helix2-turn-helix3 that binds DNA. This DNA binding site is usually pseudopalindromic. The leucine zipper, adjacent to the C-terminal end of the homeodomain (in some instances, overlapping by a few amino acids), consists of several amino acid heptad repeats (at least four) in which usually a leucine (occasionally a valine or an isoleucine) appears every seventh amino acid. The leucine zipper is important for protein dimerisation. This dimerisation is a prerequisite for DNA binding (Sessa et al. (1993) EMBO J 12(9): 3507-3517), and may proceed between two identical HDZip polypeptides (homodimer) or between two different HDZip polypeptides (heterodimer).

Homeodomain encoding genes are present in all eucaryotes, and constitute a gene family of at least 89 members in *Arabidopsis thaliana.* The leucine zipper is also found by itself in polypeptides from eucaryotes other than plants. However, the simultaneous presence of both a homeodomain and a leucine zipper comprised within the same polypeptide is plant-specific (found in at least 47 out of the 89 members in *Arabidopsis),* and has been encountered in moss in addition to vascular plants (Sakakibara et al. (2001) Mol Biol Evol 18(4): 491-502).

The *Arabidopsis* HDZip polypeptides have been classified into four different classes, HDZip I to IV, based on sequence similarity criteria (Sessa et al. (1994) In: Puigdomene P, Coruzzi G (ed), Springer, Berlin Heidelberg New York, pp 411-426). In *Arabidopsis thaliana,* there are at least five class III HDZip polypeptides *(REVOLUTA* (REV/IFL), *PHABULOSA* (PHB), *PHAVOLUTA* (PHV), *CORONA* (CNA/AtHB15) and AtHB8), all typically quite large (more than 800 amino acids). Similarly, in *Oryza sativa,* at least 5 class III HDZip polypeptides have been identified.

In addition to the homeodomain and the leucine zipper, class III HDZip polypeptides also comprise C-terminal to the leucine zipper a START (STeroidogenic Acute Regulatory (STAR) related lipid Transfer) domain for lipid/sterol binding and an extensive C-terminal region (CTR, more than half of the polypeptide length) of unknown function (Schrick et al. (2004) Genome Biology 5: R41). Furthermore, a complementary site for microRNA (MIR165/166) is found within the transcript region coding for the START domain of mRNA transcripts coding for class III HDZip polypeptides, for regulation via miRNA-mediated transcript cleavage (Williams et al. (2005) Development 132: 3657-3668).

In *Arabidopsis thaliana,* REV, PHB and PHV polypeptides have been shown to be involved in formation and function of shoot apical and axillary meristems, patterning of three dimensional structures (such as embryos or leaves), and vascular development (differentiation of lignified conducting and support tissues). In *Arabidopsis thaliana,* phylogenetic analysis reveals that these three closely related class III HDZip polypeptides form the REV clade, the two remaining class III HDZip polypeptides (CNA and AtHB8) belonging to the CNA clade (Floyd et al. (2006) Genetics 173: 373-388). When combining rice and *Arabidopsis* genes in a phylogenetic analysis, two rice class III HDZip polypeptides cluster with REV polypeptide, the OsHox10 and OsREV polypeptides.

Loss-of-function *phb, phv, cna* and *athb8 Arabidopsis thaliana* mutants are aphenotypic (Baima et al. (2001) Plant Physiol 126: 643 -655; Prigge et al. (2005) Plant Cell 17: 61 -76), but *rev* mutants form defective lateral and floral meristems and develop aberrant stem vasculature as well as curly (revolute) leaves (Otsuga et al. (2001) Plant J. 25,223 -236; Talbert et al. (1995) Development 121: 2723-2735).

The dominant alleles *rolled leaf1* (*rld1*) in corn (Juarez et al. (2004) Nature 428: 84-88), *phb-d* and *phv-d* in *Arabidopsis* (McConnell et al. (2001) Nature 411: 709-713), and *rev-d* in *Arabidopsis* (Emery et al. (2003) Curr Biol 13: 1768-1774) present similar mutant phenotypes (rolled leaves), due to a nucleotide substitution in the sequence spanning the MIR165/166 binding site (in the START domain).

In granted US patent US7056739 (and corresponding international patent application WO01/33944), are described plants and plant cells transformed with a transgene comprising an *Arabidopsis thaliana* REV nucleic acid sequence encoding a REV polypeptide represented by SEQ ID NO: 2 (of the granted patent). The transgene is reported to further comprise a heterologous promoter operably linked to a nucleic acid sequence encoding the polypeptide of SEQ ID NO: 2. Transgenic *Arabidopsis* plants overexpressing the REV nucleic acid sequence by using the CaMV promoter, presented increased leaf, stem and seed size. Partial class III HDZip genomic and cDNA 5' terminal sequences from tomato, rice, maize and barley are provided. Examples of vectors designed to reduce the expression of endogenous *Arabidopsis,* rice and corn class III HDZip polypeptides in respectively rice and corn are described, to reproduce the *rev* loss-of-function phenotype.

In international patent application WO2004/063379, are provided nucleic acid sequences of two corn class III HDZip polypeptides orthologous to the *Arabidopsis* REV polypeptide. A method to modulate the level of class III HDZip polypeptides by inhibiting expression of the mRNA transcripts in the plant cell is described.

In a number of international and US patent applications deposited by Mendel Biotechnology, Inc., the *Arabidopsis* REV polypeptide has as internal reference G438. Reduced REVOLUTA activity after T-DNA insertion into the REV locus resulted in transgenic *Arabidopsis* plants with reduced branching and reduced lignin content, whereas increased REVOLUTA activity (using the viral CaMV promoter) resulted in transgenic *Arabidopsis* plants of which around half developed slightly larger flatter leaves than wild type plants at late stages.

**Summary of the invention**

It has also surprisingly been found that reducing the expression in a plant of an endogenous REV gene using a REV delta homeodomain leucine zipper domain (HDZip) /STeroidogenic Acute Regulatory (STAR) related lipid Transfer domain (START) nucleic acid sequence gives plants having increased yield relative to control plants. The present invention therefore provides in another embodiment methods for increasing yield in plants relative to control plants, by reducing the expression in a plant of an endogenous REV gene using a REV Δ4HDZip/START nucleic acid sequence.

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)" "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag-100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41x\%[G/C^b] - 500x[L^c]^{-1} - 0.61x\% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m$= 2 (In)
For 20-35 nucleotides: $T_m$= 22 + 1.46 ($l_n$)
a or for other monovalent cation, but only accurate in the 0.01-0.4 M range.
[b] only accurate for %GC in the 30% to 75% range.
[C] L = length of duplex in base pairs.
[d] oligo, oligonucleotide; $l_n$, = effective length of primer = 2x(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1 x SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGB | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |

(continued)

| Gene Source | Reference |
|---|---|
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1 ); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. Some seed specific promoters may be specific for the endosperm, aleurone and/or embryo. Examples of seed-specific promoters are shown in Table 2b below and of endosperm-specific promoters in Table 2c. Further examples of seed-specific promoters are given in Qing Qu and Takaiwa (Plant Biotechnol. J. 2, 113-125, 2004), which disclosure is incorporated by reference herein as if fully set forth.

Table 2b: Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |

(continued)

| Gene source | Reference |
|---|---|
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley ltr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophos-phorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PR00117, putative rice 40S ribosomal protein | WO 2004/070039 |
| PRO0136 rice alanine aminotransferase | unpublished |
| PRO0147 trypsin inhibitor ITR1 (barley) | unpublished |
| PRO0151, rice WSI18 | WO 2004/070039 |
| PRO0175 rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |

(continued)

| Gene source | Reference |
|---|---|
| Maize B-Peru | Selinger et al., Genetics 149;1125-38,1998 |

Table 2c: Examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley ltr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene.

Isolated gene

The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonudeotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, a polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per acre for a crop and year, which is determined by dividing total production (includes both harvested and appraised production) by planted acres. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp.,

*Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description of the invention**

**REV DHDZip/START**

It has now surprisingly been found that reducing the expression in a plant of an endogenous REV gene using a REV delta homeodomain leucine zipper domain (HDZip) /STeroidogenic Acute Regulatory (STAR) related lipid Transfer domain (START) nucleic acid sequence gives plants having increased yield relative to control plants. The present invention therefore provides methods for increasing yield in plants relative to control plants, by reducing the expression in a plant of an endogenous REV gene using a REV ΔHDZip/START nucleic acid sequence.

Advantageously, performance of the methods according to the present invention results in plants having enhanced yield related traits, particularly increased yield, more particularly increased seed yield and/or increased biomass, relative to control plants. The terms "yield" and "seed yield" are described in more detail in the "definitions" section herein. Increased biomass may manifest itself as increased root biomass. Increased root biomass may be due to increased number of roots, increased root thickness and/or increased root length.

The term "increased yield" also refers to an increase in biomass (weight) of one or more parts of a plant, which may include aboveground (harvestable) parts and/or (harvestable) parts below ground. Such harvestable parts include vegetative biomass and/or seeds, and performance of the methods of the invention results in plants having increased yield (in vegetative biomass and/or seed) relative to the yield of control plants.

Therefore, according to the present invention, there is provided a method for increasing seed yield and/or plant biomass, which method comprises reducing the expression in a plant of an endogenous REV gene using a REV ΔHDZip/START nucleic acid sequence.

In particular, the increased seed yield is selected from one or more of the following: (i) increased seed weight; (ii) increased number of filled seeds; (iii) increased seed fill rate; (iv) increased harvest index; and (v) increased individual seed length.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression, preferably increasing expression, in a plant of a nucleic acid encoding a REV ΔHDZip/START polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a REV ΔHDZip/START polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a REV ΔHDZip/START polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a REV ΔHDZip/START polypeptide. Nutrient deficiency may result from a lack of nutrients such as nitrogen, phosphates and other phosphorous-containing compounds, potassium, calcium, cadmium, magnesium, manganese, iron and boron, amongst others.

Reference herein to an "endogenous" REV gene not only refers to a REV gene as found in a plant in its natural form (i.e., without there being any human intervention), but also refers to isolated REV nucleic acid sequences subsequently introduced into a plant. For example, a transgenic plant containing a REV transgene may encounter a substantial reduction of the transgene expression and/or substantial reduction of expression of an endogenous REV gene, according to the methods of the invention.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

"Reduction" or "decrease" of expression are used interchangeably herein, and refer, for the methods of the present invention, to a diminution, but not the elimination, of endogenous REV gene expression and/or REV polypeptide levels and/or REV polypeptide activity, using a REV ΔHDZip/START nucleic acid sequence, relative respectively to REV gene expression and/or REV polypeptide level and/or REV polypeptide activity found in control plants. The reduction of REV gene expression and/or REV polypeptide level and/or REV polypeptide activity is taken to mean in the sense of the application at least 10%, 20%, 30%, 40% or 50%, preferably at least 60%, 70 or 80%, more preferably 85%, 90%, or 95% less REV gene expression and/or REV polypeptide level and/or REV polypeptide activity in comparison to a control plant as defined herein. Preferably, reducing the expression of the endogenous REV gene using a REV ΔHDZip/START nucleic acid sequence leads to the appearance of one or more phenotypic traits.

This reduction of endogenous REV gene expression may be achieved by using any one or more of several well-known "gene silencing" methods (see definitions section for more details). The term "silencing" of a gene as used herein refers to the reduction, but not the elimination, of endogenous REV gene expression.

A preferred method for reducing expression in a plant of an endogenous REV gene via RNA-mediated silencing is by using an inverted repeat of a REV ΔHDZip/START nucleic acid sequence, preferably capable of forming a hairpin structure. The inverted repeat is cloned into an expression vector comprising control sequences. A non-coding DNA nucleic acid sequence (a spacer, for example a matrix attachment region fragment (MAR), an intron, a polylinker, etc.) is located between the two inverted REV ΔHDZip/START nucleic acid sequences forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into a RISC. The RISC further cleaves the mRNA transcripts encoding a REV polypeptide, thereby reducing the number of mRNA transcripts to be translated into a REV polypeptide. See for example, Grierson et al. (1998) WO 98/53083; Waterhouse et al. (1999) WO 99/53050).

The expression of an endogenous REV gene may also be reduced by introducing a genetic modification, within the locus of a REV gene or elsewhere in the genome. The locus of a gene as defined herein is taken to mean a genomic region, which includes the gene of interest and 10 kb up- or down stream of the coding region.

The genetic modification may be introduced, for example, by any one (or more) of the following methods: T-DNA tagging, TILLING, site-directed mutagenesis, directed evolution, homologous recombination. Following introduction of the genetic modification, there follows a step of selecting for reduced expression of an endogenous REV gene, which reduction in expression gives plants having increased yield compared to control plants.

T-DNA tagging involves insertion of a T-DNA, in the genomic region of the gene of interest or 10 kb up- or downstream of the coding region of a gene in a configuration such that the T-DNA reduces (but does not eliminate) the expression of the targeted gene.

Homologous recombination allows introduction in a genome of a selected nucleic acid at a defined selected position. Alternatively, a screening program may be set up to identify in a plant population natural variants of a REV gene which variants encode REV polypeptides with reduced activity. Such natural variants may also be used for example, to perform homologous recombination.

T-DNA tagging, TILLING, site-directed mutagenesis and directed evolution are examples of technologies that enable the generation of novel alleles and variants of REV nucleic acid sequences which variants encode REV polypeptides with reduced activity.

Other methods, such as the use of antibodies directed to an endogenous REV polypeptide for inhibiting its function *in planta,* or interference in the signalling pathway in which a REV polypeptide is involved, will be well known to the skilled man.

For optimal performance, the RNA-mediated silencing techniques used for reducing expression in a plant of an endogenous REV gene using a REV ΔHDZip/START nucleic acid sequence, requires the use of nucleic acid sequences from monocotyledonous plants for transformation of monocotyledonous plants, and from dicotyledonous plants for transformation of dicotyledonous plants. Preferably, a REV ΔHDZip/START nucleic acid sequence from any given plant species is introduced into that same species. For example, a REV ΔHDZip/START nucleic acid sequence from rice is transformed into a rice plant. The REV ΔHDZip/START nucleic acid sequence need not be introduced into the same plant variety.

Reference herein to a "nucleic acid sequence" is taken to mean a polymeric form of a deoxyribonucleotide or a ribonucleotide polymer of any length, either double- or single-stranded, or analogues thereof, that has the essential characteristic of a natural ribonucleotide in that it can hybridise to nucleic acid sequences in a manner similar to naturally occurring polynucleotides.

Reference herein to a "REV ΔHDZip/START" nucleic acid sequence is taken to mean a sufficient length of substantially contiguous nucleotides from a REV nucleic acid sequence substantially excluding the part encoding the HDZip and START domains. In order to perform gene silencing, this may be as little as 20 or fewer nucleotides, alternatively this may be as much as the REV ΔHDZip/START nucleic acid sequence (including the 5' and/or 3' UTR, either in part or in whole. A person skilled in the art would be aware that a sufficient length of substantially contiguous nucleotides from the REV nucleic acid sequence encoding the HDZip and START domains is to be excluded in performing the methods of the invention. This may be as little as 10, 11, 12, 13, 14, 15, 16, 17, 18, 19, 20 or more nucleotides. This may be as much as the complete nucleic acid sequence encoding the HDZip and START domains. By nucleic acid sequence "encoding the HDZip and START domains" is meant herein the region of the nucleic acid sequence comprising codons that are translated into amino acid residues of the HDZip and START domains (which domains do not need to be complete and/or functional). A person skilled in the art would be aware that substantially contiguous nucleotides from a REV nucleic acid sequence may overlap HDZip and START domain boundaries by a few nucleotides, typically by not more than 20 nucleotides. Also excluded in performing the methods of the invention are nucleic acid sequences that would simultaneously reduce expression of at least one other endogenous gene, regardless whether the other endogenous gene encodes for a polypeptide comprising an HDZip and START domain or not. A nucleic acid sequence encoding a (functional) polypeptide is not a requirement for the various methods discussed above for the substantial reduction of expression of an endogenous REV gene.

REV genes are well known in the art (described recently in Floyd et al. ((2006) Genetics 173: 373-388) and useful in the methods of the invention are REV ΔHDZip/START nucleic acid sequences.

Other REV ΔHDZip/START nucleic acid sequences may also be used in the methods of the invention, and may readily be identified by a person skilled in the art. REV polypeptides may be identified by the presence of one or more of several well-known features (see below). Upon identification of a REV polypeptide, a person skilled in the art could easily derive, using routine techniques, the corresponding encoding REV ΔHDZip/START nucleic acid sequence, and use a sufficient length of contiguous nucleotides of the same to perform any one or more of the gene silencing methods described above.

The term "REV polypeptide" as defined herein refers to a polypeptide that falls into the class III of the HDZip polypeptides as delineated by Sessa et al. ((1994) In : Puigdomene P, Coruzzi G (ed), Springer, Berlin Heidelberg New York, pp 411-426). REV polypeptides comprise from N-terminus to C-terminus: (i) a homeodomain (HD) domain, for DNA binding; (ii) a leucine zipper, for protein-protein interaction; (iii) a START domain for lipid/sterol binding, and (iv) a C-terminal region (CTR), of undefined function.

REV polypeptides may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. REV polypeptides comprising a homeodomain, a leucine zipper, a START domain and a CTR may readily be identified using, for example, the ClustalW multiple sequence alignment algorithm (version 1.83), with the default pairwise alignment parameters, and a scoring method in percentage. Minor manual editing may be performed to optimise the alignment, as would be apparent to a person skilled in the art. A phylogenetic tree, which is an estimate of phylogeny (or common ancestry), may be constructed using the Neighbour-Joining tree building algorithm (at EBI), to help visualize clustering of related genes and to identify orthologues and paralogues. REV polypeptides as defined herein refers to any polypeptide which, when used in the construction of a class III HDZip polypeptide phylogenetic tree, such as the one depicted in Figures 1 and 2, falls into the REV clade (comprising REV, PHB and PHV) and not the CORONA clade (comprising ATHb8 and CNA), and more specifically, which falls into the REV branch (and not the PHB/PHV branch). Upon identification of a REV polypeptide (falling into the REV branch), a person skilled in the art could easily derive, using routine techniques, the corresponding encoding REV ΔHDZip/START nucleic acid sequence and use a sufficient length of contiguous nucleotides of the same to perform any one or more of the gene silencing methods described above.

Orthologues and paralogues may easily be found by performing a so-called reciprocal blast search. This may be done by a first BLAST involving BLASTing a query sequence (for example, SEQ ID NO: 198 or SEQ ID NO: 199) against any sequence database, such as the publicly available NCBI database. BLASTN or TBLASTX (using standard default values) may be used when starting from a nucleotide sequence and BLASTP or TBLASTN (using standard default values) may be used when starting from a polypeptide sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 198 or SEQ ID NO: 199, the second BLAST would therefore be against rice sequences). High-ranking hits are those having a low E-value. In addition to E-values, comparisons are also scored by percentage identity. Percentage identity refers to the number of identical nucleotides (or amino acids) between the two compared nucleic acid (or polypeptide) sequences over a particular length. An example detailing the identification of orthologues and paralogues is given in Example 2. All REV polypeptides comprise a CTR having, in increasing order of preference, at least 50%, 55%, 60%, 65%, 70%, 75%, 80%, 85%, 90%, 95% or 98% sequence identity to the CTR of a REV polypeptide as represented by SEQ ID NO: 197. Preferably, the CTR of a REV polypeptide is as represented by SEQ ID NO: 197. More preferably, SEQ ID NO: 194 encoding a part of the CTR of a REV polypeptide is used in performing the methods according the invention. In Figure 2, the REV polypeptide paralogues and orthologues cluster together.

The terms "domain" and "motif" are defined above. The term "region" as defined herein refers to the amino acid sequence starting at the end of the START domain and ending at the stop codon of the REV polypeptide.

Special databases exist for the identification of domains. The HD and the START domains in a REV polypeptide may be identified using, for example, SMART, InterPro, Prosite, or Pfam. The HD comprises about 60 residues, the START domain about 210 residues. In the InterPro database, the HD is designated by IPR001356, PF00046 in the Pfam database and PS50071 in the PROSITE database. In the InterPro database, the START domain is designated by IPR002913, PF01852 in the Pfam database and PS50848 in the PROSITE database. For example in SEQ ID NO: 199, the HD Pfam entry spans from amino acids 27 to 87, and the START domain Pfam entry from 163 to 376. The CTR therefore begins at amino acid 377 and ends at the stop codon (at 840). Leucine zipper prediction and heptad identification may be done using specialised software such as 2ZIP, which combines a standard coiled coil prediction algorithm with an approximate search for the characteristic leucine repeat (Bornberg-Bauer et al. (1998) Computational Approaches to Identify Leucine Zippers, Nucleic Acids Res., 26(11): 2740-2746), hosted on a server at Max Planck Institute for Molecular Genetics in Berlin. For example, the leucine zipper of SEQ ID NO: 199 comprises five leucine repeats (heptads), and spans amino acids 91 to 127.

Furthermore, a REV polypeptide may also be identifiable by its ability to bind DNA and to interact with other proteins. DNA-binding activity and protein-protein interactions may readily be determined in vitro or in vivo using techniques well known in the art. Examples of *in vitro* assays for DNA binding activity include: gel retardation analysis using the HD DNA binding domain (West et al. (1998) Nucl Acid Res 26(23): 5277-87), or yeast one-hybrid assays. An example of an *in vivo* assay for protein-protein interactions is the yeast two-hybrid analysis (Fields and Song (1989) Nature 340: 245-6).

Therefore upon identification of a REV polypeptide using one or several of the features described above, a person skilled in the art may easily derive the corresponding REV ΔHDZip/START nucleic acid sequence and use a sufficient length of substantially contiguous nucleotides of the same to perform any one or more of the gene silencing methods described above (for the substantial reduction of an endogenous REV gene expression).

Preferred for use in the methods of the invention is a nucleic acid sequence as represented by SEQ ID: 194, encoding part of the CTR of an *Oryza sativa* REV polypeptide, or SEQ ID NO: 196, encoding the CTR of the same *Oryza sativa* REV polypeptide. REV ΔHDZip/START nucleic acid sequences are comprised in the nucleic acid sequences encoding REV polypeptide orthologues or paralogues. An example of a REV polypeptide paralogue to SEQ ID NO: 199 is represented by SEQ ID NO: 201, *Oryza sativa* Orysa_HOX10 (encoded by SEQ ID NO: 200, NCBI accession number AY425991.1). Examples of REV polypeptide orthologues are represented by SEQ ID NO: 203 *Arabidopsis thaliana* Arath_REV (encoded by SEQ ID NO: 202, NCBI accession number AF188994), SEQ ID NO: 205 *Zea mays* Zeama_HDIII RLD1 (rolled leaf1; encoded by SEQ ID NO: 204, NCBI accession number AY501430.1), SEQ ID NO: 207 *Populus trichocarpa* Poptr_HDIII (encoded by SEQ ID NO: 206, NCBI accession number AY919617), SEQ ID NO: 209 *Medicago truncunlata* Medtr_HDIII (encoded by SEQ ID NO: 208, NCBI accession number AC138171.17), SEQ ID NO: 211 *Saccharum officinarum* Sacof HDIIIpartial (encoded by SEQ ID NO: 210, contig of NCBI accession numbers CA125167.1 CA217027.1 CA241276.1 CA124509.1), SEQ ID NO: 213 *Triticum aestivum* Triae_HDIII (partial; encoded by SEQ ID NO: 212, contig of NCBI accession numbers CD905903 BM135681.1 BQ578798.1 CJ565259.1), SEQ ID NO: 215 *Hordeum vulgare* Horvu_HDIII (partial, encoded by SEQ ID NO: 214, contig of NCBI accession numbers BU996988.1 BJ452342.1 BJ459891.1), and SEQ ID NO: 217 *Phyllostachys praecox* Phypr_HDIII (partial; encoded by SEQ ID NO: 216, NCBI accession number DQ013803). In example 2 (and table Z) of the present application is described a method to identify nucleic acid sequences useful in performing the methods of the invention.

The source of the REV ΔHDZip/START nucleic acid sequence useful in performing the methods of the invention may be any plant source or artificial source. For optimal performance, the gene silencing techniques used for the reduction of an endogenous REV gene expression requires the use of REV ΔHDZip/START nucleic acid sequences from monocotyledonous plants for transformation of monocotyledonous plants, and use of REV ΔHDZip/START nucleic acid sequences from dicotyledonous plants for transformation of dicotyledonous plants. Preferably, REV ΔHDZip/START nucleic acid sequences from plants of the family Poaceae are transformed into plants of the family Poaceae. Further preferably, a REV ΔHDZip/START nucleic acid sequence from rice is transformed into a rice plant. The REV ΔHDZip/START nucleic acid sequence need not be introduced into the same plant variety. Most preferably, the REV ΔHDZip/START from rice is a sufficient length of substantially contiguous nucleotides of SEQ ID NO: 194 or SEQ ID NO: 196, or a sufficient length of substantially contiguous nucleotides of a REV ΔHDZip/START nucleic acid sequence from nucleic acid sequences encoding REV polypeptide orthologues or paralogues. As mentioned above, a person skilled in the art would be well aware of what would constitute a sufficient length of substantially contiguous nucleotides to perform any of the gene silencing methods defined hereinabove, this may be as little as 20 or fewer substantially contiguous nucleotides in some cases.

The invention also provides genetic constructs and vectors to facilitate introduction and/or expression of the nucleotide sequences useful in the methods according to the invention.

Therefore, there is provided a genetic construct for reduced expression in a plant of an endogenous REV gene comprising one or more control sequences, a REV ΔHDZip/START nucleic acid sequence, and optionally a transcription termination sequence. Preferably, the control sequence is a constitutive promoter.

A preferred construct for reducing expression in a plant of an endogenous REV gene is one comprising an inverted repeat of a REV ΔHDZip/START nucleic acid sequence, preferably capable of forming a hairpin structure, which inverted repeat is under the control of a constitutive promoter.

Constructs useful in the methods according to the present invention may be created using recombinant DNA technology well known to persons skilled in the art. The genetic constructs may be inserted into vectors, which may be commercially available, suitable for transforming into plants and suitable for expression of the gene of interest in the transformed cells. The invention therefore provides use of a genetic construct as defined hereinabove in the methods of the invention.

The sequence of interest is operably linked to one or more control sequences (at least to a promoter) capable of increasing expression in a plant. Advantageously, any type of promoter may be used to drive expression of the nucleic acid sequence.

In one embodiment, the REV ΔHDZip/START nucleic acid sequence is operably linked to a constitutive promoter. Preferably the promoter is a ubiquitous promoter and is expressed predominantly throughout the plant. Preferably, the constitutive promoter is substantially as represented by SEQ ID NO: 218 or SEQ ID NO: 58, further preferably the promoter capable of preferentially expressing the nucleic acid sequence throughout the plant is a GOS2 promoter, most preferably the GOS2 promoter is from rice (SEQ ID NO: 218 or SEQ ID NO: 58). It should be clear that the applicability of the present invention is not restricted to the REV ΔHDZip/START nucleic acid as represented by SEQ ID NO: 194, nor is the applicability of the invention restricted to expression of a REV ΔHDZip/START nucleic acid sequence when driven by a GOS2 promoter. An alternative constitutive promoter that is useful in the methods of the present invention is the high mobility group protein promoter (SEQ ID NO: 293, PRO0170) Examples of other constitutive promoters that may also be used to drive expression of a REV ΔHDZip/START nucleic acid sequence are shown in the definitions section.

Optionally, one or more terminator sequences may also be used in the construct introduced into a plant.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The present invention also encompasses plants including plant parts and plant cells obtainable by the methods according to the present invention having reduced expression of an endogenous REV gene using a REV ΔHDZip/START nucleic acid sequence and which have increased yield relative to control plants.

The invention also provides a method for the production of transgenic plants having increased yield relative to control plants, which transgenic plants have reduced expression of an endogenous REV gene using a REV ΔHDZip/START nucleic acid sequence.

More specifically, the present invention provides a method for the production of transgenic plants having increased yield relative to control plants, which method comprises:

(i) introducing and expressing in a plant, plant part or plant cell a genetic construct comprising one or more control sequences for reducing expression in a plant of an endogenous REV gene using a REV ΔHDZip/START nucleic acid sequence; and

(ii) cultivating the plant, plant part or plant cell under conditions promoting plant growth and development.

Preferably, the construct introduced into a plant is one comprising an inverted repeat (in part or complete) of a REV ΔHDZip/START nucleic acid sequence, preferably capable of forming a hairpin structure, which inverted repeat is under the control of a constitutive promoter.

The nucleic acid may be introduced directly into a plant cell or into the plant itself (including introduction into a tissue, organ or any other part of a plant). According to a preferred feature of the present invention, the construct is introduced into a plant by transformation.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The abovementioned growth characteristics may advantageously be modified in any plant. The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera Helianthus, Tagetes e.g. the species Helianthus annus [sunflower], Tagetes lucida, Tagetes erecta or Tagetes tenuifolia [Marigold], Brassicaceae such as the genera Brassica, Arabadopsis e.g. the species Brassica napus, Brassica rapa ssp. [canola, oilseed rape, turnip rape] or Arabidopsis thaliana; Fabaceae such as the genera Glycine e.g. the species Glycine max, Soja hispida or Soja max [soybean]; Linaceae such as the genera Linum e.g. the species Linum usitatissimum, [flax, linseed]; Poaceae such as the genera Hordeum, Secale, Avena, Sorghum, Oryza, Zea, Triticum e.g. the species Hordeum vulgare [barley]; Secale cereale [rye], Avena sativa, Avena fatua, Avena byzantina, Avena fatua var. sativa, Avena hybrida [oat], Sorghum bicolor [Sorghum, millet], Oryza sativa, Oryza latifolia [rice], Zea mays [corn, maize] Triticum aestivum, Triticum durum, Triticum turgidum, Triticum hybernum, Triticum macha, Triticum sativum or Triticum vulgare [wheat, bread wheat, common wheat]; Solanaceae such as the genera Solanum, Lycopersicon e.g. the species S o l a n u m tuberosum [potato], Lycopersicon esculentum, Lycopersicon lycopersicum, Lycopersicon pyriforme, Solanum integrifolium or Solanum lycopersicum [tomato].

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

The present invention also encompasses use of a REV ΔHDZip/START nucleic acid sequence, for reduction of endogenous REV gene expression in a plant, plant part, or plant cell for increasing plant yield as defined hereinabove.

Items

1. Method for increasing yield in plants relative to control plants, by reducing the expression in a plant of an endogenous REVOLUTA (REV) gene using a REV delta (Δ) homeodomain leucine zipper domain (HDZip) /STeroidogenic Acute Regulatory (STAR) related lipid Transfer domain (START) nucleic acid sequence, and optionally selecting for plants having increased yield.

2. Method according to item 1, wherein said reduced expression is effected by RNA-mediated silencing of gene expression.

3. Method according to item 2, wherein said RNA-mediated silencing is effected by co-suppression.

4. Method according to item 2, wherein said RNA-mediated silencing is effected by use of an antisense REV ΔHDZip/START nucleic acid sequence.

5. Method according to items 1 or 2, wherein said reduced expression is effected using an inverted repeat of a REV ΔHDZip/START nucleic acid sequence, preferably capable of forming a hairpin structure.

6. Method according to any one of items 1 to 5, wherein said REV ΔHDZip/START nucleic acid is from a plant source or artificial source.

7. Method according to any one of items 1 to 6, wherein said REV ΔHDZip/START nucleic acid sequence is from a monocotyledonous plant and used for transformation of monocotyledonous plants, or is from a dicotyledonous plant and used for transformation of dicotyledonous plants.

8. Method according to any one of items 1 to 7, wherein said REV ΔHDZip/START nucleic acid sequence is from a plant of the family Poaceae and used for transformation of plants of the family Poaceae, more preferably wherein a rice REV ΔHDZip/START nucleic acid sequence is used to transform rice plants.

9. Method according to any one of items 1 to 8, wherein said REV ΔHDZip/START nucleic acid sequence comprises a sufficient length of substantially contiguous nucleotides of SEQ ID NO: 194 or SEQ ID NO: 196.

10. Method according to any one of items 1 to 9, wherein said REV ΔHDZip/START nucleic acid sequence comprising a sufficient length of substantially contiguous nucleotides is from a nucleic acid sequence encoding a REV polypeptide orthologue or a paralogue.

11. Method according to item 10, wherein said nucleic acid sequence encoding a REV polypeptide orthologue or paralogue is represented by SEQ ID NO: 200, SEQ ID NO: 202, SEQ ID NO: 204, SEQ ID NO: 206, SEQ ID NO: 208, SEQ ID NO: 210, SEQ ID NO: 212, SEQ ID NO: 214, SEQ ID NO: 216, SEQ ID NO: 224, SEQ ID NO: 226, SEQ ID NO: 228, and SEQ ID NO: 230.

12. Method according to any one of items 1 to 11, wherein said increased yield is increased seed yield and/or increased biomass.

13. Method according to item 12, wherein said increased seed yield is selected from one or more of the following: (i) increased seed weight; (ii) increased number of filled seeds; (iii) increased seed fill rate; (iv) increased harvest index; and (v) increased individual seed length.

14. Method according to item 12, wherein said increased biomass is root biomass.

15. Plant, plant part or plant cell thereof obtainable by a method according to any one of items 1 to 14, wherein said plant, plant part or plant cell thereof has reduced expression of an endogenous REV gene using a REV ΔHDZip/START nucleic acid sequence, and having increased yield relative to control plants.

16. Construct for reducing expression in a plant of an endogenous REV gene comprising one or more control sequences, a REV ΔHDZip/START nucleic acid sequence, and optionally a transcription termination sequence.

17. Construct according to item 16, wherein said REV ΔHDZip/START nucleic acid sequence is an inverted repeat, preferably capable of forming a hairpin structure, which inverted repeat is under the control of a constitutive promoter.

18. Construct according to items 16, wherein said control sequence is a constitutive promoter.

19. Construct according to items 17 or 18, wherein said constitutive promoter is a GOS2 promoter.

20. Construct according to item 19, wherein said GOS2 promoter is substantially as represented by SEQ ID NO: 58.

21. Plant, plant part or plant cell transformed with a construct according to any one of items 16 to 20.

22. Method for the production of transgenic plants having increased yield relative to control plants, which method comprises:

(i) introducing and expressing in a plant, plant part or plant cell a genetic construct comprising one or more control sequences for reducing expression in a plant of an endogenous REV gene using a REV ΔHDZip/START nucleic acid sequence; and
(ii) cultivating the plant, plant part or plant cell under conditions promoting plant growth and development.

23. Transgenic plant having increased yield relative to control plants, characterised in that said plant has reduced expression of an endogenous REV gene using a REV ΔHDZip/START nucleic acid sequence.

24. Transgenic plant according to item 15, 21 or 23, wherein said plant is a monocotyledonous plant, such as sugarcane or wherein the plant is a cereal, such as rice, maize, wheat, barley, triticale, millet, rye oats or sorghum.

25. Harvestable parts of a transgenic plant according to any one of items 15, 21, 23 or 24.

26. Harvestable parts according to item 25, wherein said harvestable parts are seeds.

27. Products derived from a plant according to any one of items 15, 21, 23 or 24 and/or from harvestable parts of a plant according to items 25 or 26.

28. Use of a REV ΔHDZip/START nucleic acid sequence for reducing the expression in plants of an endogenous REV gene to increase yield in said plants relative to control plants.

29. Use according to item 28, wherein said increased yield is increased seed yield and/or increased biomass.

30. Use according to item 29, wherein said increased seed yield is selected from one or more of: selected from one or more of the following: (i) increased seed weight; (ii) increased number of filled seeds; (iii) increased seed fill rate; (iv) increased harvest index; and (v) increased individual seed length.

31. Use according to item 29, wherein said increased biomass is increased root biomass.

**Description of figures**

REV ΔHDZip/START

**Figure 1** shows a phylogram of class III HDZip polypeptides. There are two monocot REV polypeptides *(Oryza sativa)* that cluster up with a single dicot REV polypeptide *(Arabidopsis thaliana).* The circle indicates the clade of REV polypeptides of which the REV nucleic acid sequences ΔHDZip/START may be useful in performing the methods of the invention. After Floyd et al. (2006) Genetics 173(1): 373-88

**Figure 2**. Neighbour-joining tree output after a multiple sequence alignment of all class III HDZip polypeptides from *Arabidopsis thaliana* (5 in total) and *Oryza sativa* (5 in total), including examples of REV polypeptide orthologues and paralogues (see Example 2), using CLUSTAL W (1.83) (at GenomeNet service at the Kyoto University Bioinformatics Center), and default values (Blosum 62 as weight matrix, gap open penalty of 10; gap extension penalty of 0.05). The polypeptides of the REV branch are indicated by the curly bracket, and are separated from the other four class III HDZip polypeptides by the bold line. The circle indicates the REV branching out point.

**Figure 3** is a schematic representation of a full-length REV polypeptide. REV polypeptides comprise from N-terminus to C-terminus: (i) a homeodomain (HD) domain, for DNA binding; (ii) a leucine zipper (Zip), for protein-protein interaction; (iii) a START domain for lipid/sterol binding (comprising a miRNA complementary binding site, mir165/166), and (iv) a C-terminal region (CTR), of undefined function. For example, in one REV polypeptide from *Oryza sativa* as represented by SEQ ID NO: 199, the HD spans amino acids 27 to 87, the leucine zipper amino acids 91 to 127, the START domain amino acids 166 to 376 and the CTR amino acids 377 to 840.

**Figure 4** is a multiple sequence alignment of full length REV polypeptides of which the REV ΔHDZip/START nucleic acid sequences are useful in performing the methods according to the invention, using CLUSTAL W (1.83) (at GenomeNet service at the Kyoto University Bioinformatics Center), and default values (Blosum 62 as weight matrix, gap open penalty of 10; gap extension penalty of 0.05). The homeodomain, the leucine zipper, the START domain and the CTR are heavily boxed.

**Figure 5** is a multiple sequence alignment of the CTR of REV polypeptides (both full length and partial polypeptides, as listed in Example 2), of which the REV ΔHDZip/START nucleic acid sequences are useful in performing the methods according to the invention, using CLUSTAL W (1.83) (at GenomeNet service at the Kyoto University Bioinformatics Center), and default values (Blosum 62 as weight matrix, gap open penalty of 10; gap extension penalty of 0.05

**Figure 6** represents the binary vectors p0443 and p0448 for reduction of an endogenous REV gene expression in *Oryza sativa,* using respectively the REV ΔHDZip/START nucleic acid sequence as represented by SEQ ID NO: 194 (encoding a partial CTR from a REV polypeptide) and the REV nucleic acid sequence as represented by SEQ ID NO: 198 (encoding the entire REV polypeptide). The nucleic acid sequences are cloned as inverted repeats separated by a non-coding region (here a partial matrix attachment region (MAR) from tobacco) with the aim of obtaining an mRNA with a hairpin conformation. A constitutive promoter (PRO0129 controls the expression of both nucleic acid sequences SEQ ID NO: 194 and SEQ ID NO: 198 in the two different plasmids.

**Examples**

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Plant transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

<u>REV AHDZip/START</u>

***Example* 2: *Identification of sequences related to the nucleic acid sequence used in the methods of the invention***

Sequences (full length cDNA, ESTs or genomic) related to the nucleic acid sequence used in the methods of the present invention were identified amongst those maintained in the Entrez Nucleotides database at the National Center for Biotechnology Information using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. The polypeptide encoded by the nucleic acid sequence of the present invention was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search
Table Z provides a list of nucleic acid sequences related to the nucleic acid sequence used in the methods of the present invention.

Table Z: Nucleic acid sequences related to the nucleic acid sequence (SEQ ID NO: 194) used in the methods of the present invention, and the corresponding deduced polypeptides.

| **Name** | **source organism** | **Nucleic acid SEQ ID NO:** | **Polypeptide SEQ ID NO:** | **database accession number** |
|---|---|---|---|---|
| Orysa_REV partial CTR | *Oryza sativa* | 194 | 195 | Part of AK102830 (Os10g33960) |
| Orysa_REV CTR | *Oryza sativa* | 196 | 197 | Part of AK102830 (Os10g33960) |
| Orysa_Rev | *Oryza sativa* | 198 | 199 | AK102830 (Os10g33960) |
| Orysa_HOX10 | *Oryza sativa* | 200 | 201 | AY425991.1 |
| Arath_REV | *Arabidopsis thaliana* | 202 | 203 | AF188994 |
| Zeama_HDIII RLD1 (rolled leaf1 ) | *Zea mays* | 204 | 205 | AY501430.1 |
| Poptr_HDIII | *Populus trichocarpa* | 206 | 207 | AY919617 |
| Medtr_HDIII | *Medicago trunculata* | 208 | 209 | Spliced from AC138171.17 |
| Sacof_HDIII Partial | *Saccharum officinarum* | 210 | 211 | contig of CA125167.1 CA217027.1 CA241276.1 CA124509.1 |
| Triae_HDIII Partial | *Triticum aestivum* | 212 | 213 | contig of CD905903 BM 135681.1 BQ578798.1 CJ565259.1 |
| Horvu_HDIII Partial | *Hordeum vulgare* | 214 | 215 | compiled from BU996988.1 BJ452342.1 BJ459891.1 |
| Phypr_HDIII Partial | *Phyllostachys praecox* | 216 | 217 | DQ013803 |
| Orysa_REV | *Oryza sativa* | 223 | | Variant of SEQ ID NO: 196 |
| Brara Revoluta | *Brassica rapa* | 224 | 225 | AC 189324.1 |
| Ginbi Revoluta | *Ginkgo biloba* | 226 | 227 | DQ385525 |
| Gosba Revoluta | *Gossypium barbadense* | 228 | 229 | AY966446.1 |
| Lyces Revoluta | *Lycopersicon esculentum* | 230 | 231 | BT013577 |

*Example 3: Determination of global similarity and identity between the CTR of REV polypeptides.*

Global percentages of similarity and identity between the CTR of REV polypeptides were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table AA for the global similarity and identity between the CTR of REV polypeptides. Percentage identity is given above the diagonal and percentage similarity is given below the diagonal. Percentage identity between the CTR of REV polypeptide paralogues and orthologues ranges between 30 and 70%, reflecting the lower sequence identity conservation between them outside of the HDZip and START domains.

Table AA: MatGAT results for global similarity and identity between the CTR of REV polypeptide orthologues and paralogues.

| Global similarity and identity over the CTR of REV polypeptide orthologues and paralogues | 1 | 2 | 3 | 4 | 5 | 6 | 7 | 8 | 9 | 10 |
|---|---|---|---|---|---|---|---|---|---|---|
| 1. CTR_Horvu_HDIII | | 61.1 | 57.2 | 81.1 | 88.2 | 89.7 | 60 | 79.6 | 96.5 | 79.8 |
| 2. CTR_Arath_REV | 76.9 | | 70 | 61.8 | 63 | 63.2 | 73.3 | 62.5 | 62.9 | 63.4 |
| 3. CTR_Medtr_REV | 73.2 | 86.5 | | 59.2 | 60.3 | 60.8 | 70.2 | 59.4 | 58.6 | 60.3 |
| 4.CTR_Orysa_HOX10 | 89 | 80.7 | 76.6 | | 83.9 | 84.8 | 59.8 | 88.2 | 83 | 89.7 |
| 5. CTR Orysa_REV | 93.5 | 79.6 | 76.2 | 92.5 | | 92.7 | 61.9 | 83.2 | 90.7 | 83.9 |
| 6. CTR_Phypr_HDIII | 93.3 | 79.8 | 76.2 | 92.7 | 96.3 | | 61.9 | 84.5 | 91.8 | 84.2 |
| 7. CTR_Poptr_HDIII | 75.1 | 86.4 | 82.8 | 76.1 | 76.1 | 76.5 | | 60.4 | 61.6 | 61.3 |
| 8. CTR_Sacof_HDIII | 88.6 | 80.3 | 75.9 | 94.2 | 91.8 | 91.8 | 77.4 | | 81.9 | 94.8 |
| 9. CTR_Triae_HDIII | 97.4 | 79 | 74.9 | 90.8 | 95.7 | 95.1 | 76.3 | 90.5 | | 81.8 |
| 10. CTR-Zeama-HDIII-LRD1 | 88.8 | 81.3 | 77.8 | 95.7 | 92.5 | 92.3 | 77.8 | 97 | 90.6 | |

All REV polypeptides comprise a CTR having, in increasing order of preference, at least 50%, 55%, 60%, 65%, 70%, 75%, 80%, 85%, 90%, 95% or 98% sequence identity to the CTR of a REV polypeptide as represented by SEQ ID NO: 197.

## *Example 4: Gene Cloning*

DNA manipulation: unless otherwise stated, recombinant DNA techniques are performed according to standard protocols described in (Sambrook (2001 ) Molecular Cloning: a laboratory manual, 3rd Edition, Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Oryza sativa* Orysa_REV full-length gene SEQ ID NO: 198 was amplified by PCR using as template a custom-made *Oryza sativa* cDNA library (Invitrogen, Paisley, UK). After reverse transcription of RNA extracted from seedlings, the cDNAs were cloned into pCMV Sport 6.0. After plasmid extraction, 200 ng of template was used in a 50 μl PCR mix. Primers prm01983 (SEQ ID NO: 221; sense, start codon in bold, AttB1 site in italic:
*5'-ggggacaagtttgtacaaaaaagcaggcttaaacaatggcggcggcggtgg-3')*

and prm01984 (SEQ ID NO: 222; reverse, complementary, AttB2 site in italic:
*5'-ggggaccactttgtacaagaaagctgggtggattttgggtcacacgaaggacca* -3'),
which include the AttB sites for Gateway recombination, were used for PCR amplification. PCR was performed using Hifi Taq DNA polymerase in standard conditions. The amplified PCR fragment was purified also using standard methods. The first step of the Gateway procedure, the BP reaction, was then performed, during which the PCR fragment recombines with the pDONR201 plasmid to produce, according to the Gateway terminology, an "entry clone", p04562. Plasmid pDONR201 was purchased from Invitrogen, as part of the Gateway@ technology.

The *Oryza sativa* partial CTR (REV AHDZip/START) of the Orysa_REV gene SEQ ID NO: 194 was amplified by PCR as above, with primers prm03263 (SEQ ID NO: 219:
5'ggggacaagtttgtacaaaaaagcaggcttgtgctaaggcatccatgctac3')
and prm03264 (SEQ ID NO: 220:
5'ggggaccactttgtacaagaaagctgggtgcaccttccatgctacagcttg3').
After cloning, the resulting entry clone number was p04436.

*Example* 5: *Vector Construction*

The entry clones p04562 and p04436 were subsequently used in an LR reaction with p01519, a destination vector used for *Oryza sativa* transformation for the hairpin construct. This vector contain as functional elements within the T-DNA borders: a plant selectable marker; a screenable marker expression cassette; and two Gateway cassettes cloned as inverted repeats and separated by a MAR (fragment of around 300 bp of a *Nicotiana tabacum* matrix attachment region), intended for LR *in vivo* recombination such that the sequence of interest from the entry clone is integrated in both sense and antisense orientations to form the hairpin secondary structure. A rice GOS2 promoter (SEQ ID NO: 58) for constitutive expression of the genetic construct (PR00129) was located upstream of these Gateway cassettes.

After the LR recombination step, the resulting expression vectors, p0443 (with the partial CTR of Orysa_REV; SEQ ID NO: 194) and p0448 (with the full length Orysa_REV; comprised in SEQ ID NO: 198) (see Figure 6) were separately transformed into *Agrobacterium* strain LBA4044, and subsequently separately to *Oryza sativa* plants. Transformed rice plants were allowed to grow and were then examined for the parameters described in Example 6.

***Example* 6: *Evaluation procedure***

**6.1 Evaluation setup**

Approximately 15 to 20 independent TO rice transformants were generated. The primary transformants were transferred from a tissue culture chamber to a greenhouse for growing and harvest of T1 seed. Five events for the hairpin construct comprising the full length Orysa_REV and six events for the hairpin construct comprising the partial CTR of the Orysa_REV, of which the T1 progeny segregated 3:1 for presence/absence of the transgene, were retained. For each of these events, approximately 10 T1 seedlings containing the transgene (hetero- and homozygotes) and approximately 10 T1 seedlings lacking the transgene (nullizygotes) were selected by monitoring visual marker expression. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes (control plants) were grown side-by-side at random positions.

Five T1 events were further evaluated (if positive results were obtained in the first evaluation) in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

6.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

***Example* 7: *Evaluation results***

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass.

The plant parts below ground (in this case essentially the roots) were determined by growing the plants in specially designed pots with transparent bottoms to allow visualization of the roots. A digital camera recorded images through the bottom of the pot during plant growth.

Root features such as total projected area (which can be correlated to total root volume), average diameter and length of roots above a certain thickness threshold (length of thick roots, or thick root length) were deduced from the picture using of appropriate software.

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand kernel weight (TKW) is extrapolated from the number of filled seeds counted and their total weight. Individual seed parameters (including width, length, area, weight) were measured using a custom-made device consisting of two main components, a weighing and imaging device, coupled to software for image analysis. The harvest index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

**7.1 Measurement of yield-related parameters for transformants with reduced expression of an endogenous REV gene using a REV AHDZip/START nucleic acid sequence as represented by SEQ ID NO: 194**

As presented in Tables BB to EE, the seed yield, the number of filled seeds, the seed fill rate and the harvest index are increased in the transgenic plants with reduced expression of an endogenous REV gene using a REV AHDZip/START nucleic acid sequence compared to control plants. Results from the T1 and the T2 generations are shown.
Table BB shows the number of transgenic events with an increase in total seed yield (total seed weight), the percentage of this increase, as well as the statistical relevance of this increase according to the F-test.

Table BB: Number of transgenic events with an increase in seed yield, the percentage of the increase, and P value of the F-test in T1 and T2 generation of transgenic rice with reduced expression of an endogenous REV gene using a REV AHDZip/START nucleic acid sequence.

| **Seed weight** | | | |
|---|---|---|---|
| | **Number of events showing an increase** | **% Difference** | **P value of F test** |
| T1 generation | 4 out of 6 | 16 | 0.058 |
| T2 generation | 3 out of 4 | 4 | 0.0133 |

Table CC shows the number of transgenic events with an increase in the number of filled seeds, the percentage of this increase, as well as the statistical relevance of this increase according to the F-test.

Table CC: Number of transgenic events with an increase in number of filled seeds, the percentage of the increase, and P value of the F-test in T1 and T2 generation of transgenic rice with reduced expression of an endogenous REV gene using a REV AHDZip/START nucleic acid sequence.

| **Number of filled seeds** | | | |
|---|---|---|---|
| | **Number of events showing an increase** | **% Difference** | **P value of F test** |
| T1 generation | 4 out of 6 | 14 | 0.0884 |
| T2 generation | 3 out of 4 | 4 | 0.0156 |

Table DD shows the number of transgenic events with an increase in the seed fill rate, the percentage of this increase, as well as the statistical relevance of this increase according to the F-test.

**Table DD:** Number of transgenic events with an increase in seed fill rate, the percentage of the increase, and P value of the F-test in T1 and T2 generation of transgenic rice with reduced expression of an endogenous REV gene using a REV AHDZip/START nucleic acid sequence.

| **Seed fill rate** | | | |
|---|---|---|---|
| | **Number of events showing an increase** | **% Difference** | **P value of F test** |
| T1 generation | 5 out of 6 | 19 | 0.0002 |
| T2 generation | 4 out of 4 | 22 | <0.0001 |

Table EE shows the number of transgenic events with an increase in the harvest index, the percentage of this increase, as well as the statistical relevance of this increase according to the F-test.

Table EE Number of transgenic events with an increase in harvest index, the percentage of the increase, and P value of the F-test in T1 and T2 generation of transgenic rice with reduced expression of an endogenous REV gene using a REV AHDZip/START nucleic acid sequence.

| **Harvest index** | | | |
|---|---|---|---|
| | **Number of events showing an increase** | **% Difference** | **P value of F test** |
| T1 generation | 4 out of 6 | 14 | 0.0284 |
| T2 generation | 3 out of 4 | 9 | 0.0187 |

Two additional parameters were measured for only one evaluation:

1) the average individual seed length
2) the average root thickness

As shown on Table FF, both the average individual seed length and the average root thickness were significantly increased in transgenic rice with reduced expression of an endogenous REV gene using a REV AHDZip/START nucleic acid sequence compared to control plants.

Table FF: Number of transgenic events with an increase in harvest index and P value of the F-test in a single generation of transgenic rice with reduced expression of an endogenous REV gene using a REV AHDZip/START nucleic acid sequence.

| | **Number of events showing an increase** | **P value of F test** |
|---|---|---|
| **Average individual seed length (T2 seeds)** | 5 out of 6 | 0.009 |
| **Average root thickness (T2 plants)** | 4 out of 4 | <0.0001 |

**7.2 Measurement of yield-related parameters for transformants with reduced expression of an endogenous REV polypeptide using a nucleic acid encoding the full length Orysa_REV polypeptide, as represented by SEQ ID NO: 198:**

The same evaluation procedure as described hereinabove was performed for transgenic rice having reduced expression of an endogenous REV polypeptide using a nucleic acid sequence encoding the full length Orysa_REV polypeptide. All of the parameters measured were strongly and significantly negative, as shown in Table GG.

**Table GG**: Number of transgenic events with a DECREASE in aboveground biomass, total seed yield, number of filled seeds, number of flowers per panicle, harvest index, number of primary panicles and plant height, the percentage of the increase, and P value of the F-test in T1 generation of transgenic rice with reduced expression of an endogenous REV gene using a nucleic acid sequence encoding the full length Orysa_REV polypeptide.

| **Parameter** | **Number of events showing a decrease** | **% Difference** | **P value of F test** |
|---|---|---|---|
| Aboveground biomass | 5 out of 5 | -40 | <0.0001 |
| Total seed yield | 5 out of 5 | -65 | <0.0001 |
| Number of filled seeds | 5 out of 5 | -64 | <0.0001 |
| Number of flowers per panicle | 5 out of 5 | -39 | <0.0001 |
| Harvest index | 5 out of 5 | -53 | <0.0001 |
| Number of primary panicles | 5 out of 5 | -39 | <0.0001 |
| Plant height | 5 out of 5 | -13 | 0.0006 |

By including conserved regions in the hairpin construct (such as the HDZip and START domains) for reducing endogenous REV gene expression, the expression of other class III HDZip genes may also be reduced.

## Claims

1. Method for increasing yield in plants relative to control plants, by reducing the expression in a plant of an endogenous REVOLUTA (REV) gene using a REV delta (A) homeodomain leucine zipper domain (HDZip) /STeroidogenic Acute Regulatory (STAR) related lipid Transfer domain (START) nucleic acid sequence, and optionally selecting for plants having increased yield.

2. Method according to claim 1, wherein said reduced expression is effected by RNA-mediated silencing of gene expression.

3. Method according to claim 2, wherein said RNA-mediated silencing is effected by co-suppression.

4. Method according to claim 2, wherein said RNA-mediated silencing is effected by use of an antisense REV AHDZip/START nucleic acid sequence.

5. Method according to claims 1 or 2, wherein said reduced expression is effected using an inverted repeat of a REV 4HDZip/START nucleic acid sequence, preferably capable of forming a hairpin structure.

6. Method according to any one of claims 1 to 5, wherein said REV AHDZip/START nucleic acid sequence comprises a sufficient length of substantially contiguous nucleotides of SEQ ID NO: 194 or SEQ ID NO: 196.

7. Method according to any one of claims 1 to 6, wherein said increased yield is increased seed yield and/or increased biomass.

8. Plant, plant part or plant cell thereof obtainable by a method according to any one of claims 1 to 6, wherein said plant, plant part or plant cell thereof has reduced expression of an endogenous REV gene using a REV AHDZip/START nucleic acid sequence, and having increased yield relative to control plants.

9. Construct for reducing expression in a plant of an endogenous REV gene comprising one or more control sequences, a REV AHDZip/START nucleic acid sequence, and optionally a transcription termination sequence.

10. Construct according to claim 9, wherein said REV AHDZip/START nucleic acid sequence is an inverted repeat, preferably capable of forming a hairpin structure, which inverted repeat is under the control of a constitutive promoter.

11. Construct according to claims 9, wherein said control sequence is a constitutive promoter, preferably said constitutive promoter is a GOS2 promoter, more preferably said constitutive promoter is a GOS2 promoter substantially as represented by SEQ ID NO: 58.

12. Plant, plant part or plant cell transformed with a construct according to any one of claims 9 to 11.

13. Method for the production of transgenic plants having increased yield relative to control plants, which method comprises:

(i) introducing and expressing in a plant, plant part or plant cell a genetic construct comprising one or more control sequences for reducing expression in a plant of an endogenous REV gene using a REV AHDZip/START nucleic acid sequence; and
(ii) cultivating the plant, plant part or plant cell under conditions promoting plant growth and development.

**14.** Transgenic plant having increased yield relative to control plants, **characterised in that** said plant has reduced expression of an endogenous REV gene using a REV AHDZip/START nucleic acid sequence.

**15.** Harvestable parts of a transgenic plant according to any one of claims 8, 12 or 14, preferably said harvestable parts are seeds.

**16.** Products derived from a plant according to any one of claims 8, 12 or 14 and/or from harvestable parts of a plant according to claim 15.

**17.** Use of a REV AHDZip/START nucleic acid sequence for reducing the expression in plants of an endogenous REV gene to increase yield in said plants relative to control plants, preferably said increased yield is increased seed yield and/or increased biomass.

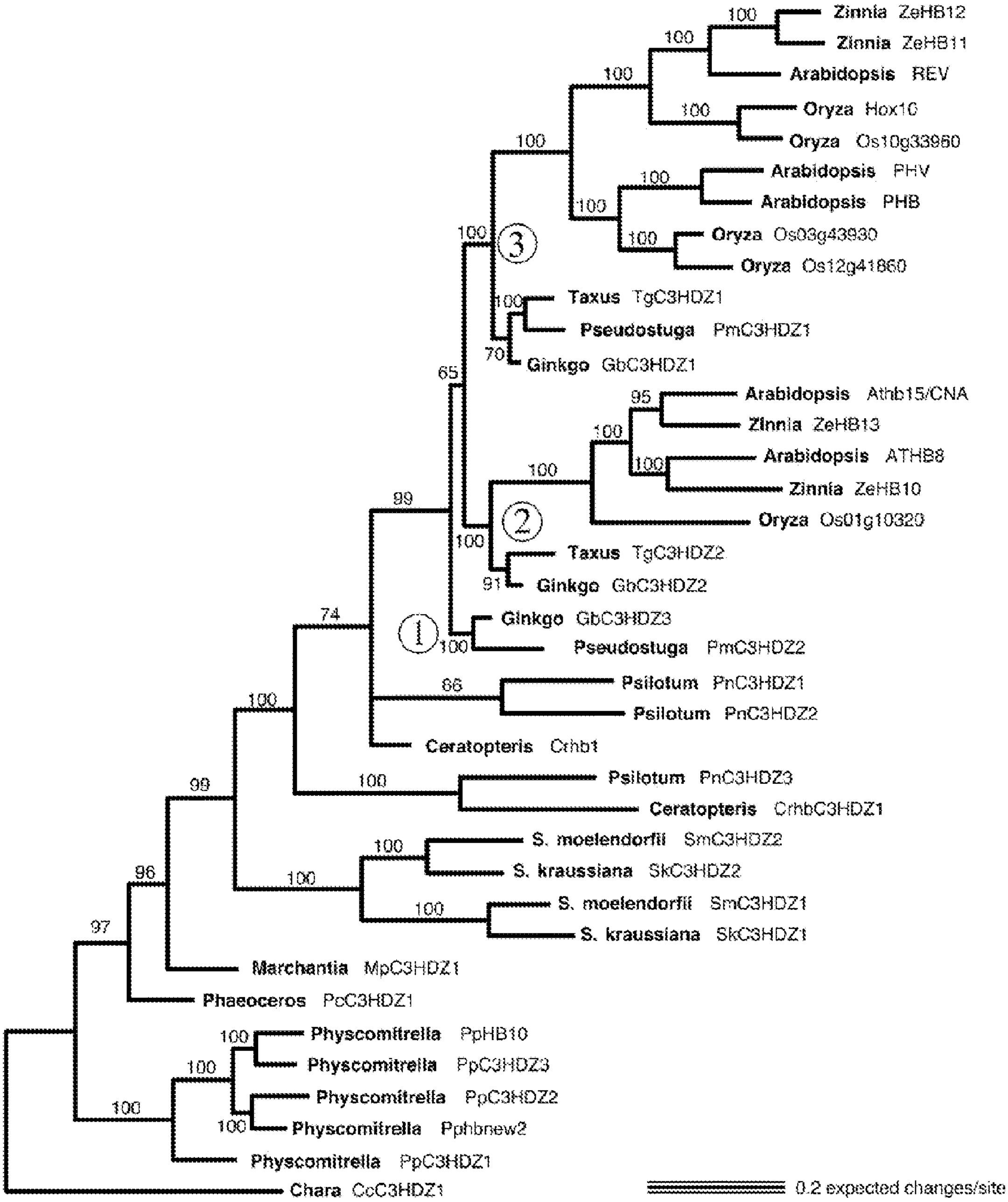


After Floyd *et al.* (2006) Genetics 173(1): 373-88

**FIGURE 1**

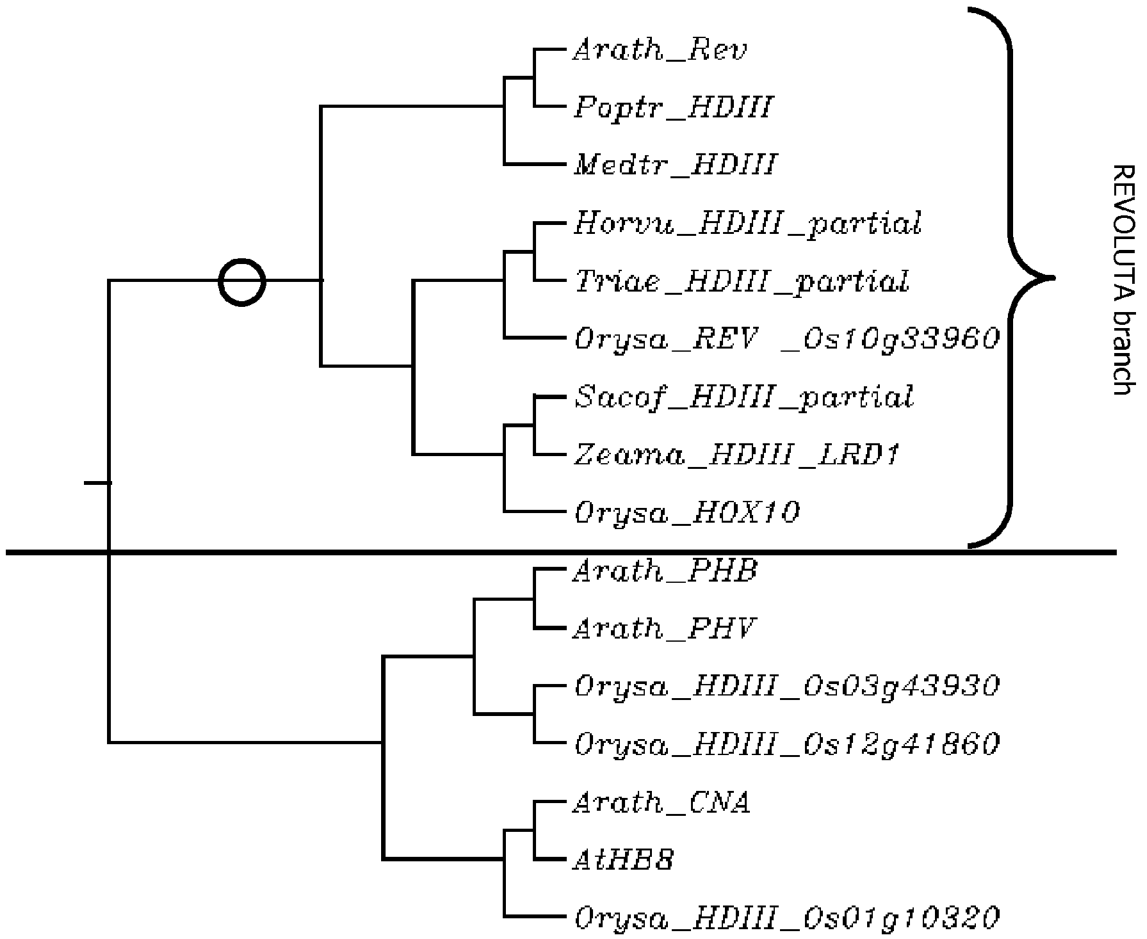


FIGURE 2

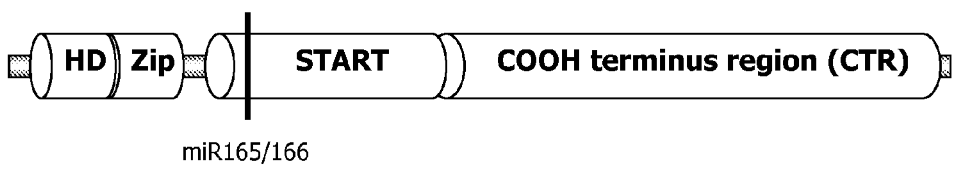


FIGURE 3

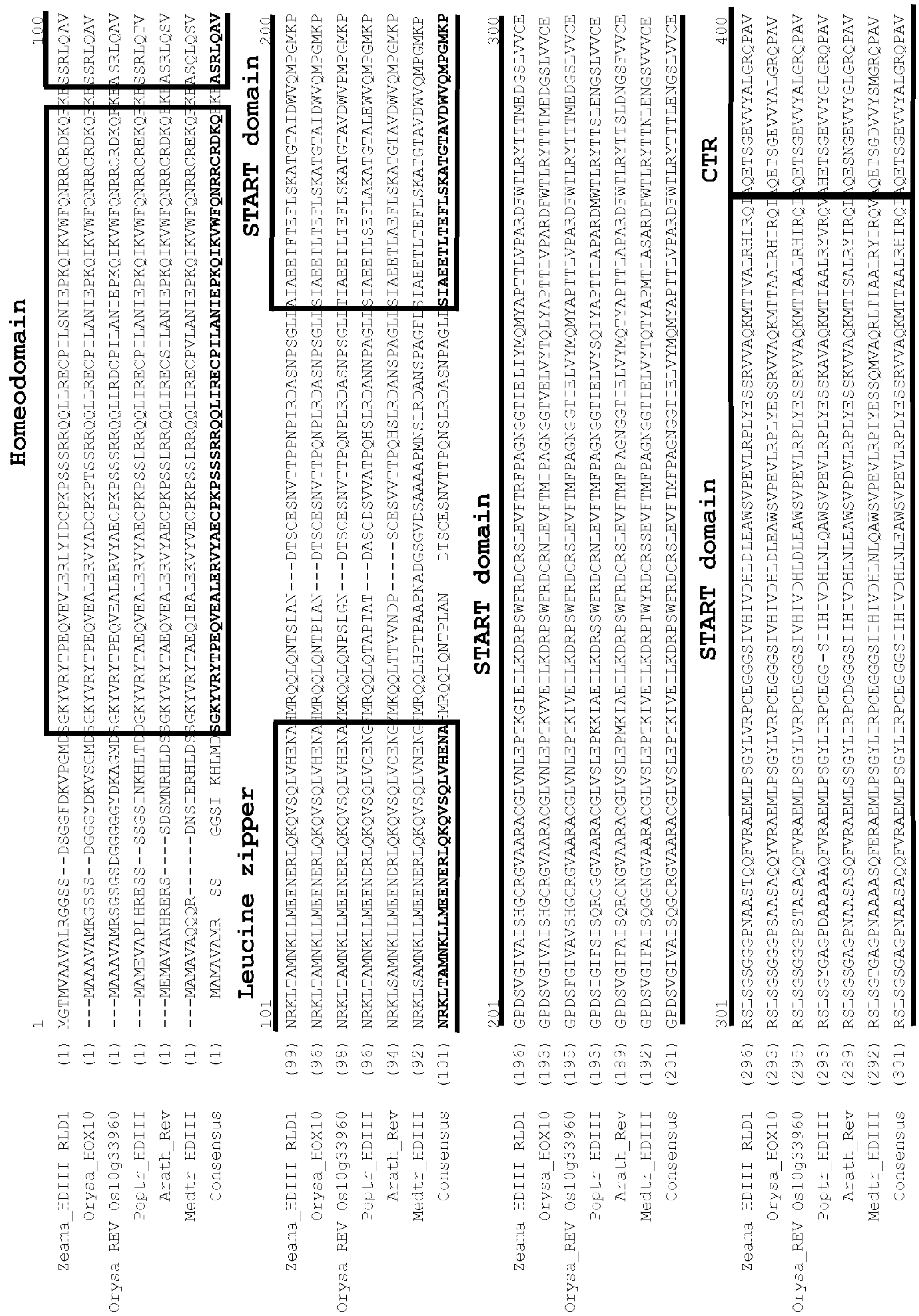
Homeodomain
Leucine zipper
START domain
START domain
START domain
CTR
Zeama_HDIII RLD1
Orysa_HOX10
Orysa_REV Os10g33960
Poptr_HDIII
Arath_Rev
Medtr_HDIII
Consensus

FIGURE 4

```
                                401                                                                                                   500
         Zeama_HDIII RLD1  (396) LRTFSQRLSRGFNDAISGFNDDGWSVMGGDGIEDVVIACNSTKKIRNTSNAGIIFGAPGGIICAKASMLLQSVPPAVLVRFLREHRSEWADYNIDAYLAS
             Orysa_HOX10  (393) LRTFSQRLSRGFNDAISGFNDDGWSIMGGDGVEDVVIACNSTKKIRSNSNAGIAFGAPGGIICAKASMLLQSVPPAVLVRFLREHRSEWADYNIDAYLAS
   Orysa_REV Os10g33960  (395) LRTFSQRLSRGFNDAISGFNDDGWSVMGGDGIEDVIIACN-AKKVRNTSTSANAFVTPGGVICAKASMLLQSVPPAVLVRFLREHRSEWADYNFDAYSAS
             Poptr_HDIII  (392) LRTFNQRLSRGFNDAINGFNDDGWSLMNADGAEDVIIAVNSTKNLIGANNSAHSLSFLGGILCAKASMLLQNVHPAVLVCFLREHHAEWADFSVDAYSAA
               Arath_Rev  (389) LRTFSQRLSRGFNDAVNGFGDDGWSTMHCDGAEDIIVAINSTKHINNTSN---SLSFLGGVLCAKASMLLQNVPPAVLIRFLREHRSEWADFNVDAYSAA
             Mectr_HDIII  (392) LRTFSQRLSRGFNDAVNGFNDNGWSVLNCDGAEGVTISVNSIKNLSGTSNPASSLSLLGGIVCAKASMLLQNTTPAVLVRFLREHRSEWADFSVDAFSAA
               Consensus  (401) LRTFSQRLSRGFNDAISGFNDDGWSVMGGDGAEDVIIACNSTKKLRNTSNAA SLS PGGII**CAKASML**LQSVPPAVLVRFLREHRSEWADFNVDAYSAA
                                501                                                                                                   600
         Zeama_HDIII RLD1  (496) SLKISACSLPGLRPMRFSEGQMIMPLAHTVENEEILEVVRLEGQPLTHDEALLSRDIHLLQ--------LCTGIDEKSVGSSFQLVFAPIDEHFPDDAPL
             Orysa_HOX10  (493) TLKISACSLTGLRPMRFSGSQIIIPLAHTVENEEILEVVRLEGQPLTHDEALLSRDIHLLQ--------LCTGIDEKSVGSSFQLVFAPIDD-FPDETPL
   Orysa_REV Os10g33960  (494) SLKISSCSLPGLRPMRFSGSQIIMPLAHTVENEEILEVVRLEGQALTHDDGLMSRDIHLLQ--------LCTGIDEKSMGSCFQLVSAPIDELFPDDAPL
             Poptr_HDIII  (492) LWKAGSYAYPGMRPMRFTGSQIIMPLGHTIEQEDLLEVIRLEGHSFAQEDAFVSQDIHLLQ--------ICSGIDENAVGACSELVFAPIDETFPDDAPL
               Arath_Rev  (486) TLKAGSFAYPGMRPTRFTGSQIIMPLGHTIEHEEMLEVVRLEGHSLAQEDAFMSRDVHLLQ--------ICTGIDENAVGACSELIFAPINEMFPDDAPL
             Mectr_HDIII  (492) SLKAGSYGYPGMRSTKFTGNQAIMPLGHTIEHEEMLEIIRLEG--LAQDDSFVSRDVHLLQVLPLTLVMLCTGIDENAVGACSELIFAPIDDMFPEDAPL
               Consensus  (501) SLKISSCSYPGLRPMRFSGSQIIMPLAHTIENEEILEVVRLEGQSLTQDDALLSRDIHLLQ         LCTGIDENAVGACSQLVFAPIDEMFPDDAPL
                                601                                                                                                   700
         Zeama_HDIII RLD1  (588) ISSGFRVIPLDVKT----DGVSSG-RTLDLASSLDVGSAAPQASGDASPDDCSLRSVLTIAFQFPYEMHLQDSVAAMARQYVRSVISAVQRVSMAISPSQ
             Orysa_HOX10  (584) ISSGFRVIPLDMKT----DGASSG-RTLDLASSLEVGSATAQASGDASADDCNLRSVLTIAFQFPYELHLQDSVAAMARQYVRSIVSAVQRVSMAISPSQ
   Orysa_REV Os10g33960  (586) ISSGFRVIPLDMKT----DGTPAG-RTLDLASSLEVG-STAQPTGDASMDDCNLRSVLTIAFQFPYEMHLQDSVATMARQYVRSIVSSVQRVSMAISPSR
             Poptr_HDIII  (584) LPSGFRIISLESKAKDTQEVLTTN-CTLDLTSSLEAGLAINHLAVDGSSCHSL-RSVLTIAFQFPFESNLQDNVATMARQYVRSVISSVQRVAMAISPSG
               Arath_Rev  (578) VPSGFRVIPVDAKTGDVQDLLTANHRTLDLTSSLEVGPSPENASGNSFSSSSS-RCILTIAFQFPFENNLQENVAGMACQYVRSVISSVQRVAMAISPSG
             Mectr_HDIII  (590) VPSGFRIVLLNSQPGDTKNTTTAN-RTLDLTSGLEVSPATAHANGDASCPNN--RCVLTVAFQFPFESGLQDNVAAMARQYVRRVVSAVQAVAMAISPSS
               Consensus  (601) ISSGFRVIPLDSKT D  DGLTAN RTLDLTSSLEVG ATAQASGDASSDDC LRSVLTIAFQFPFEMHLQDSVAAMARQYVRSVISAVQRVAMAISPS
                                701                                                                                                   800
         Zeama_HDIII RLD1  (683) SGLNAGHRMLSGFPEAATLARWVCQSY---------HYHLGMELLNQSDGAG-EALLKMLWHHPDAVLCCSFKEKPMFTFANKAGLDMLETSLVALQDLT
             Orysa_HOX10  (679) TGLNAGQRIISGFPEAATLARWVCQSY---------HYHLGVELLSQSDGDA-EQLLKMLWHYQDAILCCSFKEKPVLTFANKAGLDMLETSLVALQDLT
   Orysa_REV Os10g33960  (680) SGLNAGQKIISGFPEAPTLARWICQSY---------QFHLGVELLRQADDAG-EALLKMLWDYEDAILCCSFKEKPVFTFANEMGLNMLETSLVALQDLS
             Poptr_HDIII  (682) LSPVLGPKLSAGSPEALTLAHWICQSHRQVLLKFSSCYHLGAELLRSDSVGG-DSVLKILWHHPDAILCCSLKSLPVFTFANQAGLDMLETTLVALQDIT
               Arath_Rev  (677) ISPSLGSKLSPGSPEAVTLAQWISQSY---------SHHLGSELLTIDSLGSDDSVLKLLWDHQDAILCCSLKPQPVFMFANQAGLDMLETTLVALQDIT
             Mectr_HDIII  (687) VNTSGGAKLSPGTPEALTLAQWICQSY---------SHHLGAQLLRSDSLIG-DMLLKILWHHPDAILCCSLKQVPVFTFANQAGLDMLETTLVALQDIT
               Consensus  (701) SGLNAG KLSSGFPEALTLARWICQSY         YHLGVELLRQDSLAG DALLKMLWHHPDAILCCSLKEKPVFTFANQAGLDMLETSLVALQDIT
                                801                                                                        876
         Zeama_HDIII RLD1  (773) LDKIFDESGRKALFSDISKLMEQGYAYLPSGVCMSGMGRHVSFDQAVAWKVLGED----SNIHCLAFCFVNWSFV-
             Orysa_HOX10  (769) LDRIFDEPGKEALFSNIPKLMEQGHVYLPSGVCMSGMGRHVSFDQAVAWKVLAED----SNVHCLAFCFVNWSFV-
   Orysa_REV Os10g33960  (770) LDKIFDEAGRKALYNEIPKLMEQGYVYLPGGVCLSGMGRHVSFEQAVAWKVLGED----NNVHCLAFCFVNWSFV-
             Poptr_HDIII  (781) LDKIFNESGRQALYTEIAKLMQQGFACLPAGICMSTMGRNVSYEQAVAWKVLSAEE---NAVHCLAFSFVNWSFL-
               Arath_Rev  (768) LEKIFDESGRKAICSDFAKLMQQGFACLPSGICVSTMGRHVSYEQAVAWKVFAASEENNNNLHCLAFSFVNWSFV-
             Mectr_HDIII  (777) LDKIFDESARKNLIAYIAKLMQQGFACMPAGICMSTMGRHASYDQAVAWKVHAED----NSVHCLAFSFINWSFI-
               Consensus  (801) LDKIFDESGRKALFSDIAKLMQQGFAYLPSGICMSTMGRHVSFDQAVAWKVLAED    NNVHCLAFSFVNWSFV
```

**Complete CTR boxed**

FIGURE 4 (continued)

```
CTR_Poptr_HDIII AHETSGEVVYGLGRQPAVLRTFNQRLSRGFNDAINGFNDDGWSLMNADGAEDVIIAVNST
CTR_Arath_REV   AQESNGEVVYGLGRQPAVLRTFSQRLSRGFNDAVNGFGDDGWSTMHCDGAEDIIVAINST
CTR_Medtr_REV   AQETSGDVVYSMGRQPAVLRTFSQRLSRGFNDAVNGFNDNGWSVLNCDGAEGVTISVNSI
CTR_Triae_HDIII AQETSGEVVYALGRQPAVLRTFSQRLSRGFNDAISGFNDDGWSVMAGDGIEDVIIACNS-
CTR_Horvu_HDII  ----------ALGRQPAVLRTFSQRLSRGFNDAISGFNDDGWSVMAGDGIEDVIIACNS-
CTR_Orysa_REV   AQETSGEVVYALGRQPAVLRTFSQRLSRGFNDAISGFNDDGWSVMGGDGIEDVIIACNA-
CTR_Phypr_HDIII AQETSGEVVYALGRQPAVLRTFSQRLSRGFNDAISGFNDDGWSVMGGDGIEDVIIACNS-
CTR_Orysa\HOX10 AQETSGEVVYALGRQPAVLRTFSQRLSRGFNDAISGFNDDGWSIMGGDGVEDVVIACNST
CTR_Zeama_HDIII AQETSGEVVYALGRQPAVLRTFSQRLSRGFNDAISGFNDDGWSVMGGDGIEDVVIACNST
CTR_Sacof_HDIII AQETSGEVVYALGRQPAVLRTFSQRLSRGFNDAISGFNDDGWSVMGGDGIEDVAVACTST
                          .:**********.**********:.**.*:*** :  ** *.: :: .:

CTR_Poptr_HDIII KNLIGANNSAHSLSFLGGILCAKASMLLQNVHPAVLVCFLREHHAEWADFSVDAYSAALW
CTR_Arath_REV   KHLNNISN---SLSFLGGVLCAKASMLLQNVPPAVLIRFLREHRSEWADFNVDAYSAATL
CTR_Medtr_REV   KNLSGTSNPASSLSLLGGIVCAKASMLLQNTTPAVLVRFLREHRSEWADFSVDAFSAASL
CTR_Triae_HDIII KKIRSNNTAPNAFIAPGGVICAKASMLLQSVPPAVLVRFLREHRSEWADYNFDAYSASAL
CTR_Horvu_HDIII KKIRSNNTAPNAFIAPGGVICAKASMLLQSVPPAVLVRFLREHRSEWADYNFDAYSASAL
CTR_Orysa_REV   KKVRNTSTSANAFVTPGGVICAKASMLLQSVPPAVLVRFLREHRSEWADYNFDAYSASSL
CTR_Phypr_HDIII KKIRNNSTAANAFGAPGGVICAKASMLLQSVPPAVLVRFLREHRSEWADYNFDAYSALAL
CTR_Orysa\HOX10 KKIRSNSNAGIAFGAPGGIICAKASMLLQSVPPAVLVRFLREHRSEWADYNIDAYLASTL
CTR_Zeama_HDIII KKIRNTSNAGITFGAPGGIICAKASMLLQSVPPAVLVRFLREHRSEWADYNIDAYLASSL
CTR_Sacof_HDIII KKIRNNSNAGITFGAPGGIICAKASMLLQSVPPAVLVRFLREHRSEWADYNIDAYLASSL
                *:: . ..   ::   **::*********.. ****: *****::****:..**: *

CTR_Poptr_HDIII KAGSYAYPGMRPMRFTGSQITMPLGHTIEQEDLLEVIRLEGHSFAQEDAFVSQDIHLLQI
CTR_Arath_REV   KAGSFAYPGMRPTRFTGSQIIMPLGHTIEHEEMLEVVRLEGHSLAQEDAFMSRDVHLLQI
CTR_Medtr_REV   KAGSYGYPGMRSTKFTGNQAIMPLGHTIEHEEMLEIIRLEG--LAQDDSFVSRDVHLLQV
CTR_Triae_HDIII KSSSCSLPGLRPMRFSGSQIIMPLAHTVENEEILEVVRLEGQAL--DEGLLSRDIHLLQF
CTR_Horvu_HDIII KSSSCSLPGLRPMRFSGSQIIMPLAHTVENEEILEVVRLEGQAL--DEGLLSRDIHLLQF
CTR_Orysa_REV   KTSSCSLPGLRPMRFSGSQIIMPLAHTVENEEILEVVRLEGQALTHDDGLMSRDIHLLQL
CTR_Phypr_HDIII KTSSCSLPGLRPTRFSGSQIIMPLAHTVENEEILEVIRLEGQALTHDEGLLSRDIHLLQL
CTR_Orysa\HOX10 KTSACSLTGLRPMRFSGSQIIIPLAHTVENEEILEVVRLEGQPLTHDEALLSRDIHLLQL
CTR_Zeama_HDIII KTSACSLPGLRPMRFSEGQMIMPLAHTVENEEILEVVRLEGQPLTHDEALLSRDIHLLQL
CTR_Sacof_HDIII KTSACSLPXLQPMRXSGGQMIMPLAHTVENEEILEVIRLEGHPLTHDEALLSRDIHLLQL
                *:.: . . ::. : : .*  :**.**:*:*::**::****  :  ::.::*:*:****.

CTR_Poptr_HDIII --------CSGIDENAVGACSELVFAPIDETFPDDAPLLPSGFRIISLESKAKDTQEVLT
CTR_Arath_REV   --------CTGIDENAVGACSELIFAPINEMFPDDAPLVPSGFRVIPVDAKTGDVQDLLT
CTR_Medtr_REV   LPLTLVMLCTGIDENAVGACSELIFAPIDDMFPEDAPLVPSGFRIVLLNSQPGDTKNTTT
CTR_Triae_HDIII --------CTGLDEKSMGSCFQLVFAPIDELFPDDAPLISSGFRVIPLDMKT----DGAP
CTR_Horvu_HDIII --------CTGIDEKSMGSCFQLVFAPIDELFPDDAPLISSGFRVIPLDMKT----DGAP
CTR_Orysa_REV   --------CTGIDEKSMGSCFQLVSAPIDELFPDDAPLISSGFRVIPLDMKT----DGTP
CTR_Phypr_HDIII --------CTGIDEKSMGSCFQLVFAPIDELFPDDAPLISSGFRVIPLDMKT----DGAP
CTR_Orysa\HOX10 --------CTGIDEKSVGSSFQLVFAPIDD-FPDETPLISSGFRVIPLDMKT----DGAS
CTR_Zeama_HDIII --------CTGIDEKSVGSSFQLVFAPIDEHFPDDAPLISSGFRVIPLDVKT----DGVS
CTR_Sacof_HDIII --------CTGIDEKSVGSSFQLVFAPIDEHFPDDAPLISSGFRVIPLDMKT----DGVS
                        *:*:**:::*:. :*: ***:: **:::**:.****:: :: :.    :  .

CTR_Poptr_HDIII TN-CTLDLTSSLEAGLAINHTAVDGS--SCHSLRSVLTIAFQFPFESNLQDNVATMARQY
CTR_Arath_REV   ANHRTLDLTSSLEVGPSPENASGNSF--SSSSSRCILTIAFQFPFENNLQENVAGMACQY
CTR_Medtr_REV   AN-RTLDLTSGLEVSPATAHANGDA---SCPNNRCVLTVAFQFPFESGLQDNVAAMARQY
CTR_Triae_HDIII AG-RTLDLASSLEAGSTTLQASGGA--DDC-NLRSVLTIAFQFPYEMHLQDSVATMARQY
CTR_Horvu_HDIII TG-RTLDLASSLEAGSTTLQASGNA--DDC-NLRSVLTIAFQFPYEMHLQDSVATMARQY
CTR_Orysa_REV   AG-RTLDLASSLEVGSTA-QPTGDASMDDC-NLRSVLTIAFQFPYEMHLQDSVATMARQY
CTR_Phypr_HDIII TG-RTLDLASSLEVGSTTQQATGDASLDDCRNLRSVLTIAFQFPYEIHLQDSVATMARQY
CTR_Orysa\HOX10 SG-RTLDLASSLEVGSATAQASGDASADDC-NLRSVLTIAFQFPYELHLQDSVAAMARQY
CTR_Zeama_HDIII SG-RTLDLASSLDVGSAAPQASGDASPDDC-SLRSVLTIAFQFPYEMHLQDSVAAMARQY
CTR_Sacof_HDIII SG-RTLDLASSLDVGSAAPQASGDASPDDC-NLRSVLTIAFQFPYEMHLQDSVATMARQY
                :.  ****:*.*:.. :  :.  ..   .. . *.:**:*****:*  **:.** ** **
```

FIGURE 5

```
CTR_Poptr_HDIII VRSVISSVQRVAMAISPSGLSPVLGPKLSAGSPEALTLAHWICQSHRQVLLKFSSCYHLG
CTR_Arath_REV   VRSVISSVQRVAMAISPSGISPSLGSKLSPGSPEAVTLAQWISQSYS---------HHLG
CTR_Medtr_REV   VRRVVSAVQAVATAISPSSVNTSGGAKLSPGTPEALTLAQWICQSYS---------HHLG
CTR_Triae_HDIII VRSIVSAVQRVSMAISPSRSGLNAEQKIISGFPEAATLARWICQSYR---------FHLG
CTR_Horvu_HDIII VRSIVSAVQRVSMAISPSRSGLNAEQKIISGFPEAATLARWICQSYR---------FHLG
CTR_Orysa_REV   VRSIVSSVQRVSMAISPSRSGLNAGQKIISGFPEAPTLARWICQSYQ---------FHLG
CTR_Phypr_HDIII VRSVVSAVQRVSMAISPPRSGVNAGQKIFSGFPEAATLARWICQSYQ---------FHLG
CTR_Orysa\HOX10 VRSIVSAVQRVSMAISPSQTGLNAGQRIISGFPEAATLARWVCQSYH---------YHLG
CTR_Zeama_HDIII VRSVISAVQRVSMAISPSQSGLNAGHRMLSGFPEAATLARWVCQSYH---------YHLG
CTR_Sacof_HDIII VRSVVSAVQRVSMAISPSQSGLNA-QRTLSGFPETATLARWVCQSYH---------YHLG
                ** ::*:** *: ****.  .      :  .* **: ***:*:.**:         .***

CTR_Poptr_HDIII AELLRSDSV-GGDSVLKHLWHHPDAILCCSLKSLPVFIFANQAGLDMLETTLVALQDITL
CTR_Arath_REV   SELLTIDSLGSDDSVLKLLWDHQDAILCCSLKPQPVFMFANQAGLDMLETTLVALQDITL
CTR_Medtr_REV   AQLLRSDSL-IGDMLLKHLWHHPDAILCCSLKQVPVFIFANQAGLDMLETTLVALQDITL
CTR_Triae_HDIII VELFRQADE-AGESLLRMLWDHEDAILCCSFKEKPVFTFANEMGINMLETSFVALQDLSL
CTR_Horvu_HDIII VELFRQADE-AGESLLRMLWDHEDAILCCSFKEKPVFTFANEMGINMLETSFVALQDLSL
CTR_Orysa_REV   VELLRQADD-AGEALLKMLWDYEDAILCCSFKEKPVFTFANEMGLNMLETSLVALQDLSL
CTR_Phypr_HDIII VELLRQADE-AGESLLRMLWDYEDAILCCSFKEKPVFTFANEMGLNMLETSLVALQDLSL
CTR_Orysa\HOX10 VELLSQSDG-DAEQLLKMLWHYQDAILCCSFKEKPVFTFANKAGLDMLETSLVALQDLTL
CTR_Zeama_HDIII MELLNQSDG-AGEALLKMLWHHPDAVLCCSFKEKPMFTFANKAGLDMLETSLVALQDLTL
CTR_Sacof_HDIII VELLNQSDE-AGEALLKMLWHHPDAVLCCSFKEKPMFTFANKAGLDMLETSLIALQDLTL
                 :*:    .     : :*: **.: **:****:*   *:* ***: *::****:::****::*

CTR_Poptr_HDIII DKIFNESGRQALYTEFAKLMQQGFACLPAGICMSTMGRNVSYEQAVAWKVLSA---EENA
CTR_Arath_REV   EKIFDESGRKAICSDFAKLMQQGFACLPSGICVSTMGRHVSYEQAVAWKVFAASEENNNN
CTR_Medtr_REV   DKIFDESARKNLIAYFAKLMQQGFACMPAGICMSTMGRHASYDQAVAWKVHA----EDNS
CTR_Triae_HDIII DKIFDEAGRKALYSEIPKLMEQGFVYLPGGVCLSGMGRHVSFESAVAWKVVG----EDNN
CTR_Horvu_HDIII DKIFDEAGRKALYSEIPKLMEQGFVYLPGGVCLSGMGRHVSFENAIAWKVVG----EDNN
CTR_Orysa_REV   DKIFDEAGRKALYNEIPKLMEQGYVYLPGGVCLSGMGRHVSFEQAVAWKVLG----EDNN
CTR_Phypr_HDIII DKIFDETGRKALHSEIPKLMEQGYVYLPAGVCLSGMGRHVSFEQAVAWKVLG----EDNN
CTR_Orysa\HOX10 DRIFDEPGKEALFSNIPKLMEQGHVYLPSGVCMSGMGRHVSFDQAVAWKVLA----EDSN
CTR_Zeama_HDIII DKIFDESGRKALFSDISKLMEQGYAYLPSGVCMSGMGRHVSFDQAVAWKVLG----EDSN
CTR_Sacof_HDIII DKIFDESGRKAIFSDISKLMEQGYAYLPSGVCMSGMGRHVSFDQAVAWKVLG----EDSN
                ::**:*..:: :    :.***:**.. :*.*:*:* ***:.*::.*:**** .     ::.

CTR_Poptr_HDIII VHCIAFSFVNWSFL
CTR_Arath_REV   LHCLAFSFVNWSF-
CTR_Medtr_REV   VHCLAFSFINWSFI
CTR_Triae_HDIII VHCLAFCFVNWSF-
CTR_Horvu_HDIII VHCLAFCFVNWSFV
CTR_Orysa_REV   VHCLAFCFVNWSF-
CTR_Phypr_HDIII VHCLAFCFVNWSF-
CTR_Orysa\HOX10 VHCLAFCFVNWSFV
CTR_Zeama_HDIII IHCLAFCFVNWSFV
CTR_Sacof_HDIII VHCLAFCFVNWSF-
                :**:**.*:****
```

# FIGURE 5 (continued)

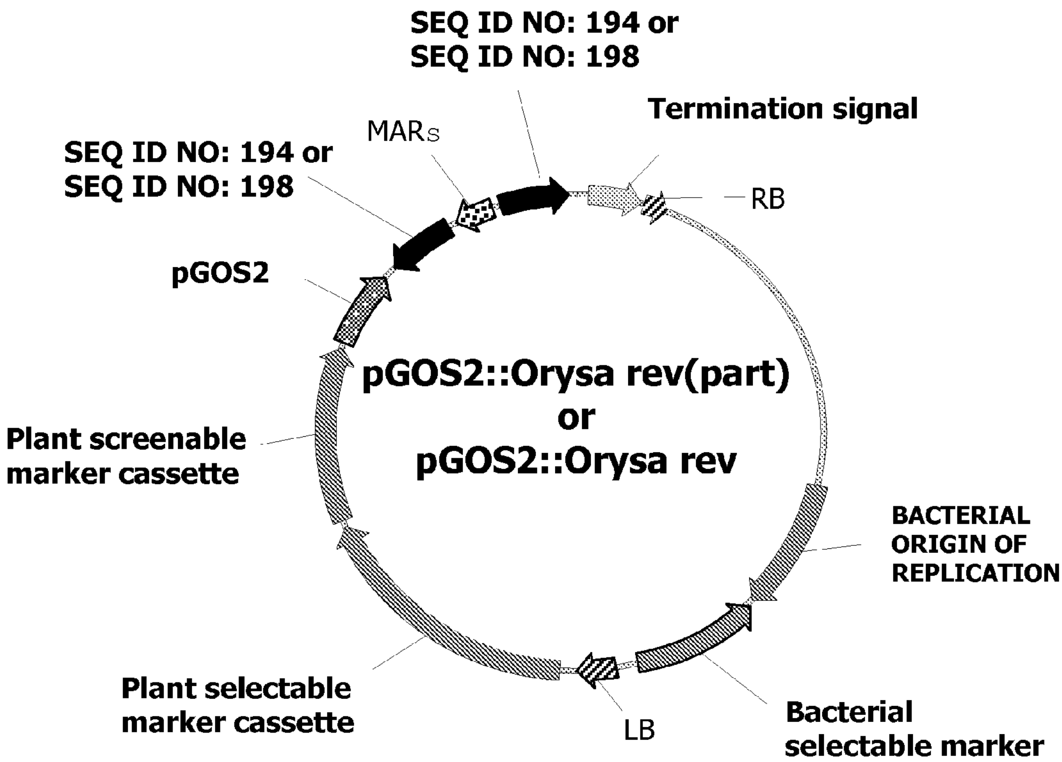
SEQ ID NO: 194 or
SEQ ID NO: 198
Termination signal
MARs
SEQ ID NO: 194 or
SEQ ID NO: 198
RB
pGOS2
pGOS2::Orysa rev(part)
or
pGOS2::Orysa rev
Plant screenable
marker cassette
BACTERIAL
ORIGIN OF
REPLICATION
Plant selectable
marker cassette
LB
Bacterial
selectable marker

FIGURE 6

European Patent Office

# EUROPEAN SEARCH REPORT

Application Number
EP 11 16 4637

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 01/33944 A1 (SLADE ANN [US]; MADISEN LINDA [US]; COMAI LUCA [US]) 17 May 2001 (2001-05-17) * page 3, paragraph 1 * * page 10, paragraph 2 - page 12, paragraph 1 * * page 35, line 17 - page 39, line 18 * | 1-17 | INV. C12N15/82 |
| X,D | WO 2004/063379 A1 (PIONEER HI BRED INT [US]; BRUCE WESLEY B [US]) 29 July 2004 (2004-07-29) * page 36, line 14 - page 38, line 15 * | 1-17 | |
| X,D | TALBERT P B ET AL: "THE REVOLUTA GENE IS NECESSARY FOR APICAL MERISTEM DEVELOPMENT AND FOR LIMITING CELL DIVISIONS IN THE LEAVES AND STERMS OF ARABIDOPSISTHALIANA", DEVELOPMENT, COMPANY OF BIOLOGISTS, CAMBRIDGE, GB, vol. 121, 1 January 1995 (1995-01-01), pages 2723-2735, XP002938883, ISSN: 0950-1991 * the whole document * | 1-17 | TECHNICAL FIELDS SEARCHED (IPC): C12N |
| A | OTSUGA D ET AL: "REVOLUTA REGULATES MERISTEM INITIATION AT LATERAL POSITIONS", THE PLANT JOURNAL, BLACKWELL SCIENTIFIC PUBLICATIONS, OXFORD, GB, vol. 25, no. 2, 1 January 2001 (2001-01-01), pages 223-236, XP002938885, ISSN: 0960-7412, DOI: 10.1046/J.1365-313X.2001.00959.X * the whole document * | 1-17 | |

-/--

The present search report has been drawn up for all claims

| Place of search | Date of completion of the search | Examiner |
|---|---|---|
| Munich | 3 February 2012 | Chakravarty, Ashok |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 16 4637

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | DATABASE Geneseq [Online]<br><br>12 June 2008 (2008-06-12),<br>"Rice cDNA clone SEQ ID No 27388.",<br>XP002668666,<br>retrieved from EBI accession no. GSN:AQD33529<br>Database accession no. AQD33529<br>* sequence *<br>& JP 2005 185101 A (NAT INST OF AGROBIO SCIENCES; SASAKI CO; RIKAGAKU KENKYUSHO; FOUNDATIO) 14 July 2005 (2005-07-14)<br>----- | 1-17 | |
| A | JAE-HEUNG KO ET AL: "Developmental and seasonal expression of PtaHB1, a Populus gene encoding a class III HD-Zip protein, is closely associated with secondary growth and inversely correlated with the level of microRNA (miR166)",<br>NEW PHYTOLOGIST,<br>vol. 169, no. 3,<br>1 January 2006 (2006-01-01), pages 469-478, XP55018021,<br>ISSN: 0028-646X, DOI: 10.1111/j.1469-8137.2005.01623.x<br>----- | 1-17 | TECHNICAL FIELDS SEARCHED (IPC) |

The present search report has been drawn up for all claims

| Place of search | Date of completion of the search | Examiner |
|---|---|---|
| Munich | 3 February 2012 | Chakravarty, Ashok |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 11 16 4637

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2012

| Patent document cited in search report | Publication date | Patent family member(s) | Publication date |
| --- | --- | --- | --- |
| WO 0133944 A1 | 17-05-2001 | AU 782918 B2 | 08-09-2005 |
| | | BR 0015368 A | 22-10-2002 |
| | | CA 2389990 A1 | 17-05-2001 |
| | | CN 1423520 A | 11-06-2003 |
| | | EP 1231830 A1 | 21-08-2002 |
| | | EP 2397031 A1 | 21-12-2011 |
| | | NZ 518530 A | 29-10-2004 |
| | | US 2006031967 A1 | 09-02-2006 |
| | | US 2011004965 A1 | 06-01-2011 |
| | | WO 0133944 A1 | 17-05-2001 |
| WO 2004063379 A1 | 29-07-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7056739 B
- WO 0133944 A
- WO 2004063379 A
- US 5811238 A
- US 6395547 B
- WO 2004070039 A
- WO 2004065596 A
- US 4962028 A
- WO 0114572 A
- WO 9514098 A
- WO 9412015 A
- EP 99106056 A
- US 5565350 A
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A


### Non-patent literature cited in the description


- **WANG et al.** *Planta,* 2003, vol. 218, 1-14
- **SESSA et al.** *EMBO J,* 1993, vol. 12 (9), 3507-3517
- **SAKAKIBARA et al.** *Mol Biol Evol,* 2001, vol. 18 (4), 491-502
- **SESSA et al.** Puigdomene P. Springer, 1994, 411-426
- **SCHRICK et al.** *Genome Biology,* 2004, vol. 5, R41
- **WILLIAMS et al.** *Development,* 2005, vol. 132, 3657-3668
- **FLOYD et al.** *Genetics,* 2006, vol. 173, 373-388
- **BAIMA et al.** *Plant Physiol,* 2001, vol. 126, 643-655
- **PRIGGE et al.** *Plant Cell,* 2005, vol. 17, 61-76
- **OTSUGA et al.** *Plant J.,* 2001, vol. 25, 223-236
- **TALBERT et al.** *Development,* 1995, vol. 121, 2723-2735
- **JUAREZ et al.** *Nature,* 2004, vol. 428, 84-88
- **MCCONNELL et al.** *Nature,* 2001, vol. 411, 709-713
- **EMERY et al.** *Curr Biol,* 2003, vol. 13, 1768-1774
- **CREIGHTON.** Proteins. 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553

- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **MATZKE et al.** *Plant Mol Biol,* 1990, vol. 14 (3), 323-32
- **COLOT et al.** *Mol Gen Genet,* 1989, vol. 216, 81-90
- **ANDERSON et al.** *NAR,* 1989, vol. 17, 461-2
- **ALBANI et al.** *Plant Cell,* 1997, vol. 9, 171-184
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.** *J. Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267

- **VELMURUGAN et al.** *J. Cell Biol.,* 2000, vol. 149, 553-566
- **KRENS, F.A. et al.** *Nature,* 1982, vol. 296, 72-74
- **NEGRUTIU L et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **LSHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific. 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **TERADA.** *Curr Opin Biotech,* vol. 15 (2), 132-8
- **RABBANI et al.** *Plant Physiol,* 2003, vol. 133, 1755-1767
- **SESSA et al.** Puigdomene. Springer, 1994, 411-426
- **NEEDLEMAN ; WUNSCH.** *J Mol Biol,* 1970, vol. 48, 443-453
- **ALTSCHUL et al.** *J Mol Biol,* 1990, vol. 215, 403-10
- **BORNBERG-BAUER et al.** Computational Approaches to Identify Leucine Zippers. *Nucleic Acids Res.,* 1998, vol. 26 (11), 2740-2746
- **WEST et al.** *Nucl Acid Res,* 1998, vol. 26 (23), 5277-87
- **FIELDS ; SONG.** *Nature,* 1989, vol. 340, 245-6
- **SAMBROOK.** Molecular Cloning: a laboratory manual. Cold Spring Harbor Laboratory Press, 2001
- **AUSUBEL et al.** *Current Protocols in Molecular Biology, Current Protocols,* 1994, vol. 1, 2
- **R.D.D. CROY.** Plant Molecular Biology Labfax. BIOS Scientific Publications Ltd (UK) and Blackwell Scientific Publications (UK, 1993
- **LSHIDA et al.** *Nature Biotech,* vol. 14 (6), 745-50
- **LSHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **ALTSCHUL et al.** *J. Mol. Biol.,* 1990, vol. 215, 403-410
- **ALTSCHUL et al.** *Nucleic Acids Res.,* 1997, vol. 25, 3389-3402
- *BMC Bioinformatics.,* 2003, vol. 4, 29
- **AUSUBEL et al.** *Current Protocols in Molecular Biology,* 1994, vol. 1 - 2

SEQUENCE LISTING

<110> CropDesign N.V.

<120> Plants having enhanced yield-related traits and a method for making the same

<130> PF58342
<160> 293
<170> PatentIn version 3.3
<210> 1
<211> 1397
<212> DNA
<213> Arabidopsis thaliana
<400> 1
atggaaaaca aaagccatag caaccaccac cggtaccatc atctatcatc ccggagccac 60
gagcaaaacc agcgtggcct aatttcgata aatgccataa tcatcattgg catctccatt 120
atctccatat tcataatcct tgcaattctc ctaataatca ttttacttca tcgacttaaa 180
tccgcaagag tcaaagcaca agaattatct tgcaaagaaa gcttcaacaa catgaacaat 240
ggtggagctt ccaccaacta cagctacact tctagccctg atgacatcaa gagagattgt 300
ctatactcaa gaaacccaac atcgttcaga caattacccc cacagacaaa aagttgtagg 360
agaagccgag ccgaaggagt agaagtgtac acatacaagg aattagagat tgcaacaaat 420
aatttcagcg aggagaagaa aatcggaaat ggagatgtgt acaaaggagt actaagtgat 480
ggaactgttg cggccattaa aaagcttcat atgtttaatg ataatgctag taaccaaaag 540
catgaagaac ggtcctttag gcttgaggtt gatcttctta gtcgattaca atgcccatat 600
ttggtggaat tacttggata ttgtgcagat caaaaccata ggatcytgat atatgaattt 660
atgcctaatg gtactgtgga acatcatctc cacgatcata attttaagaa tctaaaggat 720
cgacctcaac cattagattg gggagctcgg cttaggatcg cccttgattg cgccagagcc 780
ctaragtttc ttcatgagaa tacaatctct actgttatcc accggaactt caagtgtacc 840
aacattttgt tggatcaaaa taatcgagct aaagtttcag atttcggatt agccaaaacc 900
ggatccgata aactaaacgg tgagatttca acaagggtta ttggcaccac aggatatctt 960
gctccagagt atgcctctac tggaaagctt actacaaaat cagatgttta tagttatggt 1020
attgtgttac tacaactctt aacgggtcgc acaccgatcg attcaagacg tccacgggga 1080
caagatgtcc ttgtctcttg ggctcttcca agactaacca atagagagaa aattagtgaa 1140
atggtggatc caaccatgaa aggtcaatac tcacaaaaag atcttattca ggtagcagcc 1200
atagcagcag tgtgtgtgca accagaagca agttatagac cgttgatgac ggatgttgtt 1260
cactcgctca ttccccttgt taaagctttc aacaaaagta ctgattcttc tcggtttcct 1320
agccgtagag aaagcttgtc atttgatgat attatgccat gacactcttt tgtttaccca 1380
gctttcttgt acaaagt 1397
<210> 2
<211> 453
<212> PRT
<213> Arabidopsis thaliana
<220>
<221> UNSURE
<222> (216)..(216)
<223> Xaa can be any naturally occurring amino acid
<220>
<221> UNSURE
<222> (262)..(262)
<223> Xaa can be any naturally occurring amino acid
<400> 2
Met Glu Asn Lys Ser His Ser Asn His His Arg Tyr His His Leu Ser
1 5 10 15
Ser Arg Ser His Glu Gln Asn Gln Arg Gly Leu Ile Ser Ile Asn Ala
20 25 30
Ile Ile Ile Ile Gly Ile Ser Ile Ile Ser Ile Phe Ile Ile Leu Ala
35 40 45
Ile Leu Leu Ile Ile Ile Leu Leu His Arg Leu Lys Ser Ala Arg Val
50 55 60
Lys Ala Gln Glu Leu Ser Cys Lys Glu Ser Phe Asn Asn Met Asn Asn
65 70 75 80
Gly Gly Ala Ser Thr Asn Tyr Ser Tyr Thr Ser Ser Pro Asp Asp Ile
85 90 95
Lys Arg Asp Cys Leu Tyr Ser Arg Asn Pro Thr Ser Phe Arg Gln Leu
100 105 110
Pro Pro Gln Thr Lys Ser Cys Arg Arg Ser Arg Ala Glu Gly Val Glu
115 120 125
Val Tyr Thr Tyr Lys Glu Leu Glu Ile Ala Thr Asn Asn Phe Ser Glu
130 135 140
Glu Lys Lys Ile Gly Asn Gly Asp Val Tyr Lys Gly Val Leu Ser Asp
145 150 155 160
Gly Thr Val Ala Ala Ile Lys Lys Leu His Met Phe Asn Asp Asn Ala

```
                    165                 170                 175
Ser Asn Gln Lys His Glu Glu Arg Ser Phe Arg Leu Glu Val Asp Leu
            180                 185                 190
Leu Ser Arg Leu Gln Cys Pro Tyr Leu Val Glu Leu Leu Gly Tyr Cys
        195                 200                 205
Ala Asp Gln Asn His Arg Ile Xaa Ile Tyr Glu Phe Met Pro Asn Gly
    210                 215                 220
Thr Val Glu His His Leu His Asp His Asn Phe Lys Asn Leu Lys Asp
225                 230                 235                 240
Arg Pro Gln Pro Leu Asp Trp Gly Ala Arg Leu Arg Ile Ala Leu Asp
                245                 250                 255
Cys Ala Arg Ala Leu Xaa Phe Leu His Glu Asn Thr Ile Ser Thr Val
            260                 265                 270
Ile His Arg Asn Phe Lys Cys Thr Asn Ile Leu Leu Asp Gln Asn Asn
        275                 280                 285
Arg Ala Lys Val Ser Asp Phe Gly Leu Ala Lys Thr Gly Ser Asp Lys
    290                 295                 300
Leu Asn Gly Glu Ile Ser Thr Arg Val Ile Gly Thr Thr Gly Tyr Leu
305                 310                 315                 320
Ala Pro Glu Tyr Ala Ser Thr Gly Lys Leu Thr Thr Lys Ser Asp Val
                325                 330                 335
Tyr Ser Tyr Gly Ile Val Leu Leu Gln Leu Leu Thr Gly Arg Thr Pro
            340                 345                 350
Ile Asp Ser Arg Arg Pro Arg Gly Gln Asp Val Leu Val Ser Trp Ala
        355                 360                 365
Leu Pro Arg Leu Thr Asn Arg Glu Lys Ile Ser Glu Met Val Asp Pro
    370                 375                 380
Thr Met Lys Gly Gln Tyr Ser Gln Lys Asp Leu Ile Gln Val Ala Ala
385                 390                 395                 400
Ile Ala Ala Val Cys Val Gln Pro Glu Ala Ser Tyr Arg Pro Leu Met
                405                 410                 415
Thr Asp Val Val His Ser Leu Ile Pro Leu Val Lys Ala Phe Asn Lys
            420                 425                 430
Ser Thr Asp Ser Ser Arg Phe Pro Ser Arg Arg Glu Ser Leu Ser Phe
        435                 440                 445
Asp Asp Ile Met Pro
    450
```

<210> 3
<211> 55
<212> DNA
<213> Artificial sequence
<220>
<223> primer: prm2500
<400> 3

```
ggggacaagt ttgtacaaaa aagcaggctt cacaatggaa aacaaaagcc atagc        55
```

<210> 4
<211> 50
<212> DNA
<213> Artificial sequence
<220>
<223> primer: prm2501
<400> 4

```
ggggaccact ttgtacaaga aagctgggta aacaaaagag tgtcatggca              50
```

<210> 5
<211> 2193
<212> DNA
<213> Oryza sativa
<400> 5

```
aatccgaaaa gtttctgcac cgttttcacc ccctaactaa caatataggg aacgtgtgct    60
aaatataaaa tgagacctta tatatgtagc gctgataact agaactatgc aagaaaaact   120
catccaccta ctttagtggc aatcgggcta aataaaaaag agtcgctaca ctagtttcgt   180
tttccttagt aattaagtgg gaaaatgaaa tcattattgc ttagaatata cgttcacatc   240
tctgtcatga agttaaatta ttcgaggtag ccataattgt catcaaactc ttcttgaata   300
aaaaaatctt tctagctgaa ctcaatgggt aaagagagag attttttta aaaaaataga    360
atgaagatat tctgaacgta ttggcaaaga tttaaacata taattatata attttatagt   420
ttgtgcattc gtcatatcgc acatcattaa ggacatgtct tactccatcc caatttttat   480
ttagtaatta aagacaattg acttattttt attatttatc ttttttcgat tagatgcaag   540
gtacttacgc acacactttg tgctcatgtg catgtgtgag tgcacctcct caatacacgt   600
tcaactagca acacatctct aatatcactc gcctatttaa tacatttagg tagcaatatc   660
tgaattcaag cactccacca tcaccagacc actttttaata atatctaaaa tacaaaaaat  720
aattttacag aatagcatga aaagtatgaa acgaactatt taggtttttc acatacaaaa   780
```

```
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agaggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtaccttttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga attttttttc ccaaatatct taaaaagtca   1740
ctttctggtt cagttcaatg aattgattgc tacaaataat gcttttatag cgttatccta   1800
gctgtagttc agttaatagg taataccccct atagtttagt caggagaaga acttatccga   1860
tttctgatct ccatttttaa ttatatgaaa tgaactgtag cataagcagt attcatttgg   1920
attatttttt ttattagctc tcaccccttc attattctga gctgaaagtc tggcatgaac   1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                2193
<210>  6
<211>  15
<212>  PRT
<213>  Artificial sequence
<220>
<223>  motif 1
<220>
<221>  VARIANT
<222>  (1)..(1)
<223>  /replace = "Leu"
<220>
<221>  VARIANT
<222>  (3)..(3)
<223>  /replace = "Gly" /replace = "Arg"
<220>
<221>  VARIANT
<222>  (5)..(5)
<223>  /replace = "Met"
<220>
<221>  VARIANT
<222>  (6)..(6)
<223>  /replace = "Arg" /replace = "Gln"
<220>
<221>  VARIANT
<222>  (7)..(7)
<223>  /replace = "Ser" /replace = "Cys"
<220>
<221>  VARIANT
<222>  (8)..(8)
<223>  /replace = "Pro"
<220>
<221>  VARIANT
<222>  (9)..(9)
<223>  /replace = "Tyr"
<220>
<221>  VARIANT
<222>  (11)..(11)
<223>  /replace = "Val"
<220>
<221>  UNSURE
<222>  (12)..(12)
<223>  Xaa can be any naturally occurring amino acid, preferably one of
        Lys, Asn, Ala, Ser, Gly or Glu
<220>
<221>  VARIANT
<222>  (14)..(14)
<223>  /replace = "Leu" /replace = "Val"
```

<400> 6
Met Leu Ser Arg Leu His His Arg Asn Leu Leu Xaa Leu Ile Gly
1 5 10 15
<210> 7
<211> 11
<212> PRT
<213> Artificial sequence
<220>
<223> motif 2
<220>
<221> VARIANT
<222> (2)..(2)
<223> /replace = "Tyr" /replace = "Asn"
<220>
<221> VARIANT
<222> (3)..(3)
<223> /replace = "Phe"
<220>
<221> UNSURE
<222> (4)..(4)
<223> Xaa can be any naturally occurring amino acid, preferably one of Asp, Asn, Glu, Lys, Pro, Gln or Gly
<220>
<221> VARIANT
<222> (5)..(5)
<223> /replace = "Val" /replace = "Thr"
<220>
<221> VARIANT
<222> (7)..(7)
<223> /replace = "Met"
<220>
<221> VARIANT
<222> (8)..(8)
<223> /replace = "Arg" /replace = "Gly"
<220>
<221> VARIANT
<222> (11)..(11)
<223> /replace = "Val"
<400> 7
Leu Tyr Trp Xaa Ala Arg Leu Leu Ile Ala Leu
1 5 10
<210> 8
<211> 9
<212> PRT
<213> Artificial sequence
<220>
<223> motif 3
<220>
<221> VARIANT
<222> (2)..(2)
<223> /replace = "Lys"
<220>
<221> VARIANT
<222> (3)..(3)
<223> /replace = "Gly"
<220>
<221> VARIANT
<222> (5)..(5)
<223> /replace = "Glu"
<220>
<221> VARIANT
<222> (6)..(6)
<223> /replace = "Phe"
<400> 8
Ala Arg Ala Leu Ala Tyr Leu His Glu
1 5
<210> 9
<211> 14
<212> PRT
<213> Artificial sequence
<220>

<223> motif 4
<220>
<221> VARIANT
<222> (1)..(1)
<223> /replace = "Ile"
<220>
<221> VARIANT
<222> (5)..(5)
<223> /replace = "Asn"
<220>
<221> VARIANT
<222> (6)..(6)
<223> /replace = "Leu"
<220>
<221> VARIANT
<222> (6)..(6)
<223> amino acid is preferably not Ile, Val or Met
<220>
<221> VARIANT
<222> (8)..(8)
<223> /replace = "Ala" /replace = "Gly" /replace = "Cys"
<220>
<221> VARIANT
<222> (9)..(9)
<223> /replace = "Thr"
<220>
<221> VARIANT
<222> (11)..(11)
<223> /replace = "Val"
<220>
<221> VARIANT
<222> (14)..(14)
<223> /replace = "Asp"
<400> 9
Val Ile His Arg Asp Phe Lys Ser Ser Asn Ile Leu Leu Glu
1 5 10
<210> 10
<211> 7
<212> PRT
<213> Artificial sequence
<220>
<223> motif 5
<220>
<221> VARIANT
<222> (1)..(1)
<223> /replace = "Arg"
<220>
<221> VARIANT
<222> (3)..(3)
<223> /replace = "Ala" /replace = "Thr"
<220>
<221> VARIANT
<222> (7)..(7)
<223> /replace = "Met" /replace = "Ser"
<400> 10
Lys Val Ser Asp Phe Gly Leu
1 5
<210> 11
<211> 1524
<212> DNA
<213> Arabidopsis thaliana
<400> 11
atggaaaaca aaagccatag caaccaccac cggtaccatc atctatcatc ccggagccac 60
gagcaaaacc agcgtggcct aatttcgata aatgccataa tcatcattgg catctccatt 120
atctccatat tcataatcct tgcaattctc ctaataatca ttttacttca tcgacttaaa 180
tccgcaagag tcaaagcaca agaattatct tgcaaagaaa gcttcaacaa catgaacaat 240
ggtggagctt ccaccaacta cagctacact tctagccctg gtaaaacacc taaacattca 300
ttccataatt ttgtaaagaa tcataagttt ttaattcaaa atcaatcagc aatgagtgga 360
gattctcata tctttatatc agtattgaat tacttccaca tccaatgtgt atttcttatt 420
cgaactttaa tgttcttgtt agatgacatc aagagagatt gtctatactc aagaaaccca 480
acatcgttca gacaattacc cccacagaca aaaagttgta ggagaagccg agccgaagga 540

```
gtagaagtgt acacatacaa ggaattagag attgcaacaa ataatttcag cgaggagaag     600
aaaatcggaa atggagatgt gtacaaagga gtactaagtg atggaactgt tgcggccatt     660
aaaaagcttc atatgtttaa tgataatgct agtaaccaaa agcatgaaga acggtccttt     720
aggcttgagg ttgatcttct tagtcgatta caatgcccat atttggtgga attacttgga     780
tattgtgcag atcaaaacca taggatcctg atatatgaat ttatgcctaa tggtactgtg     840
gaacatcatc tccacgatca taattttaag aatctaaagg atcgacctca accattagat     900
tggggagctc ggcttaggat cgcccttgat tgcgccagag ccctagagtt tcttcatgag     960
aatacaatct ctactgttat ccaccggaac ttcaagtgta ccaacatttt gttggatcaa    1020
aataatcgag ctaaagtttc agatttcgga ttagccaaaa ccggatccga taaactaaac    1080
ggtgagattt caacaagggt tattggcacc acaggatatc ttgctccaga gtatgcctct    1140
actggaaagc ttactacaaa atcagatgtt tatagttatg gtattgtgtt actacaactc    1200
ttaacgggtc gcacaccgat cgattcaaga cgtccacggg gacaagatgt ccttgtctct    1260
tgggctcttc caagactaac caatagagag aaaattagtg aaatggtgga tccaaccatg    1320
aaaggtcaat actcacaaaa agatcttatt caggtagcag ccatagcagc agtgtgtgtg    1380
caaccagaag caagttatag accgttgatg acggatgttg ttcactcgct cattcccctt    1440
gttaaagctt tcaacaaaag tactgattct tctcggtttc ctagccgtag agaaagcttg    1500
tcatttgatg atattatgcc atga                                           1524
```

<210> 12
<211> 507
<212> PRT
<213> Arabidopsis thaliana
<400> 12

```
Met Glu Asn Lys Ser His Ser Asn His His Arg Tyr His His Leu Ser
1               5                   10                  15
Ser Arg Ser His Glu Gln Asn Gln Arg Gly Leu Ile Ser Ile Asn Ala
            20                  25                  30
Ile Ile Ile Ile Gly Ile Ser Ile Ile Ser Ile Phe Ile Ile Leu Ala
        35                  40                  45
Ile Leu Leu Ile Ile Ile Leu Leu His Arg Leu Lys Ser Ala Arg Val
    50                  55                  60
Lys Ala Gln Glu Leu Ser Cys Lys Glu Ser Phe Asn Asn Met Asn Asn
65                  70                  75                  80
Gly Gly Ala Ser Thr Asn Tyr Ser Tyr Thr Ser Ser Pro Gly Lys Thr
                85                  90                  95
Pro Lys His Ser Phe His Asn Phe Val Lys Asn His Lys Phe Leu Ile
            100                 105                 110
Gln Asn Gln Ser Ala Met Ser Gly Asp Ser His Ile Phe Ile Ser Val
        115                 120                 125
Leu Asn Tyr Phe His Ile Gln Cys Val Phe Leu Ile Arg Thr Leu Met
    130                 135                 140
Phe Leu Leu Asp Asp Ile Lys Arg Asp Cys Leu Tyr Ser Arg Asn Pro
145                 150                 155                 160
Thr Ser Phe Arg Gln Leu Pro Pro Gln Thr Lys Ser Cys Arg Arg Ser
                165                 170                 175
Arg Ala Glu Gly Val Glu Val Tyr Thr Tyr Lys Glu Leu Glu Ile Ala
            180                 185                 190
Thr Asn Asn Phe Ser Glu Glu Lys Lys Ile Gly Asn Gly Asp Val Tyr
        195                 200                 205
Lys Gly Val Leu Ser Asp Gly Thr Val Ala Ala Ile Lys Lys Leu His
    210                 215                 220
Met Phe Asn Asp Asn Ala Ser Asn Gln Lys His Glu Glu Arg Ser Phe
225                 230                 235                 240
Arg Leu Glu Val Asp Leu Leu Ser Arg Leu Gln Cys Pro Tyr Leu Val
                245                 250                 255
Glu Leu Leu Gly Tyr Cys Ala Asp Gln Asn His Arg Ile Leu Ile Tyr
            260                 265                 270
Glu Phe Met Pro Asn Gly Thr Val Glu His His Leu His Asp His Asn
        275                 280                 285
Phe Lys Asn Leu Lys Asp Arg Pro Gln Pro Leu Asp Trp Gly Ala Arg
    290                 295                 300
Leu Arg Ile Ala Leu Asp Cys Ala Arg Ala Leu Glu Phe Leu His Glu
305                 310                 315                 320
Asn Thr Ile Ser Thr Val Ile His Arg Asn Phe Lys Cys Thr Asn Ile
                325                 330                 335
Leu Leu Asp Gln Asn Asn Arg Ala Lys Val Ser Asp Phe Gly Leu Ala
            340                 345                 350
Lys Thr Gly Ser Asp Lys Leu Asn Gly Glu Ile Ser Thr Arg Val Ile
        355                 360                 365
Gly Thr Thr Gly Tyr Leu Ala Pro Glu Tyr Ala Ser Thr Gly Lys Leu
    370                 375                 380
Thr Thr Lys Ser Asp Val Tyr Ser Tyr Gly Ile Val Leu Leu Gln Leu
```

```
385                 390                 395                 400
Leu Thr Gly Arg Thr Pro Ile Asp Ser Arg Arg Pro Arg Gly Gln Asp
                405                 410                 415
Val Leu Val Ser Trp Ala Leu Pro Arg Leu Thr Asn Arg Glu Lys Ile
            420                 425                 430
Ser Glu Met Val Asp Pro Thr Met Lys Gly Gln Tyr Ser Gln Lys Asp
        435                 440                 445
Leu Ile Gln Val Ala Ala Ile Ala Ala Val Cys Val Gln Pro Glu Ala
    450                 455                 460
Ser Tyr Arg Pro Leu Met Thr Asp Val Val His Ser Leu Ile Pro Leu
465                 470                 475                 480
Val Lys Ala Phe Asn Lys Ser Thr Asp Ser Ser Arg Phe Pro Ser Arg
                485                 490                 495
Arg Glu Ser Leu Ser Phe Asp Asp Ile Met Pro
            500                 505
<210>  13
<211>  1482
<212>  DNA
<213>  Arabidopsis thaliana
<400>  13
caaaaatgaa caacaccaaa atggaaaaca aaagccatag caaccaccac cggtaccatc      60
atctatcatc ccggagccac gagcaaaacc agcgtggcct aatttcgata aatgccataa     120
tcatcattgg catctccatt atctccatat tcataatcct tgcaattctc ctaataatca     180
ttttacttca tcgacttaaa tccgcaagag tcaaagcaca agaattatct tgcaaagaaa     240
gcttcaacaa catgaacaat ggtggagctt ccaccaacta cagctacact tctagccctg     300
atgacatcaa gagagattgt ctatactcaa gaaacccaac atcgttcaga caattacccc     360
cacagacaaa aagttgtagg agaagccgag ccgaaggagt agaagtgtac acatacaagg     420
aattagagat tgcaacaaat aatttcagcg aggagaagaa aatcggaaat ggagatgtgt     480
acaaaggagt actaagtgat ggaactgttg cggccattaa aaagcttcat atgtttaatg     540
ataatgctag taaccaaaag catgaagaac ggtcctttag gcttgaggtt gatcttctta     600
gtcgattaca atgcccatat ttggtggaat tacttggata ttgtgcagat caaaaccata     660
ggatcctgat atatgaattt atgcctaatg gtactgtgga acatcatctc cacgatcata     720
attttaagaa tctaaaggat cgacctcaac cattagattg gggagctcgg cttaggatcg     780
cccttgattg cgccagagcc ctagagtttc ttcatgagaa tacaatctct actgttatcc     840
accggaactt caagtgtacc aacattttgt tggatcaaaa taatcgagct aaagtttcag     900
atttcggatt agccaaaacc ggatccgata aactaaacgg tgagatttca acaagggtta     960
ttggcaccac aggatatctt gctccagagt atgcctctac tggaaagctt actacaaaat    1020
cagatgttta tagttatggt attgtgttac tacaactctt aacgggtcgc acaccgatcg    1080
attcaagacg tccacgggga caagatgtcc ttgtctcttg ggctcttcca agactaacca    1140
atagagagaa aattagtgaa atggtggatc caaccatgaa aggtcaatac tcacaaaaag    1200
atcttattca ggtagcagcc atagcagcag tgtgtgtgca accagaagca agttatagac    1260
cgttgatgac ggatgttgtt cactcgctca ttccccttgt taaagctttc aacaaaagta    1320
ctgattcttc tcggtttcct agccgtagag aaagcttgtc atttgatgat attatgccat    1380
gacactcttt tgtttaatat gtattaattt ctctctttta agttgagatt ctcgttgtta    1440
ttgtatattt gtataggtaa atgaatccat ttatcttgta ct                       1482
<210>  14
<211>  458
<212>  PRT
<213>  Arabidopsis thaliana
<400>  14
Met Asn Asn Thr Lys Met Glu Asn Lys Ser His Ser Asn His His Arg
1               5                   10                  15
Tyr His His Leu Ser Ser Arg Ser His Glu Gln Asn Gln Arg Gly Leu
            20                  25                  30
Ile Ser Ile Asn Ala Ile Ile Ile Ile Gly Ile Ser Ile Ile Ser Ile
        35                  40                  45
Phe Ile Ile Leu Ala Ile Leu Leu Ile Ile Ile Leu Leu His Arg Leu
    50                  55                  60
Lys Ser Ala Arg Val Lys Ala Gln Glu Leu Ser Cys Lys Glu Ser Phe
65                  70                  75                  80
Asn Asn Met Asn Asn Gly Gly Ala Ser Thr Asn Tyr Ser Tyr Thr Ser
                85                  90                  95
Ser Pro Asp Asp Ile Lys Arg Asp Cys Leu Tyr Ser Arg Asn Pro Thr
            100                 105                 110
Ser Phe Arg Gln Leu Pro Pro Gln Thr Lys Ser Cys Arg Arg Ser Arg
        115                 120                 125
Ala Glu Gly Val Glu Val Tyr Thr Tyr Lys Glu Leu Glu Ile Ala Thr
    130                 135                 140
Asn Asn Phe Ser Glu Glu Lys Lys Ile Gly Asn Gly Asp Val Tyr Lys
145                 150                 155                 160
Gly Val Leu Ser Asp Gly Thr Val Ala Ala Ile Lys Lys Leu His Met
```

```
                    165                 170                 175
Phe Asn Asp Asn Ala Ser Asn Gln Lys His Glu Glu Arg Ser Phe Arg
            180                 185                 190
Leu Glu Val Asp Leu Leu Ser Arg Leu Gln Cys Pro Tyr Leu Val Glu
        195                 200                 205
Leu Leu Gly Tyr Cys Ala Asp Gln Asn His Arg Ile Leu Ile Tyr Glu
    210                 215                 220
Phe Met Pro Asn Gly Thr Val Glu His His Leu His Asp His Asn Phe
225                 230                 235                 240
Lys Asn Leu Lys Asp Arg Pro Gln Pro Leu Asp Trp Gly Ala Arg Leu
                245                 250                 255
Arg Ile Ala Leu Asp Cys Ala Arg Ala Leu Glu Phe Leu His Glu Asn
            260                 265                 270
Thr Ile Ser Thr Val Ile His Arg Asn Phe Lys Cys Thr Asn Ile Leu
        275                 280                 285
Leu Asp Gln Asn Asn Arg Ala Lys Val Ser Asp Phe Gly Leu Ala Lys
    290                 295                 300
Thr Gly Ser Asp Lys Leu Asn Gly Glu Ile Ser Thr Arg Val Ile Gly
305                 310                 315                 320
Thr Thr Gly Tyr Leu Ala Pro Glu Tyr Ala Ser Thr Gly Lys Leu Thr
                325                 330                 335
Thr Lys Ser Asp Val Tyr Ser Tyr Gly Ile Val Leu Leu Gln Leu Leu
            340                 345                 350
Thr Gly Arg Thr Pro Ile Asp Ser Arg Arg Pro Arg Gly Gln Asp Val
        355                 360                 365
Leu Val Ser Trp Ala Leu Pro Arg Leu Thr Asn Arg Glu Lys Ile Ser
    370                 375                 380
Glu Met Val Asp Pro Thr Met Lys Gly Gln Tyr Ser Gln Lys Asp Leu
385                 390                 395                 400
Ile Gln Val Ala Ala Ile Ala Ala Val Cys Val Gln Pro Glu Ala Ser
                405                 410                 415
Tyr Arg Pro Leu Met Thr Asp Val Val His Ser Leu Ile Pro Leu Val
            420                 425                 430
Lys Ala Phe Asn Lys Ser Thr Asp Ser Ser Arg Phe Pro Ser Arg Arg
        435                 440                 445
Glu Ser Leu Ser Phe Asp Asp Ile Met Pro
    450                 455
```

<210> 15
<211> 1516
<212> DNA
<213> Arabidopsis thaliana
<400> 15

```
caaaaaccaa aaactaattt tcacgaaatc cgaagcttaa aaattattcc tctactgttt     60
caatttcatc tcttcttcct cttgagttgg caatttctag ggttttctca ttttcctttt    120
tttccatttc tggaaactgg taaaaggctt tttctttgtc tgtgggggag aaacaaaaat    180
ggagacagac gaagcatacc aaaagaaaga gcgagctgca ttagtagcca tcgttgtcct    240
tgcttgtctt gctttatctt ctttgttcgt cgcctttagc tactactgct atattcgtaa    300
caaggtttct aagcgtcaca gaattagcaa gaggtttgat tgtgaggaaa aaggcgattg    360
tcagaaagta caagatgtta ctgaaaatgg gttgcaaatt ttcaccttta agcaattgca    420
ttcagcaact ggtggattta gcaagtctaa tgtggttggg aatggtgggt ttggcttggt    480
ttatcgtggt gtgcttaacg atggcagaaa agttgctatc aagctcatgg atcatgcagg    540
aaagcaaggg gaagaagaat tcaagatgga ggttgagtta ctgagccgtc tgcgttcacc    600
atacttgttg gcacttcttg gttattgctc agacaatagt cataagctgc ttgtgtatga    660
gttcatggca aatggtggtc tgcaggaaca cctgtatctt cccaacaggt ctggttctgt    720
cccaccaaga ttagattggg aaactaggat gagaatagct gtggaagctg caaaaggctt    780
ggaatatctc catgaacaag tatcaccccc ggtgattcat agagacttta agagcagcaa    840
tattctgctg gacagaaact tcaatgccaa agtttcagat tttggattgg ctaaagtcgg    900
atcagataaa gctggtggac acgtttctac ccgtgtatta ggcacacaag gatatgtagc    960
tcctgagtac gctttaactg gtcacttgac aacaaagtcg gatgtctata gctacggtgt   1020
tgtcctgtta gagttactaa ccggtagagt tccagtagat atgaagagag ctacaggaga   1080
aggtgttctt gtctcttggg ctttgcctca attggctgat agagacaaag tagtagacat   1140
aatggatcca acactcgaag gacaatactc tacgaaagaa gttgttcaag ttgcagcaat   1200
agcagcaatg tgtgttcaag cagaagctga ctacagaccc ttaatggcag atgtggtgca   1260
gtcactggtt ccattagtga gaaaccgtag gtcagcttca aagcttagtg gctgctctag   1320
tagctttagc ttggctcggt ctcctaactc tccgggtaaa gcaagcatag gttctcaatg   1380
aataaatcaa tcagagaaga cgaaatgtga ggcttttatt atgtaacggt atttactaaa   1440
acagacaatg atgtaaacac ttgagtgatc aaacatggag tcatagagat aatcatcaaa   1500
taaacttttt cgaatc                                                   1516
```

<210> 16
<211> 400
<212> PRT

<213> Arabidopsis thaliana
<400> 16
Met Glu Thr Asp Glu Ala Tyr Gln Lys Lys Glu Arg Ala Ala Leu Val
1 5 10 15
Ala Ile Val Val Leu Ala Cys Leu Ala Leu Ser Ser Leu Phe Val Ala
20 25 30
Phe Ser Tyr Tyr Cys Tyr Ile Arg Asn Lys Val Ser Lys Arg His Arg
35 40 45
Ile Ser Lys Arg Phe Asp Cys Glu Glu Lys Gly Asp Cys Gln Lys Val
50 55 60
Gln Asp Val Thr Glu Asn Gly Leu Gln Ile Phe Thr Phe Lys Gln Leu
65 70 75 80
His Ser Ala Thr Gly Gly Phe Ser Lys Ser Asn Val Val Gly Asn Gly
85 90 95
Gly Phe Gly Leu Val Tyr Arg Gly Val Leu Asn Asp Gly Arg Lys Val
100 105 110
Ala Ile Lys Leu Met Asp His Ala Gly Lys Gln Gly Glu Glu Glu Phe
115 120 125
Lys Met Glu Val Glu Leu Leu Ser Arg Leu Arg Ser Pro Tyr Leu Leu
130 135 140
Ala Leu Leu Gly Tyr Cys Ser Asp Asn Ser His Lys Leu Leu Val Tyr
145 150 155 160
Glu Phe Met Ala Asn Gly Gly Leu Gln Glu His Leu Tyr Leu Pro Asn
165 170 175
Arg Ser Gly Ser Val Pro Pro Arg Leu Asp Trp Glu Thr Arg Met Arg
180 185 190
Ile Ala Val Glu Ala Ala Lys Gly Leu Glu Tyr Leu His Glu Gln Val
195 200 205
Ser Pro Pro Val Ile His Arg Asp Phe Lys Ser Ser Asn Ile Leu Leu
210 215 220
Asp Arg Asn Phe Asn Ala Lys Val Ser Asp Phe Gly Leu Ala Lys Val
225 230 235 240
Gly Ser Asp Lys Ala Gly Gly His Val Ser Thr Arg Val Leu Gly Thr
245 250 255
Gln Gly Tyr Val Ala Pro Glu Tyr Ala Leu Thr Gly His Leu Thr Thr
260 265 270
Lys Ser Asp Val Tyr Ser Tyr Gly Val Val Leu Leu Glu Leu Leu Thr
275 280 285
Gly Arg Val Pro Val Asp Met Lys Arg Ala Thr Gly Glu Gly Val Leu
290 295 300
Val Ser Trp Ala Leu Pro Gln Leu Ala Asp Arg Asp Lys Val Val Asp
305 310 315 320
Ile Met Asp Pro Thr Leu Glu Gly Gln Tyr Ser Thr Lys Glu Val Val
325 330 335
Gln Val Ala Ala Ile Ala Ala Met Cys Val Gln Ala Glu Ala Asp Tyr
340 345 350
Arg Pro Leu Met Ala Asp Val Val Gln Ser Leu Val Pro Leu Val Arg
355 360 365
Asn Arg Arg Ser Ala Ser Lys Leu Ser Gly Cys Ser Ser Ser Phe Ser
370 375 380
Leu Ala Arg Ser Pro Asn Ser Pro Gly Lys Ala Ser Ile Gly Ser Gln
385 390 395 400
<210> 17
<211> 1161
<212> DNA
<213> Arabidopsis thaliana
<400> 17
atggagacag acgaagcata ccaaaagaaa gagcgagctg cattagtagc catcgttgtc 60
cttgcttgtc ttgctttatc ttctttgttc gtcgccttta gctactactg ctatattcgt 120
aacaaggaaa aaggcgattg tcagaaagta caagatgtta ctgaaaatgg gttgcaaatt 180
ttcaccttta agcaattgca ttcagcaact ggtggattta gcaagtctaa tgtggttggg 240
aatggtgggt ttggcttggt ttatcgtggt gtgcttaacg atggcagaaa agttgctatc 300
aagctcatgg atcatgcagg aaagcaaggg gaagaagaat tcaagatgga ggttgagtta 360
ctgagccgtc tgcgttcacc atacttgttg gcacttcttg gttattgctc agacaatagt 420
cataagctgc ttgtgtatga gttcatggca aatggtggtc tgcaggaaca cctgtatctt 480
cccaacaggt ctggttctgt cccaccaaga ttagattggg aaactaggat gagaatagct 540
gtggaagctg caaaaggctt ggaatatctc catgaacaag tatcaccccc ggtgattcat 600
agagacttta agagcagcaa tattctgctg gacagaaact tcaatgccaa agtttcagat 660
tttggattgg ctaaagtcgg atcagataaa gctggtggac acgtttctac ccgtgtatta 720
ggcacacaag gatatgtagc tcctgagtac gctttaactg gtcacttgac aacaaagtcg 780
gatgtctata gctacggtgt tgtcctgtta gagttactaa ccggtagagt tccagtagat 840

```
atgaagagag ctacaggaga aggtgttctt gtctcttggg ctttgcctca attggctgat     900
agagacaaag tagtagacat aatggatcca acactcgaag gacaatactc tacgaaagaa     960
gttgttcaag ttgcagcaat agcagcaatg tgtgttcaag cagaagctga ctacagaccc    1020
ttaatggcag atgtggtgca gtcactggtt ccattagtga gaaaccgtag gtcagcttca    1080
aagcttagtg gctgctctag tagctttagc ttggctcggt ctcctaactc tccgggtaaa    1140
gcaagcatag gttctcaatg a                                              1161
<210>  18
<211>  386
<212>  PRT
<213>  Arabidopsis thaliana
<400>  18
Met Glu Thr Asp Glu Ala Tyr Gln Lys Lys Glu Arg Ala Ala Leu Val
1               5                   10                  15
Ala Ile Val Val Leu Ala Cys Leu Ala Leu Ser Ser Leu Phe Val Ala
            20                  25                  30
Phe Ser Tyr Tyr Cys Tyr Ile Arg Asn Lys Glu Lys Gly Asp Cys Gln
        35                  40                  45
Lys Val Gln Asp Val Thr Glu Asn Gly Leu Gln Ile Phe Thr Phe Lys
    50                  55                  60
Gln Leu His Ser Ala Thr Gly Gly Phe Ser Lys Ser Asn Val Val Gly
65                  70                  75                  80
Asn Gly Gly Phe Gly Leu Val Tyr Arg Gly Val Leu Asn Asp Gly Arg
                85                  90                  95
Lys Val Ala Ile Lys Leu Met Asp His Ala Gly Lys Gln Gly Glu Glu
            100                 105                 110
Glu Phe Lys Met Glu Val Glu Leu Leu Ser Arg Leu Arg Ser Pro Tyr
        115                 120                 125
Leu Leu Ala Leu Leu Gly Tyr Cys Ser Asp Asn Ser His Lys Leu Leu
    130                 135                 140
Val Tyr Glu Phe Met Ala Asn Gly Gly Leu Gln Glu His Leu Tyr Leu
145                 150                 155                 160
Pro Asn Arg Ser Gly Ser Val Pro Pro Arg Leu Asp Trp Glu Thr Arg
                165                 170                 175
Met Arg Ile Ala Val Glu Ala Ala Lys Gly Leu Glu Tyr Leu His Glu
            180                 185                 190
Gln Val Ser Pro Pro Val Ile His Arg Asp Phe Lys Ser Ser Asn Ile
        195                 200                 205
Leu Leu Asp Arg Asn Phe Asn Ala Lys Val Ser Asp Phe Gly Leu Ala
    210                 215                 220
Lys Val Gly Ser Asp Lys Ala Gly Gly His Val Ser Thr Arg Val Leu
225                 230                 235                 240
Gly Thr Gln Gly Tyr Val Ala Pro Glu Tyr Ala Leu Thr Gly His Leu
                245                 250                 255
Thr Thr Lys Ser Asp Val Tyr Ser Tyr Gly Val Val Leu Leu Glu Leu
            260                 265                 270
Leu Thr Gly Arg Val Pro Val Asp Met Lys Arg Ala Thr Gly Glu Gly
        275                 280                 285
Val Leu Val Ser Trp Ala Leu Pro Gln Leu Ala Asp Arg Asp Lys Val
    290                 295                 300
Val Asp Ile Met Asp Pro Thr Leu Glu Gly Gln Tyr Ser Thr Lys Glu
305                 310                 315                 320
Val Val Gln Val Ala Ala Ile Ala Ala Met Cys Val Gln Ala Glu Ala
                325                 330                 335
Asp Tyr Arg Pro Leu Met Ala Asp Val Val Gln Ser Leu Val Pro Leu
            340                 345                 350
Val Arg Asn Arg Arg Ser Ala Ser Lys Leu Ser Gly Cys Ser Ser Ser
        355                 360                 365
Phe Ser Leu Ala Arg Ser Pro Asn Ser Pro Gly Lys Ala Ser Ile Gly
    370                 375                 380
Ser Gln
385
<210>  19
<211>  2627
<212>  DNA
<213>  Arabidopsis thaliana
<400>  19
aacggaatat ttttttctg ggagctgaaa atcgttggtg acgatggcgg attctctttc       60
tagggtttac ggaactctgt ttagtggtgt taatatatct ctcttctcgg atccaccgtt     120
gaaaaccccc tgaagagtgt tttttttcc gggtttaagg tcttttacgt ttcgtttttt     180
cttgagattt cgtcttccgc tgtgagtttc tgttctcgcg gcgagttttt gtgtagatct     240
gagggttttc aaggcaagag aaagtgtcgg tgatgtcgat tttctcactc gcgagctctt     300
```

```
ctgattcaca aagagatgac ctaataatgg cgttgaaggc ggtggttatt gtatattgtg     360
tggtttctct cgtcagtgtt caattagctg atgctcaaca tgaaggactg ccagtttcac     420
caacgttatc accttcaact tcaccagtta tcactgatct gcccctacca gctgaatttc     480
cgcggtttca cagaaagtat ttcgcaccac aacaagcaga agcacctcag cattctcccc     540
cttatagtcg tttggtcgct tctgatcatc cccctaccag ctcacatttc tccaaacctt     600
ccatgaaaag gaatgctcag tctcctggag ccggcttggc tgatattgct ccagcacaat     660
ctagcaatgg tgttcttcct gatgccttaa ctcagccacc tttgtcgccc tccatttcaa     720
attgttgcaa atcagatatg gtgcttaaac gaagaagtat tggttgccac tgcgtgtatc     780
ctataaaact ggacatcctt ctcttgaatg tttcagaaac tcctagttgg aacatgttct     840
tgaacgaatt tgctacccag cttggtctcc tacctcacca aatcgagctg attaacttct     900
atgtgctaag cttatcaagg atgaacatat cgatggatat cacccctcat tctggaatta     960
gtttctcagc tagtcaggca tccgcaataa actcttccct tatcagccac aagattcaat    1020
ttagccctac tttggtggga gattacaaac ttctaaacct tacttggttt gaggcccctg    1080
caccttcgca agcacctcta gtggcttctt cacctcataa agcaccatca caaggatcct    1140
cagcaactac gtcagtaaga tctccaggga aaaagaggca tcccaatctt attcttatct    1200
tttctatagc cgctggtgtg cttatacttg ccataatcac tgtacttgtt atttgttccc    1260
gcgcactccg agaagagaaa gctccagatc ctcacaaaga agctgtaaaa ccaaggaacc    1320
tggacgctgg ttcatttggg ggatctcttc ctcacccagc aagtacacgg tttctgtcat    1380
atgaagaact caaagaggca actagcaatt ttgaatctgc tagcattcta ggagaaggtg    1440
ggtttggcaa ggtttacaga ggcatcttag ccgatggtac tgctgtagcg attaagaagc    1500
tcacaagtgg tgggccacaa ggtgataaag aattccaggt ggagattgat atgcttagcc    1560
gtcttcatca tcgtaatctt gtgaaacttg tgggttacta tagtagtcga gattcttctc    1620
agcacctact ttgttatgag cttgttccaa atggcagcct cgaggcttgg ctccatgggc    1680
ctctcgggtt gaactgtcct cttgattggg acaccagaat gaagattgca cttgatgctg    1740
caagaggact tgcatacctt catgaagact cgcaaccctc cgttatacac agagatttta    1800
aagcctctaa tatactcctt gaaaacaact tcaacgccaa agttgcagat tttggcctag    1860
ccaaacaagc tcctgaaggc aggggtaatc acttatctac tcgtgttatg ggcacatttg    1920
gatatgttgc gcctgaatat gcaatgacgg gacacctact cgtcaagagt gatgtttata    1980
gttacggtgt ggtccttctc gaattgttaa ctggtagaaa acctgtggat atgtcacaac    2040
cttcaggcca agaaaatctc gtcacttgga caaggccagt tttaagagac aaagaccggt    2100
tagaagaact agtcgattca agacttgaag gaaaataccc gaaagaagat ttcataagag    2160
tatgcacaat cgctgcagct tgtgttgcac ctgaagctag ccagagacca acgatgggcg    2220
aagtggttca gtcacttaaa atggttcaac gggtggttga gtatcaagac ccggttttaa    2280
acacttcaaa taaagctcgt cctaaccgga gacaatcatc agctacgttc gagtcagaag    2340
taacctcttc tatgttctct tctggtcctt attctggtct aagcgctttt gatcatgaaa    2400
atattacacg aacaactgtt ttctcagaag atcttcacga aggccgatga tagaagccag    2460
ggtttcttc tttttatttg tttttctccc acttacggtg agaaaatttc caccagagaa     2520
gcttttgagt ttgggacatt attacagctc tttggatttt ggattctcct ttattggagg    2580
atagaatttt gtatatattt ttgcgttaat taattaatat ttgccac                  2627
```

<210> 20
<211> 725
<212> PRT
<213> Arabidopsis thaliana
<400> 20

```
Met Ser Ile Phe Ser Leu Ala Ser Ser Ser Asp Ser Gln Arg Asp Asp
1               5                   10                  15
Leu Ile Met Ala Leu Lys Ala Val Val Ile Val Tyr Cys Val Val Ser
            20                  25                  30
Leu Val Ser Val Gln Leu Ala Asp Ala Gln His Glu Gly Leu Pro Val
        35                  40                  45
Ser Pro Thr Leu Ser Pro Ser Thr Ser Pro Val Ile Thr Asp Leu Pro
    50                  55                  60
Leu Pro Ala Glu Phe Pro Arg Phe His Arg Lys Tyr Phe Ala Pro Gln
65                  70                  75                  80
Gln Ala Glu Ala Pro Gln His Ser Pro Pro Tyr Ser Arg Leu Val Ala
                85                  90                  95
Ser Asp His Pro Pro Thr Ser Ser His Phe Ser Lys Pro Ser Met Lys
            100                 105                 110
Arg Asn Ala Gln Ser Pro Gly Ala Gly Leu Ala Asp Ile Ala Pro Ala
        115                 120                 125
Gln Ser Ser Asn Gly Val Leu Pro Asp Ala Leu Thr Gln Pro Pro Leu
    130                 135                 140
Ser Pro Ser Ile Ser Asn Cys Cys Lys Ser Asp Met Val Leu Lys Arg
145                 150                 155                 160
Arg Ser Ile Gly Cys His Cys Val Tyr Pro Ile Lys Leu Asp Ile Leu
                165                 170                 175
Leu Leu Asn Val Ser Glu Thr Pro Ser Trp Asn Met Phe Leu Asn Glu
            180                 185                 190
Phe Ala Thr Gln Leu Gly Leu Leu Pro His Gln Ile Glu Leu Ile Asn
        195                 200                 205
Phe Tyr Val Leu Ser Leu Ser Arg Met Asn Ile Ser Met Asp Ile Thr
```

```
        210                 215                 220
Pro His Ser Gly Ile Ser Phe Ser Ala Ser Gln Ala Ser Ala Ile Asn
225                 230                 235                 240
Ser Ser Leu Ile Ser His Lys Ile Gln Phe Ser Pro Thr Leu Val Gly
                245                 250                 255
Asp Tyr Lys Leu Leu Asn Leu Thr Trp Phe Glu Ala Pro Ala Pro Ser
            260                 265                 270
Gln Ala Pro Leu Val Ala Ser Ser Pro His Lys Ala Pro Ser Gln Gly
        275                 280                 285
Ser Ser Ala Thr Thr Ser Val Arg Ser Pro Gly Lys Lys Arg His Pro
    290                 295                 300
Asn Leu Ile Leu Ile Phe Ser Ile Ala Ala Gly Val Leu Ile Leu Ala
305                 310                 315                 320
Ile Ile Thr Val Leu Val Ile Cys Ser Arg Ala Leu Arg Glu Glu Lys
                325                 330                 335
Ala Pro Asp Pro His Lys Glu Ala Val Lys Pro Arg Asn Leu Asp Ala
            340                 345                 350
Gly Ser Phe Gly Gly Ser Leu Pro His Pro Ala Ser Thr Arg Phe Leu
        355                 360                 365
Ser Tyr Glu Glu Leu Lys Glu Ala Thr Ser Asn Phe Glu Ser Ala Ser
    370                 375                 380
Ile Leu Gly Glu Gly Gly Phe Gly Lys Val Tyr Arg Gly Ile Leu Ala
385                 390                 395                 400
Asp Gly Thr Ala Val Ala Ile Lys Lys Leu Thr Ser Gly Gly Pro Gln
                405                 410                 415
Gly Asp Lys Glu Phe Gln Val Glu Ile Asp Met Leu Ser Arg Leu His
            420                 425                 430
His Arg Asn Leu Val Lys Leu Val Gly Tyr Tyr Ser Ser Arg Asp Ser
        435                 440                 445
Ser Gln His Leu Leu Cys Tyr Glu Leu Val Pro Asn Gly Ser Leu Glu
    450                 455                 460
Ala Trp Leu His Gly Pro Leu Gly Leu Asn Cys Pro Leu Asp Trp Asp
465                 470                 475                 480
Thr Arg Met Lys Ile Ala Leu Asp Ala Ala Arg Gly Leu Ala Tyr Leu
                485                 490                 495
His Glu Asp Ser Gln Pro Ser Val Ile His Arg Asp Phe Lys Ala Ser
            500                 505                 510
Asn Ile Leu Leu Glu Asn Asn Phe Asn Ala Lys Val Ala Asp Phe Gly
        515                 520                 525
Leu Ala Lys Gln Ala Pro Glu Gly Arg Gly Asn His Leu Ser Thr Arg
    530                 535                 540
Val Met Gly Thr Phe Gly Tyr Val Ala Pro Glu Tyr Ala Met Thr Gly
545                 550                 555                 560
His Leu Leu Val Lys Ser Asp Val Tyr Ser Tyr Gly Val Val Leu Leu
                565                 570                 575
Glu Leu Leu Thr Gly Arg Lys Pro Val Asp Met Ser Gln Pro Ser Gly
            580                 585                 590
Gln Glu Asn Leu Val Thr Trp Thr Arg Pro Val Leu Arg Asp Lys Asp
        595                 600                 605
Arg Leu Glu Glu Leu Val Asp Ser Arg Leu Glu Gly Lys Tyr Pro Lys
    610                 615                 620
Glu Asp Phe Ile Arg Val Cys Thr Ile Ala Ala Ala Cys Val Ala Pro
625                 630                 635                 640
Glu Ala Ser Gln Arg Pro Thr Met Gly Glu Val Val Gln Ser Leu Lys
                645                 650                 655
Met Val Gln Arg Val Val Glu Tyr Gln Asp Pro Val Leu Asn Thr Ser
            660                 665                 670
Asn Lys Ala Arg Pro Asn Arg Arg Gln Ser Ser Ala Thr Phe Glu Ser
        675                 680                 685
Glu Val Thr Ser Ser Met Phe Ser Ser Gly Pro Tyr Ser Gly Leu Ser
    690                 695                 700
Ala Phe Asp His Glu Asn Ile Thr Arg Thr Thr Val Phe Ser Glu Asp
705                 710                 715                 720
Leu His Glu Gly Arg
                725
```

<210> 21
<211> 3732
<212> DNA
<213> Arabidopsis thaliana
<400> 21

```
cttcttcttc tgccacttcg attgctttaa acttggagat taagattaca catcaaataa      60
```

```
ttcttctttg tcttcctcgt gatcatcgag gaagcttgat ctcttctatg gaatggagaa    120
tttgactctc ctccttagga tctgtcttgt ttcgtcggtt ttagttgctg cttcttcctc    180
aggatcggaa ttgttatctc cgttatcatc accaccgtct ccattacccg aaacttcaaa    240
agggtttggt caagcaccga gtaattcacc tgaatctcac aaatccgaca atgtgccacc    300
gtctaaagct tcttctcaac cgtctcttcc tcctttagct gatttggcag ctccaccacc    360
gtcagattcc gtcggaggta aagcaccggc cggtgtgcct gttgtctttg ttcctaatgc    420
tccagctcca gctacaatac ccgttaagga tttgcccgta gcttcgcctc cggttcttca    480
acccattaca ccgattgctt caccacctcg attcattcca ggagatgcac caaaggagcc    540
tcctttctca ggaagagtta ctccagcccc ggtttcatca cctgtttcgg atattcctcc    600
aattccatca gtggctctgc ctccgcctac accgagcaat gtacctccta gaaatgcttc    660
taacaatcac aagcctcctc ctatcgaaaa gtctattgct cctgttgcat caccaccaac    720
aatatcaatt gacattgcac caccagtaca tcccgtcata cctaagttaa caccctcgag    780
ctcacctgta cctacctcga ctccaactaa gggatctccg agaagaaatc cgccaactac    840
ccacccagtt ttccccatag aatctcctgc tgtctcacca gatcatgctg caaatccagt    900
aaaacatccg ccccccagcg ataacggaga tgatagtaaa agtccgggtg ctgcaccggc    960
aaatgaaact gctaaaccct tacctgtctt cccacataaa gcttctcctc cttcgattgc   1020
tccctcagcc ccaaaattta acagacactc tcatcataca tctccatcta ctactccacc   1080
gccagattct actcctagta atgtacacca ccatccttca tcaccatctc ctcctcctct   1140
atcttcacac catcaacatc atcaagaacg aaagaaaatc gcagatagtc cagctccttc   1200
accactgcca ccgcatctca tatctccaaa gaagtcaaac cgtaaaggtt caatgactcc   1260
tcctccacaa tcgcaccatg ctccttctcc tcccattcct gattctttga tttctcctgc   1320
tcacgctcca gtctctttct ctatgaaacg catctccccg gctctagcac cttctccaac   1380
ccaagtgttt cctctcaggt cctcttcaag accttcaaaa tctcggaaat tccctctagg   1440
tccacctctt caagcatttc ctcctcctcc ccctaattca gattgttctt caactatttg   1500
tttggaaccg tacacaaaca cacctccggg atctccatgt ggctgtgtct ggccaattca   1560
agttgagcta cgccttagca tggcgctcta cgacttcttc ccaatggttt cagaatttgc   1620
tcgggaaatc agcgccggag ttttcatgaa acagagccaa gtccgtataa tgggagctaa   1680
cgcggccagc gaacaacctg agaaatccat tgttctaatc gatttagtac cgcttggaga   1740
taaattcgat aacatgactg caatgctgac ttaccagaga ttctggagta aaaaagtcta   1800
tatagatgaa ccaatctttg gcggatacga cgtgatttac gtgcgttatc ctggtttacc   1860
cgcttccccg ccaacttctg gtatgaccat tatagatcaa ggaccgtact ctggtaataa   1920
taacggaagg gcggtcaaac cgctcggagt tgatgttcca aggaagccgc gcaagaaaga   1980
gctcaatggt ggaagtattg ctgtgattgt tttatccgca gctgctttta tcggtttatg   2040
tttcgttatc gtttggttct tggttttccg gcgacaaaga gatcgacgac gtctttcgaa   2100
aagaacacca cttgctcgcc cttcactgcc ttccctctcg aaaccatcag gctcggcgag   2160
atctttaact ggaagccgat ttagctcaac ttcattgtca tttgaatcca gcattgctcc   2220
gtttactctc tctgcaaaga ctttcaccgc aagtgaaata atgaaagcta caaataactt   2280
tgatgaatcg agggttcttg gtgaaggcgg atttggacga gtttacgaag gtgttttcga   2340
tgacggaact aaagtagcag ttaaggtatt gaagagagat gaccagcaag gtagcaggga   2400
gttcttagcc gaagtggaaa tgcttagtcg tcttcatcac agaaacctcg tgaatttaat   2460
tggtatttgt atcgaagatc gtaaccgttc cctggtttat gaactcatac caaatggcag   2520
cgttgaatct cacctccacg ggattgataa agcatcttcg cctttagatt gggatgctcg   2580
gttgaagata gctcttggtg cggcccgtgg tttagcgtat ttacacgaag actcgagccc   2640
gcgagttata cacagagact tcaagtccag caacatcttg ctggaaaacg atttcacacc   2700
taaagtatct gactttggat tggctcgtaa cgcccttgat gatgaagata acagacatat   2760
atcgacacgc gttatgggaa cgtttgggta tgtggcaccg gaatacgcaa tgacagggca   2820
tcttttggtg aagagtgatg tgtatagtta cggagttgtg cttctcgagc tacttacagg   2880
gcggaaacca gtggatatgt ctcaaccgcc cggacaagag aacttagttt catggactcg   2940
gccttttctt acgagtgcag aagggcttgc agctattata gatcagtctt taggacccga   3000
gatctcattc gatagcatag ctaaagtcgc ggcaatagct tctatgtgcg ttcaaccaga   3060
agtatcacac cgtcctttca tgggagaagt agtgcaagct ttgaaacttg tcagcaacga   3120
atgtgatgaa gctaaagaac tcaattcttt aacttctatc tctaaagacg attttagaga   3180
tgacactcaa gctgagagct cttgtggtga cagctcagca aggatggctc ggtatccatt   3240
gctaccaaat tacgactctg agcctgatac agagagagga ttatctacat cggaaatgta   3300
cacggggtca gggaggttcg agagacagtc taactcgggt cccttgacct cgggtcgagg   3360
caagagtttc tggcaaaaga tgaggagatt atcgaccgga agcttgagtg agcatggaac   3420
accaacggtc atgttacgat ccggttctcg ctaaacaatc ttttgcctac agaacactga   3480
agagttttg attgcccgtt taagttaaga gactgaaagg aaagaaggtg ccttctccta    3540
caagcaaaac tctttgcctc atggaaaccg gttaagataa aaccaggagt gatcatttcc   3600
cggttcaaaa tttttagttg aatagatctg tttatctgag aagaagaaac aagagattct   3660
gttaaatcta atttgaatgt acatatcacg ttttaaagta acaggcttaa gcattaagta   3720
tcatcatcct tc                                                       3732
```

<210> 22

<211> 1113

<212> PRT

<213> Arabidopsis thaliana

<400> 22

```
Met Glu Asn Leu Thr Leu Leu Leu Arg Ile Cys Leu Val Ser Ser Val
1               5                   10                  15
Leu Val Ala Ala Ser Ser Ser Gly Ser Glu Leu Leu Ser Pro Leu Ser
            20                  25                  30
```

```
Ser Pro Pro Ser Pro Leu Pro Glu Thr Ser Lys Gly Phe Gly Gln Ala
        35                  40                  45
Pro Ser Asn Ser Pro Glu Ser His Lys Ser Asp Asn Val Pro Pro Ser
    50                  55                  60
Lys Ala Ser Ser Gln Pro Ser Leu Pro Pro Leu Ala Asp Leu Ala Ala
65                  70                  75                  80
Pro Pro Pro Ser Asp Ser Val Gly Gly Lys Ala Pro Ala Gly Val Pro
                85                  90                  95
Val Val Phe Val Pro Asn Ala Pro Ala Pro Ala Thr Ile Pro Val Lys
            100                 105                 110
Asp Leu Pro Val Ala Ser Pro Pro Val Leu Gln Pro Ile Thr Pro Ile
        115                 120                 125
Ala Ser Pro Pro Arg Phe Ile Pro Gly Asp Ala Pro Lys Glu Pro Pro
    130                 135                 140
Phe Ser Gly Arg Val Thr Pro Ala Pro Val Ser Ser Pro Val Ser Asp
145                 150                 155                 160
Ile Pro Pro Ile Pro Ser Val Ala Leu Pro Pro Pro Thr Pro Ser Asn
                165                 170                 175
Val Pro Pro Arg Asn Ala Ser Asn Asn His Lys Pro Pro Pro Ile Glu
            180                 185                 190
Lys Ser Ile Ala Pro Val Ala Ser Pro Pro Thr Ile Ser Ile Asp Ile
        195                 200                 205
Ala Pro Pro Val His Pro Val Ile Pro Lys Leu Thr Pro Ser Ser Ser
    210                 215                 220
Pro Val Pro Thr Ser Thr Pro Thr Lys Gly Ser Pro Arg Arg Asn Pro
225                 230                 235                 240
Pro Thr Thr His Pro Val Phe Pro Ile Glu Ser Pro Ala Val Ser Pro
                245                 250                 255
Asp His Ala Ala Asn Pro Val Lys His Pro Pro Pro Ser Asp Asn Gly
            260                 265                 270
Asp Asp Ser Lys Ser Pro Gly Ala Ala Pro Ala Asn Glu Thr Ala Lys
        275                 280                 285
Pro Leu Pro Val Phe Pro His Lys Ala Ser Pro Pro Ser Ile Ala Pro
    290                 295                 300
Ser Ala Pro Lys Phe Asn Arg His Ser His His Thr Ser Pro Ser Thr
305                 310                 315                 320
Thr Pro Pro Pro Asp Ser Thr Pro Ser Asn Val His His His Pro Ser
                325                 330                 335
Ser Pro Ser Pro Pro Pro Leu Ser Ser His His Gln His His Gln Glu
            340                 345                 350
Arg Lys Lys Ile Ala Asp Ser Pro Ala Pro Ser Pro Leu Pro Pro His
        355                 360                 365
Leu Ile Ser Pro Lys Lys Ser Asn Arg Lys Gly Ser Met Thr Pro Pro
    370                 375                 380
Pro Gln Ser His His Ala Pro Ser Pro Pro Ile Pro Asp Ser Leu Ile
385                 390                 395                 400
Ser Pro Ala His Ala Pro Val Ser Phe Ser Met Lys Arg Ile Ser Pro
                405                 410                 415
Ala Leu Ala Pro Ser Pro Thr Gln Val Phe Pro Leu Arg Ser Ser Ser
            420                 425                 430
Arg Pro Ser Lys Ser Arg Lys Phe Pro Leu Gly Pro Pro Leu Gln Ala
        435                 440                 445
Phe Pro Pro Pro Pro Pro Asn Ser Asp Cys Ser Ser Thr Ile Cys Leu
    450                 455                 460
Glu Pro Tyr Thr Asn Thr Pro Pro Gly Ser Pro Cys Gly Cys Val Trp
465                 470                 475                 480
Pro Ile Gln Val Glu Leu Arg Leu Ser Met Ala Leu Tyr Asp Phe Phe
                485                 490                 495
Pro Met Val Ser Glu Phe Ala Arg Glu Ile Ser Ala Gly Val Phe Met
            500                 505                 510
Lys Gln Ser Gln Val Arg Ile Met Gly Ala Asn Ala Ala Ser Glu Gln
        515                 520                 525
Pro Glu Lys Ser Ile Val Leu Ile Asp Leu Val Pro Leu Gly Asp Lys
    530                 535                 540
Phe Asp Asn Met Thr Ala Met Leu Thr Tyr Gln Arg Phe Trp Ser Lys
545                 550                 555                 560
Lys Val Tyr Ile Asp Glu Pro Ile Phe Gly Gly Tyr Asp Val Ile Tyr
                565                 570                 575
Val Arg Tyr Pro Gly Leu Pro Ala Ser Pro Pro Thr Ser Gly Met Thr
            580                 585                 590
Ile Ile Asp Gln Gly Pro Tyr Ser Gly Asn Asn Asn Gly Arg Ala Val
```

```
                595                     600                     605
Lys Pro Leu Gly Val Asp Val Pro Arg Lys Pro Arg Lys Lys Glu Leu
    610                     615                     620
Asn Gly Gly Ser Ile Ala Val Ile Val Leu Ser Ala Ala Ala Phe Ile
625                     630                     635                     640
Gly Leu Cys Phe Val Ile Val Trp Phe Leu Val Phe Arg Arg Gln Arg
                645                     650                     655
Asp Arg Arg Arg Leu Ser Lys Arg Thr Pro Leu Ala Arg Pro Ser Leu
            660                     665                     670
Pro Ser Leu Ser Lys Pro Ser Gly Ser Ala Arg Ser Leu Thr Gly Ser
        675                     680                     685
Arg Phe Ser Ser Thr Ser Leu Ser Phe Glu Ser Ser Ile Ala Pro Phe
    690                     695                     700
Thr Leu Ser Ala Lys Thr Phe Thr Ala Ser Glu Ile Met Lys Ala Thr
705                     710                     715                     720
Asn Asn Phe Asp Glu Ser Arg Val Leu Gly Glu Gly Gly Phe Gly Arg
                725                     730                     735
Val Tyr Glu Gly Val Phe Asp Asp Gly Thr Lys Val Ala Val Lys Val
            740                     745                     750
Leu Lys Arg Asp Asp Gln Gln Gly Ser Arg Glu Phe Leu Ala Glu Val
        755                     760                     765
Glu Met Leu Ser Arg Leu His His Arg Asn Leu Val Asn Leu Ile Gly
    770                     775                     780
Ile Cys Ile Glu Asp Arg Asn Arg Ser Leu Val Tyr Glu Leu Ile Pro
785                     790                     795                     800
Asn Gly Ser Val Glu Ser His Leu His Gly Ile Asp Lys Ala Ser Ser
                805                     810                     815
Pro Leu Asp Trp Asp Ala Arg Leu Lys Ile Ala Leu Gly Ala Ala Arg
            820                     825                     830
Gly Leu Ala Tyr Leu His Glu Asp Ser Ser Pro Arg Val Ile His Arg
        835                     840                     845
Asp Phe Lys Ser Ser Asn Ile Leu Leu Glu Asn Asp Phe Thr Pro Lys
    850                     855                     860
Val Ser Asp Phe Gly Leu Ala Arg Asn Ala Leu Asp Asp Glu Asp Asn
865                     870                     875                     880
Arg His Ile Ser Thr Arg Val Met Gly Thr Phe Gly Tyr Val Ala Pro
                885                     890                     895
Glu Tyr Ala Met Thr Gly His Leu Leu Val Lys Ser Asp Val Tyr Ser
            900                     905                     910
Tyr Gly Val Val Leu Leu Glu Leu Leu Thr Gly Arg Lys Pro Val Asp
        915                     920                     925
Met Ser Gln Pro Pro Gly Gln Glu Asn Leu Val Ser Trp Thr Arg Pro
    930                     935                     940
Phe Leu Thr Ser Ala Glu Gly Leu Ala Ala Ile Ile Asp Gln Ser Leu
945                     950                     955                     960
Gly Pro Glu Ile Ser Phe Asp Ser Ile Ala Lys Val Ala Ala Ile Ala
                965                     970                     975
Ser Met Cys Val Gln Pro Glu Val Ser His Arg Pro Phe Met Gly Glu
            980                     985                     990
Val Val Gln Ala Leu Lys Leu Val  Ser Asn Glu Cys Asp  Glu Ala Lys
        995                  1000                 1005
Glu Leu  Asn Ser Leu Thr Ser  Ile Ser Lys Asp Asp  Phe Arg Asp
    1010                 1015                 1020
Asp Thr  Gln Ala Glu Ser Ser  Cys Gly Asp Ser Ser  Ala Arg Met
    1025                 1030                 1035
Ala Arg  Tyr Pro Leu Leu Pro  Asn Tyr Asp Ser Glu  Pro Asp Thr
    1040                 1045                 1050
Glu Arg  Gly Leu Ser Thr Ser  Glu Met Tyr Thr Gly  Ser Gly Arg
    1055                 1060                 1065
Phe Glu  Arg Gln Ser Asn Ser  Gly Pro Leu Thr Ser  Gly Arg Gly
    1070                 1075                 1080
Lys Ser  Phe Trp Gln Lys Met  Arg Arg Leu Ser Thr  Gly Ser Leu
    1085                 1090                 1095
Ser Glu  His Gly Thr Pro Thr  Val Met Leu Arg Ser  Gly Ser Arg
    1100                 1105                 1110
```

<210> 23
<211> 3162
<212> DNA
<213> Arabidopsis thaliana
<400> 23

```
ttctggtttc gaattcactt tactctcttc gcttccaatc tctctctacc actctgatac      60
```

```
cttcgccgga gaagaagctc tcgttctctc tatcgtcgga gaagctctcg ttctctgctt     120
ccctgctctg tacagatctc gagccgcatt tcgtggatct gtcaaaatcg cgttaaaacc     180
tactcgcttc tctctaaccc tagggttttt gaattttttt cgggacgagg gagaaatcaa     240
tgtctgtgaa gctttgagga agctatatgg ttacaccttt ggttctggtt agtcttcgtt     300
ttgatcggcg gagagtgttc gccgaggctt ggtgtctccg ttattgacta atggtggttt     360
ttgatttgag ataagagatg cggaactttg cgatgcttct gctgttaatt cttcttcttc     420
actctctcgc ctcgtttcct atatgctttg ctcggttatt tccgatgagt ttgccattta     480
cccgatcaaa ggcgcatcaa atgcatttct ttcatcctta tcttaatcca tcagttgctc     540
ctacaccttc accagctttt tcccccaacc caagtcgcat tccaccacta aggcacaagg     600
gtcatcaccg tcatcgtcgc tggcatttaa gacgcaatgc cactgcagtg tcaccttcgt     660
cacatgactg tcagcagaca tgtgtggagc ctctcacttc aactcccttt ggttcacctt     720
gtggttgtgt cttccccatg aaagttcagc ttctactaag tgtcgcacct ttttctattt     780
tccccgtgac caacgagtta gaaattgagg ttgctgctgg aacatacttg gaacaaagcc     840
aagtgaaaat aatgggtgcc agtgccgaca gtgaaaatca agggaaaaca gtggtggata     900
ttaaccttgt tccacttggg gaaaagttcg acaacactac agcaacactt atttatcaga     960
gattccggca caagaaagta cctctaaatg agactgtttt tggtgattat gaggttacgc    1020
acataagtta tccaggaatt ccttcttctt cgccaaatgg agatgttact ggagatgctc    1080
caggaggcct tccaattccg atcaatgcaa caacttttgc caacaaaagc cagggaatag    1140
gttttagaac gattgcaatt attgcgttgt caggttttgt cctcattctg gttttggttg    1200
gagctatttc tataatcgtg aaatggaaga aaattgggaa gtcatctaat gctgttggtc    1260
cggctttggc tccatcgatt aacaaaaggc ctggtgctgg atctatgttt tccagtagcg    1320
ccagaagctc aggctcagat tctttgatgt cttccatggc tacatgtgct ttatcagtta    1380
agaccttcac actctccgag cttgagaagg ctactgatag attcagtgcc aagagggttt    1440
tgggcgaggg cggatttggt cgtgtttatc aggggagtat ggaagatgga actgaggttg    1500
cagtgaaact gctaactagg gacaatcaga atcgagaccg tgaatttatt gccgaagtcg    1560
agatgctaag ccgtttgcac caccgcaatc ttgtgaaact gattggaata tgcattgaag    1620
gtcgtacacg ttgcttgatt tatgagctcg tccacaatgg gagtgttgag tcccacttac    1680
acgaaggaac gcttgattgg gatgcgcggt tgaagattgc acttggagca gcgagaggac    1740
tggcttatct ccatgaagat tcaaatcccc gagtaatcca ccgtgatttc aaggctagca    1800
atgttctcct agaagatgac tttaccccaa aagtctcaga ctttggactg gcaagagaag    1860
caaccgaagg aagtcaacat atttcaactc gagtcatggg gacctttggg tacgtggctc    1920
ctgagtatgc aatgacgggg catctccttg ttaaaagcga tgtctatagt tatggtgtag    1980
ttctactaga gcttctaacc ggtagaagac cagtagacat gtctcaacct tcgggagagg    2040
aaaaccttgt gacatgggcg agacccttac tagccaacag agagggcctg gagcaactgg    2100
tggaccctgc attggctgga acctacaact ttgatgacat ggcgaaagtg gctgcgattg    2160
cctccatgtg cgtccaccaa gaggtctcac acagaccatt catgggtgaa gttgtgcagg    2220
cactaaaact tatatacaac gatgcagatg agacctgtgg tgattattgc agccaaaagg    2280
actcatcagt accagactct gctgacttca aaggcgatct ggctccttcg gatagcagct    2340
ggtggaattt gacacctcgg ttaaggtatg gtcaagcttc gtcgttcata acaatggatt    2400
atagctcagg accactagag gacatggaga accggcctca ctctgcctct agcattccaa    2460
gagtaggagg tcttattcta ccaaacagat caggtccgtt aagacccatg cgtagtagaa    2520
gaaacttctt tagactgaga ggaagcatga gcgaacacgg tggaccgtcg tcgtctagac    2580
atctctggtc cggcaatggt gactggctct gagaaaccag aaaatgtaca gtgaagaaaa    2640
gggaaaagga ggctcacaaa ctcttgagct gcatggttag gttctggtta atccggccag    2700
gttcggttca tctgggtaaa gatttccggt ttgatttctt ttaggagctg tttgttgtta    2760
tgattatcct caatagagat ttaattgttt gtttttgcga ggatgcaaaa gcacagggag    2820
ggttgtattt tgaaggacgc ctgtatttag ttctctcctt ttctctctca cccacaaaaa    2880
aacattttta tatattttat tttagtgaag ttagtaatta cctaattttt tttagttttt    2940
caagttccta agaaatttgg cttttggtca tcgttgttaa gtttccggtt ttctgaattt    3000
acatccgaaa tattttttgg ttaaataaaa ttctggcttg agagtaattg gctggtgaat    3060
ggtaacaagt cacttggtca aagctgttgt aattcttttc taatttctag tatcttctgt    3120
agattggaca caatgacatg gattgttgat ctttaaagtc gt                       3162
```

<210> 24

<211> 744

<212> PRT

<213> Arabidopsis thaliana

<400> 24

```
Met Arg Asn Phe Ala Met Leu Leu Leu Leu Ile Leu Leu Leu His Ser
1               5                   10                  15
Leu Ala Ser Phe Pro Ile Cys Phe Ala Arg Leu Phe Pro Met Ser Leu
            20                  25                  30
Pro Phe Thr Arg Ser Lys Ala His Gln Met His Phe Phe His Pro Tyr
        35                  40                  45
Leu Asn Pro Ser Val Ala Pro Thr Pro Ser Pro Ala Phe Ser Pro Asn
    50                  55                  60
Pro Ser Arg Ile Pro Pro Leu Arg His Lys Gly His His Arg His Arg
65                  70                  75                  80
Arg Trp His Leu Arg Arg Asn Ala Thr Ala Val Ser Pro Ser Ser His
                85                  90                  95
Asp Cys Gln Gln Thr Cys Val Glu Pro Leu Thr Ser Thr Pro Phe Gly
            100                 105                 110
```

Ser Pro Cys Gly Cys Val Phe Pro Met Lys Val Gln Leu Leu Leu Ser
115 120 125
Val Ala Pro Phe Ser Ile Phe Pro Val Thr Asn Glu Leu Glu Ile Glu
130 135 140
Val Ala Ala Gly Thr Tyr Leu Glu Gln Ser Gln Val Lys Ile Met Gly
145 150 155 160
Ala Ser Ala Asp Ser Glu Asn Gln Gly Lys Thr Val Val Asp Ile Asn
165 170 175
Leu Val Pro Leu Gly Glu Lys Phe Asp Asn Thr Thr Ala Thr Leu Ile
180 185 190
Tyr Gln Arg Phe Arg His Lys Lys Val Pro Leu Asn Glu Thr Val Phe
195 200 205
Gly Asp Tyr Glu Val Thr His Ile Ser Tyr Pro Gly Ile Pro Ser Ser
210 215 220
Ser Pro Asn Gly Asp Val Thr Gly Asp Ala Pro Gly Gly Leu Pro Ile
225 230 235 240
Pro Ile Asn Ala Thr Thr Phe Ala Asn Lys Ser Gln Gly Ile Gly Phe
245 250 255
Arg Thr Ile Ala Ile Ile Ala Leu Ser Gly Phe Val Leu Ile Leu Val
260 265 270
Leu Val Gly Ala Ile Ser Ile Ile Val Lys Trp Lys Lys Ile Gly Lys
275 280 285
Ser Ser Asn Ala Val Gly Pro Ala Leu Ala Pro Ser Ile Asn Lys Arg
290 295 300
Pro Gly Ala Gly Ser Met Phe Ser Ser Ser Ala Arg Ser Ser Gly Ser
305 310 315 320
Asp Ser Leu Met Ser Ser Met Ala Thr Cys Ala Leu Ser Val Lys Thr
325 330 335
Phe Thr Leu Ser Glu Leu Glu Lys Ala Thr Asp Arg Phe Ser Ala Lys
340 345 350
Arg Val Leu Gly Glu Gly Gly Phe Gly Arg Val Tyr Gln Gly Ser Met
355 360 365
Glu Asp Gly Thr Glu Val Ala Val Lys Leu Leu Thr Arg Asp Asn Gln
370 375 380
Asn Arg Asp Arg Glu Phe Ile Ala Glu Val Glu Met Leu Ser Arg Leu
385 390 395 400
His His Arg Asn Leu Val Lys Leu Ile Gly Ile Cys Ile Glu Gly Arg
405 410 415
Thr Arg Cys Leu Ile Tyr Glu Leu Val His Asn Gly Ser Val Glu Ser
420 425 430
His Leu His Glu Gly Thr Leu Asp Trp Asp Ala Arg Leu Lys Ile Ala
435 440 445
Leu Gly Ala Ala Arg Gly Leu Ala Tyr Leu His Glu Asp Ser Asn Pro
450 455 460
Arg Val Ile His Arg Asp Phe Lys Ala Ser Asn Val Leu Leu Glu Asp
465 470 475 480
Asp Phe Thr Pro Lys Val Ser Asp Phe Gly Leu Ala Arg Glu Ala Thr
485 490 495
Glu Gly Ser Gln His Ile Ser Thr Arg Val Met Gly Thr Phe Gly Tyr
500 505 510
Val Ala Pro Glu Tyr Ala Met Thr Gly His Leu Leu Val Lys Ser Asp
515 520 525
Val Tyr Ser Tyr Gly Val Val Leu Leu Glu Leu Leu Thr Gly Arg Arg
530 535 540
Pro Val Asp Met Ser Gln Pro Ser Gly Glu Glu Asn Leu Val Thr Trp
545 550 555 560
Ala Arg Pro Leu Leu Ala Asn Arg Glu Gly Leu Glu Gln Leu Val Asp
565 570 575
Pro Ala Leu Ala Gly Thr Tyr Asn Phe Asp Asp Met Ala Lys Val Ala
580 585 590
Ala Ile Ala Ser Met Cys Val His Gln Glu Val Ser His Arg Pro Phe
595 600 605
Met Gly Glu Val Val Gln Ala Leu Lys Leu Ile Tyr Asn Asp Ala Asp
610 615 620
Glu Thr Cys Gly Asp Tyr Cys Ser Gln Lys Asp Ser Ser Val Pro Asp
625 630 635 640
Ser Ala Asp Phe Lys Gly Asp Leu Ala Pro Ser Asp Ser Ser Trp Trp
645 650 655
Asn Leu Thr Pro Arg Leu Arg Tyr Gly Gln Ala Ser Ser Phe Ile Thr
660 665 670
Met Asp Tyr Ser Ser Gly Pro Leu Glu Asp Met Glu Asn Arg Pro His

```
            675                     680                     685
Ser Ala Ser Ser Ile Pro Arg Val Gly Gly Leu Ile Leu Pro Asn Arg
    690                     695                     700
Ser Gly Pro Leu Arg Pro Met Arg Ser Arg Arg Asn Phe Phe Arg Leu
705                     710                     715                 720
Arg Gly Ser Met Ser Glu His Gly Gly Pro Ser Ser Ser Arg His Leu
                725                     730                     735
Trp Ser Gly Asn Gly Asp Trp Leu
            740
<210>  25
<211>  369
<212>  DNA
<213>  Oryza sativa
<400>  25
atgccaacta gtagagtcga cctgctgagc cggatgcact cgccgtacct ggtggggttg      60
ctgggctact gcgccgacca gagccaccgt ctgctggtgt tcgagttcat gcccaacggc     120
agcctcaaga gccacctcca ccggcgcgcg ctggcgccgg cggagcagcc gccgccgctg     180
gactggcaga cgcggctggg catcgcgctg gactgcgccc gcgcgctgga gttcctccac     240
gagcacagct cccccgccgt gatccaccgc gacttcaagt gcagcaacat cctcctcgac     300
cacaactacc gcgcccgcgt ctccgacttc ggcatgggca agctcggctc caacaaggcc     360
aatggccag                                                             369
<210>  26
<211>  123
<212>  PRT
<213>  Oryza sativa
<400>  26
Met Pro Thr Ser Arg Val Asp Leu Leu Ser Arg Met His Ser Pro Tyr
1               5                   10                  15
Leu Val Gly Leu Leu Gly Tyr Cys Ala Asp Gln Ser His Arg Leu Leu
            20                  25                  30
Val Phe Glu Phe Met Pro Asn Gly Ser Leu Lys Ser His Leu His Arg
        35                  40                  45
Arg Ala Leu Ala Pro Ala Glu Gln Pro Pro Pro Leu Asp Trp Gln Thr
    50                  55                  60
Arg Leu Gly Ile Ala Leu Asp Cys Ala Arg Ala Leu Glu Phe Leu His
65                  70                  75                  80
Glu His Ser Ser Pro Ala Val Ile His Arg Asp Phe Lys Cys Ser Asn
                85                  90                  95
Ile Leu Leu Asp His Asn Tyr Arg Ala Arg Val Ser Asp Phe Gly Met
            100                 105                 110
Gly Lys Leu Gly Ser Asn Lys Ala Asn Gly Gln
        115                 120
<210>  27
<211>  1233
<212>  DNA
<213>  Oryza sativa
<400>  27
atggcggatg cttgggcggc gtcccctcca tctcctacgg ctccttcgcc tttcgccgcg      60
gacgacctcg tcgacgcgcg gctcgcgccg tggccgccgt tcgccccgtg gccggcgccc     120
gggcagctcc accacaaccg ccgcggcggc gggcacccga acccgctctt caccatcctg     180
ccggcctcgg cgctggcaat aggggtcgtg ctgcttgtcg ccgtggtcgt catcctggtg     240
atgacgcgtc ggtggaagcc cggggcggtg gacggcgccg gcggcgccag ctgcaacggc     300
gacaagcctg gcggcgcccc cgcgtccagc tgcggcagca gcgtccgcgg ctacaacaac     360
tccaggtact acgccgccgc cgccgccggg tgcatatatg gcggaaggct gggtttctcg     420
gtgcagccgc ggaaccgcgg cgcgcaagtg ttcacgtacc gggagctgga gagcgcgacg     480
gacgggttca gcgagtgcaa cgtggtgggc cgcggcgcgt acggcgtggt cttccgcggc     540
cggctcggcg acggcaccac ggccgccatc aagcggctca agatggacgg ccggcgcgag     600
ggcgagcgcg agttccgcat cgagatgggc gttgctatta ctgctcaggt cgacctgctg     660
agccggatgc actcgccgta cctggtgggg ttgctgggct actgcgccga ccagagccac     720
cgtctgctgg ttttcgagtt catgcccaac ggcagcctca agagccacct ccaccggcgc     780
gcgctggcgc cggcggagca gccgccgccg ctggactggc agacgcggct gggcatcgcg     840
ctggactgcg cccgcgcgct ggagttcctc cacgagcaca gctcccccgc cgtgatccac     900
cgcgacttca agtgcagcaa catcctcctc gaccacaact accgcgcccg cgtctccgac     960
ttcggcatgg ccaagctcgg ctccaacaag gccaatggcc aggtggccgc catcacggcg    1020
atgtgcatac agacgaaggc ggactaccgg ccgctgatga cggacgtggt gcagtcgctg    1080
atccccatcg tgaagagccc actcatgtcc tgcacctcca cgccgctgag gcccgcgcac    1140
gggcaccacc acgtcgtcta catgagcccg agcaggggct cttccaacgg cggtgccctg    1200
gaaacgcgat gcgtcatgca cggcttggat tga                                 1233
<210>  28
<211>  410
<212>  PRT
```

<213> Oryza sativa
<400> 28

```
Met Ala Asp Ala Trp Ala Ala Ser Pro Pro Ser Pro Thr Ala Pro Ser
1               5                   10                  15
Pro Phe Ala Ala Asp Asp Leu Val Asp Ala Arg Leu Ala Pro Trp Pro
            20                  25                  30
Pro Phe Ala Pro Trp Pro Ala Pro Gly Gln Leu His His Asn Arg Arg
        35                  40                  45
Gly Gly Gly His Pro Asn Pro Leu Phe Thr Ile Leu Pro Ala Ser Ala
    50                  55                  60
Leu Ala Ile Gly Val Val Leu Leu Val Ala Val Val Val Ile Leu Val
65                  70                  75                  80
Met Thr Arg Arg Trp Lys Pro Gly Ala Val Asp Gly Ala Gly Gly Ala
                85                  90                  95
Ser Cys Asn Gly Asp Lys Pro Gly Gly Ala Pro Ala Ser Ser Cys Gly
            100                 105                 110
Ser Ser Val Arg Gly Tyr Asn Asn Ser Arg Tyr Tyr Ala Ala Ala Ala
        115                 120                 125
Ala Gly Cys Ile Tyr Gly Gly Arg Leu Gly Phe Ser Val Gln Pro Arg
    130                 135                 140
Asn Arg Gly Ala Gln Val Phe Thr Tyr Arg Glu Leu Glu Ser Ala Thr
145                 150                 155                 160
Asp Gly Phe Ser Glu Cys Asn Val Val Gly Arg Gly Ala Tyr Gly Val
                165                 170                 175
Val Phe Arg Gly Arg Leu Gly Asp Gly Thr Thr Ala Ala Ile Lys Arg
            180                 185                 190
Leu Lys Met Asp Gly Arg Arg Glu Gly Glu Arg Glu Phe Arg Ile Glu
        195                 200                 205
Met Gly Val Ala Ile Thr Ala Gln Val Asp Leu Leu Ser Arg Met His
    210                 215                 220
Ser Pro Tyr Leu Val Gly Leu Leu Gly Tyr Cys Ala Asp Gln Ser His
225                 230                 235                 240
Arg Leu Leu Val Phe Glu Phe Met Pro Asn Gly Ser Leu Lys Ser His
                245                 250                 255
Leu His Arg Arg Ala Leu Ala Pro Ala Glu Gln Pro Pro Pro Leu Asp
            260                 265                 270
Trp Gln Thr Arg Leu Gly Ile Ala Leu Asp Cys Ala Arg Ala Leu Glu
        275                 280                 285
Phe Leu His Glu His Ser Ser Pro Ala Val Ile His Arg Asp Phe Lys
    290                 295                 300
Cys Ser Asn Ile Leu Leu Asp His Asn Tyr Arg Ala Arg Val Ser Asp
305                 310                 315                 320
Phe Gly Met Ala Lys Leu Gly Ser Asn Lys Ala Asn Gly Gln Val Ala
                325                 330                 335
Ala Ile Thr Ala Met Cys Ile Gln Thr Lys Ala Asp Tyr Arg Pro Leu
            340                 345                 350
Met Thr Asp Val Val Gln Ser Leu Ile Pro Ile Val Lys Ser Pro Leu
        355                 360                 365
Met Ser Cys Thr Ser Thr Pro Leu Arg Pro Ala His Gly His His His
    370                 375                 380
Val Val Tyr Met Ser Pro Ser Arg Gly Ser Ser Asn Gly Gly Ala Leu
385                 390                 395                 400
Glu Thr Arg Cys Val Met His Gly Leu Asp
                405                 410
```

<210> 29
<211> 1365
<212> DNA
<213> Oryza sativa
<400> 29

```
atgtcgagcg gcggcggcgg cggcgacgac tacaagcggg aggagtcggt ggcactcatg      60
gtcatcgtct ccctcgcggc gctctccctc ctctccctcg tcgccgcctt cgcctactac     120
tgctacatca cccgcaaggt ctcccgccgc ctccactcgc tcgacctccc caagcaccac     180
cgccgctcct cctcctcctc gcctcctccc atgccccgc cgctgcctcc ccctcctcct      240
tccgcgaatg ccccgacgct cgggaaggag agcccgtcca gcaactccgc cagcgacggc     300
gcggcggcgg cggtcgtggt cggcggggag agggggccg tccaggtgtt cagctaccgc      360
cagctgcacg ccgccacggg cgggttcggg cgggcgcacg tggtggggca ggggagcttc     420
ggggccgtct accgcggggt gctccccgat gggaggaagg tcgcggtcaa gctcatggat     480
cgccccggca agcaggggga agaggagttc gagatggagg tggagctgct gagtcggctt     540
cgatcacctt atcttttggg cttgattggc cattgttcag agggtggaca ccggctgctg     600
gtctatgaat tcatggccaa tgggggtctc caggagcatc tctacccaaa tggagctttt     660
gaaaagattg aaacattttc gatctacttg gtgaaacaac gcccgatttt tgacaaaaat     720
```

```
atcgggcacc cgatttgtag gcgcgcccat ttttctcgtc aactgatatc ccacaaagct    780
gacattgtag gttcctgtgg tggtatctca aaattggact ggcctactag aatgagaata    840
gctcttgaag cagcaaaagg tctggaatat cttcacgagc gtgttaatcc acctgttata    900
cacagagact tcaagagcag caacattcta ctggacaagg acttccgtgc aagagtgtca    960
gattttggtt tggctaagct tggatctgat agagctggtg gtcatgtatc aactcgagtt   1020
ctaggcacac aaggttatgt tgcaccagat tacggtgttg tacttctgga gttgcttact   1080
ggcagggtgc cagttgatat gaagaggcct ccaggcgaag gtgttctagt taactgggca   1140
ttgcctatgc tcacagaccg agagaaggtt gtgcagatct tggatcctgc actggagggt   1200
caatattcat tgaaagatgc tgtccaagtg gctgccattg ctgccatgtg tgttcaacaa   1260
gaggctgact acaggccact aatggctgat gtggttcaat cgttggttcc gcttgtcaag   1320
aatcgttcta ctccaaagac gtgcaaccct agtgtccaag cgtag                   1365
```

<210> 30
<211> 454
<212> PRT
<213> Oryza sativa
<400> 30

```
Met Ser Ser Gly Gly Gly Gly Gly Asp Asp Tyr Lys Arg Glu Glu Ser
1               5                   10                  15
Val Ala Leu Met Val Ile Val Ser Leu Ala Ala Leu Ser Leu Leu Ser
            20                  25                  30
Leu Val Ala Ala Phe Ala Tyr Tyr Cys Tyr Ile Thr Arg Lys Val Ser
        35                  40                  45
Arg Arg Leu His Ser Leu Asp Leu Pro Lys His His Arg Arg Ser Ser
    50                  55                  60
Ser Ser Ser Pro Pro Pro Met Pro Pro Pro Leu Pro Pro Pro Pro Pro
65                  70                  75                  80
Ser Ala Asn Ala Pro Thr Leu Gly Lys Glu Ser Pro Ser Ser Asn Ser
                85                  90                  95
Ala Ser Asp Gly Ala Ala Ala Ala Val Val Val Gly Gly Glu Arg Gly
            100                 105                 110
Ala Val Gln Val Phe Ser Tyr Arg Gln Leu His Ala Ala Thr Gly Gly
        115                 120                 125
Phe Gly Arg Ala His Val Val Gly Gln Gly Ser Phe Gly Ala Val Tyr
    130                 135                 140
Arg Gly Val Leu Pro Asp Gly Arg Lys Val Ala Val Lys Leu Met Asp
145                 150                 155                 160
Arg Pro Gly Lys Gln Gly Glu Glu Glu Phe Glu Met Glu Val Glu Leu
                165                 170                 175
Leu Ser Arg Leu Arg Ser Pro Tyr Leu Leu Gly Leu Ile Gly His Cys
            180                 185                 190
Ser Glu Gly Gly His Arg Leu Leu Val Tyr Glu Phe Met Ala Asn Gly
        195                 200                 205
Gly Leu Gln Glu His Leu Tyr Pro Asn Gly Ala Phe Glu Lys Ile Glu
    210                 215                 220
Thr Phe Ser Ile Tyr Leu Val Lys Gln Arg Pro Ile Phe Asp Lys Asn
225                 230                 235                 240
Ile Gly His Pro Ile Cys Arg Arg Ala His Phe Ser Arg Gln Leu Ile
                245                 250                 255
Ser His Lys Ala Asp Ile Val Gly Ser Cys Gly Gly Ile Ser Lys Leu
            260                 265                 270
Asp Trp Pro Thr Arg Met Arg Ile Ala Leu Glu Ala Ala Lys Gly Leu
        275                 280                 285
Glu Tyr Leu His Glu Arg Val Asn Pro Pro Val Ile His Arg Asp Phe
    290                 295                 300
Lys Ser Ser Asn Ile Leu Leu Asp Lys Asp Phe Arg Ala Arg Val Ser
305                 310                 315                 320
Asp Phe Gly Leu Ala Lys Leu Gly Ser Asp Arg Ala Gly Gly His Val
                325                 330                 335
Ser Thr Arg Val Leu Gly Thr Gln Gly Tyr Val Ala Pro Asp Tyr Gly
            340                 345                 350
Val Val Leu Leu Glu Leu Leu Thr Gly Arg Val Pro Val Asp Met Lys
        355                 360                 365
Arg Pro Pro Gly Glu Gly Val Leu Val Asn Trp Ala Leu Pro Met Leu
    370                 375                 380
Thr Asp Arg Glu Lys Val Val Gln Ile Leu Asp Pro Ala Leu Glu Gly
385                 390                 395                 400
Gln Tyr Ser Leu Lys Asp Ala Val Gln Val Ala Ala Ile Ala Ala Met
                405                 410                 415
Cys Val Gln Gln Glu Ala Asp Tyr Arg Pro Leu Met Ala Asp Val Val
            420                 425                 430
Gln Ser Leu Val Pro Leu Val Lys Asn Arg Ser Thr Pro Lys Thr Cys
```

```
        435                 440                 445
Asn Pro Ser Val Gln Ala
    450
<210>  31
<211>  1923
<212>  DNA
<213>  Oryza sativa
<400>  31
ggcccccaaa cccctcaaaa ccctcgtcgt cttctcgcga tcgctattcc ctcctccatt      60
cctcctcctc cacctccgag acctagggtt ccaatgtcga gcggcggcgg cggcggcgac     120
gactacaagc gggaggagtc ggtggcactc atggtcatcg tctccctcgc ggcgctctcc     180
ctcctctccc tcgtcgccgc cttcgcctac tactgctaca tcacccgcaa ggtctcccgc     240
cgcctccact cgctcgacct ccccaagcac caccgccgct cctcctcctc ctcgcctcct     300
cccatgcccc cgccgctgcc tccccctcct ccttccgcga atgccccgac gctcgggaag     360
gagagccccgt ctagcaactc cgccagcgac ggcgcggcgg cggcggtcgt ggtcggcggg     420
gagagggggg ccgtccaggt gttcagctac cgccagctgc acgccgccac gggcgggttc     480
gggcgggcgc acgtggtggg gcaggggagc ttcggggccg tctaccgcgg ggtgctcccc     540
gatgggagga aggtcgcggt caagctcatg gatcgccccg gcaagcaggg ggaagaggag     600
ttcgagatgg aggtggagct gctgagtcgg cttcgatcac cttatctttt gggcttgatt     660
ggccattgtt cagagggtgg acaccggctg ctggtctatg aattcatggc caatgggggt     720
ctccaggagc atctctaccc aaatggaggt tcctgtggtg gtatctcaaa attggactgg     780
cctactagaa tgagaatagc tcttgaagca gcaaaaggtc tggaatatct tcacgagcgt     840
gttaatccac ctgttataca cagagacttc aagagcagca acattctact ggacaaggac     900
ttccgtgcaa gagtgtcaga ttttggtttg gctaagcttg gatctgatag agctggtggt     960
catgtatcaa ctcgagttct aggcacacaa ggttatgttg caccagaata tgctttgacc    1020
gggcatttga cgaccaaatc cgatgtctat agttacggtg ttgtacttct ggagttgctt    1080
actggcaggg tgccagttga tatgaagagg cctccaggcg aaggtgttct agttaactgg    1140
gcattgccta tgctcacaga ccgagagaag gttgtgcaga tcttggatcc tgcactggag    1200
ggtcaatatt cattgaaaga tgctgtccaa gtggctgcca ttgctgccat gtgtgtttaa    1260
caagaggctg actacaggcc actaatggct gatgtggttc aatcgttggt tccgcttgtc    1320
aagaatcgtt ctactccaaa gacgtgcaac cctagtgtcc aagcgtagaa gccacttgaa    1380
ggggaagttg aatgctcagt ttccaggttg gtgcttgact tttctggaaa ttttcatcta    1440
catctaacaa ctactgctga gtttatgaga tcagtatgct ccataactta attctcttcc    1500
tctgccagtg aattcctgat tttgcgaggt gaatcgtaag cagttagatt tagaaatcaa    1560
gcattaattt agttcgatgg accagaaagc tagtttcagt ttgaagggaa gtcctctagt    1620
catgtgcaat tagtgatcat gttaggtggt ttggcttagt acttatggtt gtgccctgtg    1680
ggaatgatca gagagtgttg tttccagctg gcagaattgc aatgtactgc tgaatttttct   1740
tttggcttgt caattatgtt aggtgtcttt ggagatccat gttcgttgtc ttaactcttg    1800
tgctaatacg ttggtctcat cagcttggcc tataaacatc gccgatgtat ctttttgtat    1860
atgtatatag taggattctg gaacttataa aaacaaacat ttgccagttg agcattttca    1920
tgc                                                                  1923
<210>  32
<211>  419
<212>  PRT
<213>  Oryza sativa
<400>  32
Gly Pro Gln Thr Pro Gln Asn Pro Arg Arg Leu Leu Ala Ile Ala Ile
1               5                   10                  15
Pro Ser Ser Ile Pro Pro Pro Pro Pro Pro Arg Pro Arg Val Pro Met
            20                  25                  30
Ser Ser Gly Gly Gly Gly Gly Asp Asp Tyr Lys Arg Glu Glu Ser Val
        35                  40                  45
Ala Leu Met Val Ile Val Ser Leu Ala Ala Leu Ser Leu Leu Ser Leu
    50                  55                  60
Val Ala Ala Phe Ala Tyr Tyr Cys Tyr Ile Thr Arg Lys Val Ser Arg
65                  70                  75                  80
Arg Leu His Ser Leu Asp Leu Pro Lys His His Arg Arg Ser Ser Ser
                85                  90                  95
Ser Ser Pro Pro Pro Met Pro Pro Pro Leu Pro Pro Pro Pro Pro Ser
            100                 105                 110
Ala Asn Ala Pro Thr Leu Gly Lys Glu Ser Pro Ser Ser Asn Ser Ala
        115                 120                 125
Ser Asp Gly Ala Ala Ala Ala Val Val Val Gly Gly Glu Arg Gly Ala
    130                 135                 140
Val Gln Val Phe Ser Tyr Arg Gln Leu His Ala Ala Thr Gly Gly Phe
145                 150                 155                 160
Gly Arg Ala His Val Val Gly Gln Gly Ser Phe Gly Ala Val Tyr Arg
                165                 170                 175
Gly Val Leu Pro Asp Gly Arg Lys Val Ala Val Lys Leu Met Asp Arg
            180                 185                 190
Pro Gly Lys Gln Gly Glu Glu Glu Phe Glu Met Glu Val Glu Leu Leu
```

```
        195                     200                     205
Ser Arg Leu Arg Ser Pro Tyr Leu Leu Gly Leu Ile Gly His Cys Ser
    210                     215                     220
Glu Gly Gly His Arg Leu Leu Val Tyr Glu Phe Met Ala Asn Gly Gly
225                     230                     235                     240
Leu Gln Glu His Leu Tyr Pro Asn Gly Gly Ser Cys Gly Gly Ile Ser
                245                     250                     255
Lys Leu Asp Trp Pro Thr Arg Met Arg Ile Ala Leu Glu Ala Ala Lys
            260                     265                     270
Gly Leu Glu Tyr Leu His Glu Arg Val Asn Pro Pro Val Ile His Arg
        275                     280                     285
Asp Phe Lys Ser Ser Asn Ile Leu Leu Asp Lys Asp Phe Arg Ala Arg
    290                     295                     300
Val Ser Asp Phe Gly Leu Ala Lys Leu Gly Ser Asp Arg Ala Gly Gly
305                     310                     315                     320
His Val Ser Thr Arg Val Leu Gly Thr Gln Gly Tyr Val Ala Pro Glu
                325                     330                     335
Tyr Ala Leu Thr Gly His Leu Thr Thr Lys Ser Asp Val Tyr Ser Tyr
            340                     345                     350
Gly Val Val Leu Leu Glu Leu Leu Thr Gly Arg Val Pro Val Asp Met
        355                     360                     365
Lys Arg Pro Pro Gly Glu Gly Val Leu Val Asn Trp Ala Leu Pro Met
    370                     375                     380
Leu Thr Asp Arg Glu Lys Val Val Gln Ile Leu Asp Pro Ala Leu Glu
385                     390                     395                     400
Gly Gln Tyr Ser Leu Lys Asp Ala Val Gln Val Ala Ala Ile Ala Ala
                405                     410                     415
Met Cys Val
```

<210> 33
<211> 3912
<212> DNA
<213> Oryza sativa
<400> 33

```
atggcgcgga tacggcgcgc gagctccgga cgagacatgt cagataattt ggtgcagcat     60
aacagacgct cagaagcaga aatttctact catgtagatg ctgcgcctcc tgatgcagcc    120
tcaaatacta gtgcagctcc ttctggatta gtacaacctc cagtatctcc tcacaatgca    180
tgttgttcac ataacatggt acaaaagaga gggagccaag attgccattg tgtttaccca    240
gtaagagttg agctttttct ccgaaatgtt tcactgacgt caaattggag cgatgaattt    300
ctgggggaac ttgcttctca actcagtctg agggttactc agtttgagat tgtaaatttc    360
tatgttgttg gggcttctgg gctaaacatc acaatgtata tagctcccca tacaggaatt    420
agtttctcag ccgaccaagt taccgcaatg aattattcac ttagtcagca tacagttcag    480
atcaaccctg tactagttgg tgactacaat cttcttaatt taacctggtt caggccactg    540
gttctagctc ctgtttattt tagtgttaag gtagagaaca ctaaaaggat gtttggtgat    600
atcaatatga tacttcctat gaatgatata aacattattt ctttatattt acagccctct    660
ccaacattca caatatcacc taaaccctct ccatctcaag catctacagt accaagacac    720
agtgcggata ccagcaatga aaaacacatg agcttgatta ctatcatttg catatttatt    780
ggtgctctaa ttgctgtctt ggtgattgcc atgtttattt gcttctgcaa attaaggaaa    840
gggaaaagga aggttcctcc agttgagaca cccaagcaaa gaacaccaga tgcggtttct    900
gcagtggact cacttcctcg cccaacaagt acaaggttcc ttgcatatga tgaactgaaa    960
gaagcaacca acaactttga tccgtcaagt atgcttggag aaggaggttt tggccgtgtc   1020
tttaaagggg tactaactga tggcactgcc gttgctataa agaagcttac tagtggaggg   1080
caccaaggag ataaggaatt tctagttgaa gtggagatgc tgagcaggtt gcaccaccga   1140
aaccttgtga aactcattgg ttactatagt aaccgtacat tgggagctag ccgccccttg   1200
gactgggaca ctagaatgag gatagcacta gatgctgcca ggggattggc ataccttcat   1260
gaggattctc agccttgcgt aatccacagg gatttcaagg cttctaatat attgcttgag   1320
gatgactttc atgctaaagt gtctgatttt ggtttggcca aacaggcacc ggaaggtcgc   1380
acaaattatc tctcaacacg tgttatggga acattcgggt atgtagcacc agagtatgca   1440
atgacaggac acttgcttgt aaaaagcgat gtatatagct atggagttgt tctacttgaa   1500
ctacttactg ggaggaggcc tgtggatatg tcacaacctt ctggccagga aaatctagtg   1560
acatggctta cacaaatgtt tcctgatctt tccactaaaa gccttgtgtc acatcaacct   1620
ttgctggcca agttgtcagg tgtagagata cttatttgct caattttgac tacgcaggca   1680
cgaccaattt tacgagataa agatacgcta gaagaactag ctgatcccaa gcttggtggc   1740
cagtatccaa aagatgattt tgtgcgagta tgcacaattg cagccgcctg tgtttcaccg   1800
gaggcaagcc aaaggccaac aatgggcgag gtggtgcagt cactgaagat ggtccaacgc   1860
tctgagttcc aggaatccat accaacacct ccagcacgac ccaatgcctc agcccctttg   1920
agactgagaa catatcgaga acggctttct ctgaagatct tcacgaaggg cggtaacagt   1980
ggtgaacaag acccggccac catggtcggc ttcgccgacc agaccgccga cgtctccgcc   2040
gccgcgttcc agaccatgta ccgcctcaac gtcggcggcg cctacatccc gccgtccaac   2100
gactccggcc tcacgcggcc gtggtacgac gacacgccgt tcgtccaggg cccccctgcgc   2160
ggcctcgtct acaacgccgg cccgcacttc cacatcaagt accccagcga cgccgccgag   2220
```

```
tacgccgcgc cgccggaggt gtacctcggc ggccgctcca tgggcaggga ccagcgcctc   2280
aaccagaact ccaacctcac ctggagcctc cacgtggagt gcaacttcac ctacgtcgtg   2340
cgcctccatt tctgcgagct ccagctcatc cacggcaacc agcgggtgtt cgacatctac   2400
atcaacaacc ggacggcgca gacggacgtg gacgtgctgg agatggcgac ggagcgcggc   2460
gtgccggtgt acaaggacta cgcggtgagg ctgagcaacg acaccgccga cgagcatctg   2520
tgggtggcgg tgcacccctc ggtgatgctc cgcccgcagt tctacgacgc catcctcaac   2580
ggcctcgagg tgttcaaggt gaacaacacc ggcggcagcc tggcgtcgcc ggaccccgtc   2640
ccgtacaagc tgctcgcgga gaaggagctc ggctggggcg ggccgccgga gttctccacc   2700
gacaacccgg ccaacatggc gtcggtgatg ggcggcacgg cgggcggcgc ggcggcggcc   2760
gggatcgtgg cggccatctg cgtggtggtg tacagcaaca agaggagcaa gaagctgggc   2820
ggcggcggcg ccgactcgca cacctccgcg tggctgccgc tgtaccactc ccacaccagc   2880
ggcaagtcgt cggggcacat cacggcgaac atcgccggca tgtgccgcca cttctcgttc   2940
gcggagatca aggcggcgac caagaacttc tccaacgacc tggccatcgg cgtgggcggg   3000
ttcggcgtgg tgtaccgcgg cgtggtggac ggcgacgtga aggtggcggt gaagcggtcc   3060
aacccgtcgt cggagcaagg cataaccgag ttccagacgg aggtggagat gctctccaag   3120
ctccgccacc gccacctcgt ctccctcatc ggcttctgcg aggaggacgg cgagatggtg   3180
ctcgtctacg actacatgga gcacggcacc ctgcgcgagc acctctacca caacggcggc   3240
aagcccacgc tgtcgtggcg ccaccgcctc gacatctgca tcggcgccgc ccgcggcctc   3300
cactacctcc acaccggcga atcgcacgtc agcaccgtcg tcaagggcag cttcggctac   3360
ctcgacccgg agtactaccg ccggcagcag ctcaccgaca agtccgacgt ctactccttc   3420
ggcgtcgtgc tgttcgaggt gctcatggcg cgcccggcgc tcgacccggc gctgccgcgc   3480
gaccaggtca gcctcgccga ctacgcgctc gcctgcaagc gcggcggcgc gctgccggac   3540
gtcgtcgacc cggcgatcag ggaccagatc gcgcccgagt gcctcgccaa gttcgccgac   3600
acggcggaga agtgcctctc cgagaacggc accgagcgcc ccaccatggg cgacgtgctc   3660
tggaacctcg agagcgcgat gcacttccag gacgcgttcg acgccgccgc cggccgcccc   3720
gtccccgcgc tcgacgccgc cgccggcagc agcagccacc tcgacgacgg gagcacggcc   3780
agcatcaaca cgctggccac gtcgtccacg tcgcacccgc acgagccgtg cgtcgacgtt   3840
gtcttggagc ccgacgacgt cgtcgcggag cgcgccacct tctcgcagct cgtccagccc   3900
accggccggt ga                                                       3912
```

<210> 34
<211> 1303
<212> PRT
<213> Oryza sativa
<400> 34

```
Met Ala Arg Ile Arg Arg Ala Ser Ser Gly Arg Asp Met Ser Asp Asn
1               5                   10                  15
Leu Val Gln His Asn Arg Arg Ser Glu Ala Glu Ile Ser Thr His Val
            20                  25                  30
Asp Ala Ala Pro Pro Asp Ala Ala Ser Asn Thr Ser Ala Ala Pro Ser
        35                  40                  45
Gly Leu Val Gln Pro Pro Val Ser Pro His Asn Ala Cys Cys Ser His
    50                  55                  60
Asn Met Val Gln Lys Arg Gly Ser Gln Asp Cys His Cys Val Tyr Pro
65                  70                  75                  80
Val Arg Val Glu Leu Phe Leu Arg Asn Val Ser Leu Thr Ser Asn Trp
                85                  90                  95
Ser Asp Glu Phe Leu Gly Glu Leu Ala Ser Gln Leu Ser Leu Arg Val
            100                 105                 110
Thr Gln Phe Glu Ile Val Asn Phe Tyr Val Val Gly Ala Ser Gly Leu
        115                 120                 125
Asn Ile Thr Met Tyr Ile Ala Pro His Thr Gly Ile Ser Phe Ser Ala
    130                 135                 140
Asp Gln Val Thr Ala Met Asn Tyr Ser Leu Ser Gln His Thr Val Gln
145                 150                 155                 160
Ile Asn Pro Val Leu Val Gly Asp Tyr Asn Leu Leu Asn Leu Thr Trp
                165                 170                 175
Phe Arg Pro Leu Val Leu Ala Pro Val Tyr Phe Ser Val Lys Val Glu
            180                 185                 190
Asn Thr Lys Arg Met Phe Gly Asp Ile Asn Met Ile Leu Pro Met Asn
        195                 200                 205
Asp Ile Asn Ile Ile Ser Leu Tyr Leu Gln Pro Ser Pro Thr Phe Thr
    210                 215                 220
Ile Ser Pro Lys Pro Ser Pro Ser Gln Ala Ser Thr Val Pro Arg His
225                 230                 235                 240
Ser Ala Asp Thr Ser Asn Glu Lys His Met Ser Leu Ile Thr Ile Ile
                245                 250                 255
Cys Ile Phe Ile Gly Ala Leu Ile Ala Val Leu Val Ile Ala Met Phe
            260                 265                 270
Ile Cys Phe Cys Lys Leu Arg Lys Gly Lys Arg Lys Val Pro Pro Val
        275                 280                 285
Glu Thr Pro Lys Gln Arg Thr Pro Asp Ala Val Ser Ala Val Asp Ser
```

```
    290                     295                     300
Leu Pro Arg Pro Thr Ser Thr Arg Phe Leu Ala Tyr Asp Glu Leu Lys
305                 310                 315                 320
Glu Ala Thr Asn Asn Phe Asp Pro Ser Ser Met Leu Gly Glu Gly Gly
                325                 330                 335
Phe Gly Arg Val Phe Lys Gly Val Leu Thr Asp Gly Thr Ala Val Ala
            340                 345                 350
Ile Lys Lys Leu Thr Ser Gly Gly His Gln Gly Asp Lys Glu Phe Leu
        355                 360                 365
Val Glu Val Glu Met Leu Ser Arg Leu His His Arg Asn Leu Val Lys
    370                 375                 380
Leu Ile Gly Tyr Tyr Ser Asn Arg Thr Leu Gly Ala Ser Arg Pro Leu
385                 390                 395                 400
Asp Trp Asp Thr Arg Met Arg Ile Ala Leu Asp Ala Ala Arg Gly Leu
                405                 410                 415
Ala Tyr Leu His Glu Asp Ser Gln Pro Cys Val Ile His Arg Asp Phe
            420                 425                 430
Lys Ala Ser Asn Ile Leu Leu Glu Asp Asp Phe His Ala Lys Val Ser
        435                 440                 445
Asp Phe Gly Leu Ala Lys Gln Ala Pro Glu Gly Arg Thr Asn Tyr Leu
    450                 455                 460
Ser Thr Arg Val Met Gly Thr Phe Gly Tyr Val Ala Pro Glu Tyr Ala
465                 470                 475                 480
Met Thr Gly His Leu Leu Val Lys Ser Asp Val Tyr Ser Tyr Gly Val
                485                 490                 495
Val Leu Leu Glu Leu Leu Thr Gly Arg Arg Pro Val Asp Met Ser Gln
            500                 505                 510
Pro Ser Gly Gln Glu Asn Leu Val Thr Trp Leu Thr Gln Met Phe Pro
        515                 520                 525
Asp Leu Ser Thr Lys Ser Leu Val Ser His Gln Pro Leu Leu Ala Lys
    530                 535                 540
Leu Ser Gly Val Glu Ile Leu Ile Cys Ser Ile Leu Thr Thr Gln Ala
545                 550                 555                 560
Arg Pro Ile Leu Arg Asp Lys Asp Thr Leu Glu Glu Leu Ala Asp Pro
                565                 570                 575
Lys Leu Gly Gly Gln Tyr Pro Lys Asp Asp Phe Val Arg Val Cys Thr
            580                 585                 590
Ile Ala Ala Ala Cys Val Ser Pro Glu Ala Ser Gln Arg Pro Thr Met
        595                 600                 605
Gly Glu Val Val Gln Ser Leu Lys Met Val Gln Arg Ser Glu Phe Gln
    610                 615                 620
Glu Ser Ile Pro Thr Pro Pro Ala Arg Pro Asn Ala Ser Ala Pro Leu
625                 630                 635                 640
Arg Leu Arg Thr Tyr Arg Glu Arg Leu Ser Leu Lys Ile Phe Thr Lys
                645                 650                 655
Gly Gly Asn Ser Gly Glu Gln Asp Pro Ala Thr Met Val Gly Phe Ala
            660                 665                 670
Asp Gln Thr Ala Asp Val Ser Ala Ala Ala Phe Gln Thr Met Tyr Arg
        675                 680                 685
Leu Asn Val Gly Gly Ala Tyr Ile Pro Pro Ser Asn Asp Ser Gly Leu
    690                 695                 700
Thr Arg Pro Trp Tyr Asp Asp Thr Pro Phe Val Gln Gly Pro Leu Arg
705                 710                 715                 720
Gly Leu Val Tyr Asn Ala Gly Pro His Phe His Ile Lys Tyr Pro Ser
                725                 730                 735
Asp Ala Ala Glu Tyr Ala Ala Pro Pro Glu Val Tyr Leu Gly Gly Arg
            740                 745                 750
Ser Met Gly Arg Asp Gln Arg Leu Asn Gln Asn Ser Asn Leu Thr Trp
        755                 760                 765
Ser Leu His Val Glu Cys Asn Phe Thr Tyr Val Val Arg Leu His Phe
    770                 775                 780
Cys Glu Leu Gln Leu Ile His Gly Asn Gln Arg Val Phe Asp Ile Tyr
785                 790                 795                 800
Ile Asn Asn Arg Thr Ala Gln Thr Asp Val Asp Val Leu Glu Met Ala
                805                 810                 815
Thr Glu Arg Gly Val Pro Val Tyr Lys Asp Tyr Ala Val Arg Leu Ser
            820                 825                 830
Asn Asp Thr Ala Asp Glu His Leu Trp Val Ala Val His Pro Ser Val
        835                 840                 845
Met Leu Arg Pro Gln Phe Tyr Asp Ala Ile Leu Asn Gly Leu Glu Val
    850                 855                 860
```

Phe Lys Val Asn Asn Thr Gly Gly Ser Leu Ala Ser Pro Asp Pro Val
865 870 875 880
Pro Tyr Lys Leu Leu Ala Glu Lys Glu Leu Gly Trp Gly Gly Pro Pro
885 890 895
Glu Phe Ser Thr Asp Asn Pro Ala Asn Met Ala Ser Val Met Gly Gly
900 905 910
Thr Ala Gly Gly Ala Ala Ala Ala Gly Ile Val Ala Ala Ile Cys Val
915 920 925
Val Val Tyr Ser Asn Lys Arg Ser Lys Lys Leu Gly Gly Gly Gly Ala
930 935 940
Asp Ser His Thr Ser Ala Trp Leu Pro Leu Tyr His Ser His Thr Ser
945 950 955 960
Gly Lys Ser Ser Gly His Ile Thr Ala Asn Ile Ala Gly Met Cys Arg
965 970 975
His Phe Ser Phe Ala Glu Ile Lys Ala Ala Thr Lys Asn Phe Ser Asn
980 985 990
Asp Leu Ala Ile Gly Val Gly Gly Phe Gly Val Val Tyr Arg Gly Val
995 1000 1005
Val Asp Gly Asp Val Lys Val Ala Val Lys Arg Ser Asn Pro Ser
1010 1015 1020
Ser Glu Gln Gly Ile Thr Glu Phe Gln Thr Glu Val Glu Met Leu
1025 1030 1035
Ser Lys Leu Arg His Arg His Leu Val Ser Leu Ile Gly Phe Cys
1040 1045 1050
Glu Glu Asp Gly Glu Met Val Leu Val Tyr Asp Tyr Met Glu His
1055 1060 1065
Gly Thr Leu Arg Glu His Leu Tyr His Asn Gly Gly Lys Pro Thr
1070 1075 1080
Leu Ser Trp Arg His Arg Leu Asp Ile Cys Ile Gly Ala Ala Arg
1085 1090 1095
Gly Leu His Tyr Leu His Thr Gly Glu Ser His Val Ser Thr Val
1100 1105 1110
Val Lys Gly Ser Phe Gly Tyr Leu Asp Pro Glu Tyr Tyr Arg Arg
1115 1120 1125
Gln Gln Leu Thr Asp Lys Ser Asp Val Tyr Ser Phe Gly Val Val
1130 1135 1140
Leu Phe Glu Val Leu Met Ala Arg Pro Ala Leu Asp Pro Ala Leu
1145 1150 1155
Pro Arg Asp Gln Val Ser Leu Ala Asp Tyr Ala Leu Ala Cys Lys
1160 1165 1170
Arg Gly Gly Ala Leu Pro Asp Val Val Asp Pro Ala Ile Arg Asp
1175 1180 1185
Gln Ile Ala Pro Glu Cys Leu Ala Lys Phe Ala Asp Thr Ala Glu
1190 1195 1200
Lys Cys Leu Ser Glu Asn Gly Thr Glu Arg Pro Thr Met Gly Asp
1205 1210 1215
Val Leu Trp Asn Leu Glu Ser Ala Met His Phe Gln Asp Ala Phe
1220 1225 1230
Asp Ala Ala Ala Gly Arg Pro Val Pro Ala Leu Asp Ala Ala Ala
1235 1240 1245
Gly Ser Ser Ser His Leu Asp Asp Gly Ser Thr Ala Ser Ile Asn
1250 1255 1260
Thr Leu Ala Thr Ser Ser Thr Ser His Pro His Glu Pro Cys Val
1265 1270 1275
Asp Val Val Leu Glu Pro Asp Asp Val Val Ala Glu Arg Ala Thr
1280 1285 1290
Phe Ser Gln Leu Val Gln Pro Thr Gly Arg
1295 1300

<210> 35
<211> 2463
<212> DNA
<213> Oryza sativa
<400> 35

atggcgcgga tacggcgcgc gagctccgga cgagctcaac tctcaggccg tgagaatttg 60
ggtttggtgt cgcgggaatg gtggtcatac tactatgtag gattcagtgc tctctgctgc 120
cgtggcgata ctggatatct ggaatctgat cgaagaacct ctgatctaag tttgagtcac 180
tacaaatgga tttgtgatat tgattatcaa attcaggcca atggatcctc ctttacaaga 240
aaatggtcat tactgaactc ttgttactgg aatgatgaga attcctgcat tgctgggttt 300
gcggaaaggc ttttggaaga tgctcgaagg aaaaccttct taaatttttt ggctgtggta 360
tttactctgg aattgattac cagaatcaat ttgattgccc aaacatcagc tctaaatgtg 420
gttgcccctg ctatatctcc atctcaaagt tggagaccag ttcgctccat gttgtcaaaa 480

```
gctaaagttg acattagtat ttcagtcagc gaacaaagac gaaagaagct atattcatca     540
ccagctactc tatctgtgca ccctccaatg tcagcgccaa gctatagctc catttctgac     600
atgtcagata atttggtgca gcataacaga cgctcagaag cagaaatttc tactcatgta     660
gatgctgcgc ctcctgatgc agcctcaaat actagtgcag ctccttctgg attagtacaa     720
cctccagtat ctcctcacaa tgcatgttgt tcacataaca tggtacaaaa gagagggagc     780
caagattgcc attgtgttta cccagtaaga gttgagcttt ttctccgaaa tgtttcactg     840
acgtcaaatt ggagcgatga atttctgggg gaacttgctt ctcaactcag tctgagggtt     900
actcagtttg agattgtaaa tttctatgtt gttggggctt ctgggctaaa catcacaatg     960
tatatagctc cccatacagg aattagtttc tcagccgacc aagttaccgc aatgaattat    1020
tcacttagtc agcatacagt tcagatcaac cctgtactag ttggtgacta caatcttctt    1080
aatttaacct ggttcaggcc actggttcta gctcctgctc caacattcac aatatcacct    1140
aaaccctctc catctcaagc atctacagta ccaagacaca gtgcggatac cagcaatgaa    1200
aaacacatga gcttgattac tatcatttgc atatttattg gtgctctaat tgctgtcttg    1260
gtgattgcca tgtttatttg cttctgcaaa ttaaggaaag ggaaaaggaa ggttcctcca    1320
gttgagacac ccaagcaaag aacaccagat gcggtttctg cagtggactc acttcctcgc    1380
ccaacaagta caaggttcct tgcatatgat gaactgaaag aagcaaccaa caactttgat    1440
ccgtcaagta tgcttggaga aggaggtttt ggccgtgtct ttaaaggggt actaactgat    1500
ggcactgccg ttgctataaa gaagcttact agtggagggc accaaggaga taaggaattt    1560
ctagttgaag tggagatgct gagcaggttg caccaccgaa accttgtgaa actcattggt    1620
tactatagta accgtgagtc atcacagaac ctactgtgct atgagcttgt tcctaatgga    1680
agcctagagg cttggcttca tggtacattg ggagctagcc gccccttgga ctgggacact    1740
agaatgagga tagcactaga tgctgccagg ggattggcat accttcatga ggattctcag    1800
ccttgcgtaa tccacaggga tttcaaggct tctaatatat tgcttgagga tgactttcat    1860
gctaaagtgt ctgattttgg tttggccaaa caggcaccgg aaggttgcac aaattatctc    1920
tcaacacgtg ttatgggaac attcgggtat gtagcaccag agtatgcaat gacaggacac    1980
ttgcttgtaa aaagcgatgt atatagctat ggagttgttc tacttgaact acttactggg    2040
aggaggcctg tggatatgtc acaaccttct ggccaggaaa atctagtgac atgggcacga    2100
ccaattttac gagataaaga tacgctagaa gaactagctg atcccaagct tggtggccag    2160
tatccaaaag atgattttgt gcgagtatgc acaattgcag ccgcctgtgt ttcaccggag    2220
gcaagccaaa ggccaacaat gggcgaggtg gtgcagtcac tgaagatggt ccaacgctct    2280
gagttccagg aatccatacc aacacctcca gcacgaccca atgttaggca gtcctcaacc    2340
acctatgaat ctgatggcac ttcatccatg ttctcttctg gaccctttttc aggcctcagc    2400
ccctttgaga ctgagaacat atcgagaacg gctttctctg aagatcttca cgaagggcgg    2460
taa                                                                  2463
```

<210> 36

<211> 820

<212> PRT

<213> Oryza sativa

<400> 36

```
Met Ala Arg Ile Arg Arg Ala Ser Ser Gly Arg Ala Gln Leu Ser Gly
1               5                   10                  15
Arg Glu Asn Leu Gly Leu Val Ser Arg Glu Trp Trp Ser Tyr Tyr Tyr
            20                  25                  30
Val Gly Phe Ser Ala Leu Cys Cys Arg Gly Asp Thr Gly Tyr Leu Glu
        35                  40                  45
Ser Asp Arg Arg Thr Ser Asp Leu Ser Leu Ser His Tyr Lys Trp Ile
    50                  55                  60
Cys Asp Ile Asp Tyr Gln Ile Gln Ala Asn Gly Ser Ser Phe Thr Arg
65                  70                  75                  80
Lys Trp Ser Leu Leu Asn Ser Cys Tyr Trp Asn Asp Glu Asn Ser Cys
                85                  90                  95
Ile Ala Gly Phe Ala Glu Arg Leu Leu Glu Asp Ala Arg Arg Lys Thr
            100                 105                 110
Phe Leu Asn Phe Leu Ala Val Val Phe Thr Leu Glu Leu Ile Thr Arg
        115                 120                 125
Ile Asn Leu Ile Ala Gln Thr Ser Ala Leu Asn Val Val Ala Pro Ala
    130                 135                 140
Ile Ser Pro Ser Gln Ser Trp Arg Pro Val Arg Ser Met Leu Ser Lys
145                 150                 155                 160
Ala Lys Val Asp Ile Ser Ile Ser Val Ser Glu Gln Arg Arg Lys Lys
                165                 170                 175
Leu Tyr Ser Ser Pro Ala Thr Leu Ser Val His Pro Pro Met Ser Ala
            180                 185                 190
Pro Ser Tyr Ser Ser Ile Ser Asp Met Ser Asp Asn Leu Val Gln His
        195                 200                 205
Asn Arg Arg Ser Glu Ala Glu Ile Ser Thr His Val Asp Ala Ala Pro
    210                 215                 220
Pro Asp Ala Ala Ser Asn Thr Ser Ala Ala Pro Ser Gly Leu Val Gln
225                 230                 235                 240
Pro Pro Val Ser Pro His Asn Ala Cys Cys Ser His Asn Met Val Gln
                245                 250                 255
```

```
Lys Arg Gly Ser Gln Asp Cys His Cys Val Tyr Pro Val Arg Val Glu
            260                 265                 270
Leu Phe Leu Arg Asn Val Ser Leu Thr Ser Asn Trp Ser Asp Glu Phe
        275                 280                 285
Leu Gly Glu Leu Ala Ser Gln Leu Ser Leu Arg Val Thr Gln Phe Glu
    290                 295                 300
Ile Val Asn Phe Tyr Val Val Gly Ala Ser Gly Leu Asn Ile Thr Met
305                 310                 315                 320
Tyr Ile Ala Pro His Thr Gly Ile Ser Phe Ser Ala Asp Gln Val Thr
                325                 330                 335
Ala Met Asn Tyr Ser Leu Ser Gln His Thr Val Gln Ile Asn Pro Val
            340                 345                 350
Leu Val Gly Asp Tyr Asn Leu Leu Asn Leu Thr Trp Phe Arg Pro Leu
        355                 360                 365
Val Leu Ala Pro Ala Pro Thr Phe Thr Ile Ser Pro Lys Pro Ser Pro
    370                 375                 380
Ser Gln Ala Ser Thr Val Pro Arg His Ser Ala Asp Thr Ser Asn Glu
385                 390                 395                 400
Lys His Met Ser Leu Ile Thr Ile Ile Cys Ile Phe Ile Gly Ala Leu
                405                 410                 415
Ile Ala Val Leu Val Ile Ala Met Phe Ile Cys Phe Cys Lys Leu Arg
            420                 425                 430
Lys Gly Lys Arg Lys Val Pro Pro Val Glu Thr Pro Lys Gln Arg Thr
        435                 440                 445
Pro Asp Ala Val Ser Ala Val Asp Ser Leu Pro Arg Pro Thr Ser Thr
    450                 455                 460
Arg Phe Leu Ala Tyr Asp Glu Leu Lys Glu Ala Thr Asn Asn Phe Asp
465                 470                 475                 480
Pro Ser Ser Met Leu Gly Glu Gly Gly Phe Gly Arg Val Phe Lys Gly
                485                 490                 495
Val Leu Thr Asp Gly Thr Ala Val Ala Ile Lys Lys Leu Thr Ser Gly
            500                 505                 510
Gly His Gln Gly Asp Lys Glu Phe Leu Val Glu Val Glu Met Leu Ser
        515                 520                 525
Arg Leu His His Arg Asn Leu Val Lys Leu Ile Gly Tyr Tyr Ser Asn
    530                 535                 540
Arg Glu Ser Ser Gln Asn Leu Leu Cys Tyr Glu Leu Val Pro Asn Gly
545                 550                 555                 560
Ser Leu Glu Ala Trp Leu His Gly Thr Leu Gly Ala Ser Arg Pro Leu
                565                 570                 575
Asp Trp Asp Thr Arg Met Arg Ile Ala Leu Asp Ala Ala Arg Gly Leu
            580                 585                 590
Ala Tyr Leu His Glu Asp Ser Gln Pro Cys Val Ile His Arg Asp Phe
        595                 600                 605
Lys Ala Ser Asn Ile Leu Leu Glu Asp Asp Phe His Ala Lys Val Ser
    610                 615                 620
Asp Phe Gly Leu Ala Lys Gln Ala Pro Glu Gly Cys Thr Asn Tyr Leu
625                 630                 635                 640
Ser Thr Arg Val Met Gly Thr Phe Gly Tyr Val Ala Pro Glu Tyr Ala
                645                 650                 655
Met Thr Gly His Leu Leu Val Lys Ser Asp Val Tyr Ser Tyr Gly Val
            660                 665                 670
Val Leu Leu Glu Leu Leu Thr Gly Arg Arg Pro Val Asp Met Ser Gln
        675                 680                 685
Pro Ser Gly Gln Glu Asn Leu Val Thr Trp Ala Arg Pro Ile Leu Arg
    690                 695                 700
Asp Lys Asp Thr Leu Glu Glu Leu Ala Asp Pro Lys Leu Gly Gly Gln
705                 710                 715                 720
Tyr Pro Lys Asp Asp Phe Val Arg Val Cys Thr Ile Ala Ala Ala Cys
                725                 730                 735
Val Ser Pro Glu Ala Ser Gln Arg Pro Thr Met Gly Glu Val Val Gln
            740                 745                 750
Ser Leu Lys Met Val Gln Arg Ser Glu Phe Gln Glu Ser Ile Pro Thr
        755                 760                 765
Pro Pro Ala Arg Pro Asn Val Arg Gln Ser Ser Thr Thr Tyr Glu Ser
    770                 775                 780
Asp Gly Thr Ser Ser Met Phe Ser Ser Gly Pro Phe Ser Gly Leu Ser
785                 790                 795                 800
Pro Phe Glu Thr Glu Asn Ile Ser Arg Thr Ala Phe Ser Glu Asp Leu
                805                 810                 815
His Glu Gly Arg
```

820

<210> 37
<211> 2670
<212> DNA
<213> Oryza sativa
<400> 37

```
atggggcggc gtggaggagg aggacgagcg ggaggcgcgt ggattggtgg ctgtgtactt      60
gcgctcgccg ccaccttggt cgtctgcgtg ctcgtgatcc gtggagctgg aggactaaat     120
gtaaaaccac ctgcagcaac tagcagacgt gttaacatac agcaggaact atatgcacca     180
agcccaacga tcagccacag aggtcttgct attcctccac ttccaacaac ttcatcccca     240
gttttcccac ctcccatcag atcttatcct ccacttcctg caaataagcc ataccccaat     300
agtccagttg tggcacctcc aaagggaagg catcaccatt ctttgcctgt aaataatacc     360
cgtgtaaaag gacctgccta ttctccttca aattctccca gtatccatag aaaacatggc     420
attccagttg ctgcacctcc aaagcaacat tccagcaatt taccaccttc acatcatagg     480
ccccacaaag gatcctttcc tgtcataagc cctactccac ataaagctga taatgcttct     540
gcgacgaaac atggacgttc tggcttacac catagtcctg cacctgcacc tgtaggcttg     600
cctccatctg aaggaaatgc acgaggaaat cctgcgtatg caccacgtca tccccatgaa     660
tatcactcgc cttcaaattc tccagaacct ggtctaccac ctgtgaatcc gcctgacagt     720
catgcattta aaaagcccaa gagtttggca ccagcacccc aatcatttcc gccaccgcct     780
ctgagttcat attgcatggc gttaaattgt caagatcctc tgaccaatag tctccctgga     840
actacatgtc tttgtgtgtg gccaataaaa gttgagcttc gtttaggtat agctttgtac     900
acattctttg cattggtatc agaacttgca caagatattg catctggagt gctcatgaaa     960
caaagtcaag ttcgtgtaat gggagcaaat gctgcaactg aggacccaga gaaaacagtt    1020
gtccttattg atcttgtgcc actgggagaa aaatttgaca aggcaacagc acttttagta    1080
tttgaaaggt tttggcacaa gcaggtcaac ataaactcta tgcattttgg aaactatgat    1140
gtgttatatg ttacctacca aggtcttcct ccgtcgccac caacagctcc cgggatgaac    1200
aatggtctta gcaatgttaa tgatccaagg ttgcatccac ttgctgttga tgtgggaaac    1260
cacagagaaa caaaaagcag ggcataattg ttataattg ttctttcaag tgtctttgct     1320
tttattttat gttctggagc tgcgttggta atttgtttca agattagaaa ccgcaaccat    1380
ttaactgaag agtcacctat gccaccgaag cctgcaggtc ctgggtctgc agttgttggg    1440
agcaggctag gaagcagacc tatttcagca tctccatctt tcagctcaag catagtgaca    1500
tataaaggga cagcgaaaac atttagcttg attgagatgg aaagagctac acaaagattt    1560
gacaactcca gaattattgg cgagggtggt tttggacgtg tttatgaagg tattcttgag    1620
gatggagaac gggttgctgt caaaattctt aagagggacg accagcaagg tacacgggaa    1680
tttttagctg aggttgagat gcttagccga ttgcatcaca gaaacctggt taagttgata    1740
ggtatatgca cagaagaaca tatccggtgt cttgtgtatg agcttgttcc aaatggtagt    1800
gtggaatctc acttgcacgg atcagataag ggaactgctc cactttattg ggatgctagg    1860
cttaaaattg cacttggtgc tgcacgtgct cttgcttatt tgcatgaaga ttctagtccc    1920
cgtgtcatac accgtgactt caagtcaagt aacatcttat tggaacatga cttcaccccc    1980
aaggtgtcag actttggcct agcaagaaca gccataggtg aggggaatga gcatatttca    2040
actcgtgtta tgggaacttt cgggtatgtt gctcctgaat acgcaatgac tgggcatctt    2100
ctagtaaaga gtgatgtcta cagctatggt gtcgtcctcc ttgagcttct taccggaagg    2160
aaaccggtgg atattttgag acctccaggg caagaaaatt tagttgcatg ggcttgtcct    2220
tttcttacta gcagagatgg cttggaaaca ataattgatc catcacttgg aaatagcatc    2280
ctatttgaca gcattgcaaa agtagcagct attgcttcta tgtgcgtgca gcctgaggtg    2340
gatcagcgcc catttatggg tgaagttgtt caagctttga agttggtatg cgatgaaggc    2400
agcgagttca atgaatcaag aagtttcagc caagatttac atattcagga ttctgggatt    2460
ataagtagag caagcctgga tgtggacgta gaacctgttg tatctgctga gctgttcaat    2520
gcatcagcac attatgacac tcttgatgca tctggttctt ttcgacgata ttcaagttca    2580
ggtcctctta gggtaggtag aaccgggcac aatagggggta gctcaagcga gcactgtggt   2640
acacaaagat tcaggataga ttcagagtag                                    2670
```

<210> 38
<211> 889
<212> PRT
<213> Oryza sativa
<400> 38

```
Met Gly Arg Arg Gly Gly Gly Gly Arg Ala Gly Gly Ala Trp Ile Gly
1               5                   10                  15
Gly Cys Val Leu Ala Leu Ala Ala Thr Leu Val Val Cys Val Leu Val
            20                  25                  30
Ile Arg Gly Ala Gly Gly Leu Asn Val Lys Pro Pro Ala Ala Thr Ser
        35                  40                  45
Arg Arg Val Asn Ile Gln Gln Glu Leu Tyr Ala Pro Ser Pro Thr Ile
    50                  55                  60
Ser His Arg Gly Leu Ala Ile Pro Pro Leu Pro Thr Thr Ser Ser Pro
65                  70                  75                  80
Val Phe Pro Pro Pro Ile Arg Ser Tyr Pro Pro Leu Pro Ala Asn Lys
                85                  90                  95
Pro Tyr Pro Asn Ser Pro Val Val Ala Pro Pro Lys Gly Arg His His
            100                 105                 110
His Ser Leu Pro Val Asn Asn Thr Arg Val Lys Gly Pro Ala Tyr Ser
```

```
        115                 120                 125
Pro Ser Asn Ser Pro Ser Ile His Arg Lys His Gly Ile Pro Val Ala
    130                 135                 140
Ala Pro Pro Lys Gln His Ser Ser Asn Leu Pro Pro Ser His His Arg
145                 150                 155                 160
Pro His Lys Gly Ser Phe Pro Val Ile Ser Pro Thr Pro His Lys Ala
                165                 170                 175
Asp Asn Ala Ser Ala Thr Lys His Gly Arg Ser Gly Leu His His Ser
            180                 185                 190
Pro Ala Pro Ala Pro Val Gly Leu Pro Pro Ser Glu Gly Asn Ala Arg
        195                 200                 205
Gly Asn Pro Ala Tyr Ala Pro Arg His Pro His Glu Tyr His Ser Pro
    210                 215                 220
Ser Asn Ser Pro Glu Pro Gly Leu Pro Pro Val Asn Pro Pro Asp Ser
225                 230                 235                 240
His Ala Phe Lys Lys Pro Lys Ser Leu Ala Pro Ala Pro Gln Ser Phe
                245                 250                 255
Pro Pro Pro Pro Leu Ser Ser Tyr Cys Met Ala Leu Asn Cys Gln Asp
            260                 265                 270
Pro Leu Thr Asn Ser Leu Pro Gly Thr Thr Cys Leu Cys Val Trp Pro
        275                 280                 285
Ile Lys Val Glu Leu Arg Leu Gly Ile Ala Leu Tyr Thr Phe Phe Ala
    290                 295                 300
Leu Val Ser Glu Leu Ala Gln Asp Ile Ala Ser Gly Val Leu Met Lys
305                 310                 315                 320
Gln Ser Gln Val Arg Val Met Gly Ala Asn Ala Ala Thr Glu Asp Pro
                325                 330                 335
Glu Lys Thr Val Val Leu Ile Asp Leu Val Pro Leu Gly Glu Lys Phe
            340                 345                 350
Asp Lys Ala Thr Ala Leu Leu Val Phe Glu Arg Phe Trp His Lys Gln
        355                 360                 365
Val Asn Ile Asn Ser Met His Phe Gly Asn Tyr Asp Val Leu Tyr Val
    370                 375                 380
Thr Tyr Gln Gly Leu Pro Pro Ser Pro Pro Thr Ala Pro Gly Met Asn
385                 390                 395                 400
Asn Gly Leu Ser Asn Val Asn Asp Pro Arg Leu His Pro Leu Ala Val
                405                 410                 415
Asp Val Gly Asn His Arg Glu Thr Lys Ser Arg Gly Ile Ile Val Ile
            420                 425                 430
Ile Val Leu Ser Ser Val Phe Ala Phe Ile Leu Cys Ser Gly Ala Ala
        435                 440                 445
Leu Val Ile Cys Phe Lys Ile Arg Asn Arg Asn His Leu Thr Glu Glu
    450                 455                 460
Ser Pro Met Pro Pro Lys Pro Ala Gly Pro Gly Ser Ala Val Val Gly
465                 470                 475                 480
Ser Arg Leu Gly Ser Arg Pro Ile Ser Ala Ser Pro Ser Phe Ser Ser
                485                 490                 495
Ser Ile Val Thr Tyr Lys Gly Thr Ala Lys Thr Phe Ser Leu Ile Glu
            500                 505                 510
Met Glu Arg Ala Thr Gln Arg Phe Asp Asn Ser Arg Ile Ile Gly Glu
        515                 520                 525
Gly Gly Phe Gly Arg Val Tyr Glu Gly Ile Leu Glu Asp Gly Glu Arg
    530                 535                 540
Val Ala Val Lys Ile Leu Lys Arg Asp Asp Gln Gln Gly Thr Arg Glu
545                 550                 555                 560
Phe Leu Ala Glu Val Glu Met Leu Ser Arg Leu His His Arg Asn Leu
                565                 570                 575
Val Lys Leu Ile Gly Ile Cys Thr Glu Glu His Ile Arg Cys Leu Val
            580                 585                 590
Tyr Glu Leu Val Pro Asn Gly Ser Val Glu Ser His Leu His Gly Ser
        595                 600                 605
Asp Lys Gly Thr Ala Pro Leu Tyr Trp Asp Ala Arg Leu Lys Ile Ala
    610                 615                 620
Leu Gly Ala Ala Arg Ala Leu Ala Tyr Leu His Glu Asp Ser Ser Pro
625                 630                 635                 640
Arg Val Ile His Arg Asp Phe Lys Ser Ser Asn Ile Leu Leu Glu His
                645                 650                 655
Asp Phe Thr Pro Lys Val Ser Asp Phe Gly Leu Ala Arg Thr Ala Ile
            660                 665                 670
Gly Glu Gly Asn Glu His Ile Ser Thr Arg Val Met Gly Thr Phe Gly
        675                 680                 685
```

```
Tyr Val Ala Pro Glu Tyr Ala Met Thr Gly His Leu Leu Val Lys Ser
    690                 695                 700
Asp Val Tyr Ser Tyr Gly Val Val Leu Leu Glu Leu Leu Thr Gly Arg
705                 710                 715                 720
Lys Pro Val Asp Ile Leu Arg Pro Pro Gly Gln Glu Asn Leu Val Ala
                725                 730                 735
Trp Ala Cys Pro Phe Leu Thr Ser Arg Asp Gly Leu Glu Thr Ile Ile
            740                 745                 750
Asp Pro Ser Leu Gly Asn Ser Ile Leu Phe Asp Ser Ile Ala Lys Val
        755                 760                 765
Ala Ala Ile Ala Ser Met Cys Val Gln Pro Glu Val Asp Gln Arg Pro
    770                 775                 780
Phe Met Gly Glu Val Val Gln Ala Leu Lys Leu Val Cys Asp Glu Gly
785                 790                 795                 800
Ser Glu Phe Asn Glu Ser Arg Ser Phe Ser Gln Asp Leu His Ile Gln
                805                 810                 815
Asp Ser Gly Ile Ile Ser Arg Ala Ser Leu Asp Val Asp Val Glu Pro
            820                 825                 830
Val Val Ser Ala Glu Leu Phe Asn Ala Ser Ala His Tyr Asp Thr Leu
        835                 840                 845
Asp Ala Ser Gly Ser Phe Arg Arg Tyr Ser Ser Ser Gly Pro Leu Arg
    850                 855                 860
Val Gly Arg Thr Gly His Asn Arg Gly Ser Ser Ser Glu His Cys Gly
865                 870                 875                 880
Thr Gln Arg Phe Arg Ile Asp Ser Glu
                885
```

<210> 39
<211> 2913
<212> DNA
<213> Oryza sativa
<400> 39

```
atgcggtcgc tcgcttgttg cgcggtgctt cttctcgctt cggttcttgg atctacaggt     60
actgatctgg gtccttctcc tgtggtcgct aactcgccag atgctcaaga tcaaacttct    120
agccctcctg aacctacaat cgccctcggt ccagtaactc ttccaacagg ttgctctgaa    180
tttctaatgt ggtgtggcat tatggctctc aagatcataa ccagcattga caattgtcct    240
tacaattgct ttgtctcagc accctctgct ccatcagcaa gccctcctgt ggcgaagggc    300
gctgtcagcc cagctgttcc cactcgacca caaaatgcgc ctactccagt aacacccccct   360
aaagaatata atgcgcctcc tccagttgag cttacgcctc ctgctcccac tcatgtggcg    420
ccagtagctc ccccgcaagc tgcagttgaa aatcctgcac cagtgcttcc tgggacacct    480
gctttgttgc cttctgttca ggctcctgct ccatctgtgg ccaggaatcc taatctaccg    540
atagtgcaac ctccttctgt gaacaatcca ccaagcggac caattggatc agggaatggc    600
gtccctccgt atccaccacc tcaaagaagt ttacctgcca ttcctccttc cacttcagga    660
gttccgcgag aaagtgtaaa acctccagtt gcaccaccta ttattgcaca agcaccacgt    720
cagcaagcac tggcaccaag tagtgaccat agcaatggaa actcagtgcc cccagcaaat    780
acatcacctc cccataagaa tagtcatatt ccacgtgcac tgccaccaaa ggaatccagt    840
agtcaaactg gcacagctca caaaccgcca attagagggc ctcatatttc tccaaccatg    900
ccaccaatcc ctccccaacc agggccgaaa gcaccatcag cccatcctat ttgggcatta    960
cctccaccac cgcctaatct agattgcaat tcattagcat gcccagagcc tctaacagat   1020
ccacctgcgg gagccccatg tgtttgtgtt ctaccaatca aagttggagt ccgcttaagt   1080
gttgatctgt attcattctt tccattggta tctgattttg cggaagaagt gtcatctggg   1140
gtaaatatgg cacagagaca ggttcgtgtt atgggtgcaa atgtagctgg tgatcagcct   1200
gacaagacag tagttcttgt tgacctagta ccaatgcaag tgaagtttga caatgctact   1260
gctttttttaa cgtttgaaaa cttatggagc aaaaaaattt ccctaaaacc atcagttttt  1320
ggggactacg agattctgta tgttgtatat ccagggcttc ctccttctcc accttcagca   1380
ccggaaagtg ttggtgacgg ggcatttgga aacaacagaa atgcaagggc aatgaagcct   1440
ctcggggttg atgtaggcag gcccaaaaaa agagtcaacg gcagcctaat tgctattgcc   1500
gtcttgtcta ctgttatagc attgattatt tgcactctag ctgcatggtt gctgataatc   1560
agattcaggg gttcggatgg cttggctcaa agatttccac atagcgcact tccaaaattt   1620
tccagatcat ctggtacagg tcaaacactg ttagctggtc gctatagttc accatcaggt   1680
ccatcaggtt cattgggctc aagtatcgca acatatgcag gacaggcaaa gacattcaaa   1740
tttgctgaga ttgagaaggc cacaaacagc tttgatgatt caacagtact tggagagggt   1800
ggcttcggat gtgtctacca gggcacgctt gaagatggaa ccagggttgc agtaaaggtt   1860
ctgaagaggt atgatggcca aggcgagaga gagttcttgg ctgaggttga gatgctggga   1920
cgcttgcatc accgaaattt ggtcaagttg ttaggcatat gtgtagagga gaacgcaaga   1980
tgtctggttt atgaacttat tccgaatggc agtgtagaat cccatttgca tggagtggat   2040
cttgagacag caccactcga ttggaatgcc cgcatgaaga tagccttggg ggctgcacga   2100
gctcttgcat atttacacga agattcaagc ccttgtgtga ttcatcgtga ttttaagtca   2160
agcaacatcc tattggagca tgatttcaca ccgaaagtgt ctgatttcgg actggccagg   2220
actgcaaggg gggaggggaa ccagcacatc tccactcgtg tcatgggaac atttggatat   2280
gttgcaccgg agtatgccat gacaggacat ctccttgtca agagcgatgt gtacagctat   2340
ggagtagtgt tgcttgagct cctcaccggt agaaagccag tggacatgtc taggcccggg   2400
```

```
gggcaagaaa acctagtttc atgggctcgc ccgcttctga ccaatgtggt aagcctacgt   2460
caagctgttg atccacttct tggccctaac gtacccctgg acaatgtcgc aaaagcagct   2520
gccatcgcat caatgtgtgt acaacctgag gttgcgcatc gcccgagcat gggtgaagta   2580
gtgcaggcct taaaacttgt ttgcagtgac ggtgatgagg gcctcggatc aggaagtttc   2640
agccaggaat tggcggcaca agctgcagca atctatgatg taactggcat ggaagctgag   2700
agggtgctgt tatctgagat gtttggctca acccctgtct tcactcccgc tgcggattca   2760
ggttctttcc gaaagcagtc cagctcaggt ccactgatga caggcaagaa cagaaagttc   2820
tggcagagat tgcgaagcct atcaagaggg agtatgagtg agcatggtgc ctcaccagat   2880
tttgagacgc gctcgcagtg tagtaatagg tga                                2913
<210>  40
<211>  970
<212>  PRT
<213>  Oryza sativa
<400>  40
Met Arg Ser Leu Ala Cys Cys Ala Val Leu Leu Leu Ala Ser Val Leu
1               5                   10                  15
Gly Ser Thr Gly Thr Asp Leu Gly Pro Ser Pro Val Val Ala Asn Ser
            20                  25                  30
Pro Asp Ala Gln Asp Gln Thr Ser Ser Pro Pro Glu Pro Thr Ile Ala
        35                  40                  45
Leu Gly Pro Val Thr Leu Pro Thr Gly Cys Ser Glu Phe Leu Met Trp
    50                  55                  60
Cys Gly Ile Met Ala Leu Lys Ile Ile Thr Ser Ile Asp Asn Cys Pro
65                  70                  75                  80
Tyr Asn Cys Phe Val Ser Ala Pro Ser Ala Pro Ser Ala Ser Pro Pro
                85                  90                  95
Val Ala Lys Gly Ala Val Ser Pro Ala Val Pro Thr Arg Pro Gln Asn
            100                 105                 110
Ala Pro Thr Pro Val Thr Pro Pro Lys Glu Tyr Asn Ala Pro Pro Pro
        115                 120                 125
Val Glu Leu Thr Pro Pro Ala Pro Thr His Val Ala Pro Val Ala Pro
    130                 135                 140
Pro Gln Ala Ala Val Glu Asn Pro Ala Pro Val Leu Pro Gly Thr Pro
145                 150                 155                 160
Ala Leu Leu Pro Ser Val Gln Ala Pro Ala Pro Ser Val Ala Arg Asn
                165                 170                 175
Pro Asn Leu Pro Ile Val Gln Pro Pro Ser Val Asn Asn Pro Pro Ser
            180                 185                 190
Gly Pro Ile Gly Ser Gly Asn Gly Val Pro Pro Tyr Pro Pro Pro Gln
        195                 200                 205
Arg Ser Leu Pro Ala Ile Pro Pro Ser Thr Ser Gly Val Pro Arg Glu
    210                 215                 220
Ser Val Lys Pro Pro Val Ala Pro Pro Ile Ile Ala Gln Ala Pro Arg
225                 230                 235                 240
Gln Gln Ala Leu Ala Pro Ser Ser Asp His Ser Asn Gly Asn Ser Val
                245                 250                 255
Pro Pro Ala Asn Thr Ser Pro Pro His Lys Asn Ser His Ile Pro Arg
            260                 265                 270
Ala Leu Pro Pro Lys Glu Ser Ser Ser Gln Thr Gly Thr Ala His Lys
        275                 280                 285
Pro Pro Ile Arg Gly Pro His Ile Ser Pro Thr Met Pro Pro Ile Pro
    290                 295                 300
Pro Gln Pro Gly Pro Lys Ala Pro Ser Ala His Pro Ile Trp Ala Leu
305                 310                 315                 320
Pro Pro Pro Pro Pro Asn Leu Asp Cys Asn Ser Leu Ala Cys Pro Glu
                325                 330                 335
Pro Leu Thr Asp Pro Pro Ala Gly Ala Pro Cys Val Cys Val Leu Pro
            340                 345                 350
Ile Lys Val Gly Val Arg Leu Ser Val Asp Leu Tyr Ser Phe Phe Pro
        355                 360                 365
Leu Val Ser Asp Phe Ala Glu Glu Val Ser Ser Gly Val Asn Met Ala
    370                 375                 380
Gln Arg Gln Val Arg Val Met Gly Ala Asn Val Ala Gly Asp Gln Pro
385                 390                 395                 400
Asp Lys Thr Val Val Leu Val Asp Leu Val Pro Met Gln Val Lys Phe
                405                 410                 415
Asp Asn Ala Thr Ala Phe Leu Thr Phe Glu Asn Leu Trp Ser Lys Lys
            420                 425                 430
Ile Ser Leu Lys Pro Ser Val Phe Gly Asp Tyr Glu Ile Leu Tyr Val
        435                 440                 445
Val Tyr Pro Gly Leu Pro Pro Ser Pro Pro Ser Ala Pro Glu Ser Val
```

```
    450                 455                 460
Gly Asp Gly Ala Phe Gly Asn Asn Arg Asn Ala Arg Ala Met Lys Pro
465                 470                 475                 480
Leu Gly Val Asp Val Gly Arg Pro Lys Lys Arg Val Asn Gly Ser Leu
                485                 490                 495
Ile Ala Ile Ala Val Leu Ser Thr Val Ile Ala Leu Ile Ile Cys Thr
            500                 505                 510
Leu Ala Ala Trp Leu Leu Ile Ile Arg Phe Arg Gly Ser Asp Gly Leu
        515                 520                 525
Ala Gln Arg Phe Pro His Ser Ala Leu Pro Lys Phe Ser Arg Ser Ser
    530                 535                 540
Gly Thr Gly Gln Thr Leu Leu Ala Gly Arg Tyr Ser Ser Pro Ser Gly
545                 550                 555                 560
Pro Ser Gly Ser Leu Gly Ser Ser Ile Ala Thr Tyr Ala Gly Gln Ala
                565                 570                 575
Lys Thr Phe Lys Phe Ala Glu Ile Glu Lys Ala Thr Asn Ser Phe Asp
            580                 585                 590
Asp Ser Thr Val Leu Gly Glu Gly Gly Phe Gly Cys Val Tyr Gln Gly
        595                 600                 605
Thr Leu Glu Asp Gly Thr Arg Val Ala Val Lys Val Leu Lys Arg Tyr
    610                 615                 620
Asp Gly Gln Gly Glu Arg Glu Phe Leu Ala Glu Val Glu Met Leu Gly
625                 630                 635                 640
Arg Leu His His Arg Asn Leu Val Lys Leu Leu Gly Ile Cys Val Glu
                645                 650                 655
Glu Asn Ala Arg Cys Leu Val Tyr Glu Leu Ile Pro Asn Gly Ser Val
            660                 665                 670
Glu Ser His Leu His Gly Val Asp Leu Glu Thr Ala Pro Leu Asp Trp
        675                 680                 685
Asn Ala Arg Met Lys Ile Ala Leu Gly Ala Ala Arg Ala Leu Ala Tyr
    690                 695                 700
Leu His Glu Asp Ser Ser Pro Cys Val Ile His Arg Asp Phe Lys Ser
705                 710                 715                 720
Ser Asn Ile Leu Leu Glu His Asp Phe Thr Pro Lys Val Ser Asp Phe
                725                 730                 735
Gly Leu Ala Arg Thr Ala Arg Gly Glu Gly Asn Gln His Ile Ser Thr
            740                 745                 750
Arg Val Met Gly Thr Phe Gly Tyr Val Ala Pro Glu Tyr Ala Met Thr
        755                 760                 765
Gly His Leu Leu Val Lys Ser Asp Val Tyr Ser Tyr Gly Val Val Leu
    770                 775                 780
Leu Glu Leu Leu Thr Gly Arg Lys Pro Val Asp Met Ser Arg Pro Gly
785                 790                 795                 800
Gly Gln Glu Asn Leu Val Ser Trp Ala Arg Pro Leu Leu Thr Asn Val
                805                 810                 815
Val Ser Leu Arg Gln Ala Val Asp Pro Leu Leu Gly Pro Asn Val Pro
            820                 825                 830
Leu Asp Asn Val Ala Lys Ala Ala Ala Ile Ala Ser Met Cys Val Gln
        835                 840                 845
Pro Glu Val Ala His Arg Pro Ser Met Gly Glu Val Val Gln Ala Leu
    850                 855                 860
Lys Leu Val Cys Ser Asp Gly Asp Glu Gly Leu Gly Ser Gly Ser Phe
865                 870                 875                 880
Ser Gln Glu Leu Ala Ala Gln Ala Ala Ala Ile Tyr Asp Val Thr Gly
                885                 890                 895
Met Glu Ala Glu Arg Val Leu Leu Ser Glu Met Phe Gly Ser Thr Pro
            900                 905                 910
Val Phe Thr Pro Ala Ala Asp Ser Gly Ser Phe Arg Lys Gln Ser Ser
        915                 920                 925
Ser Gly Pro Leu Met Thr Gly Lys Asn Arg Lys Phe Trp Gln Arg Leu
    930                 935                 940
Arg Ser Leu Ser Arg Gly Ser Met Ser Glu His Gly Ala Ser Pro Asp
945                 950                 955                 960
Phe Glu Thr Arg Ser Gln Cys Ser Asn Arg
                965                 970
```

<210> 41
<211> 1317
<212> DNA
<213> Oryza sativa
<400> 41

```
ggcagatcaa ctttgtctgt tagcagggtg agctctgcat cggcgtcaat gctctcaaca      60
```

gtggcaactt gcacaacatc agtaaagaca ttttcacttt cccagcttga aaaggccact 120
gatggttttg attcaaaaag agtgttgggt caaggtgggt ttggacgcgt ctaccatggg 180
accatggatg gcggagacga gattgctgtt aaattgctca caagagaaga caggagtggc 240
gatcgagagt tcattgcgga ggttgaaatg ctcagtcgac tacatcaccg taatcttgtc 300
aaattgattg gcatttgcat tgagcacaac aaaaggtgtc ttgtttatga gcttatacgc 360
aatggaagtg ttgaatctca ccttcatggt gctgataagg ctaagggcat gctgaactgg 420
gatgttagga tgaaaattgc tctgggtgca gctagaggct tagcatatct acatgaagac 480
tcaaaccctc atgttataca tcgtgacttc aagggcagca atatattgtt ggaggaagat 540
ttcactccta aggttactga ttttggttta gcaagggagg caactaatgg aattcaaccc 600
atttctacta gggtaatggg tacttttggg tacgttgctc cagaatatgc catgaccggg 660
catcttcttg tgaagagtga tgtctacagt tacggtgttg tcttgctgga gcttttatca 720
ggaaggaagc ccgtatgcat gtctgatacc aatggtcctc agaaccttgt gacttgggct 780
cgtcctctgc tatgccataa ggagggtttg gagaggttga tcgacccttc tctgaatggc 840
aatttcaact ttgatgatgt agctaaagta gcatccatcg cttccatgtg tgttcacaat 900
gatccgtcac agaggccatt catgggtgaa gtagtgcagg cactgaagct tatttacaat 960
gatgcggagg cggcttgcga tgattcttac agccacaggg actcatcgtg tgaccagtac 1020
gatgactacc atggggccct ggcccttgac agcggtagtg gtagctggtg gaatagaagc 1080
tcaaatcctt ctgggttttt tgacaaccgg aacccctac cagttattac catggaatac 1140
agctctggca ggatcgaagg agcgcgtgac cccgctttg cattgtcaac aggtggtcat 1200
gcacaatcac cagccctgca gaatagatca ggacccatcc ggatgaagaa aaagcttgcc 1260
tcattttacc ggtctagagg tagcttcagc gagcatggcc agctgcctcg ccattga 1317

<210> 42
<211> 438
<212> PRT
<213> Oryza sativa
<400> 42

Gly Arg Ser Thr Leu Ser Val Ser Arg Val Ser Ser Ala Ser Ala Ser
1 5 10 15
Met Leu Ser Thr Val Ala Thr Cys Thr Thr Ser Val Lys Thr Phe Ser
20 25 30
Leu Ser Gln Leu Glu Lys Ala Thr Asp Gly Phe Asp Ser Lys Arg Val
35 40 45
Leu Gly Gln Gly Gly Phe Gly Arg Val Tyr His Gly Thr Met Asp Gly
50 55 60
Gly Asp Glu Ile Ala Val Lys Leu Leu Thr Arg Glu Asp Arg Ser Gly
65 70 75 80
Asp Arg Glu Phe Ile Ala Glu Val Glu Met Leu Ser Arg Leu His His
85 90 95
Arg Asn Leu Val Lys Leu Ile Gly Ile Cys Ile Glu His Asn Lys Arg
100 105 110
Cys Leu Val Tyr Glu Leu Ile Arg Asn Gly Ser Val Glu Ser His Leu
115 120 125
His Gly Ala Asp Lys Ala Lys Gly Met Leu Asn Trp Asp Val Arg Met
130 135 140
Lys Ile Ala Leu Gly Ala Ala Arg Gly Leu Ala Tyr Leu His Glu Asp
145 150 155 160
Ser Asn Pro His Val Ile His Arg Asp Phe Lys Gly Ser Asn Ile Leu
165 170 175
Leu Glu Glu Asp Phe Thr Pro Lys Val Thr Asp Phe Gly Leu Ala Arg
180 185 190
Glu Ala Thr Asn Gly Ile Gln Pro Ile Ser Thr Arg Val Met Gly Thr
195 200 205
Phe Gly Tyr Val Ala Pro Glu Tyr Ala Met Thr Gly His Leu Leu Val
210 215 220
Lys Ser Asp Val Tyr Ser Tyr Gly Val Val Leu Leu Glu Leu Leu Ser
225 230 235 240
Gly Arg Lys Pro Val Cys Met Ser Asp Thr Asn Gly Pro Gln Asn Leu
245 250 255
Val Thr Trp Ala Arg Pro Leu Leu Cys His Lys Glu Gly Leu Glu Arg
260 265 270
Leu Ile Asp Pro Ser Leu Asn Gly Asn Phe Asn Phe Asp Asp Val Ala
275 280 285
Lys Val Ala Ser Ile Ala Ser Met Cys Val His Asn Asp Pro Ser Gln
290 295 300
Arg Pro Phe Met Gly Glu Val Val Gln Ala Leu Lys Leu Ile Tyr Asn
305 310 315 320
Asp Ala Glu Ala Ala Cys Asp Asp Ser Tyr Ser His Arg Asp Ser Ser
325 330 335
Cys Asp Gln Tyr Asp Asp Tyr His Gly Ala Leu Ala Leu Asp Ser Gly
340 345 350
Ser Gly Ser Trp Trp Asn Arg Ser Ser Asn Pro Ser Gly Phe Phe Asp

```
        355                 360                 365
Asn Arg Asn Pro Leu Pro Val Ile Thr Met Glu Tyr Ser Ser Gly Arg
    370                 375                 380
Ile Glu Gly Ala Arg Asp Pro Arg Phe Ala Leu Ser Thr Gly Gly His
385                 390                 395                 400
Ala Gln Ser Pro Ala Leu Gln Asn Arg Ser Gly Pro Ile Arg Met Lys
                405                 410                 415
Lys Lys Leu Ala Ser Phe Tyr Arg Ser Arg Gly Ser Phe Ser Glu His
            420                 425                 430
Gly Gln Leu Pro Arg His
        435
```

<210> 43
<211> 2172
<212> DNA
<213> Oryza sativa
<400> 43

```
atgccgccgc ggcgagcggc gctgctcctc ctcgctctcg ccgtgtatgt gccactggga      60
acggcaagct caacaactat cgcgtcgtac ttacttgggt tatggagtag agcacatcgg     120
cattctcttc ctgcccctgc tccagctcca gctccagctc cagcccctga aactcaccga     180
cctggtatta gacacccagt gcctaggcat cacaggaaaa ggcctcatgt tgccccacca     240
ctaccaccac catcatcatc agaaagacaa gtagcaccat atcagttgtt tccaagaatt     300
gatgagttag aaattgagat tgcagcagga acatttctga aacagagtca agttcggata     360
atgggtgctg ggagtagtct tcaagatcct gaaaaaacta ctgtcaccgt tgatttggtg     420
ccactaggtc agaagtttga taggacttca gccttactga ctagcaacag atttttgcaa     480
aagaaggtgc caataaactc gtccatattt ggtgattata acgtaatata tgttcattat     540
cctggcctgc cttctttggt ccctagtgtt ccaggatcct tgggcccaat aagcagtagc     600
caatacccct ttagtgcaaa tgtacacaat agaagacatc agaagattaa ctctaaaagt     660
gttgccataa ttgcattatc agctgttgtg cttgtattaa tgagctttgg catttgcatc     720
atatggaaat ataaaggatt tgaaaagtct cgtggcactg gtcgtgtttc gaattcgtca     780
gccacaagaa aaacaggtat gaggtcatca ttctccagta tgaccagctc gacggcatct     840
tttgtttcaa caattgcaac atgcccacct acagttaaga cattctctat ttctgagctt     900
gaaaaggcca cagaaaactt cagcttcaac aaaataatag gtgaaggagg gtatggccgt     960
gtttatcgag gtacaattga tgatgaagtt gatgttgcag tcaaattgct tacaagaaaa    1020
catcaaaaca gagatcgtga gtttattgcc gaggttgaga tgctaagtcg tttgcatcat    1080
cgtaatcttg tcaagctgat cggtatatgc attgaacgga gcacaaggtg cttggtgttt    1140
gagcttgttc caaacggaag tgtggagtct catctgcacg gttctgataa aatatacggc    1200
ccacttgatt ttgatactag aatgaaaata gcccttggtg cagcaagggg tctggcctac    1260
ctacatgagg atgccaatcc ccatgttata caccgtgatt tcaaggccag caacgttctt    1320
ttggaaaatg atttcactcc caaggttgcg gatttcgggt tggcaaagga agcctcagaa    1380
ggaatggacc atatttctac tcaggtcatg gggacttttg ggtacgttgc cccggagtat    1440
gcgatgaccg ggcatctcct ggtgaaaagc gacgtgtaca gctacggtgt cgtgctgctg    1500
gagctcctct cggggaggaa gccggtggac atgacgcagc cgccggggtc ggagaacctc    1560
gtcacctggg cgcgcccccct cctcaccgac cgggacggcc tccagcagct ggtcgacccg    1620
tcgatgccgg cggcgagcta cggcttcgag aagctggcca aggcggcggc catcgcgtcc    1680
atgtgcgtgc acgtcgaggc gtcgcaccgg ccgttcatgg gcgaggtcgt gcaggccctg    1740
aagctcatct acaacggcaa caacgacgac acctgcacca gcggctcgtt cggcggcggc    1800
ggcggcgagg agtacgagga cgaggaggcg tcgtcgccgt ggaacaaccg ctcgtggagc    1860
cacgacttcg ccgcgacgcc gccgccggcg tcgcgcaggc tggcgttccc gcgggctccg    1920
gcccgcccga cgacgatgga ctacagctcg gacccggccg acggggcggc ggggacgtcg    1980
tcggcgtcgg cgcggcggca gcggtcgacg tcgagcctgg tgctggacaa gatcgagtcg    2040
ctggcggcgt acgactggtc cggcccgctg agggccagca gggggaggaa cttctacagg    2100
ctgaggggaa gcatgagcga gcatggcggc catccttccg aagattgctc catggagggc    2160
tactggatgt ag                                                        2172
```

<210> 44
<211> 723
<212> PRT
<213> Oryza sativa
<400> 44

```
Met Pro Pro Arg Arg Ala Ala Leu Leu Leu Leu Ala Leu Ala Val Tyr
1               5                   10                  15
Val Pro Leu Gly Thr Ala Ser Ser Thr Thr Ile Ala Ser Tyr Leu Leu
            20                  25                  30
Gly Leu Trp Ser Arg Ala His Arg His Ser Leu Pro Ala Pro Ala Pro
        35                  40                  45
Ala Pro Ala Pro Ala Pro Ala Pro Glu Thr His Arg Pro Gly Ile Arg
    50                  55                  60
His Pro Val Pro Arg His His Arg Lys Arg Pro His Val Ala Pro Pro
65                  70                  75                  80
Leu Pro Pro Pro Ser Ser Ser Glu Arg Gln Val Ala Pro Tyr Gln Leu
                85                  90                  95
Phe Pro Arg Ile Asp Glu Leu Glu Ile Glu Ile Ala Ala Gly Thr Phe
```

```
                100                     105                     110
Leu Lys Gln Ser Gln Val Arg Ile Met Gly Ala Gly Ser Ser Leu Gln
            115                     120                     125
Asp Pro Glu Lys Thr Thr Val Thr Val Asp Leu Val Pro Leu Gly Gln
        130                     135                     140
Lys Phe Asp Arg Thr Ser Ala Leu Leu Thr Ser Asn Arg Phe Leu Gln
145                     150                     155                     160
Lys Lys Val Pro Ile Asn Ser Ser Ile Phe Gly Asp Tyr Asn Val Ile
                    165                     170                     175
Tyr Val His Tyr Pro Gly Leu Pro Ser Leu Val Pro Ser Val Pro Gly
                180                     185                     190
Ser Leu Gly Pro Ile Ser Ser Ser Gln Tyr Pro Phe Ser Ala Asn Val
            195                     200                     205
His Asn Arg Arg His Gln Lys Ile Asn Ser Lys Ser Val Ala Ile Ile
        210                     215                     220
Ala Leu Ser Ala Val Val Leu Val Leu Met Ser Phe Gly Ile Cys Ile
225                     230                     235                     240
Ile Trp Lys Tyr Lys Gly Phe Glu Lys Ser Arg Gly Thr Gly Arg Val
                    245                     250                     255
Ser Asn Ser Ser Ala Thr Arg Lys Thr Gly Met Arg Ser Ser Phe Ser
                260                     265                     270
Ser Met Thr Ser Ser Thr Ala Ser Phe Val Ser Thr Ile Ala Thr Cys
            275                     280                     285
Pro Pro Thr Val Lys Thr Phe Ser Ile Ser Glu Leu Glu Lys Ala Thr
        290                     295                     300
Glu Asn Phe Ser Phe Asn Lys Ile Ile Gly Glu Gly Gly Tyr Gly Arg
305                     310                     315                     320
Val Tyr Arg Gly Thr Ile Asp Asp Glu Val Asp Val Ala Val Lys Leu
                    325                     330                     335
Leu Thr Arg Lys His Gln Asn Arg Asp Arg Glu Phe Ile Ala Glu Val
                340                     345                     350
Glu Met Leu Ser Arg Leu His His Arg Asn Leu Val Lys Leu Ile Gly
            355                     360                     365
Ile Cys Ile Glu Arg Ser Thr Arg Cys Leu Val Phe Glu Leu Val Pro
        370                     375                     380
Asn Gly Ser Val Glu Ser His Leu His Gly Ser Asp Lys Ile Tyr Gly
385                     390                     395                     400
Pro Leu Asp Phe Asp Thr Arg Met Lys Ile Ala Leu Gly Ala Ala Arg
                    405                     410                     415
Gly Leu Ala Tyr Leu His Glu Asp Ala Asn Pro His Val Ile His Arg
                420                     425                     430
Asp Phe Lys Ala Ser Asn Val Leu Leu Glu Asn Asp Phe Thr Pro Lys
            435                     440                     445
Val Ala Asp Phe Gly Leu Ala Lys Glu Ala Ser Glu Gly Met Asp His
        450                     455                     460
Ile Ser Thr Gln Val Met Gly Thr Phe Gly Tyr Val Ala Pro Glu Tyr
465                     470                     475                     480
Ala Met Thr Gly His Leu Leu Val Lys Ser Asp Val Tyr Ser Tyr Gly
                    485                     490                     495
Val Val Leu Leu Glu Leu Leu Ser Gly Arg Lys Pro Val Asp Met Thr
                500                     505                     510
Gln Pro Pro Gly Ser Glu Asn Leu Val Thr Trp Ala Arg Pro Leu Leu
            515                     520                     525
Thr Asp Arg Asp Gly Leu Gln Gln Leu Val Asp Pro Ser Met Pro Ala
        530                     535                     540
Ala Ser Tyr Gly Phe Glu Lys Leu Ala Lys Ala Ala Ala Ile Ala Ser
545                     550                     555                     560
Met Cys Val His Val Glu Ala Ser His Arg Pro Phe Met Gly Glu Val
                    565                     570                     575
Val Gln Ala Leu Lys Leu Ile Tyr Asn Gly Asn Asn Asp Asp Thr Cys
                580                     585                     590
Thr Ser Gly Ser Phe Gly Gly Gly Gly Gly Glu Glu Tyr Glu Asp Glu
            595                     600                     605
Glu Ala Ser Ser Pro Trp Asn Asn Arg Ser Trp Ser His Asp Phe Ala
        610                     615                     620
Ala Thr Pro Pro Pro Ala Ser Arg Arg Leu Ala Phe Pro Arg Ala Pro
625                     630                     635                     640
Ala Arg Pro Thr Thr Met Asp Tyr Ser Ser Asp Pro Ala Asp Gly Ala
                    645                     650                     655
Ala Gly Thr Ser Ser Ala Ser Ala Arg Arg Gln Arg Ser Thr Ser Ser
                660                     665                     670
```

```
Leu Val Leu Asp Lys Ile Glu Ser Leu Ala Ala Tyr Asp Trp Ser Gly
        675                 680                 685
Pro Leu Arg Ala Ser Arg Gly Arg Asn Phe Tyr Arg Leu Arg Gly Ser
    690                 695                 700
Met Ser Glu His Gly Gly His Pro Ser Glu Asp Cys Ser Met Glu Gly
705                 710                 715                 720
Tyr Trp Met

<210>  45
<211>  1257
<212>  DNA
<213>  Saccharum officinarum
<400>  45
atgacggacg gcggcgacga gtacaagcgc gaggagtcgg tgaccctgct ggtcatcgtc     60
tccctcgccg cgctctcgct cctctccctc atcgcggcct tcgcctacta ctgctacatt    120
acgcgcaagg tctcccgccg cctccactcg ctccatctcc ccaagcgttc cagctcccct    180
cctccgcctc ctccccgggc cccgccgcgg cagcagcagg ggaaggacag cccgtccagc    240
aactccgcca gcgacggggg tggggcggcg gcggcgatgt cggtggtggt tgctggggag    300
aggggcgtgc aggtgttcag ctaccggcag ctccacgcgg ccacgggcgg gttcggtcgg    360
gcgcacatgg ttgggcaggg cagcttcggc gccgtgtacc gcggggtgct ccccgacggg    420
aggaaggtcg cggtcaagct catggaccgc ccagggaagc agggcgaaga ggagttcgag    480
atggaggtgg agttgctgag taggctccga tcgccgtatt tattgggcct gattggccat    540
tgttcggaag gtggacaccg tctactggta tatgagttca tggccaatgg gggtctccag    600
gagcatctct atccaaacag aggttcctgt ggtggaatct caaaattgga ctgggacaca    660
agaatgagaa ttgcacttga agcagccaaa ggtttggaat atcttcatga gcgtgttaat    720
ccacctgtta tacatagaga cttcaagagt agcaatattc tcctggacaa ggacttccat    780
gcaagagttt cagactttgg tctaaccaaa cttggatctg atagagctgg tggtcatgtc    840
tcaactcgag ttttgggcac acaaggctat gttgcaccag aatacgcatt gactgggcat    900
ttgactacca aatcagatgt gtacagttac ggtgttgtgc ttttggaact gcttactggc    960
agagtaccag ttgatatgaa gaggcctcct ggagaaggtg ttttagttaa ctgggcattg   1020
cctatgctca ctgaccgaga aaaagtcgtg cggatcttgg atccagcatt ggaaggtcaa   1080
tattccttga aagatgctgt ccaagtggct gctattgctg ccatgtgtgt tcaaccagag   1140
gctgactata ggccattaat ggctgatgtt gtccaatcgc tggttccact tgtcaagaac   1200
cgttctaatc agaaagcttg caaccccaat gtgcaatcat cgaagccact cgactag      1257
<210>  46
<211>  418
<212>  PRT
<213>  Saccharum officinarum
<400>  46
Met Thr Asp Gly Gly Asp Glu Tyr Lys Arg Glu Glu Ser Val Thr Leu
1               5                   10                  15
Leu Val Ile Val Ser Leu Ala Ala Leu Ser Leu Leu Ser Leu Ile Ala
            20                  25                  30
Ala Phe Ala Tyr Tyr Cys Tyr Ile Thr Arg Lys Val Ser Arg Arg Leu
        35                  40                  45
His Ser Leu His Leu Pro Lys Arg Ser Ser Ser Pro Pro Pro Pro Pro
    50                  55                  60
Pro Arg Ala Pro Pro Arg Gln Gln Gln Gly Lys Asp Ser Pro Ser Ser
65                  70                  75                  80
Asn Ser Ala Ser Asp Gly Gly Gly Ala Ala Ala Ala Met Ser Val Val
                85                  90                  95
Val Ala Gly Glu Arg Gly Val Gln Val Phe Ser Tyr Arg Gln Leu His
            100                 105                 110
Ala Ala Thr Gly Gly Phe Gly Arg Ala His Met Val Gly Gln Gly Ser
        115                 120                 125
Phe Gly Ala Val Tyr Arg Gly Val Leu Pro Asp Gly Arg Lys Val Ala
    130                 135                 140
Val Lys Leu Met Asp Arg Pro Gly Lys Gln Gly Glu Glu Glu Phe Glu
145                 150                 155                 160
Met Glu Val Glu Leu Leu Ser Arg Leu Arg Ser Pro Tyr Leu Leu Gly
                165                 170                 175
Leu Ile Gly His Cys Ser Glu Gly Gly His Arg Leu Leu Val Tyr Glu
            180                 185                 190
Phe Met Ala Asn Gly Gly Leu Gln Glu His Leu Tyr Pro Asn Arg Gly
        195                 200                 205
Ser Cys Gly Gly Ile Ser Lys Leu Asp Trp Asp Thr Arg Met Arg Ile
    210                 215                 220
Ala Leu Glu Ala Ala Lys Gly Leu Glu Tyr Leu His Glu Arg Val Asn
225                 230                 235                 240
Pro Pro Val Ile His Arg Asp Phe Lys Ser Ser Asn Ile Leu Leu Asp
                245                 250                 255
```

```
Lys Asp Phe His Ala Arg Val Ser Asp Phe Gly Leu Thr Lys Leu Gly
            260                 265                 270
Ser Asp Arg Ala Gly Gly His Val Ser Thr Arg Val Leu Gly Thr Gln
        275                 280                 285
Gly Tyr Val Ala Pro Glu Tyr Ala Leu Thr Gly His Leu Thr Thr Lys
    290                 295                 300
Ser Asp Val Tyr Ser Tyr Gly Val Val Leu Leu Glu Leu Leu Thr Gly
305                 310                 315                 320
Arg Val Pro Val Asp Met Lys Arg Pro Pro Gly Glu Gly Val Leu Val
                325                 330                 335
Asn Trp Ala Leu Pro Met Leu Thr Asp Arg Glu Lys Val Val Arg Ile
            340                 345                 350
Leu Asp Pro Ala Leu Glu Gly Gln Tyr Ser Leu Lys Asp Ala Val Gln
        355                 360                 365
Val Ala Ala Ile Ala Ala Met Cys Val Gln Pro Glu Ala Asp Tyr Arg
    370                 375                 380
Pro Leu Met Ala Asp Val Val Gln Ser Leu Val Pro Leu Val Lys Asn
385                 390                 395                 400
Arg Ser Asn Gln Lys Ala Cys Asn Pro Asn Val Gln Ser Ser Lys Pro
                405                 410                 415
Leu Asp

<210>  47
<211>  921
<212>  DNA
<213>  Glycine max
<400>  47
agcaagtcaa atgtcattgg ccatggtgga tttggacttg tttaccgggg agtgctcaac      60
gatggcagga aagttgcaat caaattcatg gatcaggcag gaaagcaagg agaagaagaa     120
tttaaagtag aggtggaact gctaagtcgg ttgcattctc catatctgct ggcattgctt     180
gggtactgct ctgatagtaa tcataaattg ttggtgtatg aatttatggc aaatggtgga     240
ctgcaggaac atctctatcc tgtcagcaat tctattatta cgcctgtaaa attggattgg     300
gaaacacgac taagaatagc acttgaagct gctaagggtt tggaatatct tcatgagcat     360
gtcagtcccc cagtgattca cagagatttt aagagtagca acatcctctt ggacaagaaa     420
tttcatgcca aggtttctga ttttggattg gcgaagcttg gacctgatag agctggtgga     480
catgtttcaa ctcgggtttt gggcacccag ggatatgttg cccctgaata tgcactaaca     540
gggcatttaa caacaaaatc agatgtgtac agttatggtg ttgtactttt ggagctgctc     600
actggccggg tgcctgttga tatgaagaga cctccagggg aaggtgttct tgtttcttgg     660
gctctgcccc ttttgactga tagagaaaag gttgtaaaga ttatggatcc ttcattggag     720
ggacaatact ctatgaaaga ggttgtccag gtggctgcaa ttgctgcaat gtgtgtgcaa     780
ccagaggcag attatcggcc tctcatggct gatgttgtgc agtcattggt tccactggtg     840
aagactcaaa ggtccccttc aaaggtagga agctcctcta gtttcaattc ccctaagttg     900
tcccctggcc caacatttta a                                               921
<210>  48
<211>  306
<212>  PRT
<213>  Glycine max
<400>  48
Ser Lys Ser Asn Val Ile Gly His Gly Gly Phe Gly Leu Val Tyr Arg
1               5                   10                  15
Gly Val Leu Asn Asp Gly Arg Lys Val Ala Ile Lys Phe Met Asp Gln
            20                  25                  30
Ala Gly Lys Gln Gly Glu Glu Glu Phe Lys Val Glu Val Glu Leu Leu
        35                  40                  45
Ser Arg Leu His Ser Pro Tyr Leu Leu Ala Leu Leu Gly Tyr Cys Ser
    50                  55                  60
Asp Ser Asn His Lys Leu Leu Val Tyr Glu Phe Met Ala Asn Gly Gly
65                  70                  75                  80
Leu Gln Glu His Leu Tyr Pro Val Ser Asn Ser Ile Ile Thr Pro Val
                85                  90                  95
Lys Leu Asp Trp Glu Thr Arg Leu Arg Ile Ala Leu Glu Ala Ala Lys
            100                 105                 110
Gly Leu Glu Tyr Leu His Glu His Val Ser Pro Pro Val Ile His Arg
        115                 120                 125
Asp Phe Lys Ser Ser Asn Ile Leu Leu Asp Lys Lys Phe His Ala Lys
    130                 135                 140
Val Ser Asp Phe Gly Leu Ala Lys Leu Gly Pro Asp Arg Ala Gly Gly
145                 150                 155                 160
His Val Ser Thr Arg Val Leu Gly Thr Gln Gly Tyr Val Ala Pro Glu
                165                 170                 175
Tyr Ala Leu Thr Gly His Leu Thr Thr Lys Ser Asp Val Tyr Ser Tyr
```

```
                180                 185                 190
Gly Val Val Leu Leu Glu Leu Leu Thr Gly Arg Val Pro Val Asp Met
            195                 200                 205
Lys Arg Pro Pro Gly Glu Gly Val Leu Val Ser Trp Ala Leu Pro Leu
    210                 215                 220
Leu Thr Asp Arg Glu Lys Val Val Lys Ile Met Asp Pro Ser Leu Glu
225                 230                 235                 240
Gly Gln Tyr Ser Met Lys Glu Val Val Gln Val Ala Ala Ile Ala Ala
                245                 250                 255
Met Cys Val Gln Pro Glu Ala Asp Tyr Arg Pro Leu Met Ala Asp Val
                260                 265                 270
Val Gln Ser Leu Val Pro Leu Val Lys Thr Gln Arg Ser Pro Ser Lys
            275                 280                 285
Val Gly Ser Ser Ser Ser Phe Asn Ser Pro Lys Leu Ser Pro Gly Pro
    290                 295                 300
Thr Phe
305
```

<210> 49
<211> 1140
<212> DNA
<213> Solanum tuberosum
<400> 49

```
acagggtgag gaagaattca aagtggaggt ggagttgcta tgccgcttgc gctcaccgta    60
tttgctgtca ttgattggct attgttcaga aagcagccat aaattgcttg tttatgagtt   120
catggcaaat ggtggtttac aggagcactt gtatccaatt aaaggttcca ataattgttg   180
tccgaagttg gactggaaga cccggttaag aatagctttg gaggctgcta aaggtttgga   240
atatctacat gagcatgtca atcccccaat tatacataga gacctcaaaa gtagcaatat   300
tcttttggac aaaaacttcc atgccaaagt ttctgacttt ggattggcca agcttggatc   360
tgataaagct ggtggccatg tctctacccg cgttttgggg acacagggat atgttgctcc   420
agaatatgca ttaacaggac acttgaccac caaatcagat gtttacagtt atggggttgt   480
cctcctagag ttgttgactg gcagagttcc agttgatatg aagagatcac ctggcgaagg   540
tgttcttgtt tcttgggcat tgccccgtct cactgatagg gaaaaagtcg tagagataat   600
ggatccagct ctggagggtc agtattcaat gaaagaagtt attcaagtag ctgctattgc   660
tgcaatgtgt gtacagcccg aggctgatta caggccactg atggcagacg ttgtgcagtc   720
gttggttcca ctggtgaaac aaccgcgacc aactgtgaag cctggtagct cgtctagctt   780
tcacgccaca cagtcccccct cccccatgt tacacaatct cctaaggctt agactttttt   840
caagcgaaat gggcatatca tatctcttga tccttaattc tagtccaact tctataacta   900
gtggccattt agtttgtgtt tggactatct agcttatgga acccccttttc atttagtaac   960
tttaatctaa caaattttga tggagcaagc cagctgatgt aatattttct catgctaaac  1020
taggtggagc aaaaacagtt tctctgtatg taacatgtca aagctcccat ctaatatggg  1080
gattgtattt gagtattctt ggttagaagc agattcaaga tttgaagtta ctatatgata  1140
```

<210> 50
<211> 276
<212> PRT
<213> Solanum tuberosum
<400> 50

```
Gln Gly Glu Glu Glu Phe Lys Val Glu Val Glu Leu Leu Cys Arg Leu
1               5                   10                  15
Arg Ser Pro Tyr Leu Leu Ser Leu Ile Gly Tyr Cys Ser Glu Ser Ser
            20                  25                  30
His Lys Leu Leu Val Tyr Glu Phe Met Ala Asn Gly Gly Leu Gln Glu
        35                  40                  45
His Leu Tyr Pro Ile Lys Gly Ser Asn Asn Cys Cys Pro Lys Leu Asp
    50                  55                  60
Trp Lys Thr Arg Leu Arg Ile Ala Leu Glu Ala Ala Lys Gly Leu Glu
65                  70                  75                  80
Tyr Leu His Glu His Val Asn Pro Pro Ile Ile His Arg Asp Leu Lys
                85                  90                  95
Ser Ser Asn Ile Leu Leu Asp Lys Asn Phe His Ala Lys Val Ser Asp
            100                 105                 110
Phe Gly Leu Ala Lys Leu Gly Ser Asp Lys Ala Gly Gly His Val Ser
        115                 120                 125
Thr Arg Val Leu Gly Thr Gln Gly Tyr Val Ala Pro Glu Tyr Ala Leu
    130                 135                 140
Thr Gly His Leu Thr Thr Lys Ser Asp Val Tyr Ser Tyr Gly Val Val
145                 150                 155                 160
Leu Leu Glu Leu Leu Thr Gly Arg Val Pro Val Asp Met Lys Arg Ser
                165                 170                 175
Pro Gly Glu Gly Val Leu Val Ser Trp Ala Leu Pro Arg Leu Thr Asp
            180                 185                 190
Arg Glu Lys Val Val Glu Ile Met Asp Pro Ala Leu Glu Gly Gln Tyr
```

```
        195                 200                 205
Ser Met Lys Glu Val Ile Gln Val Ala Ala Ile Ala Ala Met Cys Val
    210                 215                 220
Gln Pro Glu Ala Asp Tyr Arg Pro Leu Met Ala Asp Val Val Gln Ser
225                 230                 235                 240
Leu Val Pro Leu Val Lys Gln Pro Arg Pro Thr Val Lys Pro Gly Ser
                245                 250                 255
Ser Ser Ser Phe His Ala Thr Gln Ser Pro Ser Pro His Val Thr Gln
            260                 265                 270
Ser Pro Lys Ala
        275
```

<210> 51
<211> 1995
<212> DNA
<213> Nicotiana tabacum
<400> 51

```
taagatacca cagctatgct gacttatgaa agattttgga aaaagaaggt gcctctcaat     60
agaacgatgt ttggggatta tgaagtgatg cacattatct atccagggct gccttcttct    120
cctccatctg gcattgattc tggtaatggt ccaactggaa gtgctgtcaa tcaacaattc    180
cctattactg ctgattttgt aaacaagagt cagagaatga gtccccgagt catttttctc    240
attgcttcat cagcattagt actgttggtg gtatgctgtg gtgcactagt tgtcctctta    300
aaatgcagga ggactggccg accgtcaaat gctgttggtc cagtgtttac accatctatg    360
cacaagagat ctggtaaggg aattgggtcg acaatatcaa gtagcccgac tagttcaaca    420
tcgatttccc tcatttctgc tatgcctgct tctatccttt ctgtcaaaac atttacgctt    480
gcggagcttg agagggcaac tgacaagttt agtttaaaaa gggttttagg tgaaggagga    540
tttggacgtg tttatcatgg tatcttagaa gacagaacag aagttgccgt gaaagtactg    600
actagggata accaaaatgg agatcgtgaa ttcattgctg aagttgagat gctaagccga    660
ttgcatcacc gtaacctggt gaaattaatt ggtatatgca gtgaagagcg gactcgcagc    720
ttggtatatg aacttgttcg gaacggtagt gtggagtctc atttgcatgg aagagacggg    780
agaaaagagc cacttgattg ggatgtgagg ttgaaaattg ctcttggtgc tgcgagagga    840
ctagcttacc ttcatgaaga ttctaatcct cgtgtaattc atcgtgattt taaagccagc    900
aatgttttgt tagaagatga cttcacgccc aaggttgcag attttgggtt agcaagggaa    960
gcaaccgaag gaagtcacca catatctaca agagtcatgg gaacttttgg gtatgttgct   1020
cctgaatatg caatgacggg acacctactt gttaaaagtg atgtgtatag ttatggagtt   1080
gtattattgg agcttctctc cggaagaaaa cctgtggaca tgtctcaacc tcctggagaa   1140
gaaaacctgg taacttgggc gcgacctctt ctgaccacta gagaaggttt ggagcaactt   1200
gtggatcctt ctttggctgg aagctatgac tttgatgata tggcaaaggt ggctgccatt   1260
gcttcaatgt gcgttcaccc ggaggtgact caaaggccat ttatgggaga agtggtgcaa   1320
gctctcaaac taatttataa tgacaatgat gaaacttgtg ctgatggatg tagccagaag   1380
gagtcttctc tgccagattc agatttcaaa ggtgtccctt ccgatagcag ttggtggaat   1440
gctggtgggg ttacaccaag attaacatat ggacaagcct ccactttcat gaccatggat   1500
tacagttctg gtccgcttga agagttcgaa aacagaccgt tttcagcttc aagttttaat   1560
cttggtggag gggcgggttt aacaataagc cacggtaaca gatcaggtcc tttgaggact   1620
gtaaggagta aacctgctct ctataggtta aggggcagta tgagtgaaca cggtgcactt   1680
cttccaagac atgattggag ggatggcacc aattatgatg cttcttttta gagtgatttg   1740
tcaaatgtac agtgccgaag gccgtttcta tttgggaaaa aagatggttc tccggtgtag   1800
attaggagtt tgaaattgcc gctgtatccc tgagagagtg gactaagcct tctaattttg   1860
gtaatcttac attcatttta atagacagga aagataaaca ggacactcct tgttgtatat   1920
gttgtcaaat taacttttc tttagtccaa tattggattg gactactagt ctttctaaaa    1980
aaaaaaaaaa aaaaa                                                     1995
```

<210> 52
<211> 571
<212> PRT
<213> Nicotiana tabacum
<400> 52

```
Met Leu Thr Tyr Glu Arg Phe Trp Lys Lys Lys Val Pro Leu Asn Arg
1               5                   10                  15
Thr Met Phe Gly Asp Tyr Glu Val Met His Ile Ile Tyr Pro Gly Leu
            20                  25                  30
Pro Ser Ser Pro Pro Ser Gly Ile Asp Ser Gly Asn Gly Pro Thr Gly
        35                  40                  45
Ser Ala Val Asn Gln Gln Phe Pro Ile Thr Ala Asp Phe Val Asn Lys
    50                  55                  60
Ser Gln Arg Met Ser Pro Arg Val Ile Phe Leu Ile Ala Ser Ser Ala
65                  70                  75                  80
Leu Val Leu Leu Val Val Cys Cys Gly Ala Leu Val Val Leu Leu Lys
                85                  90                  95
Cys Arg Arg Thr Gly Arg Pro Ser Asn Ala Val Gly Pro Val Phe Thr
            100                 105                 110
Pro Ser Met His Lys Arg Ser Gly Lys Gly Ile Gly Ser Thr Ile Ser
        115                 120                 125
```

```
Ser Ser Pro Thr Ser Ser Thr Ser Ile Ser Leu Ile Ser Ala Met Pro
    130                 135                 140
Ala Ser Ile Leu Ser Val Lys Thr Phe Thr Leu Ala Glu Leu Glu Arg
145                 150                 155                 160
Ala Thr Asp Lys Phe Ser Leu Lys Arg Val Leu Gly Glu Gly Gly Phe
                165                 170                 175
Gly Arg Val Tyr His Gly Ile Leu Glu Asp Arg Thr Glu Val Ala Val
            180                 185                 190
Lys Val Leu Thr Arg Asp Asn Gln Asn Gly Asp Arg Glu Phe Ile Ala
        195                 200                 205
Glu Val Glu Met Leu Ser Arg Leu His His Arg Asn Leu Val Lys Leu
    210                 215                 220
Ile Gly Ile Cys Ser Glu Glu Arg Thr Arg Ser Leu Val Tyr Glu Leu
225                 230                 235                 240
Val Arg Asn Gly Ser Val Glu Ser His Leu His Gly Arg Asp Gly Arg
                245                 250                 255
Lys Glu Pro Leu Asp Trp Asp Val Arg Leu Lys Ile Ala Leu Gly Ala
            260                 265                 270
Ala Arg Gly Leu Ala Tyr Leu His Glu Asp Ser Asn Pro Arg Val Ile
        275                 280                 285
His Arg Asp Phe Lys Ala Ser Asn Val Leu Leu Glu Asp Asp Phe Thr
    290                 295                 300
Pro Lys Val Ala Asp Phe Gly Leu Ala Arg Glu Ala Thr Glu Gly Ser
305                 310                 315                 320
His His Ile Ser Thr Arg Val Met Gly Thr Phe Gly Tyr Val Ala Pro
                325                 330                 335
Glu Tyr Ala Met Thr Gly His Leu Leu Val Lys Ser Asp Val Tyr Ser
            340                 345                 350
Tyr Gly Val Val Leu Leu Glu Leu Leu Ser Gly Arg Lys Pro Val Asp
        355                 360                 365
Met Ser Gln Pro Pro Gly Glu Glu Asn Leu Val Thr Trp Ala Arg Pro
    370                 375                 380
Leu Leu Thr Thr Arg Glu Gly Leu Glu Gln Leu Val Asp Pro Ser Leu
385                 390                 395                 400
Ala Gly Ser Tyr Asp Phe Asp Asp Met Ala Lys Val Ala Ala Ile Ala
                405                 410                 415
Ser Met Cys Val His Pro Glu Val Thr Gln Arg Pro Phe Met Gly Glu
            420                 425                 430
Val Val Gln Ala Leu Lys Leu Ile Tyr Asn Asp Asn Asp Glu Thr Cys
        435                 440                 445
Ala Asp Gly Cys Ser Gln Lys Glu Ser Ser Leu Pro Asp Ser Asp Phe
    450                 455                 460
Lys Gly Val Pro Ser Asp Ser Ser Trp Trp Asn Ala Gly Gly Val Thr
465                 470                 475                 480
Pro Arg Leu Thr Tyr Gly Gln Ala Ser Thr Phe Met Thr Met Asp Tyr
                485                 490                 495
Ser Ser Gly Pro Leu Glu Glu Phe Glu Asn Arg Pro Phe Ser Ala Ser
            500                 505                 510
Ser Phe Asn Leu Gly Gly Gly Ala Gly Leu Thr Ile Ser His Gly Asn
        515                 520                 525
Arg Ser Gly Pro Leu Arg Thr Val Arg Ser Lys Pro Ala Leu Tyr Arg
    530                 535                 540
Leu Arg Gly Ser Met Ser Glu His Gly Ala Leu Leu Pro Arg His Asp
545                 550                 555                 560
Trp Arg Asp Gly Thr Asn Tyr Asp Ala Ser Phe
                565                 570
```

<210> 53
<211> 1059
<212> DNA
<213> Medicago truncatula
<400> 53

```
atgttaagtc gtttgcatca tcgcaatcta gtgaaactta tcggtatatg cattgaaggg     60
cgcaggcgtt gtctggtata cgagcttgtt cctaatggca gtgttgagtc ccatctgcat    120
ggtgatgaca agaatagggg acctctagat tgggaagcac gtatgaaaat tgcccttgga    180
gctgcgagag gattggcgta tcttcacgag gattccaatc cccgtgtaat tcatcgagac    240
ttcaaagcta gcaatgtgtt attagaagac gactttaccc ctaaggtttc tgatttcggt    300
ttagcaagag aagcaactga ggggagtaat catatttcta cacgggtgat ggggactttt    360
gggtacgttg ccccagaata tgcaatgacg ggccatttac tggttaaaag tgatgtttat    420
agttacggtg ttgtgcttct tgaacttctc acaggcagaa aaccagtgga tatgtctcaa    480
cctcagggac aggagaatct tgttacctgg gcacgggcac tgttgactag tagagaaggt    540
ttagagcagc tagtggatcc atctttggct ggaggttaca actttgatga catggcaaag    600
```

gtagcagcta ttgcgtcaat gtgtgttcac tccgaggtga cacagagacc ttttatggga 660
gaagttgtgc aggctcttaa attaatatac aatgacacag acgagactgg tggagattat 720
tgtagtcaga aggactcctc tgctcaggag tctgatttca gaggtgagct tgccccttct 780
gatagcagtt ggtggaatgg tggaggttta actcctcgat taacttatgg acaagcatct 840
tcctttatca caatggagta cagttctggt ccacttgaag atatggaaaa cagaccgttt 900
tcaacttcaa gctttaatgg agatgagcta tctttaccaa ttaggcatgg gaataggtca 960
ggtcccttaa gaacgacccg aagcaagtta tccttatata gattttcagg aagtcggagt 1020
gagcatgggg aagtttcgtc taagcgaaat tggatttga 1059

<210> 54
<211> 352
<212> PRT
<213> Medicago truncatula
<400> 54

Met Leu Ser Arg Leu His His Arg Asn Leu Val Lys Leu Ile Gly Ile
1 5 10 15
Cys Ile Glu Gly Arg Arg Arg Cys Leu Val Tyr Glu Leu Val Pro Asn
20 25 30
Gly Ser Val Glu Ser His Leu His Gly Asp Asp Lys Asn Arg Gly Pro
35 40 45
Leu Asp Trp Glu Ala Arg Met Lys Ile Ala Leu Gly Ala Ala Arg Gly
50 55 60
Leu Ala Tyr Leu His Glu Asp Ser Asn Pro Arg Val Ile His Arg Asp
65 70 75 80
Phe Lys Ala Ser Asn Val Leu Leu Glu Asp Asp Phe Thr Pro Lys Val
85 90 95
Ser Asp Phe Gly Leu Ala Arg Glu Ala Thr Glu Gly Ser Asn His Ile
100 105 110
Ser Thr Arg Val Met Gly Thr Phe Gly Tyr Val Ala Pro Glu Tyr Ala
115 120 125
Met Thr Gly His Leu Leu Val Lys Ser Asp Val Tyr Ser Tyr Gly Val
130 135 140
Val Leu Leu Glu Leu Leu Thr Gly Arg Lys Pro Val Asp Met Ser Gln
145 150 155 160
Pro Gln Gly Gln Glu Asn Leu Val Thr Trp Ala Arg Ala Leu Leu Thr
165 170 175
Ser Arg Glu Gly Leu Glu Gln Leu Val Asp Pro Ser Leu Ala Gly Gly
180 185 190
Tyr Asn Phe Asp Asp Met Ala Lys Val Ala Ala Ile Ala Ser Met Cys
195 200 205
Val His Ser Glu Val Thr Gln Arg Pro Phe Met Gly Glu Val Val Gln
210 215 220
Ala Leu Lys Leu Ile Tyr Asn Asp Thr Asp Glu Thr Gly Gly Asp Tyr
225 230 235 240
Cys Ser Gln Lys Asp Ser Ser Ala Gln Glu Ser Asp Phe Arg Gly Glu
245 250 255
Leu Ala Pro Ser Asp Ser Ser Trp Trp Asn Gly Gly Gly Leu Thr Pro
260 265 270
Arg Leu Thr Tyr Gly Gln Ala Ser Ser Phe Ile Thr Met Glu Tyr Ser
275 280 285
Ser Gly Pro Leu Glu Asp Met Glu Asn Arg Pro Phe Ser Thr Ser Ser
290 295 300
Phe Asn Gly Asp Glu Leu Ser Leu Pro Ile Arg His Gly Asn Arg Ser
305 310 315 320
Gly Pro Leu Arg Thr Thr Arg Ser Lys Leu Ser Leu Tyr Arg Phe Ser
325 330 335
Gly Ser Arg Ser Glu His Gly Glu Val Ser Ser Lys Arg Asn Trp Ile
340 345 350

<210> 55
<211> 983
<212> DNA
<213> Populus sp.
<400> 55

atgggccata agcctccaga caaatacaaa ggagtccagg ttttcacata caaggagctt 60
gaaatcgcca caaacaaatt cagtgaagca aatgtgacat ggaatgaagg gtatggagtg 120
gtgtatggag gtaccctaag tgatggaact gtggcagcaa ttaagatgct ccatagagca 180
gggaagcaag gagagcgtgc atttaggata gaggtggatt tgctaagcag gttgcactca 240
ccatacttgg tggagctact tggttattgt gctgaccaga accacaggct cctagtattt 300
gaatttatgc ctaatgggac tcttcaacac catctccatc acaagcaata tcgaccgttg 360
gattgggggga cacgattgag aattgccctt gattgtgcta gggccctgga gttccttcat 420
gagctcacaa tcccagcagt aatccatcgt gacttcaagt gtagtaacat cttactagac 480
caaaattttc gggctaaggt ttcagatttc ggatcggcta agatgggctc ggaaaggatc 540

```
aatgctcgaa attcgatgtg tttgccgagt accactggtt atctagcacc agagcatgct    600
tcaacaggta agctcaccac aaaatcagat gtatacagct atggagttgt tcttttacag    660
ctcttaactg gtcgtaaacc tgttgatacc aagcagccct ctggtgaaca tgtccttgtc    720
tcctgggctc ttccaaggct aaccaacaga gataaggtag tggaaatggt tgatccagcc    780
atgcaagacc agtattcaaa gaaggacttg atccaggtgg ctgctattgc agctgtgtgt    840
gtgcaaccag aagcagatta taggcctctc atgacagatg ttgtgcagtc tctaatccct    900
ctggtcaaga acctctcttc tgtatcttcc tcttgttcct ctaaatttat gaatcagatg    960
ctgtgcagtc caaggcctat gta                                             983
<210>  56
<211>  327
<212>  PRT
<213>  Populus sp.
<400>  56
Met Gly His Lys Pro Pro Asp Lys Tyr Lys Gly Val Gln Val Phe Thr
1               5                   10                  15
Tyr Lys Glu Leu Glu Ile Ala Thr Asn Lys Phe Ser Glu Ala Asn Val
            20                  25                  30
Thr Trp Asn Glu Gly Tyr Gly Val Val Tyr Gly Gly Thr Leu Ser Asp
        35                  40                  45
Gly Thr Val Ala Ala Ile Lys Met Leu His Arg Ala Gly Lys Gln Gly
    50                  55                  60
Glu Arg Ala Phe Arg Ile Glu Val Asp Leu Leu Ser Arg Leu His Ser
65                  70                  75                  80
Pro Tyr Leu Val Glu Leu Leu Gly Tyr Cys Ala Asp Gln Asn His Arg
                85                  90                  95
Leu Leu Val Phe Glu Phe Met Pro Asn Gly Thr Leu Gln His His Leu
            100                 105                 110
His His Lys Gln Tyr Arg Pro Leu Asp Trp Gly Thr Arg Leu Arg Ile
        115                 120                 125
Ala Leu Asp Cys Ala Arg Ala Leu Glu Phe Leu His Glu Leu Thr Ile
    130                 135                 140
Pro Ala Val Ile His Arg Asp Phe Lys Cys Ser Asn Ile Leu Leu Asp
145                 150                 155                 160
Gln Asn Phe Arg Ala Lys Val Ser Asp Phe Gly Ser Ala Lys Met Gly
                165                 170                 175
Ser Glu Arg Ile Asn Ala Arg Asn Ser Met Cys Leu Pro Ser Thr Thr
            180                 185                 190
Gly Tyr Leu Ala Pro Glu His Ala Ser Thr Gly Lys Leu Thr Thr Lys
        195                 200                 205
Ser Asp Val Tyr Ser Tyr Gly Val Val Leu Leu Gln Leu Leu Thr Gly
    210                 215                 220
Arg Lys Pro Val Asp Thr Lys Gln Pro Ser Gly Glu His Val Leu Val
225                 230                 235                 240
Ser Trp Ala Leu Pro Arg Leu Thr Asn Arg Asp Lys Val Val Glu Met
                245                 250                 255
Val Asp Pro Ala Met Gln Asp Gln Tyr Ser Lys Lys Asp Leu Ile Gln
            260                 265                 270
Val Ala Ala Ile Ala Ala Val Cys Val Gln Pro Glu Ala Asp Tyr Arg
        275                 280                 285
Pro Leu Met Thr Asp Val Val Gln Ser Leu Ile Pro Leu Val Lys Asn
    290                 295                 300
Leu Ser Ser Val Ser Ser Ser Cys Ser Ser Lys Phe Met Asn Gln Met
305                 310                 315                 320
Leu Cys Ser Pro Arg Pro Met
                325

<210>  57
<211>  282
<212>  PRT
<213>  Arabidopsis thaliana
<220>
<221>  UNSURE
<222>  (75)..(75)
<223>  Xaa can be any naturally occurring amino acid
<220>
<221>  UNSURE
<222>  (121)..(121)
<223>  Xaa can be any naturally occurring amino acid
<400>  57
Phe Ser Glu Glu Lys Lys Ile Gly Asn Gly Asp Val Tyr Lys Gly Val
1               5                   10                  15
Leu Ser Asp Gly Thr Val Ala Ala Ile Lys Lys Leu His Met Phe Asn
```

```
                20                  25                  30
Asp Asn Ala Ser Asn Gln Lys His Glu Glu Arg Ser Phe Arg Leu Glu
            35                  40                  45
Val Asp Leu Leu Ser Arg Leu Gln Cys Pro Tyr Leu Val Glu Leu Leu
    50                  55                  60
Gly Tyr Cys Ala Asp Gln Asn His Arg Ile Xaa Ile Tyr Glu Phe Met
65                  70                  75                  80
Pro Asn Gly Thr Val Glu His His Leu His Asp His Asn Phe Lys Asn
                85                  90                  95
Leu Lys Asp Arg Pro Gln Pro Leu Asp Trp Gly Ala Arg Leu Arg Ile
            100                 105                 110
Ala Leu Asp Cys Ala Arg Ala Leu Xaa Phe Leu His Glu Asn Thr Ile
        115                 120                 125
Ser Thr Val Ile His Arg Asn Phe Lys Cys Thr Asn Ile Leu Leu Asp
    130                 135                 140
Gln Asn Asn Arg Ala Lys Val Ser Asp Phe Gly Leu Ala Lys Thr Gly
145                 150                 155                 160
Ser Asp Lys Leu Asn Gly Glu Ile Ser Thr Arg Val Ile Gly Thr Thr
                165                 170                 175
Gly Tyr Leu Ala Pro Glu Tyr Ala Ser Thr Gly Lys Leu Thr Thr Lys
            180                 185                 190
Ser Asp Val Tyr Ser Tyr Gly Ile Val Leu Leu Gln Leu Leu Thr Gly
        195                 200                 205
Arg Thr Pro Ile Asp Ser Arg Arg Pro Arg Gly Gln Asp Val Leu Val
    210                 215                 220
Ser Trp Ala Leu Pro Arg Leu Thr Asn Arg Glu Lys Ile Ser Glu Met
225                 230                 235                 240
Val Asp Pro Thr Met Lys Gly Gln Tyr Ser Gln Lys Asp Leu Ile Gln
                245                 250                 255
Val Ala Ala Ile Ala Ala Val Cys Val Gln Pro Glu Ala Ser Tyr Arg
            260                 265                 270
Pro Leu Met Thr Asp Val Val His Ser Leu
        275                 280
<210>  58
<211>  2194
<212>  DNA
<213>  Oryza sativa
<400>  58
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc tttttttcgat tagatgcaag   540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc actttttaata atatctaaaa tacaaaaaat   720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa cctttttaaca gcaggctttg cggccaggag agaggaggag aggcaaagaa   960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
```

```
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                               2194
<210>  59
<211>  1620
<212>  DNA
<213>  Arabidopsis thaliana
<400>  59
atggaatttg agtgccaaga gagacttgaa gctgcgaacg atcaaagaag tgagtctggt     60
cttttgaata ccgatttgag aataggtaat gatgggtctg ttgagattcc aaatgtgaag    120
ttgtgttgtc agaaaaagaa gggaacttta gtgcccagtg gctcgaaaca gttactctct    180
gacaaggatt tgtctactac cattattgat ttacctcagg cattgatatc tgagattctt    240
aattgccttg acccaaaaga gttgggtcta gtgtcttgtg tttcaactta tctgcatagg    300
ttagcatctg agcatcacgc ttggaaggag ttctatcgtg agagatgggg actccctgta    360
gttttcggag ctgcaagttc agggctttct gatgagagat catggaaaga tctgtttgtt    420
gaaagggaat ttaggagtag gaccttttta ggacgttata gtattgatac tctgtatggt    480
cacactgaag cagtccggac tgtttttctc ttagcttctg ccaagctcgt tttcacttct    540
ggatatgatt ccattgttcg gatgtgggac atggaagaag gcttgtctat tgcggcatct    600
aaacctctcg gttgtacgat tagggcactt gctgctgata caaagctgtt ggtagctggc    660
ggtactgatg gattcattca ttgctggaaa tcactggatg gtctccggaa cctgtttgat    720
ctcacaggtt ttcagaaaga gaagactgag tttcggctat ggggacatga gggtcctatt    780
acatctcttg ccctggacat gacaagtatc tttagcggtt catgggatat gtctgttcgt    840
atatgggatc gatcttcaat gaagtgtgtc aaaaccttga ggcatagtga ttgggtttgg    900
ggacttgcac ctcatgaaac caccctcgct agcacgtcag gttctgatgt atatatttgg    960
gacgttagca gtgagactcc attggcgatc atccctgatg ctcatgaggg taccacttac   1020
tctttggcaa gaagccatac tggggatttc ctgtttactg gtggagaaga tggagggatt   1080
aaaatgtttg agatcagaag atacggtagt gaaacaagcg ttgtacttat atctcaatgg   1140
atgcctcaca ctagtcctgt ctactctctt tcttttgagt ttccttggct tgtctcagca   1200
tccggtgacg gtaaactcgc acttatcgac gttaggaaac tgttaaaaac taaccgttgt   1260
gcctattcca aaaggatttc ttcgagtact gtggaaccgc cacaacgaat gctgcacggg   1320
ttcgggtcaa acttgttctc tgtggacgtg ggttatgacc gtatagtgtg tggaggtgaa   1380
gaaggcacag tccggatatg gaacttcaca caagcattgg agatagagcg aaggacccga   1440
gctctgaaag gaatgaggca tgagaatagg atgagacgaa ggagaatgca aatggagatg   1500
aacgcaaaga atggaaggcc tgaccaatgc tccattgctg ctcataagaa ccccatcaat   1560
ggagaaagga accgagcctg gcatagtaaa cgaagagcaa gcgggaaggc gaaggcctaa   1620
<210>  60
<211>  539
<212>  PRT
<213>  Arabidopsis thaliana
<400>  60
Met Glu Phe Glu Cys Gln Glu Arg Leu Glu Ala Ala Asn Asp Gln Arg
1               5                   10                  15
Ser Glu Ser Gly Leu Leu Asn Thr Asp Leu Arg Ile Gly Asn Asp Gly
            20                  25                  30
Ser Val Glu Ile Pro Asn Val Lys Leu Cys Cys Gln Lys Lys Lys Gly
        35                  40                  45
Thr Leu Val Pro Ser Gly Ser Lys Gln Leu Leu Ser Asp Lys Asp Leu
    50                  55                  60
Ser Thr Thr Ile Ile Asp Leu Pro Gln Ala Leu Ile Ser Glu Ile Leu
65                  70                  75                  80
Asn Cys Leu Asp Pro Lys Glu Leu Gly Leu Val Ser Cys Val Ser Thr
                85                  90                  95
Tyr Leu His Arg Leu Ala Ser Glu His His Ala Trp Lys Glu Phe Tyr
            100                 105                 110
Arg Glu Arg Trp Gly Leu Pro Val Val Phe Gly Ala Ala Ser Ser Gly
        115                 120                 125
Leu Ser Asp Glu Arg Ser Trp Lys Asp Leu Phe Val Glu Arg Glu Phe
    130                 135                 140
Arg Ser Arg Thr Phe Leu Gly Arg Tyr Ser Ile Asp Thr Leu Tyr Gly
145                 150                 155                 160
His Thr Glu Ala Val Arg Thr Val Phe Leu Leu Ala Ser Ala Lys Leu
                165                 170                 175
Val Phe Thr Ser Gly Tyr Asp Ser Ile Val Arg Met Trp Asp Met Glu
            180                 185                 190
Glu Gly Leu Ser Ile Ala Ala Ser Lys Pro Leu Gly Cys Thr Ile Arg
        195                 200                 205
Ala Leu Ala Ala Asp Thr Lys Leu Leu Val Ala Gly Gly Thr Asp Gly
    210                 215                 220
Phe Ile His Cys Trp Lys Ser Leu Asp Gly Leu Arg Asn Leu Phe Asp
225                 230                 235                 240
```

```
Leu Thr Gly Phe Gln Lys Glu Lys Thr Glu Phe Arg Leu Trp Gly His
                245                 250                 255
Glu Gly Pro Ile Thr Ser Leu Ala Leu Asp Met Thr Ser Ile Phe Ser
            260                 265                 270
Gly Ser Trp Asp Met Ser Val Arg Ile Trp Asp Arg Ser Ser Met Lys
        275                 280                 285
Cys Val Lys Thr Leu Arg His Ser Asp Trp Val Trp Gly Leu Ala Pro
    290                 295                 300
His Glu Thr Thr Leu Ala Ser Thr Ser Gly Ser Asp Val Tyr Ile Trp
305                 310                 315                 320
Asp Val Ser Ser Glu Thr Pro Leu Ala Ile Ile Pro Asp Ala His Glu
                325                 330                 335
Gly Thr Thr Tyr Ser Leu Ala Arg Ser His Thr Gly Asp Phe Leu Phe
            340                 345                 350
Thr Gly Gly Glu Asp Gly Gly Ile Lys Met Phe Glu Ile Arg Arg Tyr
        355                 360                 365
Gly Ser Glu Thr Ser Val Val Leu Ile Ser Gln Trp Met Pro His Thr
    370                 375                 380
Ser Pro Val Tyr Ser Leu Ser Phe Glu Phe Pro Trp Leu Val Ser Ala
385                 390                 395                 400
Ser Gly Asp Gly Lys Leu Ala Leu Ile Asp Val Arg Lys Leu Leu Lys
                405                 410                 415
Thr Asn Arg Cys Ala Tyr Ser Lys Arg Ile Ser Ser Ser Thr Val Glu
            420                 425                 430
Pro Pro Gln Arg Met Leu His Gly Phe Gly Ser Asn Leu Phe Ser Val
        435                 440                 445
Asp Val Gly Tyr Asp Arg Ile Val Cys Gly Gly Glu Glu Gly Thr Val
    450                 455                 460
Arg Ile Trp Asn Phe Thr Gln Ala Leu Glu Ile Glu Arg Arg Thr Arg
465                 470                 475                 480
Ala Leu Lys Gly Met Arg His Glu Asn Arg Met Arg Arg Arg Arg Met
                485                 490                 495
Gln Met Glu Met Asn Ala Lys Asn Gly Arg Pro Asp Gln Cys Ser Ile
            500                 505                 510
Ala Ala His Lys Asn Pro Ile Asn Gly Glu Arg Asn Arg Ala Trp His
        515                 520                 525
Ser Lys Arg Arg Ala Ser Gly Lys Ala Lys Ala
    530                 535
```

```
<210>  61
<211>  1689
<212>  DNA
<213>  Oryza sativa
<400>  61
atggactttg attgcaaaac agcaagagga gactcttcat ctgtgaatcg ttcatgcatt      60
gtcactgaag gcactgtcgt ccaggcaaag cccgtttctc acaacgggaa agctaaacac     120
tggaatagcc tcagtacatt gaataaccag aagtgcagtt atgaattact ttccgaccca     180
aagaaaaatg ttgaaacaag tgatggtgag accgcctcaa agtgtgactc atggtgcttc     240
actgatttgc catccgcatt ggtctgtgaa gtgcttgaac acctcgatcc aaaggaactt     300
ggcattgtat cttgcgtctc caccctactg catacctag ccacagatca tcaggggtgg     360
aagaagttct actgtgaaag gtgggggatt cctactcctc cagtcaccct caatgggccc     420
ttggttccag gaggaacttc agattggaag tcttggaaaa cattatttgt ggagcgggag     480
tttcgaagta aatcattcat gggaagattc agtgtggatg ttctccgtga ccacagtgag     540
gatgtacgca ctgtgttcct tctagcatca gtaaatctga tatttactgg tggtaatgac     600
tctgtgatcc gaatgtggga cttggaggaa gggcttttga ttgataagtc ccgcccactt     660
tgttgcacca tccgggccat tgcagctgac actaggcttt tggtcactgc aggaaccaat     720
gcctttattc attgttggag ggctgttgaa ggtaattctt accctttcca catctctggg     780
aatggcactg accagagtcc tgagtttcgc ctttgggggc atgaaggacc tgtgacttgt     840
cttgccttgg attcattgag gattttcagt ggtagctggg atatgactgt ccgtgtttgg     900
gacagatccg aaatgaaatg tgttcagaag ttcatgcatg cagattgggt ttggagcgtg     960
gcacctcatg gaaatactgt tgccagtaca gctggtaggg atgcctatgt atgggatatc    1020
aggagtggtg agttggaaaa tgttatttcc aatgcccatt atggtaatgc attttcttta    1080
gctcgaacac acctagctga tgtgctgttt accggaggag aggatggggc aattcgcctg    1140
ttcaatgttt ctgaggtctc tgatgatgaa gatattaagc cagctgctac ttgggttcca    1200
cataccggcc ctgttcattc cctcgctttt gagtatccat ggcttgtctc agcctctagt    1260
gatggcaggg ttgcactgat tgatttgagg aagcttctga ccccaagaaa gtcatcaaaa    1320
caaccattca gggttaagaa ctttgatcca agttccattg aacctccaca gagaatgctt    1380
catggctttg ggtgcgatct tttttctgtc gccattggtg cagacaggat tgtctgtgga    1440
ggcgaggatg gcgctgtcaa agtctggaac ttctcagaag cactggagat tgagaagagg    1500
gcacaagctc taagaagtat gaggcaggag aaccgcatga ggcgaaaaaa ggcacaagta    1560
gagatgaatg caaatggtag aaggtctgac caatgtggct caatagccat gaaaagaaac    1620
caactgaagg gtgataagag tgtcacttgg cacagcaagc gtgccatcaa tgataaggtc    1680
```

aagtcttag 1689

<210> 62
<211> 562
<212> PRT
<213> Oryza sativa
<400> 62

```
Met Asp Phe Asp Cys Lys Thr Ala Arg Gly Asp Ser Ser Ser Val Asn
1               5                   10                  15
Arg Ser Cys Ile Val Thr Glu Gly Thr Val Val Gln Ala Lys Pro Val
            20                  25                  30
Ser His Asn Gly Lys Ala Lys His Trp Asn Ser Leu Ser Thr Leu Asn
        35                  40                  45
Asn Gln Lys Cys Ser Tyr Glu Leu Leu Ser Asp Pro Lys Lys Asn Val
    50                  55                  60
Glu Thr Ser Asp Gly Glu Thr Ala Ser Lys Cys Asp Ser Trp Cys Phe
65                  70                  75                  80
Thr Asp Leu Pro Ser Ala Leu Val Cys Glu Val Leu Glu His Leu Asp
                85                  90                  95
Pro Lys Glu Leu Gly Ile Val Ser Cys Val Ser Thr Leu Leu His Thr
            100                 105                 110
Leu Ala Thr Asp His Gln Gly Trp Lys Lys Phe Tyr Cys Glu Arg Trp
        115                 120                 125
Gly Ile Pro Thr Pro Pro Val Thr Leu Asn Gly Pro Leu Val Pro Gly
    130                 135                 140
Gly Thr Ser Asp Trp Lys Ser Trp Lys Thr Leu Phe Val Glu Arg Glu
145                 150                 155                 160
Phe Arg Ser Lys Ser Phe Met Gly Arg Phe Ser Val Asp Val Leu Arg
                165                 170                 175
Asp His Ser Glu Asp Val Arg Thr Val Phe Leu Leu Ala Ser Val Asn
            180                 185                 190
Leu Ile Phe Thr Gly Gly Asn Asp Ser Val Ile Arg Met Trp Asp Leu
        195                 200                 205
Glu Glu Gly Leu Leu Ile Asp Lys Ser Arg Pro Leu Cys Cys Thr Ile
    210                 215                 220
Arg Ala Ile Ala Ala Asp Thr Arg Leu Leu Val Thr Ala Gly Thr Asn
225                 230                 235                 240
Ala Phe Ile His Cys Trp Arg Ala Val Glu Gly Asn Ser Tyr Pro Phe
                245                 250                 255
His Ile Ser Gly Asn Gly Thr Asp Gln Ser Pro Glu Phe Arg Leu Trp
            260                 265                 270
Gly His Glu Gly Pro Val Thr Cys Leu Ala Leu Asp Ser Leu Arg Ile
        275                 280                 285
Phe Ser Gly Ser Trp Asp Met Thr Val Arg Val Trp Asp Arg Ser Glu
    290                 295                 300
Met Lys Cys Val Gln Lys Phe Met His Ala Asp Trp Val Trp Ser Val
305                 310                 315                 320
Ala Pro His Gly Asn Thr Val Ala Ser Thr Ala Gly Arg Asp Ala Tyr
                325                 330                 335
Val Trp Asp Ile Arg Ser Gly Glu Leu Glu Asn Val Ile Ser Asn Ala
            340                 345                 350
His Tyr Gly Asn Ala Phe Ser Leu Ala Arg Thr His Leu Ala Asp Val
        355                 360                 365
Leu Phe Thr Gly Gly Glu Asp Gly Ala Ile Arg Leu Phe Asn Val Ser
    370                 375                 380
Glu Val Ser Asp Asp Glu Asp Ile Lys Pro Ala Ala Thr Trp Val Pro
385                 390                 395                 400
His Thr Gly Pro Val His Ser Leu Ala Phe Glu Tyr Pro Trp Leu Val
                405                 410                 415
Ser Ala Ser Ser Asp Gly Arg Val Ala Leu Ile Asp Leu Arg Lys Leu
            420                 425                 430
Leu Thr Pro Arg Lys Ser Ser Lys Gln Pro Phe Arg Val Lys Asn Phe
        435                 440                 445
Asp Pro Ser Ser Ile Glu Pro Pro Gln Arg Met Leu His Gly Phe Gly
    450                 455                 460
Cys Asp Leu Phe Ser Val Ala Ile Gly Ala Asp Arg Ile Val Cys Gly
465                 470                 475                 480
Gly Glu Asp Gly Ala Val Lys Val Trp Asn Phe Ser Glu Ala Leu Glu
                485                 490                 495
Ile Glu Lys Arg Ala Gln Ala Leu Arg Ser Met Arg Gln Glu Asn Arg
            500                 505                 510
Met Arg Arg Lys Lys Ala Gln Val Glu Met Asn Ala Asn Gly Arg Arg
```

```
        515                 520                 525
Ser Asp Gln Cys Gly Ser Ile Ala Met Lys Arg Asn Gln Leu Lys Gly
    530                 535                 540
Asp Lys Ser Val Thr Trp His Ser Lys Arg Ala Ile Asn Asp Lys Val
545                 550                 555                 560
Lys Ser

<210>  63
<211>  1728
<212>  DNA
<213>  Medicago truncatulata
<400>  63
atggcatttg attgtccaca gagtattaag gtttctcaaa atttggtaga aaatccaggt      60
ataagcatag acatagttga attgaatcca aaacccaatt ccaaagcaat ttcaatttca     120
ttccctgatt ttgttacaaa taccaaatct gaatctggaa aaggtgagat ttttaagggg     180
aaatccaagc tgaattctaa gaaaggaatc aaagcagcct ctgctggtgc tttgaatcaa     240
tcatcagttc ctggtgatgt ggttattggt aattgtaggt caattaccga cctgcctccg     300
gtgttgatat ctgagattct gaattgtctt gatcccaaag aacttggaat tgtttcatgt     360
gtgtcgttga ttctgcgtag tcttgcttct gagcatcatg cttggaagga attctattgt     420
gaaaggtggg ggcttccagc agttcctgag gctgtcctgg attcggattc tggggttggg     480
gattcggtta aggatgaaaa gtcatggaag gatattttcg tggaaagaga ttataggagt     540
aagactttta tgggaaggta tagtatggat gtgttgtatg gacatactga ggcggttcgg     600
actgttttct tgttggcttc tacaaagctt atatttacat ctgggtatga cactgttgtg     660
aggatgtgga acatggaaag tgggttgtcg gttgcatcgt ctaaaccact tggctgcacc     720
attcgtgcgg ttgcagctga tacaaggctg ctagttgctg gtggtactga tgggttcatt     780
cattgttgga gggctgttga aggtttgcca catctgtttg agcttagaaa ctcacaacaa     840
aataagaatg aggttcgact ttggggtcat gatggtccgg ttacttcact tgctttagat     900
ttgacaagga tttatagcgg ctcttgggac acgactgttc gtgtttggga ccgtcactcg     960
atgaaatgca ctgtcgtgtt gaggcatagt gactgggttt ggggacttgt ccctcatgat    1020
actacagttg tcagcacatc aggttcaaat gtctatgttt gggatacaaa tagtggtaat    1080
ttggcaactg ttgttctaaa tgctcatgtt ggtaatactt atgctctggc aagaagccat    1140
actggggatt tcatttttac tgggggtgaa gatggttcaa ttcacatgta cgagattgtt    1200
gacggtagtt atgtgaccga agctctgcat gttgctacat gggatcccca ctctggtcct    1260
gtgtattccc ttgcctttga gtttccttgg cttgtttctg catcaagtga cggcaaattg    1320
gctctaattg acgtgaggaa gctgctgcgc aggagcaagc gtgccattgg aaagagagct    1380
acgaaggcaa agtattcggg cgaagttaat gtagagcctc cacagaggat gttgcatgga    1440
tttaagagta atcttttctc tgtaggtata ggagctgatc gaattgtttg cggaggtgaa    1500
gaaggtgtcg ttaggatttg gaatttcaca gaagctttgg aaattgaaag cagagttcgt    1560
gcattgagag gaatgcgatt agagaacaga atgagacgac gtaagaacca aacagagctc    1620
aacagtaaag gcggtaagag tgatcaatgt tcagctgcgg ccaagaagag ttcagtgact    1680
tgtatttggc cgagtaaacg tgggatgggt ggaaagacga aggcatag                 1728
<210>  64
<211>  575
<212>  PRT
<213>  Medicago truncatulata
<400>  64
Met Ala Phe Asp Cys Pro Gln Ser Ile Lys Val Ser Gln Asn Leu Val
1               5                   10                  15
Glu Asn Pro Gly Ile Ser Ile Asp Ile Val Glu Leu Asn Pro Lys Pro
            20                  25                  30
Asn Ser Lys Ala Ile Ser Ile Ser Phe Pro Asp Phe Val Thr Asn Thr
        35                  40                  45
Lys Ser Glu Ser Gly Lys Gly Glu Ile Phe Lys Gly Lys Ser Lys Leu
    50                  55                  60
Asn Ser Lys Lys Gly Ile Lys Ala Ala Ser Ala Gly Ala Leu Asn Gln
65                  70                  75                  80
Ser Ser Val Pro Gly Asp Val Val Ile Gly Asn Cys Arg Ser Ile Thr
                85                  90                  95
Asp Leu Pro Pro Val Leu Ile Ser Glu Ile Leu Asn Cys Leu Asp Pro
            100                 105                 110
Lys Glu Leu Gly Ile Val Ser Cys Val Ser Leu Ile Leu Arg Ser Leu
        115                 120                 125
Ala Ser Glu His His Ala Trp Lys Glu Phe Tyr Cys Glu Arg Trp Gly
    130                 135                 140
Leu Pro Ala Val Pro Glu Ala Val Leu Asp Ser Asp Ser Gly Val Gly
145                 150                 155                 160
Asp Ser Val Lys Asp Glu Lys Ser Trp Lys Asp Ile Phe Val Glu Arg
                165                 170                 175
Asp Tyr Arg Ser Lys Thr Phe Met Gly Arg Tyr Ser Met Asp Val Leu
            180                 185                 190
Tyr Gly His Thr Glu Ala Val Arg Thr Val Phe Leu Leu Ala Ser Thr
```

```
            195                     200                     205
Lys Leu Ile Phe Thr Ser Gly Tyr Asp Thr Val Val Arg Met Trp Asn
    210                     215                     220
Met Glu Ser Gly Leu Ser Val Ala Ser Ser Lys Pro Leu Gly Cys Thr
225                     230                     235                 240
Ile Arg Ala Val Ala Ala Asp Thr Arg Leu Leu Val Ala Gly Gly Thr
                245                     250                     255
Asp Gly Phe Ile His Cys Trp Arg Ala Val Glu Gly Leu Pro His Leu
            260                     265                     270
Phe Glu Leu Arg Asn Ser Gln Gln Asn Lys Asn Glu Val Arg Leu Trp
        275                     280                     285
Gly His Asp Gly Pro Val Thr Ser Leu Ala Leu Asp Leu Thr Arg Ile
    290                     295                     300
Tyr Ser Gly Ser Trp Asp Thr Thr Val Arg Val Trp Asp Arg His Ser
305                     310                     315                 320
Met Lys Cys Thr Val Val Leu Arg His Ser Asp Trp Val Trp Gly Leu
                325                     330                     335
Val Pro His Asp Thr Thr Val Val Ser Thr Ser Gly Ser Asn Val Tyr
            340                     345                     350
Val Trp Asp Thr Asn Ser Gly Asn Leu Ala Thr Val Val Leu Asn Ala
        355                     360                     365
His Val Gly Asn Thr Tyr Ala Leu Ala Arg Ser His Thr Gly Asp Phe
    370                     375                     380
Ile Phe Thr Gly Gly Glu Asp Gly Ser Ile His Met Tyr Glu Ile Val
385                     390                     395                 400
Asp Gly Ser Tyr Val Thr Glu Ala Leu His Val Ala Thr Trp Asp Pro
                405                     410                     415
His Ser Gly Pro Val Tyr Ser Leu Ala Phe Glu Phe Pro Trp Leu Val
            420                     425                     430
Ser Ala Ser Ser Asp Gly Lys Leu Ala Leu Ile Asp Val Arg Lys Leu
        435                     440                     445
Leu Arg Arg Ser Lys Arg Ala Ile Gly Lys Arg Ala Thr Lys Ala Lys
    450                     455                     460
Tyr Ser Gly Glu Val Asn Val Glu Pro Pro Gln Arg Met Leu His Gly
465                     470                     475                 480
Phe Lys Ser Asn Leu Phe Ser Val Gly Ile Gly Ala Asp Arg Ile Val
                485                     490                     495
Cys Gly Gly Glu Glu Gly Val Val Arg Ile Trp Asn Phe Thr Glu Ala
            500                     505                     510
Leu Glu Ile Glu Ser Arg Val Arg Ala Leu Arg Gly Met Arg Leu Glu
        515                     520                     525
Asn Arg Met Arg Arg Arg Lys Asn Gln Thr Glu Leu Asn Ser Lys Gly
    530                     535                     540
Gly Lys Ser Asp Gln Cys Ser Ala Ala Ala Lys Lys Ser Ser Val Thr
545                     550                     555                 560
Cys Ile Trp Pro Ser Lys Arg Gly Met Gly Gly Lys Thr Lys Ala
                565                     570                     575
```

<210> 65
<211> 1683
<212> DNA
<213> Triticum aestivum
<400> 65

```
atggactttg attgcaataa ggcaggagag tcttcagcca agcattgttc tagcatttgc      60
aacgagggca cactcatcca ggcaaacacc ttaattcatt gtggaaaggc taaaaagtgg     120
aatagcctca acaaattcaa caacccggag tcaagtcatg gatcacttcc aagggttaat     180
gaccccaagg aagatggtga aacaggaaat gatgcaactg cctctgagtg tagcgttatg     240
tgcttcactg atctgccgtc tgcattggtc tgtgaagtcc ttgcgcgcct tgatccaaag     300
gggcttgggg ttgtatcttg tgtctccaca gttctgcaga ccctagccac agatcatcag     360
ggatggaaga aattctactg tgagaggtgg ggacttccaa atgctcccat cggaccccta     420
gttccaggtg gaaccccaga tgggaggtcg tggaaagcat tgtttgtgga ccgggagttt     480
caaagcagat cattcatggg aagatttagt gtggatgttc tccgtggtca caatgaggat     540
gtacgcactg tgtaccttct ggcatcagca aatctgatat tcactggtgg ccatgattct     600
gtggttcgga tgtggaatat ggaggaaggg ctactaattg atgagtcccg cccatttggt     660
tgcactatcc gggcaattgc agctgacagt aggcttttgg taactggagg atccaaagcc     720
ttcattcagt gttggagggc tattgagggg gcctcgcacc tttctcacat ttctgggaat     780
ggtactaacc agaattctga atttcgccta tgggggcatg aagggcctgt gacttgtctt     840
tccttggatt caacaaggat ttacagtggt tcttgggata tgactgttcg tgtttgggac     900
agagccgaga tgaagtgtgt gcaaaagttc atgcatgctg actgggtttt ggccctggct     960
cctcatggaa atactgttgc cagtacagct ggtagagacg catatgtgtg ggatatcgga    1020
agtggcgagt taacaactat aatttccaat gcccatgttg gtaatgcata ttctgtagct    1080
cgaacacacc tggcaggtgt gctgtttact ggaggagagg acggggcaat tcgcttgttc    1140
```

```
gatgtttctg agatatcgga tgatgagaat attaaaccag ctgccacttg gttgccgcat    1200
tctggccctg ttcattctct tgcttttgag tacccatggc ttgtttctgc ctctagtgat    1260
ggcaggattg cactaattga tttgaggaag atcctgaccc ccctaaactc atcaaagcgc    1320
cgtttcaggg ttaagccctt tgatccaagt accgtagagc ctccacagcg aatgcttcat    1380
ggctttggat gctatctttt ctccgtcggc atcggtgcag atagaatcat atgtggaggc    1440
gaggatggct ctgtcagggt ctggaacttc tcggaagcac tggagattga gaagaaggca    1500
caggctttaa ggagtctgag acaggagaac cgcatgaggc ggaggaaggc gcaagtggag    1560
atgaatgcaa atggtagaag ggttgaccat tgctcagtag ccatgaaaag gaacccattg    1620
aagggtgata agagtgtcac ttggcaaatc aagcgtccca tcagtgacaa ggtcaagtct    1680
tag                                                                  1683
```

<210> 66
<211> 560
<212> PRT
<213> Triticum aestivum
<400> 66

```
Met Asp Phe Asp Cys Asn Lys Ala Gly Glu Ser Ser Ala Lys His Cys
1               5                   10                  15
Ser Ser Ile Cys Asn Glu Gly Thr Leu Ile Gln Ala Asn Thr Leu Ile
            20                  25                  30
His Cys Gly Lys Ala Lys Lys Trp Asn Ser Leu Asn Lys Phe Asn Asn
        35                  40                  45
Pro Glu Ser Ser His Gly Ser Leu Pro Arg Val Asn Asp Pro Lys Glu
    50                  55                  60
Asp Gly Glu Thr Gly Asn Asp Ala Thr Ala Ser Glu Cys Ser Val Met
65                  70                  75                  80
Cys Phe Thr Asp Leu Pro Ser Ala Leu Val Cys Glu Val Leu Ala Arg
                85                  90                  95
Leu Asp Pro Lys Gly Leu Gly Val Val Ser Cys Val Ser Thr Val Leu
            100                 105                 110
Gln Thr Leu Ala Thr Asp His Gln Gly Trp Lys Lys Phe Tyr Cys Glu
        115                 120                 125
Arg Trp Gly Leu Pro Asn Ala Pro Ile Gly Pro Leu Val Pro Gly Gly
    130                 135                 140
Thr Pro Asp Gly Arg Ser Trp Lys Ala Leu Phe Val Asp Arg Glu Phe
145                 150                 155                 160
Gln Ser Arg Ser Phe Met Gly Arg Phe Ser Val Asp Val Leu Arg Gly
                165                 170                 175
His Asn Glu Asp Val Arg Thr Val Tyr Leu Leu Ala Ser Ala Asn Leu
            180                 185                 190
Ile Phe Thr Gly Gly His Asp Ser Val Val Arg Met Trp Asn Met Glu
        195                 200                 205
Glu Gly Leu Leu Ile Asp Glu Ser Arg Pro Phe Gly Cys Thr Ile Arg
    210                 215                 220
Ala Ile Ala Ala Asp Ser Arg Leu Leu Val Thr Gly Gly Ser Lys Ala
225                 230                 235                 240
Phe Ile Gln Cys Trp Arg Ala Ile Glu Gly Ala Ser His Leu Ser His
                245                 250                 255
Ile Ser Gly Asn Gly Thr Asn Gln Asn Ser Glu Phe Arg Leu Trp Gly
            260                 265                 270
His Glu Gly Pro Val Thr Cys Leu Ser Leu Asp Ser Thr Arg Ile Tyr
        275                 280                 285
Ser Gly Ser Trp Asp Met Thr Val Arg Val Trp Asp Arg Ala Glu Met
    290                 295                 300
Lys Cys Val Gln Lys Phe Met His Ala Asp Trp Val Leu Ala Leu Ala
305                 310                 315                 320
Pro His Gly Asn Thr Val Ala Ser Thr Ala Gly Arg Asp Ala Tyr Val
                325                 330                 335
Trp Asp Ile Gly Ser Gly Glu Leu Thr Thr Ile Ile Ser Asn Ala His
            340                 345                 350
Val Gly Asn Ala Tyr Ser Val Ala Arg Thr His Leu Ala Gly Val Leu
        355                 360                 365
Phe Thr Gly Gly Glu Asp Gly Ala Ile Arg Leu Phe Asp Val Ser Glu
    370                 375                 380
Ile Ser Asp Asp Glu Asn Ile Lys Pro Ala Ala Thr Trp Leu Pro His
385                 390                 395                 400
Ser Gly Pro Val His Ser Leu Ala Phe Glu Tyr Pro Trp Leu Val Ser
                405                 410                 415
Ala Ser Ser Asp Gly Arg Ile Ala Leu Ile Asp Leu Arg Lys Ile Leu
            420                 425                 430
Thr Pro Leu Asn Ser Ser Lys Arg Arg Phe Arg Val Lys Pro Phe Asp
        435                 440                 445
```

Pro Ser Thr Val Glu Pro Pro Gln Arg Met Leu His Gly Phe Gly Cys
    450                 455                 460
Tyr Leu Phe Ser Val Gly Ile Gly Ala Asp Arg Ile Ile Cys Gly Gly
465                 470                 475                 480
Glu Asp Gly Ser Val Arg Val Trp Asn Phe Ser Glu Ala Leu Glu Ile
                485                 490                 495
Glu Lys Lys Ala Gln Ala Leu Arg Ser Leu Arg Gln Glu Asn Arg Met
            500                 505                 510
Arg Arg Arg Lys Ala Gln Val Glu Met Asn Ala Asn Gly Arg Arg Val
        515                 520                 525
Asp His Cys Ser Val Ala Met Lys Arg Asn Pro Leu Lys Gly Asp Lys
    530                 535                 540
Ser Val Thr Trp Gln Ile Lys Arg Pro Ile Ser Asp Lys Val Lys Ser
545                 550                 555                 560

<210> 67
<211> 1632
<212> DNA
<213> Populus tremuloides
<400> 67

atggcatttg aatgccaaaa gagcactgag gtgtcgaaaa gtcttgccat ttgtgttgaa 60
aaaagtaatt ccaatacgga acatcttgaa atttcgaagt ctcctttaga ctgttaccct 120
aagaagggga ttttaagcag tttgagttca aaacagcttt cttgtaatga tgttttgctc 180
aattgtcgcc ggtcaattac cgaccttcct ccggcattga tttctgagat tcttagttgc 240
cttgatcctc aagagcttgg tatagtttca tgtgtatcga gagtacttaa tagtcttgca 300
accgagaata gtgtttggaa ggaagtatat ggtgagagat ggggactccc tatagttcct 360
gcaccattgg gtgcaggggt ttcaaatgag aagtcatgga aggaactgtt tgtggagagg 420
gagtacagga gtaagatttt tttgagtcgt tatagcattg atactttgca tgggcatact 480
gaagcagtta gaacagtttc tctattggct tctgccaagc tcatttttac ctctgggtat 540
gatatgattg tgcgaatgtg ggacatggaa gacgggttgt atattgcatc atcacggcct 600
cttggttgca ctatacgagc agttgcagcg gacacgaagc tgttggttgc tggtggtact 660
gatggtttta ttcaaggctg gagggcagtt gagggtctca agcacttgtt tgaccttaaa 720
ggttctgagg tgccaaacac tgaatttaga atttgggagc atgagggacc tataacctct 780
cttgcccttgg accccacaag gatttatagt gggtcatggg acatgactgt tcgtatatgg 840
gatcgttcct cacttgaatg cataaagatc ttgaggcatg gtgactgggt gtggagcctt 900
gttccgcatg atactacagt tgctagcaca tcaggttcag acgtgtatgt ttgggacact 960
aatagtggga ctctgctaac tgttattcat agtgctcatg taggtaacac ttattctttg 1020
gctcgaagcc atacagagga tttcattttt acaggaggag aagatggggc tatgcacatg 1080
tttgagatca ctggtcctaa acctgaggct aatgttttta aggttgcaac ttggatgcct 1140
cactcagggc ctgtttattc ccttgcattt gagtttccat ggcttgtttc agcttctagt 1200
gatgggaggc tgtcactagt tgatgtgaga aaactactaa ggaccaacag acgttctcta 1260
cgaaagaatg tttcaagggt taccgataag gaccgtaaca atgttgaacc acctcagagg 1320
atgttacatg ggtttgggcc caatttgttt tcagtagatg ttggtgctga ccgtattgta 1380
tgtggaggag aggaaggtgt tgttaggatc tggaacttct caaaggcgct ggaaagggag 1440
cggacagctc gtgccatgag aggaatacgg ttggagaaca ggatgaggcg tcgtagactt 1500
caaatagaga tgagcagtaa gggtggtagg actgatcaat gctctgttgc agccaagaag 1560
aacccaatgc atgtagacaa gagtggtgtc tggcgcaata aacatgggat gagtggcaag 1620
ctgaaagcat ag 1632

<210> 68
<211> 543
<212> PRT
<213> Populus tremuloides
<400> 68

Met Ala Phe Glu Cys Gln Lys Ser Thr Glu Val Ser Lys Ser Leu Ala
1               5                   10                  15
Ile Cys Val Glu Lys Ser Asn Ser Asn Thr Glu His Leu Glu Ile Ser
            20                  25                  30
Lys Ser Pro Leu Asp Cys Tyr Pro Lys Lys Gly Ile Leu Ser Ser Leu
        35                  40                  45
Ser Ser Lys Gln Leu Ser Cys Asn Asp Val Leu Leu Asn Cys Arg Arg
    50                  55                  60
Ser Ile Thr Asp Leu Pro Pro Ala Leu Ile Ser Glu Ile Leu Ser Cys
65                  70                  75                  80
Leu Asp Pro Gln Glu Leu Gly Ile Val Ser Cys Val Ser Arg Val Leu
                85                  90                  95
Asn Ser Leu Ala Thr Glu Asn Ser Val Trp Lys Glu Val Tyr Gly Glu
            100                 105                 110
Arg Trp Gly Leu Pro Ile Val Pro Ala Pro Leu Gly Ala Gly Val Ser
        115                 120                 125
Asn Glu Lys Ser Trp Lys Glu Leu Phe Val Glu Arg Glu Tyr Arg Ser
    130                 135                 140
Lys Ile Phe Leu Ser Arg Tyr Ser Ile Asp Thr Leu His Gly His Thr

```
145                 150                 155                 160
Glu Ala Val Arg Thr Val Ser Leu Leu Ala Ser Ala Lys Leu Ile Phe
                165                 170                 175
Thr Ser Gly Tyr Asp Met Ile Val Arg Met Trp Asp Met Glu Asp Gly
            180                 185                 190
Leu Tyr Ile Ala Ser Ser Arg Pro Leu Gly Cys Thr Ile Arg Ala Val
        195                 200                 205
Ala Ala Asp Thr Lys Leu Leu Val Ala Gly Gly Thr Asp Gly Phe Ile
    210                 215                 220
Gln Gly Trp Arg Ala Val Glu Gly Leu Lys His Leu Phe Asp Leu Lys
225                 230                 235                 240
Gly Ser Glu Val Pro Asn Thr Glu Phe Arg Ile Trp Glu His Glu Gly
                245                 250                 255
Pro Ile Thr Ser Leu Ala Leu Asp Pro Thr Arg Ile Tyr Ser Gly Ser
            260                 265                 270
Trp Asp Met Thr Val Arg Ile Trp Asp Arg Ser Ser Leu Glu Cys Ile
        275                 280                 285
Lys Ile Leu Arg His Gly Asp Trp Val Trp Ser Leu Val Pro His Asp
    290                 295                 300
Thr Thr Val Ala Ser Thr Ser Gly Ser Asp Val Tyr Val Trp Asp Thr
305                 310                 315                 320
Asn Ser Gly Thr Leu Leu Thr Val Ile His Ser Ala His Val Gly Asn
                325                 330                 335
Thr Tyr Ser Leu Ala Arg Ser His Thr Glu Asp Phe Ile Phe Thr Gly
            340                 345                 350
Gly Glu Asp Gly Ala Met His Met Phe Glu Ile Thr Gly Pro Lys Pro
        355                 360                 365
Glu Ala Asn Val Phe Lys Val Ala Thr Trp Met Pro His Ser Gly Pro
    370                 375                 380
Val Tyr Ser Leu Ala Phe Glu Phe Pro Trp Leu Val Ser Ala Ser Ser
385                 390                 395                 400
Asp Gly Arg Leu Ser Leu Val Asp Val Arg Lys Leu Leu Arg Thr Asn
                405                 410                 415
Arg Arg Ser Leu Arg Lys Asn Val Ser Arg Val Thr Asp Lys Asp Arg
            420                 425                 430
Asn Asn Val Glu Pro Pro Gln Arg Met Leu His Gly Phe Gly Pro Asn
        435                 440                 445
Leu Phe Ser Val Asp Val Gly Ala Asp Arg Ile Val Cys Gly Gly Glu
    450                 455                 460
Glu Gly Val Val Arg Ile Trp Asn Phe Ser Lys Ala Leu Glu Arg Glu
465                 470                 475                 480
Arg Thr Ala Arg Ala Met Arg Gly Ile Arg Leu Glu Asn Arg Met Arg
                485                 490                 495
Arg Arg Arg Leu Gln Ile Glu Met Ser Ser Lys Gly Gly Arg Thr Asp
            500                 505                 510
Gln Cys Ser Val Ala Ala Lys Lys Asn Pro Met His Val Asp Lys Ser
        515                 520                 525
Gly Val Trp Arg Asn Lys His Gly Met Ser Gly Lys Leu Lys Ala
    530                 535                 540
```

<210> 69
<211> 1695
<212> DNA
<213> Zea mays
<400> 69

```
atggcctttg actgcaacaa ggaaagagga gtttcttcgc ctgacagttg ctcccgcatt    60
tgcaccgagg gcactctcgt ccaggcaaat cccttatctc actactggaa gcctaaacgt   120
ttggagaact tcaacaagtt ggagaaccag aagtccagtt atgaatctgc tccgagtggc   180
tctgatccaa aacaggatgc tgaagcgagt gatgaggcaa ctgcctcact gtgtggcttc   240
aggtgcttca ctgatctgcc agcttcgttg gtctgcgagg tccttgcacg tctcgatgcg   300
aaggaccttg gaattgtatc ctgtgtctcc accctcctgc acgccttagc cacagatcat   360
cagggatgga agaagttata ctgcgaaagg tgggggcttc cagacctccc caccaccctg   420
aatgggccct tgctgccggg tgtcccccta gatgggaagt tttggaaaac atttttcgtg   480
gagcgggagt ttaggagcaa atccttcatg ggaagattca atctggatat tctccgtggc   540
cacaatgagg atgtgcgtgc tgtgtccctt ttggtatcag caaatctgat attcacaggc   600
ggccgcgatt ctgtggttag gatgtggaag atggaggaag ggttattgat tgatacatcc   660
cgtgcacttg gtggcaccat ccgggcgatt gcagctgact cgaggctttt agtgagtgga   720
ggaactaatg cctatattca gtgttggagg gccgttgaag gcaatggtca attatttcac   780
atctctggaa gtggtaccga ccagaatgcg gagtttcgcc tctggggtca tgaaggtcct   840
gtgacttgtc ttgccctgga ttcactgagg atttatagtg gttcttggga catgactgtt   900
cgtgtttggg acagagacca gatggagtgt gtgcaaaagt tcatgcatgc agactgggtt   960
tgggatcttg ctctgcgtgg aaatactgtt gccagtactg ctggtagaga tgcatatgta  1020
```

```
tgggatatca gggctggcga gttaacaagc ttaatttcca atgcccatgt tggtagcgca 1080
tattcaattg cttggacaca cctgccagat gtgctgttta ctggtggaga ggatggtgct 1140
attcgattgt tcaatgtttt tgatgtctgt gatgatgagg gtgacattaa accagtggca 1200
acttgggtgc cacattcagg tcctgttcat tctcttgctt ttgagtatcc atggcttgtg 1260
tcagcctcga gtgatggcag gattgcactt attgatttga ggaagcttct gtcctccaaa 1320
agttcatcaa agggtctgcg tgttaagaga gttgatggaa gcgcaataga gcctccacag 1380
aggatgcttc atggcgttgg atgtgatctc ttctccatcg ctattggtgc agataggatt 1440
gtatgtgccg gtgaggatgg ttctgttaga gtctggaact tctcaaaagc attggagatt 1500
gagaggaggg cacaggctct aaggagtttg aggcaggaga accgcatcag gcggaggaaa 1560
tcacaagcgg agatgaatac aaatactaga aggcctgacc agtgttcaat agccatgaaa 1620
aagaaccaac tgaagggtga taagagcgca acttggcaca acaagcgtgc cattaatgag 1680
aaggccaagt cttag 1695
```

<210> 70
<211> 564
<212> PRT
<213> Zea mays
<400> 70

```
Met Ala Phe Asp Cys Asn Lys Glu Arg Gly Val Ser Ser Pro Asp Ser
1               5                   10                  15
Cys Ser Arg Ile Cys Thr Glu Gly Thr Leu Val Gln Ala Asn Pro Leu
            20                  25                  30
Ser His Tyr Trp Lys Pro Lys Arg Leu Glu Asn Phe Asn Lys Leu Glu
        35                  40                  45
Asn Gln Lys Ser Ser Tyr Glu Ser Ala Pro Ser Gly Ser Asp Pro Lys
    50                  55                  60
Gln Asp Ala Glu Ala Ser Asp Glu Ala Thr Ala Ser Leu Cys Gly Phe
65                  70                  75                  80
Arg Cys Phe Thr Asp Leu Pro Ala Ser Leu Val Cys Glu Val Leu Ala
                85                  90                  95
Arg Leu Asp Ala Lys Asp Leu Gly Ile Val Ser Cys Val Ser Thr Leu
            100                 105                 110
Leu His Ala Leu Ala Thr Asp His Gln Gly Trp Lys Lys Leu Tyr Cys
        115                 120                 125
Glu Arg Trp Gly Leu Pro Asp Leu Pro Thr Thr Leu Asn Gly Pro Leu
    130                 135                 140
Leu Pro Gly Val Pro Leu Asp Gly Lys Phe Trp Lys Thr Phe Phe Val
145                 150                 155                 160
Glu Arg Glu Phe Arg Ser Lys Ser Phe Met Gly Arg Phe Asn Leu Asp
                165                 170                 175
Ile Leu Arg Gly His Asn Glu Asp Val Arg Ala Val Ser Leu Leu Val
            180                 185                 190
Ser Ala Asn Leu Ile Phe Thr Gly Gly Arg Asp Ser Val Val Arg Met
        195                 200                 205
Trp Lys Met Glu Glu Gly Leu Leu Ile Asp Thr Ser Arg Ala Leu Gly
    210                 215                 220
Gly Thr Ile Arg Ala Ile Ala Ala Asp Ser Arg Leu Leu Val Ser Gly
225                 230                 235                 240
Gly Thr Asn Ala Tyr Ile Gln Cys Trp Arg Ala Val Glu Gly Asn Gly
                245                 250                 255
Gln Leu Phe His Ile Ser Gly Ser Gly Thr Asp Gln Asn Ala Glu Phe
            260                 265                 270
Arg Leu Trp Gly His Glu Gly Pro Val Thr Cys Leu Ala Leu Asp Ser
        275                 280                 285
Leu Arg Ile Tyr Ser Gly Ser Trp Asp Met Thr Val Arg Val Trp Asp
    290                 295                 300
Arg Asp Gln Met Glu Cys Val Gln Lys Phe Met His Ala Asp Trp Val
305                 310                 315                 320
Trp Asp Leu Ala Leu Arg Gly Asn Thr Val Ala Ser Thr Ala Gly Arg
                325                 330                 335
Asp Ala Tyr Val Trp Asp Ile Arg Ala Gly Glu Leu Thr Ser Leu Ile
            340                 345                 350
Ser Asn Ala His Val Gly Ser Ala Tyr Ser Ile Ala Trp Thr His Leu
        355                 360                 365
Pro Asp Val Leu Phe Thr Gly Gly Glu Asp Gly Ala Ile Arg Leu Phe
    370                 375                 380
Asn Val Phe Asp Val Cys Asp Asp Glu Gly Asp Ile Lys Pro Val Ala
385                 390                 395                 400
Thr Trp Val Pro His Ser Gly Pro Val His Ser Leu Ala Phe Glu Tyr
                405                 410                 415
Pro Trp Leu Val Ser Ala Ser Ser Asp Gly Arg Ile Ala Leu Ile Asp
            420                 425                 430
```

Leu Arg Lys Leu Leu Ser Ser Lys Ser Ser Ser Lys Gly Leu Arg Val
435 440 445
Lys Arg Val Asp Gly Ser Ala Ile Glu Pro Pro Gln Arg Met Leu His
450 455 460
Gly Val Gly Cys Asp Leu Phe Ser Ile Ala Ile Gly Ala Asp Arg Ile
465 470 475 480
Val Cys Ala Gly Glu Asp Gly Ser Val Arg Val Trp Asn Phe Ser Lys
485 490 495
Ala Leu Glu Ile Glu Arg Arg Ala Gln Ala Leu Arg Ser Leu Arg Gln
500 505 510
Glu Asn Arg Ile Arg Arg Arg Lys Ser Gln Ala Glu Met Asn Thr Asn
515 520 525
Thr Arg Arg Pro Asp Gln Cys Ser Ile Ala Met Lys Lys Asn Gln Leu
530 535 540
Lys Gly Asp Lys Ser Ala Thr Trp His Asn Lys Arg Ala Ile Asn Glu
545 550 555 560
Lys Ala Lys Ser

<210> 71
<211> 1278
<212> DNA
<213> Vitis vinifera
<400> 71
tggggagctc cggttgtttc ggggttttca gatgagaaat catggaggga attgtttgtg 60
gagagagagt ttaggagcaa aacctttagg ggacgattta gtattgatgt tctgtatggt 120
cgcactgagg cggttcgagc tgttttcctt ttggcctctg caaagctcat tttcacttct 180
gggtatgatt ccattgttcg aatgtgggat atggaagaag ggttgtcaat tgcgtgttca 240
cggcctctcg gttgcacaat aagagcagtg gctgcggata agaaactgtt gcttgcgggg 300
ggtagtgatg ggtttattca ttgttggaga gctgtagagg ggctctcttg cttgtttgac 360
cttgtgggtt ctcaaaacct gagtactgag ttccgaattt gggaacatga aggtcctgtg 420
acttgtcttg ctttggatat taagagaatt tatagtggct catgggacat gactgtgcgc 480
atatgggacc gttcttcgtt taaggttgta aaggtcttga ggcacacaga ctgggtttgg 540
ggccttgttc ctcgtgatac tacagttgct agcacttctg gctcagatgt gtatgtttgg 600
gacgctgata gtgggactct gctgacgata atctctaatg ctcatgtggg taatgcttat 660
gctttggcac ggagccacac aggggatttt ctattcactg ggggagaaga tggggcaata 720
catatgtttg aggtcgtgag tgattgcatg gaaaggaatg tattggaggt ttcaacgtgg 780
attcctcatt ctggtcctgt tcattccctg gcatttgagt ttccctggct tgtttcagct 840
tcagctgatg ggaggatgtc actgattgat gtgagaaagc ttttgcaaac ttgcaagcct 900
ttcttaggga agaatgtttc caaggttagg cacagggacc acaaaagtgt ggagccccct 960
cagaggatgt tacatgggtt tggctgcaat ttattctcag tggacattgg tgcagatcgt 1020
attgtctgtg gaggtgagaa aggtgtggtt agaatttgga acttctcaca agctttagaa 1080
gcagagcaga gggcccgtgc tttaagagga atacgtttag aaaccaggat gaggcggcgt 1140
aggcttcaaa tagagctgac tagtaagggt agtcgaactg atcaatgttc agttgcagcc 1200
agtaagaatc ctattaatgg tgataggagt ggtgtgtggc ccaataagcg gggaatgagt 1260
ggccggatga aagcatag 1278
<210> 72
<211> 425
<212> PRT
<213> Vitis vinifera
<400> 72
Trp Gly Ala Pro Val Val Ser Gly Phe Ser Asp Glu Lys Ser Trp Arg
1 5 10 15
Glu Leu Phe Val Glu Arg Glu Phe Arg Ser Lys Thr Phe Arg Gly Arg
20 25 30
Phe Ser Ile Asp Val Leu Tyr Gly Arg Thr Glu Ala Val Arg Ala Val
35 40 45
Phe Leu Leu Ala Ser Ala Lys Leu Ile Phe Thr Ser Gly Tyr Asp Ser
50 55 60
Ile Val Arg Met Trp Asp Met Glu Glu Gly Leu Ser Ile Ala Cys Ser
65 70 75 80
Arg Pro Leu Gly Cys Thr Ile Arg Ala Val Ala Ala Asp Lys Lys Leu
85 90 95
Leu Leu Ala Gly Gly Ser Asp Gly Phe Ile His Cys Trp Arg Ala Val
100 105 110
Glu Gly Leu Ser Cys Leu Phe Asp Leu Val Gly Ser Gln Asn Leu Ser
115 120 125
Thr Glu Phe Arg Ile Trp Glu His Glu Gly Pro Val Thr Cys Leu Ala
130 135 140
Leu Asp Ile Lys Arg Ile Tyr Ser Gly Ser Trp Asp Met Thr Val Arg
145 150 155 160
Ile Trp Asp Arg Ser Ser Phe Lys Val Val Lys Val Leu Arg His Thr

```
                165                 170                 175
Asp Trp Val Trp Gly Leu Val Pro Arg Asp Thr Thr Val Ala Ser Thr
            180                 185                 190
Ser Gly Ser Asp Val Tyr Val Trp Asp Ala Asp Ser Gly Thr Leu Leu
        195                 200                 205
Thr Ile Ile Ser Asn Ala His Val Gly Asn Ala Tyr Ala Leu Ala Arg
    210                 215                 220
Ser His Thr Gly Asp Phe Leu Phe Thr Gly Gly Glu Asp Gly Ala Ile
225                 230                 235                 240
His Met Phe Glu Val Val Ser Asp Cys Met Glu Arg Asn Val Leu Glu
                245                 250                 255
Val Ser Thr Trp Ile Pro His Ser Gly Pro Val His Ser Leu Ala Phe
            260                 265                 270
Glu Phe Pro Trp Leu Val Ser Ala Ser Ala Asp Gly Arg Met Ser Leu
        275                 280                 285
Ile Asp Val Arg Lys Leu Leu Gln Thr Cys Lys Pro Phe Leu Gly Lys
    290                 295                 300
Asn Val Ser Lys Val Arg His Arg Asp His Lys Ser Val Glu Pro Pro
305                 310                 315                 320
Gln Arg Met Leu His Gly Phe Gly Cys Asn Leu Phe Ser Val Asp Ile
                325                 330                 335
Gly Ala Asp Arg Ile Val Cys Gly Gly Glu Lys Gly Val Val Arg Ile
            340                 345                 350
Trp Asn Phe Ser Gln Ala Leu Glu Ala Glu Gln Arg Ala Arg Ala Leu
        355                 360                 365
Arg Gly Ile Arg Leu Glu Thr Arg Met Arg Arg Arg Arg Leu Gln Ile
    370                 375                 380
Glu Leu Thr Ser Lys Gly Ser Arg Thr Asp Gln Cys Ser Val Ala Ala
385                 390                 395                 400
Ser Lys Asn Pro Ile Asn Gly Asp Arg Ser Gly Val Trp Pro Asn Lys
                405                 410                 415
Arg Gly Met Ser Gly Arg Met Lys Ala
            420                 425
<210>  73
<211>  660
<212>  DNA
<213>  Senecio cambrensis
<400>  73
atggcatttg agttaaaccc gagcacatcg aattctgaaa aagataaacc tcaggataat      60
tcatctgaaa ataatttact gattgattcg gaaagcggga aacattttgc tgctaagtta     120
ctggttgacg agtgttgttt gttgaaaggt tataaaacaa ttaccgacct tcctccagcg     180
atagtttctg aaatatttaa cagccttgat ccaaaggaac tcgggatagt ttcctgtgtg     240
aatacatctt tatatcgaat tgcatttgat caccacgttt ggaaggagtt ttattgtgag     300
agatggggac ttcctattgg agtccccaat acgtctttag agaaatcatg gagagacctt     360
tttgtggaac gtgagtttcg tagtaagacg tttatgggac cttactctac tgaaactctt     420
tatggtcata ctgaagctgt tcgtacagtt tttcttttac tttcaagaaa gattataatt     480
acttcgggat atgattcagt tattcgaatg tgggacatgg aaggtggtta ttcaattgct     540
tcgtcccgtc ctcttggttg taccatccga gccgttacag cagattcaaa actgttaatt     600
gctggcggtt ccgatggttt tattcatggt tggagagccc aagaagaaga ggacatcctt     660
<210>  74
<211>  220
<212>  PRT
<213>  Senecio cambrensis
<400>  74
Met Ala Phe Glu Leu Asn Pro Ser Thr Ser Asn Ser Glu Lys Asp Lys
1               5                   10                  15
Pro Gln Asp Asn Ser Ser Glu Asn Asn Leu Leu Ile Asp Ser Glu Ser
            20                  25                  30
Gly Lys His Phe Ala Ala Lys Leu Leu Val Asp Glu Cys Cys Leu Leu
        35                  40                  45
Lys Gly Tyr Lys Thr Ile Thr Asp Leu Pro Pro Ala Ile Val Ser Glu
    50                  55                  60
Ile Phe Asn Ser Leu Asp Pro Lys Glu Leu Gly Ile Val Ser Cys Val
65                  70                  75                  80
Asn Thr Ser Leu Tyr Arg Ile Ala Phe Asp His His Val Trp Lys Glu
                85                  90                  95
Phe Tyr Cys Glu Arg Trp Gly Leu Pro Ile Gly Val Pro Asn Thr Ser
            100                 105                 110
Leu Glu Lys Ser Trp Arg Asp Leu Phe Val Glu Arg Glu Phe Arg Ser
        115                 120                 125
Lys Thr Phe Met Gly Pro Tyr Ser Thr Glu Thr Leu Tyr Gly His Thr
```

130 135 140
Glu Ala Val Arg Thr Val Phe Leu Leu Leu Ser Arg Lys Ile Ile Ile
145 150 155 160
Thr Ser Gly Tyr Asp Ser Val Ile Arg Met Trp Asp Met Glu Gly Gly
165 170 175
Tyr Ser Ile Ala Ser Ser Arg Pro Leu Gly Cys Thr Ile Arg Ala Val
180 185 190
Thr Ala Asp Ser Lys Leu Leu Ile Ala Gly Gly Ser Asp Gly Phe Ile
195 200 205
His Gly Trp Arg Ala Gln Glu Glu Glu Asp Ile Leu
210 215 220

<210> 75
<211> 847
<212> DNA
<213> Helianthus annuus
<220>
<221> misc_feature
<222> (789)..(789)
<223> n is a, c, g, or t
<400> 75

```
gcgggcgacg gagtccccgt ctctttaaat gctgagtgtt ctgatgagag atcttggaag      60
gcattatttg tggaacggga attccggagc aaaacatttc tgggccggta tacaatcgat     120
acgctttacg gtcatacaga acccgttcgc actctttttg ttttgctctc gaaaaagctt     180
ataataactt ccggttatga ctcgattatt cgaatgtggg acgttgaaga tggttattca     240
atcgcttcat ctcggcctct gggttgcacc atccgagccg ttgcagctga ttctaagctg     300
ttaattgctg gcgggtctga tgggtttata catggctgga gagctgaaga aggacaccct     360
cacttgtttg acataaaagg accccaaaac cagaacaccg agtttcgact ttgggaacat     420
caaggcccga taacttgtat cgggctagat tcatctagga tttacagcgg gtcatgggac     480
atgaccatac gcgtctggga tcgtgtctcg ctcaagtgct tgactgtctt gatgcataac     540
gactgggtgt ggagcctggt cccacatgat ggtactgtag taagtacttc tggttcagat     600
gtatatatct gggagactaa tacctttca cttattgaca ttatcaaaga tgcccatgtg      660
ggtaatgctt attctctagc tagaagtcac accggtaatt tcatcttcac tggtggtgaa     720
gatggtgcta tacatatgtt tgagattact agtaatggat gtggttcagt tcgccggcta     780
gcaacgtgng gtccacatac gggtcctgta tattctcttt catttgagtt tccatggcta     840
gtttctg                                                               847
```

<210> 76
<211> 282
<212> PRT
<213> Helianthus annuus
<220>
<221> UNSURE
<222> (263)..(263)
<223> Xaa can be any naturally occurring amino acid
<400> 76

Ala Gly Asp Gly Val Pro Val Ser Leu Asn Ala Glu Cys Ser Asp Glu
1 5 10 15
Arg Ser Trp Lys Ala Leu Phe Val Glu Arg Glu Phe Arg Ser Lys Thr
20 25 30
Phe Leu Gly Arg Tyr Thr Ile Asp Thr Leu Tyr Gly His Thr Glu Pro
35 40 45
Val Arg Thr Leu Phe Val Leu Leu Ser Lys Lys Leu Ile Ile Thr Ser
50 55 60
Gly Tyr Asp Ser Ile Ile Arg Met Trp Asp Val Glu Asp Gly Tyr Ser
65 70 75 80
Ile Ala Ser Ser Arg Pro Leu Gly Cys Thr Ile Arg Ala Val Ala Ala
85 90 95
Asp Ser Lys Leu Leu Ile Ala Gly Gly Ser Asp Gly Phe Ile His Gly
100 105 110
Trp Arg Ala Glu Glu Gly His Pro His Leu Phe Asp Ile Lys Gly Pro
115 120 125
Gln Asn Gln Asn Thr Glu Phe Arg Leu Trp Glu His Gln Gly Pro Ile
130 135 140
Thr Cys Ile Gly Leu Asp Ser Ser Arg Ile Tyr Ser Gly Ser Trp Asp
145 150 155 160
Met Thr Ile Arg Val Trp Asp Arg Val Ser Leu Lys Cys Leu Thr Val
165 170 175
Leu Met His Asn Asp Trp Val Trp Ser Leu Val Pro His Asp Gly Thr
180 185 190
Val Val Ser Thr Ser Gly Ser Asp Val Tyr Ile Trp Glu Thr Asn Thr
195 200 205
Phe Ser Leu Ile Asp Ile Ile Lys Asp Ala His Val Gly Asn Ala Tyr

```
    210                 215                 220
Ser Leu Ala Arg Ser His Thr Gly Asn Phe Ile Phe Thr Gly Gly Glu
225                 230                 235                 240
Asp Gly Ala Ile His Met Phe Glu Ile Thr Ser Asn Gly Cys Gly Ser
                245                 250                 255
Val Arg Arg Leu Ala Thr Xaa Gly Pro His Thr Gly Pro Val Tyr Ser
            260                 265                 270
Leu Ser Phe Glu Phe Pro Trp Leu Val Ser
        275                 280
<210>  77
<211>  728
<212>  DNA
<213>  Euphorbia esula
<400>  77
ggatggaaag ccgtggaggg tcttccacat ttgttcaacc ttaagggtag ttccgagaat      60
cttccaaacg atgaatttcg actttgggaa cacgagggac ctataacctc tctcgccttg     120
gatcgcacga gaatttacag tggttcttgg gacatgactg ttcgtgtatg ggatcgttcc     180
acattgaagt gccttaaaat cttgaggcat agcgattggg tatggggcct tgttccgcac     240
gataccaccg ttgccacctc atcgggttcc gatgtgtata tttgggatac tcaaaccgga     300
actcttatag ccgatattaa taacgcccat gtcgggaaca tttattctct agcccgaagc     360
cacacgggag attttctctt caccggagga gaagatgggg ctatacatat gtttgagatc     420
attggtaata atggatccaa gcctaaggtc tacaagatcg caacttggat tccacactcg     480
ggtcccgtct actccctctc gtttgaattt ccgtggcttg tttcggcttc tagtgatggg     540
aaactctcgc ttatagatgt tcgaaagcta cttcgaacaa gtaggcgctc tatgggaaag     600
aagaatcttg gtagggttag caaaatggat agtaacaatg tggagccacc tcaacggatg     660
ctccacgggt ttggacccaa tttgttttcg gtagacattg gggctgaccg gattgtttgt     720
ggagggga                                                              728
<210>  78
<211>  241
<212>  PRT
<213>  Euphorbia esula
<400>  78
Gly Trp Lys Ala Val Glu Gly Leu Pro His Leu Phe Asn Leu Lys Gly
1               5                   10                  15
Ser Ser Glu Asn Leu Pro Asn Asp Glu Phe Arg Leu Trp Glu His Glu
            20                  25                  30
Gly Pro Ile Thr Ser Leu Ala Leu Asp Arg Thr Arg Ile Tyr Ser Gly
        35                  40                  45
Ser Trp Asp Met Thr Val Arg Val Trp Asp Arg Ser Thr Leu Lys Cys
    50                  55                  60
Leu Lys Ile Leu Arg His Ser Asp Trp Val Trp Gly Leu Val Pro His
65                  70                  75                  80
Asp Thr Thr Val Ala Thr Ser Ser Gly Ser Asp Val Tyr Ile Trp Asp
                85                  90                  95
Thr Gln Thr Gly Thr Leu Ile Ala Asp Ile Asn Asn Ala His Val Gly
            100                 105                 110
Asn Ile Tyr Ser Leu Ala Arg Ser His Thr Gly Asp Phe Leu Phe Thr
        115                 120                 125
Gly Gly Glu Asp Gly Ala Ile His Met Phe Glu Ile Ile Gly Asn Asn
    130                 135                 140
Gly Ser Lys Pro Lys Val Tyr Lys Ile Ala Thr Trp Ile Pro His Ser
145                 150                 155                 160
Gly Pro Val Tyr Ser Leu Ser Phe Glu Phe Pro Trp Leu Val Ser Ala
                165                 170                 175
Ser Ser Asp Gly Lys Leu Ser Leu Ile Asp Val Arg Lys Leu Leu Arg
            180                 185                 190
Thr Ser Arg Arg Ser Met Gly Lys Lys Asn Leu Gly Arg Val Ser Lys
        195                 200                 205
Met Asp Ser Asn Asn Val Glu Pro Pro Gln Arg Met Leu His Gly Phe
    210                 215                 220
Gly Pro Asn Leu Phe Ser Val Asp Ile Gly Ala Asp Arg Ile Val Cys
225                 230                 235                 240
Gly

<210>  79
<211>  713
<212>  DNA
<213>  Lycopersicon esculentum
<400>  79
atcgtcttgc ttcggagcac catgtgtgga aggagttcta ttgtgaaagg tgggggcagc      60
caatattgtt ggcacctttg ggcgcagagc atgcagatga gaagtcgtgg aaggagcttt     120
```

tcgtggagag ggagttccgt agtaaaacat tcatgggacg ctatagtatt gatacattgt 180
atggtcatac cgaggctgtt aggactgttt ctgttttatc ttcaaagaaa cttctgttta 240
cttcagggta tgatcagata gtgcgaatgt gggacttgga agaaggttta tctattgcat 300
catctcgccc tcttggctgc actattcgtg cagttgcggc tgattcgagg ctcttagtag 360
cagggggtac agatggattc atacatggtt ggagagcaga ggatggaaat ccacatctct 420
ttgatcttag atcacctcag aaccagaaca tggaattcag agtttgggaa catgaaggac 480
caataacatg tctggcattg gatttgaata agatgtacag tggttcttgg gacatgagta 540
ttcgtgtttg ggatcgttct tctttgaaat gtctgaaagt tctaatgcac aatgactggg 600
tttggagcct tgctccccat gatacaacag tagcgagcgc tgcaggctca gatctttatg 660
tctgggaaca ctatattggg tcagaacttg ccaccatcac caatggtcat gct 713

<210> 80
<211> 236
<212> PRT
<213> Lycopersicon esculentum
<400> 80

Arg Leu Ala Ser Glu His His Val Trp Lys Glu Phe Tyr Cys Glu Arg
1 5 10 15
Trp Gly Gln Pro Ile Leu Leu Ala Pro Leu Gly Ala Glu His Ala Asp
20 25 30
Glu Lys Ser Trp Lys Glu Leu Phe Val Glu Arg Glu Phe Arg Ser Lys
35 40 45
Thr Phe Met Gly Arg Tyr Ser Ile Asp Thr Leu Tyr Gly His Thr Glu
50 55 60
Ala Val Arg Thr Val Ser Val Leu Ser Ser Lys Lys Leu Leu Phe Thr
65 70 75 80
Ser Gly Tyr Asp Gln Ile Val Arg Met Trp Asp Leu Glu Glu Gly Leu
85 90 95
Ser Ile Ala Ser Ser Arg Pro Leu Gly Cys Thr Ile Arg Ala Val Ala
100 105 110
Ala Asp Ser Arg Leu Leu Val Ala Gly Gly Thr Asp Gly Phe Ile His
115 120 125
Gly Trp Arg Ala Glu Asp Gly Asn Pro His Leu Phe Asp Leu Arg Ser
130 135 140
Pro Gln Asn Gln Asn Met Glu Phe Arg Val Trp Glu His Glu Gly Pro
145 150 155 160
Ile Thr Cys Leu Ala Leu Asp Leu Asn Lys Met Tyr Ser Gly Ser Trp
165 170 175
Asp Met Ser Ile Arg Val Trp Asp Arg Ser Ser Leu Lys Cys Leu Lys
180 185 190
Val Leu Met His Asn Asp Trp Val Trp Ser Leu Ala Pro His Asp Thr
195 200 205
Thr Val Ala Ser Ala Ala Gly Ser Asp Leu Tyr Val Trp Glu His Tyr
210 215 220
Ile Gly Ser Glu Leu Ala Thr Ile Thr Asn Gly His
225 230 235

<210> 81
<211> 717
<212> DNA
<213> Aquilegia formosa x Aquilegia pubescens
<400> 81

gtccctcgag actctactat tgctagtact gccggggtgg atatgtatgt ttgggacatt 60
gatagtgggg aattgcttgc tgtaattcaa aaggctcatg ttggcaacac tctctctttg 120
gcccgaagtc acacagggaa tttgattttc actggtgggg atgatgggac tataagtatg 180
tttgagcttt tggacgattc tacgctcttg catgtggcaa cttggagccc acatactggt 240
gctgtgcact ctcttgcatt tgagttccct tggcttgtgt cagcctctag cgacggaaag 300
ctctcattga ttgacattag acggttgctg aaatctagcc agcggtctgt gtcaagagac 360
ctcaaaaggg taaattgtcc agtttcgttg actgtggagg ctccacagag gatgttacat 420
ggttttggta gtaatttatt ctcagtgggc attggttctg atcggattgt atgtggtggt 480
gaggaaggtg tagttagaat ttggaacttt tcacaagcta tggagattga gcgtagggtt 540
caggccttaa ggggcatgag attagaaaac cgaatgaggc ggcggaaggc ccaaatagag 600
atgaacagca agggtggtcg ctctgatcag tgttcagttg cagctaagaa gaaccagatt 660
aatggagaca gggcctggca tggtaggcga ggagcgagtg gaaagctgaa ggcctag 717

<210> 82
<211> 238
<212> PRT
<213> Aquilegia formosa x Aquilegia pubescens
<400> 82

Val Pro Arg Asp Ser Thr Ile Ala Ser Thr Ala Gly Val Asp Met Tyr
1 5 10 15
Val Trp Asp Ile Asp Ser Gly Glu Leu Leu Ala Val Ile Gln Lys Ala
20 25 30

```
His Val Gly Asn Thr Leu Ser Leu Ala Arg Ser His Thr Gly Asn Leu
        35                  40                  45
Ile Phe Thr Gly Gly Asp Asp Gly Thr Ile Ser Met Phe Glu Leu Leu
    50                  55                  60
Asp Asp Ser Thr Leu Leu His Val Ala Thr Trp Ser Pro His Thr Gly
65                  70                  75                  80
Ala Val His Ser Leu Ala Phe Glu Phe Pro Trp Leu Val Ser Ala Ser
                85                  90                  95
Ser Asp Gly Lys Leu Ser Leu Ile Asp Ile Arg Arg Leu Leu Lys Ser
            100                 105                 110
Ser Gln Arg Ser Val Ser Arg Asp Leu Lys Arg Val Asn Cys Pro Val
        115                 120                 125
Ser Leu Thr Val Glu Ala Pro Gln Arg Met Leu His Gly Phe Gly Ser
    130                 135                 140
Asn Leu Phe Ser Val Gly Ile Gly Ser Asp Arg Ile Val Cys Gly Gly
145                 150                 155                 160
Glu Glu Gly Val Val Arg Ile Trp Asn Phe Ser Gln Ala Met Glu Ile
                165                 170                 175
Glu Arg Arg Val Gln Ala Leu Arg Gly Met Arg Leu Glu Asn Arg Met
            180                 185                 190
Arg Arg Arg Lys Ala Gln Ile Glu Met Asn Ser Lys Gly Gly Arg Ser
        195                 200                 205
Asp Gln Cys Ser Val Ala Ala Lys Lys Asn Gln Ile Asn Gly Asp Arg
    210                 215                 220
Ala Trp His Gly Arg Arg Gly Ala Ser Gly Lys Leu Lys Ala
225                 230                 235
```

<210> 83
<211> 621
<212> DNA
<213> Gossypium hirsutum
<400> 83

```
ggccgcttcg accggagttg cactggagac ttcctttta ctggtggaga agatggagcg      60
atacacatgt ttgagattat aagtgggtct gacgaatcta gtgtcgtgca ggttgcaaca     120
tggattcctc actcaggtgc tgtttactca cttgcatttg agtttccctg gcttgtttca     180
gcttccagtg atggtaaact tgcactaatt gatgttcgga agttgttgag ggccggtaag     240
cgcaccttgg ggaaaagggt ttcaagggat aatgacattg accaaaggaa catagagcca     300
ccccagagaa tgctccatgg atttgggtgc aatttgttct cagtcggtat tggtgcagat     360
cgaattgtct gtgggggtga agaaggtgtt gttcgtatct ggaacttctc agaagctcta     420
gaaattgagc agagggcacg ggcactaaga ggaatacgtt tggagaatag gatgaggcga     480
cgcagacttc aaattgagat gaataataag ggtggtcgaa ctgatcaatg ttctgttgca     540
gccaagaaga aaacagtcaa tggtgatagg aatagtgtct ggcacagtaa acgtagcata     600
agctccaagg tgaagacata a                                               621
```

<210> 84
<211> 206
<212> PRT
<213> Gossypium hirsutum
<400> 84

```
Gly Arg Phe Asp Arg Ser Cys Thr Gly Asp Phe Leu Phe Thr Gly Gly
1               5                   10                  15
Glu Asp Gly Ala Ile His Met Phe Glu Ile Ile Ser Gly Ser Asp Glu
            20                  25                  30
Ser Ser Val Val Gln Val Ala Thr Trp Ile Pro His Ser Gly Ala Val
        35                  40                  45
Tyr Ser Leu Ala Phe Glu Phe Pro Trp Leu Val Ser Ala Ser Ser Asp
    50                  55                  60
Gly Lys Leu Ala Leu Ile Asp Val Arg Lys Leu Leu Arg Ala Gly Lys
65                  70                  75                  80
Arg Thr Leu Gly Lys Arg Val Ser Arg Asp Asn Asp Ile Asp Gln Arg
                85                  90                  95
Asn Ile Glu Pro Pro Gln Arg Met Leu His Gly Phe Gly Cys Asn Leu
            100                 105                 110
Phe Ser Val Gly Ile Gly Ala Asp Arg Ile Val Cys Gly Gly Glu Glu
        115                 120                 125
Gly Val Val Arg Ile Trp Asn Phe Ser Glu Ala Leu Glu Ile Glu Gln
    130                 135                 140
Arg Ala Arg Ala Leu Arg Gly Ile Arg Leu Glu Asn Arg Met Arg Arg
145                 150                 155                 160
Arg Arg Leu Gln Ile Glu Met Asn Asn Lys Gly Gly Arg Thr Asp Gln
                165                 170                 175
Cys Ser Val Ala Ala Lys Lys Lys Thr Val Asn Gly Asp Arg Asn Ser
            180                 185                 190
```

Val Trp His Ser Lys Arg Ser Ile Ser Ser Lys Val Lys Thr
195 200 205

<210> 85
<211> 631
<212> DNA
<213> Sorghum bicolor
<400> 85

gatacatccc gtgcacttgg tggcaccatc cgggcgattg cagctgactc taggctttta 60
gtgagtggag gaactaatgc ctatattcag tgttggaggg ctgttgaagg caatgatcac 120
ttatttcaca tctctggaag tggtactgac cagaatgcgg agtttcgcct ctgggggcat 180
gaaggtcctg tgactagtct tgccttggat tcactgagga tttatagtgg ttcttgggac 240
atgactgttc gtgtttggga cagagcccag atggattgcg tgcaaaagtt catgcatgca 300
gactgggtgt ggtctcttgc tctgcgtgga aatactgttg ccagtactgc tggtagagat 360
gcatatgtat gggatatcag ggacggcgag ttaacaagct tagtttccaa tgcccatgtt 420
ggtaatgcat attcattggc ttggacacac ctggcggatg tgctgtttac tggtggagag 480
gatggcgcta ttcgcttgtt caatgtttct gatgtctctg atgatgagga ggacattaaa 540
ccagtggcaa cttgggtgcc acattcaggt cctgttcatt ctcttgcttt tgagtatcca 600
tggcttgtgt cagcctcgag tgatggcagg a 631

<210> 86
<211> 210
<212> PRT
<213> Sorghum bicolor
<400> 86

Asp Thr Ser Arg Ala Leu Gly Gly Thr Ile Arg Ala Ile Ala Ala Asp
1 5 10 15
Ser Arg Leu Leu Val Ser Gly Gly Thr Asn Ala Tyr Ile Gln Cys Trp
20 25 30
Arg Ala Val Glu Gly Asn Asp His Leu Phe His Ile Ser Gly Ser Gly
35 40 45
Thr Asp Gln Asn Ala Glu Phe Arg Leu Trp Gly His Glu Gly Pro Val
50 55 60
Thr Ser Leu Ala Leu Asp Ser Leu Arg Ile Tyr Ser Gly Ser Trp Asp
65 70 75 80
Met Thr Val Arg Val Trp Asp Arg Ala Gln Met Asp Cys Val Gln Lys
85 90 95
Phe Met His Ala Asp Trp Val Trp Ser Leu Ala Leu Arg Gly Asn Thr
100 105 110
Val Ala Ser Thr Ala Gly Arg Asp Ala Tyr Val Trp Asp Ile Arg Asp
115 120 125
Gly Glu Leu Thr Ser Leu Val Ser Asn Ala His Val Gly Asn Ala Tyr
130 135 140
Ser Leu Ala Trp Thr His Leu Ala Asp Val Leu Phe Thr Gly Gly Glu
145 150 155 160
Asp Gly Ala Ile Arg Leu Phe Asn Val Ser Asp Val Ser Asp Asp Glu
165 170 175
Glu Asp Ile Lys Pro Val Ala Thr Trp Val Pro His Ser Gly Pro Val
180 185 190
His Ser Leu Ala Phe Glu Tyr Pro Trp Leu Val Ser Ala Ser Ser Asp
195 200 205
Gly Arg
210

<210> 87
<211> 573
<212> DNA
<213> Ipomea nil
<400> 87

ggagaagacg gagccataca catgtttgag gttgtaaaaa acgcaaggtt tcatgtaaag 60
aaggttgcaa catggattcc tcactcgggc cctgtttatt ctcttgcatt tgaattcccc 120
tggcttgttt ccgcttcaag cgatggaaaa ctggcactta tcgatgtgag gaagttattg 180
aagacaagta ggtattcagc aacaggaagc cgtccttgta aggttgacaa tctggaccat 240
acaagtgttg agcctccgca acgaatgctt catggatttg ggtccaattt atttgcagtg 300
gatgttggtc tggatcgtat tgtgtgtgga ggtgaagaag gcgctgttag gatctggaac 360
ttctcacaag cgttggagat agagcagagg gtccgtgctt tacgaggttt aaggttagaa 420
aacaggatga ggaggcgtaa gcttcagtcc aacatgaata gcaaaggcac ccggagtgat 480
cagtgctcgg ttgcagcaaa gaagaaccaa atcaatggcg ataggaatag ctggcagaac 540
aagcgtaggg taagcgggaa gctcaaggct tag 573

<210> 88
<211> 190
<212> PRT
<213> Ipomea nil
<400> 88

Gly Glu Asp Gly Ala Ile His Met Phe Glu Val Val Lys Asn Ala Arg
1               5                   10                  15
Phe His Val Lys Lys Val Ala Thr Trp Ile Pro His Ser Gly Pro Val
            20                  25                  30
Tyr Ser Leu Ala Phe Glu Phe Pro Trp Leu Val Ser Ala Ser Ser Asp
        35                  40                  45
Gly Lys Leu Ala Leu Ile Asp Val Arg Lys Leu Leu Lys Thr Ser Arg
    50                  55                  60
Tyr Ser Ala Thr Gly Ser Arg Pro Cys Lys Val Asp Asn Leu Asp His
65                  70                  75                  80
Thr Ser Val Glu Pro Pro Gln Arg Met Leu His Gly Phe Gly Ser Asn
                85                  90                  95
Leu Phe Ala Val Asp Val Gly Leu Asp Arg Ile Val Cys Gly Gly Glu
            100                 105                 110
Glu Gly Ala Val Arg Ile Trp Asn Phe Ser Gln Ala Leu Glu Ile Glu
        115                 120                 125
Gln Arg Val Arg Ala Leu Arg Gly Leu Arg Leu Glu Asn Arg Met Arg
    130                 135                 140
Arg Arg Lys Leu Gln Ser Asn Met Asn Ser Lys Gly Thr Arg Ser Asp
145                 150                 155                 160
Gln Cys Ser Val Ala Ala Lys Lys Asn Gln Ile Asn Gly Asp Arg Asn
                165                 170                 175
Ser Trp Gln Asn Lys Arg Arg Val Ser Gly Lys Leu Lys Ala
            180                 185                 190

<210> 89
<211> 615
<212> DNA
<213> Solanum tuberosum
<400> 89

ttttccttgg cccgaattca cacagggaag ctccttattc cactgaaggg gaagatggag 60
ctatacgcat gttcgagatc attagaaatg ataaagcttc atctcaggcg tgtggcgaca 120
tggattcctc actcgggtgc tgtttattct cttgcatttg agttcccttg gcttgtttca 180
gcttccagtg atggaaaact ctcacttatt gatgtgagaa agctgttgag gacgaatcgg 240
agttatgtga tggagaagcc ttcgaaggtt gacaataggg ctaaaaatgt agagccacct 300
caaagaatgt tacatggatt tgggagcaat ctgtttgctg tggatattgg tcttgatcgt 360
atcgtatgtg ctggagaaga aggcattgtt aggatctgga acttctcaga agctttggag 420
gttgagcaga gagttcgagc attaagagga ttaaggttag agaacagaat gaggaggcgt 480
aaacttcagt ctgaaatgac tggtaaaggc agtcgtgctg atcagtgctc agttgctgct 540
aagaagaatc aattgaatgg tgataggaac agctggcgca acaagcgtag ggtgactggg 600
aagctgaagg cctag 615

<210> 90
<211> 204
<212> PRT
<213> Solanum tuberosum
<400> 90

Phe Ser Leu Ala Arg Ile His Thr Gly Lys Leu Leu Ile Pro Leu Lys
1               5                   10                  15
Gly Lys Met Glu Leu Tyr Ala Cys Ser Arg Ser Leu Glu Met Ile Lys
            20                  25                  30
Leu His Leu Arg Arg Val Ala Thr Trp Ile Pro His Ser Gly Ala Val
        35                  40                  45
Tyr Ser Leu Ala Phe Glu Phe Pro Trp Leu Val Ser Ala Ser Ser Asp
    50                  55                  60
Gly Lys Leu Ser Leu Ile Asp Val Arg Lys Leu Leu Arg Thr Asn Arg
65                  70                  75                  80
Ser Tyr Val Met Glu Lys Pro Ser Lys Val Asp Asn Arg Ala Lys Asn
                85                  90                  95
Val Glu Pro Pro Gln Arg Met Leu His Gly Phe Gly Ser Asn Leu Phe
            100                 105                 110
Ala Val Asp Ile Gly Leu Asp Arg Ile Val Cys Ala Gly Glu Glu Gly
        115                 120                 125
Ile Val Arg Ile Trp Asn Phe Ser Glu Ala Leu Glu Val Glu Gln Arg
    130                 135                 140
Val Arg Ala Leu Arg Gly Leu Arg Leu Glu Asn Arg Met Arg Arg Arg
145                 150                 155                 160
Lys Leu Gln Ser Glu Met Thr Gly Lys Gly Ser Arg Ala Asp Gln Cys
                165                 170                 175
Ser Val Ala Ala Lys Lys Asn Gln Leu Asn Gly Asp Arg Asn Ser Trp
            180                 185                 190
Arg Asn Lys Arg Arg Val Thr Gly Lys Leu Lys Ala
        195                 200

```
<210>  91
<211>  632
<212>  DNA
<213>  Zamia fischeri
<400>  91
ggcagacatt ctcaatcgcc tcaatgcaag agaactcagc atagtttcat gtgtatgtac     60
cctctttcga aggatttctt cagatagcca tggatggaag gagttctact gtgagagatg    120
ggggcttcct gcgcctagca aaatttccgc gtcttcggcc ccgggaccag agaagtcgtg    180
gagagagttg tatttggaga gagatgcccg aagcaaggcc ttcctgggcc gatttaatgt    240
agacatgctg catggccaca ctgcagctct tcgatctgtt tgccttctgc catctgcaaa    300
tctcatattt acagcaggat atgacacagt actcagaatg tggaacatgg aggaaggtct    360
cttgatcgct tgttcccggc ctcttggttg cacgctccgt gccatagcgg cagacatgga    420
tatgttggtg gtggcaggaa ctgatccttt cgtgcgctgt tggcaagcaa tttctgagct    480
tcctaactta tttgacattt cggggggtttc agggcagaac actgaatctt gccttcgtgg    540
acatgttgga cccataactt gccttggcct agattcgttg aaaatttaca gtggctcttg    600
ggacatgagc attcgggtat gggatagggt ta                                  632
<210>  92
<211>  210
<212>  PRT
<213>  Zamia fischeri
<400>  92
Ala Asp Ile Leu Asn Arg Leu Asn Ala Arg Glu Leu Ser Ile Val Ser
1               5                   10                  15
Cys Val Cys Thr Leu Phe Arg Arg Ile Ser Ser Asp Ser His Gly Trp
            20                  25                  30
Lys Glu Phe Tyr Cys Glu Arg Trp Gly Leu Pro Ala Pro Ser Lys Ile
        35                  40                  45
Ser Ala Ser Ser Ala Pro Gly Pro Glu Lys Ser Trp Arg Glu Leu Tyr
    50                  55                  60
Leu Glu Arg Asp Ala Arg Ser Lys Ala Phe Leu Gly Arg Phe Asn Val
65                  70                  75                  80
Asp Met Leu His Gly His Thr Ala Ala Leu Arg Ser Val Cys Leu Leu
                85                  90                  95
Pro Ser Ala Asn Leu Ile Phe Thr Ala Gly Tyr Asp Thr Val Leu Arg
            100                 105                 110
Met Trp Asn Met Glu Glu Gly Leu Leu Ile Ala Cys Ser Arg Pro Leu
        115                 120                 125
Gly Cys Thr Leu Arg Ala Ile Ala Ala Asp Met Asp Met Leu Val Val
    130                 135                 140
Ala Gly Thr Asp Pro Phe Val Arg Cys Trp Gln Ala Ile Ser Glu Leu
145                 150                 155                 160
Pro Asn Leu Phe Asp Ile Ser Gly Val Ser Gly Gln Asn Thr Glu Ser
                165                 170                 175
Cys Leu Arg Gly His Val Gly Pro Ile Thr Cys Leu Gly Leu Asp Ser
            180                 185                 190
Leu Lys Ile Tyr Ser Gly Ser Trp Asp Met Ser Ile Arg Val Trp Asp
        195                 200                 205
Arg Val
    210
<210>  93
<211>  507
<212>  DNA
<213>  Persea americana
<400>  93
gcgacatggc ttcctcacac gggctctgtt aattctctag catttgagtt cccatggctt     60
gtgtcatctt ccagtgatgg acgacttgct ctgattgatg tgagaaagct gttaaggtca    120
agccgacgta cgtcatcaag acaccatgtt aaagctaaac ggtctgcccc cgtaaatgtg    180
gagccgcctc aaaggatgtt gcatgggtat gggtgcaatt tgttttctgt ggatattggc    240
gcggatagga ttgtttgtgg tggagaggag ggtgttgtgc gtatctggaa cttctctaag    300
gcacttgaga ttgagcagag gattcgggct ctgaaaggga tacggttgga gaaccgaatg    360
aggcggcgga agatacaatt agagatgagc agtaagaatc gacctggaga tcaatgctct    420
gttgcagcca aaggaatca gatgcctggt gatagaaaca gggtttggcg tggaaggcgc    480
aatatgagtg aaaagccgaa ggcctaa                                        507
<210>  94
<211>  168
<212>  PRT
<213>  Persea americana
<400>  94
Ala Thr Trp Leu Pro His Thr Gly Ser Val Asn Ser Leu Ala Phe Glu
1               5                   10                  15
Phe Pro Trp Leu Val Ser Ser Ser Ser Asp Gly Arg Leu Ala Leu Ile
```

```
            20                  25                  30
Asp Val Arg Lys Leu Leu Arg Ser Ser Arg Arg Thr Ser Ser Arg His
        35                  40                  45
His Val Lys Ala Lys Arg Ser Ala Pro Val Asn Val Glu Pro Pro Gln
    50                  55                  60
Arg Met Leu His Gly Tyr Gly Cys Asn Leu Phe Ser Val Asp Ile Gly
65                  70                  75                  80
Ala Asp Arg Ile Val Cys Gly Gly Glu Glu Gly Val Val Arg Ile Trp
                85                  90                  95
Asn Phe Ser Lys Ala Leu Glu Ile Glu Gln Arg Ile Arg Ala Leu Lys
            100                 105                 110
Gly Ile Arg Leu Glu Asn Arg Met Arg Arg Arg Lys Ile Gln Leu Glu
        115                 120                 125
Met Ser Ser Lys Asn Arg Pro Gly Asp Gln Cys Ser Val Ala Ala Lys
    130                 135                 140
Arg Asn Gln Met Pro Gly Asp Arg Asn Arg Val Trp Arg Gly Arg Arg
145                 150                 155                 160
Asn Met Ser Glu Lys Pro Lys Ala
                165
```

<210> 95
<211> 498
<212> DNA
<213> Glycine max
<220>
<221> misc_feature
<222> (127)..(127)
<223> n is a, c, g, or t
<400> 95

```
gttgctgctt ggattcctca ctctgcccct gtgtattccc tggcctttga gtttccatgg      60
cttgtttctg catcaagtga tggccagcta gcactaattg atgtgagaaa gctgttgagg     120
attagcnagc gagctctagg gaaaagagtt tcaaaggtaa agcatttggg tggagacata     180
gtggagcctc cacagaggat gttgcatgga tttaagagcc atcttttctc tgtggatata     240
ggagctgaac gaattgtcag tggaggtgaa gaaggtgttg tcaggatctg gaatttcacg     300
gaagctttgg aaattgaacg gagagcccga gcattaagag gaatacgatt agagcacaga     360
atgaggcgac ggaagcttca aacagagctg agccataaaa gtggtcggag tgatcagtgt     420
tcagttgcag cccagaaaaa ttctgtcact tgtatttggc ccactaaacg tggcatgagc     480
ggaaagctga aagcatag                                                   498
```

<210> 96
<211> 165
<212> PRT
<213> Glycine max
<220>
<221> UNSURE
<222> (43)..(43)
<223> Xaa can be any naturally occurring amino acid
<400> 96

```
Val Ala Ala Trp Ile Pro His Ser Ala Pro Val Tyr Ser Leu Ala Phe
1               5                   10                  15
Glu Phe Pro Trp Leu Val Ser Ala Ser Ser Asp Gly Gln Leu Ala Leu
            20                  25                  30
Ile Asp Val Arg Lys Leu Leu Arg Ile Ser Xaa Arg Ala Leu Gly Lys
        35                  40                  45
Arg Val Ser Lys Val Lys His Leu Gly Gly Asp Ile Val Glu Pro Pro
    50                  55                  60
Gln Arg Met Leu His Gly Phe Lys Ser His Leu Phe Ser Val Asp Ile
65                  70                  75                  80
Gly Ala Glu Arg Ile Val Ser Gly Gly Glu Glu Gly Val Val Arg Ile
                85                  90                  95
Trp Asn Phe Thr Glu Ala Leu Glu Ile Glu Arg Arg Ala Arg Ala Leu
            100                 105                 110
Arg Gly Ile Arg Leu Glu His Arg Met Arg Arg Arg Lys Leu Gln Thr
        115                 120                 125
Glu Leu Ser His Lys Ser Gly Arg Ser Asp Gln Cys Ser Val Ala Ala
    130                 135                 140
Gln Lys Asn Ser Val Thr Cys Ile Trp Pro Thr Lys Arg Gly Met Ser
145                 150                 155                 160
Gly Lys Leu Lys Ala
                165
```

<210> 97
<211> 12
<212> PRT

<213> Artificial sequence
<220>
<223> motif 1
<220>
<221> UNSURE
<222> (3)..(3)
<223> Xaa can be any naturally occurring amino acid
<220>
<221> VARIANT
<222> (4)..(4)
<223> /replace = "Val" /replace = "Leu"
<220>
<221> VARIANT
<222> (6)..(6)
<223> /replace = "Arg" /replace = "Gly"
<220>
<221> UNSURE
<222> (11)..(11)
<223> Xaa can be any naturally occurring amino acid
<400> 97
Trp Lys Xaa Phe Tyr Cys Glu Arg Trp Gly Xaa Pro
1 5 10
<210> 98
<211> 16
<212> PRT
<213> Artificial sequence
<220>
<223> motif 2
<220>
<221> VARIANT
<222> (3)..(3)
<223> /replace = "Ser"
<220>
<221> VARIANT
<222> (6)..(6)
<223> /replace = "Tyr"
<220>
<221> VARIANT
<222> (12)..(12)
<223> /replace = "Ser"
<220>
<221> VARIANT
<222> (14)..(14)
<223> /replace = "Ala" /replace = "Gly"
<400> 98
Ser Leu Ala Phe Glu Phe Pro Trp Leu Val Ser Ala Ser Ser Asp Gly
1 5 10 15
<210> 99
<211> 15
<212> PRT
<213> Artificial sequence
<220>
<223> motif 3
<220>
<221> VARIANT
<222> (2)..(2)
<223> /replace = "Phe"
<220>
<221> VARIANT
<222> (8)..(8)
<223> replace = "Thr"
<220>
<221> VARIANT
<222> (9)..(9)
<223> replace = "Ser"
<220>
<221> VARIANT
<222> (10)..(10)
<223> replace = "Ile"
<220>
<221> VARIANT

```
<222>  (12)..(12)
<223>  replace = "Ile"
<400>  99
Ile Tyr Ser Gly Ser Trp Asp Met Thr Val Arg Val Trp Asp Arg
1               5                   10                  15
<210>  100
<211>  7
<212>  PRT
<213>  Artificial sequence
<220>
<223>  motif 4
<220>
<221>  VARIANT
<222>  (5)..(5)
<223>  /replace = "Asp"
<400>  100
Phe Thr Gly Gly Glu Asp Gly
1               5
<210>  101
<211>  9
<212>  PRT
<213>  Artificial sequence
<220>
<223>  motif 5
<220>
<221>  VARIANT
<222>  (2)..(2)
<223>  /replace = "Ala"
<400>  101
Glu Pro Pro Gln Arg Met Leu His Gly
1               5
<210>  102
<211>  38
<212>  PRT
<213>  Artificial sequence
<220>
<223>  conserved region 1
<400>  102
Ser Gln Trp Met Pro His Thr Ser Pro Val Tyr Ser Leu Ser Phe Glu
1               5                   10                  15
Phe Pro Trp Leu Val Ser Ala Ser Gly Asp Gly Lys Leu Ala Leu Ile
            20                  25                  30
Asp Val Arg Lys Leu Leu
        35
<210>  103
<211>  65
<212>  PRT
<213>  Artificial sequence
<220>
<223>  conserved region 2
<400>  103
Val Glu Pro Pro Gln Arg Met Leu His Gly Phe Gly Ser Asn Leu Phe
1               5                   10                  15
Ser Val Asp Val Gly Tyr Asp Arg Ile Val Cys Gly Gly Glu Glu Gly
            20                  25                  30
Thr Val Arg Ile Trp Asn Phe Thr Gln Ala Leu Glu Ile Glu Arg Arg
        35                  40                  45
Thr Arg Ala Leu Lys Gly Met Arg His Glu Asn Arg Met Arg Arg Arg
    50                  55                  60
Arg
65
<210>  104
<211>  1167
<212>  DNA
<213>  Oryza sativa
<400>  104
atccaagcca agaagaggga gagcaccaag gacacgcgac tagcagaagc cgagcgaccg      60
ccttcttcga tccatatctt ccggtcgagt tcttggtcga tctcttccct cctccacctc     120
ctcctcacag ggtatgtgcc cttcggttgt tcttggattt attgttctag gttgtgtagt     180
acgggcgttg atgttaggaa aggggatctg tatctgtgat gattcctgtt cttggatttg     240
ggatagaggg gttcttgatg ttgcatgtta tcggttcggt ttgattagta gtatggtttt     300
```

caatcgtctg gagagctcta tggaaatgaa atggtttagg gtacggaatc ttgcgatttt 360
gtgagtacct tttgtttgag gtaaaatcag agcaccggtg attttgcttg gtgtaataaa 420
agtacggttg tttggtcctc gattctggta gtgatgcttc tcgatttgac gaagctatcc 480
tttgtttatt ccctattgaa caaaaataat ccaactttga agacggtccc gttgatgaga 540
ttgaatgatt gattcttaag cctgtccaaa atttcgcagc tggcttgttt agatacagta 600
gtccccatca cgaaattcat ggaaacagtt ataatcctca ggaacagggg attccctgtt 660
cttccgattt gctttagtcc cagaattttt tttcccaaat atcttaaaaa gtcactttct 720
ggttcagttc aatgaattga ttgctacaaa taatgctttt atagcgttat cctagctgta 780
gttcagttaa taggtaatac ccctatagtt tagtcaggag aagaacttat ccgatttctg 840
atctccattt ttaattatat gaaatgaact gtagcataag cagtattcat ttggattatt 900
ttttttatta gctctcaccc cttcattatt ctgagctgaa agtctggcat gaactgtcct 960
caattttgtt ttcaaattca catcgattat ctatgcatta tcctcttgta tctacctgta 1020
gaagtttctt tttggttatt ccttgactgc ttgattacag aaagaaattt atgaagctgt 1080
aatcgggata gttatactgc ttgttcttat gattcatttc ctttgtgcag ttcttggtgt 1140
agcttgccac tttcaccagc aaagttc 1167

<210> 105
<211> 55
<212> DNA
<213> Artificial sequence
<220>
<223> primer: prm6999
<400> 105
ggggacaagt ttgtacaaaa aagcaggctt aaacaatgaa tcgtttttct cgttt 55

<210> 106
<211> 49
<212> DNA
<213> Artificial sequence
<220>
<223> primer: prm7000
<400> 106
ggggaccact ttgtacaaga aagctgggta tccaatctta tcgcttagg 49

<210> 107
<211> 1602
<212> DNA
<213> Brassica rapa
<400> 107
atggaattag agtgccaaga gagagctgaa gttgcgattg ttggcgaagg tttatccaat 60
caaggagctg agttgagaat tggagatggg tctgtcgagg ttccatgtgt gaagctttgt 120
tatcagcaaa agaagtcaac tttggtggtg cccagggaca aggatttgtc tacaaatact 180
catcgataca ccattatcga tttgcctcag gctttgatat ccgagattct taactgcctt 240
gacccaaaag agttgggcct tgtgtcctgt gtttcgactt gtctgcatag gttagcttcg 300
gagcatcacg cgtggaaagg cttctactgc gagagatggg ggctccccgt cgtcttaggg 360
ggtaaaactt ctgaagagag gtcatggaaa gagctgttta ttgagaggga gtttaggagc 420
aagacttttt taggacgtca tagtattgat accctttatg gtcacactga agcagttcgc 480
actgtgtttc ttttagcttc tgctaagctc attttcactt ctggatatga ctgcgttgtt 540
cggatgtggg gaatggaaga aggcttgtct attgcagcgt ctaaaccgct tggttgtact 600
attagagcag ttgcggctga tacgaagctc ttggtagctg gcggtactga tggttttata 660
cattgctgga aagcagtgga tggtctgaga gacttgtttg acctcacggg ttttcagaaa 720
gagaagacgg agtttcgcct ttgggagcat gagggtccta ttacatctct tgccctggat 780
atgacgagta tctttagcgg ttcgtgggac atgtctgttc gtatatggga tagatcttca 840
ttcaagtgta tcaaaacgtt gaggcatagc gattgggttt ggggactagc gcctcatgag 900
acaagcatcg cgtgtgctgc tggttcggat gtgtacattt gggacattag cagtgagact 960
ccagaggcga ttatccatga tgctcatgag ggtaacactt actctcttgc aagaagccac 1020
agtgggggatt tcttgtttac tggtggggaa gatggaggga tcaaaatgtt tgagatcaga 1080
aaacacggta atgaaacaag cgtcctgctc atatctcaat ggatgcctca cactggtccc 1140
gtctactctc tttctttcga gttcccctgg cttgtatcag catctggtga cggtaaactc 1200
gctcttatcg acgttaggaa gttgttgaag actaaccgtc gtggcttacc caagagggtt 1260
tcttcgagaa ttgtggaacc cccacagaga atgctgcacg ggttcggttc aaacctgttc 1320
tctgtggacg tgggctgtga ccgtatagtt tgtggaggtg aagaaggcgt agttcggata 1380
tggaacttca cacaagcgtt ggagattgag aggagagctc gagctctgaa aggaatgagg 1440
cttgagaaca ggatgaggcg aaggaggatg cagatggaga tgaatgcgaa gagtggaagg 1500
cctgaccaat gctcgattgc tgctcataag aaccctgtta atggagatag gaatcgagcg 1560
tggcatacaa aacgaagagc aagtggcaag gcgaaggcct aa 1602

<210> 108
<211> 533
<212> PRT
<213> Brassica rapa
<400> 108
Met Glu Leu Glu Cys Gln Glu Arg Ala Glu Val Ala Ile Val Gly Glu
1               5                   10                  15
Gly Leu Ser Asn Gln Gly Ala Glu Leu Arg Ile Gly Asp Gly Ser Val

```
                20                  25                  30
Glu Val Pro Cys Val Lys Leu Cys Tyr Gln Gln Lys Lys Ser Thr Leu
            35                  40                  45
Val Val Pro Arg Asp Lys Asp Leu Ser Thr Asn Thr His Arg Tyr Thr
    50                  55                  60
Ile Ile Asp Leu Pro Gln Ala Leu Ile Ser Glu Ile Leu Asn Cys Leu
65                  70                  75                  80
Asp Pro Lys Glu Leu Gly Leu Val Ser Cys Val Ser Thr Cys Leu His
                85                  90                  95
Arg Leu Ala Ser Glu His His Ala Trp Lys Gly Phe Tyr Cys Glu Arg
            100                 105                 110
Trp Gly Leu Pro Val Val Leu Gly Gly Lys Thr Ser Glu Glu Arg Ser
        115                 120                 125
Trp Lys Glu Leu Phe Ile Glu Arg Glu Phe Arg Ser Lys Thr Phe Leu
    130                 135                 140
Gly Arg His Ser Ile Asp Thr Leu Tyr Gly His Thr Glu Ala Val Arg
145                 150                 155                 160
Thr Val Phe Leu Leu Ala Ser Ala Lys Leu Ile Phe Thr Ser Gly Tyr
                165                 170                 175
Asp Cys Val Val Arg Met Trp Gly Met Glu Glu Gly Leu Ser Ile Ala
            180                 185                 190
Ala Ser Lys Pro Leu Gly Cys Thr Ile Arg Ala Val Ala Ala Asp Thr
        195                 200                 205
Lys Leu Leu Val Ala Gly Gly Thr Asp Gly Phe Ile His Cys Trp Lys
    210                 215                 220
Ala Val Asp Gly Leu Arg Asp Leu Phe Asp Leu Thr Gly Phe Gln Lys
225                 230                 235                 240
Glu Lys Thr Glu Phe Arg Leu Trp Glu His Glu Gly Pro Ile Thr Ser
                245                 250                 255
Leu Ala Leu Asp Met Thr Ser Ile Phe Ser Gly Ser Trp Asp Met Ser
            260                 265                 270
Val Arg Ile Trp Asp Arg Ser Ser Phe Lys Cys Ile Lys Thr Leu Arg
        275                 280                 285
His Ser Asp Trp Val Trp Gly Leu Ala Pro His Glu Thr Ser Ile Ala
    290                 295                 300
Cys Ala Ala Gly Ser Asp Val Tyr Ile Trp Asp Ile Ser Ser Glu Thr
305                 310                 315                 320
Pro Glu Ala Ile Ile His Asp Ala His Glu Gly Asn Thr Tyr Ser Leu
                325                 330                 335
Ala Arg Ser His Ser Gly Asp Phe Leu Phe Thr Gly Gly Glu Asp Gly
            340                 345                 350
Gly Ile Lys Met Phe Glu Ile Arg Lys His Gly Asn Glu Thr Ser Val
        355                 360                 365
Leu Leu Ile Ser Gln Trp Met Pro His Thr Gly Pro Val Tyr Ser Leu
    370                 375                 380
Ser Phe Glu Phe Pro Trp Leu Val Ser Ala Ser Gly Asp Gly Lys Leu
385                 390                 395                 400
Ala Leu Ile Asp Val Arg Lys Leu Leu Lys Thr Asn Arg Arg Gly Leu
                405                 410                 415
Pro Lys Arg Val Ser Ser Arg Ile Val Glu Pro Pro Gln Arg Met Leu
            420                 425                 430
His Gly Phe Gly Ser Asn Leu Phe Ser Val Asp Val Gly Cys Asp Arg
        435                 440                 445
Ile Val Cys Gly Gly Glu Glu Gly Val Val Arg Ile Trp Asn Phe Thr
    450                 455                 460
Gln Ala Leu Glu Ile Glu Arg Arg Ala Arg Ala Leu Lys Gly Met Arg
465                 470                 475                 480
Leu Glu Asn Arg Met Arg Arg Arg Arg Met Gln Met Glu Met Asn Ala
                485                 490                 495
Lys Ser Gly Arg Pro Asp Gln Cys Ser Ile Ala Ala His Lys Asn Pro
            500                 505                 510
Val Asn Gly Asp Arg Asn Arg Ala Trp His Thr Lys Arg Arg Ala Ser
        515                 520                 525
Gly Lys Ala Lys Ala
    530
```

<210> 109
<211> 1716
<212> DNA
<213> Sorghum bicolor
<400> 109

```
atggcctttg actgcaacaa ggaaagagga gattcttcgc ctgataattg ctcccgcatt     60
```

```
tgcactgagg gtactctcgt ccaggcaaat cccttatctc actactggaa gcctaagggt    120
ttgaagagcc tcaacaaatt ggagaaccag aagtccagtc atggatctgt tccgagagac    180
gataatccag aacaagaagc tgaagcgagc gatgaggcat ctgcctcaac ctgtggcatc    240
aggtgcttca ccgatctgcc ggctgctttg gtctgcgagg tccttgcacg cctcgatgca    300
aaggaccttg gaattgtatc ctgtgtctcc accctcctgc atgccttagc cacagatcat    360
cagggatgga agaagttata ctgcgaaagg tgggggcttc cagaccttcc cgacgccctg    420
aatgggccct tgttgcctgg tgcccccta gatgggaagt cttggaaaac atttttcgtg    480
gagcgggagt tcaggagtaa atcattcatg ggtagattca atgtggatat tctccgtggc    540
cacaatgagg atgttcgagc cgtcttcctt ttggtatcag caaatctgat attcactggc    600
gtcactggcg gccgcgattc tgtggttaga atgtggaaaa tggaggaagg gttattgatt    660
gatacatccc gtgcacttgg tggcaccatc cgggcgattg cagctgactc taggctttta    720
gtgagtggag gaactaatgc ctatattcag tgttggaggg ctgttgaagg caatgatcac    780
ttatttcaca tctctggaag tggtactgac cagaatgcgg agtttcgcct ctgggggcat    840
gaaggtcctg tgactagtct tgccttggat tcactgagga tttatagtgg ttcttgggac    900
atgactgttc gtgtttggga cagagcccag atggattgcg tgcaaaagtt catgcatgca    960
gactgggtgt ggtctcttgc tctgcgtgga aatactgttg ccagtactgc tggtagagat   1020
gcatatgtat gggatatcag ggacggcgag ttaacaagct tagtttccaa tgcccatgtt   1080
ggtaatgcat attcattggc ttggacacac ctggcggatg tgctgtttac tggtggagag   1140
gatggcgcta ttcgcttgtt caatgtttct gatgtctctg atgatgagga ggacattaaa   1200
ccagtggcaa cttgggtgcc acattcaggt cctgttcatt ctcttgcttt tgagtatcca   1260
tggcttgtgt cagcctcgag tgatggcagg agtgatggca ggattgcact tattgatttg   1320
aggaagcttc tgactcccaa aagatcatca aaaggtctgc gtgttaagag ctttgatgca   1380
agtgccatag agcctccaca gaggatgctt catggcgttg gatgtgatct cttctccatc   1440
gccattggtg cagataggat tgtatgtgca ggtgaggatg gttctgttag agtctggaac   1500
ttctcagaag cactggagat tgagaggagg gcacaggctc taaggagttt gaggcaggag   1560
aaccgcatga ggcggaggaa ggcacaagta gagatgaatg caaatggtag aaggcctgac   1620
cagtgctcaa tagccatgaa aaagaaccaa ctgaaggctg ataagagtgc cacttggcac   1680
aacaagcgtg ccgttaatga gaaggccaag tcttag                             1716
```

<210> 110
<211> 571
<212> PRT
<213> Sorghum bicolor
<400> 110

```
Met Ala Phe Asp Cys Asn Lys Glu Arg Gly Asp Ser Ser Pro Asp Asn
1               5                   10                  15
Cys Ser Arg Ile Cys Thr Glu Gly Thr Leu Val Gln Ala Asn Pro Leu
            20                  25                  30
Ser His Tyr Trp Lys Pro Lys Gly Leu Lys Ser Leu Asn Lys Leu Glu
        35                  40                  45
Asn Gln Lys Ser Ser His Gly Ser Val Pro Arg Asp Asp Asn Pro Glu
    50                  55                  60
Gln Glu Ala Glu Ala Ser Asp Glu Ala Ser Ala Ser Thr Cys Gly Ile
65                  70                  75                  80
Arg Cys Phe Thr Asp Leu Pro Ala Ala Leu Val Cys Glu Val Leu Ala
                85                  90                  95
Arg Leu Asp Ala Lys Asp Leu Gly Ile Val Ser Cys Val Ser Thr Leu
            100                 105                 110
Leu His Ala Leu Ala Thr Asp His Gln Gly Trp Lys Lys Leu Tyr Cys
        115                 120                 125
Glu Arg Trp Gly Leu Pro Asp Leu Pro Asp Ala Leu Asn Gly Pro Leu
    130                 135                 140
Leu Pro Gly Ala Pro Leu Asp Gly Lys Ser Trp Lys Thr Phe Phe Val
145                 150                 155                 160
Glu Arg Glu Phe Arg Ser Lys Ser Phe Met Gly Arg Phe Asn Val Asp
                165                 170                 175
Ile Leu Arg Gly His Asn Glu Asp Val Arg Ala Val Phe Leu Leu Val
            180                 185                 190
Ser Ala Asn Leu Ile Phe Thr Gly Val Thr Gly Gly Arg Asp Ser Val
        195                 200                 205
Val Arg Met Trp Lys Met Glu Glu Gly Leu Leu Ile Asp Thr Ser Arg
    210                 215                 220
Ala Leu Gly Gly Thr Ile Arg Ala Ile Ala Ala Asp Ser Arg Leu Leu
225                 230                 235                 240
Val Ser Gly Gly Thr Asn Ala Tyr Ile Gln Cys Trp Arg Ala Val Glu
                245                 250                 255
Gly Asn Asp His Leu Phe His Ile Ser Gly Ser Gly Thr Asp Gln Asn
            260                 265                 270
Ala Glu Phe Arg Leu Trp Gly His Glu Gly Pro Val Thr Ser Leu Ala
        275                 280                 285
Leu Asp Ser Leu Arg Ile Tyr Ser Gly Ser Trp Asp Met Thr Val Arg
    290                 295                 300
```

```
Val Trp Asp Arg Ala Gln Met Asp Cys Val Gln Lys Phe Met His Ala
305                 310                 315                 320
Asp Trp Val Trp Ser Leu Ala Leu Arg Gly Asn Thr Val Ala Ser Thr
                325                 330                 335
Ala Gly Arg Asp Ala Tyr Val Trp Asp Ile Arg Asp Gly Glu Leu Thr
            340                 345                 350
Ser Leu Val Ser Asn Ala His Val Gly Asn Ala Tyr Ser Leu Ala Trp
        355                 360                 365
Thr His Leu Ala Asp Val Leu Phe Thr Gly Gly Glu Asp Gly Ala Ile
    370                 375                 380
Arg Leu Phe Asn Val Ser Asp Val Ser Asp Asp Glu Glu Asp Ile Lys
385                 390                 395                 400
Pro Val Ala Thr Trp Val Pro His Ser Gly Pro Val His Ser Leu Ala
                405                 410                 415
Phe Glu Tyr Pro Trp Leu Val Ser Ala Ser Ser Asp Gly Arg Ser Asp
            420                 425                 430
Gly Arg Ile Ala Leu Ile Asp Leu Arg Lys Leu Leu Thr Pro Lys Arg
        435                 440                 445
Ser Ser Lys Gly Leu Arg Val Lys Ser Phe Asp Ala Ser Ala Ile Glu
    450                 455                 460
Pro Pro Gln Arg Met Leu His Gly Val Gly Cys Asp Leu Phe Ser Ile
465                 470                 475                 480
Ala Ile Gly Ala Asp Arg Ile Val Cys Ala Gly Glu Asp Gly Ser Val
                485                 490                 495
Arg Val Trp Asn Phe Ser Glu Ala Leu Glu Ile Glu Arg Arg Ala Gln
            500                 505                 510
Ala Leu Arg Ser Leu Arg Gln Glu Asn Arg Met Arg Arg Arg Lys Ala
        515                 520                 525
Gln Val Glu Met Asn Ala Asn Gly Arg Arg Pro Asp Gln Cys Ser Ile
    530                 535                 540
Ala Met Lys Lys Asn Gln Leu Lys Ala Asp Lys Ser Ala Thr Trp His
545                 550                 555                 560
Asn Lys Arg Ala Val Asn Glu Lys Ala Lys Ser
                565                 570
```

```
<210>  111
<211>  1779
<212>  DNA
<213>  Vitis vinifera
<400>  111
atggcatttg aatgccaaga gagtatagag gtttgtttgg tgaaaaattc aatagattct      60
gatgaacaac aggstggatt gagtgatttt aattccaatt ttaatcatat cacttcaatt     120
tttgatgcca aatctcaatt ggaaaccgaa actgcacacc gagaaagtgg agtggtttgt     180
ttgttgaaga attcaattga agaacgggct ggattgactc agtttagtcc cgattctgat     240
cacaatactt taatacrtga ctcgggaaat tcccaatcgg aaattgaaat cggaatccaa     300
aaaccttcaa cttcaaaccc caaagttaac gacacttcag gacattatcg taggttgata     360
actgatcttc cgtccgcgtt gatatcggaa attcttaatt gccttgaccc gaaagagctt     420
ggtgtggttt cgtgtgtctc aactgttctc aataggctag cgtcygagca ctccgtttgg     480
aaagatttct attgtgagag gtggggagct ccggttgttt cggggttttc agatgagaaa     540
tcatggaggg aattgtttgt ggagagggag tttaggagca aaacctttag gggacgattt     600
agcattgatg ttctgtatgg tcacactgag gcggttcgag ctgttttcct tttggcctct     660
gcaaagctca ttttcacttc tgggtatgat tccattgttc gaatgtggga tatggaagaa     720
gggttgtcaa ttgcgtgttc acggcctctc ggttgcacaa taagagcagt ggctgcggat     780
aagaaactgt tgcttgcggg gggtagtgat gggtttattc attgttggag agctgtagag     840
gggctctctt gcttgtttga ccttgtgggt tctcaaaacc tgagtactga gttccgaatt     900
tgggaacatg aaggtcctgt gacttgtctt gctttggata ttaagagaat ttatagtggc     960
tcatgggaca tgactgtgcg catatgggac cgttcttctt ttaaggttgt aaaggtcttg    1020
aggcacacag actgggtttg gggccttgtt cctcgtgata ctacagttgc tagcacttct    1080
ggctcagatg tgtatgtttg ggacgctgat agtgggactc tgctgacgat aatctctaat    1140
gctcatgtgg gtaatgctta tgctttggca cggagccaca caggggattt tctattcact    1200
gggggagaag atggggcaat acatatgttt gaggtcgtga gtgattgcat ggaaaggaat    1260
gtattggagg tttcaacgtg gattcctcat tctggtcctg ttcattccct ggcatttgag    1320
tttccctggc ttgtttcagc ttcagctgat gggaggatgt cactgattga tgtgagaaag    1380
cttttgcaaa cttgcaagcc ttccttaggg aagaatgttt ccaaggttag gcacagggac    1440
cacaaaagtg tggagccccc tcagaggatg ttacatgggt ttggctgcaa tttattctca    1500
gtggacattg gtgcagatcg tattgtctgt ggaggtgagg aaggtgtggt tagaatttgg    1560
aacttctcac aagctttaga agcagagcag agggcccgtg ctttaagagg aatacgtcta    1620
gaaaacagga tgaggcggcg taggcttcaa atagagctga ctagtaaggg tagtcgaact    1680
gatcaatgtt cagttgcagc cagtaagaat cctattaatg gtgataggag tggtgtgtgg    1740
cacaataagc ggggaatgag tggcaggatg aaagcatag                           1779
<210>  112
<211>  592
```

<212> PRT
<213> Vitis vinifera
<220>
<221> UNSURE
<222> (25)..(25)
<223> Xaa can be any naturally occurring amino acid
<220>
<221> UNSURE
<222> (86)..(86)
<223> Xaa can be any naturally occurring amino acid
<400> 112

```
Met Ala Phe Glu Cys Gln Glu Ser Ile Glu Val Cys Leu Val Lys Asn
1               5                   10                  15
Ser Ile Asp Ser Asp Glu Gln Gln Xaa Gly Leu Ser Asp Phe Asn Ser
            20                  25                  30
Asn Phe Asn His Ile Thr Ser Ile Phe Asp Ala Lys Ser Gln Leu Glu
        35                  40                  45
Thr Glu Thr Ala His Arg Glu Ser Gly Val Val Cys Leu Leu Lys Asn
    50                  55                  60
Ser Ile Glu Glu Arg Ala Gly Leu Thr Gln Phe Ser Pro Asp Ser Asp
65                  70                  75                  80
His Asn Thr Leu Ile Xaa Asp Ser Gly Asn Ser Gln Ser Glu Ile Glu
                85                  90                  95
Ile Gly Ile Gln Lys Pro Ser Thr Ser Asn Pro Lys Val Asn Asp Thr
            100                 105                 110
Ser Gly His Tyr Arg Arg Leu Ile Thr Asp Leu Pro Ser Ala Leu Ile
        115                 120                 125
Ser Glu Ile Leu Asn Cys Leu Asp Pro Lys Glu Leu Gly Val Val Ser
    130                 135                 140
Cys Val Ser Thr Val Leu Asn Arg Leu Ala Ser Glu His Ser Val Trp
145                 150                 155                 160
Lys Asp Phe Tyr Cys Glu Arg Trp Gly Ala Pro Val Val Ser Gly Phe
                165                 170                 175
Ser Asp Glu Lys Ser Trp Arg Glu Leu Phe Val Glu Arg Glu Phe Arg
            180                 185                 190
Ser Lys Thr Phe Arg Gly Arg Phe Ser Ile Asp Val Leu Tyr Gly His
        195                 200                 205
Thr Glu Ala Val Arg Ala Val Phe Leu Leu Ala Ser Ala Lys Leu Ile
    210                 215                 220
Phe Thr Ser Gly Tyr Asp Ser Ile Val Arg Met Trp Asp Met Glu Glu
225                 230                 235                 240
Gly Leu Ser Ile Ala Cys Ser Arg Pro Leu Gly Cys Thr Ile Arg Ala
                245                 250                 255
Val Ala Ala Asp Lys Lys Leu Leu Leu Ala Gly Gly Ser Asp Gly Phe
            260                 265                 270
Ile His Cys Trp Arg Ala Val Glu Gly Leu Ser Cys Leu Phe Asp Leu
        275                 280                 285
Val Gly Ser Gln Asn Leu Ser Thr Glu Phe Arg Ile Trp Glu His Glu
    290                 295                 300
Gly Pro Val Thr Cys Leu Ala Leu Asp Ile Lys Arg Ile Tyr Ser Gly
305                 310                 315                 320
Ser Trp Asp Met Thr Val Arg Ile Trp Asp Arg Ser Ser Phe Lys Val
                325                 330                 335
Val Lys Val Leu Arg His Thr Asp Trp Val Trp Gly Leu Val Pro Arg
            340                 345                 350
Asp Thr Thr Val Ala Ser Thr Ser Gly Ser Asp Val Tyr Val Trp Asp
        355                 360                 365
Ala Asp Ser Gly Thr Leu Leu Thr Ile Ile Ser Asn Ala His Val Gly
    370                 375                 380
Asn Ala Tyr Ala Leu Ala Arg Ser His Thr Gly Asp Phe Leu Phe Thr
385                 390                 395                 400
Gly Gly Glu Asp Gly Ala Ile His Met Phe Glu Val Val Ser Asp Cys
                405                 410                 415
Met Glu Arg Asn Val Leu Glu Val Ser Thr Trp Ile Pro His Ser Gly
            420                 425                 430
Pro Val His Ser Leu Ala Phe Glu Phe Pro Trp Leu Val Ser Ala Ser
        435                 440                 445
Ala Asp Gly Arg Met Ser Leu Ile Asp Val Arg Lys Leu Leu Gln Thr
    450                 455                 460
Cys Lys Pro Ser Leu Gly Lys Asn Val Ser Lys Val Arg His Arg Asp
465                 470                 475                 480
```

```
His Lys Ser Val Glu Pro Pro Gln Arg Met Leu His Gly Phe Gly Cys
                485                 490                 495
Asn Leu Phe Ser Val Asp Ile Gly Ala Asp Arg Ile Val Cys Gly Gly
            500                 505                 510
Glu Glu Gly Val Val Arg Ile Trp Asn Phe Ser Gln Ala Leu Glu Ala
        515                 520                 525
Glu Gln Arg Ala Arg Ala Leu Arg Gly Ile Arg Leu Glu Asn Arg Met
    530                 535                 540
Arg Arg Arg Arg Leu Gln Ile Glu Leu Thr Ser Lys Gly Ser Arg Thr
545                 550                 555                 560
Asp Gln Cys Ser Val Ala Ala Ser Lys Asn Pro Ile Asn Gly Asp Arg
                565                 570                 575
Ser Gly Val Trp His Asn Lys Arg Gly Met Ser Gly Arg Met Lys Ala
            580                 585                 590
<210>  113
<211>  745
<212>  DNA
<213>  Zea mays
<400>  113
atggcggaca aggagcctgt cgtggagcga tccgaggcgt ctgaggagga ggacgcctcc      60
gccgcggccg ccgcggggga ggaagaggac acgggtgccc aggtggcccc catcgtgcgg     120
cttgaggagg tactcgtcac caccggtgag gaggacgagg acgtcctgct cgacatgaag     180
gcgaagctgt accggtttga taaagacggg aatcaatgga aggaacgggg cacgggcacc     240
gtcaagcttc tcaagaacaa ggagaccggc aaggtccgac tggtcatgcg ccaggccaag     300
acgcttaaga tctgcgcgaa ccacctcgtg gcccccacca cgagaatgca ggaacatgcc     360
ggcagcgaca aatcgtgcgt ctggcacgct tctgactttg cggatggcga actcaaggag     420
gagatgtttg ccatcaggtt tggttcggta gaaaactgca agaagttcaa ggagttggtt     480
gaggagattg ctgagtcact tacagagaac gaggacaagg aaggtcaaga tggctcttcc     540
acggccggat tgctggagaa gctcactgtg agcgagggca aatctgagga aagtggcaag     600
tcggagtcca ctgattctgg caaagtgacc gaaaccaaag ccgatgcagc cccagccgag     660
tagttggtgt ggttgcacta cccagctttc ttgtacaaag ttggcattat aagaaagcat     720
tgcttatcaa tttgttgcaa cgaac                                           745
<210>  114
<211>  220
<212>  PRT
<213>  Zea mays
<400>  114
Met Ala Asp Lys Glu Pro Val Val Glu Arg Ser Glu Ala Ser Glu Glu
1               5                   10                  15
Glu Asp Ala Ser Ala Ala Ala Ala Ala Gly Glu Glu Glu Asp Thr Gly
            20                  25                  30
Ala Gln Val Ala Pro Ile Val Arg Leu Glu Glu Val Leu Val Thr Thr
        35                  40                  45
Gly Glu Glu Asp Glu Asp Val Leu Leu Asp Met Lys Ala Lys Leu Tyr
    50                  55                  60
Arg Phe Asp Lys Asp Gly Asn Gln Trp Lys Glu Arg Gly Thr Gly Thr
65                  70                  75                  80
Val Lys Leu Leu Lys Asn Lys Glu Thr Gly Lys Val Arg Leu Val Met
                85                  90                  95
Arg Gln Ala Lys Thr Leu Lys Ile Cys Ala Asn His Leu Val Ala Pro
            100                 105                 110
Thr Thr Arg Met Gln Glu His Ala Gly Ser Asp Lys Ser Cys Val Trp
        115                 120                 125
His Ala Ser Asp Phe Ala Asp Gly Glu Leu Lys Glu Glu Met Phe Ala
    130                 135                 140
Ile Arg Phe Gly Ser Val Glu Asn Cys Lys Lys Phe Lys Glu Leu Val
145                 150                 155                 160
Glu Glu Ile Ala Glu Ser Leu Thr Glu Asn Glu Asp Lys Glu Gly Gln
                165                 170                 175
Asp Gly Ser Ser Thr Ala Gly Leu Leu Glu Lys Leu Thr Val Ser Glu
            180                 185                 190
Gly Lys Ser Glu Glu Ser Gly Lys Ser Glu Ser Thr Asp Ser Gly Lys
        195                 200                 205
Val Thr Glu Thr Lys Ala Asp Ala Ala Pro Ala Glu
    210                 215                 220
<210>  115
<211>  196
<212>  PRT
<213>  Zea mays
<400>  115
Met Ala Asp Lys Glu Pro Val Val Glu Arg Pro Glu Ala Gly Glu Glu
```

```
1               5                   10                  15
Glu Glu Asp Ala Ser Ala Ala Ala Ala Ala Gly Glu Glu Glu Asp Thr
            20                  25                  30
Gly Ala Gln Val Ala Pro Ile Val Arg Leu Glu Glu Val Ala Val Thr
        35                  40                  45
Thr Gly Glu Glu Asp Glu Asp Val Leu Leu Asp Met Lys Ala Lys Leu
    50                  55                  60
Leu Pro Ile Arg Gln Arg Arg Met Arg Gln Ala Lys Thr Leu Lys Ile
65                  70                  75                  80
Cys Ala Asn His Leu Val Ala Ser Thr Thr Lys Met Gln Glu His Ala
                85                  90                  95
Gly Ser Asp Lys Ser Cys Val Trp His Ala Ala Asp Phe Ala Asp Gly
            100                 105                 110
Glu Leu Lys Glu Glu Met Phe Ala Ile Arg Phe Gly Ser Val Glu Asn
        115                 120                 125
Cys Lys Lys Phe Lys Glu Leu Val Glu Glu Ile Ala Glu Ser Leu Thr
    130                 135                 140
Lys Asn Glu Gly Lys Glu Gly Glu Asp Gly Gly Ser Thr Ala Gly Leu
145                 150                 155                 160
Leu Ala Lys Leu Thr Val Ser Glu Gly Lys Ser Glu Gly Ser Gly Lys
                165                 170                 175
Ser Glu Ser Thr Asp Ser Gly Lys Val Thr Glu Thr Lys Ala Asp Thr
            180                 185                 190
Ala Pro Ala Glu
        195
<210>  116
<211>  725
<212>  DNA
<213>  Arabidopsis thaliana
<400>  116
atgccaactt tgtacaaaaa agcaggcttc acaatggcga gcattagcaa cgagcccgag      60
cgtgagaaca gagacgaaga agagactgga gccaacgaag atgaagacac cggtgctcag     120
gttgctccta tcgtcaggct tgaagaagtc gccgtcacca ccggtgaaga agacgaagac     180
accatcctcg atctgaaatc gaagttgtat cgatttgata aagatggaag tcagtggaag     240
gagagaggtg ctggtactgt taagtttttg aaacatagag tttctgggaa gattcgtctc     300
gttatgaggc aatcgaaaac tttgaagatc tgtgctaatc atcttgttgg atcgggtatg     360
agtgttcagg aacacgctgg gaatgataag tcttgtgtat ggcacgctcg tgatttctcc     420
gatggtgaat tgaaggatga gctcttctgt atccggtttg cttcagttga gaattgcaaa     480
gcatttatgc aaaagttcaa ggaagtagct gaatctgaag aagagaaaga agagagcaaa     540
gatgcctctg ataccgctgg tcttcttgag aagttaacag tggaagagaa ggaaagtgag     600
aagaaaccag tggagaaggc agaggaaaac aaaaagagtg aagctgttga agaaaagaaa     660
acagaggagt ctgttccctc agcttaagat gcacccagct ttcttgtaca aagttggcat     720
tataa                                                                 725
<210>  117
<211>  228
<212>  PRT
<213>  Arabidopsis thaliana
<400>  117
Met Pro Thr Leu Tyr Lys Lys Ala Gly Phe Thr Met Ala Ser Ile Ser
1               5                   10                  15
Asn Glu Pro Glu Arg Glu Asn Arg Asp Glu Glu Glu Thr Gly Ala Asn
            20                  25                  30
Glu Asp Glu Asp Thr Gly Ala Gln Val Ala Pro Ile Val Arg Leu Glu
        35                  40                  45
Glu Val Ala Val Thr Thr Gly Glu Glu Asp Glu Asp Thr Ile Leu Asp
    50                  55                  60
Leu Lys Ser Lys Leu Tyr Arg Phe Asp Lys Asp Gly Ser Gln Trp Lys
65                  70                  75                  80
Glu Arg Gly Ala Gly Thr Val Lys Phe Leu Lys His Arg Val Ser Gly
                85                  90                  95
Lys Ile Arg Leu Val Met Arg Gln Ser Lys Thr Leu Lys Ile Cys Ala
            100                 105                 110
Asn His Leu Val Gly Ser Gly Met Ser Val Gln Glu His Ala Gly Asn
        115                 120                 125
Asp Lys Ser Cys Val Trp His Ala Arg Asp Phe Ser Asp Gly Glu Leu
    130                 135                 140
Lys Asp Glu Leu Phe Cys Ile Arg Phe Ala Ser Val Glu Asn Cys Lys
145                 150                 155                 160
Ala Phe Met Gln Lys Phe Lys Glu Val Ala Glu Ser Glu Glu Glu Lys
                165                 170                 175
Glu Glu Ser Lys Asp Ala Ser Asp Thr Ala Gly Leu Leu Glu Lys Leu
```

```
            180                 185                 190
Thr Val Glu Glu Lys Glu Ser Glu Lys Lys Pro Val Glu Lys Ala Glu
        195                 200                 205
Glu Asn Lys Lys Ser Glu Ala Val Glu Glu Lys Lys Thr Glu Glu Ser
    210                 215                 220
Val Pro Ser Ala
225
```

<210> 118
<211> 217
<212> PRT
<213> Arabidopsis thaliana
<400> 118

```
Met Ala Ser Ile Ser Asn Glu Pro Lys Arg Glu Asn Arg Asp Glu Glu
1               5                   10                  15
Glu Thr Gly Ala Asn Glu Asp Glu Asp Thr Gly Ala Gln Val Ala Pro
            20                  25                  30
Ile Val Arg Leu Glu Glu Val Ala Val Thr Thr Gly Glu Glu Asp Glu
        35                  40                  45
Asp Thr Ile Leu Asp Leu Lys Ser Lys Leu Tyr Arg Phe Asp Lys Asp
    50                  55                  60
Gly Ser Gln Trp Lys Glu Arg Gly Ala Gly Thr Val Lys Phe Leu Lys
65                  70                  75                  80
His Arg Val Ser Gly Lys Ile Arg Leu Val Met Arg Gln Ser Lys Thr
                85                  90                  95
Leu Lys Ile Cys Ala Asn His Leu Val Gly Ser Gly Met Ser Val Gln
            100                 105                 110
Glu His Ala Gly Asn Asp Lys Ser Cys Val Trp His Ala Arg Asp Phe
        115                 120                 125
Ser Asp Gly Glu Leu Lys Asp Glu Leu Phe Cys Ile Arg Phe Ala Ser
    130                 135                 140
Val Glu Asn Cys Lys Ala Phe Met Gln Lys Phe Lys Glu Val Ala Glu
145                 150                 155                 160
Ser Glu Glu Glu Lys Glu Glu Ser Lys Asp Ala Ser Asp Thr Ala Gly
                165                 170                 175
Leu Leu Glu Lys Leu Thr Val Glu Glu Lys Glu Ser Glu Lys Lys Pro
            180                 185                 190
Val Glu Lys Ala Glu Glu Asn Lys Lys Ser Glu Ala Val Glu Glu Lys
        195                 200                 205
Lys Thr Glu Glu Ser Val Pro Ser Ala
    210                 215
```

<210> 119
<211> 1416
<212> DNA
<213> Arabidopsis thaliana
<400> 119

```
gcgaaacccc caaattctct tctctctatc tctctcgcgt acgccgcacc gtagcgacca      60
attgaatcca cgaggaaatc tcagtaaata ctatggcgac caacgaaccc gagcacgagc     120
acagagacga ggaagaggcc ggagctaacg aggatgagga caccggagct caggtcgctc     180
cgatcgttag gcttgaggag gttgccgtca ctaccggcga ggaagacgaa gatgccgtcc     240
ttgatctgaa atcgaagctt tatcgattcg ataaggatgc gaatcagtgg aaggagagag     300
gagctggtac tgtgaagttc ttaaagcata agaacactgg gaagattcgt ctcgttatga     360
ggcaatctaa aactttgaag atctgtgcta atcacttcgt taaatcgggc atgagtgttc     420
aggaacacgt tgggaatgaa aagtcatgtg tgtggcacgc tcgtgacttt gctgatggtg     480
aactcaagga tgagcttttc tgtatccgat ttgcttctat tgagaattgc aaaacattta     540
tgcaaaagtt caaggaagtt gctgagtctg aagaagagaa agaagagagc aaagatgccg     600
ctgacactgc tggccttctt gagaaattga ctgtggaaga gacaaaaacg gaggagaaaa     660
ccgaagcgaa agctgtggag acggcaaaga ctgaagtgaa agcagaagaa aagaaagaga     720
gcgaggcaga gaaatctggt gaagcaaaga aaacagaaga aagtggtccc tcaacataag     780
aagcgtcatc atttaagttg ccaaatcctg gcgaggtaat taagcctcga aatgttttga     840
tgcatcatga gtctaccatt gtcttggcac tatctatcta tacatgtttt gtcgagtcat     900
atcaagtggt tgggggatcg cttggggtcg ggttttactg aatgctgagt cgttatgggt     960
ctaatagttt ttgagtctaa agtgtcgggt gatatgagag atttgggtga aatatttttt    1020
catgttggtt atctgaaaga gtacattggt ttcattgttt taagttttct catggatcct    1080
ttttggatgg tcttattttg aggatacaaa tgtgtttgtc catggacaag aatcagaagt    1140
ctccattttt ttttttcaa aatttaatgc atgtgatttt ttaatcttaa ggtaattgcc    1200
aattgtttga caaaaaaaag gtaattgcca atctactttg ctctcttgtg ttagtcgatg    1260
ctagtcttga ccctgataaa gatccaaaat gtatattaac agtattcata aaattcttca    1320
gttataacga actgttcacg gaaaaaaaat gacattgtac tatactgtgc taaaattacc    1380
aaagcatgat ttatataaat catatccagt aagacc                              1416
```

<210> 120
<211> 228

<212> PRT
<213> Arabidopsis thaliana
<400> 120
Met Ala Thr Asn Glu Pro Glu His Glu His Arg Asp Glu Glu Glu Ala
1 5 10 15
Gly Ala Asn Glu Asp Glu Asp Thr Gly Ala Gln Val Ala Pro Ile Val
20 25 30
Arg Leu Glu Glu Val Ala Val Thr Thr Gly Glu Glu Asp Glu Asp Ala
35 40 45
Val Leu Asp Leu Lys Ser Lys Leu Tyr Arg Phe Asp Lys Asp Ala Asn
50 55 60
Gln Trp Lys Glu Arg Gly Ala Gly Thr Val Lys Phe Leu Lys His Lys
65 70 75 80
Asn Thr Gly Lys Ile Arg Leu Val Met Arg Gln Ser Lys Thr Leu Lys
85 90 95
Ile Cys Ala Asn His Phe Val Lys Ser Gly Met Ser Val Gln Glu His
100 105 110
Val Gly Asn Glu Lys Ser Cys Val Trp His Ala Arg Asp Phe Ala Asp
115 120 125
Gly Glu Leu Lys Asp Glu Leu Phe Cys Ile Arg Phe Ala Ser Ile Glu
130 135 140
Asn Cys Lys Thr Phe Met Gln Lys Phe Lys Glu Val Ala Glu Ser Glu
145 150 155 160
Glu Glu Lys Glu Glu Ser Lys Asp Ala Ala Asp Thr Ala Gly Leu Leu
165 170 175
Glu Lys Leu Thr Val Glu Glu Thr Lys Thr Glu Glu Lys Thr Glu Ala
180 185 190
Lys Ala Val Glu Thr Ala Lys Thr Glu Val Lys Ala Glu Glu Lys Lys
195 200 205
Glu Ser Glu Ala Glu Lys Ser Gly Glu Ala Lys Lys Thr Glu Glu Ser
210 215 220
Gly Pro Ser Thr
225
<210> 121
<211> 660
<212> DNA
<213> Arabidopsis thaliana
<400> 121
atggcgagca ctgagccaga gcgtgagaac cgtgaagatg aaaccgaagt caacgaagat 60
gaagacaccg gagctcaggt agctccgatc gttaggcttg aagaggttgc agtcaccacc 120
ggcgaagaag atgaagacgc cgtcctcgat ctgaaatcga agatgtatcg attcgataaa 180
gaagggaacc aatggaagga gagaggagct ggaactgtga agttattgaa gcataaggaa 240
actggaaaag ttcgtcttgt tatgagacaa tctaaaaccc taaagatctg tgccaatcac 300
ctcatttcgt ctgggatgag tgttcaggaa catagtggga atgaaaagtc ttgtttatgg 360
cacgctactg atttctccga cggcgagttg aaagatgagc ttttctgcat tcgatttgct 420
tccattgaga attgcaaaac atttatggag aagttcactg aaatagctga aagccagcaa 480
gtagggaaag agagcaccca gggcgacgag gctgctggtt taatagagaa tctttcggtt 540
gaggaaaata taagtgagga gaaagccaag gaagcagaag agaaagagcc tgcaaaggaa 600
gataaagaaa caaaaaagga gaaggtagaa gaagagaaga aaacagaggc aagcacttaa 660
<210> 122
<211> 260
<212> PRT
<213> Arabidopsis thaliana
<400> 122
Met Ala Ser Thr Glu Pro Glu Arg Glu Asn Arg Glu Asp Glu Thr Glu
1 5 10 15
Val Asn Glu Asp Glu Asp Thr Gly Ala Gln Val Ala Pro Ile Val Arg
20 25 30
Leu Glu Glu Val Ala Val Thr Thr Gly Glu Glu Asp Glu Asp Ala Val
35 40 45
Leu Asp Leu Tyr Val Thr Arg Ser Val Asn Ile Leu Val Ser Leu Pro
50 55 60
Leu Val Lys Met Tyr Arg Phe Asp Lys Glu Gly Asn Gln Trp Lys Glu
65 70 75 80
Arg Gly Ala Gly Thr Val Lys Leu Leu Lys His Lys Glu Thr Gly Lys
85 90 95
Val Arg Leu Val Met Arg Gln Ser Lys Thr Leu Lys Ile Cys Ala Asn
100 105 110
His Leu Ile Ser Ser Gly Met Ser Val Gln Glu His Ser Gly Asn Glu
115 120 125
Lys Ser Cys Leu Trp His Ala Thr Asp Phe Ser Asp Gly Glu Leu Lys

```
    130                 135                 140
Asp Glu Leu Phe Cys Ile Arg Phe Ala Ser Ile Glu Arg Lys Met Val
145                 150                 155                 160
Trp Lys Ile Leu Glu Trp Leu Thr Asp Ser Val Ala Ser Thr Asp Cys
                165                 170                 175
Lys Thr Phe Met Glu Lys Phe Thr Glu Ile Ala Glu Ser Gln Gln Val
            180                 185                 190
Gly Lys Glu Ser Thr Gln Gly Asp Glu Ala Ala Gly Leu Ile Glu Asn
        195                 200                 205
Leu Ser Val Glu Glu Asn Ile Ser Glu Glu Lys Ala Lys Glu Ala Glu
    210                 215                 220
Glu Lys Glu Pro Ala Lys Glu Asp Lys Glu Thr Lys Lys Glu Lys Val
225                 230                 235                 240
Thr Gln Ser Val Ile Asp Met Phe Gly Val Ala Ala Lys Gly Thr Ile
                245                 250                 255
Met Trp Cys Phe
            260
```

<210> 123
<211> 1104
<212> DNA
<213> Lycopersicon esculentum
<400> 123

```
aaaaaaagca aaaagaaaag aggcaaagtt atcaaatcaa aaaccctact tcctcccttg     60
ttgttcttct tcttcttcaa atccggggta aatctttcta aaaccaaaaa tctagagtga    120
tggcaagcac tactgttgaa cccaaattgg agaagaaaga agaggaagta gaagcagaag    180
tagaagaaaa cccaactggc gatgatgaag acactggtgc acaagttgct cccattgtta    240
ggctacaaga agttgctgtc tccactggtg aagaaaatga acatgttctt cttgatctga    300
aatcaaagtt ataccgattt gacaaggaag ggagtcaatg gaaagagaga ggtgttggga    360
ctgtgaagct tctcaagcac aaggaaactg gaaaggttag gcttgtgatg aggcaatcca    420
agacgctgaa aatctgtgcc aaccacttgg tccttcctac tatgtcgatc caagaacatg    480
ctgggaatga gaagtcttgt gtgtggcatg ctgctgactt tgctgatgga gaactgaaag    540
atgagacttt ttgcatccgc tttgcttcag ttgagaattg caaggctttc aaagagaagg    600
ttgaagaaat tgctgaatct caacagacaa agtctggaca aagtgaagaa gctggtgctg    660
ctgctactga acttattgaa aagcttagtg ttgaaagcaa agataaaaat gacaaacctg    720
aagacaaaga ggcccctgca gctacagagg aaaaggaaga taagaaggaa gagaaagctg    780
aagaaaagaa ttaaaatgtt tgtattgtcg gtcagttttt tttttgaaa ggttaatagc    840
attcgttgtt tctgtctgat ccaatagata tgatagatat aagcaatgtg tctcttgtgg    900
tcttatttgt tgttgttggg atggtttgga ttttcatttg ggtcttgagt cgagtgcagc    960
ttcatgtttt attgtggtct ttggtgtttc cttgtactta tttattgtgt gtttgcaaat   1020
tttggttcat ggggtacacc acccagtata agggattacg tttgttaaaa cctttagcac   1080
tgttagtgag atcatttttg agtt                                          1104
```

<210> 124
<211> 224
<212> PRT
<213> Lycopersicon esculentum
<400> 124

```
Met Ala Ser Thr Thr Val Glu Pro Lys Leu Glu Lys Lys Glu Glu Glu
1               5                   10                  15
Val Glu Ala Glu Val Glu Glu Asn Pro Thr Gly Asp Asp Glu Asp Thr
            20                  25                  30
Gly Ala Gln Val Ala Pro Ile Val Arg Leu Gln Glu Val Ala Val Ser
        35                  40                  45
Thr Gly Glu Glu Asn Glu His Val Leu Leu Asp Leu Lys Ser Lys Leu
    50                  55                  60
Tyr Arg Phe Asp Lys Glu Gly Ser Gln Trp Lys Glu Arg Gly Val Gly
65                  70                  75                  80
Thr Val Lys Leu Leu Lys His Lys Glu Thr Gly Lys Val Arg Leu Val
                85                  90                  95
Met Arg Gln Ser Lys Thr Leu Lys Ile Cys Ala Asn His Leu Val Leu
            100                 105                 110
Pro Thr Met Ser Ile Gln Glu His Ala Gly Asn Glu Lys Ser Cys Val
        115                 120                 125
Trp His Ala Ala Asp Phe Ala Asp Gly Glu Leu Lys Asp Glu Thr Phe
    130                 135                 140
Cys Ile Arg Phe Ala Ser Val Glu Asn Cys Lys Ala Phe Lys Glu Lys
145                 150                 155                 160
Val Glu Glu Ile Ala Glu Ser Gln Gln Thr Lys Ser Gly Gln Ser Glu
                165                 170                 175
Glu Ala Gly Ala Ala Ala Thr Glu Leu Ile Glu Lys Leu Ser Val Glu
            180                 185                 190
Ser Lys Asp Lys Asn Asp Lys Pro Glu Asp Lys Glu Ala Pro Ala Ala
```

```
        195                 200                 205
Thr Glu Glu Lys Glu Asp Lys Lys Glu Glu Lys Ala Glu Glu Lys Asn
    210                 215                 220
<210>  125
<211>  442
<212>  DNA
<213>  Oryza sativa
<400>  125
caacctctcc ccccacgatc gccctcctcc ccgaaacttc tggaaccgag agcagcagac      60
gttagctctt ctcctccgat ggcggacaag gagcccgtcg tggaccgccc cgaggacgag     120
gaggaggcct ccgccgccgc cgccgccgcg ggcggcgagg aggaggacac gggcgcccag     180
gtcgccccca tcgtgcggct cgaggaggtc gccgtcacca ccggcgagga ggacgaggac     240
gtgctcctcg acatgaaggc gaagctttac cggtttgaca aggaggggaa ccagtggaag     300
gagcggggga cgggcaccgt caagctcctc aagcacaagg agaacggcaa ggtccgcctc     360
gtcatgcgcc aggccaagac gctcaagatc tgcgcgaacc acctagttgc ttcgaccacg     420
aagatgcagg agcatgcggg ca                                              442
<210>  126
<211>  121
<212>  PRT
<213>  Oryza sativa
<400>  126
Met Ala Asp Lys Glu Pro Val Val Asp Arg Pro Glu Asp Glu Glu Glu
1               5                   10                  15
Ala Ser Ala Ala Ala Ala Ala Ala Gly Gly Glu Glu Glu Asp Thr Gly
            20                  25                  30
Ala Gln Val Ala Pro Ile Val Arg Leu Glu Glu Val Ala Val Thr Thr
        35                  40                  45
Gly Glu Glu Asp Glu Asp Val Leu Leu Asp Met Lys Ala Lys Leu Tyr
    50                  55                  60
Arg Phe Asp Lys Glu Gly Asn Gln Trp Lys Glu Arg Gly Thr Gly Thr
65                  70                  75                  80
Val Lys Leu Leu Lys His Lys Glu Asn Gly Lys Val Arg Leu Val Met
                85                  90                  95
Arg Gln Ala Lys Thr Leu Lys Ile Cys Ala Asn His Leu Val Ala Ser
            100                 105                 110
Thr Thr Lys Met Gln Glu His Ala Gly
        115                 120
<210>  127
<211>  1063
<212>  DNA
<213>  Zea mays
<400>  127
gcacgcggta aacaccccac gtctcaagct gcccctttct caccgccgcg gccgccacgc      60
gcagcagcaa ccgagcagga gcgcagccag caagctcagg cccgagccct actgcaaccg     120
ccgctgctac cgcactgaac accatggccg acccggcgga gcaccgtcca gcggaggagg     180
acgaggaagc cgcggcggcc ggcgaggatg aggacaccgg cgctcaggtc gcgcccatcg     240
tgaagctgga ggaggtcgcc gttaccaccg gagaggagga cgaggacgta cttgtcgaca     300
tgaaggcgaa gctctaccgg ttcgacaagg aggcgaacca gtggaaagaa cgtggcacag     360
gcactgtgaa gctgcttaag cacaaggaga ccgccaaggt ccgcctcgtc atgcgtcagg     420
cgaagacgct taagatctgt gctaaccatc ttgtggtggc gacgactaag atgcaggagc     480
acgcagggag cgacaagtcg tgcgtgtggc acgcgctgga cttcgccgac ggtgagctca     540
aggaggagat gtttgctatc cgttttggat ctgtcgagaa ttgcaagaag ttcaaggaca     600
ctgttgaaga gatagctgaa gagcaaggaa agaccgaggt gaaagaaaac gaggaagtct     660
ccattgccgc cgcagatttg gtacagaagt taacagtgac agaggctagc aacgagggag     720
atgcagagga agaggaggct cctgcatctg accataagaa ggatgctaaa gggtaaagat     780
tgaaggcaaa caaggccaaa tgattatgat tccattcatt tcgttgtcaa ggttcatctt     840
ccccacaaat tttcattaca aagtttcatt gcattttctc ctgagatgat ttgtgtgtgt     900
gtgtgtgtgt gtgtgtgtgt gttgggtgaa atctgagttg tcagtcatct acatgtcttt     960
cttggttgtt agacttattc tcagtcgcct gtttatctgg gatggctagg tacatcatgt    1020
ttgtacaatt atttggggtt gatttaaaaa aaagaaaaaa aaa                      1063
<210>  128
<211>  210
<212>  PRT
<213>  Zea mays
<400>  128
Met Ala Asp Pro Ala Glu His Arg Pro Ala Glu Glu Asp Glu Glu Ala
1               5                   10                  15
Ala Ala Ala Gly Glu Asp Glu Asp Thr Gly Ala Gln Val Ala Pro Ile
            20                  25                  30
Val Lys Leu Glu Glu Val Ala Val Thr Thr Gly Glu Glu Asp Glu Asp
        35                  40                  45
```

```
Val Leu Val Asp Met Lys Ala Lys Leu Tyr Arg Phe Asp Lys Glu Ala
    50                  55                  60
Asn Gln Trp Lys Glu Arg Gly Thr Gly Thr Val Lys Leu Leu Lys His
65                  70                  75                  80
Lys Glu Thr Ala Lys Val Arg Leu Val Met Arg Gln Ala Lys Thr Leu
                85                  90                  95
Lys Ile Cys Ala Asn His Leu Val Val Ala Thr Thr Lys Met Gln Glu
            100                 105                 110
His Ala Gly Ser Asp Lys Ser Cys Val Trp His Ala Leu Asp Phe Ala
        115                 120                 125
Asp Gly Glu Leu Lys Glu Glu Met Phe Ala Ile Arg Phe Gly Ser Val
    130                 135                 140
Glu Asn Cys Lys Lys Phe Lys Asp Thr Val Glu Glu Ile Ala Glu Glu
145                 150                 155                 160
Gln Gly Lys Thr Glu Val Lys Glu Asn Glu Glu Val Ser Ile Ala Ala
                165                 170                 175
Ala Asp Leu Val Gln Lys Leu Thr Val Thr Glu Ala Ser Asn Glu Gly
            180                 185                 190
Asp Ala Glu Glu Glu Glu Ala Pro Ala Ser Asp His Lys Lys Asp Ala
        195                 200                 205
Lys Gly
    210
```

<210> 129
<211> 1111
<212> DNA
<213> Oryza sativa
<400> 129

```
ggacgcgcag caaccggcag cagcagcaga ggaggaggcc agggcaaccc gcaaccccaa     60
accctactcc cgccgcgcca ccgccatggc cgacccagcg gagcaccgtg aggaggagga    120
ggaggccgcg gcggcggcgg cgggcgagga cgaggacacc ggcgcccagg tcgcgcccat    180
cgtcaagctc gaggaggtcg ccgtcaccac cggcgaggag gacgaggagg tcctcctcga    240
catgaagtcg aagctgtacc gcttcgacaa ggaggggaac caatggaagg agagaggcac    300
cggcacggtg aagctgctga agcacaagga gaccggcaag gtccgtctcg tcatgcgcca    360
ggctaagacg ctcaagatct gcgccaatca cctcgtggcg acgaccacga agatgcagga    420
gcacgccggc agcgacaagt cgtgcgtgtg gcacgcgctg gacttcgccg acggcgagct    480
caaggaggaa atgttcgcca tacgctttgg atccgtcgag aactgcaaga agttcaggga    540
gatggtagaa gagattgctg agcagcaagg aaagaacgag gagaaagaaa atgaggaagt    600
ctcctctact gcagggttgg ttgagaagct ttcagtgacc gagacaaaaa aggaggaaaa    660
tgcagagaaa gaggagactc ctgcagaaga ggataagaag gacgctaaag agtgaaagca    720
cccgcaacct ctaggcagct ttgatatgcc tgaagcgagt gaagtgttaa atgagcgtca    780
tttcattcat tttgtggtca aagttcttcc tttcacaaat tttcattgtg ttttcttttg    840
gatgaatgtt tgtgctaggc aaaatttgag ttgtatcagt cgggttcttg gttactacac    900
tgttacttaa accttgagtt gtttatccga gatggggtgg cggagtgggg gcacagcatg    960
tttgtacaat tatttgaagt tgcttttgga atgggcacgg tttgggttgt ccaaaatttg   1020
gacggtgatg ccctgtcact cacaattctt atctctgtct tgcaattata tgcgatgtga   1080
agctcttcgc ctcttcggtg acgagttgtc g                                  1111
```

<210> 130
<211> 209
<212> PRT
<213> Oryza sativa
<400> 130

```
Met Ala Asp Pro Ala Glu His Arg Glu Glu Glu Glu Glu Ala Ala Ala
1               5                   10                  15
Ala Ala Ala Gly Glu Asp Glu Asp Thr Gly Ala Gln Val Ala Pro Ile
            20                  25                  30
Val Lys Leu Glu Glu Val Ala Val Thr Thr Gly Glu Glu Asp Glu Glu
        35                  40                  45
Val Leu Leu Asp Met Lys Ser Lys Leu Tyr Arg Phe Asp Lys Glu Gly
    50                  55                  60
Asn Gln Trp Lys Glu Arg Gly Thr Gly Thr Val Lys Leu Leu Lys His
65                  70                  75                  80
Lys Glu Thr Gly Lys Val Arg Leu Val Met Arg Gln Ala Lys Thr Leu
                85                  90                  95
Lys Ile Cys Ala Asn His Leu Val Ala Thr Thr Thr Lys Met Gln Glu
            100                 105                 110
His Ala Gly Ser Asp Lys Ser Cys Val Trp His Ala Leu Asp Phe Ala
        115                 120                 125
Asp Gly Glu Leu Lys Glu Glu Met Phe Ala Ile Arg Phe Gly Ser Val
    130                 135                 140
Glu Asn Cys Lys Lys Phe Arg Glu Met Val Glu Glu Ile Ala Glu Gln
145                 150                 155                 160
```

Gln Gly Lys Asn Glu Glu Lys Glu Asn Glu Glu Val Ser Ser Thr Ala
165 170 175
Gly Leu Val Glu Lys Leu Ser Val Thr Glu Thr Lys Lys Glu Glu Asn
180 185 190
Ala Glu Lys Glu Glu Thr Pro Ala Glu Glu Asp Lys Lys Asp Ala Lys
195 200 205
Glu

<210> 131
<211> 767
<212> DNA
<213> Populus balsamifera subsp. trichocarpa
<400> 131
tctcctctcc aagcaagaac atcaaatcta atggcaagca cagaacccga acacgagcac 60
agagaagatg aggaagctcc ggctggcgaa gacgaagaca ccggtgctca ggttgctccg 120
atcgtcaagc tcgaagaagt tgctgtctct accggtgaag aagatgaaga tgcgatcctc 180
gatctgaaat cgaagcttta tagatttgat aaggacggga atcagtggaa agagagaggt 240
gctggcactg tcaagttatt gaagcataaa gagtctggaa aagttcgtct tgttatgaga 300
caatctaaga ctctcaagat ctgcgctaat catctcgtgc tgccgactat gtccgtgcag 360
gagcacgcag ggaatgataa gtcgtgtgtg tggcacgcta cagatttcgc tgatggtgaa 420
ttgaaggatg agttgttctg cattcgtttc gcatctgttg aaaattgcaa aacctttatg 480
gaaatgtttc aagaagttgc tgaatcccaa gagagcaaag aggaaaatga ggatgcaact 540
gttgctgctg atgcattgga gaagttgagt gttgaaggga agaaaactga ggagaatgct 600
ggcgaagagg cacctgctgc aaccaagaat gaggaaacta agactgatac agataagaaa 660
ggggagaagc ctgctccctc aacttgaggg ttgatttgtt tgttttgcca ttcccttggc 720
agtatgatga gttcagttgt caaccatatt tcttgtccat tttgtgg 767
<210> 132
<211> 218
<212> PRT
<213> Populus balsamifera subsp. trichocarpa
<400> 132
Met Ala Ser Thr Glu Pro Glu His Glu His Arg Glu Asp Glu Glu Ala
1 5 10 15
Pro Ala Gly Glu Asp Glu Asp Thr Gly Ala Gln Val Ala Pro Ile Val
20 25 30
Lys Leu Glu Glu Val Ala Val Ser Thr Gly Glu Glu Asp Glu Asp Ala
35 40 45
Ile Leu Asp Leu Lys Ser Lys Leu Tyr Arg Phe Asp Lys Asp Gly Asn
50 55 60
Gln Trp Lys Glu Arg Gly Ala Gly Thr Val Lys Leu Leu Lys His Lys
65 70 75 80
Glu Ser Gly Lys Val Arg Leu Val Met Arg Gln Ser Lys Thr Leu Lys
85 90 95
Ile Cys Ala Asn His Leu Val Leu Pro Thr Met Ser Val Gln Glu His
100 105 110
Ala Gly Asn Asp Lys Ser Cys Val Trp His Ala Thr Asp Phe Ala Asp
115 120 125
Gly Glu Leu Lys Asp Glu Leu Phe Cys Ile Arg Phe Ala Ser Val Glu
130 135 140
Asn Cys Lys Thr Phe Met Glu Met Phe Gln Glu Val Ala Glu Ser Gln
145 150 155 160
Glu Ser Lys Glu Glu Asn Glu Asp Ala Thr Val Ala Ala Asp Ala Leu
165 170 175
Glu Lys Leu Ser Val Glu Gly Lys Lys Thr Glu Glu Asn Ala Gly Glu
180 185 190
Glu Ala Pro Ala Ala Thr Lys Asn Glu Glu Thr Lys Thr Asp Thr Asp
195 200 205
Lys Lys Gly Glu Lys Pro Ala Pro Ser Thr
210 215
<210> 133
<211> 1105
<212> DNA
<213> Saccharum officinarum
<400> 133
ggagaacacc tacccgtctc cccaccctag ccccgccgtc gcgtcctctt cgaatcgaat 60
cgcgccgcga tcacctcatc tcatggcgga caaggagcct gtcgtggagc gacccgaggc 120
ggctgaggag gaggacgcct cagccgccgc cgctgccgcg ggggaggagg aggacacggg 180
cgcccaggtg gcccccatcg tgcggcttga ggaggtagac gtcaccaccg gcgaggagga 240
cgaggacgtc ctgctcgaca tgaaggcgaa gttgtaccga tttgacaaag acggaacca 300
atggaaggaa cggggcaccg gcaccgtcaa gcttctcaag cacaaggaga ccggcaaggt 360
ccgactcgtc atgcgccagg ccaagacgct taagatctgc gcgaaccacc tcgtggcctc 420

```
gaccacgaag atgcaggagc atgccggcag cgacaagtca tgcgtctggc acgctgctga    480
ctttgccgat ggcgaactca aggaggagat gtttgccatc aggtttggtt ccgtagaaaa    540
ttgtaagaag ttcaaggagt tggttgagga gatttctgag tcgcttacaa agaacgaggg    600
caaggaaagt gaagatggtt cttccacagc tggattgctg gagaagctta ctgtgagtga    660
gcacaaatct gaggaaagtg acaagtcgga gtccactgat tctggcaaag tgaccgaaac    720
caaagccgac acagccccag ctgagtagtt ggtggtggca gatcctcccg gtgccaaatc    780
agtttgtgtc cttccagtgg aagtttggtg cccatttgcc atgaaatatg actgctaaca    840
tttgggggcg agttggagca gtctcagatg tttggatttg gtctagtctg gtttggtccg    900
ggcttgattt tgttaaaaac ttagtacatt ggggttggaa cgtttggtat gcgagtcgct    960
agtatttcac tgcatggatt gttatggatt gcggtataag gtgcatctta tattttttt    1020
atttcgttca atacacatac atgtgttgta ttaatacttt atgccaatgc ccatggggag    1080
agttgaattt gaatgtcgag agtgg                                        1105
<210>  134
<211>  221
<212>  PRT
<213>  Saccharum officinarum
<400>  134
Met Ala Asp Lys Glu Pro Val Val Glu Arg Pro Glu Ala Ala Glu Glu
1               5                   10                  15
Glu Asp Ala Ser Ala Ala Ala Ala Ala Ala Gly Glu Glu Glu Asp Thr
            20                  25                  30
Gly Ala Gln Val Ala Pro Ile Val Arg Leu Glu Glu Val Asp Val Thr
        35                  40                  45
Thr Gly Glu Glu Asp Glu Asp Val Leu Leu Asp Met Lys Ala Lys Leu
    50                  55                  60
Tyr Arg Phe Asp Lys Asp Gly Asn Gln Trp Lys Glu Arg Gly Thr Gly
65                  70                  75                  80
Thr Val Lys Leu Leu Lys His Lys Glu Thr Gly Lys Val Arg Leu Val
                85                  90                  95
Met Arg Gln Ala Lys Thr Leu Lys Ile Cys Ala Asn His Leu Val Ala
            100                 105                 110
Ser Thr Thr Lys Met Gln Glu His Ala Gly Ser Asp Lys Ser Cys Val
        115                 120                 125
Trp His Ala Ala Asp Phe Ala Asp Gly Glu Leu Lys Glu Glu Met Phe
    130                 135                 140
Ala Ile Arg Phe Gly Ser Val Glu Asn Cys Lys Lys Phe Lys Glu Leu
145                 150                 155                 160
Val Glu Glu Ile Ser Glu Ser Leu Thr Lys Asn Glu Gly Lys Glu Ser
                165                 170                 175
Glu Asp Gly Ser Ser Thr Ala Gly Leu Leu Glu Lys Leu Thr Val Ser
            180                 185                 190
Glu His Lys Ser Glu Glu Ser Asp Lys Ser Glu Ser Thr Asp Ser Gly
        195                 200                 205
Lys Val Thr Glu Thr Lys Ala Asp Thr Ala Pro Ala Glu
    210                 215                 220
<210>  135
<211>  1034
<212>  DNA
<213>  Saccharum officinarum
<400>  135
acagcagcaa ccggtagcag gagcgcagcc agcaagcgca ggccccgagc cgtactgcaa     60
ccgccgctgc caccgcgccg aacgccatgg ccgacccggc ggagcaccgc cctgcggagg    120
aggaggagga ggccgcggcg gccggcgagg atgaggacac cggcgcccag gtcgcgccca    180
tcgtaaagct ggaggaggtc gccgttacca ccggagagga ggacgaggac gtgctcctcg    240
acatgaaggc gaagctttac cggttcgaca aggaggggaa ccagtggaaa gagcgcggca    300
ctggcactgt gaagctgctc aagcacaagg agaccgccaa ggtccgcctc gttatgcgtc    360
aggcgaagac gcttaagatc tgcgctaacc atctcgtggt ggcgacgact aagatgcagg    420
agcacgcggg gagcgacaag tcgtgcgtgt ggcacgcgct ggacttcgcc gacggcgagc    480
tcaaggagga gatgttcgct atccgttttg gatctgtcga gaattgtaag aagtttaagg    540
atattgttga agagatagct gaacagcaag gaaagactga ggagaaagaa aacgaggaag    600
cctccactgc cgctgatttg gtacagaagt taacagtgac ggaggccagc aaggaggaaa    660
ctgcggagaa agaggaggct cctgcatctg gcgataagaa ggatgctaaa gattgaaggc    720
gtttatatac gcaaacaatg gtcaaatgag tgtccttcca ttcattttgt tgtcaaggtt    780
catctacccct ccacaaattt tcatcgtgtt ttctctggga tgaatgcttg tgttaggtga    840
aatcagagtc atcagtcatc tacatggttt tcttggtcat agactgttat ttttaagtcg    900
cctgtttatc tgggatggct ggggtcagca tgtctgtaca attatttgga gttgcttttg    960
gaatggacgc ggtttgatga gtagcctaaa gcttgagatg gtggtgatgt cttttcgctc   1020
cacttttctt attc                                                     1034
<210>  136
<211>  209
<212>  PRT
```

<213> Saccharum officinarum
<400> 136
Met Ala Asp Pro Ala Glu His Arg Pro Ala Glu Glu Glu Glu Glu Ala
1 5 10 15
Ala Ala Ala Gly Glu Asp Glu Asp Thr Gly Ala Gln Val Ala Pro Ile
20 25 30
Val Lys Leu Glu Glu Val Ala Val Thr Thr Gly Glu Glu Asp Glu Asp
35 40 45
Val Leu Leu Asp Met Lys Ala Lys Leu Tyr Arg Phe Asp Lys Glu Gly
50 55 60
Asn Gln Trp Lys Glu Arg Gly Thr Gly Thr Val Lys Leu Leu Lys His
65 70 75 80
Lys Glu Thr Ala Lys Val Arg Leu Val Met Arg Gln Ala Lys Thr Leu
85 90 95
Lys Ile Cys Ala Asn His Leu Val Val Ala Thr Thr Lys Met Gln Glu
100 105 110
His Ala Gly Ser Asp Lys Ser Cys Val Trp His Ala Leu Asp Phe Ala
115 120 125
Asp Gly Glu Leu Lys Glu Glu Met Phe Ala Ile Arg Phe Gly Ser Val
130 135 140
Glu Asn Cys Lys Lys Phe Lys Asp Ile Val Glu Glu Ile Ala Glu Gln
145 150 155 160
Gln Gly Lys Thr Glu Glu Lys Glu Asn Glu Glu Ala Ser Thr Ala Ala
165 170 175
Asp Leu Val Gln Lys Leu Thr Val Thr Glu Ala Ser Lys Glu Glu Thr
180 185 190
Ala Glu Lys Glu Glu Ala Pro Ala Ser Gly Asp Lys Lys Asp Ala Lys
195 200 205
Asp

<210> 137
<211> 1039
<212> DNA
<213> Medicago sativa
<400> 137
atgtctacaa gcaccgatca tcacgaagag gaagatgttc ccgccggcga tgaagaagac 60
accggcgctc aaatcgctcc gatcatccaa cttcatgaag tcgctgtttc tactggtgaa 120
gaagatgaag aagctattct cgacctaaaa gcgaaactgt accgatttga caaggtaggg 180
aatcaatgga aggaacgagg ggcaggaact gttaagtttt tgaagcataa ggttactgga 240
aaagttaggc ttgttatgag acaatcaaag actcttaaga tctgtgctaa tcatctcatt 300
ttgcctaaaa tgactgtgca agagcatgct gggaatgaga aatcttgtgt ttggcacgcg 360
aaggactttg ctgatggtga attgaaagac gagtttttct gcattcgatt tgcgtcaatt 420
gaaaattgca gaaagttcat agagacattt caagaaattg ctgaatccct gaacaaagag 480
gaaagcaagg atgcatccac tgctgctgat ctccttgaga atttgagtgt tgaagggaaa 540
gcagatgcag aaaagaaaga taaagagaaa tctgaggaga aaactaagga gaaagagaca 600
ccagaaaaag aaagcaagga agatacagaa aagaaagttg aagagcctgc ttcatctgct 660
tgattatgat atgttcatat tttcgacaag gcaatatgga aaagtttggt ggttgacctt 720
cgtgccactc ttatcaatac tctctcatag tactagtctt caggttgttt tgttgctacg 780
ttattgagtg gttgatatcc ttcgttgaat tattgtcagc aaggttggtt ttttgagtcg 840
ggtttgaatc attgagattg gcgcttttgg tctatgggtc tatgggagtt gttattttcg 900
ttctattcat ttatttagtc gaaatttgaa tgagtcatgt tggtttggtg tcggggatgg 960
ggagggtgca gtcgggaaaa attagtagta agtacaaaca aaggttttga atgcatatta 1020
ttgagtataa cattttata 1039
<210> 138
<211> 220
<212> PRT
<213> Medicago sativa
<400> 138
Met Ser Thr Ser Thr Asp His His Glu Glu Glu Asp Val Pro Ala Gly
1 5 10 15
Asp Glu Glu Asp Thr Gly Ala Gln Ile Ala Pro Ile Ile Gln Leu His
20 25 30
Glu Val Ala Val Ser Thr Gly Glu Glu Asp Glu Glu Ala Ile Leu Asp
35 40 45
Leu Lys Ala Lys Leu Tyr Arg Phe Asp Lys Val Gly Asn Gln Trp Lys
50 55 60
Glu Arg Gly Ala Gly Thr Val Lys Phe Leu Lys His Lys Val Thr Gly
65 70 75 80
Lys Val Arg Leu Val Met Arg Gln Ser Lys Thr Leu Lys Ile Cys Ala
85 90 95
Asn His Leu Ile Leu Pro Lys Met Thr Val Gln Glu His Ala Gly Asn

```
                100                 105                 110
Glu Lys Ser Cys Val Trp His Ala Lys Asp Phe Ala Asp Gly Glu Leu
            115                 120                 125
Lys Asp Glu Phe Phe Cys Ile Arg Phe Ala Ser Ile Glu Asn Cys Arg
    130                 135                 140
Lys Phe Ile Glu Thr Phe Gln Glu Ile Ala Glu Ser Leu Asn Lys Glu
145                 150                 155                 160
Glu Ser Lys Asp Ala Ser Thr Ala Ala Asp Leu Leu Glu Asn Leu Ser
                165                 170                 175
Val Glu Gly Lys Ala Asp Ala Glu Lys Lys Asp Lys Glu Lys Ser Glu
            180                 185                 190
Glu Lys Thr Lys Glu Lys Glu Thr Pro Glu Lys Glu Ser Lys Glu Asp
        195                 200                 205
Thr Glu Lys Lys Val Glu Glu Pro Ala Ser Ser Ala
    210                 215                 220
<210>  139
<211>  30
<212>  PRT
<213>  Artificial sequence
<220>
<223>  motif 2 of Zea mays
<400>  139
Glu Glu Glu Asp Thr Gly Ala Gln Val Ala Pro Ile Val Arg Leu Glu
1               5                   10                  15
Glu Val Ala Val Thr Thr Gly Glu Glu Asp Glu Asp Val Leu
            20                  25                  30
<210>  140
<211>  10
<212>  PRT
<213>  Artificial sequence
<220>
<223>  motif 3 of Zea mays
<400>  140
Arg Gln Ala Lys Thr Leu Lys Ile Cys Ala
1               5                   10
<210>  141
<211>  5
<212>  PRT
<213>  Artificial sequence
<220>
<223>  motif 4 of Zea mays
<400>  141
Asn His Leu Val Ala
1               5
<210>  142
<211>  17
<212>  PRT
<213>  Artificial sequence
<220>
<223>  motif 5 of Zea mays
<400>  142
Asp Lys Ser Cys Val Trp His Ala Ala Asp Phe Ala Asp Gly Glu Leu
1               5                   10                  15
Lys

<210>  143
<211>  5
<212>  PRT
<213>  Artificial sequence
<220>
<223>  motif 6 of Zea mays
<400>  143
Glu Ile Ala Glu Ser
1               5
<210>  144
<211>  9
<212>  PRT
<213>  Artificial sequence
<220>
<223>  motif 7 of Zea mays
<400>  144
```

Leu Ala Lys Leu Thr Val Ser Glu Gly
1 5
<210> 145
<211> 30
<212> PRT
<213> Artificial sequence
<220>
<223> motif 2 of Arabidopsis thaliana
<400> 145
Glu Asp Glu Asp Thr Gly Ala Gln Val Ala Pro Ile Val Arg Leu Glu
1 5 10 15
Glu Val Ala Val Thr Thr Gly Glu Glu Asp Glu Asp Thr Ile
20 25 30
<210> 146
<211> 10
<212> PRT
<213> Artificial sequence
<220>
<223> motif 3 of Arabidopsis thaliana
<400> 146
Arg Gln Ser Lys Thr Leu Lys Ile Cys Ala
1 5 10
<210> 147
<211> 5
<212> PRT
<213> Artificial sequence
<220>
<223> motif 4 of Arabidopsis thaliana
<400> 147
Asn His Leu Val Gly
1 5
<210> 148
<211> 17
<212> PRT
<213> Artificial sequence
<220>
<223> motif 5 of Arabidopsis thaliana
<400> 148
Asp Lys Ser Cys Val Trp His Ala Arg Asp Phe Ser Asp Gly Glu Leu
1 5 10 15
Lys

<210> 149
<211> 5
<212> PRT
<213> Artificial sequence
<220>
<223> motif 6 of Arabidopsis thaliana
<400> 149
Glu Val Ala Glu Ser
1 5
<210> 150
<211> 9
<212> PRT
<213> Artificial sequence
<220>
<223> motif 7 of Arabidopsis thaliana
<400> 150
Leu Glu Lys Leu Thr Val Glu Glu Lys
1 5
<210> 151
<211> 51
<212> DNA
<213> Artificial sequence
<220>
<223> primer: prm06703
<400> 151
ggggacaagt ttgtacaaaa aagcaggctt aaacaatggc ggacaaggag c 51
<210> 152
<211> 50
<212> DNA

```
<213>  Artificial sequence
<220>
<223>  primer: prm06704
<400>  152
ggggaccact ttgtacaaga aagctgggta gtgcaaccac accaactact                50
<210>  153
<211>  52
<212>  DNA
<213>  Artificial sequence
<220>
<223>  primer: prm06491
<400>  153
ggggacaagt ttgtacaaaa aagcaggctt cacaatggcg agcattagca ac             52
<210>  154
<211>  49
<212>  DNA
<213>  Artificial sequence
<220>
<223>  primer: prm06492
<400>  154
ggggaccact ttgtacaaga aagctgggtg catcttaagc tgagggaac                 49
<210>  155
<211>  654
<212>  DNA
<213>  Oryza sativa
<400>  155
cttctacatc ggcttaggtg tagcaacacg actttattat tattattatt attattatta     60
ttattttaca aaaatataaa atagatcagt ccctcaccac aagtagagca agttggtgag    120
ttattgtaaa gttctacaaa gctaatttaa aagttattgc attaacttat ttcatattac    180
aaacaagagt gtcaatggaa caatgaaaac catatgacat actataattt tgtttttatt    240
attgaaatta tataattcaa agagaataaa tccacatagc cgtaaagttc tacatgtggt    300
gcattaccaa aatatatata gcttacaaaa catgacaagc ttagtttgaa aaattgcaat    360
ccttatcaca ttgacacata aagtgagtga tgagtcataa tattattttc tttgctaccc    420
atcatgtata tatgatagcc acaaagttac tttgatgatg atatcaaaga acatttttag    480
gtgcacctaa cagaatatcc aaataatatg actcacttag atcataatag agcatcaagt    540
aaaactaaca ctctaaagca accgatggga aagcatctat aaatagacaa gcacaatgaa    600
aatcctcatc atccttcacc acaattcaaa tattatagtt gaagcatagt agta          654
<210>  156
<211>  1394
<212>  DNA
<213>  Oryza sativa
<400>  156
atgcttgccg tgtcgccggc gatgtgcccc gacattgagg accgcgccgc ggtggccggc     60
gatgctggca tggaggtcgt cgggatgtcg tcggacgaca tggatcagtt cgacttctcc    120
gtcgatgaca tagacttcgg ggacttcttc ctgaggctgg aggacggtga tgtgctcccg    180
gacctcgagg tcgacccggc cgagatcttc accgacttcg aggcaattgc gacgagtgga    240
ggcgaaggtg tgcaggacca ggaggtgccc accgtcgagc tcttggcgcc tgcggacgac    300
gtcggtgtgc tggatccgtg cggcgatgtc gtcgtcgggg aggagaacgc ggcgtttgcc    360
ggggctggag aggagaaggg agggtgtaac caggacgatg atgcgggggga agcgaatgtc    420
gacgatggag ccgcggcggt tgaggccaag tcttcgtcgc cgtcatcgac gacgtcgtcg    480
tcgcaggagg ctgagagccg gcacaagtca tccagcaaga gctcccatgg gaagaagaaa    540
gcgaaggtgg actggacgcc tgagcttcac cggaggttcg tgcaggcggt ggagcagctc    600
ggcatcgaca aggccgtgcc gtcgaggata cttgagatca tggggatcga ctctctcacc    660
cggcacaaca tagccagcca tcttcagaag taccggtcac acagaaaaca catgattgcg    720
agagaggcgg aggcagcgag ttggacccaa cggcggcaga tttacgccgc cggtggaggt    780
gctgtttgcg aagaggccgg agtccaacgc gtggaccgtg ccaaccattg gcttccctcc    840
tcctccgcca ccaccaccat caccggctcc gattcaacat tttgctcgcc cgttgcatgt    900
tggggccacc cgacgatgga cccgtcccga gttccagtgt ggccaccgcg gcacctcgtt    960
ccccgtggcc cggcgccacc atgggttcca ccgccgccgc cgtcggaccc tgctttctgg   1020
caccaccctt acatgagggg gccagcacat gtgccaactc aagggacacc ttgcatggcg   1080
atgcccatgc cagctgcgag atttcctgct ccaccggtgc caggagttgt cccgtgtcca   1140
atgtataggc cattgactcc accagcactg gcgagcaaga atcagcagga cgcacagctt   1200
caactccagg ttcaaccatc aagcgagagc atcgacgcag ctatcggtga tgttttatcg   1260
aaaccgtggt tgcctttgcc tcttggactg aagccacctt cagtggacag tgtgatgggc   1320
gagctgcaga ggcaaggcgt agcaaacgtt cctccagcgt gtggatgata ttacccagct   1380
ttcttgtaca aagt                                                      1394
<210>  157
<211>  455
<212>  PRT
<213>  Oryza sativa
<400>  157
```

Met Leu Ala Val Ser Pro Ala Met Cys Pro Asp Ile Glu Asp Arg Ala
1               5                   10                  15
Ala Val Ala Gly Asp Ala Gly Met Glu Val Val Gly Met Ser Ser Asp
            20                  25                  30
Asp Met Asp Gln Phe Asp Phe Ser Val Asp Asp Ile Asp Phe Gly Asp
        35                  40                  45
Phe Phe Leu Arg Leu Glu Asp Gly Asp Val Leu Pro Asp Leu Glu Val
    50                  55                  60
Asp Pro Ala Glu Ile Phe Thr Asp Phe Glu Ala Ile Ala Thr Ser Gly
65                  70                  75                  80
Gly Glu Gly Val Gln Asp Gln Glu Val Pro Thr Val Glu Leu Leu Ala
                85                  90                  95
Pro Ala Asp Asp Val Gly Val Leu Asp Pro Cys Gly Asp Val Val Val
            100                 105                 110
Gly Lys Glu Asn Ala Ala Phe Ala Gly Ala Gly Glu Glu Lys Gly Gly
        115                 120                 125
Cys Asn Gln Asp Asp Asp Ala Gly Glu Ala Asn Ala Asp Asp Gly Ala
    130                 135                 140
Ala Ala Val Glu Ala Lys Ser Ser Ser Pro Ser Ser Ser Thr Ser Ser
145                 150                 155                 160
Ser Gln Glu Ala Glu Ser Arg His Lys Ser Ser Ser Lys Ser Ser His
                165                 170                 175
Gly Lys Lys Lys Ala Lys Val Asp Trp Thr Pro Glu Leu His Arg Arg
            180                 185                 190
Phe Val Gln Ala Val Glu Gln Leu Gly Ile Asp Lys Ala Val Pro Ser
        195                 200                 205
Arg Ile Leu Glu Ile Met Gly Ile Asp Ser Leu Thr Arg His Asn Ile
    210                 215                 220
Ala Ser His Leu Gln Lys Tyr Arg Ser His Arg Lys His Met Ile Ala
225                 230                 235                 240
Arg Glu Ala Glu Ala Ala Ser Trp Thr Gln Arg Arg Gln Ile Tyr Ala
                245                 250                 255
Ala Gly Gly Gly Ala Val Ala Lys Arg Pro Glu Ser Asn Ala Trp Thr
            260                 265                 270
Val Pro Thr Ile Gly Phe Pro Pro Pro Pro Pro Pro Pro Pro Ser Pro
        275                 280                 285
Ala Pro Met Gln His Phe Ala Arg Pro Leu His Val Trp Gly His Pro
    290                 295                 300
Thr Met Asp Pro Ser Arg Val Pro Val Trp Pro Pro Arg His Leu Val
305                 310                 315                 320
Pro Arg Gly Pro Ala Pro Pro Trp Val Pro Pro Pro Pro Pro Ser Asp
                325                 330                 335
Pro Ala Phe Trp His His Pro Tyr Met Arg Gly Pro Ala His Val Pro
            340                 345                 350
Thr Gln Gly Thr Pro Cys Met Ala Met Pro Met Pro Ala Ala Arg Phe
        355                 360                 365
Pro Ala Pro Pro Val Pro Gly Val Val Pro Cys Pro Met Tyr Arg Pro
    370                 375                 380
Leu Thr Pro Pro Ala Leu Thr Ser Lys Asn Gln Gln Asp Ala Gln Leu
385                 390                 395                 400
Gln Leu Gln Val Gln Pro Ser Ser Glu Ser Ile Asp Ala Ala Ile Gly
                405                 410                 415
Asp Val Leu Ser Lys Pro Trp Leu Pro Leu Pro Leu Gly Leu Lys Pro
            420                 425                 430
Pro Ser Val Asp Ser Val Met Gly Glu Leu Gln Arg Gln Gly Val Ala
        435                 440                 445
Asn Val Pro Pro Ala Cys Gly
    450                 455

<210> 158
<211> 50
<212> DNA
<213> artificial sequence
<220>
<223> primer: prm02251
<400> 158
ggggacaagt ttgtacaaaa aagcaggctt cacaatgctt gccgtgtcgc 50
<210> 159
<211> 49
<212> DNA
<213> artificial sequence
<220>

<223> primer: prm02252
<400> 159
ggggaccact ttgtacaaga aagctgggta atatcatcca cacgctgga 49
<210> 160
<211> 2193
<212> DNA
<213> Oryza sativa
<400> 160
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtacctttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga atttttttc ccaaatatct taaaaagtca 1740
ctttctggtt cagttcaatg aattgattgc tacaaataat gcttttatag cgttatccta 1800
gctgtagttc agttaatagg taataccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttcttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193
<210> 161
<211> 21
<212> PRT
<213> artificial sequence
<220>
<223> GARP consensus sequence 1
<220>
<221> VARIANT
<222> (1)..(1)
<223> /replace = "Arg"
<220>
<221> VARIANT
<222> (2)..(2)
<223> /replace = "Met" /replace = "Val" /replace = "Ala"
<220>
<221> VARIANT
<222> (3)..(3)
<223> /replace = "Lys" /replace = "Met"
<220>
<221> VARIANT
<222> (4)..(4)
<223> /replace = "Leu"
<220>
<221> VARIANT
<222> (5)..(5)
<223> /replace = "Asp"

<220>
<221> VARIANT
<222> (7)..(7)
<223> /replace = "Thr" /replace = "Ile" /replace = "Asn"
<220>
<221> VARIANT
<222> (8)..(8)
<223> /replace = "Ala" /replace = "Pro" /replace = "Ser" /replace = "Thr" /replace = "Cys" /replace = "His" /replace = "Gln" /replace = "Asp"
<220>
<221> VARIANT
<222> (9)..(9)
<223> /replace = "Gln" /replace = "Thr" /replace = "Asp" /replace = "Ser"
<220>
<221> VARIANT
<222> (11)..(11)
<223> /replace = "Asp"
<220>
<221> VARIANT
<222> (12)..(12)
<223> /replace = "Lys" /replace = "Gln" /replace = "Ala" /replace = "His" /replace = "Asp" /replace = "Glu" /replace = "Leu" /replace = "Ile"
<220>
<221> VARIANT
<222> (13)..(13)
<223> /replace = "Arg" /replace = "Gln" /replace = "Ser" /replace = "Cys" /replace = "Val" /replace = "Ala" /replace = "His"
<220>
<221> VARIANT
<222> (15)..(15)
<223> /replace = "Leu" /replace = "Ile"
<220>
<221> VARIANT
<222> (16)..(16)
<223> /replace = "Lys" /replace = "Gln" /replace = "Asp" /replace = "His" /replace = "Asp" /replace = "Asn" /replace = "Arg" /replace = "Ser"
<220>
<221> VARIANT
<222> (17)..(17)
<223> /replace = "Val" /replace = "Cys"
<220>
<221> VARIANT
<222> (18)..(18)
<223> /replace = "Gly" /replace = "Leu" /replace = "Ile"
<220>
<221> VARIANT
<222> (19)..(19)
<223> /replace = "Glu" /replace = "Ala" /replace = "Gln" /replace = "Asp" /replace = "Gly" /replace = "Thr" /replace = "Ile" /replace = "Lys" /replace = "His"
<220>
<221> VARIANT
<222> (20)..(20)
<223> /replace = "Glu" /replace = "His" /replace = "Leu" /replace = "Ile" /replace = "Met" /replace = "Lys" /replace = "Arg" /replace = "Ser"
<400> 161

```
Lys Pro Arg Val Val Trp Ser Val Glu Leu His Arg Lys Phe Val Ala
1               5                   10                  15
Ala Val Asn Gln Leu
            20
```

<210> 162
<211> 3
<212> PRT
<213> artificial sequence
<220>
<223> GARP consensus sequence 2

<220>
<221> VARIANT
<222> (2)..(2)
<223> /replace = "Val" /replace = "Leu" /replace = "Pro" /replace = "Ser" /replace = "His" /replace = "Gln" /replace = "Ala" /replace = "Gly"
<220>
<221> VARIANT
<222> (3)..(3)
<223> /replace = "Glu" /replace = "Lys" /replace = "His" /replace = "Gln" /replace = "Asn" /replace = "Ala"
<400> 162
Gly Ile Asp
1
<210> 163
<211> 11
<212> PRT
<213> artificial sequence
<220>
<223> GARP consensus sequence 3
<220>
<221> VARIANT
<222> (1)..(1)
<223> /replace = "Thr"
<220>
<221> VARIANT
<222> (2)..(2)
<223> /replace = "Ile" /replace = "Tyr" /replace = "Phe" /replace = "Thr"
<220>
<221> VARIANT
<222> (4)..(4)
<223> /replace = "Ser"
<220>
<221> VARIANT
<222> (5)..(5)
<223> /replace = "Arg" /replace = "Thr" /replace = "Gln" /replace = "Leu" /replace = "Ser" /replace = "Gly" /replace = "Ala"
<220>
<221> VARIANT
<222> (6)..(6)
<223> /replace = "Val" /replace = "Leu"
<220>
<221> VARIANT
<222> (7)..(7)
<223> /replace = "Met" /replace = "Arg" /replace = "Lys"
<220>
<221> VARIANT
<222> (8)..(8)
<223> /replace = "Glu" /replace = " Gln" /replace = "Lys" /replace = "Arg" /replace = "Ser"
<220>
<221> VARIANT
<222> (9)..(9)
<223> /replace = "Ile" /replace = "Phe" /replace = "His" /replace = "Val" /replace = "Met" /replace = "Thr" /replace = "Arg" /replace = "Ala"
<220>
<221> VARIANT
<222> (10)..(10)
<223> /replace = "Ile" /replace = "Leu"
<220>
<221> VARIANT
<222> (11)..(11)
<223> /replace = "Lys" /replace = "Gly" /replace = "Ser" /replace = "Asp" /replace = "Gln" /replace = "Glu"
<400> 163
Ala Val Pro Lys Lys Ile Leu Asp Leu Met Asn
1 5 10
<210> 164
<211> 8

<212> PRT
<213> artificial sequence
<220>
<223> GARP consensus sequence 4
<220>
<221> VARIANT
<222> (1)..(1)
<223> /replace = "Ile" /replace = "Met" /replace = "Thr" /replace = "Glu" /replace = "Leu" /replace = "Ser" /replace = "Asn"
<220>
<221> VARIANT
<222> (2)..(2)
<223> /replace = "Asp" /replace = "Gly" /replace = "Asn" /replace = "Tyr" /replace = "Lys" /replace = "His" /replace = "Gln" /replace = "Pro"
<220>
<221> VARIANT
<222> (3)..(3)
<223> /replace = "Gly" /replace = "Lys" /replace = "Thr" /replace = "Ser" /replace = "Cys" /replace = "Arg" /replace = "Asp"
<220>
<221> VARIANT
<222> (4)..(4)
<223> /replace = "Leu"
<220>
<221> VARIANT
<222> (5)..(5)
<223> /replace = "Asp" /replace = "Ala" /replace = "Ser"
<220>
<221> VARIANT
<222> (6)..(6)
<223> /replace = "Asn" /replace = "Ile" /replace = "Leu" /replace = "Val"
<220>
<221> VARIANT
<222> (7)..(7)
<223> /replace = "His" /replace = "Asp" /replace = "Ser" /replace = "Ala" /replace = "Tyr" /replace = "Phe"
<220>
<221> VARIANT
<222> (8)..(8)
<223> /replace = "Glu" /replace = "His"
<400> 164
Val Glu Asn Ile Thr Arg Glu Asn
1 5
<210> 165
<211> 9
<212> PRT
<213> artificial sequence
<220>
<223> GARP consensus sequence 5
<220>
<221> VARIANT
<222> (1)..(1)
<223> /replace = "Ile" /replace = "Leu"
<220>
<221> VARIANT
<222> (2)..(2)
<223> /replace = "Lys"
<220>
<221> VARIANT
<222> (7)..(7)
<223> /replace = "Met" /replace = "Ile"
<220>
<221> VARIANT
<222> (8)..(8)
<223> /replace = "Phe"
<220>
<221> VARIANT
<222> (9)..(9)
<223> /replace = "Val"

<400> 165
Val Ala Ser His Leu Gln Lys Tyr Arg
1 5
<210> 166
<211> 16
<212> PRT
<213> artificial sequence
<220>
<223> Consensus sequence 6
<220>
<221> VARIANT
<222> (4)..(4)
<223> /replace = "Arg"
<220>
<221> VARIANT
<222> (6)..(6)
<223> /replace = "Met"
<220>
<221> VARIANT
<222> (7)..(7)
<223> /replace = "Ala" /replace = "Met" /replace = "Ile"
<220>
<221> VARIANT
<222> (11)..(11)
<223> /replace = "Gly" /replace = "Val"
<220>
<221> VARIANT
<222> (14)..(14)
<223> /replace = "Gly"
<220>
<221> VARIANT
<222> (15)..(15)
<223> /replace = "Asn" /replace = "Thr"
<400> 166
Ser His Arg Lys His Leu Leu Ala Arg Glu Ala Glu Ala Ala Ser Trp
1 5 10 15
<210> 167
<211> 48
<212> PRT
<213> artificial sequence
<220>
<223> GCT domain consensus sequence
<220>
<221> VARIANT
<222> (1)..(1)
<223> /replace = "Gln"
<220>
<221> VARIANT
<222> (2)..(2)
<223> /replace = "Leu"
<220>
<221> VARIANT
<222> (4)..(4)
<223> /replace = "Lys" /replace = "Ser"
<220>
<221> VARIANT
<222> (6)..(6)
<223> /replace = "Val"
<220>
<221> VARIANT
<222> (7)..(7)
<223> /replace = "Val" /replace = "Leu"
<220>
<221> VARIANT
<222> (13)..(13)
<223> /replace = "Glu"
<220>
<221> VARIANT
<222> (14)..(14)
<223> /replace = "Ala"
<220>

```
<221>  VARIANT
<222>  (15)..(15)
<223>  /replace = "Leu"
<220>
<221>  VARIANT
<222>  (16)..(16)
<223>  /replace = "Thr" /replace = "Ala" /replace = "Val"
<220>
<221>  VARIANT
<222>  (17)..(17)
<223>  /replace = "Lys" /replace = "Arg"
<220>
<221>  VARIANT
<222>  (20)..(20)
<223>  /replace = "Thr"
<220>
<221>  VARIANT
<222>  (22)..(22)
<223>  /replace = "Pro"
<220>
<221>  VARIANT
<222>  (27)..(27)
<223>  /replace = "Asn"
<220>
<221>  VARIANT
<222>  (30)..(30)
<223>  /replace = "Ala"
<220>
<221>  VARIANT
<222>  (31)..(31)
<223>  /replace = "Met" /replace = "Leu"
<220>
<221>  VARIANT
<222>  (32)..(32)
<223>  /replace = "Glu" /replace = "Gly"
<220>
<221>  VARIANT
<222>  (33)..(33)
<223>  /replace = "Ser"
<220>
<221>  VARIANT
<222>  (35)..(35)
<223>  /replace = "Ile"
<220>
<221>  VARIANT
<222>  (36)..(36)
<223>  /replace = "Ala" /replace = "Ser" /replace = "Gly"
<220>
<221>  VARIANT
<222>  (39)..(39)
<223>  /replace = "His" /replace = "Glu"
<220>
<221>  VARIANT
<222>  (40)..(40)
<223>  /replace = "Lys"
<220>
<221>  VARIANT
<222>  (41)..(41)
<223>  /replace = "His"
<220>
<221>  VARIANT
<222>  (43)..(43)
<223>  /replace = "Ile"
<220>
<221>  VARIANT
<222>  (44)..(44)
<223>  /replace = "Asn" /replace = "Pro" /replace = "Ala"
<220>
<221>  VARIANT
<222>  (45)..(45)
<223>  /replace = "Glu" /replace = "Thr" /replace = "Lys"
```

```
<220>
<221>  VARIANT
<222>  (46)..(46)
<223>  /replace = "Ile"
<220>
<221>  VARIANT
<222>  (48)..(48)
<223>  /replace = "Gln"
<400>  167
His Pro Ser Asn Glu Ser Ile Asp Ala Ala Ile Gly Asp Val Ile Ser
1               5                   10                  15
Asn Pro Trp Leu Pro Leu Pro Leu Gly Leu Lys Pro Pro Ser Val Asp
            20                  25                  30
Gly Val Met Thr Glu Leu Gln Arg Gln Gly Val Ser Asn Val Pro Pro
        35                  40                  45
<210>  168
<211>  1542
<212>  DNA
<213>  Oryza sativa
<400>  168
atgcttgagg tgtccacgct gcgaagccct aaggcggatc agcgggcggg cgtcggcggc     60
caccatgtcg tcggcttcgt cccggcgccg ccgtcgccgg ccgacgtcgc cgacgaggtc    120
gacgcgttca tcgtcgacga cagctgcctg ctcgagtaca tcgacttcag ctgctgcgac    180
gtgccgttct tccacgccga cgacggcgac atcctcccgg acctcgaggt cgaccccacg    240
gagctcctcg ccgagttcgc cagctccccg gacgacgagc cgccgccgac gacgtcggct    300
ccgggccccg gcgagccagc tgctgctgca ggagccaagg aagacgtgaa ggaagatgga    360
gccgccgccg ccgccgccgc cgccgccgct gactacgacg ggtcgccgcc gccaccgcgg    420
gggaagaaga agaaggacga cgaggaaagg tcgtcgtcgt tgccggagga gaaagacgcg    480
aagaacggcg gcggcgacga ggtcctgagc gcggtgacga cggaggattc ctcggccggt    540
gccgccaagt cgtgctcgcc gtcggcagag ggccacagca agaggaagcc gtcgtcgtcg    600
tcgtcatcgg cggcggccgg caagaactct cacggcaagc gcaaggtgaa ggtggactgg    660
acgccggagt tgcaccggcg gttcgtgcag gcggtggagc agctcgggat agacaaggcc    720
gtgccgtcca ggatcctgga gctcatgggc atcgagtgcc tcactcgcca caacatcgcc    780
agccatctcc agaaatatcg gtcgcacagg aaacatctga tggcgaggga ggcggaggcg    840
gcgagctgga cgcagaagcg gcagatgtac accgccgccg ccgccgccgc cgcggtggca    900
gccggcggcg ggccaaggaa ggacgccgcc gccgccactg cggcggtggc cccgtgggtc    960
atgccgacca tcggtttccc tccgccgcac gcggcggcga tggtgcctcc cccgccgcac   1020
cctccaccgt tctgccggcc gccgctgcac gtgtggggcc acccgaccgc cggcgtcgag   1080
ccgaccaccg cggcggcgcc accaccaccc tcgccgcacg cgcagccgcc gttgctgccc   1140
gtctggccgc gccacctggc gccgccgccg ccgccgctgc cggcggcgtg ggcgcacggc   1200
caccagccgg cgccggtgga cccggcggcg tactggcagc aacagtataa cttgcagagg   1260
tttcctgtac cgccggtgcc tggaatggtg ccgcacccca tgtacagacc gataccgccg   1320
ccgtcaccgc cgcaggggaa taaactcgct gccttgcagc ttcagcttga tgcccacccg   1380
tctaaggaga gcatagacgc agccatcgga gatgttttag tgaagccatg gctgccgctt   1440
cccctcggcc tcaagccacc gtcgctggac agcgtcatgt ctgagctgca caagcagggc   1500
atccccaagg tgccaccggc ggcgagcggt gccgccggct ga                       1542
<210>  169
<211>  513
<212>  PRT
<213>  Oryza sativa
<400>  169
Met Leu Glu Val Ser Thr Leu Arg Ser Pro Lys Ala Asp Gln Arg Ala
1               5                   10                  15
Gly Val Gly Gly His His Val Val Gly Phe Val Pro Ala Pro Pro Ser
            20                  25                  30
Pro Ala Asp Val Ala Asp Glu Val Asp Ala Phe Ile Val Asp Asp Ser
        35                  40                  45
Cys Leu Leu Glu Tyr Ile Asp Phe Ser Cys Cys Asp Val Pro Phe Phe
    50                  55                  60
His Ala Asp Asp Gly Asp Ile Leu Pro Asp Leu Glu Val Asp Pro Thr
65                  70                  75                  80
Glu Leu Leu Ala Glu Phe Ala Ser Ser Pro Asp Asp Glu Pro Pro Pro
                85                  90                  95
Thr Thr Ser Ala Pro Gly Pro Gly Glu Pro Ala Ala Ala Ala Gly Ala
            100                 105                 110
Lys Glu Asp Val Lys Glu Asp Gly Ala Ala Ala Ala Ala Ala Ala Ala
        115                 120                 125
Ala Ala Asp Tyr Asp Gly Ser Pro Pro Pro Pro Arg Gly Lys Lys Lys
    130                 135                 140
Lys Asp Asp Glu Glu Arg Ser Ser Ser Leu Pro Glu Glu Lys Asp Ala
145                 150                 155                 160
```

```
Lys Asn Gly Gly Gly Asp Glu Val Leu Ser Ala Val Thr Thr Glu Asp
                165                 170                 175
Ser Ser Ala Gly Ala Ala Lys Ser Cys Ser Pro Ser Ala Glu Gly His
            180                 185                 190
Ser Lys Arg Lys Pro Ser Ser Ser Ser Ser Ser Ala Ala Ala Gly Lys
        195                 200                 205
Asn Ser His Gly Lys Arg Lys Val Lys Val Asp Trp Thr Pro Glu Leu
    210                 215                 220
His Arg Arg Phe Val Gln Ala Val Glu Gln Leu Gly Ile Asp Lys Ala
225                 230                 235                 240
Val Pro Ser Arg Ile Leu Glu Leu Met Gly Ile Glu Cys Leu Thr Arg
                245                 250                 255
His Asn Ile Ala Ser His Leu Gln Lys Tyr Arg Ser His Arg Lys His
            260                 265                 270
Leu Met Ala Arg Glu Ala Glu Ala Ala Ser Trp Thr Gln Lys Arg Gln
        275                 280                 285
Met Tyr Thr Ala Ala Ala Ala Ala Ala Ala Val Ala Ala Gly Gly Gly
    290                 295                 300
Pro Arg Lys Asp Ala Ala Ala Ala Thr Ala Ala Val Ala Pro Trp Val
305                 310                 315                 320
Met Pro Thr Ile Gly Phe Pro Pro Pro His Ala Ala Ala Met Val Pro
                325                 330                 335
Pro Pro Pro His Pro Pro Pro Phe Cys Arg Pro Pro Leu His Val Trp
            340                 345                 350
Gly His Pro Thr Ala Gly Val Glu Pro Thr Thr Ala Ala Ala Pro Pro
        355                 360                 365
Pro Pro Ser Pro His Ala Gln Pro Pro Leu Leu Pro Val Trp Pro Arg
    370                 375                 380
His Leu Ala Pro Pro Pro Pro Pro Leu Pro Ala Ala Trp Ala His Gly
385                 390                 395                 400
His Gln Pro Ala Pro Val Asp Pro Ala Ala Tyr Trp Gln Gln Gln Tyr
                405                 410                 415
Asn Leu Gln Arg Phe Pro Val Pro Pro Val Pro Gly Met Val Pro His
            420                 425                 430
Pro Met Tyr Arg Pro Ile Pro Pro Pro Ser Pro Pro Gln Gly Asn Lys
        435                 440                 445
Leu Ala Ala Leu Gln Leu Gln Leu Asp Ala His Pro Ser Lys Glu Ser
    450                 455                 460
Ile Asp Ala Ala Ile Gly Asp Val Leu Val Lys Pro Trp Leu Pro Leu
465                 470                 475                 480
Pro Leu Gly Leu Lys Pro Pro Ser Leu Asp Ser Val Met Ser Glu Leu
                485                 490                 495
His Lys Gln Gly Ile Pro Lys Val Pro Pro Ala Ala Ser Gly Ala Ala
            500                 505                 510
Gly

<210>  170
<211>  1263
<212>  DNA
<213>  Arabidopsis thaliana
<400>  170
atgttagctc tgtctccggc gacaagagat ggttgcgacg gagcgtcaga gtttcttgat      60
acgtcgtgtg gattcacgat tataaacccg gaggaggagg aggagtttcc ggatttcgct     120
gaccacggtg atcttcttga catcattgac ttcgacgata tattcggtgt ggccggagat     180
gtgcttcctg acttggagat cgaccctgag atcttatccg ggatttctc caatcacatg      240
aacgcttctt caacgattac tacgacgtcg gataagactg atagtcaagg ggagactact     300
aagggtagtt cggggaaagg tgaagaagtc gtaagcaaaa gagacgatgt tgcggcggag     360
acggtgactt atgacggtga cagtgaccgg aaaaggaagt attcctcttc agcttcttcc     420
aagaacaatc ggatcagtaa caacgaaggg aagagaaagg tgaaggtgga ttggacacca     480
gagctacaca ggagattcgt ggaggcagtg gaacagttag gagtggacaa agctgttcct     540
tctcgaattc tggagcttat gggagtccat tgtctcactc gtcacaacgt tgctagtcac     600
ctccaaaaat ataggtctca tcggaaacat ttgctagctc gtgaggccga agcggctaat     660
tggacacgca aaggcatat ctatggagta gacaccggtg ctaatcttaa tggtcggact      720
aaaaatggat ggcttgcacc ggcacccact ctcgggtttc caccaccacc acccgtggct     780
gttgcaccgc cacctgtcca ccaccatcat tttaggcccc tgcatgtgtg gggacatccc     840
acggttgatc agtccattat gccgcatgtg tggcccaaac acttacctcc gccttctacc     900
gccatgccta atccgccgtt ttgggtctcc gattctccct attggcatcc aatgcataac     960
gggacgactc cgtatttacc gaccgtagct acgagattta gagcaccgcc agttgccgga    1020
atcccgcatg ctctgccgcc gcatcacacg atgtacaaac caaatcttgg atttggtggt    1080
gctcgtcctc cggtagactt acatccgtca aaagagagcg tggatgcagc cataggagat    1140
gtattgacga ggccatggct gccacttccg ttgggattaa atccgccggc tgttgacggt    1200
```

gttatgacag agcttcaccg tcacggtgtc tctgaggttc ctccgaccgc gtcttgtgcc 1260
tga 1263
<210> 171
<211> 420
<212> PRT
<213> Arabidopsis thaliana
<400> 171
Met Leu Ala Leu Ser Pro Ala Thr Arg Asp Gly Cys Asp Gly Ala Ser
1 5 10 15
Glu Phe Leu Asp Thr Ser Cys Gly Phe Thr Ile Ile Asn Pro Glu Glu
20 25 30
Glu Glu Glu Phe Pro Asp Phe Ala Asp His Gly Asp Leu Leu Asp Ile
35 40 45
Ile Asp Phe Asp Asp Ile Phe Gly Val Ala Gly Asp Val Leu Pro Asp
50 55 60
Leu Glu Ile Asp Pro Glu Ile Leu Ser Gly Asp Phe Ser Asn His Met
65 70 75 80
Asn Ala Ser Ser Thr Ile Thr Thr Thr Ser Asp Lys Thr Asp Ser Gln
85 90 95
Gly Glu Thr Thr Lys Gly Ser Ser Gly Lys Gly Glu Glu Val Val Ser
100 105 110
Lys Arg Asp Asp Val Ala Ala Glu Thr Val Thr Tyr Asp Gly Asp Ser
115 120 125
Asp Arg Lys Arg Lys Tyr Ser Ser Ser Ala Ser Ser Lys Asn Asn Arg
130 135 140
Ile Ser Asn Asn Glu Gly Lys Arg Lys Val Lys Val Asp Trp Thr Pro
145 150 155 160
Glu Leu His Arg Arg Phe Val Glu Ala Val Glu Gln Leu Gly Val Asp
165 170 175
Lys Ala Val Pro Ser Arg Ile Leu Glu Leu Met Gly Val His Cys Leu
180 185 190
Thr Arg His Asn Val Ala Ser His Leu Gln Lys Tyr Arg Ser His Arg
195 200 205
Lys His Leu Leu Ala Arg Glu Ala Glu Ala Ala Asn Trp Thr Arg Lys
210 215 220
Arg His Ile Tyr Gly Val Asp Thr Gly Ala Asn Leu Asn Gly Arg Thr
225 230 235 240
Lys Asn Gly Trp Leu Ala Pro Ala Pro Thr Leu Gly Phe Pro Pro Pro
245 250 255
Pro Pro Val Ala Val Ala Pro Pro Pro Val His His His His Phe Arg
260 265 270
Pro Leu His Val Trp Gly His Pro Thr Val Asp Gln Ser Ile Met Pro
275 280 285
His Val Trp Pro Lys His Leu Pro Pro Pro Ser Thr Ala Met Pro Asn
290 295 300
Pro Pro Phe Trp Val Ser Asp Ser Pro Tyr Trp His Pro Met His Asn
305 310 315 320
Gly Thr Thr Pro Tyr Leu Pro Thr Val Ala Thr Arg Phe Arg Ala Pro
325 330 335
Pro Val Ala Gly Ile Pro His Ala Leu Pro Pro His His Thr Met Tyr
340 345 350
Lys Pro Asn Leu Gly Phe Gly Gly Ala Arg Pro Pro Val Asp Leu His
355 360 365
Pro Ser Lys Glu Ser Val Asp Ala Ala Ile Gly Asp Val Leu Thr Arg
370 375 380
Pro Trp Leu Pro Leu Pro Leu Gly Leu Asn Pro Pro Ala Val Asp Gly
385 390 395 400
Val Met Thr Glu Leu His Arg His Gly Val Ser Glu Val Pro Pro Thr
405 410 415
Ala Ser Cys Ala
420
<210> 172
<211> 1403
<212> DNA
<213> Arabidopsis thaliana
<400> 172
gtatcattct tgttttctct aaatttttca aaaaaccttt agaattttca ttttttttacg 60
attccgatgt taactgtttc tccggctcca gtactcatcg gaaacaactc aaaggatact 120
tacatggcgg cagatttcgc agattttacg acggaagact tgccggactt tacgacggtc 180
ggggattttt ccgatgatct tcttgatgga atcgattact acgacgatct tttcattggt 240
ttcgatggag acgatgtttt gccggatttg gagatagatt cggagattct tggggaatat 300

```
tccggtagcg gaagagatga ggaacaagaa atggagggta acacttcgac ggcatcggag    360
acatcggaga gagacgttgg tgtgtgtaag caagagggtg gtggtggtgg tgacggtggt    420
tttagggaca aaacggtgcg tcgaggcaaa cgtaaaggga agaaaagtaa agattgttta    480
tccgatgaga acgatattaa gaaaaaacct aaggtggatt ggacgccgga gttacaccgg    540
aaatttgtac aagcggtgga gcaattaggg gtagacaagg cggtgccgtc tcgaatcttg    600
gaaattatga acgttaaatc tctcactcgt cacaacgttg ctagccatct tcagaaatat    660
aggtcacatc ggaaacatct actagcgcgt gaagcagaag ctgccagctg gaatctccgg    720
agacatgcca cggtggcagt gcccggagta ggaggaggag ggaagaagcc gtggacagct    780
cctgccttag gctatcctcc acacgtggca ccaatgcatc atggtcactt caggcctttg    840
cacgtatggg gtcatcctac gtggccaaaa cacaagccta atactccggc gtctgctcat    900
cggacgtatc caatgccggc cattgcggcg gctccggcat cttggccagg tcatccaccg    960
tactggcatc agcaaccact ctatccacag ggatatggta tggcatcatc gaatcattca   1020
agcatcggtg ttcccacaag acaattagga cccactaatc ctcccatcga cattcatccc   1080
tcgaatgaga gcatagatgc agctattggg gacgttatat caaagccgtg gctgccgctt   1140
cctttgggac tgaaaccgcc gtcggttgac ggtgttatga cggagttaca acgtcaagga   1200
gtttctaatg ttcctcctct tccttgagaa agatctccaa aatttgtcga aatctcaaac   1260
ttttaacttc attttttttgg tatcttctat gtatttttgc aagggaaaga cgataaatcc  1320
ttgttgcttg atcatatgta tttctctata tgagtgcatg tatcgaagtt aagcaacttt   1380
aatatatcgt tataatcttc gat                                            1403
```

<210> 173
<211> 386
<212> PRT
<213> Arabidopsis thaliana
<400> 173

```
Met Leu Thr Val Ser Pro Ala Pro Val Leu Ile Gly Asn Asn Ser Lys
1               5                   10                  15
Asp Thr Tyr Met Ala Ala Asp Phe Ala Asp Phe Thr Thr Glu Asp Leu
            20                  25                  30
Pro Asp Phe Thr Thr Val Gly Asp Phe Ser Asp Asp Leu Leu Asp Gly
        35                  40                  45
Ile Asp Tyr Tyr Asp Asp Leu Phe Ile Gly Phe Asp Gly Asp Asp Val
    50                  55                  60
Leu Pro Asp Leu Glu Ile Asp Ser Glu Ile Leu Gly Glu Tyr Ser Gly
65                  70                  75                  80
Ser Gly Arg Asp Glu Glu Gln Glu Met Glu Gly Asn Thr Ser Thr Ala
                85                  90                  95
Ser Glu Thr Ser Glu Arg Asp Val Gly Val Cys Lys Gln Glu Gly Gly
            100                 105                 110
Gly Gly Gly Asp Gly Gly Phe Arg Asp Lys Thr Val Arg Arg Gly Lys
        115                 120                 125
Arg Lys Gly Lys Lys Ser Lys Asp Cys Leu Ser Asp Glu Asn Asp Ile
    130                 135                 140
Lys Lys Lys Pro Lys Val Asp Trp Thr Pro Glu Leu His Arg Lys Phe
145                 150                 155                 160
Val Gln Ala Val Glu Gln Leu Gly Val Asp Lys Ala Val Pro Ser Arg
                165                 170                 175
Ile Leu Glu Ile Met Asn Val Lys Ser Leu Thr Arg His Asn Val Ala
            180                 185                 190
Ser His Leu Gln Lys Tyr Arg Ser His Arg Lys His Leu Leu Ala Arg
        195                 200                 205
Glu Ala Glu Ala Ala Ser Trp Asn Leu Arg Arg His Ala Thr Val Ala
    210                 215                 220
Val Pro Gly Val Gly Gly Gly Gly Lys Lys Pro Trp Thr Ala Pro Ala
225                 230                 235                 240
Leu Gly Tyr Pro Pro His Val Ala Pro Met His His Gly His Phe Arg
                245                 250                 255
Pro Leu His Val Trp Gly His Pro Thr Trp Pro Lys His Lys Pro Asn
            260                 265                 270
Thr Pro Ala Ser Ala His Arg Thr Tyr Pro Met Pro Ala Ile Ala Ala
        275                 280                 285
Ala Pro Ala Ser Trp Pro Gly His Pro Pro Tyr Trp His Gln Gln Pro
    290                 295                 300
Leu Tyr Pro Gln Gly Tyr Gly Met Ala Ser Ser Asn His Ser Ser Ile
305                 310                 315                 320
Gly Val Pro Thr Arg Gln Leu Gly Pro Thr Asn Pro Pro Ile Asp Ile
                325                 330                 335
His Pro Ser Asn Glu Ser Ile Asp Ala Ala Ile Gly Asp Val Ile Ser
            340                 345                 350
Lys Pro Trp Leu Pro Leu Pro Leu Gly Leu Lys Pro Pro Ser Val Asp
        355                 360                 365
Gly Val Met Thr Glu Leu Gln Arg Gln Gly Val Ser Asn Val Pro Pro
```

370 375 380
Leu Pro
385
<210> 174
<211> 2833
<212> DNA
<213> Physcomitrella patens
<400> 174
ttctaggagg tcagcttggt ccaagtccga atcgcatcca cgcgatagga tatgtcgatc 60
caaatcacac atttttacac tccccagaag gaggaaaaga agttttcgaa accagtagtg 120
aggaatcata gtttcgtttt ccgaaaccta caaattcatc atactcattt cggtaacaaa 180
tgctacttaa aagacatgtc aggaaaacat ataaagtgac agtgattctg catcaacgtg 240
acagggatcc atcagtggag aatgaataaa aaaaacaatt agagaatggt gagtagaatc 300
ctcagcaaaa ttacgtttta tttattaata ttatacttaa aagtaaaaca ggatgattca 360
tgatgtcatc accgaggttg tggagagatt caaaaagagg atggaggcta cttttatggg 420
gagggtgggt tcactttatg cagtaaagta gtagatctct atttaaaggg gggaaagagg 480
atgtctgctg aaggccagtg cagtggatga acgcaggcgc tcgtctatgt ttcatccatc 540
catccattgt gtcgatttag aggccaccat ggcttctgag ggtgagggga gaggatagat 600
agcatagagg ggggggggtg gagcaggcaa gggcaggagt gagattgtgg tggtggtgtg 660
attaggcgat ggagatatgg cgatggacat ggcgaggata gaagtcgagc ctcttgtcga 720
agtccacaac aacaccaaca acttgcttgt gcattgcgtg ctcgatgctt tgccggattc 780
ttcaccttgc ttgaaatcca gtgagacttc gtttgaagct gtggttgtaa aggaggacga 840
ggagggaggg aggccgctgt ttggcaagcc tgagctccac cctgcctcac cctcgagtga 900
tacagcggct gctgcaaatg gtgaattcgc tagatgcttg aattttgtga cggaggctga 960
ttgtggagat gtaggcgtac aatgttttga ggattttggt acgttgccgg actgtggtga 1020
ggcggggata agcagggaag agggtggagg tagagacggg gagcaggtgg agttgttaga 1080
gtctatgagc ctcgatggta gtaggaattc ggagaattta gagctagggg agctcggcga 1140
attgttgcag gggtcggaga cgctggattc ggttcctggg aacgaggttg gggaggagga 1200
ggcgctgctg ttggcgaaag cggcaaaggc gacgggcgtt gttgtttcgg cctccgatag 1260
tggtgaatgt agcagtgtcg ataggaagga aaatcaacaa agtccgaaat catgtaaaag 1320
tgccgcaccg gggaagaaga aggcgaaggt cgattggacg ccggagcttc atcggcgctt 1380
tgtccacgcg gtggaacagc ttggagtgga gaaagctttt ccctcgcgca tactagaact 1440
gatgggagta caatgtctca cccggcacaa tatcgccagt catttgcaga agtatcgctc 1500
gcatcgtcgc catcttgcag ccagggaagc cgaggcagca tcctggactc atcgtcgagc 1560
gtacacccag atgccctggt ctcgaagttc acggcgcgat ggccttcctt atcttgtacc 1620
cttacacacc cctcacatac aacctcgccc atccatggtc atggcaatgc aaccacagct 1680
tcagacgcag cacaccccgg tgtcgacgcc tcttaaggtg tgggggtatc ctacagtaga 1740
tcattcaagt gtacacatgt ggcagcaacc tgcagtggcg accccatcgt attggcaagc 1800
ccccgatggc tcttactggc agcatcctgc caccaattat gatgcgtatt cagctcgcgc 1860
ttgttatccc catcccatgc gagtttcgtt aggcactacg catgctggct ctccaatgat 1920
ggctccagga tttcctgacg agagctacta cggtgaagat gttcttgcag ctaccatgta 1980
tctttgtaac caatcatatg acagtgaatt aggacgagct gcgggcgtcg ctgcgtgcag 2040
taaaccaccg gagacgcatt tatcgaagga ggttcttgat gcagccatcg gagaagcgct 2100
tgctaaccct tggactcccc cgcccctggg actgaagccg ccgtctatgg agggagtaat 2160
cgcagagctt cagcggcagg gaatcaacac tgtgcctccc tctacctgtt agctttagtt 2220
gtttgctgta gagaatattt cattctacga ttcgcattcc aatgaccgct gaccgctggt 2280
gtcgcttggc tggtgttcat ctcgaggaat caatttggtg aaattttggt tatgtcacgg 2340
taggggggtc tattttctcc agagttcctc cggagaggtg tatgaaagga tcgattttct 2400
ttcttctttt tttttttttc tttttttctt tttttttttct tttttttttc cttcgaataa 2460
ggctgccttg gtggtgtttc ttcttagttt tttaacgagg gataccttat catatctgtc 2520
aagatatgtc actgaacgtt gctgcatgta gacttacgtt tatggtaaca ctttctcaat 2580
gccattaaaa accgaaacca gttcttctga ttgtttcatt ggaagtatcc tgacaacctg 2640
tcagcatctg ttggcatgtg gtttcctcac ccacaccctt ctttcagttg gttttgaatc 2700
tgcatctact gactgttaag gttttacgtg tatcaagtgt acgatttttc cacaagataa 2760
tttctcaaca actctgcttt cgaagcacct ttcttctcca ccaatcaaga gtggtgggaa 2820
gatggaccaa ggt 2833
<210> 175
<211> 511
<212> PRT
<213> Physcomitrella patens
<400> 175
Met Ala Met Asp Met Ala Arg Ile Glu Val Glu Pro Leu Val Glu Val
1 5 10 15
His Asn Asn Thr Asn Asn Leu Leu Val His Cys Val Leu Asp Ala Leu
20 25 30
Pro Asp Ser Ser Pro Cys Leu Lys Ser Ser Glu Thr Ser Phe Glu Ala
35 40 45
Val Val Val Lys Glu Asp Glu Glu Gly Gly Arg Pro Leu Phe Gly Lys
50 55 60
Pro Glu Leu His Pro Ala Ser Pro Ser Ser Asp Thr Ala Ala Ala Ala
65 70 75 80

```
Asn Gly Glu Phe Ala Arg Cys Leu Asn Phe Val Thr Glu Ala Asp Cys
                85                  90                  95
Gly Asp Val Gly Val Gln Cys Phe Glu Asp Phe Gly Thr Leu Pro Asp
            100                 105                 110
Cys Gly Glu Ala Gly Ile Ser Arg Glu Glu Gly Gly Gly Arg Asp Gly
        115                 120                 125
Glu Gln Val Glu Leu Leu Glu Ser Met Ser Leu Asp Gly Ser Arg Asn
    130                 135                 140
Ser Glu Asn Leu Glu Leu Gly Glu Leu Gly Glu Leu Leu Gln Gly Ser
145                 150                 155                 160
Glu Thr Leu Asp Ser Val Pro Gly Asn Glu Val Gly Glu Glu Glu Ala
                165                 170                 175
Leu Leu Leu Ala Lys Ala Ala Lys Ala Thr Gly Val Val Val Ser Ala
            180                 185                 190
Ser Asp Ser Gly Glu Cys Ser Ser Val Asp Arg Lys Glu Asn Gln Gln
        195                 200                 205
Ser Pro Lys Ser Cys Lys Ser Ala Ala Pro Gly Lys Lys Lys Ala Lys
    210                 215                 220
Val Asp Trp Thr Pro Glu Leu His Arg Arg Phe Val His Ala Val Glu
225                 230                 235                 240
Gln Leu Gly Val Glu Lys Ala Phe Pro Ser Arg Ile Leu Glu Leu Met
                245                 250                 255
Gly Val Gln Cys Leu Thr Arg His Asn Ile Ala Ser His Leu Gln Lys
            260                 265                 270
Tyr Arg Ser His Arg Arg His Leu Ala Ala Arg Glu Ala Glu Ala Ala
        275                 280                 285
Ser Trp Thr His Arg Arg Ala Tyr Thr Gln Met Pro Trp Ser Arg Ser
    290                 295                 300
Ser Arg Arg Asp Gly Leu Pro Tyr Leu Val Pro Leu His Thr Pro His
305                 310                 315                 320
Ile Gln Pro Arg Pro Ser Met Val Met Ala Met Gln Pro Gln Leu Gln
                325                 330                 335
Thr Gln His Thr Pro Val Ser Thr Pro Leu Lys Val Trp Gly Tyr Pro
            340                 345                 350
Thr Val Asp His Ser Ser Val His Met Trp Gln Gln Pro Ala Val Ala
        355                 360                 365
Thr Pro Ser Tyr Trp Gln Ala Pro Asp Gly Ser Tyr Trp Gln His Pro
    370                 375                 380
Ala Thr Asn Tyr Asp Ala Tyr Ser Ala Arg Ala Cys Tyr Pro His Pro
385                 390                 395                 400
Met Arg Val Ser Leu Gly Thr Thr His Ala Gly Ser Pro Met Met Ala
                405                 410                 415
Pro Gly Phe Pro Asp Glu Ser Tyr Tyr Gly Glu Asp Val Leu Ala Ala
            420                 425                 430
Thr Met Tyr Leu Cys Asn Gln Ser Tyr Asp Ser Glu Leu Gly Arg Ala
        435                 440                 445
Ala Gly Val Ala Ala Cys Ser Lys Pro Pro Glu Thr His Leu Ser Lys
    450                 455                 460
Glu Val Leu Asp Ala Ala Ile Gly Glu Ala Leu Ala Asn Pro Trp Thr
465                 470                 475                 480
Pro Pro Pro Leu Gly Leu Lys Pro Pro Ser Met Glu Gly Val Ile Ala
                485                 490                 495
Glu Leu Gln Arg Gln Gly Ile Asn Thr Val Pro Pro Ser Thr Cys
                500                 505                 510
```

<210> 176
<211> 2270
<212> DNA
<213> Physcomitrella patens
<400> 176

```
ggtggtggtg gtagaaggcg atggagatat ggcgatggac atggcgagga tagatgaatc      60
aaccgccgtc gaggtcaact cgctttcgct tgtgcattgc gtgttggatg ggttgcccga     120
ttcgccttgc ttgaaatcca gcccgacgtc attcgaggag gctgtggcgg aagggaggtc     180
ggtgttcggg gacgaggagg acatcatcaa caacagcaac gaccaggaca actcgtcctc     240
gtgcggtgca gtggtcacca cccacgaaga tttcgccgag tgcttgaatt ttgtgacgga     300
ggcggagtgt ggagatgtgg gtgtgcggtg tttcgaggat tttgacaagc tgccggactg     360
cggcgacgag ggggagacta gcaaagcgga ggaggagggg tgtgtacgaa taggcggagg     420
aggggagcag ggcgagctgt tagagtctgt gagccttgac tgtagtagga attcggagaa     480
tttagagctt cgggatctcg gggaattgtg ggaagggtcg gaacggcctg actcagtgcc     540
agggaacgag gtgggtgagg aggaggcgtt gctgttggcg gaggcggcca aggcgacggg     600
cgatgtcgtg tcggcctcgg atagtggaga atgtagcagt gtcgatagga aggacaatca     660
agccagtccg aaatccagta aaaatgcagc gccggggaag aagaaggcca aggtggactg     720
```

```
gacgcctgag cttcaccggc gcttcgtcca tgcagtggag cagctgggtg tggagaaagc     780
ctatccatcg cgtatcctag aactgatggg cgtgcaatgc ttgactcggc acaacatcgc     840
cagtcacttg cagaagtacc ggtcccaccg tcgccacctc gcagctcgag aagcagaggc     900
cgcgtcctgg acgcatcgtc gcacatacac tcaggctccc tggcctcgta gctcacggcg     960
cgatggcctc ccttatcttg tacctataca cacccctcac atacaacctc ggccttccat    1020
ggccatggca atgcaaccgc agcttcagac gccgcatcat ccgatatcca ctcctctcaa    1080
ggtctggggc taccccacag tagatcattc aaatgtacat atgtggcagc aacctgctgt    1140
ggcgacccca tcttactggc aagctgccga tggctcatac tggcaacatc ccgcgaccgg    1200
ttacgacgct ttctcagctc gtgcctgcta ctcgcatccc atgcagcgag ttcctgtaac    1260
gaccacgcat gcgggtttac caattgtggc gccaggattt cctgacgaga gctgctacta    1320
cggcgacgac atgcttgcag gctccatgta tctatgtaac caatcatatg atagtgaaat    1380
aggacgagct gcgggtgttg ctgcgtgcag caagccgata gagacgcatt tgtccaaaga    1440
ggtgttggat gcggccattg gcgaagctct cgccaatccc tggactcccc cacctctggg    1500
tctgaagcca ccatccatgg agggcgtcat tgcagagctt cagcggcagg ggatcaacac    1560
tgtgcctcct tcaacttgtt agctctcgac gatgtttttt tcttcctctt cctcttcctc    1620
ttcttctaga gagcaatttt tctttctacg actcatattc caatgaccgc tgaccgctgg    1680
tgtcgctggt gtcgctggtg ttcatcccga ggaactaatt tggtgaaaat tcggttaagt    1740
tgcgatagag gtctgatctc cggaaggttt attttttgtc ttctggagag gttgtataag    1800
aggttcccct gttttgcaat aaggcttcct tgatgagatt ttcctttatt tttcccctga    1860
ttctttctcc ttccatttta gtttcacaag aaggcatctt ctggccatgg cggtcacgat    1920
gtgtcacttt gatgctgcat gtggaccttt tttcttatat ctgtagtagc actcctccat    1980
ttcatgagga ataaagatca aacatcacac atcatcactc gaattcttca tctcctcctc    2040
ctcccttttc cactaggatg ccttctattg cattggttgt catgtgactc agtctttctc    2100
ttacacgcac tttaactcgc tggatgtctc actttctgac tgttcaaggt gaggtctgat    2160
aggttcgaga cgggattgct tgcgatgact caccatctcc taatttggaa ccaacattcc    2220
tcctcaacct atcaactctc actcaaactt gcattgtgtg agcattggtc               2270
<210>  177
<211>  517
<212>  PRT
<213>  Physcomitrella patens
<400>  177
Met Ala Met Asp Met Ala Arg Ile Asp Glu Ser Thr Ala Val Glu Val
1               5                   10                  15
Asn Ser Leu Ser Leu Val His Cys Val Leu Asp Gly Leu Pro Asp Ser
            20                  25                  30
Pro Cys Leu Lys Ser Ser Pro Thr Ser Phe Glu Glu Ala Val Ala Glu
        35                  40                  45
Gly Arg Ser Val Phe Gly Asp Glu Glu Asp Ile Ile Asn Asn Ser Asn
    50                  55                  60
Asp Gln Asp Asn Ser Ser Ser Cys Gly Ala Val Val Thr Thr His Glu
65                  70                  75                  80
Asp Phe Ala Glu Cys Leu Asn Phe Val Thr Glu Ala Glu Cys Gly Asp
                85                  90                  95
Val Gly Val Arg Cys Phe Glu Asp Phe Asp Lys Leu Pro Asp Cys Gly
            100                 105                 110
Asp Glu Gly Glu Thr Ser Lys Ala Glu Glu Glu Gly Cys Val Arg Ile
        115                 120                 125
Gly Gly Gly Gly Glu Gln Gly Glu Leu Leu Glu Ser Val Ser Leu Asp
    130                 135                 140
Cys Ser Arg Asn Ser Glu Asn Leu Glu Leu Arg Asp Leu Gly Glu Leu
145                 150                 155                 160
Trp Glu Gly Ser Glu Arg Pro Asp Ser Val Pro Gly Asn Glu Val Gly
                165                 170                 175
Glu Glu Glu Ala Leu Leu Leu Ala Glu Ala Ala Lys Ala Thr Gly Asp
            180                 185                 190
Val Val Ser Ala Ser Asp Ser Gly Glu Cys Ser Ser Val Asp Arg Lys
        195                 200                 205
Asp Asn Gln Ala Ser Pro Lys Ser Ser Lys Asn Ala Ala Pro Gly Lys
    210                 215                 220
Lys Lys Ala Lys Val Asp Trp Thr Pro Glu Leu His Arg Arg Phe Val
225                 230                 235                 240
His Ala Val Glu Gln Leu Gly Val Glu Lys Ala Tyr Pro Ser Arg Ile
                245                 250                 255
Leu Glu Leu Met Gly Val Gln Cys Leu Thr Arg His Asn Ile Ala Ser
            260                 265                 270
His Leu Gln Lys Tyr Arg Ser His Arg Arg His Leu Ala Ala Arg Glu
        275                 280                 285
Ala Glu Ala Ala Ser Trp Thr His Arg Arg Thr Tyr Thr Gln Ala Pro
    290                 295                 300
Trp Pro Arg Ser Ser Arg Arg Asp Gly Leu Pro Tyr Leu Val Pro Ile
305                 310                 315                 320
```

His Thr Pro His Ile Gln Pro Arg Pro Ser Met Ala Met Ala Met Gln
325 330 335
Pro Gln Leu Gln Thr Pro His His Pro Ile Ser Thr Pro Leu Lys Val
340 345 350
Trp Gly Tyr Pro Thr Val Asp His Ser Asn Val His Met Trp Gln Gln
355 360 365
Pro Ala Val Ala Thr Pro Ser Tyr Trp Gln Ala Ala Asp Gly Ser Tyr
370 375 380
Trp Gln His Pro Ala Thr Gly Tyr Asp Ala Phe Ser Ala Arg Ala Cys
385 390 395 400
Tyr Ser His Pro Met Gln Arg Val Pro Val Thr Thr Thr His Ala Gly
405 410 415
Leu Pro Ile Val Ala Pro Gly Phe Pro Asp Glu Ser Cys Tyr Tyr Gly
420 425 430
Asp Asp Met Leu Ala Gly Ser Met Tyr Leu Cys Asn Gln Ser Tyr Asp
435 440 445
Ser Glu Ile Gly Arg Ala Ala Gly Val Ala Ala Cys Ser Lys Pro Ile
450 455 460
Glu Thr His Leu Ser Lys Glu Val Leu Asp Ala Ala Ile Gly Glu Ala
465 470 475 480
Leu Ala Asn Pro Trp Thr Pro Pro Pro Leu Gly Leu Lys Pro Pro Ser
485 490 495
Met Glu Gly Val Ile Ala Glu Leu Gln Arg Gln Gly Ile Asn Thr Val
500 505 510
Pro Pro Ser Thr Cys
515

<210> 178
<211> 1843
<212> DNA
<213> Zea mays
<400> 178

aaacagcgaa acagtgcaga gacaagctac aacaacctac cctaacaggc cattccttcc 60
actgcacttc attcgatcct gagctatagc tggctgcctg agctcgctcc aagaagtgta 120
tctatagtat agacactagg cttcgtgtgg cgtgctcgag atatgcttgc agtgtcgccg 180
tcgccggtgc ggtgtgccga tgcggaggag tgcggcggag gaggcgccag caaggaaatg 240
gaggagaccg ccgtcgggcc tgtgtccgac tcggacctgg atttcgactt cacggtcgac 300
gacatagact tcggggactt cttcctcagg ctagacgacg ggatgacgc gctgccgggc 360
ctcgaggtcg accctgccga gatcgtcttc gctgacttcg aggcaatcgc caccgccggc 420
ggcgatggcg gcgtcacgga ccaggaggtg cccagtgtcc tgccctttgc ggacgcggcg 480
cacataggcg ccgtggatcc gtgttgtggt gtccttggcg aggacaacga cgcagcgtgc 540
gcagacgtgg aagaagggaa aggggagtgc gaccatgccg acgaggtagc agccgccggt 600
aataataata gcgactccgg tgaggccggc tgtggaggag cctttgccgg cgaaaaatca 660
ccgtcgtcga cggcatcgtc gtcgcaggag gctgagagcc ggcgcaaggt gtccaagaag 720
cactcccaag ggaagaagaa agcaaaggtg gattggacgc cggagcttca ccggagattc 780
gttcaggcgg tggaggagct gggcatcgac aaggcggtgc cgtccaggat cctcgagatc 840
atggggatcg actccctcac gcggcataac atagccagcc atctgcagaa gtaccgttcc 900
cacaggaagc acatgcttgc gagggaggtg gaggcagcga cgtggacgac gcaccggcgg 960
ccgatgtacg ctgcccccag cggcgccgtg aagaggcccg actctaacgc gtggaccgtg 1020
ccgaccatcg gtttccctcc gccggcgggg acccctcctc gtccggtgca gcacttcggg 1080
aggccactgc acgtctgggg ccatccgagt ccgacgccag cggtggagtc accccgggtg 1140
ccaatgtggc ctcggcatct cgcccccgc gccccgccgc cgccgccgtg ggctccgcca 1200
ccgccagctg acccggcgtc gttctggcac catgcttaca tgaggggggcc tgctgcccat 1260
atgccagacc aggtggcggt gactccatgc gtggcagtgc caatggcagc agcgcgtttc 1320
cctgctccac acgtgagggg ttctttgcca tggccacctc cgatgtacag acctctcgtt 1380
cctccagcac tcgcaggcaa gagccagcaa gacgcgctgt ttcagctaca gatacagcca 1440
tcaagcgaga gcatagatgc agcaataggt gatgtcttaa cgaagccgtg gctgccgctg 1500
cccctcggac tgaagccccc ttcggtagac agtgtcatgg gcgagctgca gaggcaaggc 1560
gtagcgaatg tgccgcaagc ttgtggatga tccccggggc tgcatagctg ctagctcgtc 1620
cctgcacgca gtgcaacaaa aggaatttgt cgacatgcct tttatgtttt gaattcccgt 1680
gaaccgttta tggagcactc tcaactcgtt cagggtcaga tgaacagaac attataggag 1740
tacattcttt gaggtttcag gtttgaggga aatcaacctg agtgcatcca agtacagtgt 1800
actgccacag tgccacgtcg gaaaatgaag tcttgacccg aat 1843

<210> 179
<211> 475
<212> PRT
<213> Zea mays
<400> 179

Met Leu Ala Val Ser Pro Ser Pro Val Arg Cys Ala Asp Ala Glu Glu
1 5 10 15
Cys Gly Gly Gly Gly Ala Ser Lys Glu Met Glu Glu Thr Ala Val Gly
20 25 30

```
Pro Val Ser Asp Ser Asp Leu Asp Phe Asp Phe Thr Val Asp Asp Ile
        35                  40                  45
Asp Phe Gly Asp Phe Phe Leu Arg Leu Asp Asp Gly Asp Asp Ala Leu
    50                  55                  60
Pro Gly Leu Glu Val Asp Pro Ala Glu Ile Val Phe Ala Asp Phe Glu
65                  70                  75                  80
Ala Ile Ala Thr Ala Gly Gly Asp Gly Gly Val Thr Asp Gln Glu Val
                85                  90                  95
Pro Ser Val Leu Pro Phe Ala Asp Ala Ala His Ile Gly Ala Val Asp
            100                 105                 110
Pro Cys Cys Gly Val Leu Gly Glu Asp Asn Asp Ala Ala Cys Ala Asp
        115                 120                 125
Val Glu Glu Gly Lys Gly Glu Cys Asp His Ala Asp Glu Val Ala Ala
    130                 135                 140
Ala Gly Asn Asn Asn Ser Asp Ser Gly Glu Ala Gly Cys Gly Gly Ala
145                 150                 155                 160
Phe Ala Gly Glu Lys Ser Pro Ser Ser Thr Ala Ser Ser Ser Gln Glu
                165                 170                 175
Ala Glu Ser Arg Arg Lys Val Ser Lys Lys His Ser Gln Gly Lys Lys
            180                 185                 190
Lys Ala Lys Val Asp Trp Thr Pro Glu Leu His Arg Arg Phe Val Gln
        195                 200                 205
Ala Val Glu Glu Leu Gly Ile Asp Lys Ala Val Pro Ser Arg Ile Leu
    210                 215                 220
Glu Ile Met Gly Ile Asp Ser Leu Thr Arg His Asn Ile Ala Ser His
225                 230                 235                 240
Leu Gln Lys Tyr Arg Ser His Arg Lys His Met Leu Ala Arg Glu Val
                245                 250                 255
Glu Ala Ala Thr Trp Thr Thr His Arg Arg Pro Met Tyr Ala Ala Pro
            260                 265                 270
Ser Gly Ala Val Lys Arg Pro Asp Ser Asn Ala Trp Thr Val Pro Thr
        275                 280                 285
Ile Gly Phe Pro Pro Pro Ala Gly Thr Pro Pro Arg Pro Val Gln His
    290                 295                 300
Phe Gly Arg Pro Leu His Val Trp Gly His Pro Ser Pro Thr Pro Ala
305                 310                 315                 320
Val Glu Ser Pro Arg Val Pro Met Trp Pro Arg His Leu Ala Pro Arg
                325                 330                 335
Ala Pro Pro Pro Pro Pro Trp Ala Pro Pro Pro Pro Ala Asp Pro Ala
            340                 345                 350
Ser Phe Trp His His Ala Tyr Met Arg Gly Pro Ala Ala His Met Pro
        355                 360                 365
Asp Gln Val Ala Val Thr Pro Cys Val Ala Val Pro Met Ala Ala Ala
    370                 375                 380
Arg Phe Pro Ala Pro His Val Arg Gly Ser Leu Pro Trp Pro Pro Pro
385                 390                 395                 400
Met Tyr Arg Pro Leu Val Pro Pro Ala Leu Ala Gly Lys Ser Gln Gln
                405                 410                 415
Asp Ala Leu Phe Gln Leu Gln Ile Gln Pro Ser Ser Glu Ser Ile Asp
            420                 425                 430
Ala Ala Ile Gly Asp Val Leu Thr Lys Pro Trp Leu Pro Leu Pro Leu
        435                 440                 445
Gly Leu Lys Pro Pro Ser Val Asp Ser Val Met Gly Glu Leu Gln Arg
    450                 455                 460
Gln Gly Val Ala Asn Val Pro Gln Ala Cys Gly
465                 470                 475
<210>  180
<211>  2192
<212>  DNA
<213>  Zea mays
<400>  180
ctcatcttct cttcttcttc ccccagtcct ccccccccttt cccctcttcg gcctctctcg    60
ctcagctcac tcttcattaa gcgagctcac gtcgttcctc ccttcttctt cttcttatcc   120
gttgttcaat tcgttcagct agccggccag agatcgagca tcatctccat catcgattca   180
tccatctcat ctttctcttc ttctattcta tgtcgtcgtc gtcccagatt agatcgaagc   240
tgctagcagt ctatccagtc tctagctagc tagctagatc aagcccgcag actatacaat   300
ataatacaag ctagctacgt gcttattatt gctttattag tctagaataa tcttgatata   360
tacgatcgaa catatatctc gatctccacc gatacacacc cggccctatc catgcttgag   420
gtgtcgacgc tgcgcggccc tactagcagc ggcagcaagg cggagcagca ctgcggcggc   480
ggcggcggct tcgtcggcga ccaccatgtg gtgttcccga cgtccggcga ctgcttcgcc   540
atggtggacg acaacctcct ggactacatc gacttcagct gcgacgtgcc cttcttcgac   600
```

```
gctgacgggg acatcctccc cgacctggag gtagacacca cggagctcct cgccgagttc     660
tcgtccaccc ctcctgcgga cgacctgctg gcagtggcag tattcggcgc cgacgaccag     720
ccggcggcgg cagtagcaca agagaagccg tcgtcgtcgt tggagcaaac atgtggtgac     780
gacaaaggtg tagcagtagc cgccgccaga agaaagctgc agacgacgac gacgacgacg     840
acgacggagg aggaggattc ttctcctgcc gggtccgggg ccaacaagtc gtcggcgtcg     900
gcagagggcc acagcagcaa gaagaagtcg gcgggcaaga actccaacgg cggcaagcgc     960
aaggtgaagg tggactggac gccggagctg caccggcggt tcgtgcaggc ggtggagcag    1020
ctgggcatcg acaaggccgt gccgtccagg atcctggaga tcatgggcac ggactgcctc    1080
acaaggcaca acattgccag ccacctccag aagtaccggt cgcacagaaa gcacctgatg    1140
gcgcgggagg cggaggccgc cacctgggcg cagaagcgcc acatgtacgc gccgccagct    1200
ccaaggacga cgacgacgac ggacgccgcc aggccgccgt gggtggtgcc gacgaccatc    1260
gggttcccgc cgccgcgctt ctgccgcccg ctgcacgtgt ggggccaccc gccgccgcac    1320
gccgccgcgg ctgaagcagc agcggcgact cccatgctgc ccgtgtggcc gcgtcacctg    1380
gcgccgcccc ggcacctggc gccgtgggcg cacccgacgc cggtggaccc ggcgttctgg    1440
caccagcagt acagcgctgc caggaaatgg ggcccacagg cagccgccgt gacgcaaggg    1500
acgccatgcg tgccgctgcc gaggtttccg gtgcctcacc ccatctacag cagaccggcg    1560
atggtacctc cgccgccaag caccaccaag ctagctcaac tgcatctgga gctccaagcg    1620
cacccgtcca aggagagcat cgacgcagcc atcggagatg ttttagtgaa gccatggctg    1680
ccgcttccac tggggctcaa gccgccgtcg ctcgacagcg tcatgtcgga gctgcacaag    1740
caaggcgtac caaaaatccc accggcggct gccaccacca ccggcgccac cggatgacat    1800
actatctcgt cgacaataca tgcatgtaca atagacggat gtctagtagt atagtagatt    1860
gctgctagct agctagccgc tagagtgtag tagcatatgc atgccttttt tttcttcttc    1920
tttttttgcc cttattatta agctggctaa tagcgattga gatggagctt gacacagatc    1980
tgctagctag ctatttgagg gtttctcttg tatgctacct attgctgctg cttgctgctg    2040
agacaagtaa tgtacgtacg cctgtgcaaa cgacacatcg gattgtattg tattactagt    2100
tatatgaaca acaacaataa taataggcac atgcatgcct gcctagtatg tgagctgcta    2160
gctagctaca tgcatgttgc gcattccttt cc                                  2192
<210>  181
<211>  461
<212>  PRT
<213>  Zea mays
<400>  181
Met Leu Glu Val Ser Thr Leu Arg Gly Pro Thr Ser Ser Gly Ser Lys
1               5                   10                  15
Ala Glu Gln His Cys Gly Gly Gly Gly Gly Phe Val Gly Asp His His
            20                  25                  30
Val Val Phe Pro Thr Ser Gly Asp Cys Phe Ala Met Val Asp Asp Asn
        35                  40                  45
Leu Leu Asp Tyr Ile Asp Phe Ser Cys Asp Val Pro Phe Phe Asp Ala
    50                  55                  60
Asp Gly Asp Ile Leu Pro Asp Leu Glu Val Asp Thr Thr Glu Leu Leu
65                  70                  75                  80
Ala Glu Phe Ser Ser Thr Pro Pro Ala Asp Asp Leu Leu Ala Val Ala
                85                  90                  95
Val Phe Gly Ala Asp Asp Gln Pro Ala Ala Ala Val Ala Gln Glu Lys
            100                 105                 110
Pro Ser Ser Ser Leu Glu Gln Thr Cys Gly Asp Asp Lys Gly Val Ala
        115                 120                 125
Val Ala Ala Ala Arg Arg Lys Leu Gln Thr Thr Thr Thr Thr Thr Thr
    130                 135                 140
Thr Glu Glu Glu Asp Ser Ser Pro Ala Gly Ser Gly Ala Asn Lys Ser
145                 150                 155                 160
Ser Ala Ser Ala Glu Gly His Ser Ser Lys Lys Lys Ser Ala Gly Lys
                165                 170                 175
Asn Ser Asn Gly Gly Lys Arg Lys Val Lys Val Asp Trp Thr Pro Glu
            180                 185                 190
Leu His Arg Arg Phe Val Gln Ala Val Glu Gln Leu Gly Ile Asp Lys
        195                 200                 205
Ala Val Pro Ser Arg Ile Leu Glu Ile Met Gly Thr Asp Cys Leu Thr
    210                 215                 220
Arg His Asn Ile Ala Ser His Leu Gln Lys Tyr Arg Ser His Arg Lys
225                 230                 235                 240
His Leu Met Ala Arg Glu Ala Glu Ala Ala Thr Trp Ala Gln Lys Arg
                245                 250                 255
His Met Tyr Ala Pro Pro Ala Pro Arg Thr Thr Thr Thr Thr Asp Ala
            260                 265                 270
Ala Arg Pro Pro Trp Val Val Pro Thr Thr Ile Gly Phe Pro Pro Pro
        275                 280                 285
Arg Phe Cys Arg Pro Leu His Val Trp Gly His Pro Pro Pro His Ala
    290                 295                 300
Ala Ala Ala Glu Ala Ala Ala Ala Thr Pro Met Leu Pro Val Trp Pro
```

305 310 315 320
Arg His Leu Ala Pro Pro Arg His Leu Ala Pro Trp Ala His Pro Thr
325 330 335
Pro Val Asp Pro Ala Phe Trp His Gln Gln Tyr Ser Ala Ala Arg Lys
340 345 350
Trp Gly Pro Gln Ala Ala Ala Val Thr Gln Gly Thr Pro Cys Val Pro
355 360 365
Leu Pro Arg Phe Pro Val Pro His Pro Ile Tyr Ser Arg Pro Ala Met
370 375 380
Val Pro Pro Pro Pro Ser Thr Thr Lys Leu Ala Gln Leu His Leu Glu
385 390 395 400
Leu Gln Ala His Pro Ser Lys Glu Ser Ile Asp Ala Ala Ile Gly Asp
405 410 415
Val Leu Val Lys Pro Trp Leu Pro Leu Pro Leu Gly Leu Lys Pro Pro
420 425 430
Ser Leu Asp Ser Val Met Ser Glu Leu His Lys Gln Gly Val Pro Lys
435 440 445
Ile Pro Pro Ala Ala Ala Thr Thr Thr Gly Ala Thr Gly
450 455 460

<210> 182
<211> 1221
<212> DNA
<213> Triticum aestivum
<400> 182

```
gaattgggca cgaggaccgc cccattgtgc cggacgcata atcgccgtcg tcgacaacgt      60
cgtcgtccac ggaggccgag agccgccaca agtcatcaag caagaactcc cacggaaaga     120
agaaagccaa ggtggactgg acgccggagc tgcaccggag gttcgtgcag gccgtagagc     180
agctcggcat cgacaaggca gtgccgtcta ggattttgga gatcatgggg atcaactcac     240
tcactcggca caacatagca agccatttgc agaagtaccg gtctcaccgg aagcacatga     300
ttgcgcggga ggcggaggcg gcgagctgga cccaacgacg acagatgtac gccgccggcg     360
ggccggccgc ggccgtgaag aggcaggact cgaacatgtg gaccgtgcca accatcggct     420
tcgcgccggc gcaccctcct cctcctcctc cccctccttc accggcagcc atgcagcact     480
acgctcggcc gttgcacgtc tggggtcacc ctacgatgga ctcgccccgg atgccgatgt     540
ggccgaggca cacaatatcc cgcgccccga tgccggcgtg ggctcccccg ccgccgccgc     600
cgtctgaccc ggctttctgg caccaccctt acatgagggg gcccgcagcg tatatgccga     660
cccatgggac tccttgcatg gcaatgccga tgacaccgaa atttcctgct gcacccgtgc     720
ccgtggccat gccgtgccca gtctatgcgt cccccctcccc ggcaccagcg ctggcgagca     780
agagccaaca agattcgcag ctccagctcc aagcacaacc atcaaatgag agcatagacg     840
cggccattgg tgacgtttta tccaaaccgt ggctgccgct gccgctgggg ctgaagcccc     900
cttcgttggg cagcgtcatg ggcgagcttg aaaggcaagg cgtggccaat gtgccccaag     960
cctgcgggtg agcgttggag ggtgcatccg cgtgcactcc atgcatgact gctgctccgt    1020
tcgatggagc agctagcagc aacaacaaca tcgtcgacat gtctttgttc ctttgaacgt    1080
tttgtggatc tctctctctc tctcttggtc aacttgtgca taatttcgat caagagaaac    1140
atcgttattt tgagttcact cccgagacac atttttgtta cacctaaact ttaagtttgc    1200
ggtaacagca gcatcaacaa c                                               1221
```

<210> 183
<211> 248
<212> PRT
<213> Triticum aestivum
<400> 183

Met Gly Ile Asn Ser Leu Thr Arg His Asn Ile Ala Ser His Leu Gln
1 5 10 15
Lys Tyr Arg Ser His Arg Lys His Met Ile Ala Arg Glu Ala Glu Ala
20 25 30
Ala Ser Trp Thr Gln Arg Arg Gln Met Tyr Ala Ala Gly Gly Pro Ala
35 40 45
Ala Ala Val Lys Arg Gln Asp Ser Asn Met Trp Thr Val Pro Thr Ile
50 55 60
Gly Phe Ala Pro Ala His Pro Pro Pro Pro Pro Pro Pro Pro Ser Pro
65 70 75 80
Ala Ala Met Gln His Tyr Ala Arg Pro Leu His Val Trp Gly His Pro
85 90 95
Thr Met Asp Ser Pro Arg Met Pro Met Trp Pro Arg His Thr Ile Ser
100 105 110
Arg Ala Pro Met Pro Ala Trp Ala Pro Pro Pro Pro Pro Pro Ser Asp
115 120 125
Pro Ala Phe Trp His His Pro Tyr Met Arg Gly Pro Ala Ala Tyr Met
130 135 140
Pro Thr His Gly Thr Pro Cys Met Ala Met Pro Met Thr Pro Lys Phe
145 150 155 160
Pro Ala Ala Pro Val Pro Val Ala Met Pro Cys Pro Val Tyr Ala Ser

```
                    165                 170                 175
Pro Ser Pro Ala Pro Ala Leu Ala Ser Lys Ser Gln Gln Asp Ser Gln
            180                 185                 190
Leu Gln Leu Gln Ala Gln Pro Ser Asn Glu Ser Ile Asp Ala Ala Ile
        195                 200                 205
Gly Asp Val Leu Ser Lys Pro Trp Leu Pro Leu Pro Leu Gly Leu Lys
    210                 215                 220
Pro Pro Ser Leu Gly Ser Val Met Gly Glu Leu Glu Arg Gln Gly Val
225                 230                 235                 240
Ala Asn Val Pro Gln Ala Cys Gly
                245
```

<210> 184
<211> 839
<212> DNA
<213> Allium cepa
<400> 184

```
tgcaaatatt gttcaacgta tataaagaca atttagtatg ctaacaatct ctccccttga    60
aagttctaat ccagatgcaa aagacgaggg agattttgta ctagaagaca tcagctttga   120
tgacttgttc gtaggattcg atgaacttcc cgaccttgaa attgaccctt gtgatatatt   180
tgctgatttt tctttcaata gtagcgagga gggagatgga aataacaagg gttcaacatc   240
ggaagaaaat gatgtacagc ataaaagaga cggatactat aaggagcact caggtaaaga   300
agagacggag gttgtgagtg caagaacaag ggaggatgaa aagaagccgc cttcatctaa   360
atcagagaat gttaaaagcc taaagtcatc aggcaaaaag tcatctcagt ctaaaaagaa   420
agctaaggtg gactggactc cggagcttca tcggagattt gttcaggcag tggagcaact   480
tggggtagac aaagcagtac catccaggat tttagagctc atgggaattg attgtctcac   540
aagacataat attgcaagcc atttacagaa atatcggtcg cacagaaagc atttgctagc   600
aagagaagca gaagcagcaa gctggagtca cagacgtcag ttgtacgtga gcagcactaa   660
caatggaaag aggaggagga aaaacaatga acatgaaacc tagttgggcc ccacagtcac   720
agcccattgg tggatttcct cccaacaatg catcatccct caacatcaga acttcagaac   780
ctttgcacgt ctggggccca tccacaggca gtggagcatc ctcagctagt tccagtctg    839
```

<210> 185
<211> 221
<212> PRT
<213> Allium cepa
<400> 185

```
Met Leu Thr Ile Ser Pro Leu Glu Ser Ser Asn Pro Asp Ala Lys Asp
1               5                   10                  15
Glu Gly Asp Phe Val Leu Glu Asp Ile Ser Phe Asp Asp Leu Phe Val
            20                  25                  30
Gly Phe Asp Glu Leu Pro Asp Leu Glu Ile Asp Pro Cys Asp Ile Phe
        35                  40                  45
Ala Asp Phe Ser Phe Asn Ser Ser Glu Glu Gly Asp Gly Asn Asn Lys
    50                  55                  60
Gly Ser Thr Ser Glu Glu Asn Asp Val Gln His Lys Arg Asp Gly Tyr
65                  70                  75                  80
Tyr Lys Glu His Ser Gly Lys Glu Glu Thr Glu Val Val Ser Ala Arg
                85                  90                  95
Thr Arg Glu Asp Glu Lys Lys Pro Pro Ser Ser Lys Ser Glu Asn Val
            100                 105                 110
Lys Ser Leu Lys Ser Ser Gly Lys Lys Ser Ser Gln Ser Lys Lys Lys
        115                 120                 125
Ala Lys Val Asp Trp Thr Pro Glu Leu His Arg Arg Phe Val Gln Ala
    130                 135                 140
Val Glu Gln Leu Gly Val Asp Lys Ala Val Pro Ser Arg Ile Leu Glu
145                 150                 155                 160
Leu Met Gly Ile Asp Cys Leu Thr Arg His Asn Ile Ala Ser His Leu
                165                 170                 175
Gln Lys Tyr Arg Ser His Arg Lys His Leu Leu Ala Arg Glu Ala Glu
            180                 185                 190
Ala Ala Ser Trp Ser His Arg Arg Gln Leu Tyr Val Ser Ser Thr Asn
        195                 200                 205
Asn Gly Lys Arg Arg Arg Lys Asn Asn Glu His Glu Thr
    210                 215                 220
```

<210> 186
<211> 454
<212> DNA
<213> Hordeum vulgare
<400> 186

```
catcgtgggc gatgaggttt gcagcgcggt gacgacggac gattcttccg ctgcggtcgg    60
gtccgagaac agcaagtcgt cggcgtcggc agagggccac agcaagagga cgtcggcagc   120
cgcagcgacc aagagctccc acggcaggcg gaaggtgaag gtggactgga cgccggagtt   180
```

```
gcaccggcgg ttcgtgcagg cgggggagca gctggggctg gacaaggcgg tgccgtcgcg     240
gatcctggag ctcatgggca acgagtaccg tctcacgcgc cacaacatcg ccagccatct     300
ccagaagtac cggtcacaca ggaagcacct gatggcgagg gagggcgagg cgggtagctg     360
gacgccgcag ccgcaaatgt gctggggtgc gaaggtggtg agggtggggc gggctctgac     420
gttgtagggg ttatatagcg gggtggcggc gggg                                  454
<210>  187
<211>  144
<212>  PRT
<213>  Hordeum vulgare
<400>  187
Ser Leu Ser Ile Val Gly Asp Glu Val Cys Ser Ala Val Thr Thr Asp
1               5                   10                  15
Asp Ser Ser Ala Ala Val Gly Ser Glu Asn Ser Lys Ser Ser Ala Ser
            20                  25                  30
Ala Glu Gly His Ser Lys Arg Thr Ser Ala Ala Ala Ala Thr Lys Ser
        35                  40                  45
Ser His Gly Arg Arg Lys Val Lys Val Asp Trp Thr Pro Glu Leu His
    50                  55                  60
Arg Arg Phe Val Gln Ala Gly Glu Gln Leu Gly Leu Asp Lys Ala Val
65                  70                  75                  80
Pro Ser Arg Ile Leu Glu Leu Met Gly Asn Glu Tyr Arg Leu Thr Arg
                85                  90                  95
His Asn Ile Ala Ser His Leu Gln Lys Tyr Arg Ser His Arg Lys His
            100                 105                 110
Leu Met Ala Arg Glu Gly Glu Ala Gly Ser Trp Thr Pro Gln Pro Gln
        115                 120                 125
Met Cys Trp Gly Ala Lys Val Val Arg Val Gly Arg Ala Leu Thr Leu
    130                 135                 140
<210>  188
<211>  811
<212>  DNA
<213>  Sorghum bicolor
<400>  188
gcacgaggct tgaaattccc tccgccgccg ccgccgccgc cgcctcctcc tcctccttct      60
cctcatccga tgcagcactt cggaaggccg ctgcatgcct ggggccatcc gacgccaacg     120
gtggagtcac cccgggtgcc aatgtggcct cggcatctcg tcccccgcac cccgccgccg     180
ccgtgggctc ctccgccgcc atctgacccg gcgttctggc accatgctta catgaggggg     240
cctgcacaca tgccgggcca ggtgactccc tgcgtggcag tgccaatgcc agctgcgcgt     300
ttccctgctc cacccgtgag ggtgctttg ccatgtccac ctccaatgta cagacctctc      360
gttcctccaa cactcgatac gcagtttcag ctacagacac agccatcaag tgagagcata     420
gatgcagcaa taggtgatgt tttaacgaaa ccgtggctgc cactgcccct gggactgaag     480
ccccccttcag tagacagtgt catgggcgag ctgcagaggc aaggtgtggc aaatgtgcca    540
ccagcttgtg gatgatcccc ggggctccat agctagctgc ttgtccctgc agtccaacaa     600
aaagaatttg tcgacatgcc ttttctgttt caaattttca tgaaccattt atggaccact     660
ctctcttagt taacttgttc agggtcagag agcaaaacag tacatccttt caggtttgag     720
ggaaatcaac ctgagtacat ccaagtacaa tgtgccatga cggaataatg aagtcttggc     780
cttggcccga ataatcagta gctgccatct c                                    811
<210>  189
<211>  184
<212>  PRT
<213>  Sorghum bicolor
<400>  189
Ala Arg Gly Leu Lys Phe Pro Pro Pro Pro Pro Pro Pro Pro Pro Pro
1               5                   10                  15
Pro Pro Pro Ser Pro His Pro Met Gln His Phe Gly Arg Pro Leu His
            20                  25                  30
Ala Trp Gly His Pro Thr Pro Thr Val Glu Ser Pro Arg Val Pro Met
        35                  40                  45
Trp Pro Arg His Leu Val Pro Arg Thr Pro Pro Pro Pro Trp Ala Pro
    50                  55                  60
Pro Pro Pro Ser Asp Pro Ala Phe Trp His His Ala Tyr Met Arg Gly
65                  70                  75                  80
Pro Ala His Met Pro Gly Gln Val Thr Pro Cys Val Ala Val Pro Met
                85                  90                  95
Pro Ala Ala Arg Phe Pro Ala Pro Pro Val Arg Gly Ala Leu Pro Cys
            100                 105                 110
Pro Pro Pro Met Tyr Arg Pro Leu Val Pro Pro Thr Leu Asp Thr Gln
        115                 120                 125
Phe Gln Leu Gln Thr Gln Pro Ser Ser Glu Ser Ile Asp Ala Ala Ile
    130                 135                 140
Gly Asp Val Leu Thr Lys Pro Trp Leu Pro Leu Pro Leu Gly Leu Lys
```

145 150 155 160
Pro Pro Ser Val Asp Ser Val Met Gly Glu Leu Gln Arg Gln Gly Val
165 170 175
Ala Asn Val Pro Pro Ala Cys Gly
180

<210> 190
<211> 593
<212> DNA
<213> Saccharum officinarum
<220>
<221> misc_feature
<222> (521)..(521)
<223> n is a, c, g, or t
<220>
<221> misc_feature
<222> (524)..(524)
<223> n is a, c, g, or t
<220>
<221> misc_feature
<222> (534)..(534)
<223> n is a, c, g, or t
<400> 190

```
catcgacaag gccgtgccgt cccggatcct cgagataatg ggcatggatt gcctcacaag     60
acacaacatt gccagccacc tccagaagta ccggtcgcac agaaagcacc tgatggcgcg    120
ggaggcggag gccgccacct gggcgcagaa gcggcacatg tacgcggcgg ccggcggagt    180
agccccgagg acggacgcac cacacggcag ccggccgtgg gtggtgccga ccatcgggtt    240
cccgccgccg gcgcccccgc ccttctgccg gccgctgcac gtgtggggcc acccgcccca    300
cgccgccgcc gctgaagccg ctgctgcggt ggcggcgact ccgactccga tgctgcccgt    360
gtggccgcgg cacctggcgc cgccccggcc gctgccgccg tgggcgcacc cgcacccgcc    420
ggccgtggac ccggcgttct ggcaccagca gtacaacgcg gccaggaaat ggggtccaca    480
ggcaaccgca gtgacgcaag ggacgccggc gtgcgtgccg ncgnccgccg ccgncatgct    540
gccgaggttt tcccgggttg gccccggggg ggggggggga aaaaaaattt ttt           593
```

<210> 191
<211> 197
<212> PRT
<213> Saccharum officinarum
<220>
<221> UNSURE
<222> (174)..(175)
<223> Xaa can be any naturally occurring amino acid
<220>
<221> UNSURE
<222> (178)..(178)
<223> Xaa can be any naturally occurring amino acid
<400> 191

Ile Asp Lys Ala Val Pro Ser Arg Ile Leu Glu Ile Met Gly Met Asp
1 5 10 15
Cys Leu Thr Arg His Asn Ile Ala Ser His Leu Gln Lys Tyr Arg Ser
20 25 30
His Arg Lys His Leu Met Ala Arg Glu Ala Glu Ala Ala Thr Trp Ala
35 40 45
Gln Lys Arg His Met Tyr Ala Ala Ala Gly Gly Val Ala Pro Arg Thr
50 55 60
Asp Ala Pro His Gly Ser Arg Pro Trp Val Val Pro Thr Ile Gly Phe
65 70 75 80
Pro Pro Pro Ala Pro Pro Pro Phe Cys Arg Pro Leu His Val Trp Gly
85 90 95
His Pro Pro His Ala Ala Ala Ala Glu Ala Ala Ala Ala Val Ala Ala
100 105 110
Thr Pro Thr Pro Met Leu Pro Val Trp Pro Arg His Leu Ala Pro Pro
115 120 125
Arg Pro Leu Pro Pro Trp Ala His Pro His Pro Pro Ala Val Asp Pro
130 135 140
Ala Phe Trp His Gln Gln Tyr Asn Ala Ala Arg Lys Trp Gly Pro Gln
145 150 155 160
Ala Thr Ala Val Thr Gln Gly Thr Pro Ala Cys Val Pro Xaa Xaa Ala
165 170 175
Ala Xaa Met Leu Pro Arg Phe Ser Arg Val Gly Pro Gly Gly Gly Gly
180 185 190
Gly Lys Lys Ile Phe
195

<210> 192
<211> 1816
<212> DNA
<213> Oryza sativa
<400> 192
ggcaactaac aatacctcgc acctagtcac caccgcacca accgcgcgcg accggccccc 60
cgtcagtcga aagctgagcc tgagcgagca actgatgccg ctagctatag ctgctgcctg 120
cagcacgcag cgcccgtaac ctaacgtatc atttacttcc attgcactgc acatcctgtg 180
agttcgttgt ctgagtgaga gctggacgtg ctgcaccgag ctcctggccg agatgcttgc 240
cgtgtcgccg gcgatgtgcc ccgacattga ggaccgcgcc gcggtggccg gcgatgctgg 300
catggaggtc gtcgggatgt cgtcggacga catggatcag ttcgacttct ccgtcgatga 360
catagacttc ggggacttct tcctgaggct ggaggacggt gatgtgctcc cggacctcga 420
ggtcgacccg gccgagatct tcaccgactt cgaggcaatc gcgacgagtg gaggcgaagg 480
tgtgcaggac caggaggtgc ccaccgtcga gctcttggcg cctgcggacg acgtcggtgt 540
gctggatccg tgcggcgatg tcgtcgtcgg ggaggagaac gcggcgtttg ccggggctgg 600
agaggagaag gtagggtgta accaggacga tgatgcgggg gaagcgaatg tcgacgatgg 660
agccgcggcg gttgaggcca agtcttcgtc gccgtcatcg acgacgtcgt cgtcgcagga 720
ggctgagagc cggcacaagt catccagcaa gagctcccat gggaagaaga aagcgaaggt 780
ggactggacg cctgagcttc accggaggtt cgtgcaggcg gtggagcagc tcggcatcga 840
caaggccgtg ccgtcgagga tacttgagat catggggatc gactctctca cccggcacaa 900
catagccagc catcttcaga agtaccggtc acacagaaaa cacatgattg cgagagaggc 960
ggaggcagcg agttggaccc aacggcggca gatttacgcc gccggtggag gtgctgttgc 1020
gaagaggccg gagtccaacg cgtggaccgt gccaaccatt ggcttccctc ctcctccgcc 1080
accaccacca tcaccggctc cgattcaaca ttttgctcgc ccgttgcatg tttggggcca 1140
cccgacgatg gacccgtccc gagttccagt gtggccaccg cggcacctcg ttccccgtgg 1200
cccggcgcca ccatgggttc caccgccgcc gccgtcggac cctgctttct ggcaccaccc 1260
ttacatgagg gggccagcac atgtgccaac tcaagggaca ccttgcatgg cgatgcccat 1320
gccagctgcg agatttcctg ctccaccggt gccaggagtt gtcccgtgtc caatgtatag 1380
gccattgact ccaccagcac tggcgagcaa gaatcagcag gacgcacagc ttcaactcca 1440
ggttcaacca tcaagcgaga gcatcgacgc agctatcggt gatgttttat cgaaaccgtg 1500
gttgcctttg cctcttggac tgaagccacc ttcagtggac agtgtgatgg gcgagctgca 1560
gaggcaaggc gtagcaaacg ttcctccagc gtgtggatga tatttgcatg atagattgat 1620
ggttccgtta ctgactgatt gcttatctgg tcagttcacc aaaaaatggg aaaattcgcc 1680
gacatgtcct tttatttttc ttttggccta gcgtttcttg gacatctcgt ttaattttta 1740
acttgtgcag agttcgaaag acatctttgt ttcgactccg ggagaaatta acctgcgtat 1800
tgtaaatgta aaatgt 1816

<210> 193
<211> 455
<212> PRT
<213> Oryza sativa
<400> 193

Met Leu Ala Val Ser Pro Ala Met Cys Pro Asp Ile Glu Asp Arg Ala
1               5                   10                  15
Ala Val Ala Gly Asp Ala Gly Met Glu Val Val Gly Met Ser Ser Asp
            20                  25                  30
Asp Met Asp Gln Phe Asp Phe Ser Val Asp Asp Ile Asp Phe Gly Asp
        35                  40                  45
Phe Phe Leu Arg Leu Glu Asp Gly Asp Val Leu Pro Asp Leu Glu Val
    50                  55                  60
Asp Pro Ala Glu Ile Phe Thr Asp Phe Glu Ala Ile Ala Thr Ser Gly
65                  70                  75                  80
Gly Glu Gly Val Gln Asp Gln Glu Val Pro Thr Val Glu Leu Leu Ala
                85                  90                  95
Pro Ala Asp Asp Val Gly Val Leu Asp Pro Cys Gly Asp Val Val Val
            100                 105                 110
Gly Glu Glu Asn Ala Ala Phe Ala Gly Ala Gly Glu Glu Lys Val Gly
        115                 120                 125
Cys Asn Gln Asp Asp Asp Ala Gly Glu Ala Asn Val Asp Asp Gly Ala
    130                 135                 140
Ala Ala Val Glu Ala Lys Ser Ser Ser Pro Ser Ser Thr Thr Ser Ser
145                 150                 155                 160
Ser Gln Glu Ala Glu Ser Arg His Lys Ser Ser Ser Lys Ser Ser His
                165                 170                 175
Gly Lys Lys Lys Ala Lys Val Asp Trp Thr Pro Glu Leu His Arg Arg
            180                 185                 190
Phe Val Gln Ala Val Glu Gln Leu Gly Ile Asp Lys Ala Val Pro Ser
        195                 200                 205
Arg Ile Leu Glu Ile Met Gly Ile Asp Ser Leu Thr Arg His Asn Ile
    210                 215                 220
Ala Ser His Leu Gln Lys Tyr Arg Ser His Arg Lys His Met Ile Ala
225                 230                 235                 240

```
Arg Glu Ala Glu Ala Ala Ser Trp Thr Gln Arg Arg Gln Ile Tyr Ala
                245                 250                 255
Ala Gly Gly Gly Ala Val Ala Lys Arg Pro Glu Ser Asn Ala Trp Thr
            260                 265                 270
Val Pro Thr Ile Gly Phe Pro Pro Pro Pro Pro Pro Pro Pro Ser Pro
        275                 280                 285
Ala Pro Ile Gln His Phe Ala Arg Pro Leu His Val Trp Gly His Pro
    290                 295                 300
Thr Met Asp Pro Ser Arg Val Pro Val Trp Pro Pro Arg His Leu Val
305                 310                 315                 320
Pro Arg Gly Pro Ala Pro Pro Trp Val Pro Pro Pro Pro Pro Ser Asp
                325                 330                 335
Pro Ala Phe Trp His His Pro Tyr Met Arg Gly Pro Ala His Val Pro
            340                 345                 350
Thr Gln Gly Thr Pro Cys Met Ala Met Pro Met Pro Ala Ala Arg Phe
        355                 360                 365
Pro Ala Pro Pro Val Pro Gly Val Val Pro Cys Pro Met Tyr Arg Pro
    370                 375                 380
Leu Thr Pro Pro Ala Leu Ala Ser Lys Asn Gln Gln Asp Ala Gln Leu
385                 390                 395                 400
Gln Leu Gln Val Gln Pro Ser Ser Glu Ser Ile Asp Ala Ala Ile Gly
                405                 410                 415
Asp Val Leu Ser Lys Pro Trp Leu Pro Leu Pro Leu Gly Leu Lys Pro
            420                 425                 430
Pro Ser Val Asp Ser Val Met Gly Glu Leu Gln Arg Gln Gly Val Ala
        435                 440                 445
Asn Val Pro Pro Ala Cys Gly
    450                 455
```

<210> 194
<211> 1096
<212> DNA
<213> Oryza sativa
<400> 194

```
tgtgctaagg catccatgct actgcagagt gtcccacctg cagttttggt tcgatttttg     60
agggaacatc gttctgaatg ggcggattat aacttcgatg catattcagc ttcatctctg    120
aagacaagct catgttcact tcctgggttg cggcctatga gattttctgg gagccagatc    180
attatgccac ttgctcacac ggtggagaat gaagagattt tagaagttgt ccgtcttgaa    240
ggacaagcac ttacacatga tgatggtctt atgtctagag atattcacct gcttcagctt    300
tgcactggaa tagatgagaa atcaatggga tcctgcttcc agcttgtctt tgcaccaatc    360
gatgagcttt tccctgatga tgctccgtta atatcttcag gctttcgtgt tataccgctg    420
gacatgaaaa cagatggtac acctgctggt agaacattag atttggcatc tagccttgag    480
gttggttcaa ctgcacagcc cacaggggat gcatctatgg atgactgtaa tctacgatca    540
gtgctgacaa ttgcctttca gttcccttat gaaatgcatc tccaagacag cgttgcaact    600
atggcccggc aatatgtccg cagtattgtt ttctctgttc agagagtatc aatggctgtt    660
tctccttctc ggtctggctt gaatgctggg cagaagataa tttcaggctt ccctgaagcc    720
ccaacgctag ctcgttggat ttgccaaagc taccagttcc atttgggggt ggagttactt    780
aggcaggcag atgatgctgg ggaagcacta ttgaaaatgc tatgggatta cgaagacgct    840
attttgtgct gttctttcaa ggaaaagcct gtatttactt ttgccaacga gatgggacta    900
aacatgctag aaacatctct cgtcgctctc caagatctct cactggacaa gatatttgat    960
gaagccggta ggaaggccct atacaacgag atcccgaaat tgatggaaca gggttacgtg   1020
tacctgcctg gtggagtgtg cttgtccggg atggggcgcc atgtttcttt cgagcaagct   1080
gtagcatgga aggtgc                                                   1096
```

<210> 195
<211> 365
<212> PRT
<213> Oryza sativa
<400> 195

```
Cys Ala Lys Ala Ser Met Leu Leu Gln Ser Val Pro Pro Ala Val Leu
1               5                   10                  15
Val Arg Phe Leu Arg Glu His Arg Ser Glu Trp Ala Asp Tyr Asn Phe
            20                  25                  30
Asp Ala Tyr Ser Ala Ser Ser Leu Lys Thr Ser Ser Cys Ser Leu Pro
        35                  40                  45
Gly Leu Arg Pro Met Arg Phe Ser Gly Ser Gln Ile Ile Met Pro Leu
    50                  55                  60
Ala His Thr Val Glu Asn Glu Glu Ile Leu Glu Val Val Arg Leu Glu
65                  70                  75                  80
Gly Gln Ala Leu Thr His Asp Asp Gly Leu Met Ser Arg Asp Ile His
                85                  90                  95
Leu Leu Gln Leu Cys Thr Gly Ile Asp Glu Lys Ser Met Gly Ser Cys
            100                 105                 110
```

Phe Gln Leu Val Ser Ala Pro Ile Asp Glu Leu Phe Pro Asp Asp Ala
115 120 125
Pro Leu Ile Ser Ser Gly Phe Arg Val Ile Pro Leu Asp Met Lys Thr
130 135 140
Asp Gly Thr Pro Ala Gly Arg Thr Leu Asp Leu Ala Ser Ser Leu Glu
145 150 155 160
Val Gly Ser Thr Ala Gln Pro Thr Gly Asp Ala Ser Met Asp Asp Cys
165 170 175
Asn Leu Arg Ser Val Leu Thr Ile Ala Phe Gln Phe Pro Tyr Glu Met
180 185 190
His Leu Gln Asp Ser Val Ala Thr Met Ala Arg Gln Tyr Val Arg Ser
195 200 205
Ile Val Ser Ser Val Gln Arg Val Ser Met Ala Ile Ser Pro Ser Arg
210 215 220
Ser Gly Leu Asn Ala Gly Gln Lys Ile Ile Ser Gly Phe Pro Glu Ala
225 230 235 240
Pro Thr Leu Ala Arg Trp Ile Cys Gln Ser Tyr Gln Phe His Leu Gly
245 250 255
Val Glu Leu Leu Arg Gln Ala Asp Asp Ala Gly Glu Ala Leu Leu Lys
260 265 270
Met Leu Trp Asp Tyr Glu Asp Ala Ile Leu Cys Cys Ser Phe Lys Glu
275 280 285
Lys Pro Val Phe Thr Phe Ala Asn Glu Met Gly Leu Asn Met Leu Glu
290 295 300
Thr Ser Leu Val Ala Leu Gln Asp Leu Ser Leu Asp Lys Ile Phe Asp
305 310 315 320
Glu Ala Gly Arg Lys Ala Leu Tyr Asn Glu Ile Pro Lys Leu Met Glu
325 330 335
Gln Gly Tyr Val Tyr Leu Pro Gly Gly Val Cys Leu Ser Gly Met Gly
340 345 350
Arg His Val Ser Phe Glu Gln Ala Val Ala Trp Lys Val
355 360 365

<210> 196
<211> 1395
<212> DNA
<213> Oryza sativa
<400> 196

```
gctcaagaga caagcgggga ggttgtatac gctttgggga ggcaacctgc tgttttgcgg      60
acatttagtc agaggttgag tagaggcttc aatgatgcta taagtggttt caatgatgat     120
ggttggtctg tcatgggtgg ggatggcatt gaagatgtga tcattgcttg caatgcaaag     180
aaggttagga atactagcac ttcggccaat gcttttgtaa ctccaggagg tgttatatgt     240
gctaaggcat ccatgctact gcagagtgtc ccacctgcag ttttggttcg atttttgagg     300
gaacatcgtt ctgaatgggc ggattataac ttcgatgcat attcagcttc atctctgaag     360
acaagctcat gttcacttcc tgggttgcgg cctatgagat tttctgggag ccagatcatt     420
atgccacttg ctcacacggt ggagaatgag gagattttag aagttgtccg tcttgaagga     480
caagcactta cacatgatga tggtcttatg tctagagata ttcacctgct tcagctttgc     540
actggaatag atgagaaatc aatgggatcc tgcttccagc ttgtctctgc accaatcgat     600
gagcttttcc ctgatgatgc tccgttaata tcttcaggct ttcgtgttat accgctggac     660
atgaaaacag atggtacacc tgctggtaga acattagatt tggcatctag ccttgaagtt     720
ggttcaactg cacagcccac aggggatgca tctatggatg actgtaatct acgatcagtg     780
ctgacaattg cctttcagtt cccttatgaa atgcatctcc aagacagcgt tgcaactatg     840
gcccggcaat atgtccgcag tattgtttcc tctgttcaga gagtatcaat ggctatttct     900
ccttctcggt ctggcttgaa tgctgggcag aagataattt caggcttccc tgaagcccca     960
acgctagctc gttggatttg ccaaagctac cagttccatt tgggggtgga gttacttagg    1020
caggcagatg atgctgggga agcactattg aaaatgctat gggattacga agacgctatt    1080
ttgtgctgtt ctttcaagga aaagcctgtg tttacttttg ccaacgagat gggactaaac    1140
atgctagaaa catctctcgt cgctctccaa gatctctcac tggacaagat atttgatgaa    1200
gccggtagga aggccctata caacgagatc ccgaaattga tggaacaggg ttacgtgtac    1260
ctgcctggtg gagtgtgctt gtccgggatg gggcgccatg tttctttcga gcaagctgta    1320
gcatggaagg tgctcggaga agacaacaat gtgcactgcc tcgccttctg cttcgtcaac    1380
tggtccttcg tgtga                                                     1395
```

<210> 197
<211> 464
<212> PRT
<213> Oryza sativa
<400> 197

Ala Gln Glu Thr Ser Gly Glu Val Val Tyr Ala Leu Gly Arg Gln Pro
1 5 10 15
Ala Val Leu Arg Thr Phe Ser Gln Arg Leu Ser Arg Gly Phe Asn Asp
20 25 30
Ala Ile Ser Gly Phe Asn Asp Asp Gly Trp Ser Val Met Gly Gly Asp

```
        35                  40                  45
Gly Ile Glu Asp Val Ile Ile Ala Cys Asn Ala Lys Lys Val Arg Asn
    50                  55                  60
Thr Ser Thr Ser Ala Asn Ala Phe Val Thr Pro Gly Gly Val Ile Cys
65                  70                  75                  80
Ala Lys Ala Ser Met Leu Leu Gln Ser Val Pro Pro Ala Val Leu Val
                85                  90                  95
Arg Phe Leu Arg Glu His Arg Ser Glu Trp Ala Asp Tyr Asn Phe Asp
            100                 105                 110
Ala Tyr Ser Ala Ser Ser Leu Lys Thr Ser Ser Cys Ser Leu Pro Gly
        115                 120                 125
Leu Arg Pro Met Arg Phe Ser Gly Ser Gln Ile Ile Met Pro Leu Ala
    130                 135                 140
His Thr Val Glu Asn Glu Glu Ile Leu Glu Val Val Arg Leu Glu Gly
145                 150                 155                 160
Gln Ala Leu Thr His Asp Asp Gly Leu Met Ser Arg Asp Ile His Leu
                165                 170                 175
Leu Gln Leu Cys Thr Gly Ile Asp Glu Lys Ser Met Gly Ser Cys Phe
            180                 185                 190
Gln Leu Val Ser Ala Pro Ile Asp Glu Leu Phe Pro Asp Asp Ala Pro
        195                 200                 205
Leu Ile Ser Ser Gly Phe Arg Val Ile Pro Leu Asp Met Lys Thr Asp
    210                 215                 220
Gly Thr Pro Ala Gly Arg Thr Leu Asp Leu Ala Ser Ser Leu Glu Val
225                 230                 235                 240
Gly Ser Thr Ala Gln Pro Thr Gly Asp Ala Ser Met Asp Asp Cys Asn
                245                 250                 255
Leu Arg Ser Val Leu Thr Ile Ala Phe Gln Phe Pro Tyr Glu Met His
            260                 265                 270
Leu Gln Asp Ser Val Ala Thr Met Ala Arg Gln Tyr Val Arg Ser Ile
        275                 280                 285
Val Ser Ser Val Gln Arg Val Ser Met Ala Ile Ser Pro Ser Arg Ser
    290                 295                 300
Gly Leu Asn Ala Gly Gln Lys Ile Ile Ser Gly Phe Pro Glu Ala Pro
305                 310                 315                 320
Thr Leu Ala Arg Trp Ile Cys Gln Ser Tyr Gln Phe His Leu Gly Val
                325                 330                 335
Glu Leu Leu Arg Gln Ala Asp Asp Ala Gly Glu Ala Leu Leu Lys Met
            340                 345                 350
Leu Trp Asp Tyr Glu Asp Ala Ile Leu Cys Cys Ser Phe Lys Glu Lys
        355                 360                 365
Pro Val Phe Thr Phe Ala Asn Glu Met Gly Leu Asn Met Leu Glu Thr
    370                 375                 380
Ser Leu Val Ala Leu Gln Asp Leu Ser Leu Asp Lys Ile Phe Asp Glu
385                 390                 395                 400
Ala Gly Arg Lys Ala Leu Tyr Asn Glu Ile Pro Lys Leu Met Glu Gln
                405                 410                 415
Gly Tyr Val Tyr Leu Pro Gly Gly Val Cys Leu Ser Gly Met Gly Arg
            420                 425                 430
His Val Ser Phe Glu Gln Ala Val Ala Trp Lys Val Leu Gly Glu Asp
        435                 440                 445
Asn Asn Val His Cys Leu Ala Phe Cys Phe Val Asn Trp Ser Phe Val
    450                 455                 460
```

<210> 198
<211> 3000
<212> DNA
<213> Oryza sativa
<400> 198

```
gacgtcaggt tatttttttt tctcctcgtc tcctgacgat ttcgcttttg ctcgaaatct     60
cccaggtttg ttgttgttgt gttgaaggcg gagaagaggg gaggctttgg tggtggtgga    120
ggaggaggat ggcggcggcg gtggcgatgc ggagcggcag cggcagcgac ggcggcggcg    180
gcgggtacga caaggccggg atggactccg gcaagtacgt gcggtacacg ccggagcagg    240
tggaggcgct ggagagggtg tacgccgagt gccccaagcc cagctcctcc cgccgccagc    300
agctgctccg cgactgtccc atcctcgcca acatcgagcc caagcagatc aaggtctggt    360
tccagaacag aaggtgccga gataagcagc ggaaggaggc atcaaggctt caggccgtga    420
accgaaaatt gacggcgatg aataagcttc tcatggagga gaatgagcgt cttcagaagc    480
aggtctccca gctggtccat gagaacgcgt acatgaagca gcaacttcag aatccgtcat    540
tgggcaatga tacaagctgt gaatcaaatg tgaccactcc tcagaaccct ctgagagatg    600
caagtaaccc gtctggactc cttacaattg cggaggagac cctgacagag ttcctctcca    660
aggctacagg gactgctgtt gattgggtgc caatgcctgg gatgaagcct ggtccggatt    720
cgtttggtat tgtggccgtt tcacatggtt gccgtggtgt tgctgcccgt gcctgtggtt    780
```

```
tggtgaatct agaaccaaca aagatcgtgg agatcttaaa agaccgccca tcttggttcc     840
gtgattgtcg aagtcttgaa gtcttcacaa tgtttccagc tggaaatggt ggcacgatcg     900
aacttgttta catgcagatg tatgctccta ctactttggt tcctgcacga gatttttgga     960
cacttagata cacaactaca atggaggatg gcagccttgt ggtctgtgag agatcattga    1020
gtggttctgg aggtggtcca agtacagcct ccgcacagca atttgtaaga gctgagatgc    1080
ttcctagcgg ctatctagtg cgcccatgcg agggtggtgg ctccatcgtg catattgtgg    1140
accatctgga tcttgaggct tggagtgttc cagaagtgct tcggccactc tacgagtcat    1200
ctagggtagt tgctcagaaa atgactactg cagcactacg gcacatcaga caaattgctc    1260
aagagacaag cggggaggtt gtatacgctt tggggaggca acctgctgtt ttgcggacat    1320
ttagtcagag gttgagtaga ggcttcaatg atgctataag tggtttcagt gatgatggtt    1380
ggtctgtcat gggtggggat ggcattgaag atgtgatcat tgcttgcaat gcaaagaagg    1440
ttaggaatac tagcacttcg gccaatgctt ttgtaactcc aggaggtgtt atatgtgcta    1500
aggcatccat gctactgcag agtgtcccac ctgcagtttt ggttcgattt ttgagggaac    1560
atcgttctga atgggcggat tataacttcg atgcatattc agcttcatct ctgaagacaa    1620
gctcatgttc acttcctggg ttgcggccta tgagattttc tgggagccag atcattatgc    1680
cacttgctca cacggtggag aatgaggaga ttttagaagt tgtccgtctt gaaggacaag    1740
cacttacaca tgatgatggt cttatgtcta gagatattca cctgcttcag ctttgcactg    1800
gaatagatga gaaatcaatg ggatcctgct tccagcttgt ctctgcacca atcgatgagc    1860
ttttccctga tgatgctccg ttaatatctt caggctttcg tgttataccg ctggacatga    1920
aaacagatgg tacacctgct ggtagaacat tagatttggc atctagcctt gaagttggtt    1980
caactgcaca gcccacaggg gatgcatcta tggatgactg taatctacga tcagtgctga    2040
caattgcctt tcagttccct tatgaaatgc atctccaaga cagcgttgca actatggccc    2100
ggcaatatgt ccgcagtatt gtttcctctg ttcagagagt atcaatggct atttctcctt    2160
ctcggtctgg cttgaatgct gggcagaaga taatttcagg cttccctgaa gccccaacgc    2220
tagctcgttg gatttgccaa agctaccagt tccatttggg ggtggagtta cttaggcagg    2280
cagatgatgc tggggaagca ctattgaaaa tgctatggga ttacgaagac gctattttgt    2340
gctgttcttt caaggaaaag cctgtgttta cttttgccaa cgagatggga ctaaacatgc    2400
tagaaacatc tctcgtcgct ctccaagatc tctcactgga caagatattt gatgaagccg    2460
gtaggaaggc cctatacaac gagatcccga aattgatgga acagggttac gtgtacctgc    2520
ctggtggagt gtgcttgtcc gggatggggc gccatgtttc tttcgagcaa gctgtagcat    2580
ggaaggtgct cggagaagac aacaatgtgc actgcctcgc cttctgcttc gtcaactggt    2640
ccttcgtgtg acccaaaatc caatccacca tgctcgcagt catcgtcgtt gtcgtcagat    2700
ctccattttt tgcaatctct gtagaagaga ggacaccaaa ccaaccacaa acctgtcagc    2760
tgttagctaa tgattctact agcttttcta gatctttgaa ggcaaaatgc tgccggtgcc    2820
tgtaaagagt gtgtgcgtgc gtgtgtaaat ttgggcgcgt tttgcactaa tttgatctgg    2880
ggtaaggagg cgctggctgc tgctgtgctg tagtttggta aaagttgagt acgctaacct    2940
tggatggtct cgaactgtat gatgaaattc ctagcagtaa aatttcaact tggatccgcg    3000
<210>  199
<211>  840
<212>  PRT
<213>  Oryza sativa
<400>  199
Met Ala Ala Ala Val Ala Met Arg Ser Gly Ser Gly Ser Asp Gly Gly
1               5                   10                  15
Gly Gly Gly Tyr Asp Lys Ala Gly Met Asp Ser Gly Lys Tyr Val Arg
            20                  25                  30
Tyr Thr Pro Glu Gln Val Glu Ala Leu Glu Arg Val Tyr Ala Glu Cys
        35                  40                  45
Pro Lys Pro Ser Ser Ser Arg Arg Gln Gln Leu Leu Arg Asp Cys Pro
    50                  55                  60
Ile Leu Ala Asn Ile Glu Pro Lys Gln Ile Lys Val Trp Phe Gln Asn
65                  70                  75                  80
Arg Arg Cys Arg Asp Lys Gln Arg Lys Glu Ala Ser Arg Leu Gln Ala
                85                  90                  95
Val Asn Arg Lys Leu Thr Ala Met Asn Lys Leu Leu Met Glu Glu Asn
            100                 105                 110
Glu Arg Leu Gln Lys Gln Val Ser Gln Leu Val His Glu Asn Ala Tyr
        115                 120                 125
Met Lys Gln Gln Leu Gln Asn Pro Ser Leu Gly Asn Asp Thr Ser Cys
    130                 135                 140
Glu Ser Asn Val Thr Thr Pro Gln Asn Pro Leu Arg Asp Ala Ser Asn
145                 150                 155                 160
Pro Ser Gly Leu Leu Thr Ile Ala Glu Glu Thr Leu Thr Glu Phe Leu
                165                 170                 175
Ser Lys Ala Thr Gly Thr Ala Val Asp Trp Val Pro Met Pro Gly Met
            180                 185                 190
Lys Pro Gly Pro Asp Ser Phe Gly Ile Val Ala Val Ser His Gly Cys
        195                 200                 205
Arg Gly Val Ala Ala Arg Ala Cys Gly Leu Val Asn Leu Glu Pro Thr
    210                 215                 220
Lys Ile Val Glu Ile Leu Lys Asp Arg Pro Ser Trp Phe Arg Asp Cys
```

```
225                 230                 235                 240
Arg Ser Leu Glu Val Phe Thr Met Phe Pro Ala Gly Asn Gly Gly Thr
                245                 250                 255
Ile Glu Leu Val Tyr Met Gln Met Tyr Ala Pro Thr Thr Leu Val Pro
            260                 265                 270
Ala Arg Asp Phe Trp Thr Leu Arg Tyr Thr Thr Thr Met Glu Asp Gly
        275                 280                 285
Ser Leu Val Val Cys Glu Arg Ser Leu Ser Gly Ser Gly Gly Gly Pro
    290                 295                 300
Ser Thr Ala Ser Ala Gln Gln Phe Val Arg Ala Glu Met Leu Pro Ser
305                 310                 315                 320
Gly Tyr Leu Val Arg Pro Cys Glu Gly Gly Gly Ser Ile Val His Ile
                325                 330                 335
Val Asp His Leu Asp Leu Glu Ala Trp Ser Val Pro Glu Val Leu Arg
            340                 345                 350
Pro Leu Tyr Glu Ser Ser Arg Val Val Ala Gln Lys Met Thr Thr Ala
        355                 360                 365
Ala Leu Arg His Ile Arg Gln Ile Ala Gln Glu Thr Ser Gly Glu Val
    370                 375                 380
Val Tyr Ala Leu Gly Arg Gln Pro Ala Val Leu Arg Thr Phe Ser Gln
385                 390                 395                 400
Arg Leu Ser Arg Gly Phe Asn Asp Ala Ile Ser Gly Phe Asn Asp Asp
                405                 410                 415
Gly Trp Ser Val Met Gly Gly Asp Gly Ile Glu Asp Val Ile Ile Ala
            420                 425                 430
Cys Asn Ala Lys Lys Val Arg Asn Thr Ser Thr Ser Ala Asn Ala Phe
        435                 440                 445
Val Thr Pro Gly Gly Val Ile Cys Ala Lys Ala Ser Met Leu Leu Gln
    450                 455                 460
Ser Val Pro Pro Ala Val Leu Val Arg Phe Leu Arg Glu His Arg Ser
465                 470                 475                 480
Glu Trp Ala Asp Tyr Asn Phe Asp Ala Tyr Ser Ala Ser Ser Leu Lys
                485                 490                 495
Thr Ser Ser Cys Ser Leu Pro Gly Leu Arg Pro Met Arg Phe Ser Gly
            500                 505                 510
Ser Gln Ile Ile Met Pro Leu Ala His Thr Val Glu Asn Glu Glu Ile
        515                 520                 525
Leu Glu Val Val Arg Leu Glu Gly Gln Ala Leu Thr His Asp Asp Gly
    530                 535                 540
Leu Met Ser Arg Asp Ile His Leu Leu Gln Leu Cys Thr Gly Ile Asp
545                 550                 555                 560
Glu Lys Ser Met Gly Ser Cys Phe Gln Leu Val Ser Ala Pro Ile Asp
                565                 570                 575
Glu Leu Phe Pro Asp Asp Ala Pro Leu Ile Ser Ser Gly Phe Arg Val
            580                 585                 590
Ile Pro Leu Asp Met Lys Thr Asp Gly Thr Pro Ala Gly Arg Thr Leu
        595                 600                 605
Asp Leu Ala Ser Ser Leu Glu Val Gly Ser Thr Ala Gln Pro Thr Gly
    610                 615                 620
Asp Ala Ser Met Asp Asp Cys Asn Leu Arg Ser Val Leu Thr Ile Ala
625                 630                 635                 640
Phe Gln Phe Pro Tyr Glu Met His Leu Gln Asp Ser Val Ala Thr Met
                645                 650                 655
Ala Arg Gln Tyr Val Arg Ser Ile Val Ser Ser Val Gln Arg Val Ser
            660                 665                 670
Met Ala Ile Ser Pro Ser Arg Ser Gly Leu Asn Ala Gly Gln Lys Ile
        675                 680                 685
Ile Ser Gly Phe Pro Glu Ala Pro Thr Leu Ala Arg Trp Ile Cys Gln
    690                 695                 700
Ser Tyr Gln Phe His Leu Gly Val Glu Leu Leu Arg Gln Ala Asp Asp
705                 710                 715                 720
Ala Gly Glu Ala Leu Leu Lys Met Leu Trp Asp Tyr Glu Asp Ala Ile
                725                 730                 735
Leu Cys Cys Ser Phe Lys Glu Lys Pro Val Phe Thr Phe Ala Asn Glu
            740                 745                 750
Met Gly Leu Asn Met Leu Glu Thr Ser Leu Val Ala Leu Gln Asp Leu
        755                 760                 765
Ser Leu Asp Lys Ile Phe Asp Glu Ala Gly Arg Lys Ala Leu Tyr Asn
    770                 775                 780
Glu Ile Pro Lys Leu Met Glu Gln Gly Tyr Val Tyr Leu Pro Gly Gly
785                 790                 795                 800
```

Val Cys Leu Ser Gly Met Gly Arg His Val Ser Phe Glu Gln Ala Val
805 810 815
Ala Trp Lys Val Leu Gly Glu Asp Asn Asn Val His Cys Leu Ala Phe
820 825 830
Cys Phe Val Asn Trp Ser Phe Val
835 840

<210> 200
<211> 3252
<212> DNA
<213> Oryza sativa
<400> 200

```
ctctctcccg tgctgctctc ccttctcctc tcctcaatta ccacgccgca gccgcagccc      60
cccaacccta gctagtacgg cgggcgacct gagggggtag ctgcctacct ctatacctgc     120
tatctccgtt cgatccgggt cgggctcgtg ctgctggggg cgggggcaat caatcggcga     180
gctcggtgat ccatggccgt ggctgtgggg tggctgtgag ggtgcccccg gcggcgctcc     240
cctccgcgcc tgccggcgag ggggctcgga ctgaagggat ctaggcgagc tgaaaattga     300
agtgcaggca aggagataag agcagcgtcc aaattgtgag tacttcatta gcaggaggta     360
gtggttgtgc ttgcttggct cctttgcaat ttggctttgg cgaggtagca atggctgcgg     420
cagtggcaat gcgagggagt agcagtgatg gaggtggcta tgataaggtt tccgggatgg     480
actccggtaa atatgtgcgc tacacgcctg agcaggtgga ggcgcttgag cgggtgtacg     540
ccgattgccc caagccaacc tcctcccgca ggcagcaact gctgcgtgag tgccccatac     600
ttgctaacat tgagcccaag cagatcaagg tctggttcca gaacagaagg tgccgggata     660
agcagcggaa ggagtcttca cggcttcagg ctgtcaacag gaaattgacg gcaatgaaca     720
agctacttat ggaagagaat gagcgactcc agaagcaggt ctcccaattg gttcatgaga     780
atgcccacat gcgacagcag ctgcagaata ctccgctggc aaatgataca agctgtgaat     840
caaatgtgac taccccctcaa aaccctttaa gggatgcaag taaccccctct gggctccttt     900
caattgcaga ggagaccttg acagagttcc tctcaaaggc tactggtaca gctattgatt     960
gggtccagat gcctgggatg aagcctggtc cggattcggt tggtattgtg gccatttcac    1020
atggttgccg tggtgttgct gcccgtgcct gtggtttggt gaacctagaa ccaacaaaag    1080
tggtagagat attgaaagat cgtccatctt ggttccgtga ttgtcgaaac ctggaagtct    1140
ttacaatgat tccagcagga aatggaggaa cggttgaact tgtctacaca cagttgtatg    1200
ctccaacaac tttagttcct gcacgagatt tttggacgtt acggtacaca accacaatgg    1260
aagatggcag tcttgtggtc tgtgagagat ctttaagtgg ttcagggggc ggtccaagtg    1320
ctgcctctgc tcagcaatat gtgagagcgg aaatgcttcc aagtggatac ctggttcgcc    1380
catgtgaagg tgggggatca attgtgcaca tagtggacca tctggatctt gaggcatgga    1440
gtgttcctga ggtgcttcgg ccactctatg aatcttcaag ggtagtcgct cagaaaatga    1500
ctactgcggc actccggcac atcagacaaa ttgctcaaga aacaagtggg gaagtggtgt    1560
atgccttggg gaggcaacca gcagtgctac ggacttttag tcaaaggctg agcagaggct    1620
ttaacgatgc cattagtggt ttcaatgatg atgggtggtc tataatgggc ggagacggtg    1680
ttgaagatgt agttattgct tgcaactcaa ctaagaaaat taggagtaac agcaatgctg    1740
gcatcgcctt tggagccccc ggaggtatta tatgtgctaa ggcatcaatg ttactgcaga    1800
gtgttcctcc agcagtactg gttcgatttc tgagggagca tagatctgaa tgggccgatt    1860
acaatattga tgcatatttg gcttcaactc tgaaaacaag tgcatgttca cttactgggt    1920
tgcgacccat gagattttct gggagccaaa tcatcattcc acttgctcac acagttgaga    1980
atgaggagat tcttgaagtt gttcgccttg agggtcaacc tcttactcat gatgaagctc    2040
ttctttcaag ggatatccac ctgcttcagc tctgcactgg aatagacgag aagtctgtgg    2100
gatcctcctt tcagcttgtg tttgcaccga ttgatgattt cccagatgaa actccattga    2160
tttcttctgg cttccgtgtt ataccacttg atatgaaaac agatggtgca tcctctggta    2220
ggacattaga tttggcatct agtcttgaag taggttcagc aacagctcaa gcctccggag    2280
atgcatctgc agatgattgt aacttgcgat ctgttctgac gatcgctttt caattcccct    2340
acgagttgca tctccaagac agtgttgcag ctatggctcg ccaatatgtc cgtagcattg    2400
tttctgctgt gcaaagagtg tcaatggcta tctctccctc tcaaactggt ctaaatgccg    2460
gacagaggat aatctctggt ttccctgaag cagcaaccct tgctcgatgg gtttgccaga    2520
gctaccatta ccatctaggg gtagagttac ttagtcaatc agatggagat gcagaacaat    2580
tgttgaagat gctatggcat taccaagatg ctattttgtg ctgctcattc aaggaaaaac    2640
cggtgtttac atttgccaac aaagcaggac tggacatgct agaaacttcc cttgtcgcct    2700
tacaggacct cacattggac aggatctttg atgagcctgg aaaagaagca ttgttctcaa    2760
acattcccaa attgatggag cagggccatg tctacctgcc atcaggcgtg tgcatgtcag    2820
gaatgggtcg gcatgtttct ttcgatcagg ccgtggcttg gaaagtgctt gccgaggata    2880
gcaatgttca ctgcctggcc ttctgtttcg tcaactggtc ctttgtgtga ccatccccaca    2940
tccactgcga agtgaagatt catttttgct tgttcaagac tgttttgcac tagatctaga    3000
cctgagaatc tagtagtatt tgcgaaattt ctcgcttatg taaatgtaat ctcgctccca    3060
ttccatcaag tacccaagta caaggcagtg gccaagtggt tttagttgta gggggatcgg    3120
agcaatcctg acggttgggt acttgggttc cttccagcaa tgtaaaatcg gatcctgtag    3180
ggcgtcgaaa actgcattgg caagattctt cgaaatgcag accaaattta gctagtacat    3240
cgtaactttg gc                                                       3252
```

<210> 201
<211> 839
<212> PRT
<213> Oryza sativa
<400> 201

```
Met Ala Ala Ala Val Ala Met Arg Gly Ser Ser Ser Asp Gly Gly Gly
1               5                   10                  15
Tyr Asp Lys Val Ser Gly Met Asp Ser Gly Lys Tyr Val Arg Tyr Thr
            20                  25                  30
Pro Glu Gln Val Glu Ala Leu Glu Arg Val Tyr Ala Asp Cys Pro Lys
        35                  40                  45
Pro Thr Ser Ser Arg Arg Gln Gln Leu Leu Arg Glu Cys Pro Ile Leu
    50                  55                  60
Ala Asn Ile Glu Pro Lys Gln Ile Lys Val Trp Phe Gln Asn Arg Arg
65                  70                  75                  80
Cys Arg Asp Lys Gln Arg Lys Glu Ser Ser Arg Leu Gln Ala Val Asn
                85                  90                  95
Arg Lys Leu Thr Ala Met Asn Lys Leu Leu Met Glu Glu Asn Glu Arg
            100                 105                 110
Leu Gln Lys Gln Val Ser Gln Leu Val His Glu Asn Ala His Met Arg
        115                 120                 125
Gln Gln Leu Gln Asn Thr Pro Leu Ala Asn Asp Thr Ser Cys Glu Ser
    130                 135                 140
Asn Val Thr Thr Pro Gln Asn Pro Leu Arg Asp Ala Ser Asn Pro Ser
145                 150                 155                 160
Gly Leu Leu Ser Ile Ala Glu Glu Thr Leu Thr Glu Phe Leu Ser Lys
                165                 170                 175
Ala Thr Gly Thr Ala Ile Asp Trp Val Gln Met Pro Gly Met Lys Pro
            180                 185                 190
Gly Pro Asp Ser Val Gly Ile Val Ala Ile Ser His Gly Cys Arg Gly
        195                 200                 205
Val Ala Ala Arg Ala Cys Gly Leu Val Asn Leu Glu Pro Thr Lys Val
    210                 215                 220
Val Glu Ile Leu Lys Asp Arg Pro Ser Trp Phe Arg Asp Cys Arg Asn
225                 230                 235                 240
Leu Glu Val Phe Thr Met Ile Pro Ala Gly Asn Gly Gly Thr Val Glu
                245                 250                 255
Leu Val Tyr Thr Gln Leu Tyr Ala Pro Thr Thr Leu Val Pro Ala Arg
            260                 265                 270
Asp Phe Trp Thr Leu Arg Tyr Thr Thr Thr Met Glu Asp Gly Ser Leu
        275                 280                 285
Val Val Cys Glu Arg Ser Leu Ser Gly Ser Gly Gly Gly Pro Ser Ala
    290                 295                 300
Ala Ser Ala Gln Gln Tyr Val Arg Ala Glu Met Leu Pro Ser Gly Tyr
305                 310                 315                 320
Leu Val Arg Pro Cys Glu Gly Gly Gly Ser Ile Val His Ile Val Asp
                325                 330                 335
His Leu Asp Leu Glu Ala Trp Ser Val Pro Glu Val Leu Arg Pro Leu
            340                 345                 350
Tyr Glu Ser Ser Arg Val Val Ala Gln Lys Met Thr Thr Ala Ala Leu
        355                 360                 365
Arg His Ile Arg Gln Ile Ala Gln Glu Thr Ser Gly Glu Val Val Tyr
    370                 375                 380
Ala Leu Gly Arg Gln Pro Ala Val Leu Arg Thr Phe Ser Gln Arg Leu
385                 390                 395                 400
Ser Arg Gly Phe Asn Asp Ala Ile Ser Gly Phe Asn Asp Asp Gly Trp
                405                 410                 415
Ser Ile Met Gly Gly Asp Gly Val Glu Asp Val Val Ile Ala Cys Asn
            420                 425                 430
Ser Thr Lys Lys Ile Arg Ser Asn Ser Asn Ala Gly Ile Ala Phe Gly
        435                 440                 445
Ala Pro Gly Gly Ile Ile Cys Ala Lys Ala Ser Met Leu Leu Gln Ser
    450                 455                 460
Val Pro Pro Ala Val Leu Val Arg Phe Leu Arg Glu His Arg Ser Glu
465                 470                 475                 480
Trp Ala Asp Tyr Asn Ile Asp Ala Tyr Leu Ala Ser Thr Leu Lys Thr
                485                 490                 495
Ser Ala Cys Ser Leu Thr Gly Leu Arg Pro Met Arg Phe Ser Gly Ser
            500                 505                 510
Gln Ile Ile Ile Pro Leu Ala His Thr Val Glu Asn Glu Glu Ile Leu
        515                 520                 525
Glu Val Val Arg Leu Glu Gly Gln Pro Leu Thr His Asp Glu Ala Leu
    530                 535                 540
Leu Ser Arg Asp Ile His Leu Leu Gln Leu Cys Thr Gly Ile Asp Glu
545                 550                 555                 560
Lys Ser Val Gly Ser Ser Phe Gln Leu Val Phe Ala Pro Ile Asp Asp
```

```
                        565                 570                 575
Phe Pro Asp Glu Thr Pro Leu Ile Ser Ser Gly Phe Arg Val Ile Pro
            580                 585                 590
Leu Asp Met Lys Thr Asp Gly Ala Ser Ser Gly Arg Thr Leu Asp Leu
        595                 600                 605
Ala Ser Ser Leu Glu Val Gly Ser Ala Thr Ala Gln Ala Ser Gly Asp
    610                 615                 620
Ala Ser Ala Asp Asp Cys Asn Leu Arg Ser Val Leu Thr Ile Ala Phe
625                 630                 635                 640
Gln Phe Pro Tyr Glu Leu His Leu Gln Asp Ser Val Ala Ala Met Ala
                645                 650                 655
Arg Gln Tyr Val Arg Ser Ile Val Ser Ala Val Gln Arg Val Ser Met
            660                 665                 670
Ala Ile Ser Pro Ser Gln Thr Gly Leu Asn Ala Gly Gln Arg Ile Ile
        675                 680                 685
Ser Gly Phe Pro Glu Ala Ala Thr Leu Ala Arg Trp Val Cys Gln Ser
    690                 695                 700
Tyr His Tyr His Leu Gly Val Glu Leu Leu Ser Gln Ser Asp Gly Asp
705                 710                 715                 720
Ala Glu Gln Leu Leu Lys Met Leu Trp His Tyr Gln Asp Ala Ile Leu
                725                 730                 735
Cys Cys Ser Phe Lys Glu Lys Pro Val Phe Thr Phe Ala Asn Lys Ala
            740                 745                 750
Gly Leu Asp Met Leu Glu Thr Ser Leu Val Ala Leu Gln Asp Leu Thr
        755                 760                 765
Leu Asp Arg Ile Phe Asp Glu Pro Gly Lys Glu Ala Leu Phe Ser Asn
    770                 775                 780
Ile Pro Lys Leu Met Glu Gln Gly His Val Tyr Leu Pro Ser Gly Val
785                 790                 795                 800
Cys Met Ser Gly Met Gly Arg His Val Ser Phe Asp Gln Ala Val Ala
                805                 810                 815
Trp Lys Val Leu Ala Glu Asp Ser Asn Val His Cys Leu Ala Phe Cys
            820                 825                 830
Phe Val Asn Trp Ser Phe Val
        835
```

<210> 202
<211> 3169
<212> DNA
<213> Arabidopsis thaliana
<400> 202

```
agagtcttca aaacttttgc agcttcaatt gtacctgggt ttcttcttca ttgttcctaa      60
ggtttctgtg tccttcaatt cttctgatat aatgcttctt taagagagtt gacatcatca     120
ctttcttggg gtactcttct ctgtttctcc ccagaaaatc caactctgta attttgggtc     180
tttattctgt ttttctcttt gaagaatctt taaaattctc agatcttctg aatctctctt     240
ctttaaaact ttttttaact ttattttttg tactcgcttc tttgccttca tttttctcgt     300
atccacatgt cgttggtctt tcgctacaag ccacgaccgt agaatcttct tttgtctgaa     360
aagaattaca atttacgttt ctcttacgat acgacggact ttccgaagaa attaatttaa     420
agagaaaaga agaagaagcc aaagaagaag aagaagctag aagaaacagt aaagtttgag     480
acttttttttg agggtcgagc taaaatggag atggcggtgg ctaaccaccg tgagagaagc     540
agtgacagta tgaatagaca tttagatagt agcggtaagt acgttaggta cacagctgag     600
caagtcgagg ctcttgagcg tgtctacgct gagtgtccta agcctagctc tctccgtcga     660
caacaattga tccgtgaatg ttccattttg gccaatattg agcctaagca gatcaaagtc     720
tggtttcaga accgcaggtg tcgagataag cagaggaaag aggcgtcgag gctccagagc     780
gtaaaccgga agctctctgc gatgaataaa ctgttgatgg aggagaatga taggttgcag     840
aagcaggttt ctcagcttgt ctgcgaaaat ggatatatga aacagcagct aactactgtt     900
gttaacgatc caagctgtga atctgtggtc acaactcctc agcattcgct tagagatgcg     960
aatagtcctg ctggattgct ctcaatcgca gaggagactt tggcagagtt cctatccaag    1020
gctacaggaa ctgctgttga ttgggttcag atgcctggga tgaagcctgg tccggattcg    1080
gttggcatct ttgccatttc gcaaagatgc aatggagtgg cagctcgagc ctgtggtctt    1140
gttagcttag aacctatgaa gattgcagag atcctcaaag atcggccatc ttggttccgt    1200
gactgtagga gccttgaagt tttcactatg ttcccggctg gtaatggtgg cacaatcgag    1260
cttgtttata tgcagacgta tgcaccaacg actctggctc ctgcccgcga tttctggacc    1320
ctgagataca caacgagcct cgacaatggg agttttgtgg tttgtgagag gtcgctatct    1380
ggctctggag ctgggcctaa tgctgcttca gcttctcagt ttgtgagagc agaaatgctt    1440
tctagtgggt atttaataag gccttgtgat ggtggtggtt ctattattca cattgtcgat    1500
caccttaatc ttgaggcttg gagtgttccg gatgtgcttc gaccccttta tgagtcatcc    1560
aaagtcgttg cacaaaaaat gaccatttcc gcgttgcggt atatcaggca attagcccaa    1620
gagtctaatg gtgaagtagt gtatggatta ggaaggcagc ctgctgttct tagaaccttt    1680
agccaaagat taagcagggg cttcaatgat gcggttaatg ggtttggtga cgacgggtgg    1740
tctacgatgc attgtgatgg agcggaagat attatcgttg ctattaactc tacaaagcat    1800
ttgaataata tttctaattc tctttcgttc cttggaggcg tgctctgtgc caaggcttca    1860
```

```
atgcttctcc aaaatgttcc tcctgcggtt ttgatccggt tccttagaga gcatcgatct 1920
gagtgggctg atttcaatgt tgatgcatat tccgctgcta cacttaaagc tggtagcttt 1980
gcttatccgg gaatgagacc aacaagattc actgggagtc agatcataat gccactagga 2040
catacaattg aacacgaaga aatgctagaa gttgttagac tggaaggtca ttctcttgct 2100
caagaagatg catttatgtc acgggatgtc catctccttc agatttgtac cgggattgac 2160
gagaatgccg ttggagcttg ttctgaactg atatttgctc cgattaatga gatgttcccg 2220
gatgatgctc cacttgttcc ctctggattc cgagtcatac ccgttgatgc taaaacggga 2280
gatgtacaag atctgttaac cgctaatcac cgtacactag acttaacttc tagccttgaa 2340
gtcggtccat cacctgagaa tgcttctgga aactcttttt ctagctcaag ctcgagatgt 2400
attctcacta tcgcgtttca attccctttt gaaaacaact tgcaagaaaa tgttgctggt 2460
atggcttgtc agtatgtgag gagcgtgatc tcatcagttc aacgtgttgc aatggcgatc 2520
tcaccgtctg ggataagccc gagtctgggc tccaaattgt ccccaggatc tcctgaagct 2580
gttactcttg ctcagtggat ctctcaaagt tacagtcatc acttaggctc ggagttgctg 2640
acgattgatt cacttggaag cgacgactcg gtactaaaac ttctatggga tcaccaagat 2700
gccatcctgt gttgctcatt aaagccacag ccagtgttca tgtttgcgaa ccaagctggt 2760
ctagacatgc tagagacaac acttgtagcc ttacaagata taacactcga aaagatattc 2820
gatgaatcgg gtcgtaaggc tatctgttcg gacttcgcca agctaatgca acagggattt 2880
gcttgcttgc cttcaggaat ctgtgtgtca acgatgggaa gacatgtgag ttatgaacaa 2940
gctgttgctt ggaaagtgtt tgctgcatct gaagaaaaca acaacaatct gcattgtctt 3000
gccttctcct ttgtaaactg gtcttttgtg tgattcgatt gacagaaaaa gactaattta 3060
aatttacgtt agagaactca aatttttggt tgttgtttag gtgtctctgt tttgtttttt 3120
aaaattattt tgatcaaatg ttactcactt tcttctttca aaaaaaaaa             3169
```

<210> 203
<211> 842
<212> PRT
<213> Arabidopsis thaliana
<400> 203

```
Met Glu Met Ala Val Ala Asn His Arg Glu Arg Ser Ser Asp Ser Met
1               5                   10                  15
Asn Arg His Leu Asp Ser Ser Gly Lys Tyr Val Arg Tyr Thr Ala Glu
            20                  25                  30
Gln Val Glu Ala Leu Glu Arg Val Tyr Ala Glu Cys Pro Lys Pro Ser
        35                  40                  45
Ser Leu Arg Arg Gln Gln Leu Ile Arg Glu Cys Ser Ile Leu Ala Asn
    50                  55                  60
Ile Glu Pro Lys Gln Ile Lys Val Trp Phe Gln Asn Arg Arg Cys Arg
65                  70                  75                  80
Asp Lys Gln Arg Lys Glu Ala Ser Arg Leu Gln Ser Val Asn Arg Lys
                85                  90                  95
Leu Ser Ala Met Asn Lys Leu Leu Met Glu Glu Asn Asp Arg Leu Gln
            100                 105                 110
Lys Gln Val Ser Gln Leu Val Cys Glu Asn Gly Tyr Met Lys Gln Gln
        115                 120                 125
Leu Thr Thr Val Val Asn Asp Pro Ser Cys Glu Ser Val Val Thr Thr
    130                 135                 140
Pro Gln His Ser Leu Arg Asp Ala Asn Ser Pro Ala Gly Leu Leu Ser
145                 150                 155                 160
Ile Ala Glu Glu Thr Leu Ala Glu Phe Leu Ser Lys Ala Thr Gly Thr
                165                 170                 175
Ala Val Asp Trp Val Gln Met Pro Gly Met Lys Pro Gly Pro Asp Ser
            180                 185                 190
Val Gly Ile Phe Ala Ile Ser Gln Arg Cys Asn Gly Val Ala Ala Arg
        195                 200                 205
Ala Cys Gly Leu Val Ser Leu Glu Pro Met Lys Ile Ala Glu Ile Leu
    210                 215                 220
Lys Asp Arg Pro Ser Trp Phe Arg Asp Cys Arg Ser Leu Glu Val Phe
225                 230                 235                 240
Thr Met Phe Pro Ala Gly Asn Gly Gly Thr Ile Glu Leu Val Tyr Met
                245                 250                 255
Gln Thr Tyr Ala Pro Thr Thr Leu Ala Pro Ala Arg Asp Phe Trp Thr
            260                 265                 270
Leu Arg Tyr Thr Thr Ser Leu Asp Asn Gly Ser Phe Val Val Cys Glu
        275                 280                 285
Arg Ser Leu Ser Gly Ser Gly Ala Gly Pro Asn Ala Ala Ser Ala Ser
    290                 295                 300
Gln Phe Val Arg Ala Glu Met Leu Ser Ser Gly Tyr Leu Ile Arg Pro
305                 310                 315                 320
Cys Asp Gly Gly Gly Ser Ile Ile His Ile Val Asp His Leu Asn Leu
                325                 330                 335
Glu Ala Trp Ser Val Pro Asp Val Leu Arg Pro Leu Tyr Glu Ser Ser
            340                 345                 350
```

Lys Val Val Ala Gln Lys Met Thr Ile Ser Ala Leu Arg Tyr Ile Arg
355 360 365
Gln Leu Ala Gln Glu Ser Asn Gly Glu Val Val Tyr Gly Leu Gly Arg
370 375 380
Gln Pro Ala Val Leu Arg Thr Phe Ser Gln Arg Leu Ser Arg Gly Phe
385 390 395 400
Asn Asp Ala Val Asn Gly Phe Gly Asp Asp Gly Trp Ser Thr Met His
405 410 415
Cys Asp Gly Ala Glu Asp Ile Ile Val Ala Ile Asn Ser Thr Lys His
420 425 430
Leu Asn Asn Ile Ser Asn Ser Leu Ser Phe Leu Gly Gly Val Leu Cys
435 440 445
Ala Lys Ala Ser Met Leu Leu Gln Asn Val Pro Pro Ala Val Leu Ile
450 455 460
Arg Phe Leu Arg Glu His Arg Ser Glu Trp Ala Asp Phe Asn Val Asp
465 470 475 480
Ala Tyr Ser Ala Ala Thr Leu Lys Ala Gly Ser Phe Ala Tyr Pro Gly
485 490 495
Met Arg Pro Thr Arg Phe Thr Gly Ser Gln Ile Ile Met Pro Leu Gly
500 505 510
His Thr Ile Glu His Glu Glu Met Leu Glu Val Val Arg Leu Glu Gly
515 520 525
His Ser Leu Ala Gln Glu Asp Ala Phe Met Ser Arg Asp Val His Leu
530 535 540
Leu Gln Ile Cys Thr Gly Ile Asp Glu Asn Ala Val Gly Ala Cys Ser
545 550 555 560
Glu Leu Ile Phe Ala Pro Ile Asn Glu Met Phe Pro Asp Asp Ala Pro
565 570 575
Leu Val Pro Ser Gly Phe Arg Val Ile Pro Val Asp Ala Lys Thr Gly
580 585 590
Asp Val Gln Asp Leu Leu Thr Ala Asn His Arg Thr Leu Asp Leu Thr
595 600 605
Ser Ser Leu Glu Val Gly Pro Ser Pro Glu Asn Ala Ser Gly Asn Ser
610 615 620
Phe Ser Ser Ser Ser Ser Arg Cys Ile Leu Thr Ile Ala Phe Gln Phe
625 630 635 640
Pro Phe Glu Asn Asn Leu Gln Glu Asn Val Ala Gly Met Ala Cys Gln
645 650 655
Tyr Val Arg Ser Val Ile Ser Ser Val Gln Arg Val Ala Met Ala Ile
660 665 670
Ser Pro Ser Gly Ile Ser Pro Ser Leu Gly Ser Lys Leu Ser Pro Gly
675 680 685
Ser Pro Glu Ala Val Thr Leu Ala Gln Trp Ile Ser Gln Ser Tyr Ser
690 695 700
His His Leu Gly Ser Glu Leu Leu Thr Ile Asp Ser Leu Gly Ser Asp
705 710 715 720
Asp Ser Val Leu Lys Leu Leu Trp Asp His Gln Asp Ala Ile Leu Cys
725 730 735
Cys Ser Leu Lys Pro Gln Pro Val Phe Met Phe Ala Asn Gln Ala Gly
740 745 750
Leu Asp Met Leu Glu Thr Thr Leu Val Ala Leu Gln Asp Ile Thr Leu
755 760 765
Glu Lys Ile Phe Asp Glu Ser Gly Arg Lys Ala Ile Cys Ser Asp Phe
770 775 780
Ala Lys Leu Met Gln Gln Gly Phe Ala Cys Leu Pro Ser Gly Ile Cys
785 790 795 800
Val Ser Thr Met Gly Arg His Val Ser Tyr Glu Gln Ala Val Ala Trp
805 810 815
Lys Val Phe Ala Ala Ser Glu Glu Asn Asn Asn Asn Leu His Cys Leu
820 825 830
Ala Phe Ser Phe Val Asn Trp Ser Phe Val
835 840

<210> 204
<211> 2700
<212> DNA
<213> Zea mays
<400> 204

gagagctaag agcaacaagg cgacgagatt tgtgtggcta cgattttgcg ttgccgacgg 60
aacatgggaa caatggttgc agcggtggcg ttgcgagggg gaagcagcga tagcggagga 120
tttgataagg ttcccgggat ggactcggga aaatacgtgc gctacacccc ggagcaagtt 180
gaggtgctcg agcggctcta catagattgc cccaagccaa gctcctcacg gcggcagcaa 240

ctgctgcgcg agtgtcctat actctccaac attgagccaa agcagatcaa ggtctggttc 300
cagaaccgga ggtgccgcga taagcagcgg aaggagtctt cgcggcttca ggctgtgaac 360
agaaagttga cggcaatgaa caagcttcta atggaagaga atgagcggct tcagaagcaa 420
gtctctcagt tggttcatga aaacgcgcac atgcggcagc agctgcagaa tacttcattg 480
gccaatgaca caagctgtga atcaaatgtc actacccctc caaaccctat aagggacgca 540
agtaaccctt ctggactcct tgcgattgca gaggagacct tcacagagtt cctctcaaag 600
gctactggga cagctattga ttgggtccag atgcctggga tgaagcctgg tccggattca 660
gttggtatcg tggccatttc gcatggttgc cgtggcgttg ctgcccgcgc ctgtggtttg 720
gtgaatctag aaccaacaaa aggcatagag atcttgaaag atcgtccctc ttggttccgt 780
gattgccgaa gtcttgaagt gtttacaagg tttccagctg gaaatggggg aacaattgaa 840
cttatttaca tgcagatgta tgccccaaca actttagtcc ctgcacgtga tttttggaca 900
ctacgataca cgacaacaat ggaagatggc agccttgtgg tctgtgagag atccttgagt 960
ggttctggtg gtggtccaaa tgcagcctct acacaacaat ttgttagggc tgagatgctt 1020
ccaagtgggt atttagttcg cccatgcgaa ggtggaggat caattgtgca tatagtggac 1080
catctagatc tcgaagcatg gagtgttcct gaagtgcttc gaccactgta tgagtcttct 1140
agagttgttg ctcagaaaat gactactgtg gcactgcgcc accttagaca aattgctcaa 1200
gaaacaagtg gagaagtagt gtacgccttg ggaaggcaac ctgcagtcct acggaccttt 1260
agtcaaagac taagcagggg gtttaatgat gccattagcg gtttcaatga tgatggctgg 1320
tctgtaatgg ggggagatgg cattgaagac gttgttattg cttgcaactc aaccaagaaa 1380
attaggaata ccagcaatgc tgggattaca tttggagccc caggaggcat tatttgtgcg 1440
aaggcatcca tgttactgca gagcgtccca ccggcagtac tggtacgatt tctgagggag 1500
catagatctg aatgggctga ttacaatatt gatgcgtatt tggcttcatc attgaagacc 1560
agtgcgtgct cacttcctgg gttgcgaccc atgagatttt ctgaggggca gatgatcatg 1620
ccacttgctc acacagttga gaacgaggag attcttgaag ttgtccgcct tgaaggtcag 1680
cctcttactc atgatgaagc tcttctttca agggacatcc accttctcca gctttgcact 1740
ggaatagatg agaaatctgt gggttcctcc ttccagcttg tgtttgcacc aattgatgag 1800
catttcccag atgatgctcc attgatttct tctggctttc gtgtcatacc acttgatgtg 1860
aaaacagatg gtgtatcctc tggtaggacg ctagatttgg catctagtct tgatgtgggc 1920
tctgctgcac cccaagcctc aggggatgca tctccagatg actgcagttt gagatctgtg 1980
ctgacaatcg cctttcaatt cccgtatgag atgcaccttc aggacagcgt tgcagcaatg 2040
gcccgtcaat atgttcgtag tgtcatttct gctgtgcaaa gagtgtcgat ggctatatct 2100
ccctcccaat ctggtctaaa tgctgggcat aggatgcttt ctggcttccc tgaagctgcc 2160
acacttgcta gatgggtttg ccagagttat cactaccatc taggtatgga attacttaat 2220
caatcagatg gagctggtga agcattgttg aaaatgctct ggcatcatcc agatgctgtt 2280
ctgtgctgct cctttaagga gaaacctatg tttacgtttg caaacaaggc agggctggac 2340
atgttagaaa catctcttgt tgccctgcaa gacctgacgc tagacaagat cttcgacgag 2400
tcaggaagga aagcactatt ctcagacatc tcgaaactaa tggaacaggg ctacgcgtac 2460
ctgccgtcag gcgtgtgcat gtcaggaatg ggccgccatg tttctttcga ccaagctgta 2520
gcgtggaagg tgctcggcga ggatagcaac atccactgcc tggcgttctg cttcgtcaac 2580
tggtccttcg tgtgactgac gcccaacccg tcgggtggtt atggcgagct gaccctttt 2640
tgtatggaaa atcatcagct gtgacaaaag gcatatgttg catgcactag ttgaagaaac 2700

<210> 205
<211> 843
<212> PRT
<213> Zea mays
<400> 205

Met Gly Thr Met Val Ala Ala Val Ala Leu Arg Gly Gly Ser Ser Asp
1               5                   10                  15
Ser Gly Gly Phe Asp Lys Val Pro Gly Met Asp Ser Gly Lys Tyr Val
            20                  25                  30
Arg Tyr Thr Pro Glu Gln Val Glu Val Leu Glu Arg Leu Tyr Ile Asp
        35                  40                  45
Cys Pro Lys Pro Ser Ser Ser Arg Arg Gln Gln Leu Leu Arg Glu Cys
    50                  55                  60
Pro Ile Leu Ser Asn Ile Glu Pro Lys Gln Ile Lys Val Trp Phe Gln
65                  70                  75                  80
Asn Arg Arg Cys Arg Asp Lys Gln Arg Lys Glu Ser Ser Arg Leu Gln
                85                  90                  95
Ala Val Asn Arg Lys Leu Thr Ala Met Asn Lys Leu Leu Met Glu Glu
            100                 105                 110
Asn Glu Arg Leu Gln Lys Gln Val Ser Gln Leu Val His Glu Asn Ala
        115                 120                 125
His Met Arg Gln Gln Leu Gln Asn Thr Ser Leu Ala Asn Asp Thr Ser
    130                 135                 140
Cys Glu Ser Asn Val Thr Thr Pro Pro Asn Pro Ile Arg Asp Ala Ser
145                 150                 155                 160
Asn Pro Ser Gly Leu Leu Ala Ile Ala Glu Glu Thr Phe Thr Glu Phe
                165                 170                 175
Leu Ser Lys Ala Thr Gly Thr Ala Ile Asp Trp Val Gln Met Pro Gly
            180                 185                 190
Met Lys Pro Gly Pro Asp Ser Val Gly Ile Val Ala Ile Ser His Gly

```
                195                     200                     205
Cys Arg Gly Val Ala Ala Arg Ala Cys Gly Leu Val Asn Leu Glu Pro
    210                     215                     220
Thr Lys Gly Ile Glu Ile Leu Lys Asp Arg Pro Ser Trp Phe Arg Asp
225                     230                     235                     240
Cys Arg Ser Leu Glu Val Phe Thr Arg Phe Pro Ala Gly Asn Gly Gly
                    245                     250                     255
Thr Ile Glu Leu Ile Tyr Met Gln Met Tyr Ala Pro Thr Thr Leu Val
                260                     265                     270
Pro Ala Arg Asp Phe Trp Thr Leu Arg Tyr Thr Thr Thr Met Glu Asp
            275                     280                     285
Gly Ser Leu Val Val Cys Glu Arg Ser Leu Ser Gly Ser Gly Gly Gly
    290                     295                     300
Pro Asn Ala Ala Ser Thr Gln Gln Phe Val Arg Ala Glu Met Leu Pro
305                     310                     315                     320
Ser Gly Tyr Leu Val Arg Pro Cys Glu Gly Gly Gly Ser Ile Val His
                325                     330                     335
Ile Val Asp His Leu Asp Leu Glu Ala Trp Ser Val Pro Glu Val Leu
            340                     345                     350
Arg Pro Leu Tyr Glu Ser Ser Arg Val Val Ala Gln Lys Met Thr Thr
        355                     360                     365
Val Ala Leu Arg His Leu Arg Gln Ile Ala Gln Glu Thr Ser Gly Glu
    370                     375                     380
Val Val Tyr Ala Leu Gly Arg Gln Pro Ala Val Leu Arg Thr Phe Ser
385                     390                     395                     400
Gln Arg Leu Ser Arg Gly Phe Asn Asp Ala Ile Ser Gly Phe Asn Asp
                405                     410                     415
Asp Gly Trp Ser Val Met Gly Gly Asp Gly Ile Glu Asp Val Val Ile
            420                     425                     430
Ala Cys Asn Ser Thr Lys Lys Ile Arg Asn Thr Ser Asn Ala Gly Ile
        435                     440                     445
Thr Phe Gly Ala Pro Gly Gly Ile Ile Cys Ala Lys Ala Ser Met Leu
    450                     455                     460
Leu Gln Ser Val Pro Pro Ala Val Leu Val Arg Phe Leu Arg Glu His
465                     470                     475                     480
Arg Ser Glu Trp Ala Asp Tyr Asn Ile Asp Ala Tyr Leu Ala Ser Ser
                485                     490                     495
Leu Lys Thr Ser Ala Cys Ser Leu Pro Gly Leu Arg Pro Met Arg Phe
            500                     505                     510
Ser Glu Gly Gln Met Ile Met Pro Leu Ala His Thr Val Glu Asn Glu
        515                     520                     525
Glu Ile Leu Glu Val Val Arg Leu Glu Gly Gln Pro Leu Thr His Asp
    530                     535                     540
Glu Ala Leu Leu Ser Arg Asp Ile His Leu Leu Gln Leu Cys Thr Gly
545                     550                     555                     560
Ile Asp Glu Lys Ser Val Gly Ser Ser Phe Gln Leu Val Phe Ala Pro
                565                     570                     575
Ile Asp Glu His Phe Pro Asp Asp Ala Pro Leu Ile Ser Ser Gly Phe
            580                     585                     590
Arg Val Ile Pro Leu Asp Val Lys Thr Asp Gly Val Ser Ser Gly Arg
        595                     600                     605
Thr Leu Asp Leu Ala Ser Ser Leu Asp Val Gly Ser Ala Ala Pro Gln
    610                     615                     620
Ala Ser Gly Asp Ala Ser Pro Asp Asp Cys Ser Leu Arg Ser Val Leu
625                     630                     635                     640
Thr Ile Ala Phe Gln Phe Pro Tyr Glu Met His Leu Gln Asp Ser Val
                645                     650                     655
Ala Ala Met Ala Arg Gln Tyr Val Arg Ser Val Ile Ser Ala Val Gln
            660                     665                     670
Arg Val Ser Met Ala Ile Ser Pro Ser Gln Ser Gly Leu Asn Ala Gly
        675                     680                     685
His Arg Met Leu Ser Gly Phe Pro Glu Ala Ala Thr Leu Ala Arg Trp
    690                     695                     700
Val Cys Gln Ser Tyr His Tyr His Leu Gly Met Glu Leu Leu Asn Gln
705                     710                     715                     720
Ser Asp Gly Ala Gly Glu Ala Leu Leu Lys Met Leu Trp His His Pro
                725                     730                     735
Asp Ala Val Leu Cys Cys Ser Phe Lys Glu Lys Pro Met Phe Thr Phe
            740                     745                     750
Ala Asn Lys Ala Gly Leu Asp Met Leu Glu Thr Ser Leu Val Ala Leu
        755                     760                     765
```

```
Gln Asp Leu Thr Leu Asp Lys Ile Phe Asp Glu Ser Gly Arg Lys Ala
    770                 775                 780
Leu Phe Ser Asp Ile Ser Lys Leu Met Glu Gln Gly Tyr Ala Tyr Leu
785                 790                 795                 800
Pro Ser Gly Val Cys Met Ser Gly Met Gly Arg His Val Ser Phe Asp
                805                 810                 815
Gln Ala Val Ala Trp Lys Val Leu Gly Glu Asp Ser Asn Ile His Cys
            820                 825                 830
Leu Ala Phe Cys Phe Val Asn Trp Ser Phe Val
        835                 840
```

<210> 206
<211> 2669
<212> DNA
<213> Populus trichocarpa
<400> 206

```
ctgtgtttga ttatttattt tgtggtggaa atggccatgg aagtggcccc tctgcaccga      60
gagagcagca gtagtgggag cataaacaag caccttactg atgatgggaa gtatgttcgg     120
tacacagcag agcaagtgga ggctcttgaa agagtttatg cggagtgccc taagcccagc     180
tctttgcgta ggcaacaatt gattagagag tgccccattt tagctaatat tgagccgaag     240
cagatcaaag tctggtttca aaatcgcagg tgtagagaga agcagagaaa agagtcctca     300
agactccaga ctgtgaacag gaaattgaca gcaatgaata agctgttgat ggaggagaat     360
gatcgccttc aaaaacaggt gtcccagctg gtgtgcgaaa atggtttcat gcggcaacaa     420
ctgcaaactg caccaacagc aactgatgca agctgtgact ctgtggttgc cactccacaa     480
cattcactaa gagatgccaa taaccctgct ggactcctct caatagctga ggagaccttg     540
tcagagttcc ttgcaaaggc cacaggaact gctcttgagt gggtccagat gcctggaatg     600
aagcctggtc cggattcgat tgggattttt tccatttcac aaaggtgcgg tggagtggca     660
gcaagagcct gcggtcttgt aagtttagag cctaaaaaga ttgcagagat ccttaaagat     720
cgttcatctt ggttccgtga ttgtcggaac cttgaagttt tcactatgtt tcctgctgga     780
aatggtggaa caattgaact agtgtacagt cagatatatg ctccaactac tcttgctcca     840
gcacgggata tgtggactct gagatacact acaagtttag agaatggcag ccttgtggtc     900
tgtgagagat cactttctgg ttatggtgct ggccccgatg cagctgccgc tgcccagttt     960
gtgagggctg aaatgcttcc tagtggctat ttgataaggc catgtgaagg agggtcaatt    1020
atccacatcg tggatcacct gaatcttcag gcatggagtg tgcctgaggt gcttcgacca    1080
ctttatgaat catcaaaagc agtggcacag aaaatgacca ttgcggcact gcgttatgtc    1140
aggcaagtag ctcatgaaac cagtggtgag gtggtgtatg gtttgggcag gcaaccagct    1200
gttctgagaa cctttaacca aagattaagc agaggtttca atgatgccat caatgggttt    1260
aatgatgatg gctggtcact gatgaatgcg gatggagctg aggatgtaat aattgcagtt    1320
aactcaacca agaatttgat tggtgctaat aattctgctc attctctttc gttcttggga    1380
gggattcttt gtgcgaaagc ttccatgcta ctgcaaaatg tgcatcctgc tgtgctggtc    1440
tgtttcctaa gggagcatca cgctgagtgg gctgatttca gtgttgatgc ctattctgct    1500
gcattgtgga aggctggttc atatgcatat ccaggaatga ggcccatgag gtttactggg    1560
agccaaatca ccatgccact gggccacaca attgaacaag aagacttgct tgaagttatt    1620
cgacttgaag gccattcttt tgcacaagaa gatgcttttg tttcacagga catccatctc    1680
ctacagatat gtagtggaat tgatgagaat gctgttggag cctgttctga actagttttt    1740
gctccaattg atgaaacgtt tccagatgat gctccactgc taccttctgg tttccgcatc    1800
atttctctgg aatcaaaagc aaaggataca caggaggtat tgactacaaa ttgtactctg    1860
gatctaacat caagtcttga agcggggctg gcaataaacc acactgcagt ggatggctca    1920
tcgtgtcata gtttgcgatc agtgttgact attgccttcc agttcccttt tgagagcaat    1980
ctacaggata atgttgccac catggcacgt cagtacgtac gtagtgtgat ttcatctgtc    2040
cagagggttg ccatggccat atctccatca ggattgagtc cagttttggg accaaagcta    2100
tctgcaggat ctcctgaagc tctaaccttg gctcactgga tctgccaaag tcacaggcaa    2160
gttctgctta agttttcatc atgttaccat ttaggagcag aattactgag atctgattct    2220
gtgggtggag attctgtcct gaaacatcta tggcatcatc cagatgcaat tttatgttgt    2280
tcattgaagt cgctgccagt tttcatcttt gccaatcagg cagggcttga catgctggag    2340
acaactctag tggctctaca agatattaca ctggataaga tattcaatga atctggacgc    2400
caggcattgt atacagaatt tgcaaagtta atgcaacagg gatttgcatg cttgcctgct    2460
ggaatctgca tgtcaacaat ggggcgtaat gtttcatatg agcaagctgt tgcttggaaa    2520
gtgctatctg cagaagagaa cgctgttcat tgcattgctt tctcttttgt aaactggtct    2580
tttttgtgaa ctccacagtt catttatcca actatgcagt cgaatacgag aacaacggta    2640
tggtagagat ttttgaaaat gaaaagaga                                     2669
```

<210> 207
<211> 852
<212> PRT
<213> Populus trichocarpa
<400> 207

```
Met Ala Met Glu Val Ala Pro Leu His Arg Glu Ser Ser Ser Ser Gly
1               5                   10                  15
Ser Ile Asn Lys His Leu Thr Asp Asp Gly Lys Tyr Val Arg Tyr Thr
            20                  25                  30
Ala Glu Gln Val Glu Ala Leu Glu Arg Val Tyr Ala Glu Cys Pro Lys
        35                  40                  45
```

Pro Ser Ser Leu Arg Arg Gln Gln Leu Ile Arg Glu Cys Pro Ile Leu
50 55 60
Ala Asn Ile Glu Pro Lys Gln Ile Lys Val Trp Phe Gln Asn Arg Arg
65 70 75 80
Cys Arg Glu Lys Gln Arg Lys Glu Ser Ser Arg Leu Gln Thr Val Asn
85 90 95
Arg Lys Leu Thr Ala Met Asn Lys Leu Leu Met Glu Glu Asn Asp Arg
100 105 110
Leu Gln Lys Gln Val Ser Gln Leu Val Cys Glu Asn Gly Phe Met Arg
115 120 125
Gln Gln Leu Gln Thr Ala Pro Thr Ala Thr Asp Ala Ser Cys Asp Ser
130 135 140
Val Val Ala Thr Pro Gln His Ser Leu Arg Asp Ala Asn Asn Pro Ala
145 150 155 160
Gly Leu Leu Ser Ile Ala Glu Glu Thr Leu Ser Glu Phe Leu Ala Lys
165 170 175
Ala Thr Gly Thr Ala Leu Glu Trp Val Gln Met Pro Gly Met Lys Pro
180 185 190
Gly Pro Asp Ser Ile Gly Ile Phe Ser Ile Ser Gln Arg Cys Gly Gly
195 200 205
Val Ala Ala Arg Ala Cys Gly Leu Val Ser Leu Glu Pro Lys Lys Ile
210 215 220
Ala Glu Ile Leu Lys Asp Arg Ser Ser Trp Phe Arg Asp Cys Arg Asn
225 230 235 240
Leu Glu Val Phe Thr Met Phe Pro Ala Gly Asn Gly Gly Thr Ile Glu
245 250 255
Leu Val Tyr Ser Gln Ile Tyr Ala Pro Thr Thr Leu Ala Pro Ala Arg
260 265 270
Asp Met Trp Thr Leu Arg Tyr Thr Thr Ser Leu Glu Asn Gly Ser Leu
275 280 285
Val Val Cys Glu Arg Ser Leu Ser Gly Tyr Gly Ala Gly Pro Asp Ala
290 295 300
Ala Ala Ala Ala Gln Phe Val Arg Ala Glu Met Leu Pro Ser Gly Tyr
305 310 315 320
Leu Ile Arg Pro Cys Glu Gly Gly Ser Ile Ile His Ile Val Asp His
325 330 335
Leu Asn Leu Gln Ala Trp Ser Val Pro Glu Val Leu Arg Pro Leu Tyr
340 345 350
Glu Ser Ser Lys Ala Val Ala Gln Lys Met Thr Ile Ala Ala Leu Arg
355 360 365
Tyr Val Arg Gln Val Ala His Glu Thr Ser Gly Glu Val Val Tyr Gly
370 375 380
Leu Gly Arg Gln Pro Ala Val Leu Arg Thr Phe Asn Gln Arg Leu Ser
385 390 395 400
Arg Gly Phe Asn Asp Ala Ile Asn Gly Phe Asn Asp Asp Gly Trp Ser
405 410 415
Leu Met Asn Ala Asp Gly Ala Glu Asp Val Ile Ile Ala Val Asn Ser
420 425 430
Thr Lys Asn Leu Ile Gly Ala Asn Asn Ser Ala His Ser Leu Ser Phe
435 440 445
Leu Gly Gly Ile Leu Cys Ala Lys Ala Ser Met Leu Leu Gln Asn Val
450 455 460
His Pro Ala Val Leu Val Cys Phe Leu Arg Glu His His Ala Glu Trp
465 470 475 480
Ala Asp Phe Ser Val Asp Ala Tyr Ser Ala Ala Leu Trp Lys Ala Gly
485 490 495
Ser Tyr Ala Tyr Pro Gly Met Arg Pro Met Arg Phe Thr Gly Ser Gln
500 505 510
Ile Thr Met Pro Leu Gly His Thr Ile Glu Gln Glu Asp Leu Leu Glu
515 520 525
Val Ile Arg Leu Glu Gly His Ser Phe Ala Gln Glu Asp Ala Phe Val
530 535 540
Ser Gln Asp Ile His Leu Leu Gln Ile Cys Ser Gly Ile Asp Glu Asn
545 550 555 560
Ala Val Gly Ala Cys Ser Glu Leu Val Phe Ala Pro Ile Asp Glu Thr
565 570 575
Phe Pro Asp Asp Ala Pro Leu Leu Pro Ser Gly Phe Arg Ile Ile Ser
580 585 590
Leu Glu Ser Lys Ala Lys Asp Thr Gln Glu Val Leu Thr Thr Asn Cys
595 600 605
Thr Leu Asp Leu Thr Ser Ser Leu Glu Ala Gly Leu Ala Ile Asn His

```
    610                 615                 620
Thr Ala Val Asp Gly Ser Ser Cys His Ser Leu Arg Ser Val Leu Thr
625                 630                 635                 640
Ile Ala Phe Gln Phe Pro Phe Glu Ser Asn Leu Gln Asp Asn Val Ala
                645                 650                 655
Thr Met Ala Arg Gln Tyr Val Arg Ser Val Ile Ser Ser Val Gln Arg
            660                 665                 670
Val Ala Met Ala Ile Ser Pro Ser Gly Leu Ser Pro Val Leu Gly Pro
        675                 680                 685
Lys Leu Ser Ala Gly Ser Pro Glu Ala Leu Thr Leu Ala His Trp Ile
    690                 695                 700
Cys Gln Ser His Arg Gln Val Leu Leu Lys Phe Ser Ser Cys Tyr His
705                 710                 715                 720
Leu Gly Ala Glu Leu Leu Arg Ser Asp Ser Val Gly Gly Asp Ser Val
                725                 730                 735
Leu Lys His Leu Trp His His Pro Asp Ala Ile Leu Cys Cys Ser Leu
            740                 745                 750
Lys Ser Leu Pro Val Phe Ile Phe Ala Asn Gln Ala Gly Leu Asp Met
        755                 760                 765
Leu Glu Thr Thr Leu Val Ala Leu Gln Asp Ile Thr Leu Asp Lys Ile
    770                 775                 780
Phe Asn Glu Ser Gly Arg Gln Ala Leu Tyr Thr Glu Phe Ala Lys Leu
785                 790                 795                 800
Met Gln Gln Gly Phe Ala Cys Leu Pro Ala Gly Ile Cys Met Ser Thr
                805                 810                 815
Met Gly Arg Asn Val Ser Tyr Glu Gln Ala Val Ala Trp Lys Val Leu
            820                 825                 830
Ser Ala Glu Glu Asn Ala Val His Cys Ile Ala Phe Ser Phe Val Asn
        835                 840                 845
Trp Ser Phe Leu
    850
```

<210> 208
<211> 2634
<212> DNA
<213> Medicago truncuLata
<400> 208

```
aggtgtgat tgtttaagtt aatttgagat taggaagatg gctatggctg ttgcacaaca 60
acaaagagat aacagcattg agagacacct tgattcgtct ggcaaatatg tgaggtacac 120
tgctgaacag attgaagctt tggaaaaggt ttatgtggaa tgccctaagc ctagttcatt 180
gagaaggcaa cagctgattc gggagtgccc ggttctggcc aacattgagc ctaagcagat 240
caaggtttgg tttcagaata ggaggtgtag ggagaagcag agaaaagagg cttctcagct 300
tcagagtgtg aacaggaaac tttctgcgat gaataagctg ttgatggagg aaaatgagag 360
gctgcagaag caggtttcac agctggtgaa tgagaatgga tttatgcgcc agcaactaca 420
ccctacccca gcagctccaa atgctgacgg tagtggcgtt gattccgcgg ctgctgctcc 480
tatgaactca ttgagagatg ctaatagccc tgctggattc ctatcaattg cggaggagac 540
attgacagag ttcctttcaa aggctacagg aactgctgtc gattgggtcc agatgcctgg 600
gatgaagcct ggtccggatt cggttgggat atttgccatt tctcaaggtg gcaacggagt 660
ggcagctcga gcctgtggtc ttgttagttt agaacctact aagattgtgg agatattaaa 720
agatcgccca acttggtacc gtgattgtcg gagttcagaa gttttcacaa tgttcccagc 780
tggaaatgga ggaacaattg aacttgttta cacacagaca tatgctccaa tgacactggc 840
ttctgctcgc gacttctgga ctctaagata cactacaaat ttggaaaacg gaagtgttgt 900
ggtttgtgaa aggtcactgt ctggtactgg tgctggccct aatgctgcag ccgcctcaca 960
gtttgagagg gctgaaatgc tccctagtgg ctatttgatt cgaccatgtg aaggtggagg 1020
atcgatcatc cacattgtag accacctaaa cctgcaggca tggagtgtgc cagaagtgct 1080
gcggccgatc tatgaatcgt cgcaaatggt agctcagaga ctgacaattg cggcacttcg 1140
ctatatcagg caagtagctc aagaaacaag tggtgacgtg gtgtatagca tgggtcggca 1200
acctgcagtt cttagaactt ttagccaacg gttgagcaga ggtttcaatg acgctgtcaa 1260
tggattcaat gataatggtt ggtctgttct gaactgtgat ggtgctgagg gtgttactat 1320
ttcagtaaat tcaatcaaga atttgagtgg cacttctaat ccagcaagtt ccctttcact 1380
ccttggagga attgtctgtg caaaagcttc tatgttactc caaaacacca ctcctgctgt 1440
tttagttcgc tttctgaggg agcatcgctc ggagtgggct gattttagtg ttgatgcctt 1500
ttctgctgca tcacttaaag ctggctccta tggctatcct ggaatgaggt ctacaaagtt 1560
caccggcaat caagcaatca tgcctcttgg acatacaatt gaacatgaag agatgctaga 1620
aattatccgc cttgaaggtc ttgctcaaga tgattctttt gtttctaggg atgttcatct 1680
cttacaggtg cttcctctga cccttgttat gttatgtact ggaattgatg agaatgctgt 1740
gggggcttgt tccgagctca tatttgctcc aattgatgac atgttcccag aagatgctcc 1800
cttagtgcct tctggtttcc gcattgtcct gttgaattct caaccaggtg atacaaagaa 1860
cacaacaaca gcaaatcgaa ccttggattt gacatctggt cttgaagtaa gcccggcaac 1920
agctcatgct aacggagacg catcgtgtcc taacaatcga tgtgtgttga ctgttgcctt 1980
tcagtttcct tttgagagcg gtctgcagga taatgttgca gccatggcac gtcaatatgt 2040
ccggcgtgta gtttctgccg tgcaggcggt tgcaacggct atatctccat ccagtgttaa 2100
```

```
cacttctggt ggagcaaagc tctcccctgg cactccagaa gcacttacac tagctcaatg   2160
gatctgccag agttatagtc atcatctggg cgcgcaactg ctgagatctg attctcttat   2220
tggtgatatg ctactgaaac atttgtggca tcatccagat gctattttat gctgctcttt   2280
gaagcaagtg cccgtattca tctttgctaa ccaggctggc cttgacatgt tggaaacaac   2340
tctagtggct ctacaagata tcacactgga caaaatattt gatgagtctg cacgcaagaa   2400
tttgattgca tattttgcga agttaatgca gcaggggttt gcttgtatgc cagctgggat   2460
ctgcatgtca acaatggggc gacatgcttc atatgatcaa gccgtcgcgt ggaaagtgca   2520
tgctgaagac aacagtgttc attgcttggc tttctcattc attaattggt catttatatg   2580
acctattgct ggatttaaac ccccactttt ttttcccact tgttgaatac aaaa         2634
<210>  209
<211>  847
<212>  PRT
<213>  Medicago truncul
<400>  209
Met Ala Met Ala Val Ala Gln Gln Gln Arg Asp Asn Ser Ile Glu Arg
1               5                   10                  15
His Leu Asp Ser Ser Gly Lys Tyr Val Arg Tyr Thr Ala Glu Gln Ile
            20                  25                  30
Glu Ala Leu Glu Lys Val Tyr Val Glu Cys Pro Lys Pro Ser Ser Leu
        35                  40                  45
Arg Arg Gln Gln Leu Ile Arg Glu Cys Pro Val Leu Ala Asn Ile Glu
    50                  55                  60
Pro Lys Gln Ile Lys Val Trp Phe Gln Asn Arg Arg Cys Arg Glu Lys
65                  70                  75                  80
Gln Arg Lys Glu Ala Ser Gln Leu Gln Ser Val Asn Arg Lys Leu Ser
                85                  90                  95
Ala Met Asn Lys Leu Leu Met Glu Glu Asn Glu Arg Leu Gln Lys Gln
            100                 105                 110
Val Ser Gln Leu Val Asn Glu Asn Gly Phe Met Arg Gln Gln Leu His
        115                 120                 125
Pro Thr Pro Ala Ala Pro Asn Ala Asp Gly Ser Gly Val Asp Ser Ala
    130                 135                 140
Ala Ala Ala Pro Met Asn Ser Leu Arg Asp Ala Asn Ser Pro Ala Gly
145                 150                 155                 160
Phe Leu Ser Ile Ala Glu Glu Thr Leu Thr Glu Phe Leu Ser Lys Ala
                165                 170                 175
Thr Gly Thr Ala Val Asp Trp Val Gln Met Pro Gly Met Lys Pro Gly
            180                 185                 190
Pro Asp Ser Val Gly Ile Phe Ala Ile Ser Gln Gly Gly Asn Gly Val
        195                 200                 205
Ala Ala Arg Ala Cys Gly Leu Val Ser Leu Glu Pro Thr Lys Ile Val
    210                 215                 220
Glu Ile Leu Lys Asp Arg Pro Thr Trp Tyr Arg Asp Cys Arg Ser Ser
225                 230                 235                 240
Glu Val Phe Thr Met Phe Pro Ala Gly Asn Gly Gly Thr Ile Glu Leu
                245                 250                 255
Val Tyr Thr Gln Thr Tyr Ala Pro Met Thr Leu Ala Ser Ala Arg Asp
            260                 265                 270
Phe Trp Thr Leu Arg Tyr Thr Thr Asn Leu Glu Asn Gly Ser Val Val
        275                 280                 285
Val Cys Glu Arg Ser Leu Ser Gly Thr Gly Ala Gly Pro Asn Ala Ala
    290                 295                 300
Ala Ala Ser Gln Phe Glu Arg Ala Glu Met Leu Pro Ser Gly Tyr Leu
305                 310                 315                 320
Ile Arg Pro Cys Glu Gly Gly Gly Ser Ile Ile His Ile Val Asp His
                325                 330                 335
Leu Asn Leu Gln Ala Trp Ser Val Pro Glu Val Leu Arg Pro Ile Tyr
            340                 345                 350
Glu Ser Ser Gln Met Val Ala Gln Arg Leu Thr Ile Ala Ala Leu Arg
        355                 360                 365
Tyr Ile Arg Gln Val Ala Gln Glu Thr Ser Gly Asp Val Val Tyr Ser
    370                 375                 380
Met Gly Arg Gln Pro Ala Val Leu Arg Thr Phe Ser Gln Arg Leu Ser
385                 390                 395                 400
Arg Gly Phe Asn Asp Ala Val Asn Gly Phe Asn Asp Asn Gly Trp Ser
                405                 410                 415
Val Leu Asn Cys Asp Gly Ala Glu Gly Val Thr Ile Ser Val Asn Ser
            420                 425                 430
Ile Lys Asn Leu Ser Gly Thr Ser Asn Pro Ala Ser Ser Leu Ser Leu
        435                 440                 445
Leu Gly Gly Ile Val Cys Ala Lys Ala Ser Met Leu Leu Gln Asn Thr
```

```
    450                 455                 460
Thr Pro Ala Val Leu Val Arg Phe Leu Arg Glu His Arg Ser Glu Trp
465                 470                 475                 480
Ala Asp Phe Ser Val Asp Ala Phe Ser Ala Ala Ser Leu Lys Ala Gly
                485                 490                 495
Ser Tyr Gly Tyr Pro Gly Met Arg Ser Thr Lys Phe Thr Gly Asn Gln
            500                 505                 510
Ala Ile Met Pro Leu Gly His Thr Ile Glu His Glu Glu Met Leu Glu
        515                 520                 525
Ile Ile Arg Leu Glu Gly Leu Ala Gln Asp Asp Ser Phe Val Ser Arg
    530                 535                 540
Asp Val His Leu Leu Gln Val Leu Pro Leu Thr Leu Val Met Leu Cys
545                 550                 555                 560
Thr Gly Ile Asp Glu Asn Ala Val Gly Ala Cys Ser Glu Leu Ile Phe
                565                 570                 575
Ala Pro Ile Asp Asp Met Phe Pro Glu Asp Ala Pro Leu Val Pro Ser
            580                 585                 590
Gly Phe Arg Ile Val Leu Leu Asn Ser Gln Pro Gly Asp Thr Lys Asn
        595                 600                 605
Thr Thr Thr Ala Asn Arg Thr Leu Asp Leu Thr Ser Gly Leu Glu Val
    610                 615                 620
Ser Pro Ala Thr Ala His Ala Asn Gly Asp Ala Ser Cys Pro Asn Asn
625                 630                 635                 640
Arg Cys Val Leu Thr Val Ala Phe Gln Phe Pro Phe Glu Ser Gly Leu
                645                 650                 655
Gln Asp Asn Val Ala Ala Met Ala Arg Gln Tyr Val Arg Arg Val Val
            660                 665                 670
Ser Ala Val Gln Ala Val Ala Thr Ala Ile Ser Pro Ser Ser Val Asn
        675                 680                 685
Thr Ser Gly Gly Ala Lys Leu Ser Pro Gly Thr Pro Glu Ala Leu Thr
    690                 695                 700
Leu Ala Gln Trp Ile Cys Gln Ser Tyr Ser His His Leu Gly Ala Gln
705                 710                 715                 720
Leu Leu Arg Ser Asp Ser Leu Ile Gly Asp Met Leu Leu Lys His Leu
                725                 730                 735
Trp His His Pro Asp Ala Ile Leu Cys Cys Ser Leu Lys Gln Val Pro
            740                 745                 750
Val Phe Ile Phe Ala Asn Gln Ala Gly Leu Asp Met Leu Glu Thr Thr
        755                 760                 765
Leu Val Ala Leu Gln Asp Ile Thr Leu Asp Lys Ile Phe Asp Glu Ser
    770                 775                 780
Ala Arg Lys Asn Leu Ile Ala Tyr Phe Ala Lys Leu Met Gln Gln Gly
785                 790                 795                 800
Phe Ala Cys Met Pro Ala Gly Ile Cys Met Ser Thr Met Gly Arg His
                805                 810                 815
Ala Ser Tyr Asp Gln Ala Val Ala Trp Lys Val His Ala Glu Asp Asn
            820                 825                 830
Ser Val His Cys Leu Ala Phe Ser Phe Ile Asn Trp Ser Phe Ile
        835                 840                 845
<210>  210
<211>  2211
<212>  DNA
<213>  Saccharum officinarum
<220>
<221>  misc_feature
<222>  (926)..(926)
<223>  n is a, c, g, or t
<220>
<221>  misc_feature
<222>  (944)..(944)
<223>  n is a, c, g, or t
<400>  210
gattcggttg gtatcgtggc catttcgcat ggttgccgtg gtgttgctgc ccgcgcctgt      60
ggtttggtga atctagaacc aacaagagtc atagagatct tgaaagatcg tccctcttgg     120
ttccgtgatt gtcgaagtct tgaagtgttt acaatgtttc cagctggaaa tgggggaaca     180
gttgaactta tctacatgca gatgtatgcc ccaacaactt tagtccctgc acgtgatttt     240
tggacgttac gatacacgac aacaatggaa gatggtagcc ttgtggtctg tgagagatcc     300
ttgagtggtt ctggaggcgg tccaaatgca gcctctgcac agcaatttgt tagggctgaa     360
atgcttccaa gtgggtattt agttcgccca tgcgaaggtg gaggttcgat tgtgcatata     420
gtggaccatc tagatctcga ggcatggagt gttcctgaag tgcttcgacc gctgtatgag     480
tcttccagag tagttgctca gaaaatgact acggtggcac tgcgccacct tagacaaatt     540
```

```
gctcaagaaa caagtggaga agtagtgtac gccttgggaa ggcaacctgc agtactacgg    600
acctttagtc aaagactaag caggggtttt aatgatgcca ttagtggttt caatgatgat    660
ggctggtctg taatgggggg agatggcatt gaagacgttg ctgttgcttg cacctcaacc    720
aagaaaatta ggaataacag caatgcgggg attacatttg gagcccctgg aggcattatt    780
tgtgcgaagg catccatgtt actgcagagt gtcccaccgg cagtactggt ccgatttctg    840
agggagcata gatctgaatg ggctgattac aatattgatg cgtatttggc ttcatcactg    900
aagaccagtg catgctcact tccggngttg caacctatga gatnttctgg ggggcagatg    960
atcatgccac ttgctcacac agtggagaat gaggagattc ttgaagttat ccgccttgaa   1020
ggacatcctc ttactcatga tgaagctctt ctttcaagag acatccacct tctccagctt   1080
tgcactggaa tagatgagaa atctgtgggt tcctccttcc agcttgtgtt tgcaccaatt   1140
gatgagcact ttccagatga tgctccattg atttcttctg gctttcgtgt cataccactt   1200
gatatgaaaa cagatggtgt atcctctggc aggacgctag atttggcatc tagtcttgat   1260
gtgggttctg ctgcacccca agcctcaggg gatgcatctc cagatgactg taatttgaga   1320
tctgtgctga caatcgcctt tcaattccct tacgagatgc accttcagga cagtgttgca   1380
actatggctc gtcaatatgt tcgtagtgtt gtttctgctg tgcaaagagt gtcgatggct   1440
atatctccct cccaatctgg tctaaatgct cagaggacac tttctggctt ccctgaaact   1500
gccacacttg ctagatgggt ttgccagagt tatcactacc atctaggcgt ggaattactt   1560
aatcaatcag atgaagctgg cgaagcattg ttgaaaatgc tctggcatca tccagacgct   1620
gttctgtgct gctcttttaa ggagaaacct atgtttacgt ttgcaaacaa ggcagggctt   1680
gacatgttag aaacatctct tattgccctg caagacctga cactagacaa gattttcgac   1740
gagtcaggaa ggaaagcaat attctcagat atctcaaaac taatggaaca gggctacgca   1800
tacctgccgt caggtgtgtg catgtcagga atgggtcgcc atgtttcttt cgaccaagct   1860
gtggcgtgga aggtgctcgg tgaggacagc aacgtgcact gcctggcttt ctgcttcgtc   1920
aactggtcct tcgtgtaacg cccacccatc cgatggggtg gcgagcctgt cggtctgtgt   1980
gatgagccag cccaattttg tatgatgatc ctgataagga aatcattgac ccgggcaaaa   2040
tgcttatggt gcatgcacta ttttgaggcc ctgaaatccc gggttttatt aaaaaaactg   2100
gagaattttc ccaggttttt tcccactttt cgttggcccc cccacccaca gggtgtttgt   2160
acaatttctt tggcgagggg caccccactc ctgcagtttt tttttccata c             2211
```

<210> 211
<211> 645
<212> PRT
<213> Saccharum officinarum
<220>
<221> UNSURE
<222> (309)..(309)
<223> Xaa can be any naturally occurring amino acid
<220>
<221> UNSURE
<222> (315)..(315)
<223> Xaa can be any naturally occurring amino acid
<400> 211

```
Asp Ser Val Gly Ile Val Ala Ile Ser His Gly Cys Arg Gly Val Ala
1               5                   10                  15
Ala Arg Ala Cys Gly Leu Val Asn Leu Glu Pro Thr Arg Val Ile Glu
            20                  25                  30
Ile Leu Lys Asp Arg Pro Ser Trp Phe Arg Asp Cys Arg Ser Leu Glu
        35                  40                  45
Val Phe Thr Met Phe Pro Ala Gly Asn Gly Gly Thr Val Glu Leu Ile
    50                  55                  60
Tyr Met Gln Met Tyr Ala Pro Thr Thr Leu Val Pro Ala Arg Asp Phe
65                  70                  75                  80
Trp Thr Leu Arg Tyr Thr Thr Thr Met Glu Asp Gly Ser Leu Val Val
                85                  90                  95
Cys Glu Arg Ser Leu Ser Gly Ser Gly Gly Gly Pro Asn Ala Ala Ser
            100                 105                 110
Ala Gln Gln Phe Val Arg Ala Glu Met Leu Pro Ser Gly Tyr Leu Val
        115                 120                 125
Arg Pro Cys Glu Gly Gly Gly Ser Ile Val His Ile Val Asp His Leu
    130                 135                 140
Asp Leu Glu Ala Trp Ser Val Pro Glu Val Leu Arg Pro Leu Tyr Glu
145                 150                 155                 160
Ser Ser Arg Val Val Ala Gln Lys Met Thr Thr Val Ala Leu Arg His
                165                 170                 175
Leu Arg Gln Ile Ala Gln Glu Thr Ser Gly Glu Val Val Tyr Ala Leu
            180                 185                 190
Gly Arg Gln Pro Ala Val Leu Arg Thr Phe Ser Gln Arg Leu Ser Arg
        195                 200                 205
Gly Phe Asn Asp Ala Ile Ser Gly Phe Asn Asp Asp Gly Trp Ser Val
    210                 215                 220
Met Gly Gly Asp Gly Ile Glu Asp Val Ala Val Ala Cys Thr Ser Thr
225                 230                 235                 240
```

```
Lys Lys Ile Arg Asn Asn Ser Asn Ala Gly Ile Thr Phe Gly Ala Pro
                245                 250                 255
Gly Gly Ile Ile Cys Ala Lys Ala Ser Met Leu Leu Gln Ser Val Pro
            260                 265                 270
Pro Ala Val Leu Val Arg Phe Leu Arg Glu His Arg Ser Glu Trp Ala
        275                 280                 285
Asp Tyr Asn Ile Asp Ala Tyr Leu Ala Ser Ser Leu Lys Thr Ser Ala
    290                 295                 300
Cys Ser Leu Pro Xaa Leu Gln Pro Met Arg Xaa Ser Gly Gly Gln Met
305                 310                 315                 320
Ile Met Pro Leu Ala His Thr Val Glu Asn Glu Glu Ile Leu Glu Val
                325                 330                 335
Ile Arg Leu Glu Gly His Pro Leu Thr His Asp Glu Ala Leu Leu Ser
            340                 345                 350
Arg Asp Ile His Leu Leu Gln Leu Cys Thr Gly Ile Asp Glu Lys Ser
        355                 360                 365
Val Gly Ser Ser Phe Gln Leu Val Phe Ala Pro Ile Asp Glu His Phe
    370                 375                 380
Pro Asp Asp Ala Pro Leu Ile Ser Ser Gly Phe Arg Val Ile Pro Leu
385                 390                 395                 400
Asp Met Lys Thr Asp Gly Val Ser Ser Gly Arg Thr Leu Asp Leu Ala
                405                 410                 415
Ser Ser Leu Asp Val Gly Ser Ala Ala Pro Gln Ala Ser Gly Asp Ala
            420                 425                 430
Ser Pro Asp Asp Cys Asn Leu Arg Ser Val Leu Thr Ile Ala Phe Gln
        435                 440                 445
Phe Pro Tyr Glu Met His Leu Gln Asp Ser Val Ala Thr Met Ala Arg
    450                 455                 460
Gln Tyr Val Arg Ser Val Val Ser Ala Val Gln Arg Val Ser Met Ala
465                 470                 475                 480
Ile Ser Pro Ser Gln Ser Gly Leu Asn Ala Gln Arg Thr Leu Ser Gly
                485                 490                 495
Phe Pro Glu Thr Ala Thr Leu Ala Arg Trp Val Cys Gln Ser Tyr His
            500                 505                 510
Tyr His Leu Gly Val Glu Leu Leu Asn Gln Ser Asp Glu Ala Gly Glu
        515                 520                 525
Ala Leu Leu Lys Met Leu Trp His His Pro Asp Ala Val Leu Cys Cys
    530                 535                 540
Ser Phe Lys Glu Lys Pro Met Phe Thr Phe Ala Asn Lys Ala Gly Leu
545                 550                 555                 560
Asp Met Leu Glu Thr Ser Leu Ile Ala Leu Gln Asp Leu Thr Leu Asp
                565                 570                 575
Lys Ile Phe Asp Glu Ser Gly Arg Lys Ala Ile Phe Ser Asp Ile Ser
            580                 585                 590
Lys Leu Met Glu Gln Gly Tyr Ala Tyr Leu Pro Ser Gly Val Cys Met
        595                 600                 605
Ser Gly Met Gly Arg His Val Ser Phe Asp Gln Ala Val Ala Trp Lys
    610                 615                 620
Val Leu Gly Glu Asp Ser Asn Val His Cys Leu Ala Phe Cys Phe Val
625                 630                 635                 640
Asn Trp Ser Phe Val
                645
```

<210> 212
<211> 2076
<212> DNA
<213> Triticum aestivum
<400> 212

```
agggtattgt tgccgtttca catggttgcc gaggtgtcgc tgcccgtgcc tgtggtctgg      60
tgaatctaca accaacaaag attgtggaga tcttgaaaga ccgcccatct tggttccgtg     120
attgtcggag tcttgaattt tttacgatgc ttccagctgg aaacggcggg accattgaac     180
tcgtttacat gcagatgtat gctcctacca ctttagttcc tgcacgtgat ttttggacgc     240
tgagatacac aactacaatg gaagatggaa gtctcgtggt ttgtgagaga tctttgagtg     300
gttcaggagg tggtccaagt actgcctcag cacagcaatt tgtaagggct gagatgcttc     360
ctagtggcta tttagttcgg ccatgcgacg gtgggggttc aattgtgcat atagtggatc     420
atctggacct tgaggcttgg agtgttccag aagtgcttcg cccgctctat gagtcttcta     480
gggtagttgc tcagaaaatg actactgcgg cattacgaca catcagacag attgctcaag     540
aaactagtgg ggaggttgta tatgctctag ggaggcaacc tgctgttctg cggacattta     600
gtcagaggct gagtagagga tttaatgatg ctataagtgg cttcaatgat gatggttggt     660
ctgtaatggc tggagacggc attgaagatg tgattattgc ttgcaactca aaaaagatta     720
gaagcaataa cactgctccc aatgcgttta tagctcctgg aggtgttata tgtgctaaag     780
catcaatgtt actgcagagt gttccaccag cagtactggt tcggtttctg agggaacatc     840
```

ggtctgaatg ggctgattat aactttgatg catattcggc ttcagcgctg aaatcaagct 900
catgctcact tcctgggttg cgtcccatga gattttctgg gagccagatc atcatgccac 960
ttgctcacac agtggagaat gaggagattc tagaagttgt tcgtcttgaa gggcaagcgc 1020
tcgatgaggg tcttttatca agagatatcc acctgcttca gttttgcact ggactagacg 1080
agaaatcaat gggatcctgc ttccaacttg tctttgcacc aattgatgag cttttccctg 1140
atgatgctcc attaatatct tcaggctttc gtgttatacc actggacatg aaaacagatg 1200
gtgcacccgc cggtagaaca ctagatttgg cctctagcct tgaagctggt tcaactacac 1260
tgcaagcctc aggcggtgcg gatgattgta atctacgatc agtgctgaca attgcctttc 1320
aattccctta cgagatgcat ctccaagata gtgttgcaac tatggcccgg cagtatgtcc 1380
gcagcattgt ctccgccgtt cagagagtgt cgatggctat ctctccctct cgatctggct 1440
tgaatgctga acagaagata atttctggct tccctgaagc tgcaacacta gctcgctgga 1500
tatgccaaag ttaccggttc catctggggg tcgagttatt tagacaggca gatgaagctg 1560
gggaatcttt attgagaatg ctctgggatc atgaagatgc tattttgtgc tgttctttca 1620
aggaaaagcc tgtatttacg tttgcaaacg agatgggaat taacatgttg gaaacatctt 1680
tcgttgccct tcaagatctc tcgttggaca agatatttga tgaagctggt agaaaggcac 1740
tatactccga gatcccaaag ttgatggagc agggctttgt ttacctgcca gggggtgtgt 1800
gcttgtctgg gatgggccgc catgtctcat ttgagagtgc tgtagcatgg aaagtagttg 1860
gtgaggacaa caatgtgcac tgcctcgcct tctgcttcgt caactggtct ttcgtgtgat 1920
catccatcac ccttgcctgt cccatgcagc tccatttttg ctctctctgt aggggagaac 1980
cgccgccact ggaaagtagt tttacgttat ctttaactag tttgtttcta gatttgaaga 2040
gccagcatcg ggaagaattc tgcctagtga aaattg 2076

<210> 213
<211> 638
<212> PRT
<213> Triticum aestivum
<400> 213

Gly Ile Val Ala Val Ser His Gly Cys Arg Gly Val Ala Ala Arg Ala
1               5                   10                  15
Cys Gly Leu Val Asn Leu Gln Pro Thr Lys Ile Val Glu Ile Leu Lys
            20                  25                  30
Asp Arg Pro Ser Trp Phe Arg Asp Cys Arg Ser Leu Glu Phe Phe Thr
        35                  40                  45
Met Leu Pro Ala Gly Asn Gly Gly Thr Ile Glu Leu Val Tyr Met Gln
    50                  55                  60
Met Tyr Ala Pro Thr Thr Leu Val Pro Ala Arg Asp Phe Trp Thr Leu
65                  70                  75                  80
Arg Tyr Thr Thr Thr Met Glu Asp Gly Ser Leu Val Val Cys Glu Arg
                85                  90                  95
Ser Leu Ser Gly Ser Gly Gly Gly Pro Ser Thr Ala Ser Ala Gln Gln
            100                 105                 110
Phe Val Arg Ala Glu Met Leu Pro Ser Gly Tyr Leu Val Arg Pro Cys
        115                 120                 125
Asp Gly Gly Gly Ser Ile Val His Ile Val Asp His Leu Asp Leu Glu
    130                 135                 140
Ala Trp Ser Val Pro Glu Val Leu Arg Pro Leu Tyr Glu Ser Ser Arg
145                 150                 155                 160
Val Val Ala Gln Lys Met Thr Thr Ala Ala Leu Arg His Ile Arg Gln
                165                 170                 175
Ile Ala Gln Glu Thr Ser Gly Glu Val Val Tyr Ala Leu Gly Arg Gln
            180                 185                 190
Pro Ala Val Leu Arg Thr Phe Ser Gln Arg Leu Ser Arg Gly Phe Asn
        195                 200                 205
Asp Ala Ile Ser Gly Phe Asn Asp Asp Gly Trp Ser Val Met Ala Gly
    210                 215                 220
Asp Gly Ile Glu Asp Val Ile Ile Ala Cys Asn Ser Lys Lys Ile Arg
225                 230                 235                 240
Ser Asn Asn Thr Ala Pro Asn Ala Phe Ile Ala Pro Gly Gly Val Ile
                245                 250                 255
Cys Ala Lys Ala Ser Met Leu Leu Gln Ser Val Pro Pro Ala Val Leu
            260                 265                 270
Val Arg Phe Leu Arg Glu His Arg Ser Glu Trp Ala Asp Tyr Asn Phe
        275                 280                 285
Asp Ala Tyr Ser Ala Ser Ala Leu Lys Ser Ser Ser Cys Ser Leu Pro
    290                 295                 300
Gly Leu Arg Pro Met Arg Phe Ser Gly Ser Gln Ile Ile Met Pro Leu
305                 310                 315                 320
Ala His Thr Val Glu Asn Glu Glu Ile Leu Glu Val Val Arg Leu Glu
                325                 330                 335
Gly Gln Ala Leu Asp Glu Gly Leu Leu Ser Arg Asp Ile His Leu Leu
            340                 345                 350
Gln Phe Cys Thr Gly Leu Asp Glu Lys Ser Met Gly Ser Cys Phe Gln

```
                355                     360                     365
Leu Val Phe Ala Pro Ile Asp Glu Leu Phe Pro Asp Asp Ala Pro Leu
    370                     375                     380
Ile Ser Ser Gly Phe Arg Val Ile Pro Leu Asp Met Lys Thr Asp Gly
385                     390                     395                 400
Ala Pro Ala Gly Arg Thr Leu Asp Leu Ala Ser Ser Leu Glu Ala Gly
                405                     410                     415
Ser Thr Thr Leu Gln Ala Ser Gly Gly Ala Asp Asp Cys Asn Leu Arg
            420                     425                     430
Ser Val Leu Thr Ile Ala Phe Gln Phe Pro Tyr Glu Met His Leu Gln
        435                     440                     445
Asp Ser Val Ala Thr Met Ala Arg Gln Tyr Val Arg Ser Ile Val Ser
    450                     455                     460
Ala Val Gln Arg Val Ser Met Ala Ile Ser Pro Ser Arg Ser Gly Leu
465                     470                     475                 480
Asn Ala Glu Gln Lys Ile Ile Ser Gly Phe Pro Glu Ala Ala Thr Leu
                485                     490                     495
Ala Arg Trp Ile Cys Gln Ser Tyr Arg Phe His Leu Gly Val Glu Leu
            500                     505                     510
Phe Arg Gln Ala Asp Glu Ala Gly Glu Ser Leu Leu Arg Met Leu Trp
        515                     520                     525
Asp His Glu Asp Ala Ile Leu Cys Cys Ser Phe Lys Glu Lys Pro Val
    530                     535                     540
Phe Thr Phe Ala Asn Glu Met Gly Ile Asn Met Leu Glu Thr Ser Phe
545                     550                     555                 560
Val Ala Leu Gln Asp Leu Ser Leu Asp Lys Ile Phe Asp Glu Ala Gly
                565                     570                     575
Arg Lys Ala Leu Tyr Ser Glu Ile Pro Lys Leu Met Glu Gln Gly Phe
            580                     585                     590
Val Tyr Leu Pro Gly Gly Val Cys Leu Ser Gly Met Gly Arg His Val
        595                     600                     605
Ser Phe Glu Ser Ala Val Ala Trp Lys Val Val Gly Glu Asp Asn Asn
    610                     615                     620
Val His Cys Leu Ala Phe Cys Phe Val Asn Trp Ser Phe Val
625                     630                     635
```

<210> 214
<211> 1554
<212> DNA
<213> Hordeum vulgare
<400> 214

```
gcgctgggga ggcagcctgc tgttctgcgg acatttagtc agaggctgag tagaggattt      60
aatgatgcta taagtggctt caatgatgat ggttggtctg taatggccgg agatggcatt     120
gaagatgtga ttatcgcttg caactcaaag aagattagga gcaataacac tgctcccaat     180
gcttttatag ctcctggagg tgttatatgt gctaaagcat caatgttact gcagagtgtt     240
ccaccagcag tactggttcg atttctaagg gaacatcggt ctgaatgggc tgattataac     300
tttgatgcat attcggcttc agcgctgaaa tcaagttcat gctcacttcc tgggttgcgc     360
cctatgagat tttctgggag ccagatcatc atgccacttg ctcacacggt ggagaatgag     420
gagattctag aagttgttcg tcttgaaggg caagcgctcg atgagggtct tttatcaaga     480
gatatccacc tgcttcagtt ttgcactgga atagacgaga aatcaatggg gtcctgcttc     540
caacttgtct ttgccccaat tgatgagctt ttccctgatg atgctccatt aatatcttca     600
ggcttccgtg ttataccact ggacatgaaa acagatggtg cacccaccgg tagaacacta     660
gatttggcct ctagccttga agctggttca actacactgc aagcctcagg caatgcggac     720
gattgtaatc tacgatcagt gctgacaatt gcctttcaat tcccttacga gatgcatctc     780
caagatagtg ttgcaaccat ggcccggcag tatgtccgca gcattgtctc tgccgttcag     840
agagtgtcga tggctatctc tccctctcga tctggtttga atgctgaaca gaagataatt     900
tctggcttcc ctgaagctgc aacacttgct cgctggatat gccaaagcta ccggttccat     960
ctgggggtcg agttatttag acaggcagat gaagctgggg aatctttatt gagaatgctc    1020
tgggatcatg aagatgctat tttgtgctgt tctttcaagg aaaagcctgt atttacgttt    1080
gcaaacgaga tggggattaa catgttggaa acatctttcg ttgcccttca agacctctcg    1140
ttggacaaga tatttgatga agctggtaga aaggcactat actctgagat cccaaagttg    1200
atggagcagg gctttgttta cctgccaggt ggtgtgtgct tgtctgggat gggccgccat    1260
gtctccttcg agaatgctat agcatggaaa gtagtcggtg aggacaacaa tgtgcactgc    1320
cttgccttct gcttcgtcaa ctggtctttc gtgtgatcat cctcccaagg caatccgtgc    1380
ccgtcgcacg cagctccatt tttgctctct ctctctgtag gagagaaccg ctgccgctgg    1440
aaagtagttt catgctatct ttaactagtt tgtttgtaga tttgaagagc cagcatccgg    1500
aagaattctg ccttgtgtaa atctgctcac atttccacta atttacccag gcaa          1554
```

<210> 215
<211> 451
<212> PRT
<213> Hordeum vulgare
<400> 215

Ala Leu Gly Arg Gln Pro Ala Val Leu Arg Thr Phe Ser Gln Arg Leu
1               5                   10                  15
Ser Arg Gly Phe Asn Asp Ala Ile Ser Gly Phe Asn Asp Asp Gly Trp
            20                  25                  30
Ser Val Met Ala Gly Asp Gly Ile Glu Asp Val Ile Ile Ala Cys Asn
        35                  40                  45
Ser Lys Lys Ile Arg Ser Asn Asn Thr Ala Pro Asn Ala Phe Ile Ala
    50                  55                  60
Pro Gly Gly Val Ile Cys Ala Lys Ala Ser Met Leu Leu Gln Ser Val
65                  70                  75                  80
Pro Pro Ala Val Leu Val Arg Phe Leu Arg Glu His Arg Ser Glu Trp
                85                  90                  95
Ala Asp Tyr Asn Phe Asp Ala Tyr Ser Ala Ser Ala Leu Lys Ser Ser
            100                 105                 110
Ser Cys Ser Leu Pro Gly Leu Arg Pro Met Arg Phe Ser Gly Ser Gln
        115                 120                 125
Ile Ile Met Pro Leu Ala His Thr Val Glu Asn Glu Glu Ile Leu Glu
    130                 135                 140
Val Val Arg Leu Glu Gly Gln Ala Leu Asp Glu Gly Leu Leu Ser Arg
145                 150                 155                 160
Asp Ile His Leu Leu Gln Phe Cys Thr Gly Ile Asp Glu Lys Ser Met
                165                 170                 175
Gly Ser Cys Phe Gln Leu Val Phe Ala Pro Ile Asp Glu Leu Phe Pro
            180                 185                 190
Asp Asp Ala Pro Leu Ile Ser Ser Gly Phe Arg Val Ile Pro Leu Asp
        195                 200                 205
Met Lys Thr Asp Gly Ala Pro Thr Gly Arg Thr Leu Asp Leu Ala Ser
    210                 215                 220
Ser Leu Glu Ala Gly Ser Thr Thr Leu Gln Ala Ser Gly Asn Ala Asp
225                 230                 235                 240
Asp Cys Asn Leu Arg Ser Val Leu Thr Ile Ala Phe Gln Phe Pro Tyr
                245                 250                 255
Glu Met His Leu Gln Asp Ser Val Ala Thr Met Ala Arg Gln Tyr Val
            260                 265                 270
Arg Ser Ile Val Ser Ala Val Gln Arg Val Ser Met Ala Ile Ser Pro
        275                 280                 285
Ser Arg Ser Gly Leu Asn Ala Glu Gln Lys Ile Ile Ser Gly Phe Pro
    290                 295                 300
Glu Ala Ala Thr Leu Ala Arg Trp Ile Cys Gln Ser Tyr Arg Phe His
305                 310                 315                 320
Leu Gly Val Glu Leu Phe Arg Gln Ala Asp Glu Ala Gly Glu Ser Leu
                325                 330                 335
Leu Arg Met Leu Trp Asp His Glu Asp Ala Ile Leu Cys Cys Ser Phe
            340                 345                 350
Lys Glu Lys Pro Val Phe Thr Phe Ala Asn Glu Met Gly Ile Asn Met
        355                 360                 365
Leu Glu Thr Ser Phe Val Ala Leu Gln Asp Leu Ser Leu Asp Lys Ile
    370                 375                 380
Phe Asp Glu Ala Gly Arg Lys Ala Leu Tyr Ser Glu Ile Pro Lys Leu
385                 390                 395                 400
Met Glu Gln Gly Phe Val Tyr Leu Pro Gly Gly Val Cys Leu Ser Gly
                405                 410                 415
Met Gly Arg His Val Ser Phe Glu Asn Ala Ile Ala Trp Lys Val Val
            420                 425                 430
Gly Glu Asp Asn Asn Val His Cys Leu Ala Phe Cys Phe Val Asn Trp
        435                 440                 445
Ser Phe Val
    450

<210> 216
<211> 2622
<212> DNA
<213> Phyllostachys praecox
<400> 216

cggcgggtac gacaaggccg ggatagactc gggcaagtac gtgcgataca cgccggagca 60
ggtggaggcg ctcgagcgga tgtatgctga gtgccccaag cccagctcca cgcgcaggca 120
gcagctgctg cgcgagtgcc cgattctggc caacatcgag cccaagcaga tcaaggtctg 180
gttccagaac cgaaggtgcc gtgataagca gcggaaggag gcttcccggc ttcaggccgt 240
gaacagaaaa ttgaccgcaa tgaacaagct tctcatggaa gagaatgatc gtctccagaa 300
gcaggtttcc cagctggttc atgagaatgc atacatgaag cagcagctgc agaatccatc 360
attggccaat gatacaagct gtgaatcaaa tgtgaccact caaaaccctc tgaaggatgc 420
aagtaaccct tctggactcc tttcaattgc ggaggagacc ttgacagagt tcctctccaa 480

```
ggctacaggg actgctgttg attgggttca gatgcctggg atgaagcctg gtccggattc     540
ggttggtatt gtggccattt cacatggttg ccgtggtgtt gctgcccgtg cctgtgattt     600
ggtgaatcta gaaccaacaa aagttgtgga gatcttgaaa gaccgtccat cttggttctg     660
tgatcgtcaa agcctggaag tcttcacaat gtttccagct ggaaatggag gaacaattga     720
acttgtctac acgcagttgt atgctccaac aactttagtc cctgcacgtg atttttggac     780
tctaaggtac acaaccacaa tggaagatgg cagccttgtg gtctgtgaga gatccttgag     840
tggttcaggg ggtggtccaa gtgcagcctc tgcacagcaa tttgtaagag ccgaaatgct     900
tccaagtgga tatttagttc gcccatgcga gggtgggggc tcaattgtgc acatagtgga     960
ccatctggac cttgaggctt ggagtgttcc tgaagtgctt cggccgctct acgagtcgtc    1020
tagggtagtt gcccagaaaa tgactacagc ggcactacgg cacatcagac aaattgctca    1080
agaaactagt ggggaggttg tatatgcttt ggggaggcag cctgccgttc tacggacatt    1140
tagtcagagg ttgagtagag gatttaacga tgctataagt ggtttcaatg atgatggttg    1200
gtctgtcatg ggtggagatg gcattgaaga tgtaatcatt gcttgcaact caaagaagat    1260
taggaacaat agcactgctg ccaatgcttt tggagcccct ggaggcgtta tatgtgctaa    1320
ggcatccatg ttactgcaga gtgttccacc agcagtactg gttcggtttc tgagggaaca    1380
tcggtctgaa tgggctgatt ataactttga tgcatattcg gctttagcac tgaagacgag    1440
ctcatgttca cttcctgggt tgcggcctac gagattttct gggagccaga tcatcatgcc    1500
acttgctcac acagtggaga atgaggagat tctagaagtc attcgtcttg aagggcaggc    1560
acttacacat gatgaaggtc ttttatcaag agatatccac ctgcttcagc tttgcactgg    1620
aatagatgag aaatcaatgg gatcctgctt ccagcttgtc tttgcaccca tcgatgagct    1680
tttccctgat gatgccccat tgatatcttc aggctttcgt gttataccac tggacatgaa    1740
aacagatggt gcacctactg gtagaacatt agatttggcc tctagccttg aagttggttc    1800
aactacacaa caagctacag ggatgcatc tttggatgat tgtaggaatc tgcgatcagt     1860
gctgacaatt gcctttcaat tcccttatga aattcatctc caggatagtg ttgcaactat    1920
ggcccggcaa tacgtccgta gcgttgtttc tgctgtgcag agagtgtcga tggctatttc    1980
tccccctcga tctggtgtga atgctgggca gaagatattt tctggcttcc ctgaagccgc    2040
aacacttgct cgctggattt gccaaagcta ccagttccat ttgggagtgg agttacttag    2100
gcaggcagat gaagccgggg aatcattatt gagaatgctc tgggattacg aagatgctat    2160
cttgtgctgt tctttcaagg aaaagcctgt atttactttt gccaacgaga tgggacttaa    2220
catgttagaa acatctctcg ttgctctaca agacctctca ttggacaaga tatttgatga    2280
aactggtaga aaggcactac attcggagat cccaaaattg atggaacagg gctatgttta    2340
cctgccggct ggtgtgtgct tgtccggaat gggccgccat gtctctttcg agcaagctgt    2400
agcatggaaa gtacttggtg aggacaacaa tgtgcactgc ctggccttct gcttcgtcaa    2460
ctggtctttc gtgtgatcca tccgacgcaa tccatgtccg ttgtgagcag caccattttc    2520
gcattctctg tagaagagaa ccatcgtcgc tgttagttaa tgctgtcttt aactagtttc    2580
tagatttgga aaagccagtc tgcaaaaaaa aaaaaaaaaa aa                       2622
```

<210> 217
<211> 824
<212> PRT
<213> Phyllostachys praecox
<400> 217

```
Gly Gly Tyr Asp Lys Ala Gly Ile Asp Ser Gly Lys Tyr Val Arg Tyr
1               5                   10                  15
Thr Pro Glu Gln Val Glu Ala Leu Glu Arg Met Tyr Ala Glu Cys Pro
            20                  25                  30
Lys Pro Ser Ser Thr Arg Arg Gln Gln Leu Leu Arg Glu Cys Pro Ile
        35                  40                  45
Leu Ala Asn Ile Glu Pro Lys Gln Ile Lys Val Trp Phe Gln Asn Arg
    50                  55                  60
Arg Cys Arg Asp Lys Gln Arg Lys Glu Ala Ser Arg Leu Gln Ala Val
65                  70                  75                  80
Asn Arg Lys Leu Thr Ala Met Asn Lys Leu Leu Met Glu Glu Asn Asp
                85                  90                  95
Arg Leu Gln Lys Gln Val Ser Gln Leu Val His Glu Asn Ala Tyr Met
            100                 105                 110
Lys Gln Gln Leu Gln Asn Pro Ser Leu Ala Asn Asp Thr Ser Cys Glu
        115                 120                 125
Ser Asn Val Thr Thr Gln Asn Pro Leu Lys Asp Ala Ser Asn Pro Ser
    130                 135                 140
Gly Leu Leu Ser Ile Ala Glu Glu Thr Leu Thr Glu Phe Leu Ser Lys
145                 150                 155                 160
Ala Thr Gly Thr Ala Val Asp Trp Val Gln Met Pro Gly Met Lys Pro
                165                 170                 175
Gly Pro Asp Ser Val Gly Ile Val Ala Ile Ser His Gly Cys Arg Gly
            180                 185                 190
Val Ala Ala Arg Ala Cys Asp Leu Val Asn Leu Glu Pro Thr Lys Val
        195                 200                 205
Val Glu Ile Leu Lys Asp Arg Pro Ser Trp Phe Cys Asp Arg Gln Ser
    210                 215                 220
Leu Glu Val Phe Thr Met Phe Pro Ala Gly Asn Gly Gly Thr Ile Glu
225                 230                 235                 240
```

```
Leu Val Tyr Thr Gln Leu Tyr Ala Pro Thr Thr Leu Val Pro Ala Arg
                245                 250                 255
Asp Phe Trp Thr Leu Arg Tyr Thr Thr Thr Met Glu Asp Gly Ser Leu
            260                 265                 270
Val Val Cys Glu Arg Ser Leu Ser Gly Ser Gly Gly Gly Pro Ser Ala
        275                 280                 285
Ala Ser Ala Gln Gln Phe Val Arg Ala Glu Met Leu Pro Ser Gly Tyr
    290                 295                 300
Leu Val Arg Pro Cys Glu Gly Gly Gly Ser Ile Val His Ile Val Asp
305                 310                 315                 320
His Leu Asp Leu Glu Ala Trp Ser Val Pro Glu Val Leu Arg Pro Leu
                325                 330                 335
Tyr Glu Ser Ser Arg Val Val Ala Gln Lys Met Thr Thr Ala Ala Leu
            340                 345                 350
Arg His Ile Arg Gln Ile Ala Gln Glu Thr Ser Gly Glu Val Val Tyr
        355                 360                 365
Ala Leu Gly Arg Gln Pro Ala Val Leu Arg Thr Phe Ser Gln Arg Leu
    370                 375                 380
Ser Arg Gly Phe Asn Asp Ala Ile Ser Gly Phe Asn Asp Asp Gly Trp
385                 390                 395                 400
Ser Val Met Gly Gly Asp Gly Ile Glu Asp Val Ile Ile Ala Cys Asn
                405                 410                 415
Ser Lys Lys Ile Arg Asn Asn Ser Thr Ala Ala Asn Ala Phe Gly Ala
            420                 425                 430
Pro Gly Gly Val Ile Cys Ala Lys Ala Ser Met Leu Leu Gln Ser Val
        435                 440                 445
Pro Pro Ala Val Leu Val Arg Phe Leu Arg Glu His Arg Ser Glu Trp
    450                 455                 460
Ala Asp Tyr Asn Phe Asp Ala Tyr Ser Ala Leu Ala Leu Lys Thr Ser
465                 470                 475                 480
Ser Cys Ser Leu Pro Gly Leu Arg Pro Thr Arg Phe Ser Gly Ser Gln
                485                 490                 495
Ile Ile Met Pro Leu Ala His Thr Val Glu Asn Glu Glu Ile Leu Glu
            500                 505                 510
Val Ile Arg Leu Glu Gly Gln Ala Leu Thr His Asp Glu Gly Leu Leu
        515                 520                 525
Ser Arg Asp Ile His Leu Leu Gln Leu Cys Thr Gly Ile Asp Glu Lys
    530                 535                 540
Ser Met Gly Ser Cys Phe Gln Leu Val Phe Ala Pro Ile Asp Glu Leu
545                 550                 555                 560
Phe Pro Asp Asp Ala Pro Leu Ile Ser Ser Gly Phe Arg Val Ile Pro
                565                 570                 575
Leu Asp Met Lys Thr Asp Gly Ala Pro Thr Gly Arg Thr Leu Asp Leu
            580                 585                 590
Ala Ser Ser Leu Glu Val Gly Ser Thr Thr Gln Gln Ala Thr Gly Asp
        595                 600                 605
Ala Ser Leu Asp Asp Cys Arg Asn Leu Arg Ser Val Leu Thr Ile Ala
    610                 615                 620
Phe Gln Phe Pro Tyr Glu Ile His Leu Gln Asp Ser Val Ala Thr Met
625                 630                 635                 640
Ala Arg Gln Tyr Val Arg Ser Val Val Ser Ala Val Gln Arg Val Ser
                645                 650                 655
Met Ala Ile Ser Pro Pro Arg Ser Gly Val Asn Ala Gly Gln Lys Ile
            660                 665                 670
Phe Ser Gly Phe Pro Glu Ala Ala Thr Leu Ala Arg Trp Ile Cys Gln
        675                 680                 685
Ser Tyr Gln Phe His Leu Gly Val Glu Leu Leu Arg Gln Ala Asp Glu
    690                 695                 700
Ala Gly Glu Ser Leu Leu Arg Met Leu Trp Asp Tyr Glu Asp Ala Ile
705                 710                 715                 720
Leu Cys Cys Ser Phe Lys Glu Lys Pro Val Phe Thr Phe Ala Asn Glu
                725                 730                 735
Met Gly Leu Asn Met Leu Glu Thr Ser Leu Val Ala Leu Gln Asp Leu
            740                 745                 750
Ser Leu Asp Lys Ile Phe Asp Glu Thr Gly Arg Lys Ala Leu His Ser
        755                 760                 765
Glu Ile Pro Lys Leu Met Glu Gln Gly Tyr Val Tyr Leu Pro Ala Gly
    770                 775                 780
Val Cys Leu Ser Gly Met Gly Arg His Val Ser Phe Glu Gln Ala Val
785                 790                 795                 800
Ala Trp Lys Val Leu Gly Glu Asp Asn Asn Val His Cys Leu Ala Phe
```

```
                805                 810                 815
Cys Phe Val Asn Trp Ser Phe Val
            820
<210>  218
<211>  2193
<212>  DNA
<213>  Oryza sativa
<400>  218
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga attttttttc ccaaatatct taaaaagtca    1740
ctttctggtt cagttcaatg aattgattgc tacaaataat gcttttatag cgttatccta    1800
gctgtagttc agttaatagg taataccccct atagtttagt caggagaaga acttatccga    1860
tttctgatct ccatttttaa ttatatgaaa tgaactgtag cataagcagt attcatttgg    1920
attatttttt ttattagctc tcaccccttc attattctga gctgaaagtc tggcatgaac    1980
tgtcctcaat tttgttttca aattcacatc gattatctat gcattatcct cttgtatcta    2040
cctgtagaag tttctttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193
<210>  219
<211>  51
<212>  DNA
<213>  Artificial sequence
<220>
<223>  primer: prm03263
<400>  219
ggggacaagt ttgtacaaaa aagcaggctt gtgctaaggc atccatgcta c               51
<210>  220
<211>  51
<212>  DNA
<213>  Artificial sequence
<220>
<223>  primer: prm03264
<400>  220
ggggaccact ttgtacaaga aagctgggtg caccttccat gctacagctt g               51
<210>  221
<211>  50
<212>  DNA
<213>  Artificial sequence
<220>
<223>  primer: prm01983
<400>  221
ggggacaagt ttgtacaaaa aagcaggctt cacaatggcg gcggcggtgg                 50
<210>  222
<211>  54
```

<212> DNA
<213> Artificial sequence
<220>
<223> primer: prm01984
<400> 222
ggggaccact ttgtacaaga aagctgggtg gattttgggt cacacgaagg acca 54
<210> 223
<211> 1096
<212> DNA
<213> Oryza sativa
<400> 223
tgtgctaagg catccatgct actgcagagt gtccctcctg cagttttggt tcgatttttg 60
agggaacatc gttctgaatg ggcggattat aacttcgatg catattcagc ttcatctctg 120
aagacaagct catgttcact tcctgggttg cggcctatga gattttctgg gagccagatc 180
attatgccac ttgctcacac ggtggagaat gaagagattt tagaagttgt ccgtcttgaa 240
ggacaagcac ttacacatga tgatggtctt atgtctagag atattcacct gcttcagctt 300
tgcactggaa tagatgagaa atcaatggga tcctgcttcc agcttgtctt tgcaccaatc 360
gatgagcttt tccctgatga tgctccgtta atatcttcag gctttcgtgt tataccgctg 420
gacatgaaaa cagatggtac acctgctggt agaacattag atttggcatc tagccttgag 480
gttggttcaa ctgcacagcc cacaggggat gcatctatgg atgactgtaa tctacgatca 540
gtgctgacaa ttgcctttca gttcccttat gaaatgcatc tccaagacag cgttgcaact 600
atggcccggc aatatgtccg cagtattgtt tcctctgttc agagagtatc aatggctgtt 660
tctccttctc ggtctggctt gaatgctggg cagaagataa tttcaggctt ccctgaagcc 720
ccaacgctag ctcgttggat ttgccaaagc taccagttcc atttggggt ggagttactt 780
aggcaggcag atgatgctgg ggaagcacta ttgaaaatgc tatgggatta cgaagacgct 840
attttgtgct gttctttcaa ggaaaagcct gtatttactt ttgccaacga gatgggacta 900
aacatgctag aaacatctct cgtcgctctc caagatctct cactggacaa gatatttgat 960
gaagccggta ggaaggccct atacaacgag atcccgaaat tgatggaaca gggttacgtg 1020
tacctgcctg gtggagtgtg cttgtccggg atggggcgcc atgtttcttt cgagcaagct 1080
gtagcatgga aggtgc 1096
<210> 224
<211> 2553
<212> DNA
<213> Brassica rapa
<400> 224
atggagatgg cggtggcgaa tcaccgagag agaagcagtg atagcatgaa cagacattta 60
gacagcagcg gcaagtacgt caggtacacc gctgagcaag tcgaggccct cgagcgtgtc 120
tacgccgagt gtcccaagcc tagctcgctc cggagacagc agttgatccg tgaatgttct 180
atcttggcca acatcgagcc taagcagatc aaagtctggt tccaaaaccg gaggtgtcga 240
gataagcaga ggaaagaggc gtcgaggctc cagagcgtaa acaggaagct ttcggctatg 300
aacaagctgt tgatggagga gaacgatagg ttgcagaagc aagtttctca gctcgtctgt 360
gaaaatggat acatgaagca gcagcttact actactgttg tgaatgatcc aagctgtgat 420
tctgtggtga caactcctca gcattcgctt agagacgcta atagtcctgc tgggctgctc 480
tcgatcgcag aggagacatt ggcagagttc ctatccaagg ctacaggaac tgctgttgat 540
tgggttcaga tgcctgggat gaagcctggt ccggattcgg ttgggatctt tgctatatcg 600
caaagatgca gtggggtggc agctcgagcc tgtggtcttg ttagtttaga gcctgtcaag 660
attgcagaga tactcaaaga taggccatct tggttccgtg actgtaggag ccttgaagtc 720
ttcactatgt tcccggctgg taacggcggc accattgagc tcgtctatat gcagacatat 780
gcaccaacga ctctggctcc tgcccgcgat ttctggaccc tgagatacac aacgagccta 840
gacaatggca gttttgtggt ttgtgagagg tcactctctg gttctggtgc tggtcctaac 900
gctgcatcag cttctcagtt tgtaagagca gaaatgcttt ctagtgggta tctaataagg 960
ccttgtgatg gtggcggttc cattattcac attgtcgatc accttaatct tgaggcttgg 1020
agtgttcccg atgtgcttcg accccttac gagtcatcta aagtcgtagc acagaaaatg 1080
accatttcag cattgaggta tatcaggcaa ctagctcaag agtctaatgg tgaattagtg 1140
tatggattag gaagacagcc tgctgtttta agaacattta gccaaagatt aagcaggggt 1200
tttaatgatg cggttaatgg gtttggtgac gacggatggt ctacaatgca ttgtgatggg 1260
gctgaagaca taatcgttgc tattaactct acaaagcatt tgaataatat gtctaattct 1320
ctttccttcc ttggaggtgt cctttgtgcc aaggcttcta tgcttctcca aaatgttcct 1380
cctgcggttt tgatccggtt tctaagagag catcggtccg agtgggctga cttcaatgtt 1440
gatgcatatt ctgctgcaac gcttaaagcc ggttcttttg cttatccggg tatgaggcct 1500
acaaggttca ccgggagcca aatcataatg cctctaggac ataccatcga acacgaagaa 1560
atgcttgaag ttgttagact agaaggtcat tctcttgcac aagaagatgc gttcatgtcc 1620
cgtgatgtcc atcttcttca ggtatgtact gggattgatg agaacgctgt tggagcatgt 1680
tcagaactaa tatttgctcc catcaatgag atgttcccgg atgatgctcc acttgttcct 1740
tctggattca gagtcatacc cgttgatgct aaaacgggag atgcgcaaga tctgttgacc 1800
gctaaccacc ggacgctaga cttgacttct agccttgagg ttggtccaac accagagaac 1860
gcttctggaa actcttcttc tagctcaagc tcaagatgta tcctcaccat cgcgtttcag 1920
ttccctttttg aaaacaactt gcaagacaat gttgctggta tggcttgtca gtacgtgcgg 1980
agcgtgatct catcggttca acgtgttgca atggccatct caccctctgg gataagccca 2040
agtctgggat ccaaactgtc cccggggtct cctgaagctg ttacacttgc ccaatggatc 2100
tcacaaagtt acagtcacca cttaggctcg gagttgctga cggttgactc gcttggaagc 2160

```
aacgactcgg tattgaaact tctatgggat caccaagatg ccattttgtg ttgctcttta    2220
aagcctcagc cagtgtttat gttcgcgaac caagctggtt tagacatgtt agagacgacg    2280
cttgtagcct tacaagacat tacactcgaa aagatctttg atgaatctgg tcgaaaggct    2340
ctctgctccg atttcgccaa gctaatgcaa cagggatatg catgcttgcc ttcaggaata    2400
tgtttgtcta cgatgggaag acatgtgact tatgaacaag ctgttgcttg gaaagtgttt    2460
gctcctgctg ctgcatcatc cgaaaacaac gatggcaatg ataatacttt gcactgtctt    2520
gctttctcct ttgtaaactg gtcgtttgtg taa                                 2553
<210>  225
<211>  850
<212>  PRT
<213>  Brassica rapa
<400>  225
Met Glu Met Ala Val Ala Asn His Arg Glu Arg Ser Ser Asp Ser Met
1               5                   10                  15
Asn Arg His Leu Asp Ser Ser Gly Lys Tyr Val Arg Tyr Thr Ala Glu
            20                  25                  30
Gln Val Glu Ala Leu Glu Arg Val Tyr Ala Glu Cys Pro Lys Pro Ser
        35                  40                  45
Ser Leu Arg Arg Gln Gln Leu Ile Arg Glu Cys Ser Ile Leu Ala Asn
    50                  55                  60
Ile Glu Pro Lys Gln Ile Lys Val Trp Phe Gln Asn Arg Arg Cys Arg
65                  70                  75                  80
Asp Lys Gln Arg Lys Glu Ala Ser Arg Leu Gln Ser Val Asn Arg Lys
                85                  90                  95
Leu Ser Ala Met Asn Lys Leu Leu Met Glu Glu Asn Asp Arg Leu Gln
            100                 105                 110
Lys Gln Val Ser Gln Leu Val Cys Glu Asn Gly Tyr Met Lys Gln Gln
        115                 120                 125
Leu Thr Thr Thr Val Val Asn Asp Pro Ser Cys Asp Ser Val Val Thr
    130                 135                 140
Thr Pro Gln His Ser Leu Arg Asp Ala Asn Ser Pro Ala Gly Leu Leu
145                 150                 155                 160
Ser Ile Ala Glu Glu Thr Leu Ala Glu Phe Leu Ser Lys Ala Thr Gly
                165                 170                 175
Thr Ala Val Asp Trp Val Gln Met Pro Gly Met Lys Pro Gly Pro Asp
            180                 185                 190
Ser Val Gly Ile Phe Ala Ile Ser Gln Arg Cys Ser Gly Val Ala Ala
        195                 200                 205
Arg Ala Cys Gly Leu Val Ser Leu Glu Pro Val Lys Ile Ala Glu Ile
    210                 215                 220
Leu Lys Asp Arg Pro Ser Trp Phe Arg Asp Cys Arg Ser Leu Glu Val
225                 230                 235                 240
Phe Thr Met Phe Pro Ala Gly Asn Gly Gly Thr Ile Glu Leu Val Tyr
                245                 250                 255
Met Gln Thr Tyr Ala Pro Thr Thr Leu Ala Pro Ala Arg Asp Phe Trp
            260                 265                 270
Thr Leu Arg Tyr Thr Thr Ser Leu Asp Asn Gly Ser Phe Val Val Cys
        275                 280                 285
Glu Arg Ser Leu Ser Gly Ser Gly Ala Gly Pro Asn Ala Ala Ser Ala
    290                 295                 300
Ser Gln Phe Val Arg Ala Glu Met Leu Ser Ser Gly Tyr Leu Ile Arg
305                 310                 315                 320
Pro Cys Asp Gly Gly Gly Ser Ile Ile His Ile Val Asp His Leu Asn
                325                 330                 335
Leu Glu Ala Trp Ser Val Pro Asp Val Leu Arg Pro Leu Tyr Glu Ser
            340                 345                 350
Ser Lys Val Val Ala Gln Lys Met Thr Ile Ser Ala Leu Arg Tyr Ile
        355                 360                 365
Arg Gln Leu Ala Gln Glu Ser Asn Gly Glu Leu Val Tyr Gly Leu Gly
    370                 375                 380
Arg Gln Pro Ala Val Leu Arg Thr Phe Ser Gln Arg Leu Ser Arg Gly
385                 390                 395                 400
Phe Asn Asp Ala Val Asn Gly Phe Gly Asp Asp Gly Trp Ser Thr Met
                405                 410                 415
His Cys Asp Gly Ala Glu Asp Ile Ile Val Ala Ile Asn Ser Thr Lys
            420                 425                 430
His Leu Asn Asn Met Ser Asn Ser Leu Ser Phe Leu Gly Gly Val Leu
        435                 440                 445
Cys Ala Lys Ala Ser Met Leu Leu Gln Asn Val Pro Pro Ala Val Leu
    450                 455                 460
Ile Arg Phe Leu Arg Glu His Arg Ser Glu Trp Ala Asp Phe Asn Val
```

465 470 475 480
Asp Ala Tyr Ser Ala Ala Thr Leu Lys Ala Gly Ser Phe Ala Tyr Pro
485 490 495
Gly Met Arg Pro Thr Arg Phe Thr Gly Ser Gln Ile Ile Met Pro Leu
500 505 510
Gly His Thr Ile Glu His Glu Glu Met Leu Glu Val Val Arg Leu Glu
515 520 525
Gly His Ser Leu Ala Gln Glu Asp Ala Phe Met Ser Arg Asp Val His
530 535 540
Leu Leu Gln Val Cys Thr Gly Ile Asp Glu Asn Ala Val Gly Ala Cys
545 550 555 560
Ser Glu Leu Ile Phe Ala Pro Ile Asn Glu Met Phe Pro Asp Asp Ala
565 570 575
Pro Leu Val Pro Ser Gly Phe Arg Val Ile Pro Val Asp Ala Lys Thr
580 585 590
Gly Asp Ala Gln Asp Leu Leu Thr Ala Asn His Arg Thr Leu Asp Leu
595 600 605
Thr Ser Ser Leu Glu Val Gly Pro Thr Pro Glu Asn Ala Ser Gly Asn
610 615 620
Ser Ser Ser Ser Ser Ser Ser Arg Cys Ile Leu Thr Ile Ala Phe Gln
625 630 635 640
Phe Pro Phe Glu Asn Asn Leu Gln Asp Asn Val Ala Gly Met Ala Cys
645 650 655
Gln Tyr Val Arg Ser Val Ile Ser Ser Val Gln Arg Val Ala Met Ala
660 665 670
Ile Ser Pro Ser Gly Ile Ser Pro Ser Leu Gly Ser Lys Leu Ser Pro
675 680 685
Gly Ser Pro Glu Ala Val Thr Leu Ala Gln Trp Ile Ser Gln Ser Tyr
690 695 700
Ser His His Leu Gly Ser Glu Leu Leu Thr Val Asp Ser Leu Gly Ser
705 710 715 720
Asn Asp Ser Val Leu Lys Leu Leu Trp Asp His Gln Asp Ala Ile Leu
725 730 735
Cys Cys Ser Leu Lys Pro Gln Pro Val Phe Met Phe Ala Asn Gln Ala
740 745 750
Gly Leu Asp Met Leu Glu Thr Thr Leu Val Ala Leu Gln Asp Ile Thr
755 760 765
Leu Glu Lys Ile Phe Asp Glu Ser Gly Arg Lys Ala Leu Cys Ser Asp
770 775 780
Phe Ala Lys Leu Met Gln Gln Gly Tyr Ala Cys Leu Pro Ser Gly Ile
785 790 795 800
Cys Leu Ser Thr Met Gly Arg His Val Thr Tyr Glu Gln Ala Val Ala
805 810 815
Trp Lys Val Phe Ala Pro Ala Ala Ala Ser Ser Glu Asn Asn Asp Gly
820 825 830
Asn Asp Asn Thr Leu His Cys Leu Ala Phe Ser Phe Val Asn Trp Ser
835 840 845
Phe Val
850

<210> 226
<211> 2529
<212> DNA
<213> Ginkgo biloba
<400> 226

atggcagtaa tagcacagaa agacgttaaa ggtgccatgg atacgagcaa gtatgttcgc 60
tatacttctg aacaagtcga agctcttgaa cgcgtttaca gcgagtgtcc gaagcctagt 120
tctcttcgtc ggcagcagct tattagagag tgtccaatac tttctaatat tgagcccaag 180
caaatcaaag tttggttcca aaatcgcagg tgtcgagaga aacagaggaa ggaggcatca 240
cgccttcaaa ctgttaacag gaagcttacg gcaatgaaca aattgctgat ggaggaaaat 300
gatcgccttc aaaagcaggt ttcacagttg gtgtacgaga acggttatat gagacagcag 360
ctacagaatg catctgtagc aacaacagac acaagttgtg agtctgttgt gactagtggt 420
cagcaccagc ataatccaac acctcagcat cctccaaggg atgctagccc cgctggactc 480
ctgtctatcg cagaggagac cttggcagag ttcctttcaa aggctacagg aactgctgtt 540
gactgggtcc agatgcctgg gatgaagcct ggtccggatt cgattggtat tgtggctatt 600
tcacacagtt gtagtggagt agctgcacga gcttgcggtc ttgtgggttt agaacctaca 660
aagattgcag aaattctcaa agatcgccca tcttggcttc gtgattgccg ttgccttgat 720
gtgttgactc cctttcctac tggaaatgga gggacaattg agcttttata catgcagaca 780
tatgctccca caactttagc ttctgctaga gatttttgga ctctgagata caccacagta 840
ttggaagatg gtagtcttgt ggtttgtgaa aggtccttaa gtggtgccca gggtggtcca 900
agcatagctc ctgcacagca ctttgtaaga gcagaaatgc ttcccagtgg gtatttgata 960
agaccttgtg aaggtggagg ttctattatc catattgttg accatatgga tttggagcca 1020

tggagtgtgc ctgaggtgtt acgtccacta tatgagtcat ctactgtact cgcccaaaag 1080
atgactattg cagccttgcg ccgcatacgc caaatagcac aggaggttac aggtgaagtg 1140
gtctttggtt ggggtaggca gccagctgtt ctgcggacat ttagccagag actgagcagg 1200
ggtttcaatg aggctgtgaa tggcttcaca gatgatggat ggtctttgat gggtaatgat 1260
ggaatggagg acgtgactat tgcaataaat tcatctccaa gcaaactcct aggttctcag 1320
gttaattctt ctaatgggct tacagctgtt ggtgggggta tactgtgtgc aaaggcatcc 1380
atgcttttac agaatgtgcc tccagcatta cttgttcgct tcttgcggga gcatcgatcg 1440
gagtgggcag attgtaacat tgacgcctat tctgcagctg cattaaaggc aagtccttat 1500
agtgtcccag gttcacgagc aggaggcttt tcaggaagtc aagttatcct ccccctggca 1560
cataccgtgg aacatgaaga gttcttggag gtcattaagc tggagggcca tggcctcaca 1620
caggaagaag ctgtgctatc tagggatatg tttctgttgc agctttgcag tggaattgat 1680
gaaaatgcag ctggtgcttg tgcccaactt gtgtttgcac caattgatga atcatttgct 1740
gatgatgctc ctttgttgcc gtctggtttc cgagttattc ctctggactc tagaacagat 1800
ggtactagtg gtcccaatcg gacattggat ctggcttcag ctcttgaggt tggatcagct 1860
ggaactagaa cttctggcga ttctggagcc aactcgttca atctaagatc tgtgttaact 1920
attgcattcc aattcactta tgagaatcat ttgcgagaaa atgtggcatc catggcccgt 1980
caatatgtac gcagtgttgt agcatctgtg cagagggttg ccatggcatt agcccctctt 2040
cgactgagtt cacatgtggg gccaaggcta ccacctggca ctccagaagc acttactctt 2100
gctcggtgga tttgtcaaag ctacagattc cacctaggtg tagagctgct tcgcgctgat 2160
tgtgaggcca gtgaatccgt gctgaaacta ctctggcacc attcagatgc aattgtgtgc 2220
tgttctttga agtcgctacc agttttcaca tttgcaaatc aagcaggcct tgacatgctg 2280
gagacaaccc tggttgcctt gcaagatata tcattggaca aaatactcga tgaaaatgga 2340
cgcaaaagtt tatgctcaga ttttgcacaa attatgcaac agggctatgc ttatctgcct 2400
gcggggatat gtgtctctag tatgggcagg cctgtttcat atgaccgggc tgttgcttgg 2460
aaggtcctaa atgatgcaga cagcacccac tgcatggttt tcatgtttat gaattggtct 2520
ttcatgtga 2529

<210> 227
<211> 842
<212> PRT
<213> Ginkgo biloba
<400> 227

Met Ala Val Ile Ala Gln Lys Asp Val Lys Gly Ala Met Asp Thr Ser
1 5 10 15
Lys Tyr Val Arg Tyr Thr Ser Glu Gln Val Glu Ala Leu Glu Arg Val
20 25 30
Tyr Ser Glu Cys Pro Lys Pro Ser Ser Leu Arg Arg Gln Gln Leu Ile
35 40 45
Arg Glu Cys Pro Ile Leu Ser Asn Ile Glu Pro Lys Gln Ile Lys Val
50 55 60
Trp Phe Gln Asn Arg Arg Cys Arg Glu Lys Gln Arg Lys Glu Ala Ser
65 70 75 80
Arg Leu Gln Thr Val Asn Arg Lys Leu Thr Ala Met Asn Lys Leu Leu
85 90 95
Met Glu Glu Asn Asp Arg Leu Gln Lys Gln Val Ser Gln Leu Val Tyr
100 105 110
Glu Asn Gly Tyr Met Arg Gln Gln Leu Gln Asn Ala Ser Val Ala Thr
115 120 125
Thr Asp Thr Ser Cys Glu Ser Val Val Thr Ser Gly Gln His Gln His
130 135 140
Asn Pro Thr Pro Gln His Pro Pro Arg Asp Ala Ser Pro Ala Gly Leu
145 150 155 160
Leu Ser Ile Ala Glu Glu Thr Leu Ala Glu Phe Leu Ser Lys Ala Thr
165 170 175
Gly Thr Ala Val Asp Trp Val Gln Met Pro Gly Met Lys Pro Gly Pro
180 185 190
Asp Ser Ile Gly Ile Val Ala Ile Ser His Ser Cys Ser Gly Val Ala
195 200 205
Ala Arg Ala Cys Gly Leu Val Gly Leu Glu Pro Thr Lys Ile Ala Glu
210 215 220
Ile Leu Lys Asp Arg Pro Ser Trp Leu Arg Asp Cys Arg Cys Leu Asp
225 230 235 240
Val Leu Thr Pro Phe Pro Thr Gly Asn Gly Gly Thr Ile Glu Leu Leu
245 250 255
Tyr Met Gln Thr Tyr Ala Pro Thr Thr Leu Ala Ser Ala Arg Asp Phe
260 265 270
Trp Thr Leu Arg Tyr Thr Thr Val Leu Glu Asp Gly Ser Leu Val Val
275 280 285
Cys Glu Arg Ser Leu Ser Gly Ala Gln Gly Gly Pro Ser Ile Ala Pro
290 295 300
Ala Gln His Phe Val Arg Ala Glu Met Leu Pro Ser Gly Tyr Leu Ile
305 310 315 320

```
Arg Pro Cys Glu Gly Gly Gly Ser Ile Ile His Ile Val Asp His Met
                325                 330                 335
Asp Leu Glu Pro Trp Ser Val Pro Glu Val Leu Arg Pro Leu Tyr Glu
            340                 345                 350
Ser Ser Thr Val Leu Ala Gln Lys Met Thr Ile Ala Ala Leu Arg Arg
        355                 360                 365
Ile Arg Gln Ile Ala Gln Glu Val Thr Gly Glu Val Val Phe Gly Trp
    370                 375                 380
Gly Arg Gln Pro Ala Val Leu Arg Thr Phe Ser Gln Arg Leu Ser Arg
385                 390                 395                 400
Gly Phe Asn Glu Ala Val Asn Gly Phe Thr Asp Asp Gly Trp Ser Leu
                405                 410                 415
Met Gly Asn Asp Gly Met Glu Asp Val Thr Ile Ala Ile Asn Ser Ser
            420                 425                 430
Pro Ser Lys Leu Leu Gly Ser Gln Val Asn Ser Ser Asn Gly Leu Thr
        435                 440                 445
Ala Val Gly Gly Gly Ile Leu Cys Ala Lys Ala Ser Met Leu Leu Gln
    450                 455                 460
Asn Val Pro Pro Ala Leu Leu Val Arg Phe Leu Arg Glu His Arg Ser
465                 470                 475                 480
Glu Trp Ala Asp Cys Asn Ile Asp Ala Tyr Ser Ala Ala Ala Leu Lys
                485                 490                 495
Ala Ser Pro Tyr Ser Val Pro Gly Ser Arg Ala Gly Gly Phe Ser Gly
            500                 505                 510
Ser Gln Val Ile Leu Pro Leu Ala His Thr Val Glu His Glu Glu Phe
        515                 520                 525
Leu Glu Val Ile Lys Leu Glu Gly His Gly Leu Thr Gln Glu Glu Ala
    530                 535                 540
Val Leu Ser Arg Asp Met Phe Leu Leu Gln Leu Cys Ser Gly Ile Asp
545                 550                 555                 560
Glu Asn Ala Ala Gly Ala Cys Ala Gln Leu Val Phe Ala Pro Ile Asp
                565                 570                 575
Glu Ser Phe Ala Asp Asp Ala Pro Leu Leu Pro Ser Gly Phe Arg Val
            580                 585                 590
Ile Pro Leu Asp Ser Arg Thr Asp Gly Thr Ser Gly Pro Asn Arg Thr
        595                 600                 605
Leu Asp Leu Ala Ser Ala Leu Glu Val Gly Ser Ala Gly Thr Arg Thr
    610                 615                 620
Ser Gly Asp Ser Gly Ala Asn Ser Phe Asn Leu Arg Ser Val Leu Thr
625                 630                 635                 640
Ile Ala Phe Gln Phe Thr Tyr Glu Asn His Leu Arg Glu Asn Val Ala
                645                 650                 655
Ser Met Ala Arg Gln Tyr Val Arg Ser Val Val Ala Ser Val Gln Arg
            660                 665                 670
Val Ala Met Ala Leu Ala Pro Ser Arg Leu Ser Ser His Val Gly Pro
        675                 680                 685
Arg Leu Pro Pro Gly Thr Pro Glu Ala Leu Thr Leu Ala Arg Trp Ile
    690                 695                 700
Cys Gln Ser Tyr Arg Phe His Leu Gly Val Glu Leu Leu Arg Ala Asp
705                 710                 715                 720
Cys Glu Ala Ser Glu Ser Val Leu Lys Leu Leu Trp His His Ser Asp
                725                 730                 735
Ala Ile Val Cys Cys Ser Leu Lys Ser Leu Pro Val Phe Thr Phe Ala
            740                 745                 750
Asn Gln Ala Gly Leu Asp Met Leu Glu Thr Thr Leu Val Ala Leu Gln
        755                 760                 765
Asp Ile Ser Leu Asp Lys Ile Leu Asp Glu Asn Gly Arg Lys Ser Leu
    770                 775                 780
Cys Ser Asp Phe Ala Gln Ile Met Gln Gln Gly Tyr Ala Tyr Leu Pro
785                 790                 795                 800
Ala Gly Ile Cys Val Ser Ser Met Gly Arg Pro Val Ser Tyr Asp Arg
                805                 810                 815
Ala Val Ala Trp Lys Val Leu Asn Asp Ala Asp Ser Thr His Cys Met
            820                 825                 830
Val Phe Met Phe Met Asn Trp Ser Phe Met
        835                 840
```

<210> 228
<211> 2511
<212> DNA
<213> Gossypium barbadense
<400> 228

```
atggctatgg cgatgacaca tcatcacaac agggaaagca gcatagacaa gcatttagat      60
acaggcaagt atgttaggta cacagctgaa caagttgaag ctttggaacg tgtttatgct     120
gaatgtccaa aaccaagttc tttgcgtagg caacagttaa taagggaatg tcctattctt     180
tctaacattg agcctaaaca gttcaaagct ttgttccaaa atcgcaggtg tagagagaaa     240
caaaggaaag aagcttcaag gcttcaaaca gtaaatagaa aattaacagc aatgaataag     300
ctgttaatgg aagagaatga taggttgcaa aaacaggttt ctcagttggt ttgtgagaat     360
gggtacatga gacagcaatt gcatactgta aatgcatcgg cgactgatgc gagctgtgac     420
tctgcggtaa ccactcctca gcattcactg agaaatgcta ataaccctgc tggactcctc     480
tctatcgcgg aggagacctt ggcagagttc ctttctaagg ctacgggaac tgctgtcaat     540
tgggtccaga tgcctgggat gaagcctggt ccagattcag ttgggatatt tgccacttcc     600
caaagttgta gtggaatggc agctcgagct tgtggtcttg taagtttaga acctacaaag     660
attgcagaga ttcttaaaga tcgtccatct tggttccggg actgtcggaa gcttgaagtt     720
ttcaccatgt ttccagctgg caatggtggt acgattgagc ttgtatacac acagatgttt     780
gctcctacta cattggctcc tgcaagggac ttttggactc taaggtacac tacaacttta     840
gagaacggca gtctcgtggt gtgtgagagg tctctttcgg gttccggtgc tggcccgagt     900
gtggcttccg cagctcaatt tgtgagagct gaagtgctcc ctagtggcta tttgattagg     960
ccttgtgagg gtggagggtc gattatccat attgttgacc acttgaatct tgaggcatgg    1020
agtgtgccgg aggtcttgcg ccccctttat gaatcatcca gagtaattgc tcagaaaatg    1080
actattccgg cgctgcgcta tgttaggcag attgctcaag agacaagcgg cgaggtggtt    1140
tacagtttgg gcaggcagcc tgctgtgctt agaacattta gccaaagatt aagcaggggc    1200
ttcaatgagg caataaatgg attcaacgaa gatggctggt cgataatgaa ttgtgatggt    1260
acagaggatg tgataattgc tattaattcc ggcaagagct tgagcaacag ttcgaatttg    1320
accaccggtc tttcattcct cgggggcgtt ctatgtgcaa aggcatccat gctgcttcaa    1380
aatgttcctc ctgctgttct tgtccgattc ttgagggaac atcgtttgga atgggctgat    1440
ttcaatgtcg atgcttattc agctgcatca ttgaaggccg gaacatacac ttatcctgga    1500
atgagaccta caagttttac tgggagtcag atcatcatgc cactcggcca gacggtcgaa    1560
catgaagagc tgctcgaagt tataagactc gaaggccagt ctcttacgca agaagacgcg    1620
cttctatcaa gggacattca tctattacag atatgtagtg gaattgatga caatgcggtt    1680
ggggcctgtt cggagcttgt gtttgcacca atagatgaga tgtttccgga tgatgctgcc    1740
ttactacctt caggattccg cattatcccg ttggagtcaa aaccagattc gttggctaca    1800
aatcggactt tggatcttac ttcgagtctc gaagtggggc ctgcaacaag ccaagctgcc    1860
ggagattctc cgagtcagaa tgcacgatcg gtgttgacaa ttgccttcca gtttcccttc    1920
gatacaaatc ttcgggataa tgttgcgacc atggcacgcc agtatgtccg tagcgtcatt    1980
tcttctgtcc agaggrttgc tatggccata tctccatgyg gatcgagccc aactatagga    2040
ccaaagccat ctccaggttc tcccgaagcg cttacgttgg ctcattggat ctgccagagc    2100
tacagtttcc atttgggggga agagttgttg aaatccgaat cacttggtgg cgactcagta    2160
ttgaagaatc tttggcaaca tcaggatgca atattgtgtt gttcgttgaa gtctgtaccg    2220
gttttcatct tcgcaaatca ggccggtcta gacatgcttg agacaactct agtggatcta    2280
ccagacatca cactggacaa aatattcgat gagtcgggac ggaaggcatt gtgctctgat    2340
ttcaccaagt tgatgcagca gggattcact cacttgctgg ccggagtttg catgtcgaca    2400
atgggccgcc acgtctcgta tgaacaagct gttgcttgga aagtacttgc agctgatgca    2460
aacactgtcc actgcttggc attctctttt ataaactggt cttttgtgtg a              2511
```

<210> 229
<211> 836
<212> PRT
<213> Gossypium barbadense
<220>
<221> UNSURE
<222> (666)..(666)
<223> Xaa can be any naturally occurring amino acid
<400> 229

```
Met Ala Met Ala Met Thr His His His Asn Arg Glu Ser Ser Ile Asp
1               5                   10                  15
Lys His Leu Asp Thr Gly Lys Tyr Val Arg Tyr Thr Ala Glu Gln Val
            20                  25                  30
Glu Ala Leu Glu Arg Val Tyr Ala Glu Cys Pro Lys Pro Ser Ser Leu
        35                  40                  45
Arg Arg Gln Gln Leu Ile Arg Glu Cys Pro Ile Leu Ser Asn Ile Glu
    50                  55                  60
Pro Lys Gln Phe Lys Ala Leu Phe Gln Asn Arg Arg Cys Arg Glu Lys
65                  70                  75                  80
Gln Arg Lys Glu Ala Ser Arg Leu Gln Thr Val Asn Arg Lys Leu Thr
                85                  90                  95
Ala Met Asn Lys Leu Leu Met Glu Glu Asn Asp Arg Leu Gln Lys Gln
            100                 105                 110
Val Ser Gln Leu Val Cys Glu Asn Gly Tyr Met Arg Gln Gln Leu His
        115                 120                 125
Thr Val Asn Ala Ser Ala Thr Asp Ala Ser Cys Asp Ser Ala Val Thr
    130                 135                 140
Thr Pro Gln His Ser Leu Arg Asn Ala Asn Asn Pro Ala Gly Leu Leu
145                 150                 155                 160
```

```
Ser Ile Ala Glu Glu Thr Leu Ala Glu Phe Leu Ser Lys Ala Thr Gly
                165                 170                 175
Thr Ala Val Asn Trp Val Gln Met Pro Gly Met Lys Pro Gly Pro Asp
            180                 185                 190
Ser Val Gly Ile Phe Ala Thr Ser Gln Ser Cys Ser Gly Met Ala Ala
        195                 200                 205
Arg Ala Cys Gly Leu Val Ser Leu Glu Pro Thr Lys Ile Ala Glu Ile
    210                 215                 220
Leu Lys Asp Arg Pro Ser Trp Phe Arg Asp Cys Arg Lys Leu Glu Val
225                 230                 235                 240
Phe Thr Met Phe Pro Ala Gly Asn Gly Gly Thr Ile Glu Leu Val Tyr
                245                 250                 255
Thr Gln Met Phe Ala Pro Thr Thr Leu Ala Pro Ala Arg Asp Phe Trp
            260                 265                 270
Thr Leu Arg Tyr Thr Thr Thr Leu Glu Asn Gly Ser Leu Val Val Cys
        275                 280                 285
Glu Arg Ser Leu Ser Gly Ser Gly Ala Gly Pro Ser Val Ala Ser Ala
    290                 295                 300
Ala Gln Phe Val Arg Ala Glu Val Leu Pro Ser Gly Tyr Leu Ile Arg
305                 310                 315                 320
Pro Cys Glu Gly Gly Gly Ser Ile Ile His Ile Val Asp His Leu Asn
                325                 330                 335
Leu Glu Ala Trp Ser Val Pro Glu Val Leu Arg Pro Leu Tyr Glu Ser
            340                 345                 350
Ser Arg Val Ile Ala Gln Lys Met Thr Ile Pro Ala Leu Arg Tyr Val
        355                 360                 365
Arg Gln Ile Ala Gln Glu Thr Ser Gly Glu Val Val Tyr Ser Leu Gly
    370                 375                 380
Arg Gln Pro Ala Val Leu Arg Thr Phe Ser Gln Arg Leu Ser Arg Gly
385                 390                 395                 400
Phe Asn Glu Ala Ile Asn Gly Phe Asn Glu Asp Gly Trp Ser Ile Met
                405                 410                 415
Asn Cys Asp Gly Thr Glu Asp Val Ile Ile Ala Ile Asn Ser Gly Lys
            420                 425                 430
Ser Leu Ser Asn Ser Ser Asn Leu Thr Thr Gly Leu Ser Phe Leu Gly
        435                 440                 445
Gly Val Leu Cys Ala Lys Ala Ser Met Leu Leu Gln Asn Val Pro Pro
    450                 455                 460
Ala Val Leu Val Arg Phe Leu Arg Glu His Arg Leu Glu Trp Ala Asp
465                 470                 475                 480
Phe Asn Val Asp Ala Tyr Ser Ala Ala Ser Leu Lys Ala Gly Thr Tyr
                485                 490                 495
Thr Tyr Pro Gly Met Arg Pro Thr Ser Phe Thr Gly Ser Gln Ile Ile
            500                 505                 510
Met Pro Leu Gly Gln Thr Val Glu His Glu Glu Leu Leu Glu Val Ile
        515                 520                 525
Arg Leu Glu Gly Gln Ser Leu Thr Gln Glu Asp Ala Leu Leu Ser Arg
    530                 535                 540
Asp Ile His Leu Leu Gln Ile Cys Ser Gly Ile Asp Asp Asn Ala Val
545                 550                 555                 560
Gly Ala Cys Ser Glu Leu Val Phe Ala Pro Ile Asp Glu Met Phe Pro
                565                 570                 575
Asp Asp Ala Ala Leu Leu Pro Ser Gly Phe Arg Ile Ile Pro Leu Glu
            580                 585                 590
Ser Lys Pro Asp Ser Leu Ala Thr Asn Arg Thr Leu Asp Leu Thr Ser
        595                 600                 605
Ser Leu Glu Val Gly Pro Ala Thr Ser Gln Ala Ala Gly Asp Ser Pro
    610                 615                 620
Ser Gln Asn Ala Arg Ser Val Leu Thr Ile Ala Phe Gln Phe Pro Phe
625                 630                 635                 640
Asp Thr Asn Leu Arg Asp Asn Val Ala Thr Met Ala Arg Gln Tyr Val
                645                 650                 655
Arg Ser Val Ile Ser Ser Val Gln Arg Xaa Ala Met Ala Ile Ser Pro
            660                 665                 670
Cys Gly Ser Ser Pro Thr Ile Gly Pro Lys Pro Ser Pro Gly Ser Pro
        675                 680                 685
Glu Ala Leu Thr Leu Ala His Trp Ile Cys Gln Ser Tyr Ser Phe His
    690                 695                 700
Leu Gly Glu Glu Leu Leu Lys Ser Glu Ser Leu Gly Gly Asp Ser Val
705                 710                 715                 720
Leu Lys Asn Leu Trp Gln His Gln Asp Ala Ile Leu Cys Cys Ser Leu
```

```
                725                     730                     735
Lys Ser Val Pro Val Phe Ile Phe Ala Asn Gln Ala Gly Leu Asp Met
            740                     745                     750
Leu Glu Thr Thr Leu Val Asp Leu Pro Asp Ile Thr Leu Asp Lys Ile
            755                     760                     765
Phe Asp Glu Ser Gly Arg Lys Ala Leu Cys Ser Asp Phe Thr Lys Leu
    770                     775                     780
Met Gln Gln Gly Phe Thr His Leu Leu Ala Gly Val Cys Met Ser Thr
785                     790                     795                     800
Met Gly Arg His Val Ser Tyr Glu Gln Ala Val Ala Trp Lys Val Leu
                805                     810                     815
Ala Ala Asp Ala Asn Thr Val His Cys Leu Ala Phe Ser Phe Ile Asn
            820                     825                     830
Trp Ser Phe Val
        835
<210>  230
<211>  2526
<212>  DNA
<213>  Lycopersicon esculentum
<400>  230
atggctatgg tggctcaaca gcatagggag agtagtagtg gtagtattac taaacatctt      60
gatagtagtg gaaagtatgt tcgatatacg gctgagcaag ttgaggcttt ggagagggtt     120
tatgcagagt gtcctaagcc tagctcgttg cgtcgacagc aattgatccg tgaatgtcat     180
attctgtcga atatcgagcc taagcagatc aaagtttggt ttcagaacag aaggtgtcga     240
gagaagcaaa ggaaagagtc ttctcgattg cagactgtga acagaaagtt gtctgcgatg     300
aataaactgt tgatggagga gaatgaccgc ttgcagaaac aagtctcgca gcttgtatgt     360
gaaaatggct atatgcggca acaattgcaa agtgtatcgg cggccactac tgatgtaagt     420
tgtgaatcag tggtaaccac tcctcagcat tcccttagag atgctaacaa ccctgctgga     480
ctactaccaa ttgcagaaga aaccttggca gagttccttt ctaaggctac aggaactgct     540
gtcgattggg tcccgatgcc tgggatgaag cctggtccgg attcagttgg gatttttgcc     600
atctcacaca gttgcagtgg agtggcagcc cgagcatgtg gtcttgttag tttagagcca     660
acaaagattg ctgatatcct caaagatcga ccttcttggt tccgcgactg ccggaatgtt     720
gaagttatca caatgtttcc tgctggaaat ggtggtacag ttgagctttt gtatacccag     780
atatatgctc ccacaactct ggctcccgcg cgtgattttt ggacgctgag atacacaaca     840
accctagaca atggtagtct cgtggtttgt gaaagatccc tatctggtaa tgggcctggc     900
ccaaatccta ctgctgcttc ccagtttgta agagctcaaa tgcttccatc tggatatctg     960
atccgaccgt gtgatggtgg aggatcaatc atacatattg ttgatcacct gaatcttgag    1020
gcatggagtg cccctgagat tttgcgtcca ctctatgaat cgtcgaaagt tgtggcacag    1080
aaaatgacta ttgcagcact gcgatatgca aggcaactag ctcaagagac tagcgacgag    1140
gtagtatatg gtctaggaag gcaacctgct gttcttcgaa catttagcca gagattatgc    1200
agagggttca atgatgccat caatggattc ggtgacgatg gctggtcaat gttaagttca    1260
gatggtgctg aagatgtcat agttgctgtc aattcaagga agaacctcgc aaccacctcc    1320
attcctcttt ccccgcttgg tggcgtcctt tgtgccaaag catcaatgct actccagaat    1380
gtcccccctg ccgtactggt tcggtttctg agggagcacc gttcagagtg ggcagacttt    1440
aatgttgatg cttttgtagc ttctgcattg aagtcttgtc cgtatacata tcctgggatg    1500
aggcctacca gatttaccgg tagccagata ataatgccac ttggccacac aattgagcat    1560
gaagaaatgc ttgaagttat tagacttgaa gggcactcta ttggacagga agatgctttt    1620
atgccaagag atattcacct tttacagatg tgtagtggca ctgatgagaa tgcagttgga    1680
gcctgttctg aactagtttt tgcccctatt gatgagatgt ttccagatga tgcacctttg    1740
cttccctccg gatttcgcgt tattcctctc gaatcaaaat caggtgatgc ccaggatacg    1800
ttgaacgcac atagaacatt agatctagca tcaagtcttg aagtgggccc agcaacaaac    1860
tcgactactg gagatgcagc ttcttgttac agtgcacgat ctgtactgac aatcgctttc    1920
caatttccat ttgaggacaa tcttcaggac aatgtggcta ccatggcccg acagtatgtt    1980
cgcagtgtgg tttcgtctgt ccaacgagtt gccatggcaa tatctcctac gggaatgaat    2040
cctacactgg gggccaagct ttctccaggc tcaccagaag ctgtaacatt gtcgcattgg    2100
atctgccaga gctatagtta tcacatggga acagagttac ttcgagctga ttctagtggc    2160
gatgaatcag tactaaagaa tctctggcaa caccaggatg caattttgtg ctgctcattg    2220
aagtcgctgc cagttttcat ttttgctaat aaggccggac tcgacatgct ggagacaaca    2280
ttagttgcat tacaggacat ttctctagat aagatatttg atgaatccgg aaggaaagtg    2340
ttgctctcag aatttgccaa gattatggaa cagggtttcg cgtgtttgcc tggtggtatc    2400
tgcatgtcaa caatgggacg acatatttca tatgaacaag ctatcgcgtg gaaagtgttt    2460
gcgtcgtctg aagaaaatgc tgtccactgt ttggcctttt cgtttatcaa ctggtcattc    2520
gtgtaa                                                               2526
<210>  231
<211>  841
<212>  PRT
<213>  Lycopersicon esculentum
<400>  231
Met Ala Met Val Ala Gln Gln His Arg Glu Ser Ser Ser Gly Ser Ile
1               5                   10                  15
Thr Lys His Leu Asp Ser Ser Gly Lys Tyr Val Arg Tyr Thr Ala Glu
```

```
            20                  25                  30
Gln Val Glu Ala Leu Glu Arg Val Tyr Ala Glu Cys Pro Lys Pro Ser
        35                  40                  45
Ser Leu Arg Arg Gln Gln Leu Ile Arg Glu Cys His Ile Leu Ser Asn
    50                  55                  60
Ile Glu Pro Lys Gln Ile Lys Val Trp Phe Gln Asn Arg Arg Cys Arg
65                  70                  75                  80
Glu Lys Gln Arg Lys Glu Ser Ser Arg Leu Gln Thr Val Asn Arg Lys
                85                  90                  95
Leu Ser Ala Met Asn Lys Leu Leu Met Glu Glu Asn Asp Arg Leu Gln
            100                 105                 110
Lys Gln Val Ser Gln Leu Val Cys Glu Asn Gly Tyr Met Arg Gln Gln
        115                 120                 125
Leu Gln Ser Val Ser Ala Ala Thr Thr Asp Val Ser Cys Glu Ser Val
    130                 135                 140
Val Thr Thr Pro Gln His Ser Leu Arg Asp Ala Asn Asn Pro Ala Gly
145                 150                 155                 160
Leu Leu Pro Ile Ala Glu Glu Thr Leu Ala Glu Phe Leu Ser Lys Ala
                165                 170                 175
Thr Gly Thr Ala Val Asp Trp Val Pro Met Pro Gly Met Lys Pro Gly
            180                 185                 190
Pro Asp Ser Val Gly Ile Phe Ala Ile Ser His Ser Cys Ser Gly Val
        195                 200                 205
Ala Ala Arg Ala Cys Gly Leu Val Ser Leu Glu Pro Thr Lys Ile Ala
    210                 215                 220
Asp Ile Leu Lys Asp Arg Pro Ser Trp Phe Arg Asp Cys Arg Asn Val
225                 230                 235                 240
Glu Val Ile Thr Met Phe Pro Ala Gly Asn Gly Gly Thr Val Glu Leu
                245                 250                 255
Leu Tyr Thr Gln Ile Tyr Ala Pro Thr Thr Leu Ala Pro Ala Arg Asp
            260                 265                 270
Phe Trp Thr Leu Arg Tyr Thr Thr Thr Leu Asp Asn Gly Ser Leu Val
        275                 280                 285
Val Cys Glu Arg Ser Leu Ser Gly Asn Gly Pro Gly Pro Asn Pro Thr
    290                 295                 300
Ala Ala Ser Gln Phe Val Arg Ala Gln Met Leu Pro Ser Gly Tyr Leu
305                 310                 315                 320
Ile Arg Pro Cys Asp Gly Gly Gly Ser Ile Ile His Ile Val Asp His
                325                 330                 335
Leu Asn Leu Glu Ala Trp Ser Ala Pro Glu Ile Leu Arg Pro Leu Tyr
            340                 345                 350
Glu Ser Ser Lys Val Val Ala Gln Lys Met Thr Ile Ala Ala Leu Arg
        355                 360                 365
Tyr Ala Arg Gln Leu Ala Gln Glu Thr Ser Asp Glu Val Val Tyr Gly
    370                 375                 380
Leu Gly Arg Gln Pro Ala Val Leu Arg Thr Phe Ser Gln Arg Leu Cys
385                 390                 395                 400
Arg Gly Phe Asn Asp Ala Ile Asn Gly Phe Gly Asp Asp Gly Trp Ser
                405                 410                 415
Met Leu Ser Ser Asp Gly Ala Glu Asp Val Ile Val Ala Val Asn Ser
            420                 425                 430
Arg Lys Asn Leu Ala Thr Thr Ser Ile Pro Leu Ser Pro Leu Gly Gly
        435                 440                 445
Val Leu Cys Ala Lys Ala Ser Met Leu Leu Gln Asn Val Pro Pro Ala
    450                 455                 460
Val Leu Val Arg Phe Leu Arg Glu His Arg Ser Glu Trp Ala Asp Phe
465                 470                 475                 480
Asn Val Asp Ala Phe Val Ala Ser Ala Leu Lys Ser Cys Pro Tyr Thr
                485                 490                 495
Tyr Pro Gly Met Arg Pro Thr Arg Phe Thr Gly Ser Gln Ile Ile Met
            500                 505                 510
Pro Leu Gly His Thr Ile Glu His Glu Glu Met Leu Glu Val Ile Arg
        515                 520                 525
Leu Glu Gly His Ser Ile Gly Gln Glu Asp Ala Phe Met Pro Arg Asp
    530                 535                 540
Ile His Leu Leu Gln Met Cys Ser Gly Thr Asp Glu Asn Ala Val Gly
545                 550                 555                 560
Ala Cys Ser Glu Leu Val Phe Ala Pro Ile Asp Glu Met Phe Pro Asp
                565                 570                 575
Asp Ala Pro Leu Leu Pro Ser Gly Phe Arg Val Ile Pro Leu Glu Ser
            580                 585                 590
```

```
Lys Ser Gly Asp Ala Gln Asp Thr Leu Asn Ala His Arg Thr Leu Asp
        595                 600                 605
Leu Ala Ser Ser Leu Glu Val Gly Pro Ala Thr Asn Ser Thr Thr Gly
    610                 615                 620
Asp Ala Ala Ser Cys Tyr Ser Ala Arg Ser Val Leu Thr Ile Ala Phe
625                 630                 635                 640
Gln Phe Pro Phe Glu Asp Asn Leu Gln Asp Asn Val Ala Thr Met Ala
                645                 650                 655
Arg Gln Tyr Val Arg Ser Val Val Ser Ser Val Gln Arg Val Ala Met
            660                 665                 670
Ala Ile Ser Pro Thr Gly Met Asn Pro Thr Leu Gly Ala Lys Leu Ser
        675                 680                 685
Pro Gly Ser Pro Glu Ala Val Thr Leu Ser His Trp Ile Cys Gln Ser
    690                 695                 700
Tyr Ser Tyr His Met Gly Thr Glu Leu Leu Arg Ala Asp Ser Ser Gly
705                 710                 715                 720
Asp Glu Ser Val Leu Lys Asn Leu Trp Gln His Gln Asp Ala Ile Leu
                725                 730                 735
Cys Cys Ser Leu Lys Ser Leu Pro Val Phe Ile Phe Ala Asn Lys Ala
            740                 745                 750
Gly Leu Asp Met Leu Glu Thr Thr Leu Val Ala Leu Gln Asp Ile Ser
        755                 760                 765
Leu Asp Lys Ile Phe Asp Glu Ser Gly Arg Lys Val Leu Leu Ser Glu
    770                 775                 780
Phe Ala Lys Ile Met Glu Gln Gly Phe Ala Cys Leu Pro Gly Gly Ile
785                 790                 795                 800
Cys Met Ser Thr Met Gly Arg His Ile Ser Tyr Glu Gln Ala Ile Ala
                805                 810                 815
Trp Lys Val Phe Ala Ser Ser Glu Glu Asn Ala Val His Cys Leu Ala
            820                 825                 830
Phe Ser Phe Ile Asn Trp Ser Phe Val
        835                 840
```

<210> 232
<211> 258
<212> DNA
<213> Saccharum officinarum
<400> 232

```
atgaggatgt tcttccggca cttgctatct tgcatcctcc tgctcttgct aatgtcacac      60
ttgccaagcc cgatcctcgg cttgagaaca ttgagaggag aggaggcgaa gagcgacttg     120
aggcgccatg agcatgagct tccgccagcg gtatctcctt caaaagaggt ggacaacgat     180
gacgctgccg cctccagtaa gttcacagtt tcaagaagaa tggttcctca aggtccaaac     240
cctctccaca acagatga                                                   258
```

<210> 233
<211> 85
<212> PRT
<213> Saccharum officinarum
<400> 233

```
Met Arg Met Phe Phe Arg His Leu Leu Ser Cys Ile Leu Leu Leu Leu
1               5                   10                  15
Leu Met Ser His Leu Pro Ser Pro Ile Leu Gly Leu Arg Thr Leu Arg
            20                  25                  30
Gly Glu Glu Ala Lys Ser Asp Leu Arg Arg His Glu His Glu Leu Pro
        35                  40                  45
Pro Ala Val Ser Pro Ser Lys Glu Val Asp Asn Asp Asp Ala Ala Ala
    50                  55                  60
Ser Ser Lys Phe Thr Val Ser Arg Arg Met Val Pro Gln Gly Pro Asn
65                  70                  75                  80
Pro Leu His Asn Arg
                85
```

<210> 234
<211> 53
<212> DNA
<213> Artificial sequence
<220>
<223> primer: prm05843
<400> 234

```
ggggacaagt ttgtacaaaa aagcaggctt aaacaatgag gatgttcttc cgg             53
```

<210> 235
<211> 49
<212> DNA
<213> Artificial sequence

<220>
<223> primer: prm05844
<400> 235
ggggaccact ttgtacaaga aagctgggtt cctctcatct gttgtggag 49
<210> 236
<211> 668
<212> DNA
<213> Oryza sativa
<400> 236
ccttctacat cggcttaggt gtagcaacac gactttatta ttattattat tattattatt 60
attattttac aaaaatataa aatagatcag tccctcacca caagtagagc aagttggtga 120
gttattgtaa agttctacaa agctaattta aaagttattg cattaactta tttcatatta 180
caaacaagag tgtcaatgga acaatgaaaa ccatatgaca tactataatt ttgtttttat 240
tattgaaatt atataattca aagagaataa atccacatag ccgtaaagtt ctacatgtgg 300
tgcattacca aaatatatat agcttacaaa acatgacaag cttagtttga aaaattgcaa 360
tcccttatcac attgacacat aaagtgagtg atgagtcata atattatttt tcttgctacc 420
catcatgtat atatgatagc cacaaagtta ctttgatgat gatatcaaag aacattttta 480
ggtgcaccta acagaatatc caaataatat gactcactta gatcataata gagcatcaag 540
taaaactaac actctaaagc aaccgatggg aaagcatcta taaatagaca agcacaatga 600
aaatcctcat catccttcac cacaattcaa atattatagt tgaagcatag tagtagaatc 660
caacaaca 668
<210> 237
<211> 14
<212> PRT
<213> Artificial sequence
<220>
<223> CLE domain, consensus sequence
<220>
<221> VARIANT
<222> (1)..(1)
<223> /replace="Glu" /replace="Arg" /replace="Met" /replace="Pro" /replace="Leu
<220>
<221> VARIANT
<222> (2)..(2)
<223> /replace="Glu" /replace="Asp" /replace="Arg" /replace="Ser"
<220>
<221> VARIANT
<222> (4)..(4)
<223> /replace="Ile" /replace="Leu" /replace="Arg" /replace="Phe" /replace="Gln" /replace="Val" /replace="Met"
<220>
<221> VARIANT
<222> (5)..(5)
<223> /replace="Ile" /replace="Ser" /replace="Leu"
<220>
<221> VARIANT
<222> (6)..(6)
<223> /replace="Pro" /replace="Leu" /replace="Arg"
<220>
<221> VARIANT
<222> (7)..(7)
<223> /replace="Thr" /replace="Gln" /replace="Cys" /replace="Asn" /replace="Gly" /replace="Lys" /replace="Ser"
<220>
<221> VARIANT
<222> (8)..(8)
<223> /replace="Gly"
<220>
<221> VARIANT
<222> (9)..(9)
<223> /replace="Pro"
<220>
<221> VARIANT
<222> (10)..(10)
<223> /replace="Asn" /replace="Tyr"
<220>
<221> VARIANT
<222> (12)..(12)
<223> /replace="Leu" /replace="Gln" /replace="Arg" /replace="Tyr" /replace="His"

<400> 237
Ser Lys Arg Lys Val Pro Arg Asn Ser Asp Pro Ile His His
1 5 10
<210> 238
<211> 14
<212> PRT
<213> Artificial sequence
<220>
<223> CLE domain, consensus sequence
<220>
<221> VARIANT
<222> (1)..(1)
<223> /replace="Pro"
<220>
<221> VARIANT
<222> (2)..(2)
<223> /replace="Glu" /replace="Lys"
<220>
<221> VARIANT
<222> (4)..(4)
<223> /replace="Leu" /replace="Ile"
<220>
<221> VARIANT
<222> (5)..(5)
<223> /replace="Ser" /replace="Ile"
<220>
<221> VARIANT
<222> (7)..(7)
<223> /replace="Gly" /replace="Cys" /replace="Thr" /replace="Ser"
<220>
<221> VARIANT
<222> (10)..(10)
<223> /replace="Asp"
<220>
<221> VARIANT
<222> (12)..(12)
<223> /replace="Gln" /replace="His"
<220>
<221> VARIANT
<222> (14)..(14)
<223> /replace="Asn"
<400> 238
Ser Arg Arg Met Val Pro Gln Gly Pro Asn Pro Leu His His
1 5 10
<210> 239
<211> 477
<212> DNA
<213> Populus trichocarpa x Populus deltoides
<400> 239
gcaaattccc cctgctctaa aatccatttt cctatctatc aacctctctg gtctctatat 60
ccccaccatt ttcatcatga ggaataaaaa tcctcagctg ttccttattt tcctgatagt 120
gttcctggta ctcgttcatg gcaccacttg ccgcgatgcc aaaagatcta ctagcaatgg 180
agaaacagta caagggtcga aaaccaaaca ttcttcaatg tttctacaag cgctttcttc 240
catttttaag gcttcagaat ccagcactaa caacatcaag gcacttcaca ccgtctcgcg 300
caggcttgtt ccttgtggac caaacccact ccacaactga tcatcgaatc aacaaaattc 360
caatctgttt ccatttgttg aaatatatat agtgttttta aaatattttt gttgaataat 420
ctatacatat tttccctata catccagttt tgaaaaaaga aaaaaaaaaa aaaaaaa 477
<210> 240
<211> 87
<212> PRT
<213> Populus trichocarpa x Populus deltoides
<400> 240
Met Arg Asn Lys Asn Pro Gln Leu Phe Leu Ile Phe Leu Ile Val Phe
1 5 10 15
Leu Val Leu Val His Gly Thr Thr Cys Arg Asp Ala Lys Arg Ser Thr
20 25 30
Ser Asn Gly Glu Thr Val Gln Gly Ser Lys Thr Lys His Ser Ser Met
35 40 45
Phe Leu Gln Ala Leu Ser Ser Ile Phe Lys Ala Ser Glu Ser Ser Thr
50 55 60
Asn Asn Ile Lys Ala Leu His Thr Val Ser Arg Arg Leu Val Pro Cys

65 70 75 80
Gly Pro Asn Pro Leu His Asn
85

<210> 241
<211> 443
<212> DNA
<213> Oryza sativa
<400> 241
tctctctctc tcacacacac acacacacaa actagagact tatgccatga ggaggttctc 60
caagcagcac ctggtccctt tcattctgct cctgctactt gtcatgtctc acttgccaat 120
ctcaagcctt ggttcaagga gagcattcag agaagaagct gtcagtggtt tcagaagcca 180
tgagcttgct ccaaccatgg ctccttctca agagaaagaa gcaggcgtcg tcgccggcgc 240
cggtagcatc tgtggccaga agtatgctgt ctcaagaaga atggttcctc agggaccaaa 300
ccctctccac aactgatgaa ctcatgcact gcaatctgcc gtgatcatca gcttctccaa 360
tacaggtgaa tggtgcagcc atcattggtt acctttaagt ccgtctgctt aattaactag 420
atatttcata gtattttatc aca 443

<210> 242
<211> 89
<212> PRT
<213> Oryza sativa
<400> 242
Met Arg Arg Phe Ser Lys Gln His Leu Val Pro Phe Ile Leu Leu Leu
1 5 10 15
Leu Leu Val Met Ser His Leu Pro Ile Ser Ser Leu Gly Ser Arg Arg
20 25 30
Ala Phe Arg Glu Glu Ala Val Ser Gly Phe Arg Ser His Glu Leu Ala
35 40 45
Pro Thr Met Ala Pro Ser Gln Glu Lys Glu Ala Gly Val Val Ala Gly
50 55 60
Ala Gly Ser Ile Cys Gly Gln Lys Tyr Ala Val Ser Arg Arg Met Val
65 70 75 80
Pro Gln Gly Pro Asn Pro Leu His Asn
85

<210> 243
<211> 677
<212> DNA
<213> Saccharum officinarum
<400> 243
tttgaaactg ttacaactgg ttcgcctgca cctgcctgcc cggaacgacc cacgcgtccg 60
cgctctctct ctccctctcc ctctacctct ctctaacata tgccatgagg atgttcttcc 120
ggcactagct atcttgcatc ctactgctct aggtaatgtc acacttgcca agctcgatcc 180
tcggcttgag aacatcgaga ggagaggagg cgaagagcga cttgaggcgc catgagcatg 240
agcttccgcc agccgtatct ccttcaaaag aggtggacaa cgacgacgct gccgccgcca 300
gtaagttaac agtttcaaga agaatggttc ctcaaggtcc aaaccctctc cacaacagat 360
gagaggatcg gagcagcatg ctgctgctgc ttctggtatg gtcttcagcc aaggtagcag 420
ctagctagct cttctcagtc tcagctaatg gtgtagtgag tggcacgcac tcaacaaagt 480
acccttcctc tctttccttt ttttttaacc cgtcatatga atgatacaaa tggatgcata 540
tataagttga atactacttc ccatgtaatg tgtttttgtt gcattgattt catttatagg 600
cagctttgta catagatttt tatgatatta aggtgtaaaa gttgttgtgc tagatggatc 660
tagattttga atgtttg 677

<210> 244
<211> 68
<212> PRT
<213> Saccharum officinarum
<400> 244
Met Ser His Leu Pro Ser Ser Ile Leu Gly Leu Arg Thr Ser Arg Gly
1 5 10 15
Glu Glu Ala Lys Ser Asp Leu Arg Arg His Glu His Glu Leu Pro Pro
20 25 30
Ala Val Ser Pro Ser Lys Glu Val Asp Asn Asp Asp Ala Ala Ala Ala
35 40 45
Ser Lys Leu Thr Val Ser Arg Arg Met Val Pro Gln Gly Pro Asn Pro
50 55 60
Leu His Asn Arg
65

<210> 245
<211> 533
<212> DNA
<213> Arabidopsis thaliana
<400> 245
gcaacgagaa aaaccgtcct tggtgacaac tcatgcttgc actcaagcct taagctagct 60

```
aaacctatct cgcgcactac tagaattcaa ataaaactct ataaatagaa accctcatga     120
gatctcttct ttcctcatat acactcatac acaccacgtg aacaatctat ctctctttct     180
attgcttttc tatatataca gaaactaatt aattgtatct gtaatggcta agttaagctt     240
cactttctgc ttcttgttgt ttcttctgtt atcctcaatc gccgctggaa gccgccctct     300
tgagggggct cgggtcgggg tgaaggtgag aggcctaagc ccttctatcg aggctacgag     360
tccgactgta gaggatgatc aagctgcggg tagccatggg aaatctccag agcggttaag     420
cccaggagga cccgacccac aacatcacta gttattttgt gtttttcaat ttcttcgaca     480
tgtttattac ttatcaataa tttggttgca acgaagctgt ttttcttttt tgt            533
<210>  246
<211>  75
<212>  PRT
<213>  Arabidopsis thaliana
<400>  246
Met Ala Lys Leu Ser Phe Thr Phe Cys Phe Leu Leu Phe Leu Leu Leu
1               5                   10                  15
Ser Ser Ile Ala Ala Gly Ser Arg Pro Leu Glu Gly Ala Arg Val Gly
            20                  25                  30
Val Lys Val Arg Gly Leu Ser Pro Ser Ile Glu Ala Thr Ser Pro Thr
        35                  40                  45
Val Glu Asp Asp Gln Ala Ala Gly Ser His Gly Lys Ser Pro Glu Arg
    50                  55                  60
Leu Ser Pro Gly Gly Pro Asp Pro Gln His His
65                  70                  75
<210>  247
<211>  417
<212>  DNA
<213>  Brassica napus
<400>  247
ctttcatatt gtatctcccg cagccaaaca aaaacttttt ttttgacaaa gatagaacat      60
gaagatcaag agtttgatat tggcttcctc ttttctgatt cttgccttca ttcatcactc     120
agaatcagct tcatttcgga gtttgctgat gaagaatgga ttgtacgaag aagaagaagc     180
aaaaattcta ttgggcgact ccaaagaaac gataactaat tctacagctt tggagtctaa     240
acggataatt ccgacgggtc caaatccact tcacaacagg taactttgat catttaagaa     300
caagatatgt tgtgagtatg tctcttcctc tgtttttgtt acaagtatct atcttactgt     360
gtaatgtaat ggtgaatgta taatacattt tctaattaaa ttaccttatt taaaaaa        417
<210>  248
<211>  74
<212>  PRT
<213>  Brassica napus
<400>  248
Met Lys Ile Lys Ser Leu Ile Leu Ala Ser Ser Phe Leu Ile Leu Ala
1               5                   10                  15
Phe Ile His His Ser Glu Ser Ala Ser Phe Arg Ser Leu Leu Met Lys
            20                  25                  30
Asn Gly Leu Tyr Glu Glu Glu Glu Ala Lys Ile Leu Leu Gly Asp Ser
        35                  40                  45
Lys Glu Thr Ile Thr Asn Ser Thr Ala Leu Glu Ser Lys Arg Ile Ile
    50                  55                  60
Pro Thr Gly Pro Asn Pro Leu His Asn Arg
65                  70
<210>  249
<211>  774
<212>  DNA
<213>  Arabidopsis thaliana
<400>  249
aacagtttct atatttctct ctgtatctct ctcactcagt cactttctct ctaaaaaatg      60
gattctaaaa gctttgtgct actactacta ctcttctgct tcttgttcct tcatgatgct     120
tctggtctca ctctctcttt cactcctcta tatgtctata acccatgtaa atggattctt     180
ataagtctca acataacttt tttttgttat ttactatttc accagatctc actcaagctc     240
atgctcacgt tcaaggactt tccaaccgca aggttatctt caacttgtac tcattaaggc     300
ctctcagcat tcatgtgtta tgttcatgta gatgtccggt ccagttcaac aactgtttca     360
ttgctttagt tgtcacgaga aatatttgta tatattatta tggtgtgcaa aacatagtaa     420
aatgttgttc aattggcaga tgatgatgat gaaaatggaa agtgaatggg ttggagcaaa     480
tggagaagca gagaaggcaa agacgaaggg tttaggacta catgaagagt taaggactgt     540
tccttcggga cctgacccgt tgcaccatca tgtgaaccca ccaagacagc caagaaacaa     600
ctttcagctc ccttgaccta atctcttgtt gctttaaatt atttcatatt gtaaattact     660
ttctgcttta tcggttttac catttcggga gtcttttttg tgtgcaatct gtttcgtttg     720
gtgtagtttc atgaaagtga atgtaagata tgcattacgt ttgttgctga agtg           774
<210>  250
<211>  96
<212>  PRT
```

<213> Arabidopsis thaliana
<400> 250
Met Asp Ser Lys Ser Phe Val Leu Leu Leu Leu Leu Phe Cys Phe Leu
1 5 10 15
Phe Leu His Asp Ala Ser Asp Leu Thr Gln Ala His Ala His Val Gln
20 25 30
Gly Leu Ser Asn Arg Lys Met Met Met Met Lys Met Glu Ser Glu Trp
35 40 45
Val Gly Ala Asn Gly Glu Ala Glu Lys Ala Lys Thr Lys Gly Leu Gly
50 55 60
Leu His Glu Glu Leu Arg Thr Val Pro Ser Gly Pro Asp Pro Leu His
65 70 75 80
His His Val Asn Pro Pro Arg Gln Pro Arg Asn Asn Phe Gln Leu Pro
85 90 95
<210> 251
<211> 353
<212> DNA
<213> Oryza sativa
<400> 251
atggaaggtg taggtgctag gcagaggagg aaccctctga tacccagacc aaacggttca 60
aagaggcatc tgcagcatca gcatcagcca aatgctgccg agaagaagac cgccgcgaca 120
tcgaattact tcagtatcga ggcgttcctc gtgctcgtct tcctcaccat gtcattgctc 180
atacttccat tggtgcttcc cccattgcct ccgccgccat cgctgctgct gctgctgcca 240
gtctgcctgc tcatcctgct ggttgtgctg gccttcatgc caacggatgt gcggagcatg 300
gcttcctctt acttgtaaat acatctccta ggggaattta tttttgtttt tga 353
<210> 252
<211> 105
<212> PRT
<213> Oryza sativa
<400> 252
Met Glu Gly Val Gly Ala Arg Gln Arg Arg Asn Pro Leu Ile Pro Arg
1 5 10 15
Pro Asn Gly Ser Lys Arg His Leu Gln His Gln His Gln Pro Asn Ala
20 25 30
Ala Glu Lys Lys Thr Ala Ala Thr Ser Asn Tyr Phe Ser Ile Glu Ala
35 40 45
Phe Leu Val Leu Val Phe Leu Thr Met Ser Leu Leu Ile Leu Pro Leu
50 55 60
Val Leu Pro Pro Leu Pro Pro Pro Pro Ser Leu Leu Leu Leu Leu Pro
65 70 75 80
Val Cys Leu Leu Ile Leu Leu Val Val Leu Ala Phe Met Pro Thr Asp
85 90 95
Val Arg Ser Met Ala Ser Ser Tyr Leu
100 105
<210> 253
<211> 56
<212> DNA
<213> Artificial sequence
<220>
<223> primer: prm08170
<400> 253
ggggacaagt ttgtacaaaa aagcaggctt aaacaatgga aggtgtaggt gctagg 56
<210> 254
<211> 51
<212> DNA
<213> Artificial sequence
<220>
<223> primer: prm08171
<400> 254
ggggaccact ttgtacaaga aagctgggtc aaaaacaaaa ataaattccc c 51
<210> 255
<211> 2193
<212> DNA
<213> Oryza sativa
<400> 255
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag attttttta aaaaaataga 360

```
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga atttttttc ccaaatatct taaaaagtca    1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccatttttaa ttatatgaaa tgaactgtag cataagcagt attcatttgg    1920
attatttttt ttattagctc tcaccccttc attattctga gctgaaagtc tggcatgaac    1980
tgtcctcaat tttgttttca aattcacatc gattatctat gcattatcct cttgtatcta    2040
cctgtagaag tttctttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193
```

<210> 256
<211> 3
<212> PRT
<213> Artificial sequence
<220>
<223> conserved motif 1a
<400> 256
Tyr Phe Ser
1

<210> 257
<211> 3
<212> PRT
<213> Artificial sequence
<220>
<223> conserved motif 1b
<400> 257
Tyr Phe Thr
1

<210> 258
<211> 3
<212> PRT
<213> Artificial sequence
<220>
<223> conserved motif 1c
<400> 258
Tyr Phe Gly
1

<210> 259
<211> 3
<212> PRT
<213> Artificial sequence
<220>
<223> conserved motif 1d
<400> 259
Tyr Leu Gly
1

<210> 260
<211> 7
<212> PRT
<213> Artificial sequence

<220>
<223> conserved motif 2
<220>
<221> VARIANT
<222> (1)..(1)
<223> /replace = "Ala" /replace = "Ile"
<220>
<221> VARIANT
<222> (7)..(7)
<223> /replace = "Ser"
<400> 260
Val Leu Ala Phe Met Pro Thr
1 5
<210> 261
<211> 3
<212> PRT
<213> Artificial sequence
<220>
<223> conserved motif 3
<220>
<221> VARIANT
<222> (1)..(1)
<223> /replace = "Pro"
<400> 261
Ser Tyr Leu
1
<210> 262
<211> 178
<212> PRT
<213> Oryza sativa
<400> 262
Met Tyr Leu Leu Ser Pro Arg Asn Gly Asp Glu Glu Asp Glu Gln Glu
1 5 10 15
Glu Ile Gln Glu Leu Ile Ser Asp Asp Glu Pro Pro Asn Leu Lys Leu
20 25 30
Ala Ser Cys Ala Thr Ala Ala Ser Ser Ser Ser Ser Ser Gly Ser Asp
35 40 45
Met Glu Lys Gly Arg Gly Lys Ala Cys Gly Gly Gly Ser Thr Ala Pro
50 55 60
Pro Pro Pro Pro Pro Ser Ser Ser Gly Lys Ser Gly Gly Gly Gly Gly
65 70 75 80
Ser Asn Ile Arg Glu Ala Ala Ala Ser Gly Gly Gly Gly Gly Val Trp
85 90 95
Gly Lys Tyr Phe Ser Val Glu Ser Leu Leu Leu Leu Val Cys Val Thr
100 105 110
Ala Ser Leu Val Ile Leu Pro Leu Val Leu Pro Pro Leu Pro Pro Pro
115 120 125
Pro Ser Met Leu Met Leu Val Pro Val Ala Met Leu Val Leu Leu Leu
130 135 140
Ala Leu Ala Phe Met Pro Thr Thr Thr Ser Ser Ser Ser Ser Ala Gly
145 150 155 160
Gly Gly Gly Gly Gly Gly Arg Asn Gly Ala Thr Thr Gly His Ala Pro
165 170 175
Tyr Leu

<210> 263
<211> 126
<212> PRT
<213> Oryza sativa
<400> 263
Met Leu Leu Glu His Leu Met Ile Thr Met Glu Glu Gln Met Phe Arg
1 5 10 15
Glu Gln Gln Met Gln Arg Gly Gly Arg His His Gln His His Thr Thr
20 25 30
Arg Glu Gln Glu Gln Gln Gln Lys Gln Gln Gln Arg Arg Arg Leu Met
35 40 45
Asn Asn Ala Thr Asn Gly Gly Gly Gly Asp Gly Gly Ser Arg Cys Tyr
50 55 60
Phe Ser Thr Glu Ala Ile Leu Val Leu Ala Cys Val Thr Val Ser Leu
65 70 75 80
Leu Val Leu Pro Leu Ile Leu Pro Pro Leu Pro Pro Pro Pro Thr Leu

```
                85                  90                  95
Leu Leu Leu Leu Pro Val Cys Leu Leu Ala Leu Leu Val Val Leu Ala
            100                 105                 110
Phe Met Pro Thr Asp Met Arg Thr Met Ala Ser Ser Tyr Leu
        115                 120                 125
<210>  264
<211>  105
<212>  PRT
<213>  Zea mays
<220>
<221>  UNSURE
<222>  (65)..(75)
<223>  Xaa can be any naturally occurring amino acid
<400>  264
Met Ala Ser Arg Ser Ser Ala Met Glu Gly Gly Ala Ala Ile Gln Arg
1               5                   10                  15
Arg Asn Ala Val Lys Arg His Leu Gln Gln Arg Gln Gln Glu Ala Asp
            20                  25                  30
Phe Leu Asp Lys Lys Val Ile Ala Ser Thr Tyr Phe Ser Ile Gly Ala
        35                  40                  45
Phe Leu Val Leu Ala Cys Leu Thr Val Ser Leu Leu Ile Leu Pro Leu
    50                  55                  60
Xaa Xaa Xaa Xaa Xaa Xaa Xaa Xaa Xaa Xaa Xaa Leu Leu Trp Leu Pro
65                  70                  75                  80
Val Cys Leu Leu Val Leu Leu Val Val Leu Ala Phe Met Pro Thr Asp
                85                  90                  95
Val Arg Ser Met Ala Ser Ser Tyr Leu
            100                 105
<210>  265
<211>  110
<212>  PRT
<213>  Triticum aestivum
<400>  265
Met Asp Ser Gln Phe Gly Ala Leu Glu Arg Gly Gly Ser Arg Gln Arg
1               5                   10                  15
Arg Ser Pro Val Leu Ala Arg Pro Asn Thr Thr Lys Arg His Ile Gln
            20                  25                  30
Gln Gln Arg Ala Asn Ala Ala Asp Lys Lys Val Val Met Pro Asn Tyr
        35                  40                  45
Phe Ser Ile Glu Ala Phe Phe Val Leu Ala Cys Leu Thr Val Ser Leu
    50                  55                  60
Leu Ile Leu Pro Leu Val Leu Pro Pro Leu Pro Pro Pro Pro Ser Leu
65                  70                  75                  80
Leu Leu Phe Val Pro Val Cys Leu Leu Ile Leu Leu Met Val Leu Ala
                85                  90                  95
Phe Met Pro Thr Asp Met Arg Ser Met Ala Thr Ser Tyr Leu
            100                 105                 110
<210>  266
<211>  110
<212>  PRT
<213>  Hordeum vulgare
<400>  266
Met Asp Ser Gln Phe Gly Ala Met Asp Arg Gly Gly Ser Arg Gln Arg
1               5                   10                  15
Ser Ser Pro Val Leu Ala Arg Pro Asn Thr Ala Lys Arg Gln Met Gln
            20                  25                  30
Gln Gln Arg Ala Asn Ala Ala Asp Lys Lys Val Val Ile Pro Asn Tyr
        35                  40                  45
Phe Gly Val Glu Ala Phe Phe Val Leu Ala Cys Leu Thr Val Ser Leu
    50                  55                  60
Leu Ile Leu Pro Leu Val Leu Pro Pro Leu Pro Pro Pro Pro Ser Leu
65                  70                  75                  80
Leu Leu Leu Leu Pro Val Cys Leu Leu Ile Leu Leu Met Val Leu Ala
                85                  90                  95
Phe Met Pro Thr Asp Val Arg Ser Met Ala Thr Ser Tyr Leu
            100                 105                 110
<210>  267
<211>  99
<212>  PRT
<213>  Saccharum officinarum
<220>
```

<221> UNSURE
<222> (62)..(62)
<223> Xaa can be any naturally occurring amino acid
<400> 267

```
Met Glu Gly Gly Gly Gln Ile Gln Arg Arg Asn Asn Ala Val Lys Arg
1               5                   10                  15
His Leu Gln Gln Arg Gln Gln Glu Ala Asp Phe Leu Asp Lys Lys Val
            20                  25                  30
Ile Ala Ser Thr Tyr Phe Ser Ile Glu Ala Phe Leu Val Leu Ala Cys
        35                  40                  45
Leu Thr Val Ser Leu Leu Ile Leu Pro Leu Val Leu Pro Xaa Leu Pro
    50                  55                  60
Ala Pro Ala Ser Leu Leu Leu Trp Leu Pro Val Trp Leu Leu Glu Leu
65                  70                  75                  80
Leu Ile Val Leu Ala Phe Met Pro Thr Asp Val Arg Ser Met Ala Ser
                85                  90                  95
Ser Tyr Leu
```

<210> 268
<211> 99
<212> PRT
<213> Saccharum officinarum
<400> 268

```
Met Glu Gly Gly Gly Gln Ile Gln Arg Arg Asn Asn Ala Val Lys Arg
1               5                   10                  15
His Leu Gln Gln Arg Gln Gln Glu Ala Asp Phe Leu Asp Lys Lys Val
            20                  25                  30
Ile Ala Ser Thr Tyr Phe Ser Ile Glu Ala Phe Leu Val Leu Ala Cys
        35                  40                  45
Leu Thr Val Ser Leu Leu Ile Leu Pro Leu Val Leu Pro Pro Leu Pro
    50                  55                  60
Pro Pro Pro Ser Leu Leu Leu Trp Leu Pro Val Cys Leu Leu Ile Leu
65                  70                  75                  80
Leu Ile Val Leu Ala Phe Met Pro Thr Asp Val Arg Ser Met Ala Ser
                85                  90                  95
Ser Tyr Leu
```

<210> 269
<211> 107
<212> PRT
<213> Sorghum bicolor
<400> 269

```
Met Ala Ser Arg Ser Ser Ala Leu Glu Gly Gly Gly Ala Ala Ile Gln
1               5                   10                  15
Arg Arg Asn Asn Ala Val Lys Arg His Leu Gln Gln Arg Gln Gln Glu
            20                  25                  30
Ala Asp Phe His Asp Lys Lys Val Ile Ala Ser Thr Tyr Phe Ser Ile
        35                  40                  45
Gly Ala Phe Leu Val Leu Ala Cys Leu Thr Phe Ser Leu Leu Ile Leu
    50                  55                  60
Pro Leu Val Leu Pro Pro Leu Pro Pro Pro Pro Ser Leu Leu Leu Trp
65                  70                  75                  80
Leu Pro Val Cys Leu Leu Val Leu Leu Val Val Leu Ala Phe Met Pro
                85                  90                  95
Thr Asp Val Arg Ser Val Ala Ala Ser Tyr Leu
            100                 105
```

<210> 270
<211> 130
<212> PRT
<213> Arabidopsis thaliana
<400> 270

```
Met Ile Arg Glu Ile Ser Asn Leu Gln Lys Asp Ile Ile Asn Ile Gln
1               5                   10                  15
Asp Ser Tyr Ser Asn Asn Arg Val Met Asp Val Gly Arg Asn Asn Arg
            20                  25                  30
Lys Asn Met Ser Phe Arg Ser Ser Pro Glu Lys Ser Lys Gln Glu Leu
        35                  40                  45
Arg Arg Ser Phe Ser Ala Gln Lys Arg Met Met Ile Pro Ala Asn Tyr
    50                  55                  60
Phe Ser Leu Glu Ser Leu Phe Leu Leu Val Gly Leu Thr Ala Ser Leu
65                  70                  75                  80
```

Leu Ile Leu Pro Leu Val Leu Pro Pro Leu Pro Pro Pro Pro Phe Met
85 90 95
Leu Leu Leu Val Pro Ile Gly Ile Met Val Leu Leu Val Val Leu Ala
100 105 110
Phe Met Pro Ser Ser His Ser Asn Ala Asn Thr Asp Val Thr Cys Asn
115 120 125
Phe Met
130

<210> 271
<211> 135
<212> PRT
<213> Arabidopsis thaliana
<400> 271

Met Ile Arg Glu Phe Ser Ser Leu Gln Asn Asp Ile Ile Asn Ile Gln
1 5 10 15
Glu His Tyr Ser Leu Asn Asn Asn Met Asp Val Arg Gly Asp His Asn
20 25 30
Arg Lys Asn Thr Ser Phe Arg Gly Ser Ala Pro Ala Pro Ile Met Gly
35 40 45
Lys Gln Glu Leu Phe Arg Thr Leu Ser Ser Gln Asn Ser Pro Arg Arg
50 55 60
Leu Ile Ser Ala Ser Tyr Phe Ser Leu Glu Ser Met Val Val Leu Val
65 70 75 80
Gly Leu Thr Ala Ser Leu Leu Ile Leu Pro Leu Ile Leu Pro Pro Leu
85 90 95
Pro Pro Pro Pro Phe Met Leu Leu Leu Ile Pro Ile Gly Ile Met Val
100 105 110
Leu Leu Met Val Leu Ala Phe Met Pro Ser Ser Asn Ser Lys His Val
115 120 125
Ser Ser Ser Ser Thr Phe Met
130 135

<210> 272
<211> 139
<212> PRT
<213> Vitis vinifera
<400> 272

Met Ser Ile Glu Gln Pro Glu Ala Asp Ser Arg Leu Ser Glu Gly Pro
1 5 10 15
Leu Ile Asn Leu Gln Asp Arg Tyr Leu Ser Gly Ile Met Glu Ala Arg
20 25 30
Gly Arg Arg Asn Ser Ala Pro Leu Gln Val Glu Arg Lys Asn Pro Thr
35 40 45
Pro Pro Met Ala Glu Gly Lys Lys Met Glu Tyr Asn Arg Thr Pro Leu
50 55 60
Ser Arg Glu Asn Ser Arg Arg Leu Ile Pro Ala Ser Tyr Phe Ser Leu
65 70 75 80
Glu Ser Leu Leu Leu Leu Ile Cys Leu Thr Ala Ser Leu Leu Ile Leu
85 90 95
Pro Leu Ile Leu Pro Pro Leu Pro Pro Pro Pro Phe Met Leu Leu Leu
100 105 110
Leu Pro Ile Gly Ile Leu Ala Val Leu Met Ile Leu Ala Phe Met Pro
115 120 125
Ser Asn Val Arg Asp Leu Thr Tyr Thr Tyr Val
130 135

<210> 273
<211> 126
<212> PRT
<213> Citrus sp.
<220>
<221> UNSURE
<222> (103)..(103)
<223> Xaa can be any naturally occurring amino acid
<400> 273

Met Asn Ser Asp Asn Ser Glu Ser Arg Gln Arg Leu Ser Lys Gly Ile
1 5 10 15
Ile Asn Leu Gln Asp Arg Tyr Pro Thr Ser Ile Met Asp Arg Gly Val
20 25 30
Arg Lys Ile Ala Thr Pro Pro Val Glu Lys Arg Lys Val Glu Tyr His
35 40 45
Arg Ser Tyr Ser Gln Gly Ala Ser Arg Lys Leu Phe Ser Ala Ser Tyr
50 55 60

```
Phe Thr Leu Glu Ser Leu Leu Leu Leu Val Cys Leu Thr Ala Ser Leu
65                  70                  75                  80
Leu Ile Leu Pro Leu Val Leu Pro Pro Leu Pro Pro Pro Pro Phe Leu
                85                  90                  95
Leu Leu Leu Val Pro Ile Xaa Ile Leu Ala Val Leu Leu Val Leu Ala
            100                 105                 110
Phe Met Pro Ser Asn Val Arg Asp Ile Thr Ser Thr Tyr Val
        115                 120                 125
<210>  274
<211>  118
<212>  PRT
<213>  Lycorpersicon esculentum
<400>  274
Met Asn Met Asp Met Glu Ser Ser Glu Ala Lys Leu Arg Ser Ser Lys
1               5                   10                  15
Gly Phe Ile Asn Leu Glu Glu His Gln Gln Tyr Phe Asn Asn Ile Met
            20                  25                  30
Glu Gly Asn Lys Met Glu His Lys Arg Ser Phe Thr Gln Gly His Gly
        35                  40                  45
Lys Lys Met Leu Ser Met Asn Tyr Phe Ser Leu Glu Ser Ile Ile Leu
    50                  55                  60
Leu Leu Gly Leu Thr Ala Ser Leu Leu Leu Leu Pro Leu Met Leu Pro
65                  70                  75                  80
Pro Leu Pro Pro Pro Pro Phe Met Leu Leu Leu Val Pro Ile Phe Ile
                85                  90                  95
Leu Val Val Leu Met Ile Leu Ala Phe Met Pro Ser Asn Val Arg Asn
            100                 105                 110
Val Thr Cys Ser Tyr Leu
        115
<210>  275
<211>  87
<212>  PRT
<213>  Lycorpersicon esculentum
<400>  275
Met Glu Gly Asn Lys Met Glu His Lys Arg Ser Phe Thr Gln Gly His
1               5                   10                  15
Gly Lys Lys Met Leu Ser Met Asn Tyr Phe Ser Leu Glu Ser Ile Ile
            20                  25                  30
Leu Leu Leu Gly Leu Thr Ala Ser Leu Leu Leu Leu Pro Leu Met Leu
        35                  40                  45
Pro Pro Leu Pro Pro Pro Pro Phe Met Leu Leu Leu Val Pro Ile Phe
    50                  55                  60
Ile Leu Val Val Leu Met Ile Leu Ala Phe Met Pro Ser Asn Val Arg
65                  70                  75                  80
Asn Val Thr Cys Ser Tyr Leu
                85
<210>  276
<211>  106
<212>  PRT
<213>  Arabidopsis thaliana
<400>  276
Met Asp Val Gly Arg Asn Asn Arg Lys Asn Met Ser Phe Arg Ser Ser
1               5                   10                  15
Pro Glu Lys Ser Lys Gln Glu Leu Arg Arg Ser Phe Ser Ala Gln Lys
            20                  25                  30
Arg Met Met Ile Pro Ala Asn Tyr Phe Ser Leu Glu Ser Leu Phe Leu
        35                  40                  45
Leu Val Gly Leu Thr Ala Ser Leu Leu Ile Leu Pro Leu Val Leu Pro
    50                  55                  60
Pro Leu Pro Pro Pro Pro Phe Met Leu Leu Leu Val Pro Ile Gly Ile
65                  70                  75                  80
Met Val Leu Leu Val Val Leu Ala Phe Met Pro Ser Ser His Ser Asn
                85                  90                  95
Ala Asn Thr Asp Val Thr Cys Asn Phe Met
            100                 105
<210>  277
<211>  537
<212>  DNA
<213>  Oryza sativa
<400>  277
atgtacttgt tgagcccaag aaatggcgac gaggaggacg aacaggagga aatccaggag      60
```

```
ctgatcagcg acgacgagcc gcccaatctc aagttggcat cctgcgccac tgcagccagc    120
agcagcagca gcagcggcag cgacatggag aagggaagag gtaaagcctg cggcggcggg    180
agtacggcgc cgccgccgcc gccgccgtcg tcgtcaggta aatccggcgg cggcggcggc    240
agcaatatca gggaggcggc ggctagcggc ggcggcggcg gcgtgtgggg caagtacttc    300
tcggtggagt cgctgctcct gctggtgtgc gtgacggcgt cgctggtgat cctcccgctc    360
gtgctgccgc cgctgccccc gccgccgtcg atgctgatgc tggtgccggt ggcgatgctg    420
gtgctgctgc tggcgctggc gttcatgccg acgacgacgt cgtcgtcgtc gtccgccggc    480
ggcggcggcg gcggcggccg caatggggcg acgacgggac atgctcccta cttgtag       537
<210>  278
<211>  538
<212>  DNA
<213>  Zea mays
<220>
<221>  misc_feature
<222>  (177)..(208)
<223>  n is a, c, g, or t
<220>
<221>  misc_feature
<222>  (493)..(493)
<223>  n is a, c, g, or t
<400>  278
ttcacattac actcatgact cgtcttagga cgaatcctgc agctgcaaaa cacagaaaat     60
ctggccaaga cctatttatc tatttacagg taagaggagg ccatgctgcg cacatctgtc    120
ggcatgaagg ccagtacaac cagcaagacg agcaggcaga ccggcagcca cagcagnnnn    180
nnnnnnnnnn nnnnnnnnnn nnnnnnnncc agcggcagta tcagcagcga gacggtgagg    240
caggcgagca cgaggaatgc cccgatgctg aagtaggtgg acgcgatgac cttcttgtcg    300
aggaaatccg cctcctgctg acgctgctgc agatgccgct tcacggcatt cctcctttgt    360
attgccgccc ctccttccat cgcgctagat cggcttgcca tgtgttcttt ggtgtaggcg    420
gaggtggaga ccagtcgcag tgagtggttg ccaaagtaag caagaagtga aaggcggtgt    480
agaaccgcct tgngttttct gaacgttttg taatcagatc tgagctcggg tggtcgaa      538
<210>  279
<211>  578
<212>  DNA
<213>  Vitis vinifera
<400>  279
tttttttttt tttttttaat caagaaataa aataactgat tttcatctga atatcaagac     60
aaataacaaa aattgtatga tgagaagagg tgcacttttg aacaccacca tttacacata    120
tgtataagtc aaatctctga cattagaagg catgaaagcc aagatcataa gcactgctag    180
aatgccaatg gggagcagaa gcagcatgaa aggagggggt ggcaatggtg gtagtatcag    240
gggaaggatc agcaatgaag ccgtgagaca gatgagcaaa agcaatgact ccaagctgaa    300
atagcttgct gggatcagtc tcctgctgtt ctctcgtgag aggggagttc tattatattc    360
catcttcttt ccttcggcca taggaggagt agggtttttc ctctcaactt gcagaggagc    420
agagttcctc cttcctctcg cttccatgat gccactcaaa tatcgatctt gcagattgat    480
caaaggtcct tcagacagtc ttgaatctgc ttcaggctgc tcaatactca tttaaatttc    540
tcctttaccg gtcaccaagt tccaaccggt tcaaagta                            578
<210>  280
<211>  704
<212>  DNA
<213>  Citrus reticulata
<220>
<221>  misc_feature
<222>  (619)..(619)
<223>  n is a, c, g, or t
<400>  280
agggtttctt caaagatagg tagccatttg cacatttgaa tctgcttgtt ggatattgtc     60
aaggaggctg ttggaattag gccacatttc agaatctggt ttcatcctgg atcgctgggc    120
atttgaaggc attttgtgat catcgctgtt taaaatttgg ccgcatatta gaatctgggt    180
tctcatcggt tttccgtaca tttaccttga ccacattttg atatctgggt tgagctgcat    240
tttagccttc gtatttaaaa ggacttgatc taatctgggg tcttggtgag ccggggcaac    300
tgatcatagt aaatgaattc tgataattct gagtcgagac agagactatc aaagggcatt    360
ataaacttgc aagatcgata tccgaccagc attatggatc gtggtgtaag aaaaattgca    420
actcctccgg tcgagaagag gaaagttgag tatcaccgaa gttactcgca aggggcatcc    480
agaaaactgt tttcggcaag ctatttcacc ctggaatcat tgcttttgct cgtatgtctg    540
acggcctcat tgctgatcct gccattggtg cttccgccct tgccgccccc gccattcctg    600
ctgcttctgg ttcctatang tattctagcc gtgcttttgg tcttggcatt catgccttct    660
aatgtaagag atataacttc cacgtacgtg taaatggtgt tgct                     704
<210>  281
<211>  382
<212>  DNA
<213>  Lycorpersicon esculentum
<400>  281
```

gaaaaaaatg tatttaatca ttatgtaaaa aacaagtgaa tctactttga tattttcttc 60
taaattaaac cacacaatta aagatatgag caagtcacat tcctaacatt agaaggcata 120
aaagctaaga tcataagaac aacaagaatg aaaattggga ctaacaacaa cataaaaggt 180
ggtggtggca atggtggaag catcaatggc aaaagtaaca aagatgctgt aagaccaagt 240
aacaaaataa ttgactctaa gctaaaataa ttcattgaca acattttctt gccatgtcct 300
tgtgtaaatg atctcttatg ctccatctta ttgccttcca taatgttgtt gaaatattgt 360
tgatgttcct ccaaattaat aa 382

<210> 282
<211> 624
<212> DNA
<213> Lycorpersicon esculentum
<400> 282

tttgttaaag attggcacat tttcaagttc agtattcatt cgatttttga tatctacata 60
aaaaaaaagt gtcctggtac tactcaatat tcctcagaac gacttcatat tcaggtctcg 120
aattcaaaac ctcacatcaa gattcttagg aaatttcaag attggttgaa aaactcatat 180
ccttctctaa gtttcaagat tggttccaaa ttaaaactcg agacttctga gtaagagcgt 240
acgactagta atgaacatgg acatggaatc atcagaggca aaattgagat catcaaaagg 300
gtttattaat ttggaggaac atcaacaata tttcaacaac attatggaag gcaataagat 360
ggagcataag agatcattta cacaaggaca tggcaagaaa atgttgtcaa tgaattattt 420
tagcttagag tcaattattt tgttacttgg tcttacagca tctttgttac ttttgccatt 480
gatgcttcca ccattgccac caccaccttt tatgttgttg ttagtcccaa ttttcattct 540
tgttgttctt atgatcttag cttttatgcc ttctaatgtt aggaatgtga cttgctcata 600
tctttaattg tgtggtttaa ttta 624

<210> 283
<211> 1130
<212> DNA
<213> Oryza sativa
<400> 283

catgcggcta atgtagatgc tcactgcgct agtagtaagg tactccagta cattatggaa 60
tatacaaagc tgtaatactc gtatcagcaa gagagaggca cacaagttgt agcagtagca 120
caggattaga aaaacgggac gacaaatagt aatggaaaaa caaaaaaaaa caaggaaaca 180
catggcaata taaatggaga aatcacaaga ggaacagaat ccgggcaata cgctgcgaaa 240
gtactcgtac gtaaaaaaaa gaggcgcatt catgtgtgga cagcgtgcag cagaagcagg 300
gatttgaaac cactcaaatc caccactgca aaccttcaaa cgaggccatg gtttgaagca 360
tagaaagcac aggtaagaag cacaacgccc tcgctctcca ccctcccacc caatcgcgac 420
gcacctcgcg gatcggtgac gtggcctcgc cccccaaaaa tatcccgcgg cgtgaagctg 480
acaccccggg cccacccacc tgtcacgttg gcacatgttg gttatggttc ccggccgcac 540
caaaatatca acgcggcgcg gcccaaaatt tccaaaatcc cgcccaagcc cctggcgcgt 600
gccgctcttc cacccaggtc cctctcgtaa tccataatgg cgtgtgtacc ctcggctggt 660
tgtacgtggg cgggttaccc tgggggtgtg ggtggatgac gggtgggccc ggaggaggtc 720
cggccccgcg cgtcatcgcg gggcggggtg tagcgggtgc gaaaaggagg cgatcggtac 780
gaaaattcaa attaggaggt ggggggcggg gcccttggag aataagcgga atcgcagata 840
tgcccctgac ttggcttggc tcctcttctt cttatccctt gtcctcgcaa ccccgcttcc 900
ttctctcctc tcctcttctc ttctcttctc tggtggtgtg ggtgtgtccc tgtctcccct 960
ctccttcctc ctctcctttc ccctcctctc ttcccccctc tcacaagaga gagagcgcca 1020
gactctcccc aggtgaggtg agaccagtct ttttgctcga ttcgacgcgc ctttcacgcc 1080
gcctcgcgcg gatctgaccg cttccctcgc ccttctcgca ggattcagcc 1130

<210> 284
<211> 77
<212> PRT
<213> Medicago sativa
<400> 284

Met Val Arg Cys Phe Ser Leu Gly Ser Val Leu Ile Leu Ile Ala Leu
1 5 10 15
Ala Ala Ser Met Val Val Leu Pro Leu Met Leu Pro Pro Leu Pro Pro
20 25 30
Pro Pro Leu Ala Leu Leu Phe Phe Pro Val Gly Ile Met Ala Ala Leu
35 40 45
Val Val Leu Ala Phe Ser Pro Ser Glu Asn Val Lys Asn Val Val Val
50 55 60
Tyr Ser Ser Ser Ser Ser Gly Ile Ala Asn Ser Lys Arg
65 70 75

<210> 285
<211> 78
<212> PRT
<213> Medicago sativa
<400> 285

Met Ile Met Val Ala Ser Lys Glu Lys Thr Asn Ser Gly Gly Cys Met
1 5 10 15
Phe Arg Tyr Ser Val Leu Ile Leu Ser Leu Leu Ala Leu Ser Ile Leu
20 25 30

```
Val Leu Pro Leu Val Met Pro Pro Leu Pro Pro Pro Pro Leu Leu Leu
        35                  40                  45
Leu Leu Val Pro Val Phe Ile Met Leu Leu Leu Phe Phe Ile Ala Phe
    50                  55                  60
Ser Pro Ser Lys Lys Val Pro Asn Lys Ala Ser Phe Val Ser
65                  70                  75
<210>  286
<211>  613
<212>  DNA
<213>  Hordeum vulgare
<400>  286
cttccgagaa agatgcttca tattgcactc atcttatgcc aacacacaat cagaaacaaa      60
aaccacgcag caagcctatt tacaagtaag aggtggccat gctccgcaca tcagtcggca     120
tgaaggccag caccatcaac aggatgagca agcagaccgg caagagcagc agtagcgatg     180
gcggtggggg caacggaggc agcaccaatg gcagtatgag caatgaaacg gtgaggcagg     240
cgagcacgaa gaacgcttcg acgccgaagt agttcggtat gacaaccttc ttgtcagcag     300
catttgccct ctgctgctgc atttgcctct ttgcagtatt tggtctggct aacacagggc     360
tgctcctctg cctactaccg cctctgtcca ttgcaccgaa ctggctatcc atctattctt     420
tggtgagtgc agatcagcgg ctatccagga actgagatga ataagaactt gtggaatctg     480
aaggttttta acagatctga agtctttgtg agtccgtgac tgaactgcag atcatctgct     540
gtggaagaac tgcaccgcaa gtggcaatac cttcgatcct gaaacttgca ttttggtgat     600
gcccgtacca cgc                                                        613
<210>  287
<211>  617
<212>  DNA
<213>  Saccharum officinarum
<220>
<221>  misc_feature
<222>  (451)..(451)
<223>  n is a, c, g, or t
<400>  287
ggagaacgtg cttgttcagg tggttcaaca gtgcggacca gagaacaatg cgaggttcag      60
gattaaaggt attctggctt cagaggcagt tcttccaaag caagtgacag gcgattccat     120
caccggagct aagatcttat tacaacattc agaaacacag gagtcctccc accgcctttc     180
acttcttgct tacttctgca accactcgcc gtgactgatc tccacgcaca ccaaagaaca     240
caacacgtgg caagccgatc tagcgcgatg gaaggagggg ggcaaataca gaggaggaat     300
aatgccgtga agcggcacct gcagcagcgg cagcaggagg cggatttcct cgacaagaag     360
gtcatcgcgt ccacctattt cagcatcgag gcgttcctcg tgctcgcctg cctcaccgtc     420
tcgctgctga tactgccgct tgtgctgccg ncgctgccgg cgccggcgtc gctgctgctg     480
tggctgccgg tctggctgct cgaactgctg attgtgcttg ccttcatgcc gacagatgtg     540
cgcagcatgg cctcctctta cttgtaaaaa aatagataaa taggccacct ttggcaattt     600
tctggggttt ggaggtg                                                    617
<210>  288
<211>  638
<212>  DNA
<213>  Saccharum officinarum
<400>  288
gatttttttcc aagccagtga ccggcgattc atcaaccgga gctgagatct tatacaacat      60
tcagaaacac aggagtcctc gcaccgcttt ccactcttgg ctaattttgc aaccactcgc     120
cgtgactgat ctccacgcac accaaagaac acaacacgtg gcaagccgat ctagcgcgat     180
ggaaggaggg gggcaaatac agaggaggaa taatgccgtg aagcggcacc tgcagcagcg     240
gcagcaggag gcggatttcc tcgacaagaa ggtcatcgcg tccacctatt tcagcatcga     300
ggcgttcctc gtgctcgcct gcctcaccgt ctcgctgctg atactgccgc tggtgctgcc     360
gccgctgccg ccgccgccgt cgctgctgct gtggctgccg gtctgcctgc tcatcctgct     420
gattgtgctg gccttcatgc cgacagatgt gcgcagcatg gcctcctctt acttgtaata     480
aatagataaa taggccacct tggtcaatat tctgtgattt ggaggtgatt cgtcctgaga     540
tgagtctcga ttgatgtcag ctactcccaa ggggaaatgc atgtaacact tggtggccga     600
cggtggcaag ataatcatgc taccatgtca gttaaacc                             638
<210>  289
<211>  778
<212>  DNA
<213>  Sorghum bicolor
<400>  289
gctgttggtg ttgttcttga gatctctttc ttgatcttgt gtgggattaa agagggattc      60
ttgccttcct acgggagaaa gagaaaaggg gaagaacgtg cttgttccgg tggttcaaca     120
gtgcggagac ccggagaaca atgcgaggtt caggattaaa ggtgttctgg cttcaggtgc     180
agttcttcca aagcaggtga caggcgattc gatcaccgga gctcagatct gacgaaaaca     240
aaacacagtc ctcctcccac cgcctttcag ttcttgctta cttctgcaac cactcactgc     300
gaccgtacac caaagaacac tgcacatggc aagccgatct agcgcgctgg aaggaggggg     360
ggcagcaata cagcggagga ataatgccgt gaagcggcac ctgcagcagc ggcagcagga     420
ggcggatttc cacgacaaga aggtcatcgc gtccacctac ttcagcatcg gcgcgttcct     480
```

```
ggtgctcgcc tgcctcacct tctcgctgct catcctgccg ctggtgctgc cgccgctgcc     540
gccgccgccg tcgctgctgc tgtggctgcc ggtctgcctg ctcgtcctgc tggttgtgct     600
ggccttcatg ccgacagatg tgcgcagcgt ggcggcctct tacttgtaaa tagccagata     660
aataggccac cacctttggc cagttttctg tgttttcggg ggtgattcgt cctaagatga     720
gtcatgatcg agtgtaatgt gaagcatctt ttccaggggt agtagatttc aatgaagt       778
<210>  290
<211>  732
<212>  DNA
<213>  Arabidopsis thaliana
<400>  290
ttgtcttcct catttcccta ctagtacttg tttcacacag tttcttgatc caaccaaaac      60
caatacacaa agcttctcaa actccttcac ctcaaagctt cttcctttac atctgaatcg     120
ttgagttaac tcggatttgt tctgcatcct ctgtttctga atcgtgggcc atccttattt     180
tgtctcgaat tcttcaccaa ttgcttcgat caagctgcat tggttaacca gttgccctaa     240
agatcagatc tttgagcaaa attttgtcac tgatcttcta aatccaaacc agacacagca     300
aaacaacctc tgtagatgat tcgagaaatc tcaaacttac aaaaagatat tataaacatt     360
caagacagtt attcgaacaa ccgagtcatg gacgtcggaa gaaacaaccg gaaaaacatg     420
agctttcgaa gttcgccgga gaaaagcaag caagagttac ggcggagttt ctcggcgcag     480
aaaaggatga tgatcccggc gaattatttc agtttagagt ctctgttcct attggttggt     540
ctaacggcat ctctgttaat acttccgtta gttttgccgc cgttacctcc gcctccgttt     600
atgctgctat tggttcccat tgggattatg gttttactcg tcgttcttgc cttcatgcct     660
tcttctcatt ctaatgctaa tacagatgta acttgcaatt tcatgtaaat ctgaaattta     720
ttatatgatg at                                                         732
<210>  291
<211>  891
<212>  DNA
<213>  Arabidopsis thaliana
<400>  291
cccactttat ttcttcttct ctggttttct taccaaaaga aactttcttc gtcttcctct      60
gtatttaagc tttaacaccc tgtttttggt ttccaacgtt caatcttcat cttcttctcg     120
ctgaaggtgt gtttggctct aacggtttaa agctttcttg aaacaccaat tgaatctttt     180
ctctctaccg gcaaaaaaaa aagattagtc cttttaggtc tggaaacgcc aagatcactc     240
gttctaaacc ttagattttg tctgcatttc gggataatca tttcatcgtc agggttcttc     300
aaccaaacta catttacaga agaagaagaa gaagaaaagt tcgttacttt ttatgcgttt     360
ggataaacaa actcaagttt cttcttcata catcgatctg attttccaga tcaaacttcg     420
aaaagagaaa aagccttctt taaatgattc gtgagttctc cagtctacaa aacgacatca     480
taaacattca agaacattat tctctcaaca acaacatgga cgtgagagga gatcataacc     540
ggaaaaacac gagttttcgt ggttcagctc cagctccgat tatggggaag caagaattgt     600
ttcggacatt gtcgtcgcag aacagtccaa ggaggctaat atcagcgagt tacttcagtt     660
tagaatcaat ggttgtgctt gttggtctca cagcatctct cttgatctta ccgttgattc     720
ttccaccatt gcctcctcct ccttttatgc tgcttttgat tcctattggg attatggttt     780
tgcttatggt tcttgctttc atgccttctt ctaattccaa acatgtttct tcttcttcca     840
cttttatgta ataaacgttt ctttaattga agaaagaaat ccttaaacaa a              891
<210>  292
<211>  732
<212>  DNA
<213>  Arabidopsis thaliana
<400>  292
ttgtcttcct catttcccta ctagtacttg tttcacacag tttcttgatc caaccaaaac      60
caatacacaa agcttctcaa actccttcac ctcaaagctt cttcctttac atctgaatcg     120
ttgagttaac tcggatttgt tctgcatcct ctgtttctga atcgtgggcc atccttattt     180
tgtctcgaat tcttcaccaa ttgcttcgat caagctgcat tggttaacca gttgccctaa     240
agatcagatc tttgagcaaa attttgtcac tgatcttcta aatccaaacc agacacagca     300
aaacaacctc tgtagatgat tcgagaaatc tcaaacttac aaaaagatat tataaacatt     360
caagacagtt attcgaacaa ccgagtcatg gacgtcggaa gaaacaaccg gaaaaacatg     420
agctttcgaa gttcgccgga gaaaagcaag caagagttac ggcggagttt ctcggcgcag     480
aaaaggatga tgatcccggc gaattatttc agtttagagt ctctgttcct attggttggt     540
ctaacggcat ctctgttaat acttccgtta gttttgccgc cgttacctcc gcctccgttt     600
atgctgctat tggttcccat tgggattatg gttttactcg tcgttcttgc cttcatgcct     660
tcttctcatt ctaatgctaa tacagatgta acttgcaatt tcatgtaaat ctgaaattta     720
ttatatgatg at                                                         732
<210>  293
<211>  1130
<212>  DNA
<213>  Oryza sativa
<400>  293
catgcggcta atgtagatgc tcactgcgct agtagtaagg tactccagta cattatggaa      60
tatacaaagc tgtaatactc gtatcagcaa gagagaggca cacaagttgt agcagtagca     120
caggattaga aaaacgggac gacaaatagt aatggaaaaa caaaaaaaaa caaggaaaca     180
catggcaata taaatggaga aatcacaaga ggaacagaat ccgggcaata cgctgcgaaa     240
gtactcgtac gtaaaaaaaa gaggcgcatt catgtgtgga cagcgtgcag cagaagcagg     300
```

```
gatttgaaac cactcaaatc caccactgca aaccttcaaa cgaggccatg gtttgaagca    360
tagaaagcac aggtaagaag cacaacgccc tcgctctcca ccctcccacc caatcgcgac    420
gcacctcgcg gatcggtgac gtggcctcgc cccccaaaaa tatcccgcgg cgtgaagctg    480
acaccccggg cccacccacc tgtcacgttg gcacatgttg gttatggttc ccggccgcac    540
caaaatatca acgcggcgcg gcccaaaatt tccaaaatcc cgcccaagcc cctggcgcgt    600
gccgctcttc cacccaggtc cctctcgtaa tccataatgg cgtgtgtacc ctcggctggt    660
tgtacgtggg cgggttaccc tgggggtgtg ggtggatgac gggtgggccc ggaggaggtc    720
cggccccgcg cgtcatcgcg gggcggggtg tagcgggtgc gaaaaggagg cgatcggtac    780
gaaaattcaa attaggaggt gggggcgggg gcccttggag aataagcgga atcgcagata    840
tgcccctgac ttggcttggc tcctcttctt cttatccctt gtcctcgcaa ccccgcttcc    900
ttctctcctc tcctcttctc ttctcttctc tggtggtgtg ggtgtgtccc tgtctcccct    960
ctccttcctc ctctcctttc ccctcctctc ttcccccctc tcacaagaga gagagcgcca   1020
gactctcccc aggtgaggtg agaccagtct ttttgctcga ttcgacgcgc ctttcacgcc   1080
gcctcgcgcg gatctgaccg cttccctcgg ccttctcgca ggattcagcc              1130
```